# EUROPEAN PATENT APPLICATION

(11) **EP 4 215 519 A1**
(43) Date of publication of application: **26.07.2023**
(21) Application number: 21869370.3
(22) Date of filing: 14.09.2021
(51) Int. Cl.: C07D 205/04, A61K 31/59, A61K 47/54, A61P 9/00, A61P 25/00, A61P 25/16, A61P 25/18, A61P 25/22, A61P 25/28, C07D 207/06, C07D 207/08, C07D 207/10, C07D 207/12, C07D 211/38, C07D 211/48, C07D 241/04, C07D 265/30, C07D 265/34, C07D 295/096, C07D 295/26

(54) **VITAMIN D DERIVATIVE HAVING CYCLIC AMINE IN SIDE CHAIN**

(30) Priority: 15.09.2020 JP 2020154545; 01.06.2021 JP 2021092346
(71) Applicant: Teijin Pharma Limited, Tokyo 100-0013 (JP)
(72) Inventor: SAITOH, Hiroshi, Tokyo 100-0013 (JP); HORIE, Kyohei, Tokyo 100-0013 (JP); SUGA, Akiko, Tokyo 100-0013 (JP); KAIBARA, Yuya, Tokyo 100-0013 (JP); MASHIKO, Toshiya, Tokyo 100-0013 (JP)
(74) Representative: Hoffmann Eitle
(86) International application number: PCT/JP2021/033806
(87) International publication number: WO 2022/059684

(57) **Abstract**

Provided is a vitamin D derivative demonstrating excellent central nervous system migration, or a pharmaceutically acceptable salt or a solvate thereof. Specifically provided is a vitamin D derivative represented by formula (1), or a pharmaceutically acceptable salt or a solvate thereof.

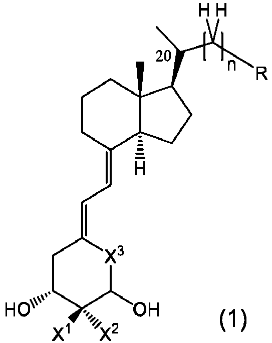

## Description

### [Technical field]

The present invention relates to a vitamin D derivative, a pharmaceutically acceptable salt and solvate thereof which are useful as a pharmaceutical, a therapeutic agent using them, and a pharmaceutical composition containing them. More specifically, the Vitamin D derivative of the present invention is a Vitamin D derivative having a cyclic tertiary amine in the side chain, and the present invention also relates to a remyelination promoter and a therapeutic agent clinically applicable as a remyelination promoter, containing such a vitamin D derivative and a pharmaceutically acceptable salt and solvate thereof, and the therapeutic agent is for multiple sclerosis, neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, Guillain-Barre syndrome, Fisher's syndrome, chronic inflammatory demyelinating polyneuropathy, Charcot-Marie-Tooth disease, Parkinson's disease, schizophrenia, bipolar disorder, major depressive disorder, autistic spectrum disorder, attention deficit hyperactivity disorder, obsessive-compulsive disorder, post-traumatic stress disorders, drug-dependent depression, autism, Alzheimer-type dementia, Down's syndrome, ischemic stroke, and the like.

### [Background Art]

Oligodendrocyte forms a myelin sheath in neuronal axon, and the main role thereof is to increase the conduction velocity by inducing saltatory conduction. In addition, the oligodendrocyte is involved in the neuronal metabolism.

Demyelination and dysmyelination is reported in some inflammatory demyelinating diseases, neurodegenerative diseases, and psychiatric disorders. Demyelination is a condition in which the myelin sheath is destroyed and disappears, and the disappearance of the myelin sheath causes various neurologic symptoms. Multiple sclerosis is a well-known neuroimmune disease that causes demyelination, and other known central nervous system inflammatory demyelinating diseases that cause demyelination includes neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, and the like. In addition, known peripheral nervous system demyelinating diseases include Guillain-Barre syndrome, Fisher's syndrome, chronic inflammatory demyelinating polyneuropathy, Charcot-Marie-Tooth disease, and the like. Furthermore, the myelin sheath is often damaged in ischemic stroke, and the myelin sheath disorder causes subsequent functional decline. It is also reported that in Alzheimer's disease of a neurodegenerative disease, myelin sheath disorder suppresses plasticity of the myelin sheath, leading to cognitive function decline.

In addition, dysmyelination is found in the brains in patients with various psychiatric disorders such as schizophrenia, bipolar disorder, major depressive disorder, autistic spectrum disorder (ASD), attention deficit hyperactivity disorder, obsessive-compulsive disorder, post-traumatic stress disorders (PTSD), drug-dependent depression, and the like, and is indicated to have an association with these diseases.

From the mentioned above, restoring the state of demyelination and dysmyelination to normal is important for the treatment of central nervous system disorder or peripheral nervous system disorder.

Recently, it has been reported that 1α,25-dihydroxyvitamin D₃ has a promoting action of induction of differentiation from oligodendrocyte progenitor cell and neural stem cell into oligodendrocyte (NPLs 1 and 2). Two pathways are known as actions of 1α,25-dihydroxyvitamin D₃ and a derivative thereof (NPL 3). One action is regulating gene expression (genomic action) by binding to a vitamin D receptor (VDR), which is one of the nuclear receptors. The other action is inducing signal transduction (non-genomic action) by binding to Protein disulfide isomerase A3 (PDIA3). It has not been clarified that promotion of oligodendrocyte differentiation by 1α,25-dihydroxyvitamin D₃, which is reported in NPLs 1 and 2, is due to genomic action or non-genomic action. On the other hand, the main action of 1α,25-dihydroxyvitamin D₃ and a derivative thereof is calcium-phosphorus metabolism. Generally, vitamin D derivative having a strong genomic action, which is expressed in a transcription-promoting activity value, has a strong activity of calcium-metabolism, which increases the blood calcium concentration and causes hypercalcemia. Therefore, the dose is limited, and the expected pharmacological effects may not be exerted.

Furthermore, 1α,25-dihydroxyvitamin D₃ is reported to have very low central nervous system penetration (NPLs 4 and 5). According to these documents, a sufficient concentration of 1α,25-dihydroxyvitamin D₃ in brain requires a very high dose administration. However, a high dose administration of 1α,25-dihydroxyvitamin D₃ cause an increase in calcium concentration in the blood, and thus is difficult.

Therefore, there is desired a vitamin D derivative that has excellent central nervous system penetration to be able to exert an effect in the brain, furthermore a vitamin D derivative that dissociates the myelin regeneration promoting action from the blood calcium raising action, but such a derivative has not been reported so far.

### [Citation List]

### [Patent Literature]

### [Non-Patent Literature]

[NPL 1] A. G. de la Fuente et al., Journal of Cell Biology, 2015, 211 (5), 975-985
[NPL 2] H. A. Shirazi et al., Experimental and Molecular Pathology, 2015, 98 (2), 240-245
[NPL 3] M. A. Zmijewski et al., Experimental Dermatology, 2020, 29, 876-884
[NPL 4] M. R. Durk et al., The Journal of Neuroscience, 2014, 34(21), 7091-7101
[NPL 5] E. C. Y Chow et al., The American Journal of Physiology: Endocrinology and Metabolism, 2013, 304(9), E977-989

### [Summary of Invention]

### [Technical Problem]

An object of the present invention is to provide a vitamin D derivative or a pharmaceutically acceptable salt or solvate thereof which have excellent central nervous system penetration.

Another object of the present invention is to provide a vitamin D derivative or a pharmaceutically acceptable salt or solvate thereof, which promotes the differentiation of an oligodendrocyte progenitor cell or neural stem cell to an oligodendrocyte, and thus promotes the regeneration of myelin sheath.

Another object of the present invention is to provide a therapeutic agent, containing the vitamin D derivative or pharmaceutically acceptable salt or solvate thereof as an active ingredient, for one or more diseases selected from the group consisting of multiple sclerosis, neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, Guillain-Barre syndrome, Fisher's syndrome, chronic inflammatory demyelinating polyneuropathy, Charcot-Marie-Tooth disease, Parkinson's disease, schizophrenia, bipolar disorder, major depressive disorder, autistic spectrum disorder, attention deficit hyperactivity disorder, obsessive-compulsive disorder, post-traumatic stress disorders, drug-dependent depression, autism, Alzheimer-type dementia, Down's syndrome, and ischemic stroke.

Furthermore, another object of the present invention is to provide a pharmaceutical composition comprising the vitamin D derivative or pharmaceutically acceptable salt or solvate thereof.

### [Solution to Problem]

As a result of diligent research for the above purpose, the present inventors have reached the following inventions.

That is, the present invention is a vitamin D derivative represented by the following formula (1), or a pharmaceutically acceptable salt or solvate thereof. 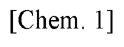 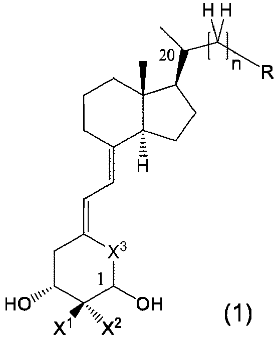 [In the formula, R represents any structure of Ra, Rb, Rc, Rd, or Re in the following formulas.

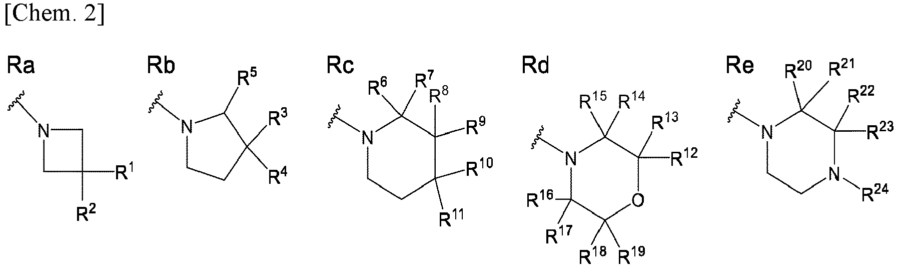

R¹, R³, R⁸ and R¹⁰ each independently represent a C₁ to C₆ alkyl group optionally substituted with 1 to 3 halogen atoms, a C₃ to C₆ cycloalkyl group, a C₁ to C₆ alkoxy group optionally substituted with 1 to 3 halogen atoms, a halogen atom, or a hydrogen atom.

R², R⁴, R⁹ and R¹¹ each independently represent a hydrogen atom, a hydroxy group, or a C₁ to C₆ alkyl group optionally substituted with 1 to 3 halogen atoms. (Wherein, when R¹, R³, R⁸ and R¹⁰ each independently represent a C₁ to C₆ alkoxy group optionally substituted with 1 to 3 halogen atoms, or a halogen atom, each of R², R⁴, R⁹ and R¹¹, substituting the same carbon atom together with R¹, R³, R⁸, or R¹⁰, is not a hydroxy group.)

R⁶, R⁷, R¹², R¹³ , R¹⁴ , R¹⁵, R¹⁶, R¹⁷, R¹⁸, R¹⁹, R²⁰, R²¹, R²², and R²³ each independently represent a hydrogen atom, a C₁ to C₆ alkyl group optionally substituted with 1 to 3 halogen atoms, or a C₃ to C₆ cycloalkyl group.

Each pair of R¹ and R², R³ and R⁴, R⁶ and R⁷, R⁸ and R⁹, R¹⁰ and R¹¹, R¹² and R¹³, R¹⁴ and R¹⁵, R¹⁶ and R¹⁷, R¹⁸ and R¹⁹, R²⁰ and R²¹, and R²² and R²³ can be bonded with each other to form a 3- to 5-membered ring structure.

R⁵ represents a hydrogen atom, a C₁ to C₆ alkyl group optionally substituted with a OR⁵⁰¹ or a C₃ to C₆ cycloalkyl group optionally substituted with a -OR⁵⁰¹, and R⁵⁰¹ represents a hydrogen atom or a C₁ to C₆ alkyl group.

R²⁴ represents a hydrogen atom, a C₁ to C₃ alkyl group or a C₁ to C₃ alkylsulfonyl group.

The stereochemistry at C-2 in the pyrrolidine ring (Rb) represents either (R) configuration or (S) configuration.

X¹ and X² each independently represent a hydrogen atom or a C₁ to C₃ alkyl group, or X¹ and X² together form a methylidene group, or -(CH₂)ₘ- (wherein m is an integer of 2 to 5).

X³ represents a CH₂ group, or a C=CH₂ group (wherein, when X¹ and X² together form a methylidene group, X³ is not a C=CH₂ group).

n represents an integer of 1 to 3.

The stereochemistry of the hydroxyl group at C-1 represents either (R) configuration or (S) configuration.

The stereochemistry of the methyl group at C-20 represents either (R) configuration or (S) configuration.]

Further, the present invention is a pharmaceutical composition containing a vitamin D derivative represented by the above formula (1), a pharmaceutically acceptable salt or solvate thereof, and a pharmaceutically acceptable carrier or base.

Furthermore, the present invention is a remyelination promoter and a therapeutic agent clinically applicable as a remyelination promoter, containing a vitamin D derivative represented by the above formula (1) or a pharmaceutically acceptable salt or solvate thereof, and the therapeutic agent is for one or more diseases selected from multiple sclerosis, neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, Guillain-Barre syndrome, Fisher's syndrome, chronic inflammatory demyelinating polyneuropathy, Charcot-Marie-Tooth disease, Parkinson's disease, schizophrenia, bipolar disorder, major depressive disorder, autistic spectrum disorder, attention deficit hyperactivity disorder, obsessive-compulsive disorder, post-traumatic stress disorders, drug-dependent depression, autism, Alzheimer-type dementia, Down's syndrome, and ischemic stroke.

### [Advantageous Effects of Invention]

According to the present invention, there provided is a novel vitamin D derivative, or a pharmaceutically acceptable salt or solvate thereof which is effective for the treatment of various central nervous system diseases, such as multiple sclerosis, neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, Guillain-Barre syndrome, Fisher's syndrome, chronic inflammatory demyelinating polyneuropathy, Charcot-Marie-Tooth disease, Parkinson's disease, schizophrenia, bipolar disorder, major depressive disorder, autistic spectrum disorder, attention deficit hyperactivity disorder, obsessive-compulsive disorder, post-traumatic stress disorders, drug-dependent depression, autism, Alzheimer-type dementia, and ischemic stroke.

### [Description of Embodiments]

Terms used alone or in combination in the present description will be explained below. Unless otherwise stated, the explanation of each substituent shall be common to each site. When a variable exists in each of any components, the variable is defined independently from other components. In addition, combinations of substituents and variables are permissible only if such combinations result in chemically stable compounds. When the substituent itself is substituted with two or more groups, these many groups can exist on the same or different carbon atom as long as a stable structure is formed.

In the present invention, "halogen atom" means a fluorine atom, a chlorine atom, a bromine atom, and an iodine atom.

In the present invention, "C₁ to C₆ alkyl group" means a monovalent saturated linear or branched aliphatic hydrocarbon group having 1 to 6 carbon atoms, and includes methyl group, ethyl group, n-propyl group, n-butyl group, n-pentyl group, n-hexyl group, isopropyl group, isobutyl group, s-butyl group, t-butyl group, isopentyl group, 2-methylbutyl group, neopentyl group, 1-ethylpropyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, 1,3-dimethylbutyl group, 2,3-dimethylbutyl group, 1-ethylbutyl group, 2-ethylbutyl group, t-pentyl group, and isohexyl group.

In the present invention, "methylidene group" means a =CH₂ group.

In the present invention, "C₃ to C₆ cycloalkyl group" means a cycloalkyl group having 3 to 6 carbon atoms. Examples of those cyclic alkyl groups include, though not limited to these, cyclopropyl group, cyclobutyl group, cyclopentyl group, and cyclohexyl group.

In the present invention, "C₁ to C₆ alkoxy group" means a group composed of an alkyl group having 1 to 6 carbon atoms among the above "C₁ to C₆ alkyl group" and an oxy group. Examples of these include methoxy group, ethoxy group, n-propyloxy group, isopropyloxy group, n-butoxy group, s-butoxy group, 2-methylpropoxy group, n-pentyloxy group, isopentyloxy group, 2-methylbutoxy group, 1-ethylpropoxy group, 2,2-dimethylpropoxy group, n-hexyloxy group, 4-methylpentoxy group, 3-methylpentoxy group, 2-methylpentoxy group, 3,3-dimethylbutoxy group, 2,2-dimethylbutoxy group, 1,1-dimethylbutoxy group, and t-butoxy group.

In the present invention, "C₁ to C₆ alkylsulfonyl group" means a group composed of the above "C₁ to C₆ alkyl group" and a sulfonyl group. Examples of these include methylsulfonyl group, ethylsulfonyl group, and isopropylsulfonyl group.

In the above definition, for example, "C" in such as "C₁" represents a carbon atom, and the numeral following "C" represents the number of carbon atoms. For example, "C₁ to C₆" represents a range from 1 to 6 carbon atoms. Of course, in the present invention, if the numeral for carbon atoms is changed, it means the same group having the changed number of carbon atoms. For example, "C₁ to C₃ alkyl group" means those having 1 to 3 carbon atoms among the alkyl groups defined by "C₁ to C₆ alkyl group". The rule of the numeral for carbon atoms is the same in other groups.

In the present invention, "C₁ to C₆ alkyl group optionally substituted with 1 to 3 halogen atoms" means a C₁ to C₆ alkyl group which may have 1 to 3 halogen atoms at the substitutable positions. When a C₁ to C₆ alkyl group is substituted with a plurality of halogen atoms, the C₁ to C₆ alkyl group may be substituted with halogen atoms of the same kind or different kind. "C₁ to C₆ alkoxy group optionally substituted with 1 to 3 halogen atoms" and the like also obey the same rule.

In the present invention, "vitamin D derivative" means a compound having a secosteroid structure (that is, 4-(2-cyclohexylideneethylidene)octahydro-1H-indene).

In the above formula (1), X¹ and X² each independently represent a hydrogen atom or a C₁ to C₃ alkyl group, or X¹ and X² together form a methylidene group, or - (CH)₂)ₘ- (wherein m is an integer of 2 to 5.) Among them, it is preferable that X¹ and X² each independently represent a hydrogen atom or a methyl group, or X¹ and X² together form a methylidene group.

In the above formula (1), X³ represents a CH₂ or C=CH₂ group (wherein, when X¹ and X² together form a methylidene group, X³ is not a C=CH₂ group.)

Preferred examples of combinations of X¹, X², and X³ are (i) when X¹ represents a hydrogen atom or a methyl group, X² represents a hydrogen atom, and X³ represents a C=CH₂ group; (ii) X¹ and X² represent a hydrogen atom, and X³ represents a CH₂ group; and (iii) X¹ and X² together form a methylidene group, and X³ represents a CH₂ group.

In the above formula (1), n represents an integer of 1 to 3.

The stereochemistry at C-20 in the above formula (1) may be either (R)-configured or (S)-configured.

The stereochemistry at C-1 in the above formula (1) may be either (R)-configured or (S)-configured.

In the above formula (1), R represents the above-mentioned structures of Ra to Re. Among these, pyrrolidine ring (Rb), piperidine ring (Rc), and morpholine ring (Rd) are particularly preferable structures.

R¹, R³, R⁸ and R¹⁰ each independently represent a C₁ to C₆ alkyl group optionally substituted with 1 to 3 halogen atoms, a C₃ to C₆ cycloalkyl group, a C₁ to C₆ alkoxy group optionally substituted with 1 to 3 halogen atoms, a halogen atom, or a hydrogen atom. Preferred groups for R¹, R³, R⁸ and R¹⁰ include a C₁ to C₆ alkyl group optionally substituted with 1 to 3 fluorine atoms, a C₃ to C₆ cycloalkyl group, a C₁ to C₆ alkoxy group optionally substituted with 1 to 3 fluorine atoms, a fluorine atom, or a hydrogen atom, and more preferred groups include methyl group, ethyl group, methoxy group, ethoxy group, difluoromethyl group, 1,1-difluoroethyl group, 2,2-difluoroethyl group, difluoromethoxy group, 2,2-difluoroethoxy group, 3,3-difluoropropyl group and 2,2-difluoropropyl group.

R², R⁴, R⁹ and R¹¹ each independently represent a hydrogen atom, a hydroxy group, or a C₁ to C₃ alkyl group optionally substituted with 1 to 3 halogen atoms. Among these, a hydrogen atom, a hydroxy group, or a C₁ to C₃ alkyl group optionally substituted with 1 to 3 fluorine atoms is preferable, and a hydrogen atom or a hydroxy group is more preferable. When R¹, R³, R⁸ and R¹⁰ each independently represent a C₁ to C₆ alkoxy group optionally substituted with 1 to 3 halogen atoms, or a halogen atom, each of R², R⁴, R⁹ and R¹¹, substituting the same carbon atom together with R¹, R³, R⁸, or R¹⁰, is preferably not a hydroxy group but a hydrogen atom in this case.

R⁶, R⁷, R¹², R¹³, R¹⁴, R¹⁵, R¹⁶, R¹⁷, R¹⁸ R¹⁹, R²⁰, R²¹, R²², and R²³, each independently represent a hydrogen atom, a C₁ to C₆ alkyl group optionally substituted with 1 to 3 halogen atoms, or a C₃ to C₆ cycloalkyl group. Among these, a hydrogen atom, a C₁ to C₆ alkyl group optionally substituted with 1 to 3 fluorine atoms, or a C₃ to C₆ cycloalkyl group is preferable, and more preferable groups include hydrogen atom, methyl group, ethyl group, and difluoromethyl group.

Also, each pair of R¹ and R², R³ and R⁴, R⁶ and R⁷, R⁸ and R⁹, R¹⁰ and R¹¹, R¹² and R¹³, R¹⁴ and R¹⁵, R¹⁶ and R¹⁷, R¹⁸ and R¹⁹, R²⁰ and R²¹, and R²² and R²³ can be bonded with each other to form a 3- to 5-membered ring structure. Here, the 3- to 5-membered ring structure is a hydrocarbon ring, and can form a cyclopropyl ring, a cyclobutyl ring, and a cyclopentyl ring together with a carbon atom which is substituted with R¹ and R², R³ and R⁴, R⁶ and R⁷, R⁸ and R⁹, R¹⁰ and R¹¹, R¹² and R¹³, R¹⁴ and R¹⁵, R¹⁶ and R¹⁷, R¹⁸ and R¹⁹, R²⁰ and R²¹, and R²² and R²³.

R⁵ represents a hydrogen atom, a C₁ to C₆ alkyl group optionally substituted with a -OR⁵⁰¹ or a C₁ to C₆ alkyl group optionally substituted with a -OR⁵⁰¹. R⁵⁰¹ represents a hydrogen atom or a C₁ to C₆ alkyl group. Among these, a hydrogen atom or a - C(CH₃)₂-OR⁵⁰¹ is preferable.

R²⁴ represents a hydrogen atom, a C₁ to C₃ alkyl group, or a C₁ to C₃ alkylsulfonyl group, and a methylsulfonyl group is particularly preferred.

Further, among preferred examples of a vitamin D derivative represented by formula (1) or a pharmaceutically acceptable salt or solvate thereof of the present invention, more specific examples include a vitamin D derivative represented by the following formulas (1A), (1B) and (1B-4) or a pharmaceutically acceptable salt or solvate thereof.

A vitamin D derivative represented by formula (1A) or a pharmaceutically acceptable salt or solvate thereof 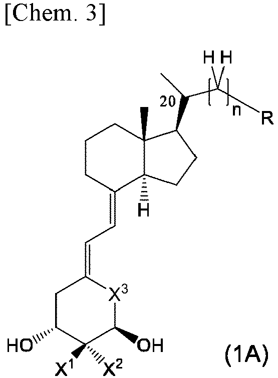 [In the formula, R represents any structure of Ra, Rb, Rc, Rd, or Re in the following formulas.

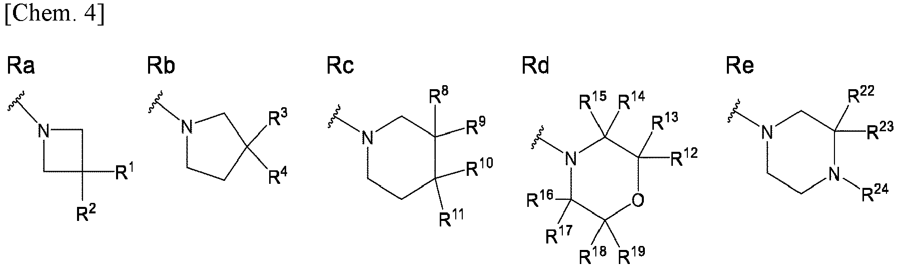

R¹, R³, R⁸ and R¹⁰ each independently represent a C₁ to C₆ alkyl group optionally substituted with 1 to 3 halogen atoms, a C₃ to C₆ cycloalkyl group, a C₁ to C₆ alkoxy group optionally substituted with 1 to 3 halogen atoms, a halogen atom, or a hydrogen atom.

R², R⁴, R⁹ and R¹¹ each independently represent a hydrogen atom, a hydroxy group, or a C₁ to C₆ alkyl group optionally substituted with 1 to 3 halogen atoms. (Wherein, when R¹, R³, R⁸ and R¹⁰ each independently represent a C₁ to C₆ alkoxy group optionally substituted with 1 to 3 halogen atoms, or a halogen atom, each of R², R⁴, R⁹ and R¹¹, substituting the same carbon atom together with R¹, R³, R⁸, or R¹⁰, is not a hydroxy group.)

R¹², R¹³, R¹⁴, R¹⁵, R¹⁶, R¹⁷, R¹⁸, R¹⁹, R²², and R²³ each independently represent a hydrogen atom, a C₁ to C₆ alkyl group optionally substituted with 1 to 3 halogen atoms, or a C₃ to C₆ cycloalkyl group.

Each pair of R¹ and R², R³ and R⁴, R⁸ and R⁹, R¹⁰ and R¹¹, R¹² and R¹³, R¹⁴ and R¹⁵, R¹⁶ and R¹⁷, R¹⁸ and R¹⁹, and R²² and R²³ can be bonded with each other to form a 3-to 5-membered ring structure.

R²⁴ represents a hydrogen atom, a C₁ to C₃ alkyl group or a C₁ to C₃ alkylsulfonyl group.

X¹ and X² each independently represent a hydrogen atom or a C₁ to C₃ alkyl group, or X¹ and X² together form a methylidene group, or -(CH₂)ₘ- (wherein m is an integer of 2 to 5).

X³ represents a CH₂ group, or a C=CH₂ group (wherein, when X¹ and X² together form a methylidene group, X³ is not a C=CH₂ group).

n represents an integer of 1 to 3.

The stereochemistry of the methyl group at C-20 represents either (R) configuration or (S) configuration.]

Each element of R¹, R², R³, R⁴, R⁸, R⁹, R¹⁰, R¹¹, R¹², R¹³, R¹⁴, R¹⁵, R¹⁶, R¹⁷, R¹⁸, R¹⁹, R²², R²³, R²⁴, X¹, X², X³ and the like in a vitamin D derivative represented by formulas (1A) or a pharmaceutically acceptable salt or solvate thereof can be applied directly from those described in a vitamin D derivative represented by formula (1) or a pharmaceutically acceptable salt or solvate thereof. That is, the preferred group in a vitamin D derivative represented by formula (1) is also preferable in a vitamin D derivative represented by formulas (1A), and a combination of each preferable constituent elements described in a vitamin D derivative represented by formula (1) is also preferable in a vitamin D derivative represented by formulas (1A).

A vitamin D derivative represented by formula (1B) or a pharmaceutically acceptable salt or solvate thereof 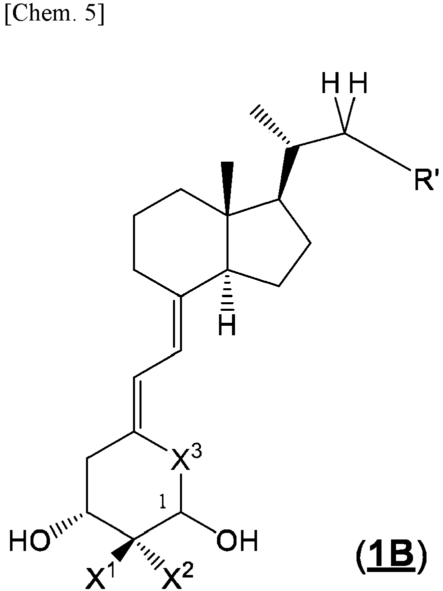 [In the formula, R' represents any structure of Rb', Rc', or Re' in the following formulas.

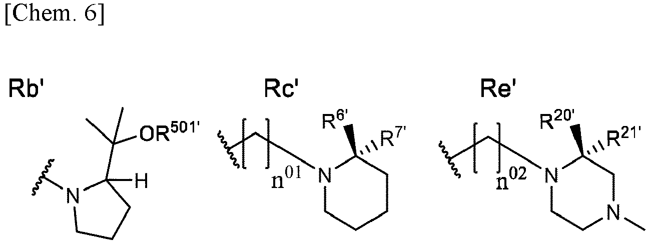

R^{6'}, R^{7'}, R^{20'}, and R^{21'} each independently represent a C₁ to C₆ alkyl group optionally substituted with 1 to 3 halogen atoms, a C₃ to C₆ cycloalkyl group or a hydrogen atom (for each pair of R^{6'}, R^{7'}, R^{20'}, and R^{21'}, except when both are a hydrogen atom at the same time).

Each pair of R^{6'} and R^{7'}, and R^{20'} and R^{21'} can be bonded with each other to form a 3- to 5- membered ring structure.

R^{501'} represents a hydrogen atom or a C₁ to C₆ alkyl group.

n⁰¹ and n⁰² each independently represent an integer of 0 or 1.

The stereochemistry at C-2 in the pyrrolidine ring (Rb') represents either (R) configuration or (S) configuration.

X¹ and X² each independently represent a hydrogen atom or a C₁ to C₃ alkyl group, or X¹ and X² together form a methylidene group, or -(CH₂)ₘ- (wherein m is an integer of 2 to 5).

X³ represents a CH₂ group, or a C=CH₂ group (wherein, when X¹ and X² together form a methylidene group, X³ is not a C=CH₂ group.).

The stereochemistry of the hydroxyl group at C-1 represents either (R) configuration or (S) configuration.]

In the above formula (1B), preferred examples of combinations of X¹, X², and X³ are (i) when X¹and X² represent a hydrogen atom, and X³ represents a C=CH₂ group; (ii) X¹ and X² represent a hydrogen atom, and X³ represents a CH₂ group; and (iii) X¹ and X² together form a methylidene group, and X³ represents a CH₂ group.

Preferred groups of R^{6'}, R^{7'}, R^{20'}, and R^{21'} include methyl group, ethyl group, monofluoromethyl group, difluoromethyl group, 1,1-difluoroethyl group, 2,2-difluoroethyl group, and hydrogen atom.

Preferred examples of the 3- to 5-membered ring structure being formed by each pair of R^{6'} and R^{7'}, and R^{20'} and R^{21'} with each other to include cyclopropyl ring and cyclobutyl ring.

Preferred groups of R^{501'} include methyl group, ethyl group, and hydrogen atom.

A vitamin D derivative represented by formula (1B-4) or a pharmaceutically acceptable salt or solvate thereof 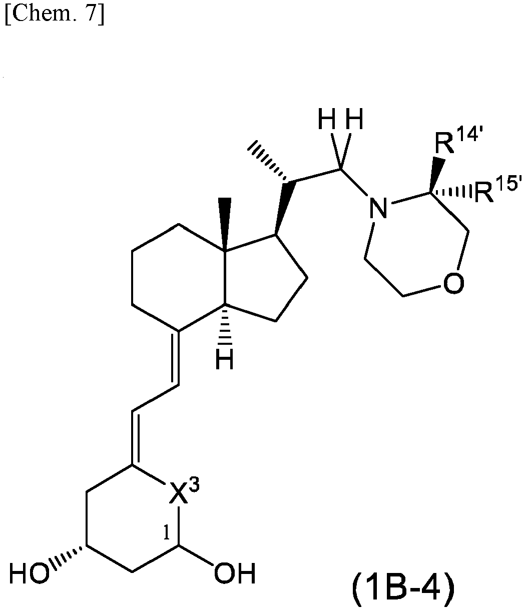 [In the formula (1B-4), R^{14'} and R^{15'} each independently represent a C₁ to C₆ alkyl group optionally substituted with 1 to 3 halogen atoms, a C₃ to C₆ cycloalkyl group, or a hydrogen atom. (Except when R^{14'} and R^{15'} are a hydrogen atom at the same time.)

R^{14'} and R^{15'} can be bonded with each other to form a 3- to 5-membered ring structure.

X³ represents a CH₂ group or C=CH₂ group.

The stereochemistry of the hydroxyl group at C-1 represents either (R) configuration or (S) configuration.]

In the above formula (1B-4), preferred groups of R^{14'} and R^{15'} include methyl group, ethyl group, monofluoromethyl group, difluoromethyl group, 1,1-difluoroethyl group, 2,2-difluoroethyl group, and hydrogen atom.

Preferred examples of the 3- to 5-membered ring structure being formed by each pair of R^{14'} and R^{15'} with each other to include cyclopropyl ring and cyclobutyl ring.

Further, among preferred examples of a vitamin D derivative represented by formula (1A) or a pharmaceutically acceptable salt or solvate thereof of the present invention, more specific examples include a vitamin D derivative represented by the following formulas (1A-1), (1A-2), and (1A-3) or a pharmaceutically acceptable salt or solvate thereof.

A vitamin D derivative represented by formula (1A-1) or a pharmaceutically acceptable salt or solvate thereof 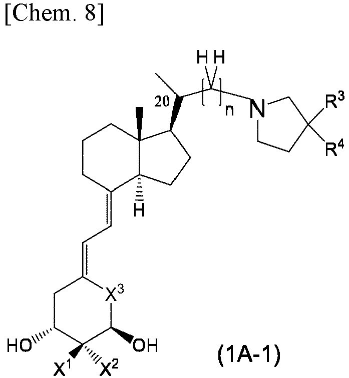 [In the formula, R³ represents a C₁ to C₆ alkyl group optionally substituted with 1 to 3 fluorine atoms, a C₃ to C₆ cycloalkyl group, a C₁ to C₆ alkoxy group optionally substituted with 1 to 3 fluorine atoms, a fluorine atom, or a hydrogen atom.

R⁴ represents a hydrogen atom, a hydroxy group, or a C₁ to C₃ alkyl group optionally substituted with 1 to 3 fluorine atoms. (Wherein, when R³ represents a C₁ to C₃ alkoxy group optionally substituted with 1 to 3 fluorine atoms, or a fluorine atom, R⁴ is not a hydroxy group.)

R³ and R⁴ can be bonded with each other to form a 3- to 5-membered ring structure.

X¹ and X² each independently represent a hydrogen atom or a methyl group, or X¹ and X² together form a methylidene group.

X³ represents a CH₂ group, or a C=CH₂ group (wherein, when X¹ and X² together form a methylidene group, X³ is not a C=CH₂ group.).

n represents an integer of 1 to 3.

The stereochemistry of the methyl group at C-20 represents either (R) configuration or (S) configuration.]

A vitamin D derivative represented by formula (1A-2) or a pharmaceutically acceptable salt or solvate thereof 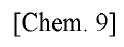 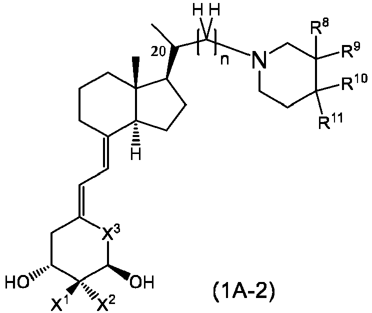 [In the formula, R⁸ and R¹⁰ each independently represent a C₁ to C₆ alkyl group optionally substituted with 1 to 3 fluorine atoms, a C₃ to C₆ cycloalkyl group, a C₁ to C₆ alkoxy group optionally substituted with 1 to 3 fluorine atoms, a fluorine atom, or a hydrogen atom.

R^{9,} and R¹¹ each independently represent a hydrogen atom, a hydroxy group, or a C₁ to C₃ alkyl group optionally substituted with 1 to 3 fluorine atoms. (Wherein, when R⁸ and R¹⁰ each independently represent a C₁ to C₃ alkoxy group optionally substituted with 1 to 3 fluorine atoms, or a fluorine atom, each of R⁹ and R¹¹, substituting the same carbon atom together with R⁸ or R¹⁰, is not a hydroxy group.)

Each pair of R⁸ and R⁹, and R¹⁰ and R¹¹ can be bonded with each other to form a 3- to 5-membered ring structure.

X¹ and X² each independently represent a hydrogen atom or a methyl group, or X¹ and X² together form a methylidene group.

X³ represents a CH₂ group, or a C=CH₂ group (wherein, when X¹ and X² together form a methylidene group, X³ is not a C=CH₂ group.)

n represents an integer of 1 to 3.

The stereochemistry of the methyl group at C-20 represents either (R) configuration or (S) configuration.]

A vitamin D derivative represented by formula (1A-3) or a pharmaceutically acceptable salt or solvate thereof 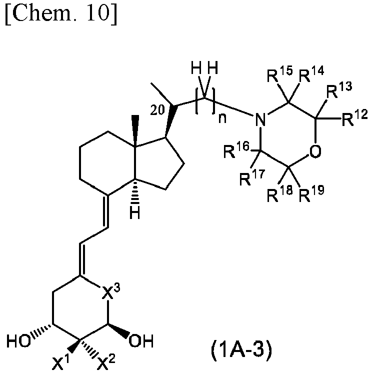 [In the formula, R¹², R¹³, R¹⁴, R¹⁵, R¹⁶, R¹⁷, R¹⁸ and R¹⁹ each independently represent a hydrogen atom, a C₁ to C₆ alkyl group optionally substituted with 1 to 3 fluorine atoms, or a C₃ to C₆ cycloalkyl group.

Each pair of R¹² and R¹³, R¹⁴ and R¹⁵, R¹⁶ and R¹⁷, and R¹⁸ and R¹⁹ can be bonded with each other to form a 3- to 5-membered ring structure.

X¹ and X² each independently represent a hydrogen atom or a methyl group, or

X¹ and X² together form a methylidene group.

X³ represents a CH₂ group or a C=CH₂ group (wherein, when X¹ and X² together form a methylidene group, X³ is not a C=CH₂ group.)

n represents an integer of 1 to 3.

The stereochemistry of the methyl group at C-20 represents either (R) configuration or (S) configuration.]

It is not always necessary to distinguish between the vitamin D derivative represented by formula (1A-3) and the vitamin D derivative represented by formula (1B-4). But when it is necessary to distinguish between the vitamin D derivative represented by formula (1A-3) and the vitamin D derivative represented by formula (1B-4), in the formula (1A-3) case where X¹ and X² represent a hydrogen atom, n = 1, R¹², R¹³, R¹⁸ and R¹⁹ represent hydrogen atoms, and either or both of R¹⁴ and R¹⁵, or R¹⁶ and R¹⁷ each independently represent a C₁ to C₆ alkyl group optionally substituted with 1 to 3 fluorine atoms, or a C₃ to C₆ cycloalkyl group are excluded.

Each element of R³, R⁴, R⁸, R⁹, R¹⁰, R¹¹, R¹², R¹³, R¹⁶, R¹⁷, R¹⁸, R¹⁹, X¹, X², X³ and the like in a vitamin D derivative represented by formulas (1A-1), (1A-2), and (1A-3) or a pharmaceutically acceptable salt or solvate thereof can be applied directly from those described in a vitamin D derivative represented by formula (1A) or a pharmaceutically acceptable salt or solvate thereof. That is, the preferred group in a vitamin D derivative represented by formula (1A) is also preferable in a vitamin D derivative represented by formulas (1A-1), (1A-2), and (1A-3) and a combination of each preferable constituent elements described in a vitamin D derivative represented by formula (1A) is also preferable in a vitamin D derivative represented by formulas (1A-1), (1A-2), and (1A-3).

Further, among preferred examples of a vitamin D derivative represented by formula (1B) or a pharmaceutically acceptable salt or solvate thereof of the present invention, more specific examples include a vitamin D derivative represented by the following formulas (1B-1), (1B-2), and (1B-3) or a pharmaceutically acceptable salt or solvate thereof.

A vitamin D derivative represented by formula (1B-1) or a pharmaceutically acceptable salt or solvate thereof. 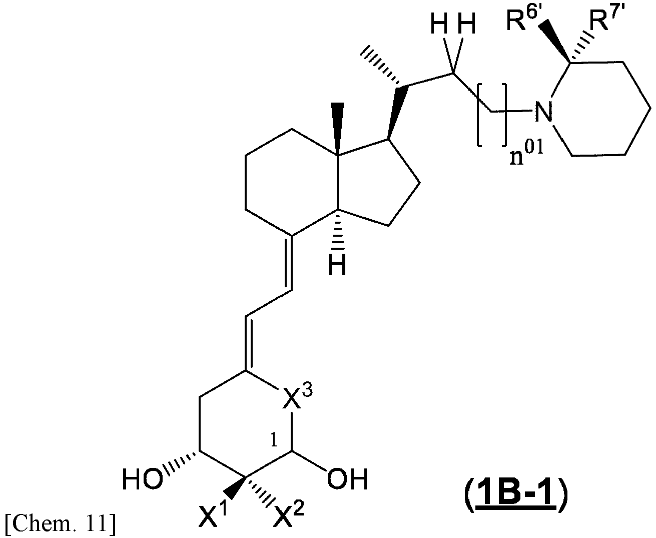 [In the formula (1B-1), the stereochemistry of the hydroxyl group at C-1 represents either (R) configuration or (S) configuration.

X¹ and X² each independently represent a hydrogen atom or a C₁ to C₃ alkyl group, or X¹ and X² together form a methylidene group or -(CH₂)ₘ- (wherein m is an integer of 2 to 5).

X³ represents a CH₂ group or C=CH₂ group (wherein, when X¹ and X² together form a methylidene group, X³ is not a C=CH₂ group.)

R^{6'} and R^{7'} each independently represent a C₁ to C₆ alkyl group optionally substituted with 1 to 3 halogen atoms, a C₃ to C₆ cycloalkyl group, or a hydrogen atom. (Except when R^{6'} and R^{7'} are a hydrogen atom at the same time.)

R^{6'} and R^{7'} can be bonded with each other to form a 3- to 5-membered ring structure.

n ⁰¹ represents an integer of 0 or 1.]

A vitamin D derivative represented by formula (1B-2) or a pharmaceutically acceptable salt or solvate thereof. 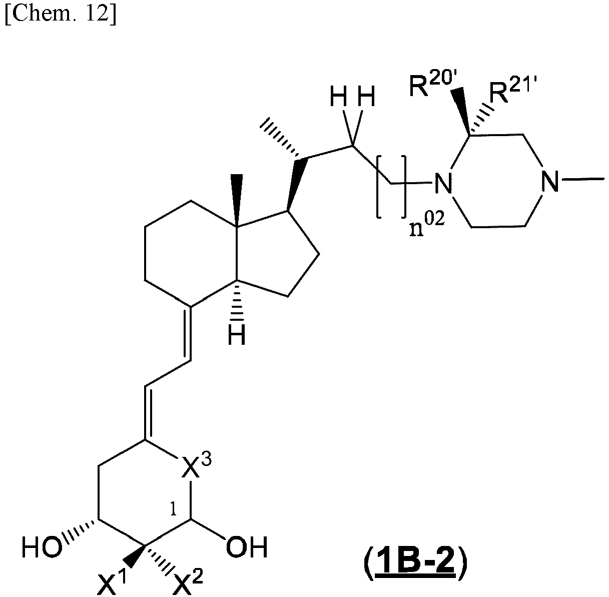 [In the formula (1B-2), the stereochemistry of the hydroxyl group at C-1 represents either (R) configuration or (S) configuration.

X¹ and X² each independently represent a hydrogen atom or C₁ to C₃ alkyl group, or X¹ and X² together form a methylidene group or -(CH₂)ₘ- (wherein m is an integer of 2 to 5).

X³ is a CH₂ group or C=CH₂ group (wherein, when X¹ and X² together form a methylidene group, X³ is not a C=CH₂ group.)

R^{20'} and R^{21'} each independently represent a C₁ to C₆ alkyl group optionally substituted with 1 to 3 halogen atoms, a C₃ to C₆ cycloalkyl group, or a hydrogen atom. (Except when R^{20'} and R^{21'} are a hydrogen atom at the same time.)

R^{20'} and R^{21'} can be bonded with each other to form a 3- to 5-membered ring structure.

n⁰² represents an integer of 0 or 1.]

A vitamin D derivative represented by formula (1B-3) or a pharmaceutically acceptable salt or solvate thereof. 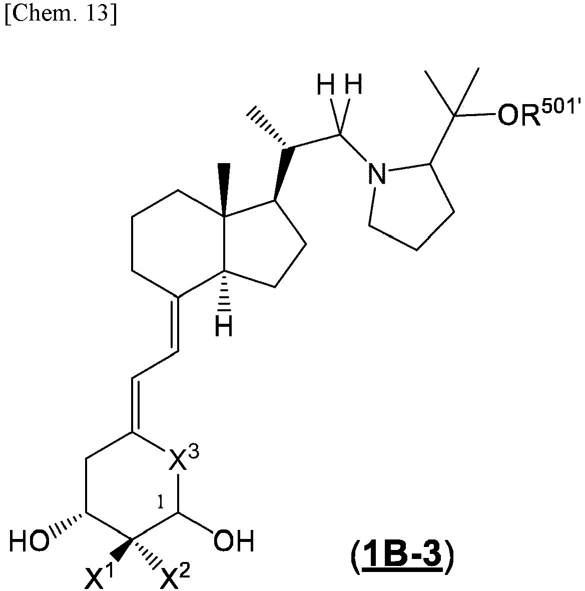 [In the formula (1B-3), the stereochemistry of the hydroxyl group at C-1 represents either (R) configuration or (S) configuration.

X¹ and X² each independently represent a hydrogen atom or C₁ to C₃ alkyl group, or X¹ and X² together form a methylidene group or -(CH₂)ₘ- (wherein m is an integer of 2 to 5.)

X³ represents a CH₂ group or C=CH₂ group (wherein, when X¹ and X² together form a methylidene group, X³ is not a C=CH₂ group.)

R^{501'} represents a hydrogen atom or C₁ to C₆ alkyl group.

The stereochemistry at C-2 in the pyrrolidine ring (Rb') represents either (R) configuration or (S) configuration.]

Each element of R^{6'}, R^{7'}, R^{20'}, R^{21'}, R^{501'}, n⁰¹, n⁰², X¹, X², X³ and the like in a vitamin D derivative represented by formulas (1B-1), (1B-2), and (1B-3) or a pharmaceutically acceptable salt or solvate thereof can be applied directly from those described in a vitamin D derivative represented by formula (1B) or a pharmaceutically acceptable salt or solvate thereof. That is, the preferred group in a vitamin D derivative represented by formula (1B) is also preferable in a vitamin D derivative represented by formulas (1B-1), (1B-2), and (1B-3), and a combination of each preferable constituent elements described in a vitamin D derivative represented by formula (1B) is also preferable in a vitamin D derivative represented by formulas (1B-1), (1B-2),and (1B-3).

Preferred specific examples of a vitamin D derivative represented by formula (1) of the present invention include the compounds shown in the following table.

**[Table 1-1]**

| Compound number | | Compound number | |
|---|---|---|---|
| A001 | 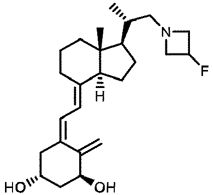 | A002 | 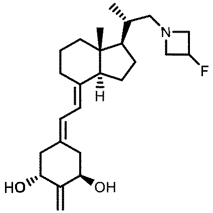 |
| A003 | 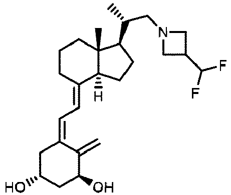 | A004 | 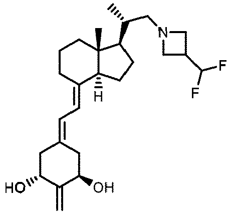 |
| A005 | 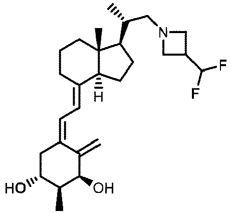 | A006 | 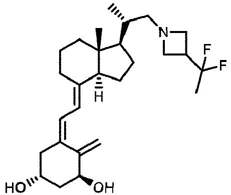 |
| A007 | 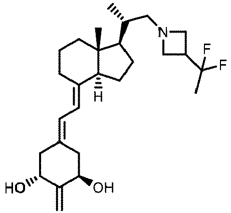 | A008 | 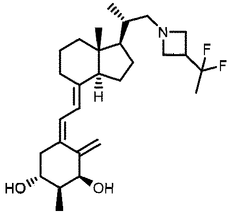 |
| A009 | 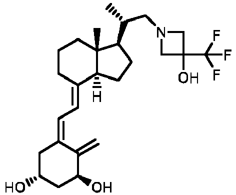 | A010 | 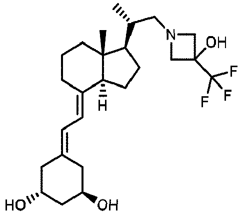 |

**[Table 1-2]**

| Compound number | | Compound number | |
|---|---|---|---|
| A011 | 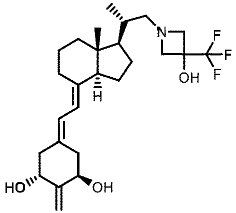 | A012 | 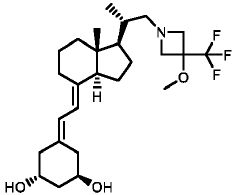 |
| A013 | 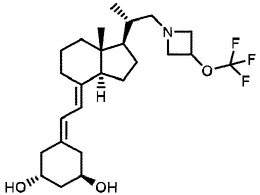 | A014 | 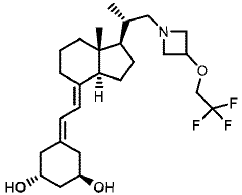 |
| A015 | 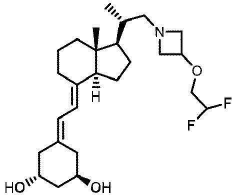 | A016 | 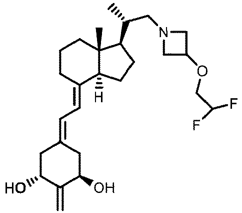 |
| A017 | 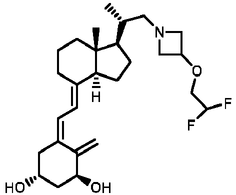 | A018 | 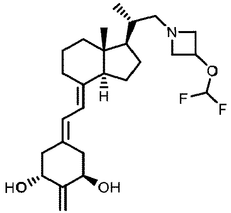 |
| A019 | 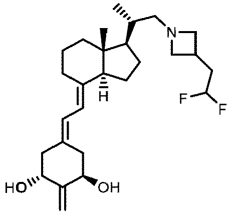 | A020 | 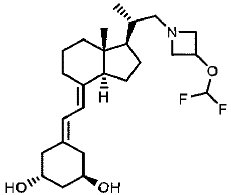 |

**[Table 1-3]**

| Compoun d number | | Compoun d number | |
|---|---|---|---|
| A021 | 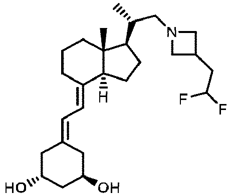 | A022 | 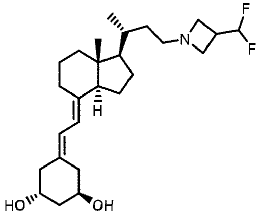 |
| A023 | 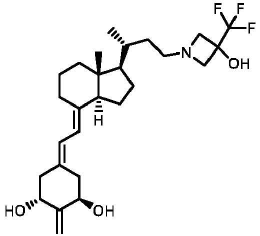 | A024 | 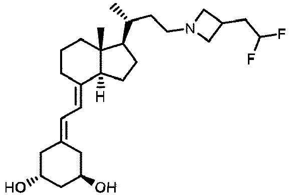 |
| A025 | 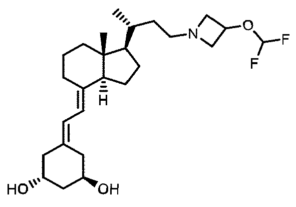 | A026 | 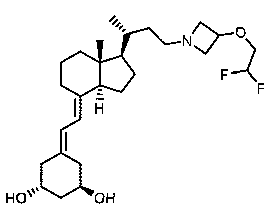 |
| A027 | 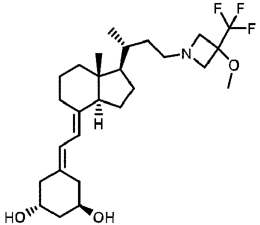 | A028 | 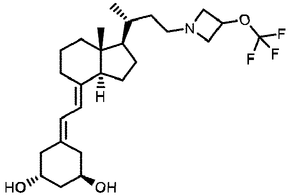 |
| A029 | 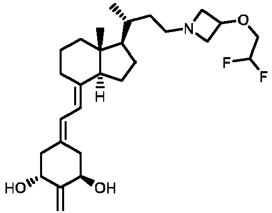 | A030 | 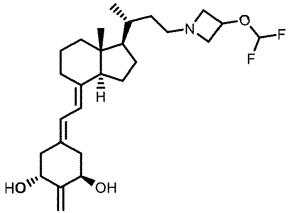 |

**[Table 1-4]**

| Compound number | | Compound number | |
|---|---|---|---|
| A031 | 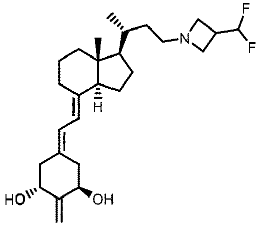 | A032 | 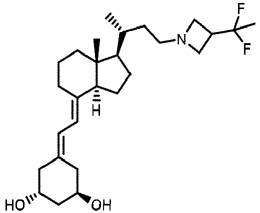 |
| A033 | 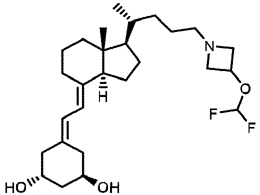 | A034 | 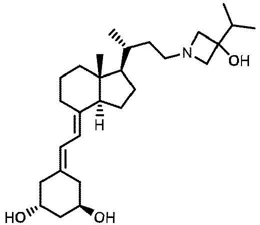 |

**[Table 1-5]**

| Compound number | | Compound number | |
|---|---|---|---|
| B001 | 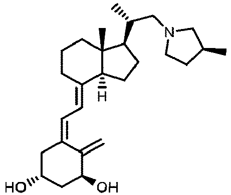 | B002 | 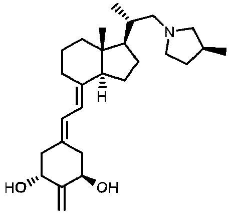 |
| B003 | 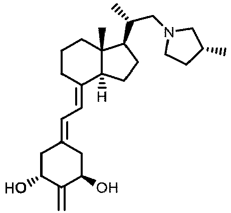 | B004 | 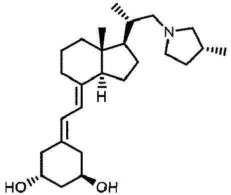 |
| B005 | 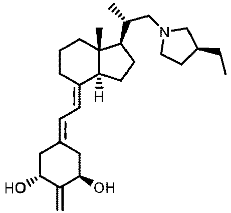 | B006 | 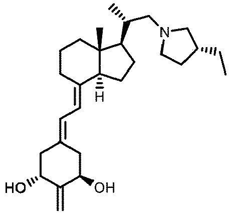 |
| B007 | 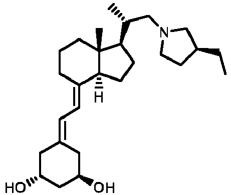 | B008 | 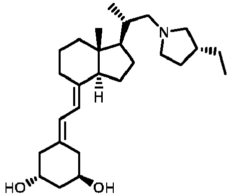 |
| B009 | 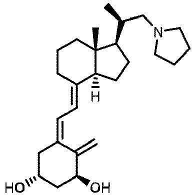 | B010 | 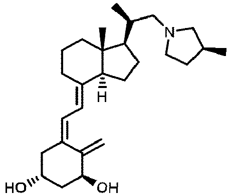 |

**[Table 1-6]**

| Compound number | | Compound number | |
|---|---|---|---|
| B011 | 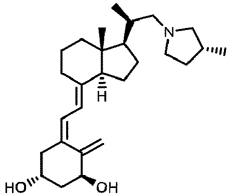 | B012 | 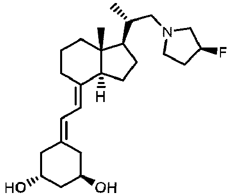 |
| B013 | 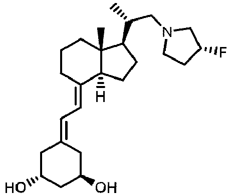 | B014 | 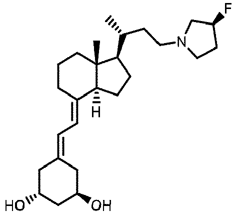 |
| B015 | 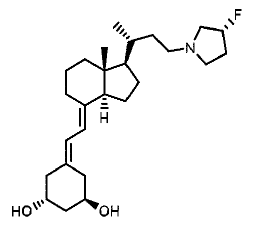 | B016 | 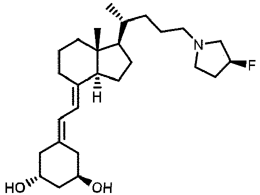 |
| B017 | 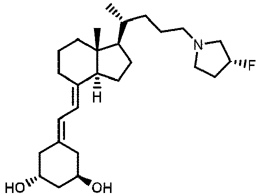 | B018 | 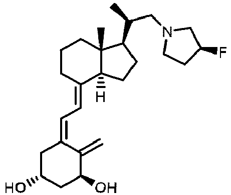 |
| B019 | 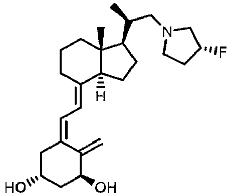 | B020 | 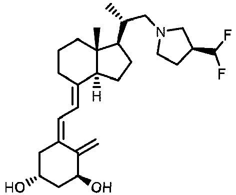 |

**[Table 1-7]**

| Compound number | | Compound number | |
|---|---|---|---|
| B021 | 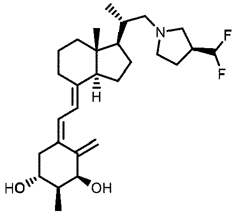 | B022 | 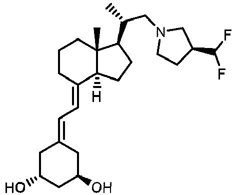 |
| B023 | 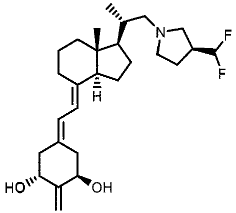 | B024 | 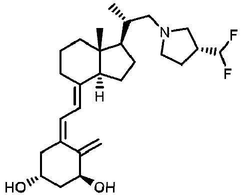 |
| B025 | 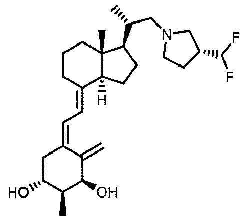 | B026 | 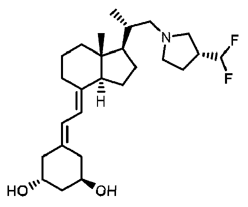 |
| B027 | 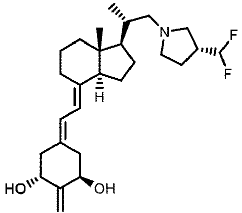 | B028 | 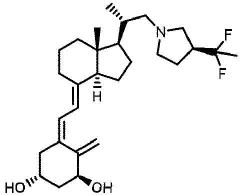 |
| B029 | 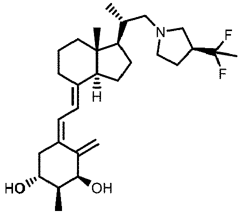 | B030 | 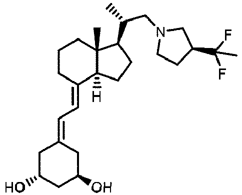 |

**[Table 1-8]**

| Compound number | | Compound number | |
|---|---|---|---|
| B031 | 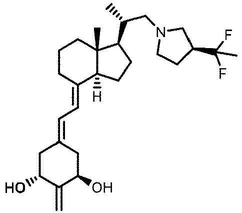 | B032 | 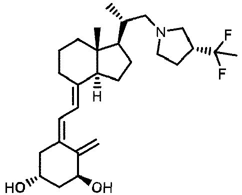 |
| B033 | 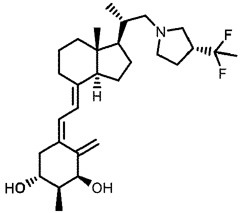 | B034 | 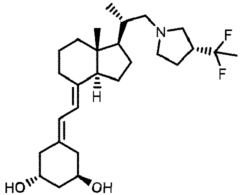 |
| B035 | 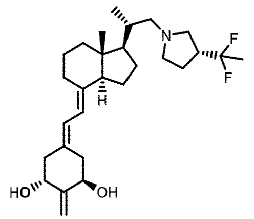 | B036 | 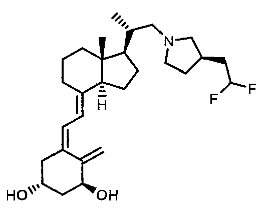 |
| B037 | 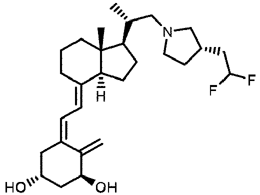 | B038 | 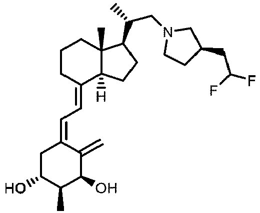 |
| B039 | 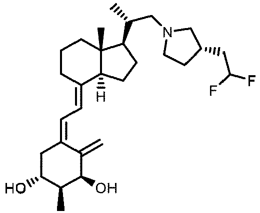 | B040 | 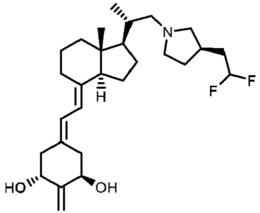 |

**[Table 1-9]**

| Compound number | | Compound number | |
|---|---|---|---|
| B041 | 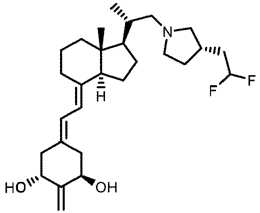 | B042 | 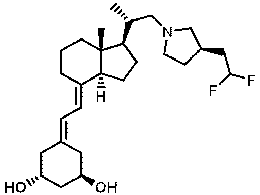 |
| B043 | 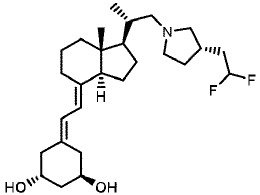 | B044 | 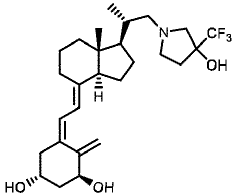 |
| B045 | 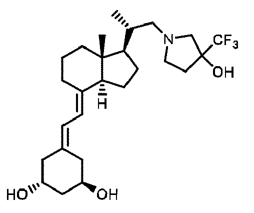 | B046 | 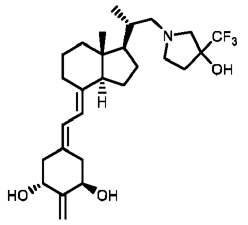 |
| B047 | 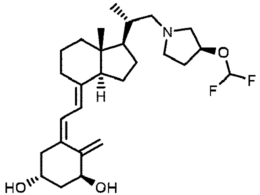 | B048 | 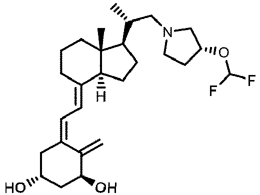 |
| B049 | 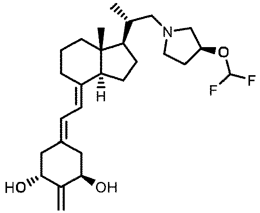 | B050 | 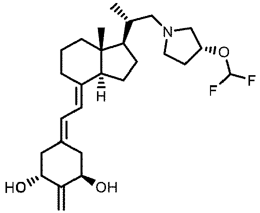 |

**[Table 1-10]**

| Compound number | | Compound number | |
|---|---|---|---|
| B051 | 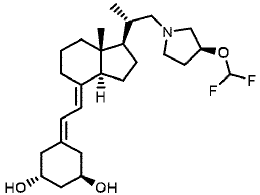 | B052 | 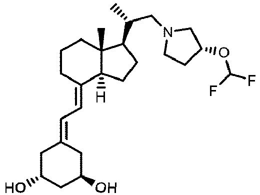 |
| B053 | 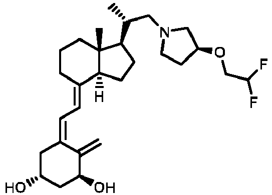 | B054 | 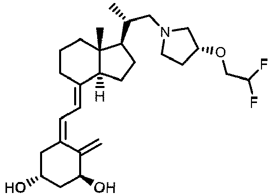 |
| B055 | 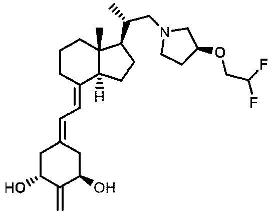 | B056 | 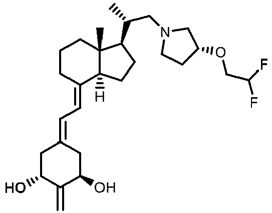 |
| B057 | 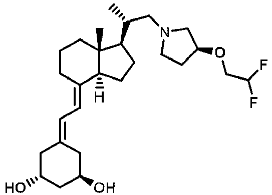 | B058 | 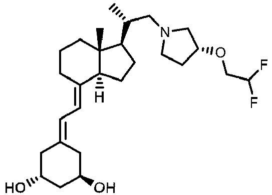 |
| B059 | 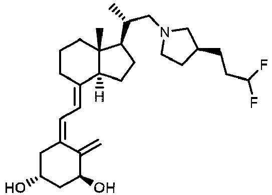 | B060 | 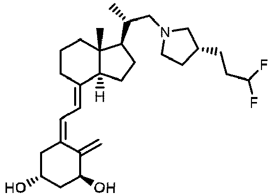 |

**[Table 1-11]**

| Compound number | | Compound number | |
|---|---|---|---|
| B061 | 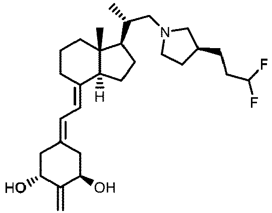 | B062 | 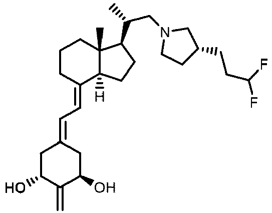 |
| B063 | 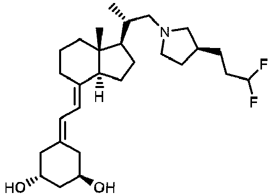 | B064 | 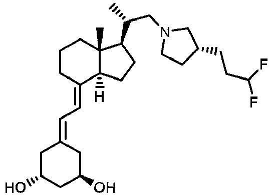 |
| B065 | 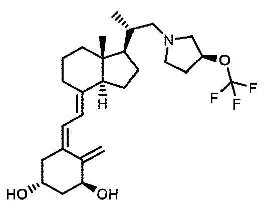 | B066 | 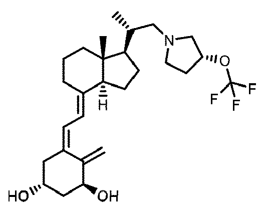 |
| B067 | 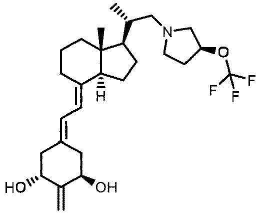 | B068 | 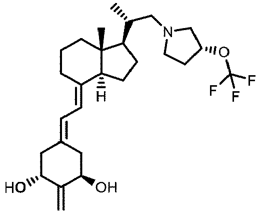 |
| B069 | 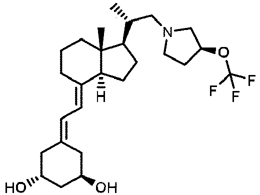 | B070 | 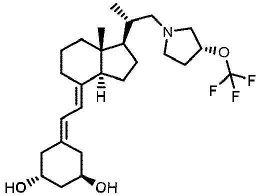 |

**[Table 1-12]**

| Compound number | | Compound number | |
|---|---|---|---|
| B071 | 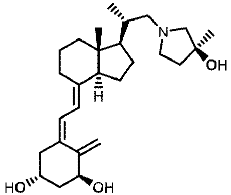 | B072 | 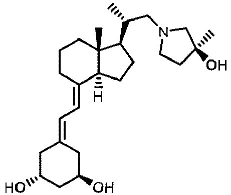 |
| B073 | 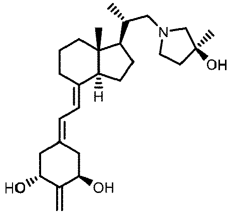 | B074 | 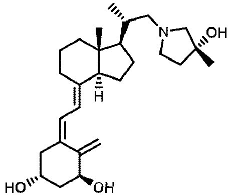 |
| B075 | 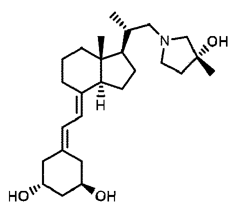 | B076 | 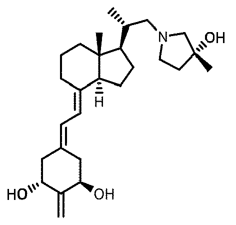 |
| B077 | 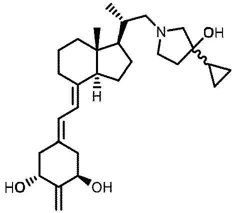 | B078 | 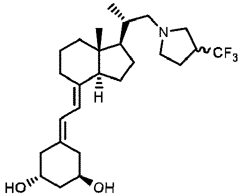 |
| B079 | 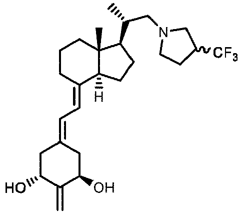 | B080 | 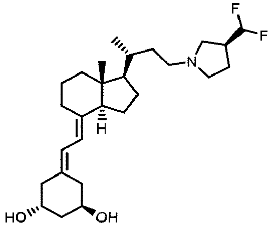 |

**[Table 1-13]**

| Compound number | | Compound number | |
|---|---|---|---|
| B081 | 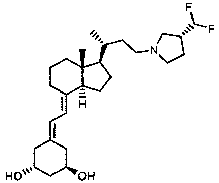 | B082 | 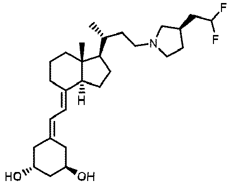 |
| BOSS | 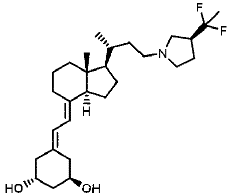 | B084 | 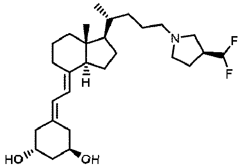 |
| B085 | 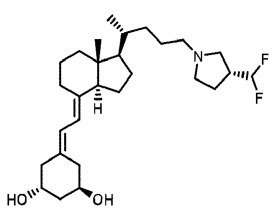 | B086 | 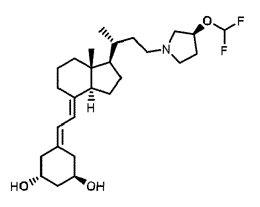 |
| B087 | 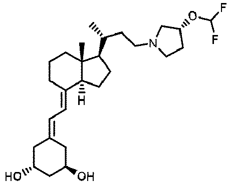 | B088 | 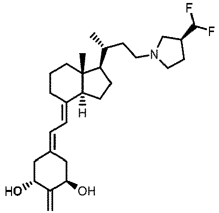 |
| B089 | 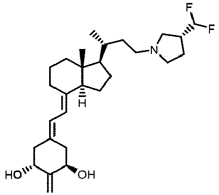 | B090 | 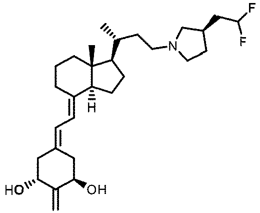 |

**[Table 1-14]**

| Compound number | | Compound number | |
|---|---|---|---|
| B091 | 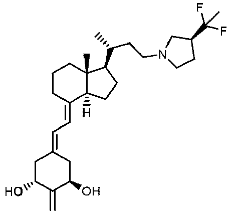 | B092 | 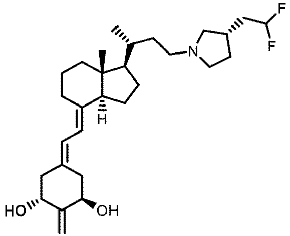 |
| B093 | 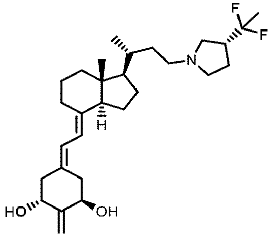 | B094 | 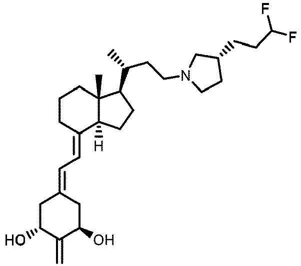 |
| B095 | 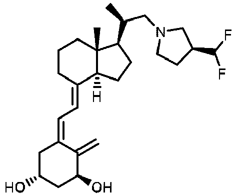 | B096 | 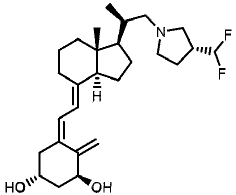 |
| B097 | 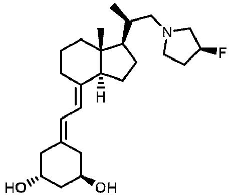 | B098 | 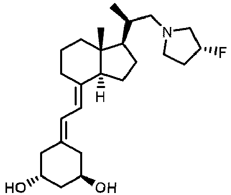 |
| B099 | 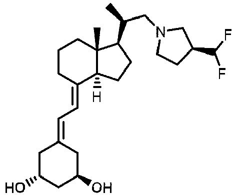 | B100 | 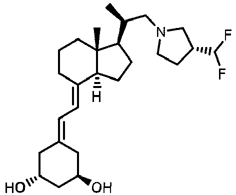 |

**[Table 1-15]**

| Compound number | | Compound number | |
|---|---|---|---|
| B101 | 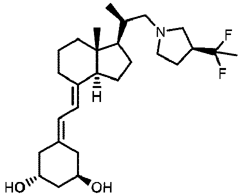 | B102 | 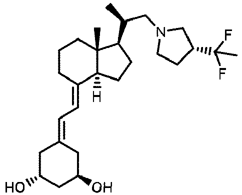 |
| B103 | 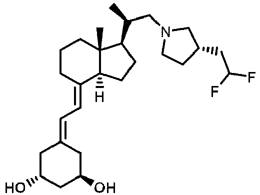 | | |

**[Table 1-16]**

| Compound number | Structure | Compound number | Structure |
|---|---|---|---|
| C001 | 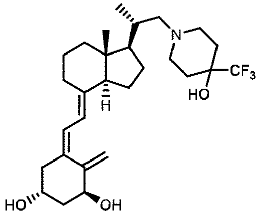 | C002 | 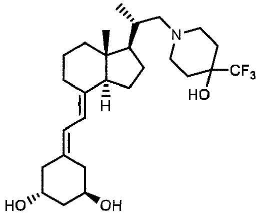 |
| C003 | 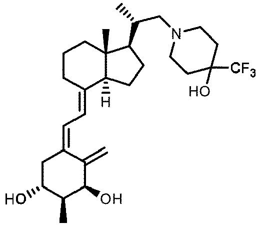 | C004 | 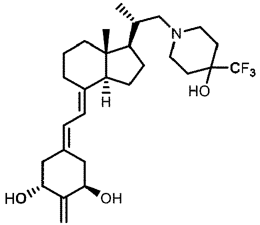 |
| C005 | 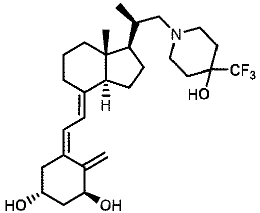 | C006 | 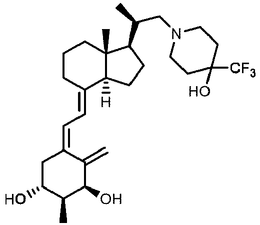 |
| C007 | 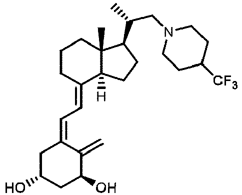 | C008 | 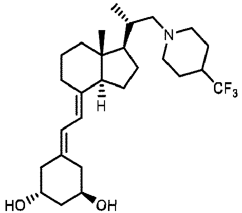 |
| C009 | 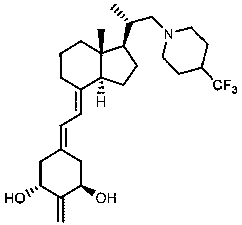 | C010 | 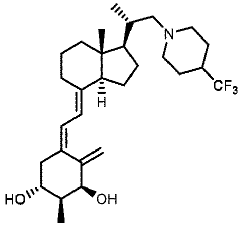 |

**[Table 1-17]**

| Compound number | Structure | Compound number | Structure |
|---|---|---|---|
| C011 | 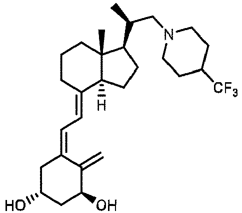 | C012 | 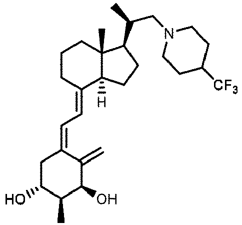 |
| C013 | 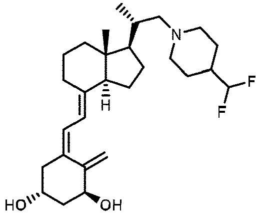 | C014 | 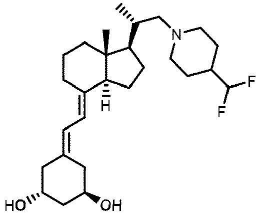 |
| C015 | 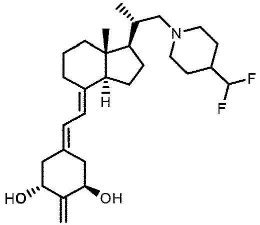 | C016 | 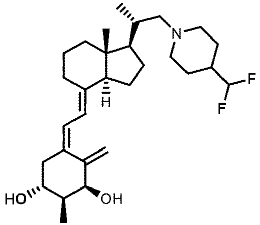 |
| C017 | 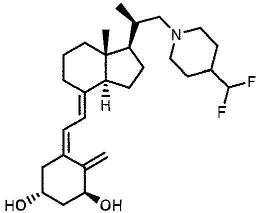 | C018 | 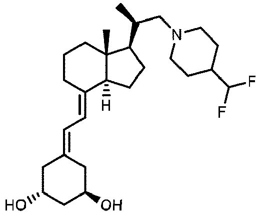 |
| C19 | 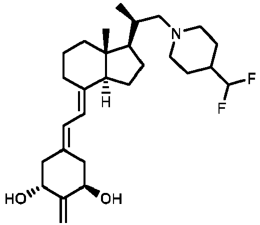 | C020 | 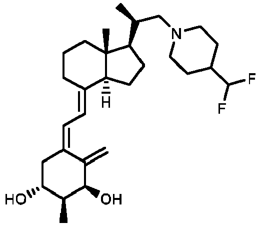 |

**[Table 1-18]**

| Compound number | Structure | Compound number | Structure |
|---|---|---|---|
| C021 | 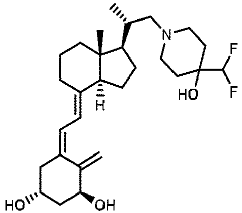 | C022 | 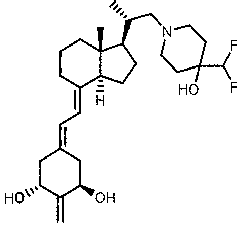 |
| C023 | 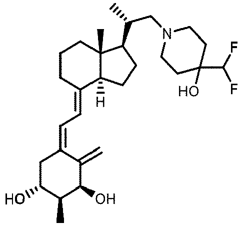 | C024 | 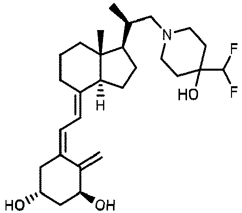 |
| C025 | 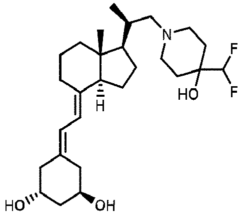 | C026 | 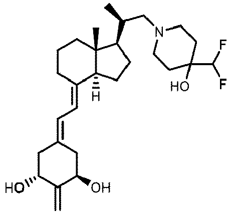 |
| C027 | 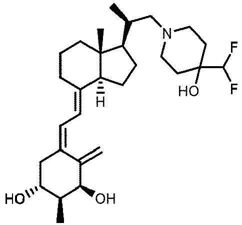 | C028 | 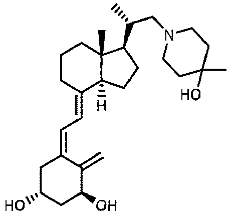 |
| C029 | 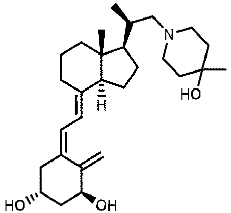 | C030 | 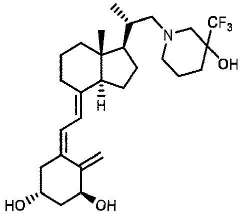 |

**[Table 1-19]**

| Compound number | Structure | Compound number | Structure |
|---|---|---|---|
| C031 | 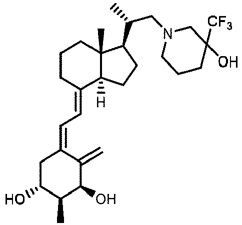 | C032 | 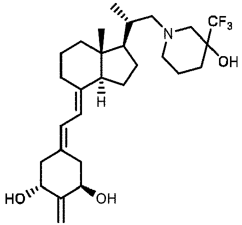 |
| C033 | 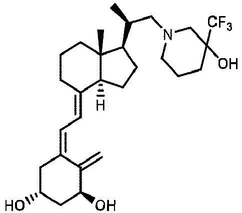 | C034 | 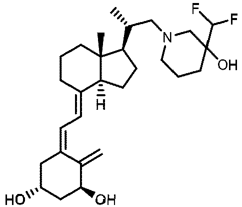 |
| C035 | 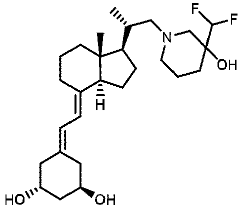 | C036 | 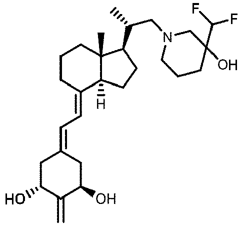 |
| C037 | 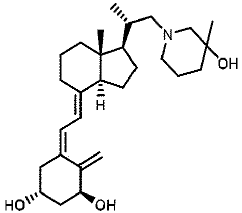 | C038 | 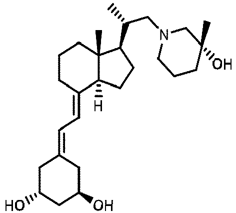 |
| C039 | 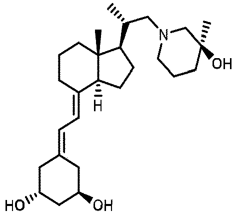 | C040 | 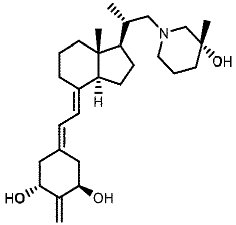 |

**[Table 1-20]**

| Compound number | Structure | Compound number | Structure |
|---|---|---|---|
| C041 | 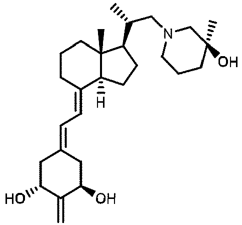 | C042 | 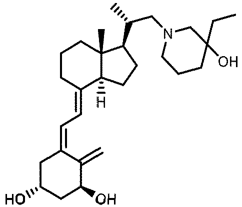 |
| C043 | 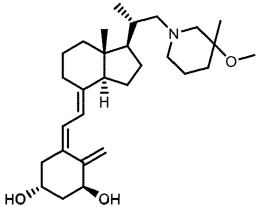 | C044 | 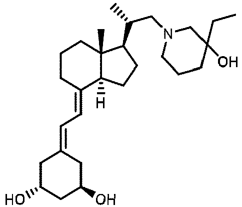 |
| C045 | 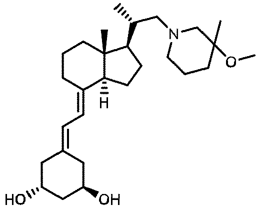 | C046 | 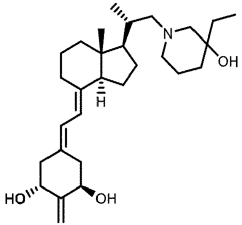 |
| C047 | 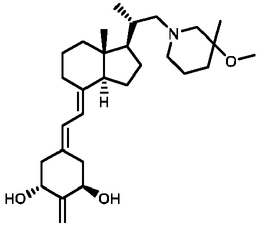 | C048 | 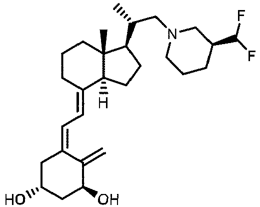 |
| C049 | 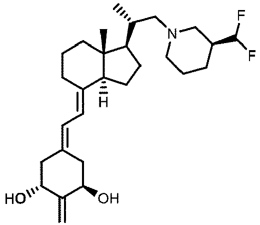 | C050 | 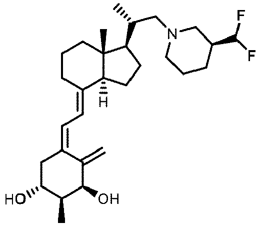 |

**[Table 1-21]**

| Compound number | Structure | Compound number | Structure |
|---|---|---|---|
| C051 | 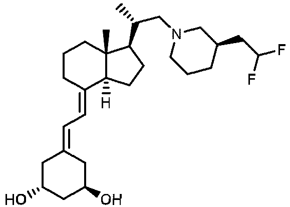 | C052 | 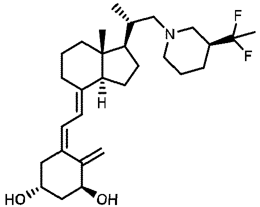 |
| C053 | 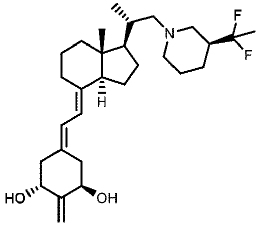 | C054 | 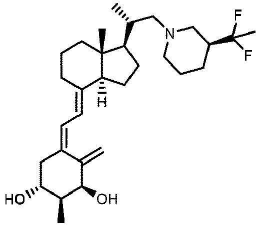 |
| C055 | 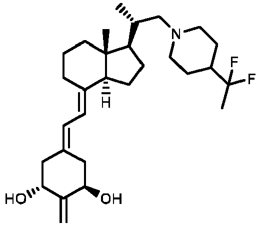 | C056 | 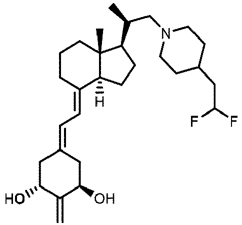 |
| C057 | 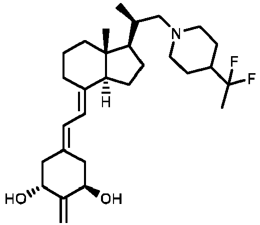 | C058 | 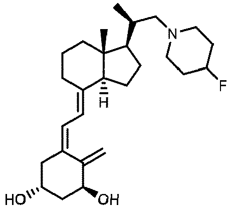 |
| C059 | 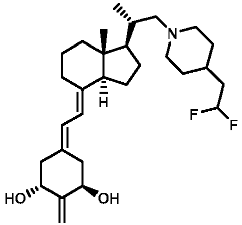 | C060 | 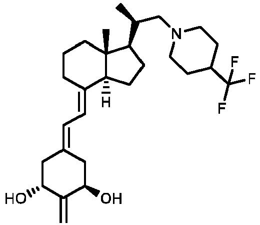 |

**[Table 1-22]**

| Compound number | | Compound number | |
|---|---|---|---|
| D001 | 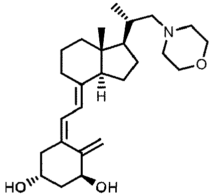 | D002 | 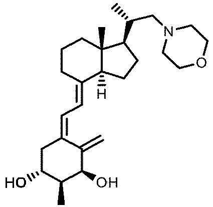 |
| D003 | 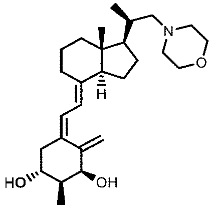 | D004 | 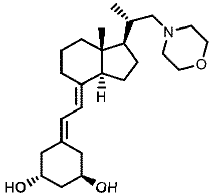 |
| D005 | 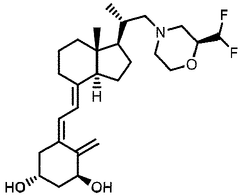 | D006 | 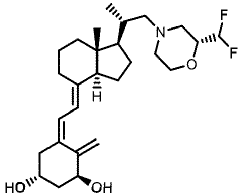 |
| D007 | 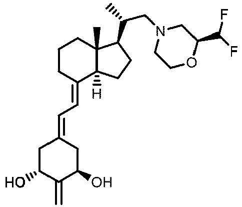 | D008 | 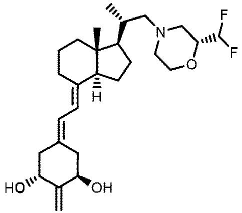 |
| D009 | 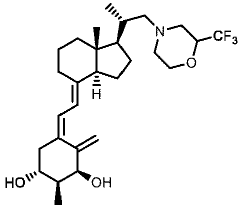 | D010 | 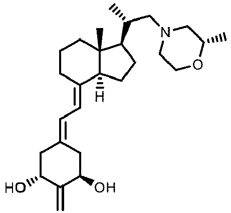 |

**[Table 1-23]**

| Compound number | | Compound number | |
|---|---|---|---|
| D011 | 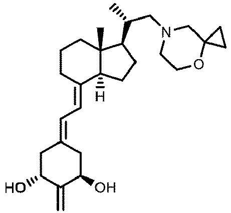 | D012 | 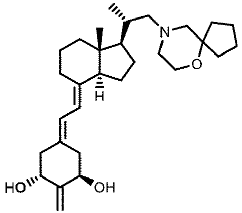 |
| D013 | 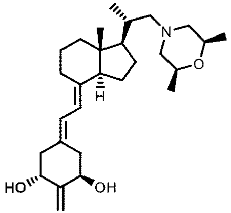 | D014 | 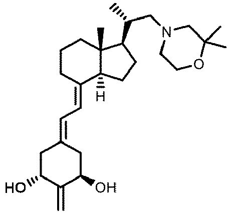 |
| D015 | 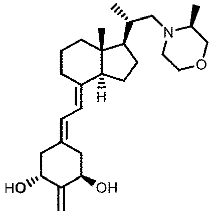 | D016 | 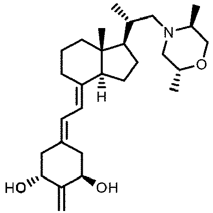 |
| D017 | 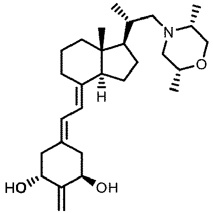 | D018 | 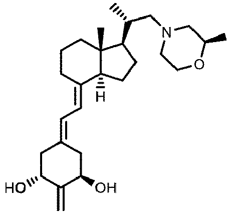 |
| D019 | 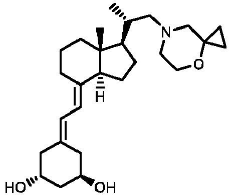 | D020 | 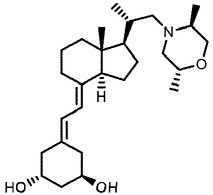 |

**[Table 1-24]**

| Compound number | | Compound number | |
|---|---|---|---|
| D021 | 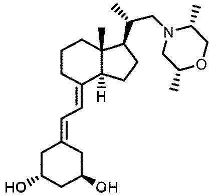 | D022 | 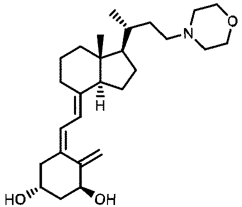 |
| D023 | 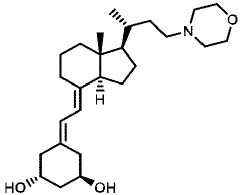 | D024 | 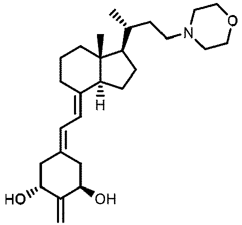 |
| D025 | 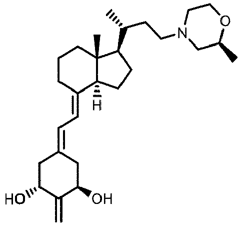 | D026 | 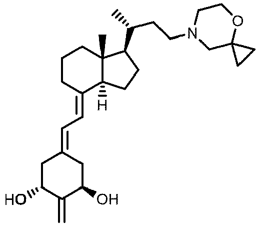 |
| D027 | 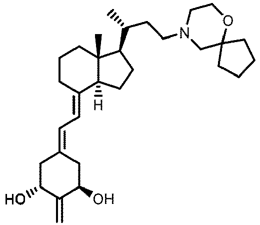 | D028 | 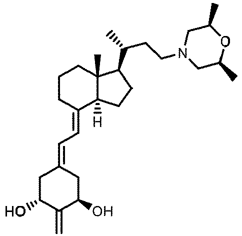 |
| D029 | 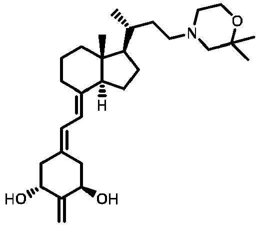 | D030 | 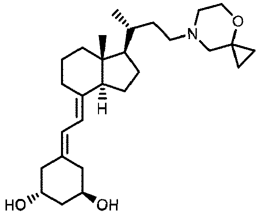 |

**[Table 1-25]**

| Compound number | | Compound number | |
|---|---|---|---|
| D031 | 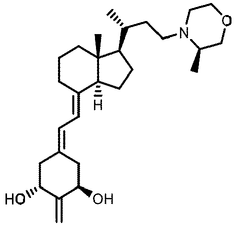 | D032 | 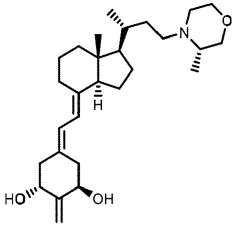 |
| D033 | 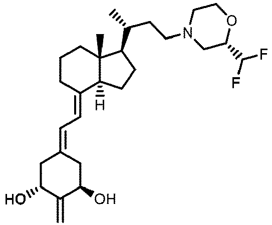 | D034 | 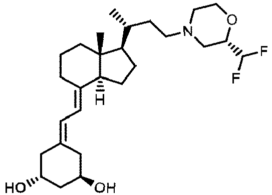 |
| D035 | 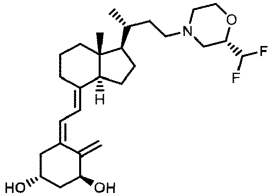 | D036 | 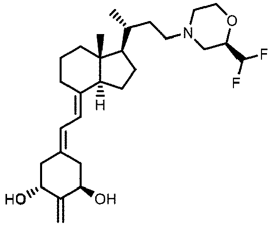 |
| D037 | 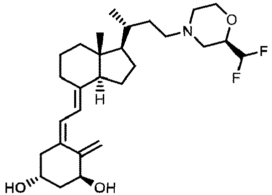 | D038 | 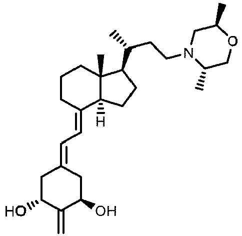 |
| D039 | 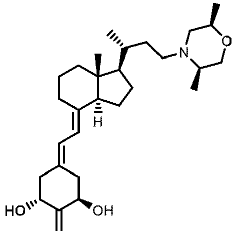 | D040 | 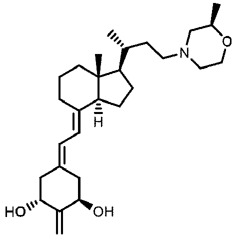 |

**[Table 1-26]**

| Compound number | | Compound number | |
|---|---|---|---|
| D041 | 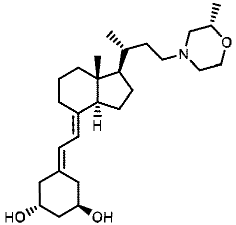 | D042 | 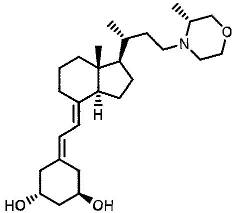 |
| D043 | 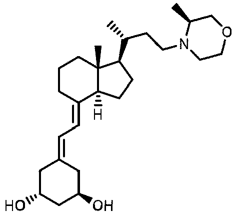 | D044 | 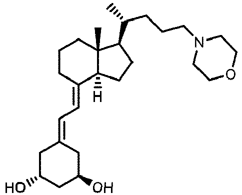 |
| D045 | 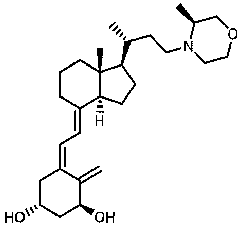 | D046 | 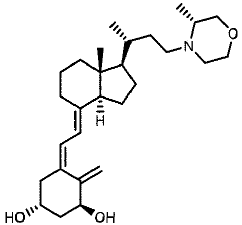 |
| D047 | 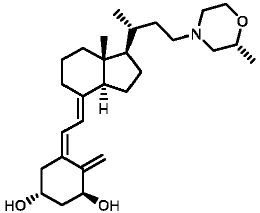 | D048 | 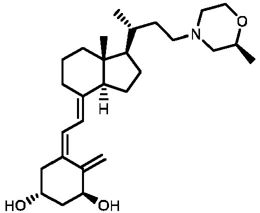 |
| D049 | 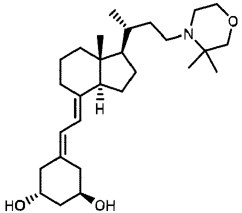 | | |

**[Table 1-27]**

| Compound number | | Compound number | |
|---|---|---|---|
| E001 | 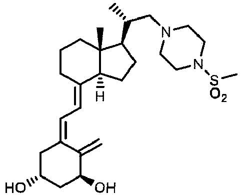 | E002 | 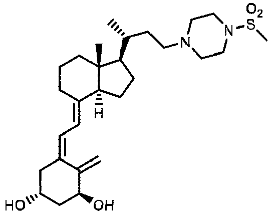 |
| E003 | 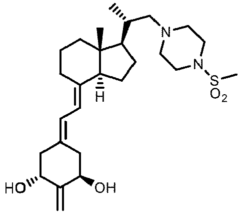 | E004 | 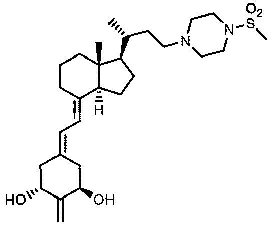 |
| E005 | 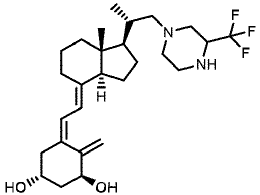 | E006 | 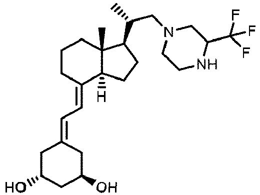 |
| E007 | 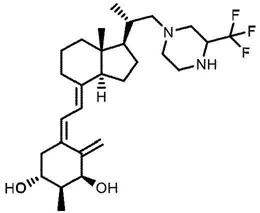 | E008 | 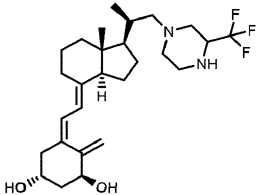 |
| E009 | 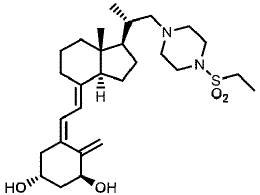 | | |

**[Table 2-1]**

| Compound number | | Compound number | |
|---|---|---|---|
| F001 | 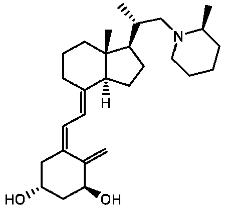 | F002 | 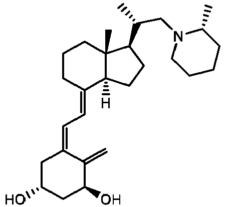 |
| F003 | 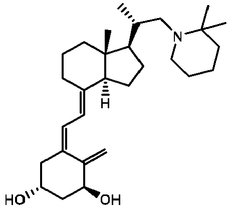 | F004 | 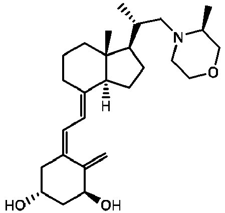 |
| F005 | 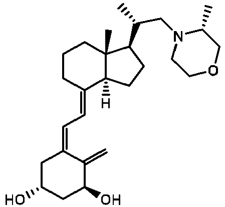 | F006 | 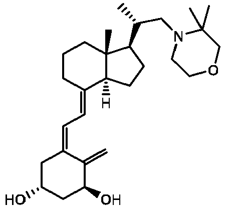 |
| F007 | 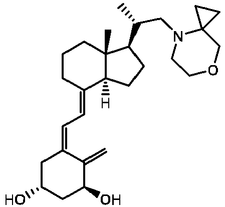 | F008 | 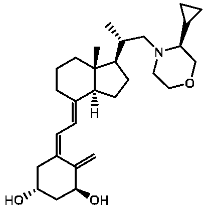 |
| F009 | 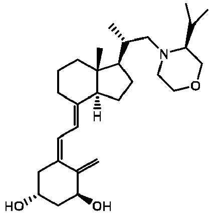 | F010 | 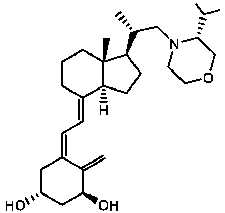 |

**[Table 2-2]**

| Compound number | | Compound number | |
|---|---|---|---|
| F011 | 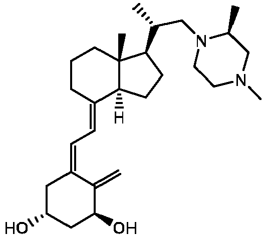 | F012 | 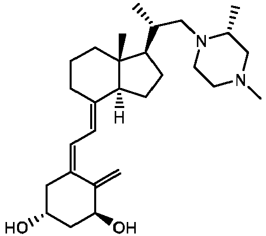 |
| F013 | 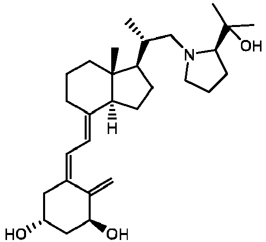 | F014 | 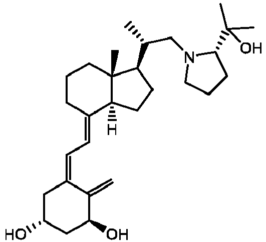 |
| F015 | 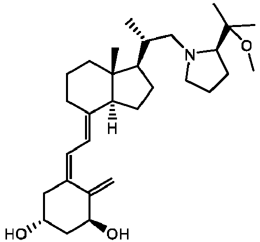 | F016 | 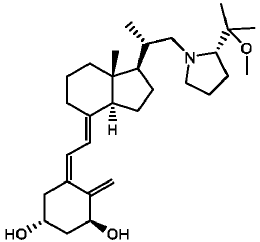 |

**[Table 2-3]**

| Compound number | | Compound number | |
|---|---|---|---|
| G001 | 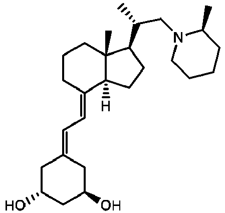 | G002 | 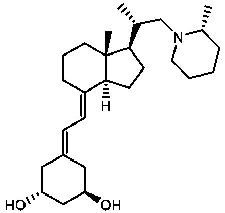 |
| G003 | 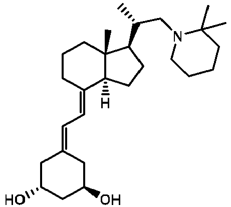 | G004 | 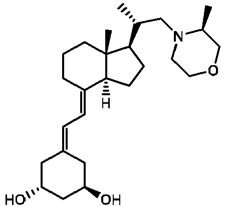 |
| G005 | 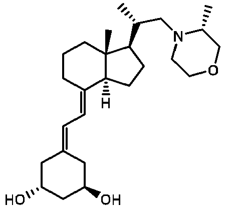 | G006 | 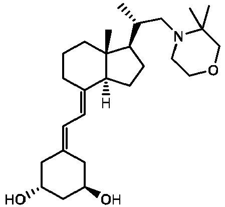 |
| G007 | 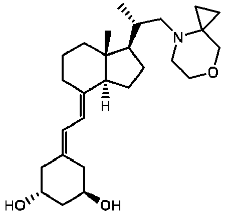 | G008 | 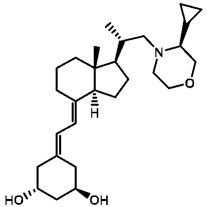 |
| G009 | 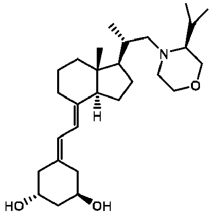 | G010 | 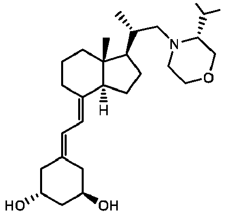 |

**[Table 2-4]**

| Compound number | | Compound number | |
|---|---|---|---|
| G011 | 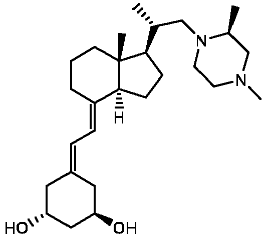 | G012 | 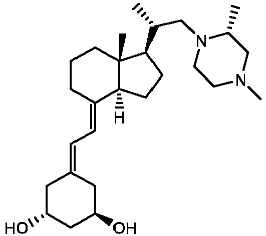 |
| G013 | 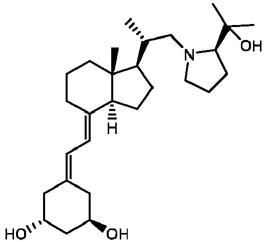 | G014 | 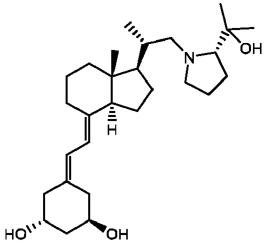 |
| G015 | 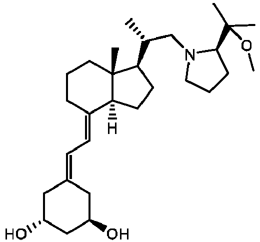 | G016 | 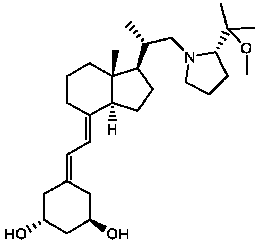 |
| G018 | 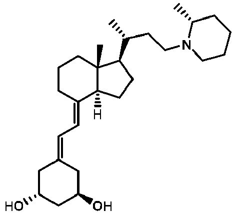 | G020 | 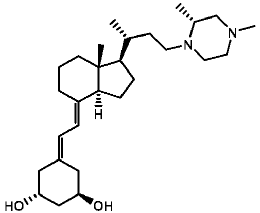 |

**[Table 2-5]**

| Compound number | Construction | Compound number | Construction |
|---|---|---|---|
| H001 | 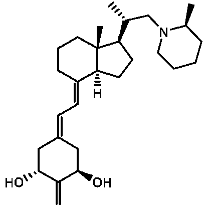 | H002 | 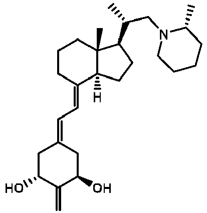 |
| H003 | 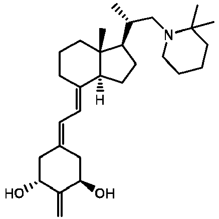 | H011 | 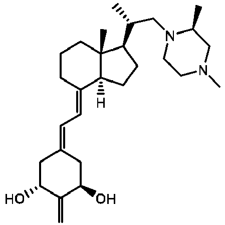 |
| H012 | 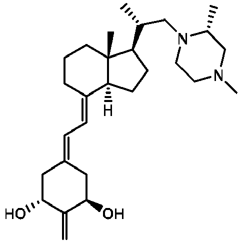 | H013 | 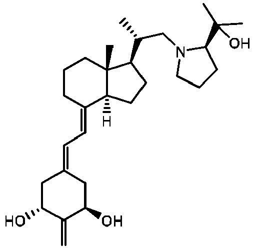 |
| H014 | 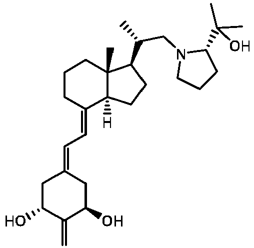 | H015 | 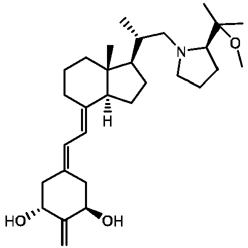 |
| H016 | 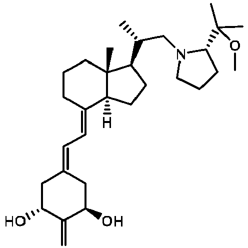 | | |

**[Table 2-6]**

| Compound number | | Compound number | |
|---|---|---|---|
| I001 | 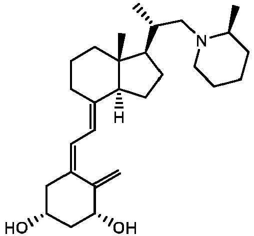 | I002 | 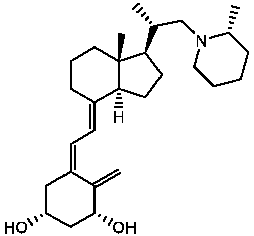 |
| I003 | 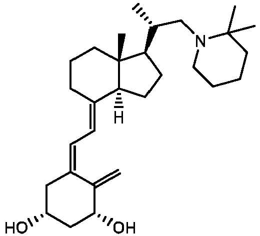 | I004 | 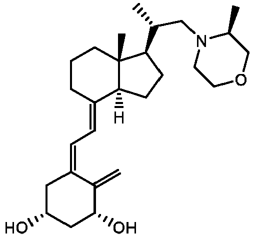 |
| I005 | 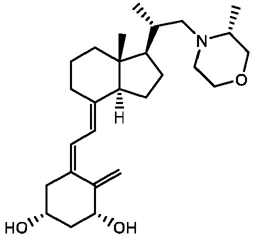 | I006 | 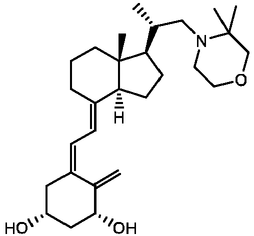 |

Among these, more preferable compounds include Compound Nos. B001, B002, B003, B004, B005, B006, B007, B008, B018, B020, B021, B022, B023, B024, B025, B026, B027, B028, B029, B030, B031, B032, B033, B034, B035, B036, B037, B038, B039, B040, B041, B042, B043, B044, B045, B046, B047, B048, B049, B050, B051, B052, B053, B054, B055, B056, B057, B058, B059, B060, B061, B062, B063, B064, B080, B081, B086, B087, C001, C002, C005, C006, C019, C022, C036, C037, C039, C048, C049, C050, C051, C052, C053, C054, C055, C056, C057, C059, D004, D008, D010, D019, D022, D023, D024, D025, D031, D032, D033, D034, D035, D040, D041, D042, D043, D044, D045, D046, D047, D048, D049, F001, F002, F003, F004, F005, F006, F013, F014, F015, F016, G001, G002, G003, G004, G005, G006, G013, G014, G015, G016, G018, G020, H001, H002, H003, H013, H014, H015, H016, I001, I002, I003, I004, I005, and I006.

A vitamin D derivative of the present invention can be converted into a pharmaceutically acceptable salt thereof, as necessary. Examples of such a salt include hydrochloride, hydrobromide, methanesulfonate, para-toluenesulfonate, acetate, trifluoroacetate, fumarate, maleate, malate, succinate, oxalate, citrate, and benzoate. Particularly preferred salts include hydrochloride, acetate, fumarate, maleate, malate, and succinate.

In addition, a vitamin D derivative of the present invention can be converted into a pharmaceutically acceptable solvate thereof, as necessary. Examples of such a solvent include water, methanol, ethanol, 1-propanol, 2-propanol, butanol, acetonitrile, acetone, methyl ethyl ketone, methyl acetate, and ethyl acetate. Particularly, preferred salts include water, methanol, ethanol, and acetonitrile.

### <General synthesis example>

A vitamin D derivative represented by the above formula (1) may be synthesized by any method. For example, when n = 1 in the above formula (1), synthesis can be performed as in scheme 1. That is, the target product (1) can be obtained by coupling compound (2) and cyclic amine compound (3) in the presence of a base, then deprotecting the protecting group of a hydroxy group, and purifying. 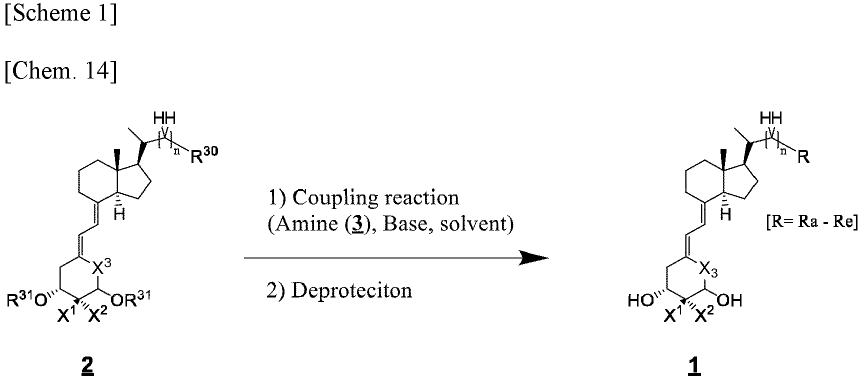 (Wherein R³⁰ in compound (2) in the above Scheme 1 represents a leaving group. Examples of the leaving group include a chlorine atom (Cl), a bromine atom (Br), an iodine atom (I), a methanesulfonyl group (OMs), and a para-toluenesulfonyl group (OTs). Particularly, examples of a preferred leaving group include an iodine atom and a para-toluenesulfonyl group.

Further, R³¹ in compound (2) represents a protecting group for a hydroxy group. Examples of the protecting group include a trimethylsilyl group (TMS), a triethylsilyl group (TES), a t-butyldimethylsilyl group (TBS), and a t-butyldiphenylsilyl group (TBDPS) group. Among these, a t-butyldimethylsilyl group (TBS) is preferable.)

The cyclic amine compound (3) used to be subjected to the coupling reaction (step 1) with compound (2) may be a free form or a salt. The compound (2) is preferably added in an amount of 1 to 3 equivalents. The base in this step is not particularly limited, and examples of a preferred base include potassium carbonate, potassium hydrogen carbonate, and cesium carbonate. The base is added in an amount of 1 to 5 equivalents, and preferably 3 to 5 equivalents. Further, in order to accelerate the reaction, potassium iodide or sodium iodide may be added to the reaction mixture. The amount of potassium iodide or sodium iodide to be added is preferably 1 to 2 equivalents. The solvent is also not particularly limited, and examples of a preferred solvent include N,N-dimethylformamide and N-methyl-2-pyrrolidone. The coupling reaction is preferably performed at a temperature of 40°C to 70°C and for a reaction time of 6 hours to 48 hours.

Step 1 may proceed to the deprotection reaction (step 2) after purification, or may proceed to the deprotection reaction (step 2) with the crude product of step 1.

The condition for the deprotection reaction (step 2) in the above scheme 1 is not particularly limited as long as it satisfies the deprotection condition for a silyl protecting group, and there can be mentioned deprotection with tetrabutylammonium fluoride (TBAF) or deprotection with hydrochloric acid. Preferable conditions are as follows, in tetrahydrofuran (THF), addition of 1 to 3 equivalents of tetrabutylammonium fluoride (TBAF) per hydroxy group, and stirring at room temperature to reflux temperature. Another preferable condition is as follows: in acetone or 2-butane, addition of 1 to 3 equivalents of hydrochloric acid per hydroxy group, and stirring at room temperature. The concentration of hydrochloric acid is preferably 1 M to 6 M. The vitamin D derivative (1) of the present invention can be obtained through purification after the posttreatment of the reaction by a generally used purification method, such as silica gel column chromatography or HPLC.

The compound of the above formula (2) can be synthesized, for example, as in the following scheme 2. 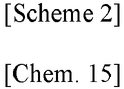 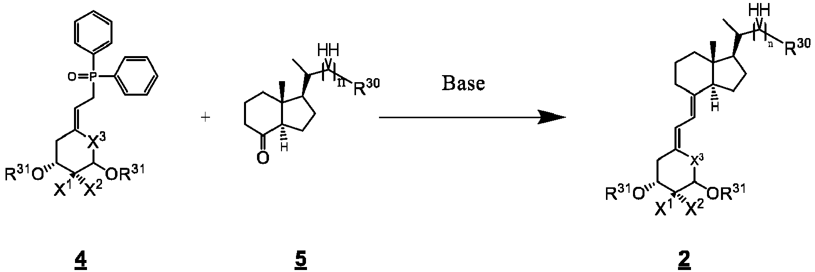 (Wherein, X¹, X², and X³ in the above scheme 2 are the same as defined in the above formula (1). R³⁰ and R³¹ are the same as defined in Scheme 1.)

That is, compound (2) can be obtained by a coupling reaction with subjecting phosphine oxide derivative (4) and compound (5) under a basic condition. Examples of a preferred base in this coupling reaction include sodium hydride, n-butyllithium, lithium diisopropylamide (LDA), lithium bis(trimethylsilyl)amide (LHMDS), potassium bis(trimethylsilyl)amide (KHMDS), and sodium bis(trimethylsilyl)amide (NaHMDS). The use amount of the base is preferably 1.1 to 2 equivalents of compound 4. The reaction is preferably carried out at -78 to -0°C for 1 to 3 hours.

In the above formula (2), when R³⁰ is a para-toluenesulfonyl group (OTs) and R³¹ is a t-butyldimethylsilyl group (TBS), each compounds of combination as shown in the table below are well-known.

**[Table 3]**

| compound | Configuration at C-1 | Configuration at C-20 | X³ | X¹ and X² | CAS Registry No. |
|---|---|---|---|---|---|
| 2a | S | S | C=CH₂ | X¹ and X² = H | 114694-13-2 |
| 2b | R | S | CH₂ | X¹ and X² = H | 1621078-74-2 |
| 2c | R | S | CH₂ | X¹ and X² together form methylidene (C=CH₂) | 610304-70-6 |
| 2d | S | R | C=CH₂ | X¹ and X² = H | 302904-94-5 |
| 2e | R | R | CH₂ | X¹ and X² together form methylidene (C=CH₂) | 1251827-23-2 |

If a compound in the above formula (2) is not well-known, it can be synthesized as follows.

For example, when X¹ is a methyl group, X² is a hydrogen atom, and X³ is C=CH₂, the compound can be synthesized as in scheme 3 below. That is, in the presence of a Pd catalyst, compound (2g) can be synthesized by coupling reaction with well-known compound (6c) (CAS Registry No. 173388-39-1) and well-known compound (7b) (CAS Registry No. 203126-90-3). The target vitamin D derivative (1) can be obtained, in the same manner as in Scheme 1, from compound (2g) by coupling with cyclic amine compound (3) and deprotecting.

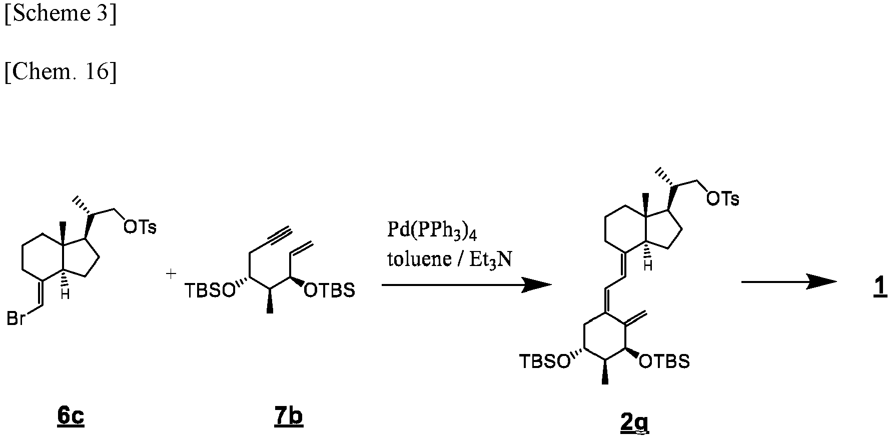

The vitamin D derivative represented by the above formula (1) can be obtained by a different synthesis method: well-known compound (6c) and cyclic amine compound (3) are subjected to a coupling reaction in the presence of a base to synthesize compound (8), compound (8) is subjected to a coupling reaction with compound (9) in the presence of a Pd catalyst, followed by a deprotection reaction. 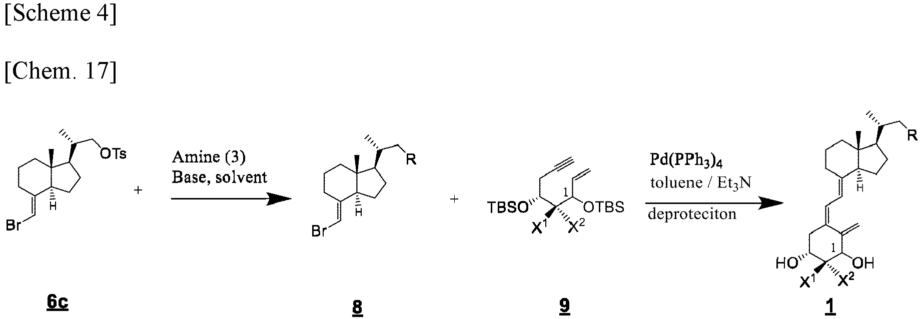 [Wherein, R, X¹, and X² in the above scheme are the same as defined in the above formula (1). The stereochemistry at C-1 in compound (1) and compound (9) are either (R) configuration or (S) configuration.]

The compound with n = 2 in the above formula (1) can be synthesized, for example, as in scheme 5 below. That is, compound (11) is obtained by tosylating the primary hydroxy group of well-known compound (10) (CAS Registry No. 300344-39-2), then desilylating, and oxidizing. Compound (11) and compound (4) described in scheme 2 are subjected to a coupling reaction to obtain compound (12). Using compound (12), cyclic amine compound (3) is subjected to a coupling reaction and a deprotection reaction in the same manner as in scheme 1 to obtain the vitamin D derivative (1) of the present invention. 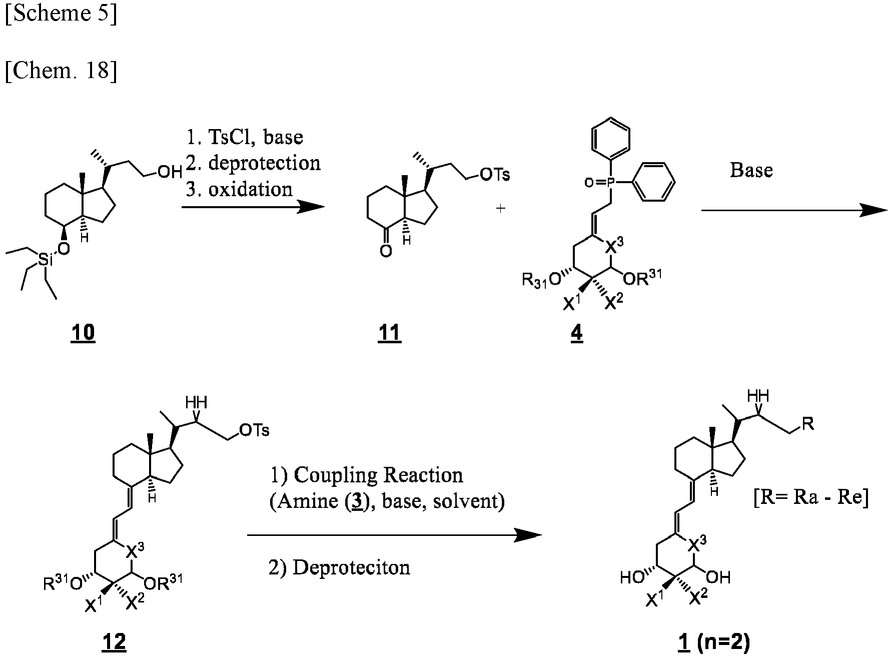 [Wherein, R, X¹, X², and X³ of the above scheme are the same as defined in the above formula (1), and R³⁰ and R³¹ are the same as defined in scheme 1. In the case of a vitamin D derivative (1) in this scheme, n = 2.]

Using compound (2) (n = 1) of scheme 1, the vitamin D derivative (1) (n = 2) can be synthesized in the manner as in scheme 6 below. That is, compound (2) is subjected to nitrilation to obtain compound (13), compound (13) is treated with diisobutylaluminum hydride (DIBAL-H) to obtain aldehyde form compound (14), aldehyde form compound (14) is then reduced to obtain alcohol form compound (15). Compound (15) is tosylated to obtain OTs form compound (16), and then compound (16) is subjected to a coupling reaction with cyclic amine compound (3) in the same manner as in scheme 1, followed by deprotection to obtain the target vitamin D derivative represented by the above formula (1). Each reaction in Scheme 6 of cyanation, DIBAL reduction, and reduction of aldehyde group is performed under commonly used conditions. For example, the cyanation is performed by reaction with 1 to 3 equivalents of KCN and 0.1 to 0.3 equivalents of 18-crown-6 in N,N-dimethylformamide. The reaction temperature is preferably 80°C to 100°C. DIBAL reduction is performed in about 0.5 to 2 hours by adding 1 to 2 equivalents of diisobutylaluminum hydride (DIBAL-H) in toluene at -78°C to 0°C. The reduction of aldehyde group is performed by reaction with 1 to 2 equivalents of sodium borohydride (NaBH4) at 0°C to room temperature to afford the compound (16). 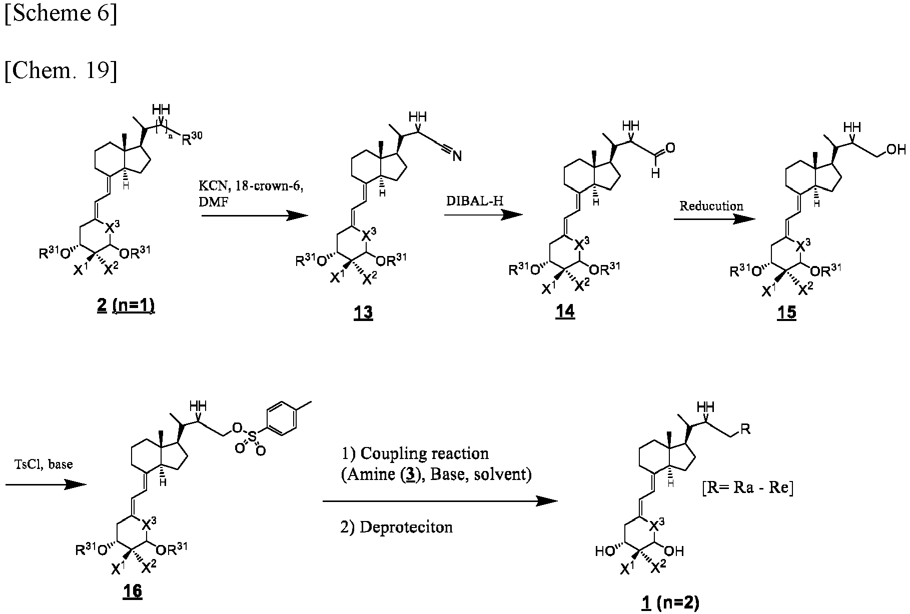 [Wherein, R, X¹, X², and X³ in the above scheme are the same as defined in the above formula (1), and R³⁰ and R³¹ are the same as defined in scheme 1.]

In addition to the above schemes, as shown in the following scheme 7, aldehyde form compound (14) is subjected to a reductive amination with cyclic amine compound (3) and subsequent deprotection to synthesize the vitamin D derivative represented by the above formula (1). 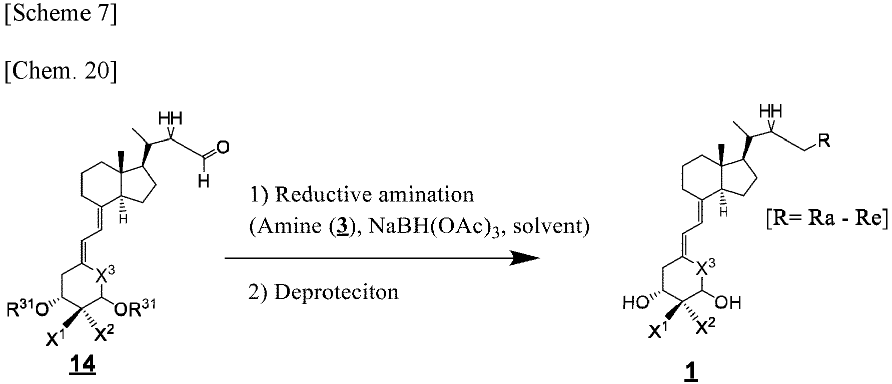 [Wherein, R, X¹, X², and X³ in the above scheme are the same as defined in the above formula (1), and R³¹ is the same as defined in scheme 1.]

Sodium triacetoxyborohydride and sodium cyanoborohydride are preferable reagent for reductive amination. The solvent is also not particularly limited, and examples of a preferred solvent include THF. Further, the cyclic amine compound (3) may be reacted as a solvent. The reductive amination is performed by subjecting compound (14) to a reaction with 1 to 5 equivalents of amine compound (3) and a reducing agent (1 to 3 equivalents) in tetrahydrofuran (THF) and subsequently deprotecting to afford compound (1).

In addition, for example, when n⁰¹ = 1 and n⁰² = 1 for Rc' and Re' of the above formula (1B), they can be synthesized, for example, as in scheme 8 below. That is, the tosyl group of compound (2, R³⁰=OTs) is substituted with a cyano group to obtain compound (13b), and then compound (14b) is obtained by DIBAL reduction. Compound (15) is obtained by subjecting compound (14b) to a reductive amination reaction with amine (3), and then being deprotected to obtain vitamin D derivative (1B) (R' = Rc', Re') of the present invention. Each reaction of cyanation, DIBAL reduction, and reductive amination in scheme 8 is performed under similar conditions described in scheme 6 and 7. 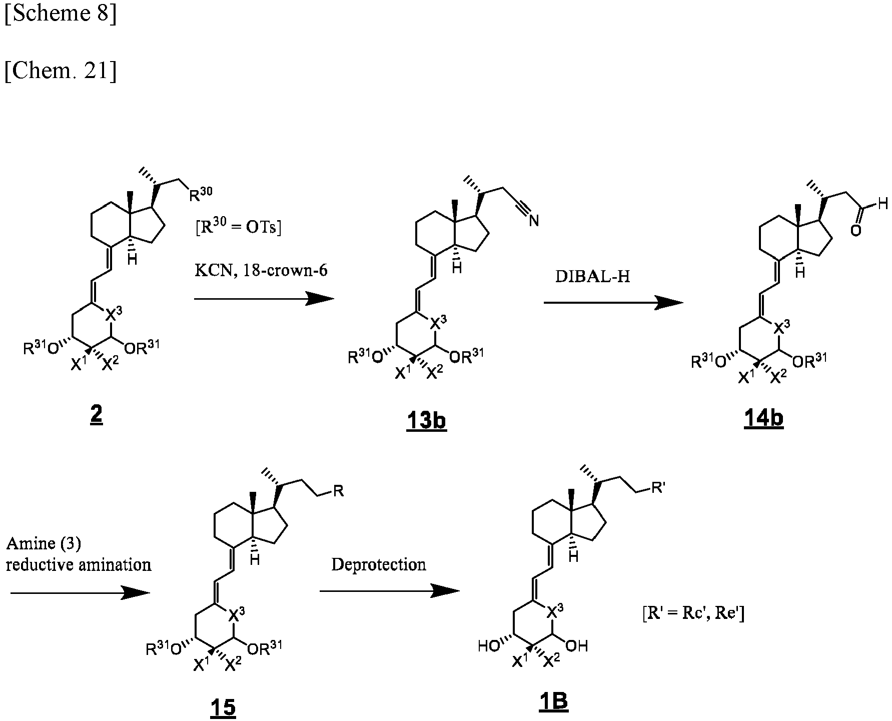 [Wherein R', X¹, X², and X³ in the above scheme are the same as defined in the above formula (1B), R³¹ is the same as defined in scheme 1.]

A vitamin D derivative represented by formula (1) of the present invention, or a pharmaceutically acceptable salt or solvate thereof, has excellent central nervous system penetration, and further has an excellent differentiation-inducing/promoting action from an oligodendrocyte progenitor cell or a neural stem cell into an oligodendrocyte. Due to the excellent differentiation-inducing/promoting action to an oligodendrocyte, a vitamin D derivative represented by formula (1) of the present invention, or a pharmaceutically acceptable salt or solvate thereof is useful as a remyelination promoter.

A vitamin D derivative represented by formula (1) of the present invention, or a pharmaceutically acceptable salt or solvate thereof is clinically applicable as a demyelination promoter, and can be used as a therapeutic agent for diseases associated with demyelination or dysmyelination, such as multiple sclerosis, neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, Guillain-Barre syndrome, Fisher's syndrome, chronic inflammatory demyelinating polyneuropathy, Charcot-Marie-Tooth disease, Parkinson's disease, schizophrenia, bipolar disorder, major depressive disorder, autistic spectrum disorder, attention deficit hyperactivity disorder, obsessive-compulsive disorder, post-traumatic stress disorders, drug-dependent depression, autism, Alzheimer-type dementia, and ischemic stroke.

Among the vitamin D derivatives represented by the formula (1) of the present invention, or pharmaceutically acceptable salts or solvates thereof, the vitamin D derivatives represented by the formulas (1B) and (1B-4), or pharmaceutically acceptable salt or solvate thereof, promote oligodendrocyte progenitor cell (OPC) differentiation despite its weak activity on vitamin D receptors.

The therapeutic agent containing a vitamin D derivative of the present invention or a pharmaceutically acceptable salt or solvate thereof as an active ingredient is prepared into a pharmaceutical composition using those usually used for formulation such as a carrier, a base, an excipient, and other additives. The carrier, base and excipient used in the pharmaceutical composition may be solid or liquid and examples thereof include lactose, magnesium stearate starch, talc, gelatin, agar, pectin, gum arabic, olive oil, sesame oil, cacao butter, ethylene glycol, medium chain fatty acid triglyceride, and others commonly used. Administration may be in any form such as oral administration by tablet, pill, capsule, soft capsule, granule, powder, liquid, and the like, or parenteral administration by injection such as intravenous injection, intramuscular injection and the like, and suppository, transdermal and nasal administration, and the like.

The therapeutically effective amount of the active ingredient in the therapeutic agent of the present invention varies depending on the administration route, age, sex, and degree of disease of the patient, but is usually about 0.1 to 10000 µg/day, **and the number** of administrations is usually 1 to 3 times/day to 1 to 3 times/week, and the preparation is preferably prepared so as to satisfy such conditions. However, since the dose varies depending on various conditions, a dose smaller than the above dose sometimes may be sufficient, or a dose exceeding the above range sometimes may be required.

### [EXAMPLES]

Hereinafter, the present invention will be described in more detail with reference to Examples, but the present invention is not limited thereto.
The abbreviations in the present invention are as follows.
Boc = t-butoxycarbonyl
DAST = diethylaminosulfur trifluoride
DIBAL-H = diisobutylaluminum hydride
DMF = N,N-dimethylformamide
LHMDS = lithium hexamethyldisilazane
MBP = myelin basic protein
MS4A = molecular sieves 4A
NMO = N-methylmorpholine N-oxide
NMP = N-methyl-2-pyrrolidone
PTLC = preparative thin-layer chromatography
TBAF = tetrabutylammonium fluoride
TBS = t-butyldimethylsilyl group
TES = triethylsilyl group
TESCl = chlorotriethylsilane
THF = tetrahydrofuran
TPAP = tetrapropylammonium perruthenate
Ts = p-toluenesulfonyl
TsCl = p-toluenesulfonyl chloride

In the following examples, when the compound of the present invention is obtained by preparative HPLC, the preparative conditions are as follows. Column: YMC-Pack ODS AM (inner diameter 3-30 cm) manufactured by YMC Co., Ltd. Mobile phase A solution: 5% acetonitrile-water (with 0.1% acetic acid) Mobile phase B solution: 95% acetonitrile-water (with 0.1% acetic acid) Addition amount: Crude product is dissolved in 1.3 mL of methanol and injected.
Liquid delivery amount: 12 mL/min
UV: 265 nm
Liquid delivery program:
   0 to 5 minutes: 5% B solution/95% A solution
   5 to 45 minutes: Gradient to 100% B solution in 40 minutes
   45 to 50 minutes: 100% B solution
   50 to 55 minutes: Gradient to 5% B solution/95% A solution in 5 minutes

When the analysis is performed by HPLC/MS in the following examples, the analysis conditions are as follows.
Column: Gemini C18 (3 µm, inner diameter 4.6-30 mm) manufactured by Phenomenex Inc.
Mobile phase A solution: 5% acetonitrile-water (with 0.1% trifluoroacetic acid)
Mobile phase B solution: 95% acetonitrile-water (with 0.1% trifluoroacetic acid)
Liquid delivery amount: 1.2 mL/min
UV: 254 nm
Liquid delivery program:
   0 to 0.01 minutes: 2% B solution/98% A solution
   0.01 to 0.3 minutes: Gradient to 40% B solution/60% A solution
   0.3 to 2.3 minutes: Gradient to 100% B solution
   2.3 to 4.2 minutes: 100% B solution
   4.2 to 4.3 minutes: Gradient to 2% B solution/98% A solution
   4.3 to 5.2 minutes: 2% B solution/98% A solution
   5.2 minutes: Analysis completed

### [Reference example 1]

### Synthesis of (2S)-2-((1R,3aS,7aR,E)-4-((Z)-2((3S,5R)-3,5-bis((t-butyldimethylsilyl)oxy)-2-methylenecyclohexylidene)ethylidene)-7a-methyloctahydro-1H-inden-1-yl)propyl 4-methylbenzenesulfonate (Compound (2a))

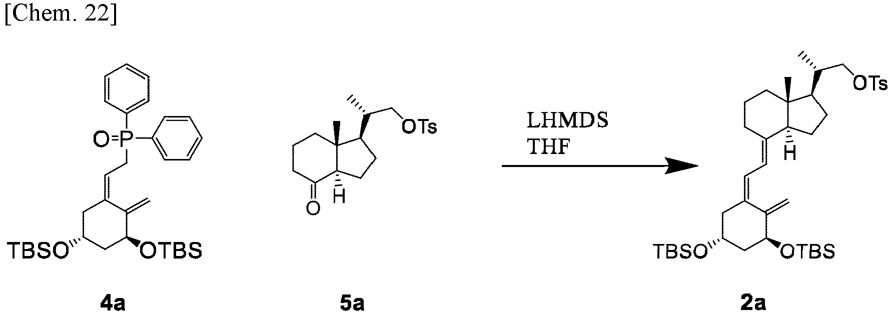

Under an argon atmosphere, LHMDS [1 M in toluene, 10 mL] was added to a solution of ((Z)-2-((3 S,5R)-3,5-bis((t-butyldimethylsilyl)oxy)-2-methylenecyclooxylidene)ethyl)diphenylphosphine oxide (Compound 4a, CAS Registry No. 81522-68-1) [7.42 g, 12.7 mmol] in THF [50 mL], and the mixture was stirred at - 78°C for 1 hour. To the mixture, a solution of (2S)-2-((1R,3aR,7aR)-7a-methyl-4-oxooctadehydro-1H-inden-1-yl)propyl 4-methylbenzenesulfonate (Compound (5a), CAS Registry No. 342645-83-4) [3.6 g, 9.9 mmol] in THF [20 mL] was added and the mixture was further stirred at the same temperature for 1 hour. The reaction mixture was warmed to room temperature and stirred for 30 minutes. A saturated aqueous ammonium chloride solution was added to the reaction mixture, and the mixture was extracted with a mixed solvent of heptane/ethyl acetate (1/1). The organic layer was washed with water followed by 50% methanol-water, dried over anhydrous magnesium sulfate, and concentrated under reduced pressure. The residue was purified by silica gel column chromatography to obtain compound (2a) [4.79 g, 6.57 mmol] (Yield = 67%).

¹H-NMR (CDCl₃) δ: 7.79 (2H, d, J = 8.2 Hz), 7.31 (2H, d, J = 8.2 Hz), 6.22 (1H, d, J = 11.0 Hz), 5.99 (1H, d, J = 11.0 Hz), 5.17 (1H, d, J = 2.0 Hz), 4.84 (1H, d, J = 2.0 Hz), 4.38-4.16 (2H, m), 3.98 (1H, dd, J = 9.1, 3.2 Hz), 3.80 (1H, dd, J = 9.1, 6.4 Hz), 2.83 (1H, d, J = 12.3 Hz), 2.45 (3H, s), 2.42 (1H, d, J = 4.1 Hz), 2.22 (1H, dd, J = 13.0, 7.5 Hz), 1.97-1.57 (8H, m), 1.53-1.15 (9H, m), 0.99 (3H, d, J = 6.4 Hz), 0.87 (9H, s), 0.86 (9H, s), 0.49 (3H, s), 0.06 (9H, s), 0.04 (3H, s).

### [Reference example 2]

### Synthesis of (2S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl 4-methylbenzenesulfonate (Compound 2b)

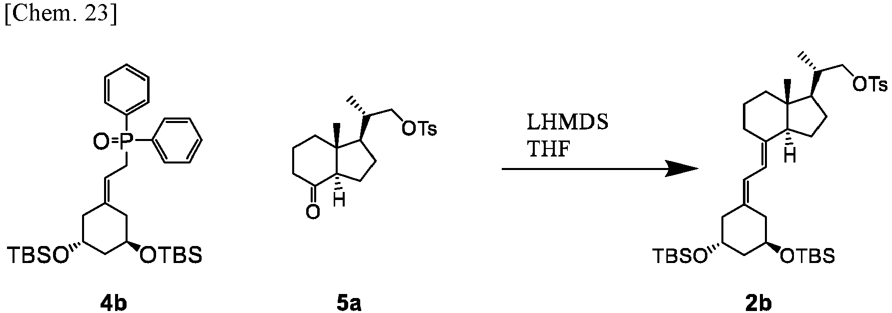

Under an argon atmosphere, LHMDS [1 M in toluene, 20 mL] was added to a solution of (2-((3R,5R)-3,5-bis((t-butyldimethylsilyl)oxy)cyclohexylidene)ethyl)diphenylphosphineoxide (Compound 4b, CAS Registry No. 139356-39-1) [8.55 g, 15.0 mmol] and (2S)-2-((1R,3aR,7aR)-7a-methyl-4-oxooctadehydro-1H-inden-1-yl)propyl 4-methylbenzenesulfonate (compound (5a), CAS Registry No.342645-83-4) [5.06 g, 13.9 mmol] in THF [85 mL] at -78°C and the mixture was stirred at -78°C for 2 hours. The reaction mixture was warmed to 0°C and stirred for 1 hour. A saturated aqueous ammonium chloride solution was added to the reaction mixture at room temperature, and the mixture was extracted with a mixed solvent of heptane/ethyl acetate (1/1). The organic layer was dried over anhydrous magnesium sulfate and concentrated under reduced pressure. Methanol was added to the residue to form a precipitate. The suspension was stirred for 2 hours. The precipitate was collected and dried to obtain compound (2b) [6.40 g, 8.92 mmol] (Yield = 64%).

¹H-NMR (CDCl₃) δ: 7.79 (2H, d, J = 8.2 Hz), 7.35 (2H, d, J = 8.2 Hz), 6.15 (1H, d, J = 11.0 Hz), 5.79 (1H, d, J = 11.4 Hz), 4.12-4.02 (2H, m), 3.98 (1H, dd, J = 9.1, 2.7 Hz), 3.81 (1H, dd, J = 9.1, 6.4 Hz), 2.80 (1H, dd, J = 12.1, 3.9 Hz), 2.46 (3H, s), 2.36 (2H, dd, J = 13.0, 4.8 Hz), 2.25 (1H, dd, J = 14.2, 2.7 Hz), 2.10 (1H, dd, J = 12.8, 8.2 Hz), 2.00-1.90 (2H, m), 1.80-1.60 (6H, m), 1.55-1.16 (6H, m), 0.99 (3H, d, J = 6.4 Hz), 0.87 (9H, s), 0.85 (9H, s), 0.50 (3H, s), 0.05 (3H, s), 0.05 (3H, s), 0.04 (6H, s).

### [Reference example 3]

### Synthesis of (S)-2-((1R,3aS,7aR,E)-4-(2-((3S,5R)-3,5-bis((t-butyldimethylsilyl)oxy)-4-methylenecyclohexylidene)ethylidene)-7a-methyloctahydro-1H-inden-1-yl)propyl 4-methylbenzenesulfonate (Compound 2c)

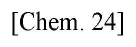

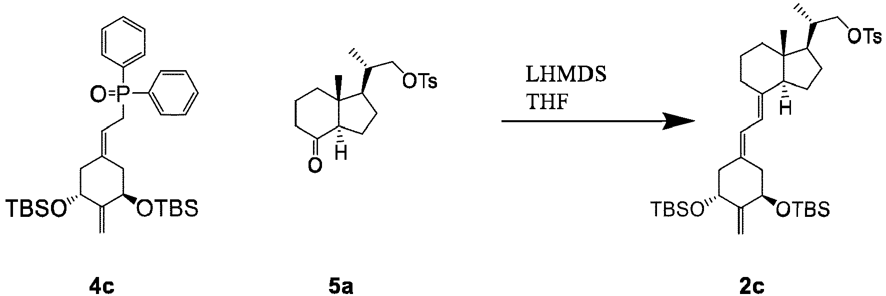

Under an argon atmosphere, LHMDS [1 M in THF, 8.5 mL] was added to a solution of (2-((3R,5R)-3,5-bis((t-butyldimethylsilyl)oxy)-4-methylcyclohexylidene)ethyl)diphenylphosphineoxide (Compound (4c), CAS Registry No. 213250-64-7, 2.50 g, 4.29 mmol) and (2S)-2-((1R,3aR,7aR)-7a-methyl-4-oxooctadehydro-1H-inden-1-yl)propyl 4-methylbenzenesulfonate (Compound (5a), CAS Registry No. 342645-83-4, 2.35 g, 6.45 mmol) in THF [40 mL] at -78°C, and the mixture was stirred at -78°C for 2 hours. The reaction mixture was warmed to room temperature and quenched by adding a saturated aqueous ammonium chloride solution. The reaction mixture was extracted with ethyl acetate, the organic layer was dried over anhydrous magnesium sulfate, and concentrated under reduced pressure. The residue was purified by silica gel column chromatography to obtain compound (2c) (1.70 g, 2.30 mmol) (Yield = 54%).

¹H-NMR (CDCl₃) δ: 7.79 (2H, d, J = 8.8 Hz), 7.35 (2H, d, J = 7.8 Hz), 6.20 (1H, d, J = 11.2 Hz), 5.82 (1H, d, J = 11.2 Hz), 4.97 (1H, s), 4.92 (1H, s), 4.45-4.40 (2H, m), 3.99 (1H, dd, J = 9.3, 2.9 Hz), 3.81 (1H, dd, J = 9.0, 6.6 Hz), 2.81 (1H, dd, J = 12.2, 3.4 Hz), 2.54-2.42 (2H, m), 2.45 (3H, s), 2.32 (1H, dd, J = 13.2, 3.4 Hz), 2.17 (1H, dd, J = 12.7, 8.3 Hz), 2.02-1.90 (2H, m), 1.78-1.63 (4H, m), 1.55-1.15 (9H, m), 1.00 (3H, d, J = 6.3 Hz), 0.89 (9H, s), 0.85 (9H, s), 0.51 (3H, s), 0.07 (3H, s), 0.05 (3H, s), 0.04 (3H, s), 0.02 (3H, s).

### [Reference example 4]

### Synthesis of (S)-2-((1R,3aS,7aR,E)-4-((Z)-2-((3S,4S,5R)-3,5-bis((t-butyldimethylsilyl)oxy)-4-methyl-2-methylenecyclohexylidene)ethylidene)-7a-methyloctahydro-1H-inden-1-yl)propyl4-methylbenzenesulfonate (Compound 2g)

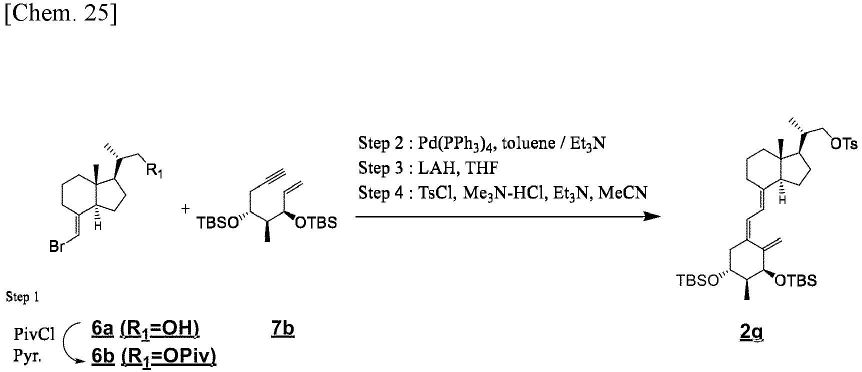

### Step 1

Pivaloyl chloride [0.22 mL, 1.78 mmol] was added to a solution of (2S)-2-((1R,3aS,7aR,E)-4-(bromomethylene)-7a-methyloctahydro-1H-inden-1-yl)propan-1-ol (compound (6a), CAS Registry No. 218437-70-8) [345 mg, 1.20 mmol] in pyridine [7 mL] at 0°C, and the reaction mixture was stirred at room temperature for 1 hour. The reaction mixture was quenched with a saturated aqueous sodium hydrogen carbonate solution and transferred to saturated brine, and the mixture was extracted with ethyl acetate. The organic layer was dried over anhydrous magnesium sulfate and concentrated under reduced pressure. The residue was purified by silica gel column chromatography to obtain compound 6b [415 mg, 1.12 mmol] (Yield = 93%).

¹H-NMR (CDCl₃) δ: 5.67 (1H, d, J = 1.5 Hz), 4.07 (1H, dd, J = 10.7, 3.0 Hz), 3.79 (1H, dd, J = 10.7, 7.3 Hz), 2.95-2.85 (1H, m), 2.02-1.59 (9H, m), 1.55-1.25 (7H, m), 1.21 (9H, s), 1.03 (3H, d, J = 6.8 Hz), 0.59 (3H, s).

### Step 2

Compound 6b [415 mg, 1.12 mmol] obtained in step 1, (5R,6S,7R)-2,2,3,3,6,9,9,10,10-nonamethyl-5-(prop-2-yn-1-yl)-7-vinyl-4,8-dioxa-3,9-disilaundecane (Compound (7b), CAS Registry No. 203126-90-3) [513 mg, 1.34 mmol], and tetrakis(triphenylphosphine)palladium(0) [134.4 mg, 0.116 mmol] were added to a mixed solution of toluene [4 mL] and triethylamine [4 mL], and the mixture was heated and stirred at 100°C for 3 hours under a nitrogen atmosphere. After cooling to room temperature, saturated brine was added to the mixture, and the mixture was extracted with ethyl acetate. The organic layer was dried over anhydrous magnesium sulfate and concentrated under reduced pressure. The residue was purified by silica gel column chromatography to obtain 0.82 g of a crude product containing a coupling product.

### Step 3

Lithium aluminum hydride [LAH, 115 mg, 3.03 mmol] was added at 0°C to a solution of the crude product [0.82 g] obtained in step 2 in THF [20 mL], and the mixture was stirred at room temperature for 1 hour. The reaction mixture was quenched with methanol at 0°C, a 5 M aqueous sodium hydroxide solution [6 mL] was added to the mixture, and the mixture was stirred at room temperature for 30 minutes. The reaction mixture was extracted with ethyl acetate, the organic layer was washed sequentially with saturated brine and a saturated aqueous ammonium chloride solution, dried over anhydrous magnesium sulfate, and concentrated under reduced pressure. The residue was purified by silica gel column chromatography to obtain 0.47 g of a deprotected product.

### Step 4

para-Toluenesulfonyl chloride [0.25 g, 1.3 mmol] was added to a solution of the deprotected product [0.47 g, 0.8 mmol] obtained in step 3, trimethylamine hydrochloride [160 mg, 1.67 mmol], and triethylamine [0.3 mL, 2 mmol] in acetonitrile [10 mL] at room temperature and the mixture was stirred at room temperature for 2 hours. The reaction mixture was quenched with methanol and saturated brine was added to the mixture. The reaction mixture was extracted with ethyl acetate, the organic layer was dried over anhydrous magnesium sulfate, and concentrated under reduced pressure. The residue was purified by silica gel column chromatography to obtain compound 2g [0.43 g, 0.58 mmol].

¹H-NMR (CDCl₃) δ: 7.79 (2H, d, J = 7.8 Hz), 7.34 (2H, d, J = 8.3 Hz), 6.22 (1H, d, J = 11.2 Hz), 6.01 (1H, d, J = 11.2 Hz), 5.12 (1H, d, J = 2.4 Hz), 4.86 (1H, d, J = 2.4 Hz), 4.20 (1H, d, J = 2.4 Hz), 3.98 (1H, dd, J = 9.0, 2.7 Hz), 3.83-3.78 (2H, m), 2.82 (1H, d, J = 12.2 Hz), 2.51-2.45 (5H, m), 2.16 (1H, dd, J = 13.2, 8.3 Hz), 1.97-1.63 (11H, m), 1.53-1.04 (10H, m), 0.99 (3H, d, J = 6.3 Hz), 0.95 (3H, d, J = 6.8 Hz), 0.88 (9H, s), 0.85 (9H, s), 0.49 (3H, s), 0.07 (3H, s), 0.05 (3H, s), 0.05 (3H, s), 0.02 (3H, s).

### [Example 1]

### Synthesis of (1R,3S,Z)-5-(2-((1R,3aS,7aR,E)-1-((R)-1-(3-fluoroazetidin-1-yl)propan-2-yl)-7a-methyloctahydro-4H-inden-4-ylidene)ethylidene)-4-methylenecyclohexane-1,3-diol (Compound A001)

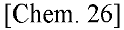

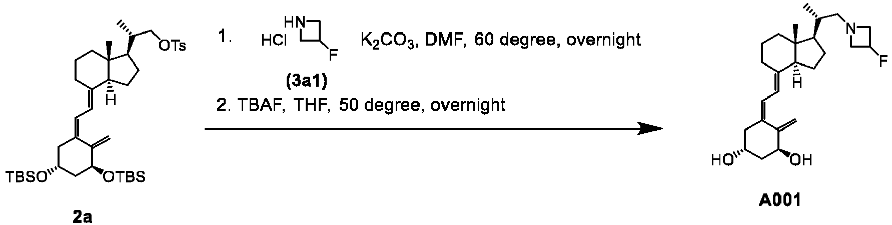

### Step 1

A solution of compound (2a) [90 mg, 0.123 mmol] described in Reference example 1, 3-fluoroazetidine hydrochloride compound (3a1) [50 mg, 0.448 mmol], and K₂CO₃ [90 mg, 0.651 mmol] in DMF [1 mL] was heated and stirred at 60°C overnight. The reaction mixture was cooled to room temperature, saturated brine was added to the mixture, and the mixture was extracted with ethyl acetate. The organic layer was dried over anhydrous magnesium sulfate and concentrated under reduced pressure. The residue was partially purified by PTLC to obtain a coupling product [21.0 mg, 0.0332 mmol].

### Step 2

TBAF [1 M in THF, 0.3 mL, 0.3 mmol] was added to a solution of the coupling product [21.0 mg, 0.0332 mmol] obtained in step 1 in THF [1 mL], and the mixture was heated and stirred at 50°C overnight. The reaction mixture was cooled to room temperature, a saturated aqueous sodium hydrogen carbonate solution was added to the mixture, and the mixture was extracted with ethyl acetate. The organic layer was washed with saturated brine, dried over anhydrous magnesium sulfate, and concentrated under reduced pressure. The residue was purified by preparative HPLC to obtain compound A001 [5.8 mg, 0.014 mmol].
¹H-NMR (CD₃OD) δ: 6.31 (1H, d, J = 11.2 Hz), 6.08 (1H, d, J = 11.2 Hz), 5.28 (1H, dd, J = 2.2, 1.2 Hz), 5.26-5.03 (1H, m), 4.34 (1H, t, J = 5.9 Hz), 4.15-4.09 (1H, m), 3.90-3.80 (2H, m), 3.54-3.40 (2H, m), 2.86 (1H, dd, J = 12.2, 3.9 Hz), 2.71 (1H, dd, J = 12.2, 2.9 Hz), 2.53-2.46 (2H, m), 2.25 (1H, dd, J = 13.4, 6.6 Hz), 2.05-1.97 (3H, m), 1.92-1.28 (14H, m), 1.00 (3H, d, J = 6.8 Hz), 0.59 (3H, s).
LC-MS: Exact Mass = 403.29 (C₂₅H₃₈FNO₂) Obs. mass = 404.45 (M+H),

In the following examples, each compound was synthesized in the same manner as in the synthesis method for compound A001 described in Example 1. In each example, only the raw material and the amine compound used are described. As for the base, potassium carbonate is used as in Example 1, and the equivalent thereof is appropriately changed depending on the raw material to be used according to the conditions of Example 1.

### [Example 2]

### Synthesis of (1R,3R)-5-(2-((1R,3aS,7aR,E)-1-((S)-1-(3-fluoroazetidin-1-yl)propan-2-yl)-7a-methyloctahydro-4H-inden-4-ylidene)ethylidene)-2-methylenecyclohexane-1,3-diol (Compound A002)

Compound A002 [9.0 mg, 0.022 mmol] was obtained by performing the reaction in the same manner as in Example 1 using compound (2c) [50 mg, 0.069 mmol] and 3-fluoroazetidine hydrochloride [30 mg, 0.269 mmol].
¹H-NMR (CD₃OD) δ: 6.26 (1H, d, J = 10.7 Hz), 5.91 (1H, d, J = 11.2 Hz), 5.30-5.08 (1H, m), 5.05 (2H, d, J = 6.8 Hz), 4.42-4.36 (2H, m), 3.96-3.87 (2H, m), 3.61-3.48 (2H, m), 2.87-2.46 (5H, m), 2.31-2.25 (2H, m), 2.10-2.00 (2H, m), 1.70-1.28 (9H, m), 1.01 (3H, d, J = 6.3 Hz), 0.60 (3H, s).
LC-MS: Exact Mass = 403.29 (C₂₅H₃₈FNO₂) Obs. mass = 404.45 (M+H),

### [Example 3]

### Synthesis of (1R,3R,Z)-5-(2-((1R,3aS,7aR,E)-1-((S)-1-(3-(difluoromethyl)azetidin-1-yl)propan-2-yl)-7a-methyloctahydro-4H-inden-4-ylidene)ethylidene)-4-methylenecyclohexane-1,3-diol (Compound A003)

Compound A003 [8.7 mg, 0.022 mmol] was obtained by performing the reaction in the same manner as in Example 1 using compound (2a) [90 mg, 0.123 mmol] and 3-(difluoromethyl)azetidine hydrochloride [50 mg, 0.448 mmol].
¹H-NMR (CD₃OD) δ: 6.31 (1H, d, J = 10.7 Hz), 6.22-5.93 (2H, m), 5.28 (1H, dd, J = 2.2, 1.2 Hz), 4.34 (1H, t, J = 5.9 Hz), 4.15-4.09 (1H, m), 3.85-3.60 (4H, m), 3.20-3.08 (1H, m), 2.86 (2H, dt, J = 12.2, 3.0 Hz), 2.60-2.48 (2H, m), 2.25 (1H, dd, J = 13.4, 6.6 Hz), 2.05-2.00 (3H, m), 1.93-1.27 (13H, m), 1.01 (3H, d, J = 6.8 Hz), 0.60 (3H, s).
LC-MS: Exact Mass = 435.29 (C₂₆H₃₉F₂NO₂) Obs. mass = 436.45 (M+H),

### [Example 4]

### Synthesis of (1R,3R)-5-(2-((1R,3aS,7aR,E)-1-((R)-1-(3-(difluoromethyl)azetidin-1-yl)propan-2-yl)-7a-methyloctahydro-4H-inden-4-ylidene)ethylidene)-2-methylenecyclohexane-1,3-diol (Compound A004)

Compound A004 [7.8 mg, 0.018 mmol] was obtained by performing the reaction in the same manner as in Example 1 using compound (2c) [50 mg, 0.069 mmol] and 3-(difluoromethyl)azetidine hydrochloride [30 mg, 0.269 mmol].
¹H-NMR (CD₃OD) δ: 6.26 (1H, d, J = 10.7 Hz), 6.08 (1H, td, J = 56.5, 4.6 Hz), 5.91 (1H, d, J = 11.7 Hz), 5.05 (2H, d, J = 7.5 Hz), 4.42-4.36 (2H, m), 3.80 (2H, q, J = 8.0 Hz), 3.66-3.61 (2H, m), 3.16-3.05 (1H, m), 2.86 (2H, dd, J = 12.2, 2.4 Hz), 2.66 (1H, dd, J = 13.2, 4.4 Hz), 2.57 (1H, dd, J = 12.2, 10.2 Hz), 2.48 (1H, dd, J = 13.2, 3.9 Hz), 2.31-2.25 (2H, m), 2.10-2.00 (3H, m), 1.70-1.31 (10H, m), 1.01 (3H, d, J = 6.3 Hz), 0.61 (3H, s). Exact Mass = 435.29 (C₂₆H₃₉F₂NO₂) Obs. mass = 436.45 (M+H),

### [Example 5]

### Synthesis of (1R,2S,3S,Z)-5-(2-((1R,3aS,7aR,E)-1-((S)-1-(3-(difluoromethyl)azetidin-1-yl)propan-2-yl)-7a-methyloctahydro-4H-inden-4-ylidene)ethylidene)-2-methyl-4-methylenecyclohexane-1,3-diol (Compound A005)

Compound A005 [4.5 mg, 0.010 mmol] was obtained by performing the reaction in the same manner as in Example 1 using compound (2g) [50 mg, 0.067 mmol] and 3-(difluoromethyl)azetidine hydrochloride [30 mg, 0.269 mmol].
¹H-NMR (CD₃OD) δ: 6.32 (1H, d, J = 10.7 Hz), 6.23-5.90 (2H, m), 5.22 (1H, d, J = 2.0 Hz), 4.22 (1H, d, J = 3.4 Hz), 3.78-3.68 (3H, m), 3.57 (2H, dd, J = 15.6, 6.8 Hz), 3.13-3.02 (1H, m), 2.88-2.78 (2H, m), 2.62-2.48 (2H, m), 2.17 (1H, dd, J = 13.2, 8.3 Hz), 2.02 (2H, dd, J = 12.4, 4.6 Hz), 1.91-1.26 (12H, m), 1.03 (3H, d, J = 6.8 Hz), 1.00 (3H, d, J = 6.3 Hz), 0.58 (3H, s).
Exact Mass = 435.29 (C₂₇H₄₁F₂NO₂) Obs. mass = 436.45 (M+H)

### [Example 6]

### Synthesis of (1R,3S,Z)-5-(2-((1R,3aS,7aR,E)-1-((S)-1-(3-(1,1-difluoroethyl)azetidin-1-yl)propan-2-yl)-7a-methyloctahydro-4H-inden-4-ylidene)ethylidene)-4-methylenecyclohexane-1,3-diol (Compound A006)

Compound A006 [6.8 mg, 0.015 mmol] was obtained by performing the reaction in the same manner as in Example 1 using compound (2a) [90 mg, 0.123 mmol] and 3-(1,1-difluoroethyl)azetidine hydrochloride [60 mg, 0.381 mmol].
¹H-NMR (CD₃OD) δ: 6.31 (1H, d, J = 11.2 Hz), 6.09 (1H, d, J = 11.2 Hz), 5.28 (1H, dd, J = 2.2, 1.2 Hz), 4.34 (1H, t, J = 5.9 Hz), 4.15-4.08 (1H, m), 3.95-3.85 (2H, m), 3.68 (2H, q, J = 8.5 Hz), 3.29-3.10 (2H, m), 2.88 (2H, td, J = 12.0, 3.3 Hz), 2.61 (1H, dd, J = 12.2, 10.2 Hz), 2.51 (1H, dd, J = 13.7, 3.4 Hz), 2.25 (1H, dd, J = 13.4, 6.6 Hz), 2.08-1.95 (4H, m), 1.93-1.26 (18H, m), 1.01 (3H, d, J = 6.3 Hz), 0.60 (3H, s).
Exact Mass = 449.31 (C₂₇H₄₁F₂NO₂) Obs. mass = 450.50 (M+H)

### [Example 7]

### Synthesis of (1R,3R)-5-(2-((1R,3aS,7aR,E)-1-((S)-1-(3-(1,1-difluoroethyl)azetidin-1-yl)propan-2-yl)-7a-methyloctahydro-4H-inden-4-ylidene)ethylidene)-2-methylenecyclohexane-1,3-diol (Compound A007)

Compound A007 [7.6 mg, 0.017 mmol] was obtained by performing the reaction in the same manner as in Example 1 using compound (2c) [50 mg, 0.069 mmol] and 3-(1,1-difluoroethyl)azetidine hydrochloride [30 mg, 0.19 mmol].
¹H-NMR (CD₃OD) δ: 6.26 (1H, d, J = 11.2 Hz), 5.92 (1H, d, J = 11.2 Hz), 5.05 (2H, dd, J= 7.0, 2.0 Hz), 4.43-4.34 (2H, m), 3.97-3.88 (2H, m), 3.75-3.65 (2H, m), 3.26-3.15 (1H, m), 2.93 (1H, dd, J = 12.4, 2.7 Hz), 2.85 (1H, dd, J = 12.0, 3.7 Hz), 2.69-2.60 (2H, m), 2.48 (1H, dd, J= 13.4, 4.1 Hz), 2.32-2.25 (2H, m), 2.11-2.00 (3H, m), 1.68-1.52 (9H, m), 1.42-1.30 (3H, m), 1.02 (3H, d, J = 6.8 Hz), 0.61 (3H, s).
Exact Mass = 449.31 (C₂₇H₄₁F₂NO₂) Obs. mass = 450.45 (M+H)

### [Example 8]

### Synthesis of (1R,2S,3S,Z)-5-(2-((1R,3aS,7aR,E)-1-((S)-1-(3-(1,1-difluoroethyl)azetidin-1-yl)propan-2-yl)-7a-methyloctahydro-4H-inden-4-ylidene)ethylidene)-2-methyl-4-methylenecyclohexane-1,3-diol (Compound A008)

Compound A008 [4.7 mg, 0.010 mmol] was obtained by performing the reaction in the same manner as in Example 1 using compound (2g) [50 mg, 0.067 mmol] and 3-(1,1-difluoroethyl)azetidine hydrochloride [60 mg, 0.38 mmol].
¹H-NMR (CD₃OD) δ: 6.32 (1H, d, J = 11.2 Hz), 6.09 (1H, d, J = 10.7 Hz), 5.22 (1H, d, J = 2.0 Hz), 4.22 (1H, d, J = 3.4 Hz), 3.92-3.83 (2H, m), 3.75-3.61 (3H, m), 3.27-3.12 (1H, m), 2.87 (2H, dd, J= 11.0, 3.7 Hz), 2.63-2.55 (2H, m), 2.20-2.00 (3H, m), 1.92-1.27 (15H, m), 1.03 (3H, d, J = 6.8 Hz), 1.01 (3H, d, J = 6.8 Hz), 0.59 (3H, s).
Exact Mass = 463.32 (C₂₈H₄₃F₂NO₂) Obs. mass = 464.50 (M+H)

### [Example 9]

### (1R,3S,Z)-5-(2-((1R,3aS,7aR,E)-1-((S)-1-(3-hydroxy-3-(trifluoromethyl)azetidin-1-yl)propan-2-yl)-7a-methyloctahydro-4H-inden-4-ylidene)ethylidene)-4-methylenecyclohexane-1,3-diol (Compound A009)

Compound A009 [7.5 mg, 0.016 mmol] was obtained by performing the reaction in the same manner as in Example 1 using compound (2a) [90 mg, 0.123 mmol] and 3-(trifluoromethyl)-3-azetidinol hydrochloride [44 mg, 0.247 mmol].
¹H-NMR (CD₃OD) δ: 6.32 (1H, d, J = 11.2 Hz), 6.09 (1H, d, J = 11.2 Hz), 5.29 (1H, dd, J = 2.4, 1.5 Hz), 4.35 (1H, t, J = 5.9 Hz), 4.16-4.10 (1H, m), 3.62 (2H, dd, J = 9.3, 2.9 Hz), 3.25 (2H, t, J = 8.8 Hz), 2.87 (1H, dd, J = 11.5, 4.1 Hz), 2.58 (1H, dd, J = 12.0, 3.2 Hz), 2.52 (1H, dd, J = 13.2, 3.4 Hz), 2.34-2.24 (2H, m), 2.06-2.04 (1H, m), 2.03-2.00 (3H, m), 1.93-1.20 (14H, m), 0.99 (3H, d, J = 6.8 Hz), 0.59 (3H, s).
Exact Mass = 469.28 (C₂₆H₃₈F₃NO₃) Obs. mass = 470.60 (M+H)

### [Example 10]

### Synthesis of (1R,3R)-5-(2-((1R,3aS,7aR,E)-1-((S)-1-(3-hydroxy-3-(trifluoromethyl)azetidin-1-yl)propan-2-yl)-7a-methyloctahydro-4H-inden-4-ylidene)ethylidene)cyclohexane-1,3-diol (Compound A010)

Compound A010 [8.4 mg, 0.018 mmol] was obtained by performing the reaction in the same manner as in Example 1 using compound (2b) [50 mg, 0.070 mmol] and 3-(trifluoromethyl)-3-azetidinol hydrochloride [37 mg, 0.208 mmol].
¹H-NMR (CD₃OD) δ: 6.22 (1H, d, J = 11.2 Hz), 5.89 (1H, d, J = 11.2 Hz), 4.07-3.95 (2H, m), 3.62 (2H, dd, J = 9.3, 2.9 Hz), 3.25 (2H, t, J = 9.0 Hz), 2.84 (1H, dd, J = 12.7, 3.9 Hz), 2.62-2.57 (2H, m), 2.41 (1H, dd, J = 13.2, 3.4 Hz), 2.32 (1H, dd, J = 11.7, 9.8 Hz), 2.25-2.11 (2H, m), 2.05-2.00 (2H, m), 1.95-1.26 (13H, m), 1.00 (3H, d, J = 6.3 Hz), 0.59 (3H, s).
Exact Mass = 457.28 (C₂₅H₃₈F₃NO₃) Obs. mass = 458.65 (M+H)

### [Example 11]

### Synthesis of (1R,3R)-5-(2-((1R,3aS,7aR,E)-1-((S)-1-(3-hydroxy-3-(trifluoromethyl)azetidin-1-yl)propan-2-yl)-7a-methyloctahydro-4H-inden-4-ylidene)ethylidene)-2-methylenecyclohexane-1,3-diol (Compound A011)

Compound A011 [11.0 mg, 0.023 mmol] was obtained by performing the reaction in the same manner as in Example 1 using compound (2c) [50 mg, 0.069 mmol] and 3-(trifluoromethyl)-3-azetidinol hydrochloride [40.2 mg, 0.226 mmol].
Exact Mass = 469.28 (C₂₆H₃₈F₃NO₃) Obs. mass = 470.35 (M+H)

### [Example 12]

### Synthesis of (1R,3R)-5-(2-((1R,3aS,7aR,E)-1-((S)-1-(3-methoxy-3-trifluoromethylazetidin-1-yl)propan-2-yl)-7a-methyloctahydro-4H-inden-4-ylidene)ethylidene)cyclohexane-1,3-diol (Compound A012)

Compound A012 [3.0 mg, 0.006 mmol] was obtained by performing the reaction in the same manner as in Example 1 using compound (2b) [50 mg, 0.070 mmol] and 3-methoxy-3-(trifluoromethyl)-azetidine hydrochloride [30 mg, 0.157 mmol].
¹H-NMR (CD₃OD) δ: 6.21 (1H, d, J = 11.0 Hz), 5.89 (1H, d, J = 11.4 Hz), 4.05-3.96 (2H, m), 3.45 (3H, s), 3.45 (3H, dd, J = 8.0, 3.0 Hz), 3.35 (4H, t, J = 12.1 Hz), 2.83 (1H, dd, J = 11.7, 3.9 Hz), 2.59 (1H, dd, J = 13.3, 3.7 Hz), 2.52 (1H, dd, J = 11.9, 3.2 Hz), 2.40 (1H, dd, J = 13.5, 3.4 Hz), 2.30-2.13 (3H, m), 2.04-1.28 (17H, m), 0.99 (3H, d, J = 6.4 Hz), 0.58 (3H, s).
Exact Mass = 471.30 (C₂₆H₄₀F₃NO₃) Obs. mass = 472.35 (M+H)

### [Example 13]

### Synthesis of (1R,3R)-5-(2-((1R,3aS,7aR,E)-7a-methyl-1-((S)-1-(3-trifluoromethoxy)azetidin-1-yl)propan-2-yl)octahydro-4H-inden-4-ylidene)ethylidene)cyclohexane-1,3-diol (Compound A013)

Compound A013 [1.5 mg, 0.003 mmol] was obtained by performing the reaction in the same manner as in Example 1 using compound (2b) [50 mg, 0.070 mmol] and 3-(trifluoromethoxy)-azetidine hydrochloride [50 mg, 0.169 mmol].
Exact Mass = 457.28 (C₂₅H₃₈F₃NO₃) Obs. mass = 458.25 (M+H)

### [Example 14]

### Synthesis of (1R,3R)-5-(2-((1R,3aS,7aR,E)-7a-methyl-1-((S)-1-(3-(2,2,2-trifluoroethoxy)azetidin-1-yl)propan-2-yl)octahydro-4H-inden-4-ylidene)ethylidene)cyclohexane-1,3-diol (Compound A014)

Compound A014 [7.4 mg, 0.016 mmol] was obtained by performing the reaction in the same manner as in Example 1 using compound (2b) [50 mg, 0.070 mmol] and 3-(2,2,2-trifluoroethoxy)-azetidine hydrochloride [30 mg, 0.193 mmol].
¹H-NMR (CD₃OD) δ: 6.19 (1H, d, J = 11.0 Hz), 5.87 (1H, d, J = 11.0 Hz), 4.26-4.20 (1H, m), 4.05-3.93 (2H, m), 3.88 (2H, q, J = 9.0 Hz), 3.61 (2H, q, J = 7.6 Hz), 2.99 (1H, t, J = 6.9 Hz), 2.93 (1H, t, J = 6.9 Hz), 2.81 (1H, dd, J = 11.9, 4.1 Hz), 2.57 (1H, dd, J = 13.3, 3.7 Hz), 2.47 (1H, dd, J = 11.7, 3.0 Hz), 2.39 (1H, dd, J = 13.3, 3.2 Hz), 2.27 (1H, dd, J = 11.9, 9.6 Hz), 2.21-2.11 (2H, m), 2.02-1.25 (15H, m), 0.97 (3H, d, J = 6.4 Hz), 0.56 (3H, s).
Exact Mass = 471.30 (C₂₆H₄₀F₃NO₃) Obs. mass = 472.25 (M+H)

### [Example 15]

### Synthesis of (1R,3R)-5-(2-((1R,3aS,7aR,E)-1-((S)-1-(3-(2,2-difluoroethoxy)azetidin-1-yl)propan-2-yl)-7a-methyloctahydro-4H-inden-4-ylidene)ethylidene)cyclohexane-1,3-diol (Compound A015)

Compound A015 [12.8 mg, 0.028 mmol] was obtained by performing the reaction in the same manner as in Example 1 using compound (2b) [50 mg, 0.070 mmol] and 3-(2,2-difluoroethoxy)-azetidine hydrochloride [35 mg, 0.202 mmol].
¹H-NMR (CD₃OD) δ: 6.16 (1H, d, J = 10.7 Hz), 5.85 (1H, tt, J = 3.5, 55.0 Hz), 5.84 (1H, d, J = 10.7 Hz), 4.17-4.11 (1H, m), 4.02-3.90 (2H, m), 3.63-3.52 (4H, m), 2.92 (2H, dt, J = 22.9, 6.8 Hz), 2.78 (1H, dd, J = 12.0, 3.7 Hz), 2.54 (1H, dd, J = 13.2, 3.4 Hz), 2.44 (1H, dd, J = 12.2, 2.9 Hz), 2.36 (1H, dd, J = 13.2, 3.4 Hz), 2.26-2.09 (3H, m), 1.99-1.24 (15H, m), 0.94 (3H, d, J = 6.3 Hz), 0.53 (3H, s).
Exact Mass = 453.31 (C₂₆H₄₁F₂NO₃) Obs. mass = 454.25 (M+H)

### [Example 16]

### Synthesis of (1R,3R)-5-(2-((1R,3aS,7aR,E)-1-((S)-1-(3-(2,2-difluoroethoxy)azetidin-1-yl)propan-2-yl)-7a-methyloctahydro-4H-inden-4-ylidene)ethylidene)-2-methylenecyclohexane-1,3-diol (Compound A016)

Compound A016 [20.6 mg, 0.044 mmol] was obtained by performing the reaction in the same manner as in Example 1 using compound (2c) [100 mg, 0.137 mmol] and 3-(2,2-difluoroethoxy)-azetidine hydrochloride [70 mg, 0.403 mmol].
¹H-NMR (CD₃OD) δ: 6.24 (1H, d, J = 11.0 Hz), 6.03-5.74 (2H, m), 5.03 (2H, d, J = 6.9 Hz), 4.43-4.30 (2H, m), 4.20-4.14 (1H, m), 3.68-3.55 (4H, m), 2.97 (1H, t, J = 6.9 Hz), 2.92 (1H, t, J = 6.9 Hz), 2.83 (1H, dd, J = 11.9, 3.7 Hz), 2.65 (1H, dd, J = 13.3, 4.6 Hz), 2.49-2.42 (2H, m), 2.30-2.20 (3H, m), 2.03-1.87 (3H, m), 1.68-1.44 (6H, m), 1.40-1.25 (3H, m), 0.97 (3H, d, J = 6.4 Hz), 0.57 (3H, s).
Exact Mass = 453.31 (C₂₇H₄₁F₂NO₃) Obs. mass = 454.25 (M+H)

### [Example 17]

### Synthesis of (1R,3R,Z)-5-(2-((1R,3aS,7aR,E)-1-((S)-1-(3-(2,2-difhioroethoxy)azetidin-1-yl)propan-2-yl)-7a-methyloctahydro-4H-inden-4-ylidene)ethylidene)-4-methylenecyclohexane-1,3-diol (Compound A017)

Compound A017 [24.6 mg, 0.053 mmol] was obtained by performing the reaction in the same manner as in Example 1 using compound (2a) [100 mg, 0.137 mmol] and 3-(2,2-difluoroethoxy)-azetidine hydrochloride [70 mg, 0.403 mmol].
¹H-NMR (CD₃OD) δ: 6.27 (1H, d, J = 10.7 Hz), 6.04 (1H, d, J = 11.2 Hz), 5.85 (1H, tt, J = 55.4, 3.8 Hz), 5.24 (1H, d, J = 1.0 Hz), 4.85 (1H, s), 4.30 (1H, t, J = 5.9 Hz), 4.18-4.03 (2H, m), 3.63-3.50 (4H, m), 2.97-2.80 (3H, m), 2.49-2.41 (2H, m), 2.25-2.18 (2H, m), 1.96-1.20 (17H, m), 0.94 (3H, d, J = 6.3 Hz), 0.53 (3H, s).
Exact Mass = 465.31 (C₂₇H₄₁F₂NO₃) Obs. mass = 466.30 (M+H)

### [Example 18]

### Synthesis of (1R,3R)-5-(2-((1R,3aS,7aR,E)-1-((S)-1-(3-difluoromethoxy)azetidin-1-yl)propan-2-yl)-7a-methyloctahydro-4H-inden-4-ylidene)ethylidene)-2-methylenecyclohexane-1,3-diol (Compound A018)

Compound A018 [15 mg, 0.033 mmol] was obtained by performing the reaction in the same manner as in Example 1 using compound (2c) [50 mg, 0.069 mmol] and 3-(difluoromethoxy)-azetidine hydrochloride [33 mg, 0.207 mmol].
¹H-NMR (CD₃OD) δ: 6.43 (1H, t, J = 75.0 Hz), 6.27 (1H, d, J = 11.2 Hz), 5.92 (1H, d, J = 11.2 Hz), 5.06 (2H, d, J = 6.8 Hz), 4.89-4.82 (1H, m), 4.40 (2H, ddd, J = 13.7, 7.1, 4.6 Hz), 3.98 (2H, q, J = 7.5 Hz), 3.50 (2H, ddd, J = 18.8, 9.5, 6.1 Hz), 2.88-2.84 (1H, m), 2.80 (1H, dd, J = 12.2, 2.9 Hz), 2.68 (1H, dd, J = 13.4, 4.1 Hz), 2.57 (1H, dd, J = 12.0, 10.0 Hz), 2.49 (1H, dd, J = 13.4, 3.7 Hz), 2.32-2.26 (2H, m), 2.09-2.00 (2H, m), 1.95-1.90 (1H, m), 1.71-1.49 (6H, m), 1.42-1.30 (3H, m), 1.02 (3H, d, J = 6.3 Hz), 0.61 (3H, s).
Exact Mass = 451.30 (C₂₆H₃₉F₂NO₃) Obs. mass = 452.40 (M+H)

### [Example 19]

### Synthesis of (1R,3R)-5-(2-((1R,3aS,7aR,E)-1-((S)-1-(3-(2,2-difluoroethyl)azetidin-1-yl)propan-2-yl)-7a-methyloctahydro-4H-inden-4-ylidene)ethylidene)-2-methylenecyclohexane-1,3-diol (Compound A019)

Compound A019 [10.1 mg, 0.0225 mmol] was obtained by performing the reaction in the same manner as in Example 1 using compound (2c) [50 mg, 0.069 mmol] and 3-(2,2-difluoroethyl)-azetidine hydrochloride [35 mg, 0.222 mmol].
Exact Mass = 449.31 (C₂₇H₄₁F₂NO₂) Obs. mass = 450.25 (M+H)

### [Example 20]

### Synthesis of (1R,3R)-5-(2-((1R,3aS,7aR,E)-1-((S)-1-(3-difhioromethoxy)azetidin-1-yl)propan-2-yl)-7a-methyloctahydro-4H-inden-4-ylidene)ethylidene)cyclohexane-1,3-diol (Compound A020)

Compound A020 [10.0 mg, 0.023 mmol] was obtained by performing the reaction in the same manner as in Example 1 using compound (2b) [50 mg, 0.070 mmol] and 3-(difluoromethoxy)-azetidine hydrochloride [30 mg, 0.244 mmol].
¹H-NMR (CD₃OD) δ: 6.36 (1H, t, J = 75.0 Hz), 6.21 (1H, d, J = 11.2 Hz), 5.89 (1H, d, J = 11.2 Hz), 4.74-4.68 (1H, m), 4.08-3.95 (2H, m), 3.65 (2H, q, J = 6.8 Hz), 3.09 (1H, t, J = 6.8 Hz), 3.03 (1H, t, J = 6.8 Hz), 2.83 (1H, dd, J = 12.4, 3.7 Hz), 2.59 (1H, dd, J = 13.7, 3.9 Hz), 2.49 (1H, dd, J = 11.7, 2.9 Hz), 2.41 (1H, t, J = 6.6 Hz), 2.29 (1H, dd, J = 11.7, 9.3 Hz), 2.23-1.27 (18H, m), 0.99 (3H, d, J = 6.3 Hz), 0.58 (3H, s).
Exact Mass = 439.29 (C₂₅H₃₉F₂NO₃) Obs. mass = 440.20 (M+H)

### [Example 21]

### Synthesis of (1R,3R)-5-(2-((1R,3aS,7aR,E)-1-((S)-1-(3-(2,2-difluoroethyl)azetidin-1-yl)propan-2-yl)-7a-methyloctahydro-4H-inden-4-ylidene)ethylidene)cyclohexane-1,3-diol (Compound A021)

Compound A021 [ 12.2 mg, 0.028 mmol] was obtained by performing the reaction in the same manner as in Example 1 using compound (2b) [50 mg, 0.070 mmol] and 3-(2,2-difluoroethyl)-azetidine hydrochloride [35 mg, 0.222 mmol].
¹H-NMR (CD₃OD) δ: 6.21 (1H, d, J = 11.4 Hz), 6.00-5.69 (2H, m), 4.06-3.95 (2H, m), 3.52 (2H, dd, J = 17.2, 7.5 Hz), 2.93-2.78 (3H, m), 2.73-2.65 (1H, m), 2.59 (1H, dd, J = 13.3, 3.7 Hz), 2.48 (1H, dd, J = 11.9, 2.7 Hz), 2.40 (1H, dd, J = 13.5, 3.4 Hz), 2.25-1.97 (7H, m), 1.95-1.72 (3H, m), 1.68-1.44 (6H, m), 1.38-1.23 (3H, m), 0.98 (3H, d, J = 6.4 Hz), 0.58 (3H, s).
Exact Mass = 437.31 (C₂₆H₄₁F₂NO₂) Obs. mass = 438.35 (M+H)

### [Example 22]

### Synthesis of (1R,3S,Z)-5-(2-((1R,3aS,7aR,E)-7a-methyl-1-((S)-1-((S)-3-methylpyrrolidin-1-yl)propan-2-yl)octahydro-4H-inden-4-ylidene)ethylidene)-4-methylenecyclohexane-1,3-diol (Compound B001)

Compound B001 [17.5 mg, 0.042 mmol] was obtained by performing the reaction in the same manner as in Example 1 using compound (2a) [90 mg, 0.123 mmol] and 3-(S)-methylpyrrolidine hydrochloride [40 mg, 0.331 mmol].
¹H-NMR (CD₃OD) δ: 6.32 (1H, d, J = 11.2 Hz), 6.09 (1H, d, J = 11.2 Hz), 5.28 (1H, dd, J = 2.4, 1.5 Hz), 4.89 (1H, dd, J = 2.4, 1.5 Hz), 4.34 (1H, dd, J = 6.3, 5.4 Hz), 4.15-4.10 (1H, m), 3.55 (1H, dd, J = 11.0, 7.6 Hz), 3.42-3.35 (2H, m), 3.09 (1H, dd, J = 12.7, 2.9 Hz), 2.99 (1H, t, J = 12.0 Hz), 2.87 (1H, dd, J = 12.0, 3.7 Hz), 2.78 (1H, t, J = 10.0 Hz), 2.53-2.44 (2H, m), 2.30-2.16 (2H, m), 2.07-1.96 (4H, m), 1.90-1.30 (15H, m), 1.14 (3H, d, J = 6.6 Hz), 1.13 (3H, d, J = 6.3 Hz), 0.63 (3H, s).
Exact Mass = 413.31 (C₂₇H₄₃NO₂) Obs. mass = 414.45 (M+H)

### [Example 23]

### Synthesis of (1R,3R)-5-(2-((1R,3aS,7aR,E)-7a-methyl-1-((S)-1-((S)-3-methylpyrrolidin-1-yl)propan-2-yl)octahydro-4H-inden-4-ylidene)ethylidene)-2-methylenecyclohexane-1,3-diol (Compound B002)

Compound B002 [13.7 mg, 0.033 mmol] was obtained by performing the reaction in the same manner as in Example 1 using compound (2c) [50 mg, 0.069 mmol] and 3-(S)-methylpyrrolidine hydrochloride [20 mg, 0.235 mmol].
¹H-NMR (CD₃OD) δ: 6.26 (1H, d, J = 11.2 Hz), 5.92 (1H, d, J = 11.2 Hz), 5.05 (2H, d, J = 5.9 Hz), 4.44-4.35 (2H, m), 3.55 (1H, dd, J = 10.7, 7.8 Hz), 3.43-3.35 (2H, m), 3.10 (1H, dd, J = 12.7, 2.9 Hz), 2.99 (1H, t, J = 12.0 Hz), 2.89-2.75 (2H, m), 2.66 (1H, dd, J = 13.2, 4.4 Hz), 2.52-2.41 (2H, m), 2.32-2.17 (3H, m), 2.10-1.95 (3H, m), 1.89-1.34 (11H, m), 1.14 (3H, d, J = 6.6 Hz), 1.13 (3H, d, J = 6.3 Hz), 0.64 (3H, s).
Exact Mass = 413.33 (C₂₇H₄₃NO₂) Obs. mass = 414.45 (M+H)

### [Example 24]

### Synthesis of (1R,3R)-5-(2-((1R,3aS,7aR,E)-7a-methyl-1-((S)-1-((R)-3-methylpyrrolidin-1-yl)propan-2-yl)octahydro-4H-inden-4-ylidene)ethylidene)-2-methylenecyclohexane-1,3-diol (Compound B003)

Compound B003 [5.7 mg, 0.014 mmol] was obtained by performing the reaction in the same manner as in Example 1 using compound (2c) [50 mg, 0.069 mmol] and 3-(R)-methylpyrrolidine hydrochloride [30 mg, 0.247 mmol].
Exact Mass = 413.33 (C₂₇H₄₃NO₂) Obs. mass = 414.25 (M+H)

### [Example 25]

### Synthesis of (1R,3R)-5-(2-((1R,3aS,7aR,E)-7a-methyl-1-((S)-1-((R)-3-methylpyrrolidin-1 -yl)propan-2-yl)octahydro-4H-inden-4-ylidene)ethylidene)cyclohexane-1,3-diol (Compound B004)

Compound B004 [15.3 mg, 0.038 mmol] was obtained by performing the reaction in the same manner as in Example 1 using compound (2b) [55.3 mg, 0.077 mmol] and 3-(R)-methylpyrrolidine hydrochloride [28.3 mg, 0.233 mmol].
¹H-NMR (CD₃OD) δ: 6.21 (1H, d, J = 10.7 Hz), 5.89 (1H, d, J = 10.7 Hz), 4.06-3.95 (2H, m), 2.83 (1H, dd, J= 12.0, 3.7 Hz), 2.74-2.50 (5H, m), 2.46-2.12 (7H, m), 2.07-1.47 (17H, m), 1.35 (2H, ddd, J = 23.4, 10.7, 2.9 Hz), 1.26 (1H, dd, J = 18.5, 9.3 Hz), 1.05 (3H, d, J = 6.8 Hz), 0.60 (3H, s).
Exact Mass = 401.33 (C₂₆H₄₃NO₂) Obs. mass = 402.25 (M+H)

### [Example 26]

### Synthesis of (1R,3R)-5-(2-((1R,3aS,7aR,E)-1-((S)-1-((S)-3-ethylpyrrolidin-1-yl)propan-2-yl)-7a-methyloctahydro-4H-inden-4-ylidene)ethylidene)-2-methylenecyclohexane-1,3-diol (Compound B005)

Compound B005 [7.0 mg, 0.016 mmol] was obtained by performing the reaction in the same manner as in Example 1 using compound (2c) [50 mg, 0.069 mmol] and 3-(S)-ethylpyrrolidine hydrochloride [30 mg, 0.221 mmol].
Exact Mass = 427.35 (C₂₈H₄₅NO₂) Obs. mass = 428.25 (M+H)

### [Example 27]

### Synthesis of (1R,3R)-5-(2-((1R,3aS,7aR,E)-1-((S)-1-((R)-3-ethylpyrrolidin-1-yl)propan-2-yl)-7a-methyloctahydro-4H-inden-4-ylidene)ethylidene)-2-methylenecyclohexane-1,3-diol (Compound B006)

Compound B006 [5.0 mg, 0.011 mmol] was obtained by performing the reaction in the same manner as in Example 1 using compound (2c) [50 mg, 0.069 mmol] and 3-(R)-ethylpyrrolidine hydrochloride [30 mg, 0.221 mmol].
¹H-NMR (CD₃OD) δ: 6.26 (1H, d, J = 11.4 Hz), 5.91 (1H, d, J = 11.4 Hz), 5.05 (2H, d, J = 6.9 Hz), 4.41 (1H, dd, J = 6.4, 4.6 Hz), 4.37 (1H, dd, J = 7.8, 4.6 Hz), 2.85 (1H, dd, J = 11.9, 4.1 Hz), 2.74 (2H, q, J = 7.6 Hz), 2.67 (1H, dd, J = 13.7, 4.6 Hz), 2.48 (1H, dd, J = 13.5, 3.9 Hz), 2.38-2.22 (5H, m), 2.12-1.92 (6H, m), 1.70-1.22 (12H, m), 1.05 (3H, d, J = 6.9 Hz), 0.90 (3H, t, J = 7.5 Hz), 0.60 (3H, s).
Exact Mass = 427.35 (C₂₈H₄₅NO₂) Obs. mass = 428.25 (M+H)

### [Example 28]

### Synthesis of (1R,3R)-5-(2-((1R,3aS,7aR,E)-1-((S)-1-((S)-3-ethylpyrrolidin-1-yl)propan-2-yl)-7a-methyloctahydro-4H-inden-4-ylidene)ethylidene)cyclohexane-1,3-diol (Compound B007)

Compound B007 [9.4 mg, 0.023 mmol] was obtained by performing the reaction in the same manner as in Example 1 using compound (2b) [50 mg, 0.070 mmol] and 3-(S)-ethylpyrrolidine hydrochloride [37 mg, 0.273 mmol].

¹H-NMR (CD₃OD) δ: 6.21 (1H, d, J = 11.0 Hz), 5.89 (1H, d, J = 11.0 Hz), 4.08-3.95 (2H, m), 2.85-2.71 (3H, m), 2.59 (1H, d, J = 11.0 Hz), 2.42-2.28 (4H, m), 2.23-1.51 (16H, m), 1.45-1.23 (6H, m), 1.05 (3H, d, J = 5.9 Hz), 0.90 (3H, t, J = 7.5 Hz), 0.60 (3H, s).

### [Example 29]

### Synthesis of (1R,3R)-5-(2-((1R,3aS,7aR,E)-1-((S)-1-((R)-3-ethylpyrrolidin-1-yl)propan-2-yl)-7a-methyloctahydro-4H-inden-4-ylidene)ethylidene)cyclohexane-1,3-diol (Compound B008)

Compound B008 [8.6 mg, 0.021 mmol] was obtained by performing the reaction in the same manner as in Example 1 using compound (2b) [50 mg, 0.070 mmol] and 3-(R)-ethylpyrrolidine hydrochloride [39 mg, 0.288 mmol].

¹H-NMR (CD₃OD) δ: 6.21 (1H, d, J = 11.4 Hz), 5.89 (1H, d, J = 11.4 Hz), 4.07-3.95 (2H, m), 2.93 (1H, t, J = 8.0 Hz), 2.83 (1H, dd, J = 11.9, 3.7 Hz), 2.68 (1H, t, J = 7.5 Hz), 2.59 (1H, dd, J = 13.5, 3.7 Hz), 2.48-2.13 (6H, m), 2.10-1.20 (20H, m), 1.05 (3H, d, J = 6.9 Hz), 0.90 (3H, t, J = 7.3 Hz), 0.60 (3H, s).

### [Example 30]

### Synthesis of (1R,3R)-5-(2-((1R,3aS,7aR,E)-1-((S)-1-((S)-3-fluoropyrrolidin-1-yl)propan-2-yl)-7a-methyloctahydro-4H-inden-4-ylidene)ethylidene)cyclohexane-1,3-diol (Compound B012)

Compound B012 [8.9 mg, 0.022 mmol] was obtained by performing the reaction in the same manner as in Example 1 using compound (2b) [50 mg, 0.070 mmol] and 3-(S)-fluoropyrrolidine hydrochloride [30 mg, 0.239 mmol].
¹H-NMR (CD₃OD) δ: 6.20 (1H, d, J = 11.0 Hz), 5.87 (1H, d, J = 11.4 Hz), 5.19-5.02 (1H, m), 4.05-3.93 (2H, m), 2.84-2.65 (4H, m), 2.59-2.54 (1H, m), 2.39 (1H, dd, J = 13.3, 3.2 Hz), , 1.04 (3H, d, J = 6.4 Hz), 0.93 (1H, t, J = 7.3 Hz), 0.58 (3H, s).
Exact Mass = 405.30 (C₂₅H₄₀FNO₂) Obs. mass = 406.30 (M+H)

### [Example 31]

### Synthesis of (1R,3R)-5-(2-((1R,3aS,7aR,E)-1-((S)-1-((R)-3-fluoropyrrolidin-1-yl)propan-2-yl)-7a-methyloctahydro-4H-inden-4-ylidene)ethylidene)cyclohexane-1,3-diol (Compound B013)

Compound B013 [8.4 mg, 0.021 mmol] was obtained by performing the reaction in the same manner as in Example 1 using compound (2b) [50 mg, 0.070 mmol] and 3-(R)-fluoropyrrolidine hydrochloride [30 mg, 0.239 mmol].
¹H-NMR (CD₃OD) δ: 6.20 (1H, d, J = 11.0 Hz), 5.87 (1H, d, J = 11.0 Hz), 5.20-5.03 (1H, m), 4.04-3.93 (2H, m), 2.92-1.47 (25H, m), 1.38-1.23 (4H, m), 1.04 (3H, d, J = 6.9 Hz), 0.58 (3H, s).
Exact Mass = 405.30 (C₂₅H₄₀FNO₂) Obs. mass = 406.30 (M+H)

### [Example 32]

### Synthesis of (1R,3S,Z)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)-4-methylenecyclohexane-1,3-diol (Compound B020)

Compound B020 [22.8 mg, 0.051 mmol] was obtained by performing the reaction in the same manner as in Example 1 using compound (2a) [90 mg, 0.123 mmol] and 3-(S)-(difluoromethyl)pyrrolidine hydrochloride [60 mg, 0.381 mmol].
¹H-NMR (CD₃OD) δ: 6.32 (1H, d, J = 11.2 Hz), 6.09 (1H, d, J = 11.2 Hz), 5.89 (1H, td, J = 56.7, 4.7 Hz), 5.28 (1H, dd, J = 2.4, 1.5 Hz), 4.34 (1H, t, J = 5.9 Hz), 4.15-4.09 (1H, m), 3.21 (1H, dd, J = 10.5, 8.5 Hz), 3.00-2.00 (14H, m), 1.90-1.27 (13H, m), 1.09 (3H, d, J = 6.3 Hz), 0.62 (3H, s).
Exact Mass = 449.31 (C₂₇H₄₁F₂NO₂) Obs. mass = 450.30 (M+H)

### [Example 33]

### Synthesis of (1R,2S,3S,Z)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)-2-methyl-4-methylenecyclohexane-1,3-diol (Compound B021)

Compound B021 [3.8 mg, 0.008 mmol] was obtained by performing the reaction in the same manner as in Example 1 using compound (2g) [50 mg, 0.067 mmol] and 3-(S)-(difluoromethyl)pyrrolidine hydrochloride [35 mg, 0.222 mmol].
¹H-NMR (CD₃OD) δ: 6.33 (1H, d, J = 11.2 Hz), 6.09 (1H, d, J = 11.2 Hz), 5.81 (1H, td, J = 57.0, 5.7 Hz), 5.22 (1H, d, J = 2.0 Hz), 4.88 (1H, d, J = 2.0 Hz), 4.22 (1H, d, J = 3.4 Hz), 3.72 (1H, td, J = 8.2, 4.2 Hz), 2.98-2.83 (2H, m), 2.76-2.52 (5H, m), 2.44 (2H, d, J = 7.3 Hz), 2.17 (1H, dd, J = 13.2, 8.3 Hz), 2.07-1.93 (3H, m), 1.84-1.24 (11H, m), 1.06 (3H, d, J = 6.3 Hz), 1.03 (3H, d, J = 6.8 Hz), 0.59 (3H, s).
Exact Mass = 463.32 (C₂₈H₄₃F₂NO₂) Obs. mass = 464.35 (M+H)

### [Example 34]

### Synthesis of (1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)cyclohexane-1,3-diol (Compound B022)

Compound B022 [10.5 mg, 0.024 mmol] was obtained by performing the reaction in the same manner as in Example 1 using compound (2b) [55 mg, 0.077 mmol] and 3-(S)-(difluoromethyl)pyrrolidine hydrochloride [25 mg, 0.208 mmol].
¹H-NMR (CD₃OD) δ: 6.21 (1H, d, J = 10.7 Hz), 5.89 (1H, d, J = 10.7 Hz), 5.78 (1H, td, J = 57.0, 6.0 Hz), 4.06-3.95 (2H, m), 2.82 (2H, dd, J = 18.8, 10.0 Hz), 2.66-2.50 (4H, m), 2.44-1.92 (11H, m), 1.86-1.23 (13H, m), 1.05 (3H, d, J = 6.3 Hz), 0.60 (3H, s).
Exact Mass = 437.31 (C₂₆H₄₁F₂NO₂) Obs. mass = 438.35 (M+H)

### [Example 35]

### Synthesis of (1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)-2-methylenecyclohexane-1,3-diol (Compound B023)

Compound B023 [9.0 mg, 0.020 mmol] was obtained by performing the reaction in the same manner as in Example 1 using compound (2c) [50 mg, 0.069 mmol] and 3-(S)-(difluoromethyl)pyrrolidine hydrochloride [33 mg, 0.209 mmol].
¹H-NMR (CD₃OD) δ: 6.27 (1H, d, J = 10.7 Hz), 6.05-5.76 (2H, m), 5.05 (2H, d, J = 6.3 Hz), 4.42-4.36 (2H, m), 3.25 (1H, t, J = 9.5 Hz), 3.03 (2H, t, J = 7.1 Hz), 2.91-2.72 (5H, m), 2.66 (1H, dd, J = 13.7, 4.4 Hz), 2.48 (1H, dd, J = 13.4, 4.1 Hz), 2.32-1.96 (7H, m), 1.93-1.29 (10H, m), 1.10 (3H, d, J = 6.3 Hz), 0.63 (3H, s).
Exact Mass = 449.31 (C₂₇H₄₁F₂NO₂) Obs. mass = 450.30 (M+H)

### [Example 36]

### Synthesis of (1R,3S,Z)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)-4-methylenecyclohexane-1,3-diol (Compound B024)

Compound B024 [7.2 mg, 0.016 mmol] was obtained by performing the reaction in the same manner as in Example 1 using compound (2a) [90 mg, 0.123 mmol] and 3-(R)-(difluoromethyl)pyrrolidine hydrochloride [45 mg, 0.372 mmol].
¹H-NMR (CD₃OD) δ: 6.32 (1H, d, J = 11.2 Hz), 6.08 (1H, d, J = 11.2 Hz), 5.75 (1H, td, J = 57.0, 5.0 Hz), 5.28 (1H, dd, J = 2.4, 1.5 Hz), 4.89 (1H, dd, J = 2.4, 1.0 Hz), 4.34 (1H, t, J = 5.9 Hz), 4.15-4.09 (1H, m), 2.86 (1H, dd, J = 12.2, 3.4 Hz), 2.61-1.19 (27H, m), 1.04 (3H, d, J = 6.3 Hz), 0.59 (3H, s).
Exact Mass = 449.31 (C₂₇H₄₁F₂NO₂) Obs. mass = 450.45 (M+H)

### [Example 37]

### Synthesis of (1R,2S,3S,Z)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)-2-methyl-4-methylenecyclohexane-1,3-diol (Compound B0025)

Compound B025 [7.0 mg, 0.015 mmol] was obtained by performing the reaction in the same manner as in Example 1 using compound (2g) [50 mg, 0.067 mmol] and 3-(R)-(difluoromethyl)pyrrolidine hydrochloride [25 mg, 0.206 mmol].
¹H-NMR (CD₃OD) δ: 6.33 (1H, d, J = 11.2 Hz), 6.09 (1H, d, J = 10.7 Hz), 5.75 (1H, td, J = 57.3, 5.2 Hz), 5.22 (1H, d, J = 1.5 Hz), 4.89 (1H, s), 4.22 (1H, d, J = 3.4 Hz), 3.72 (1H, td, J= 8.1, 4.4 Hz), 2.86 (1H, dd, J= 11.0, 4.0 Hz), 2.63-1.24 (29H, m), 1.04 (3H, d, J = 7.3 Hz), 1.04 (3H, d, J = 6.8 Hz), 0.58 (3H, s).
Exact Mass = 463.32 (C₂₈H₄₃F₂NO₂) Obs. mass = 464.35 (M+H)

### [Example 38]

### Synthesis of (1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)cyclohexane-1,3-diol (Compound B026)

Compound B026 [7.2 mg, 0.016 mmol] was obtained by performing the reaction in the same manner as in Example 1 using compound (2b) [55 mg, 0.077 mmol] and 3-(R)-(difluoromethyl)pyrrolidine hydrochloride [27 mg, 0.226 mmol].
¹H-NMR (CD₃OD) δ: 6.22 (1H, d, J = 10.7 Hz), 5.89 (1H, d, J = 10.7 Hz), 5.76 (1H, td, J = 57.0, 5.0 Hz), 4.06-3.96 (2H, m), 2.84 (1H, dd, J = 12.0, 3.8 Hz), 2.65-2.52 (5H, m), 2.47-1.49 (21H, m), 1.40-1.22 (4H, m), 1.05 (3H, d, J = 6.3 Hz), 0.60 (3H, s).
Exact Mass = 437.31 (C₂₆H₄₁F₂NO₂) Obs. mass = 438.3 (M+H)

### [Example 39]

### Synthesis of (1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)-2-methylenecyclohexane-1,3-diol (Compound B027)

Compound B027 [11.0 mg, 0.025 mmol] was obtained by performing the reaction in the same manner as in Example 1 using compound (2c) [50 mg, 0.069 mmol] and 3-(R)-(difluoromethyl)pyrrolidine hydrochloride [25 mg, 0.206 mmol].
¹H-NMR (CD₃OD) δ: 6.26 (1H, d, J = 11.2 Hz), 5.91 (1H, d, J = 11.2 Hz), 5.77 (1H, td, J = 57.0, 5.0 Hz), 5.05 (2H, d, J = 6.3 Hz), 4.43-4.34 (2H, m), 2.85 (1H, dd, J = 12.2, 3.9 Hz), 2.71-2.25 (11H, m), 2.07-1.92 (4H, m), 1.83-1.24 (10H, m), 1.05 (3H, d, J = 6.3 Hz), 0.61 (3H, s).
Exact Mass = 449.31 (C₂₇H₄₁F₂NO₂) Obs. mass = 450.45 (M+H)

### [Reference example 5]

### Synthesis of 3-(S)-(1,1-difluoroethyl)pyrrolidine hydrochloride (Compound 3b6)

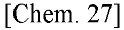

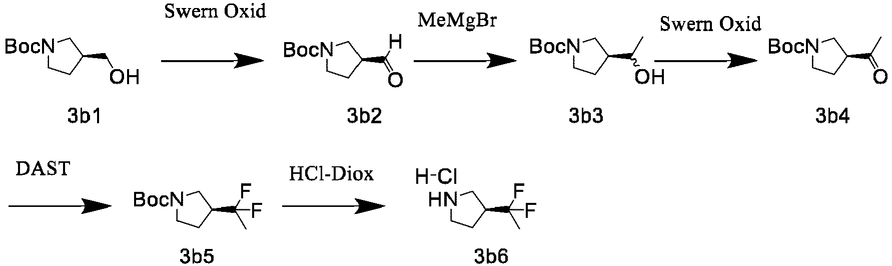

### Step 1

Under cooling to -78°C, a solution of dimethyl sulfoxide [4.1 mL, 58 mmol] in dichloromethane [10 mL] was added to a solution of oxalyl chloride [2.45 mL, 28.6 mmol] in dichloromethane [25 mL] and the mixture was stirred at the same temperature for 10 minutes. A solution of t-butyl (S)-3-(hydroxymethyl)pyrrolidine-1-carboxylate [3.84 g, 19.1 mmol] in dichloromethane [15 mL] was added to the mixture. The mixture was stirred at -78°C for 45 minutes. Triethylamine [15 mL, 92 mmol] was added to the mixture and the mixture was further stirred at the same temperature for 30 minutes. The reaction mixture was warmed to 0°C and stirred at the same temperature for 30 minutes. The reaction system was quenched with water and extracted with dichloromethane. The organic layer was dried over anhydrous magnesium sulfate and concentrated under reduced pressure. The residue was purified by silica gel column chromatography to obtain t-butyl (S)-3-formylpyrrolidine-1-carboxylate (Compound 3b2) [3.78 g, 19.0 mmol] (Yield = 99%).

¹H-NMR(CDCl₃) δ:9.69 (1H, J = 1.5 Hz), 3.72-3.65 (1H, m), 3.53-3.35 (3H, m), 3.03 (1H, s), 2.25-2.05 (2H, m), 1.46 (9H, s).

### Step 2

Methylmagnesium bromide [1 M in THF, 15.1 mL, 15.1 mmol] was added to a solution of t-butyl (S)-3-formylpyrrolidine-1-carboxylate (Compound 3b2) [2.00 g, 10 mmol] in THF [40 mL] at -78°C under a nitrogen atmosphere, and the reaction mixture was stirred at the same temperature for 1 hour. The reaction system was warmed to 0°C and was further stirred for 1 hour. The reaction mixture was quenched with a saturated aqueous ammonium chloride solution and extracted with ethyl acetate. The organic layer was dried over anhydrous magnesium sulfate and concentrated under reduced pressure. The residue was purified by silica gel column chromatography to obtain t-butyl (S)-3-(1-hydroxyethyl)pyrrolidine-1-carboxylate (Compound 3b3) [1.60 g, 7.41 mmol] (Yield = 74%).

¹H-NMR(CDCl₃) δ: 3.70-2.90 (5H, m), 2.15-2.03 (1H, m), 1.46 (9H, s), 1.30-1.21 (4H, m).

### Step 3

Under cooling to -78°C, a solution of dimethyl sulfoxide [1.6 mL, 23 mmol] in dichloromethane [10 mL] was added to a solution of oxalyl chloride [1 mL, 11.7 mmol] in dichloromethane [10 mL] and the mixture was stirred at the same temperature for 10 minutes. A solution of t-butyl (S)-3-(1-hydroxyethyl)pyrrolidine-1-carboxylate (Compound 3b3) [1.60 g, 7.41 mmol] in dichloromethane [10 mL] was added to the mixture. The mixture was stirred at -78°C for 45 minutes. Triethylamine [4.2 mL, 30 mmol] was added to the mixture and the mixture was further stirred at the same temperature for 30 minutes. The reaction mixture was warmed to 0°C and stirred at the same temperature for 30 minutes. The reaction mixture was quenched with water and extracted with dichloromethane. The organic layer was dried over anhydrous magnesium sulfate and concentrated under reduced pressure. The residue was purified by silica gel column chromatography to obtain t-butyl (S)-3-acetylpyrrolidine-1-carboxylate (Compound 3b4) [1.45 g, 6.80 mmol] (Yield = 91%).

¹H-NMR(CDCl₃) δ: 3.65-3.10 (5H, m), 3.14 (1H, s), 2.08 (3H, s), 2.05-1.95 (2H, m), 1.46 (9H, s).

### Step 4

A solution of t-butyl (S)-3-acetylpyrrolidine-1-carboxylate (Compound 3b4) [1.10 g, 5.16 mmol] in dichloromethane [20 mL] was cooled to 0°C. DAST [0.93 mL, 2.5 mmol] was added thereto. The reaction mixture was stirred overnight and warmed to room temperature. The reaction mixture was ice-cooled and carefully quenched with water. The mixture was extracted with dichloromethane at room temperature and the organic layer was washed with a saturated aqueous sodium hydrogen carbonate solution. The organic layer was dried over anhydrous magnesium sulfate and concentrated under reduced pressure, and the residue was purified by silica gel column chromatography to obtain t-butyl (S)-3-(1,1-difluoroethyl)pyrrolidine-1-carboxylate (Compound 3b5) [0.58 g, 2.5 mmol] (Yield = 48%).

### Step 5

A solution of the above t-butyl (S)-3-(1, 1-difluoroethyl)pyrrolidine-1-carboxylate (Compound 3b5) [0.58 g, 2.5 mmol] in 4 M hydrogen chloride in-dioxane [10 mL] was stirred at room temperature overnight. The reaction mixture was concentrated under reduced pressure to obtain 3-(S)-(1,1-difluoroethyl)pyrrolidine hydrochloride (Compound 3b6) [354 mg, 2.06 mmol].

¹H-NMR(DMSO-d6) δ: 9.40 (2H, s), 3.40-3.0 (4H, m), 2.92-2.38 (1H, m), 2.05-2.00 (1H, m), 1.90-1.80 (1H, m), 1.65 (3H, t, J = 20 Hz).

### [Reference example 6]

### Synthesis of 3-(R)-(1,1-difluoroethyl)pyrrolidine hydrochloride (3b12)

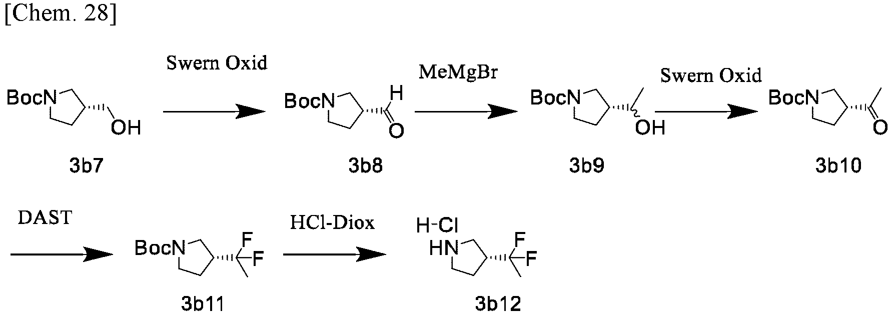

### Step 1

t-Butyl (R)-3-formylpyrrolidine-1-carboxylate (Compound 3b8) [2.42 g, 12.1 mmol] was obtained by performing the reaction in the same manner as in step 1 of Reference example 5 using t-butyl (R)-3-(hydroxymethyl)pyrrolidine-1-carboxylate (3b7) [3.16 g, 15.7 mmol] as a starting material.

### Step 2

t-Butyl (R)-3-(1-hydroxyethyl)pyrrolidine-1-carboxylate (Compound 3b9) [1.64 g, 7.62 mmol] was obtained by performing the reaction in the same manner as in step 2 of Reference example 5 using t-butyl (R)-3-formylpyrrolidine-1-carboxylate (Compound 3b8) [2.42 g, 12.1 mmol] as a starting material.

### Step 3

t-Butyl (R)-3-acetylpyrrolidine-1-carboxylate (Compound 3b10) [1.50 g, 7.03 mmol]was obtained by performing the reaction in the same manner as in step 3 of Reference example 5 using t-butyl (R)-3-(1-hydroxyethyl)pyrrolidine-1-carboxylate (Compound 3b9) [1.64 g, 7.62 mmol] as a starting material. (Yield = 92%)

### Step 4

DAST [1.8 mL, 13.6 mmol] was added to a solution of t-butyl (R)-3-acetylpyrrolidine-1-carboxylate (Compound 3b10) [1.50 g, 7.03 mmol] in dichloromethane [30 mL] at 0°C and the mixture was stirred at room temperature overnight. The next morning, DAST [1.8 mL, 13.6 mmol] was further added to the mixture, and the mixture was further stirred at room temperature for 1 day. The reaction mixture was cooled to 0°C, quenched with a saturated aqueous sodium carbonate solution and extracted with dichloromethane. The organic layer was dried over anhydrous magnesium sulfate and concentrated under reduced pressure. The residue was purified by silica gel column chromatography to obtain t-butyl (R)-3-(1,1-difluoroethyl)pyrrolidine-1-carboxylate (Compound 3b11) [0.87 g, 3.7 mmol] (Yield = 53%).

### Step 5

3-(R)-(1,1-difluoroethyl)pyrrolidine hydrochloride (3b12) [0.63 g, 3.7 mmol] was obtained by performing the reaction in the same manner as in step 5 of Reference example 5 using t-butyl (R)-3-(1,1-difluoroethyl)pyrrolidine-1-carboxylate (Compound 3b11) [0.87 g, 3.7 mmol] as a starting material. (Yield = 100%)

¹H-NMR (DMSO-D₆) δ: 9.60 (1H, br s), 9.40 (1H, br s), 3.37-3.30 (1H, br m), 3.25-3.00 (3H, br m), 2.96-2.81 (1H, m), 2.12-2.03 (1H, m), 1.91-1.77 (1H, m), 1.66 (3H, t, J = 19.3 Hz).

### [Example 40]

### Synthesis of (1R,3S,Z)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)-4-methylenecyclohexane-1,3-diol (Compound B028)

Compound B028 [12.7 mg, 0.027 mmol] was obtained by performing the reaction in the same manner as in Example 1 using compound (2a) [90 mg, 0.123 mmol] and 3-(S)-(1,1-difluoroethyl)pyrrolidine hydrochloride (3b6) [45 mg, 0.262 mmol].

¹H-NMR (CD₃OD) δ: 6.31 (1H, d, J = 10.7 Hz), 6.09 (1H, d, J = 11.2 Hz), 5.28 (1H, dd, J = 2.4, 1.0 Hz), 4.33 (1H, d, J = 5.4 Hz), 4.14-4.09 (1H, m), 3.43 (1H, dd, J = 10.7, 8.3 Hz), 3.25-2.78 (8H, m), 2.51 (1H, dd, J = 13.7, 3.4 Hz), 2.28-1.97 (7H, m), 1.92-1.28 (17H, m), 1.11 (3H, d, J = 6.3 Hz), 0.63 (3H, d, J = 7.3 Hz).

### [Example 41]

### Synthesis of (1R,2S,3S,Z)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B029)

Compound B029 [5.8 mg, 0.012 mmol] was obtained by performing the reaction in the same manner as in Example 1 using compound (2g) [50 mg, 0.067 mmol] and 3-(S)-(1,1-difluoroethyl)pyrrolidine hydrochloride (3b6) [35 mg, 0.204 mmol].
¹H-NMR (CD₃OD) δ: 6.33 (1H, d, J = 11.2 Hz), 6.09 (1H, d, J = 11.2 Hz), 5.22 (1H, d, J = 1.5 Hz), 4.89 (1H, d, J = 1.5 Hz), 4.22 (1H, d, J = 3.4 Hz), 3.72 (1H, td, J = 8.2, 4.2 Hz), 3.01 (1H, t, J = 9.0 Hz), 2.87 (1H, dd, J = 12.0, 3.7 Hz), 2.82-2.38 (8H, m), 2.17 (1H, dd, J = 13.2, 8.3 Hz), 2.06-1.94 (4H, m), 1.88-1.43 (13H, m), 1.39-1.24 (3H, m), 1.06 (3H, d, J = 6.8 Hz), 1.04 (3H, d, J = 7.3 Hz), 0.59 (3H, s).
Exact Mass = 477.34 (C₂₉H₄₅F₂NO₂) Obs. mass = 478.45 (M+H)

### [Example 42]

### Synthesis of (1R,3R)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)cyclohexane-1,3-diol (Compound B030)

Compound B030 [13.5 mg, 0.030 mmol] was obtained by performing the reaction in the same manner as in Example 1 using compound (2b) [55 mg, 0.077 mmol] and 3-(S)-(1,1-difluoroethyl)pyrrolidine hydrochloride (3b6) [29.2 mg, 0.216 mmol]. ¹H-NMR (CD₃OD) δ: 6.21 (1H, d, J = 11.2 Hz), 5.89 (1H, d, J = 11.2 Hz), 4.06-3.95 (2H, m), 2.93 (1H, t, J = 9.0 Hz), 2.83 (1H, dd, J = 12.0, 3.2 Hz), 2.73-2.50 (4H, m), 2.42-2.31 (4H, m), 2.23-1.47 (20H, m), 1.41-1.22 (3H, m), 1.06 (3H, d, J = 6.3 Hz), 0.60 (3H, s). Exact Mass = 451.33 (C₂₇H₄₃F₂NO₂) Obs. mass = 452.45 (M+H)

### [Example 43]

### Synthesis of (1R,3R)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)-2methylenecyclohexane-1,3-diol (Compound B031)

Compound B031 [12.9 mg, 0.028 mmol] was obtained by performing the reaction in the same manner as in Example 1 using compound (2c) [50 mg, 0.069 mmol] and 3-(S)-(1,1-difluoroethyl)pyrrolidine hydrochloride (3b6) [35 mg, 0.204 mmol].
¹H-NMR (CD₃OD) δ: 6.26 (1H, d, J = 11.2 Hz), 5.92 (1H, d, J = 11.2 Hz), 5.05 (2H, d, J = 6.3 Hz), 4.43-4.34 (2H, m), 3.42 (1H, dd, J = 10.7, 8.3 Hz), 3.24-2.78 (7H, m), 2.66 (1H, dd, J = 13.2, 4.4 Hz), 2.48 (1H, dd, J = 13.2, 3.9 Hz), 2.32-1.97 (7H, m), 1.83-1.30 (13H, m), 1.12 (3H, d, J = 6.8 Hz), 0.64 (3H, s).
Exact Mass = 463.33 (C₂₈H₄₃F₂NO₂) Obs. mass = 464.3 (M+H)

### [Example 44]

### Synthesis of (1R,3S,Z)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)-4-methylenecyclohexane-1,3-diol (Compound B032)

Compound B032 [12.7 mg, 0.027 mmol] was obtained by performing the reaction in the same manner as in Example 1 using compound (2a) [90 mg, 0.123 mmol] and 3-(R)-(1,1-difluoroethyl)pyrrolidine hydrochloride (3b12) [50 mg, 0.291 mmol].
¹H-NMR (CD₃OD) δ: 6.32 (1H, d, J = 11.2 Hz), 6.09 (1H, d, J = 11.2 Hz), 5.28 (1H, dd, J = 2.2, 1.2 Hz), 4.34 (1H, t, J = 5.9 Hz), 4.15-4.10 (1H, m), 3.12-3.00 (2H, m), 2.94-2.75 (5H, m), 2.66 (2H, d, J = 7.3 Hz), 2.51 (1H, dd, J = 13.4, 3.2 Hz), 2.26 (1H, dd, J = 13.7, 6.8 Hz), 2.13-1.94 (5H, m), 1.90-1.23 (16H, m), 1.09 (3H, d, J = 6.3 Hz), 0.62 (3H, s).
Exact Mass = 463.33 (C₂₈H₄₃F₂NO₂) Obs. mass = 464.45 (M+H)

### [Example 45]

### Synthesis of (1R,2S,3S,Z)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B033)

Compound B033 [6.8 mg, 0.014 mmol] was obtained by performing the reaction in the same manner as in Example 1 using compound (2g) [50 mg, 0.067 mmol] and 3-(R)-(1,1-difluoroethyl)pyrrolidine hydrochloride (3b12) [25 mg, 0.146 mmol]. ¹H-NMR (CD₃OD) δ: 6.33 (1H, d, J = 11.2 Hz), 6.09 (1H, d, J = 11.2 Hz), 5.22 (1H, d, J = 2.0 Hz), 4.22 (1H, d, J = 3.4 Hz), 3.72 (1H, td, J = 8.2, 4.2 Hz), 2.89-1.94 (16H, m), 1.88-1.22 (16H, m), 1.05 (6H, d, J = 6.8 Hz), 1.03 (6H, d, J = 6.8 Hz), 0.59 (3H, s). Exact Mass = 477.34 (C₂₉H₄₅F₂NO₂) Obs. mass = 478.5 (M+H)

### [Example 46]

### Synthesis of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (Compound B034)

Compound B034 [11.8 mg, 0.026 mmol] was obtained by performing the reaction in the same manner as in Example 1 using compound (2b) [55 mg, 0.077 mmol] and 3-(R)-(1,1-difluoroethyl)pyrrolidine hydrochloride (3b12) [28.2 mg, 0.164 mmol].
¹H-NMR (CD₃OD) δ: 6.21 (1H, d, J = 10.7 Hz), 5.89 (1H, d, J = 10.7 Hz), 4.06-3.95 (2H, m), 2.83 (1H, dd, J= 12.0, 3.7 Hz), 2.74-2.50 (5H, m), 2.46-2.12 (7H, m), 2.07-1.47 (17H, m), 1.35 (2H, ddd, J = 23.4, 10.7, 2.9 Hz), 1.26 (1H, dd, J = 18.5, 9.3 Hz), 1.05 (3H, d, J = 6.8 Hz), 0.60 (3H, s).
Exact Mass = 451.33 (C₂₇H₄₃F₂NO₂) Obs. mass = 452.3 (M+H)

### [Example 47]

### Synthesis of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)-2-methylenecyclohexane-1,3-diol (Compound B035)

Compound B035 [12.9 mg, 0.028 mmol] was obtained by performing the reaction in the same manner as in Example 1 using compound (2c) [50 mg, 0.069 mmol] and 3-(R)-(1,1-difluoroethyl)pyrrolidine hydrochloride (3b12) [26 mg, 0.152 mmol].
¹H-NMR (CD₃OD) δ: 6.26 (1H, d, J = 11.2 Hz), 5.92 (1H, d, J = 11.2 Hz), 5.05 (2H, d, J = 6.3 Hz), 4.43-4.34 (2H, m), 3.17-3.05 (2H, m), 2.99-2.64 (8H, m), 2.48 (1H, dd, J = 13.2, 3.9 Hz), 2.31-2.25 (2H, m), 2.16-1.96 (5H, m), 1.78-1.28 (13H, m), 1.10 (3H, d, J = 6.3 Hz), 0.63 (3H, s).
Exact Mass = 463.33 (C₂₈H₄₃F₂NO₂) Obs. mass = 464.5 (M+H)

### [Reference example 7]

### Synthesis of 3-(R)-(2,2-difluoroethyl)pyrrolidine hydrochloride (3b16)

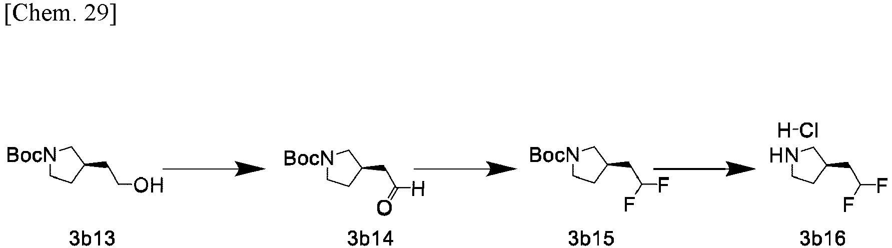

### Step 1

Under cooling to -78°C, a solution of dimethyl sulfoxide [1.0 mL, 14 mmol] in dichloromethane [10 mL] was added to a solution of oxalyl chloride [0.66 mL, 7.7 mmol] in dichloromethane [10 mL], and the mixture was stirred at the same temperature for 10 minutes. A solution of t-butyl-3-(R)-(2-hydroxyethyl)pyrrolidine-1-carboxylate (Compound 3b13) [1.0 g, 4.64 mmol] in dichloromethane [10 mL] was added to the mixture. The mixture was stirred at -78°C for 1 hour. Triethylamine [3.3 mL, 23 mmol] was added to the mixture and the mixture was further stirred at the same temperature for 40 minutes. The reaction mixture was warmed to 0°C and stirred at the same temperature for 30 minutes. The reaction mixture was quenched with water and extracted with ethyl acetate. The organic layer was dried over anhydrous magnesium sulfate and concentrated under reduced pressure. The residue was purified by silica gel column chromatography to obtain t-butyl-3-(R)-(2-oxoethyl)pyrrolidine-1-carboxylate (Compound 3b14) [0.85 g, 4.0 mmol] (Yield = 86%).

### Step 2

DAST [0.7 mL, 5 mmol] was added to a solution of t-butyl-3-(R)-(2-oxoethyl)pyrrolidine-1-carboxylate (Compound 3b14) [0.85 g, 4.0 mmol] in dichloromethane [20 mL] at 0°C, and the mixture was stirred at room temperature overnight. The reaction mixture was cooled to 0°C, quenched with water, and extracted with dichloromethane. The organic layer was washed with a saturated aqueous sodium hydrogen carbonate solution, dried over anhydrous magnesium sulfate, and concentrated under reduced pressure. The residue was purified by silica gel column chromatography to obtain t-butyl-3-(R)-(2,2-difluoroethyl)pyrroliine-1-carboxylate (Compound 3b15) [799 mg, 3.4 mmol] (Yield = 85%).

### Step 3

A solution of t-butyl-3-(R)-(2,2-difluoroethyl)pyrrolidine-1-carboxylate (Compound 3b15) [799 mg, 3.4 mmol] in 4 M hydrogen chloride in dioxane [6 mL] was stirred at room temperature overnight. The reaction mixture was concentrated under reduced pressure and dried to obtain 3-(R)-(2,2-difluoroethyl)pyrrolidine hydrochloride (Compound 3b16) [562.7 mmol] (Yield = 96%).

¹H-NMR (DMSO-d6) δ: 9.88 (2H, br s), 5.90 (1H, tt, J = 55.9, 3.8 Hz), 3.53 (2H, d, J = 40.5 Hz), 3.29 (1H, br s), 2.95 (1H, br s), 2.62-2.54 (1H, m), 2.33-2.20 (1H, m), 2.11-1.99 (2H, m), 1.80-1.65 (1H, m).

### [Reference example 8]

### Synthesis of 3-(S)-(2,2-difluoroethyl)pyrrolidine hydrochloride (3b20)

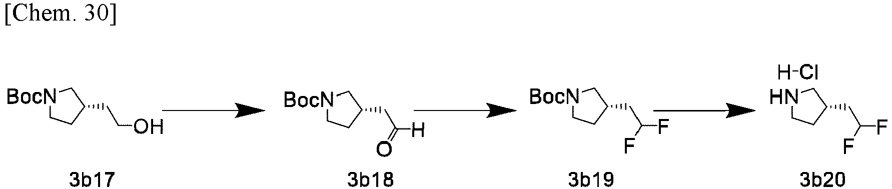

### Step 1

t-Butyl-3-(S)-(2-oxoethyl)pyrrolidine-1-carboxylate (Compound 3b18) [0.72 g, 3.4 mmol] was obtained by performing the reaction in the same manner as in step 1 of Reference example 7 using t-butyl-3-(S)-(2-hydroxyethyl)pyrrolidine-1-carboxylate (Compound 3b17) [1.0 g, 4.64 mmol] as a starting material. (Yield = 73%)

### Step 2

t-Butyl-3-(S)-(2,2-difluoroethyl)pyrrolidine-1-carboxylate (Compound 3b19) [679 mg, 2.89 mmol] was obtained by performing the reaction in the same manner as in step 2 of Reference example 7 using t-butyl-3-(S)-(2-oxoethyl)pyrrolidine-1-carboxylate (Compound 3b18) [0.72 g, 3.4 mmol] as a starting material. (Yield = 62%)

### Step 3

3-(S)-(2,2-Difluoroethyl)pyrrolidine hydrochloride (Compound 3b20) [524.9 mg, 3.06 mmol] was obtained by performing the reaction in the same manner as in step 3 of Reference example 7 using t-butyl-3-(S)-(2,2-difluoroethyl)pyrrolidine-1-carboxylate (Compound 3b19) [679 mg, 2.89 mmol] as a starting material. (Yield = quant)

¹H-NMR (DMSO-d6) δ: 9.86 (2H, br s), 5.91 (1H, tt, J = 55.9, 3.7 Hz), 3.53 (2H, d, J = 38.5 Hz), 3.29 (1H, br s), 2.95 (1H, br s), 2.65-2.50 (1H, m), 2.35-2.20 (1H, m), 2.15-2.00 (2H, m), 1.80-1.65 (1H, m).

### [Example 48]

### Synthesis of (1R,3S,Z)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-4-methylcyclohexane-1,3-diol (Compound B036)

Compound B036 [17.2 mg, 0.037 mmol] was obtained by performing the reaction in the same manner as in Example 1 using compound (2a) [90 mg, 0.123 mmol] and 3-(R)-(2,2-difluoroethyl)pyrrolidine hydrochloride (3b16) [50 mg, 0.292 mmol].
¹H-NMR (CD₃OD) δ: 6.32 (1H, d, J = 10.7 Hz), 6.12-5.81 (2H, m), 5.28 (1H, d, J = 1.0 Hz), 4.34 (1H, t, J = 5.9 Hz), 4.15-4.10 (1H, m), 3.45 (1H, dd, J = 10.2, 7.8 Hz), 3.23-3.18 (2H, m), 2.95-2.80 (3H, m), 2.74 (1H, t, J = 10.2 Hz), 2.59-2.45 (2H, m), 2.30-1.90 (8H, m), 1.89-1.31 (14H, m), 1.11 (3H, d, J = 6.3 Hz), 0.63 (3H, s).
Exact Mass = 463.33 (C₂₈H₄₃F₂NO₂) Obs. mass = 464.5 (M+H)

### [Example 49]

### Synthesis of (1R,3S,Z)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)-4-methylenecyclohexane-1,3-diol (Compound B037)

Compound B037 [24.1 mg, 0.052 mmol] was obtained by performing the reaction in the same manner as in Example 1 using compound (2a) [90 mg, 0.123 mmol] and 3-(S)-(2,2-difluoroethyl)pyrrolidine hydrochloride (3b20) [50 mg, 0.292 mmol].
¹H-NMR (CD₃OD) δ: 6.32 (1H, d, J = 10.7 Hz), 6.09 (1H, d, J = 10.7 Hz), 5.98 (1H, tt, J = 3.0, 56.0 Hz), 5.29 (1H, t, J = 1.2 Hz), 4.34 (1H, t, J = 5.9 Hz), 4.12 (1H, td, J = 6.5, 3.3 Hz), 3.55 (1H, dd, J = 11.0, 8.1 Hz), 3.41-3.34 (2H, m), 3.08-2.93 (4H, m), 2.88 (1H, dd, J = 12.0, 3.7 Hz), 2.69-2.55 (1H, m), 2.51 (1H, dd, J = 13.7, 3.4 Hz), 2.35-2.04 (8H, m), 2.01-1.96 (3H, m), 1.90-1.33 (16H, m), 1.13 (3H, d, J = 6.8 Hz), 0.63 (3H, s).
Exact Mass = 463.33 (C₂₈H₄₃F₂NO₂) Obs. mass = 464.5 (M+H)

### [Example 50]

### Synthesis of (1R,2S,3S,Z)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B038)

Compound B038 [10.2 mg, 0.021 mmol] was obtained by performing the reaction in the same manner as in Example 1 using compound (2g) [55 mg, 0.074 mmol] and 3-(R)-(2,2-difluoroethyl)pyrrolidine hydrochloride (3b16) [40 mg, 0.184 mmol].
¹H-NMR (CD₃OD) δ: 6.33 (1H, d, J = 11.2 Hz), 6.09 (1H, d, J = 11.2 Hz), 5.88 (1H, tt, J = 56.6, 4.6 Hz), 5.22 (1H, d, J = 1.5 Hz), 4.22 (1H, d, J = 3.4 Hz), 3.72 (1H, td, J = 8.1, 4.2 Hz), 2.94 (1H, t, J = 8.3 Hz), 2.86 (1H, dd, J = 12.4, 4.1 Hz), 2.68-2.52 (3H, m), 2.33-2.03 (9H, m), 1.98-1.24 (20H, m), 1.05 (3H, d, J = 7.0 Hz), 1.03 (3H, d, J = 6.3 Hz), 0.96-0.87 (1H, m), 0.58 (3H, s).
Exact Mass = 477.34 (C₂₉H₄₅F₂NO₂) Obs. mass = 478.3 (M+H)

### [Example 51]

### Synthesis of (1R,2S,3S,Z)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B039)

Compound B039 [9.2 mg, 0.019 mmol] was obtained by performing the reaction in the same manner as in Example 1 using compound (2g) [55 mg, 0.074 mmol] and 3-(S)-(2,2-difluoroethyl)pyrrolidine hydrochloride (3b20) [40 mg, 0.184 mmol].
¹H-NMR (CD₃OD) δ: 6.33 (1H, d, J = 10.7 Hz), 6.09 (1H, d, J = 11.2 Hz), 5.87 (1H, tt, J = 56.6, 4.6 Hz), 5.22 (1H, d, J = 1.5 Hz), 4.22 (1H, d, J = 3.4 Hz), 3.72 (1H, td, J = 8.1, 4.4 Hz), 2.86 (1H, dd, J = 12.4, 4.1 Hz), 2.79-2.68 (2H, m), 2.59 (1H, dd, J = 13.4, 4.1 Hz), 2.39-1.21 (23H, m), 1.04 (3H, d, J = 6.3 Hz), 1.03 (3H, d, J = 7.0 Hz), 0.58 (3H, s).
Exact Mass = 477.34 (C₂₉H₄₅F₂NO₂) Obs. mass = 478.35 (M+H)

### [Example 52]

### Synthesis of (1R,3R)-5-(2-((1R,3 aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-2-methylenecyclohexane-1,3-diol (Compound B040)

Compound B040 [11.4 mg, 0.025 mmol] was obtained by performing the reaction in the same manner as in Example 1 using compound (2c) [50 mg, 0.069 mmol] and 3-(R)-(2,2-difluoroethyl)pyrrolidine hydrochloride (3b16) [30 mg, 0.175 mmol].
¹H-NMR (CD₃OD) δ: 6.26 (1H, d, J = 11.2 Hz), 5.96 (1H, tt, J = 56.5, 4.0 Hz), 5.92 (1H, d, J = 11.2 Hz), 5.05 (2H, d, J = 5.9 Hz), 4.39 (2H, ddd, J = 14.3, 7.2, 4.5 Hz), 3.44 (1H, dd, J = 10.7, 7.8 Hz), 3.24-3.12 (2H, m), 2.94-2.82 (3H, m), 2.74 (1H, t, J = 10.2 Hz), 2.66 (1H, dd, J = 13.2, 4.4 Hz), 2.56 (1H, q, J = 8.1 Hz), 2.48 (1H, dd, J = 13.7, 3.9 Hz), 2.34-2.17 (3H, m), 2.08-1.93 (5H, m), 1.83-1.28 (10H, m), 1.12 (3H, d, J = 6.3 Hz), 0.63 (3H, s).
Exact Mass = 463.32 (C₂₈H₄₃F₂NO₂) Obs. mass = 464.35 (M+H)

### [Example 53]

### Synthesis of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)-2-methylenecyclohexane-1,3-diol (Compound B041)

Compound B041 [11.8 mg, 0.026 mmol] was obtained by performing the reaction in the same manner as in Example 1 using compound (2c) [50 mg, 0.069 mmol] and 3-(S)-(2,2-difluoroethyl)pyrrolidine hydrochloride (3b20) [30 mg, 0.175 mmol].
¹H-NMR (CD₃OD) δ: 6.26 (1H, d, J = 11.2 Hz), 5.96 (2H, tt, J = 56.5, 4.0 Hz), 5.92 (2H, d, J = 11.2 Hz), 5.05 (2H, d, J = 5.9 Hz), 4.39 (2H, ddd, J = 14.4, 7.1, 4.4 Hz), 3.37 (1H, dd, J = 10.2, 8.3 Hz), 3.26-3.20 (1H, m), 3.15-3.05 (1H, m), 2.92-2.74 (4H, m), 2.66 (1H, dd, J = 13.4, 4.1 Hz), 2.62-2.53 (1H, m), 2.48 (1H, dd, J = 13.4, 4.1 Hz), 2.32-2.19 (3H, m), 2.08-1.92 (5H, m), 1.83-1.30 (10H, m), 1.11 (3H, d, J = 6.3 Hz), 0.63 (3H, s).
Exact Mass = 463.32 (C₂₈H₄₃F₂NO₂) Obs. mass = 464.40 (M+H)

### [Example 54]

### Synthesis of (1R,3R)-5-(2-((1R,3 aS,7aR,E)-1-((S)-1-((R)-3-(2,2-difluoroethyl)pyrrolidin-1-yl)propan-2-yl)-7a-methyloctahydro-4H-inden-4-ylidene)ethylidene)cyclohexane-1,3-diol (Compound B042)

Compound B042 [14.2 mg, 0.031 mmol] was obtained by performing the reaction in the same manner as in Example 1 using compound (2b) [50 mg, 0.070 mmol] and 3-(R)-(2,2-difluoroethyl)pyrrolidine hydrochloride (3b16) [30 mg, 0.175 mmol].
¹H-NMR (CD₃OD) δ: 6.21 (1H, d, J = 11.2 Hz), 5.92 (1H, tt, J = 56.5, 4.2 Hz), 5.89 (1H, d, J = 10.0 Hz), 4.06-3.95 (2H, m), 3.17 (1H, t, J = 8.3 Hz), 2.97-2.80 (3H, m), 2.61-1.91 (15H, m), 1.86-1.26 (13H, m), 1.08 (3H, d, J = 6.8 Hz), 0.61 (3H, s).
Exact Mass = 451.33 (C₂₇H₄₃F₂NO₂) Obs. mass = 452.30 (M+H)

### [Example 55]

### Synthesis of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol (Compound B043)

Compound B043 [8.3 mg, 0.018 mmol] was obtained by performing the reaction in the same manner as in Example 1 using compound (2b) [50 mg, 0.070 mmol] and 3-(S)-(2,2-difluoroethyl)pyrrolidine hydrochloride (3b20) [30 mg, 0.175 mmol].
¹H-NMR (CD₃OD) δ: 6.21 (1H, d, J = 10.7 Hz), 5.91 (1H, tt, J = 56.6, 4.0 Hz), 5.89 (1H, d, J = 10.7 Hz), 4.05-3.96 (2H, m), 3.05-2.80 (3H, m), 2.65-2.39 (8H, m), 2.23-1.90 (10H, m), 1.87-1.25 (14H, m), 1.07 (3H, d, J = 6.3 Hz), 0.61 (3H, s).
Exact Mass = 451.33 (C₂₇H₄₃F₂NO₂) Obs. mass = 452.25 (M+H)

### [Example 56]

### Synthesis of (1R,3S,Z)-5-(2-((1R,3aS,7aR,E)-1-((2S)-1-(3-hydroxy-3-(trifluoromethyl)pyrrolidin-1-yl)propan-2-yl)-7a-methyloctahydro-4H-inden-4-ylidene)ethylidene)-4-methylenecyclohexane-1,3-diol (Compound B044a, Compound B044b)

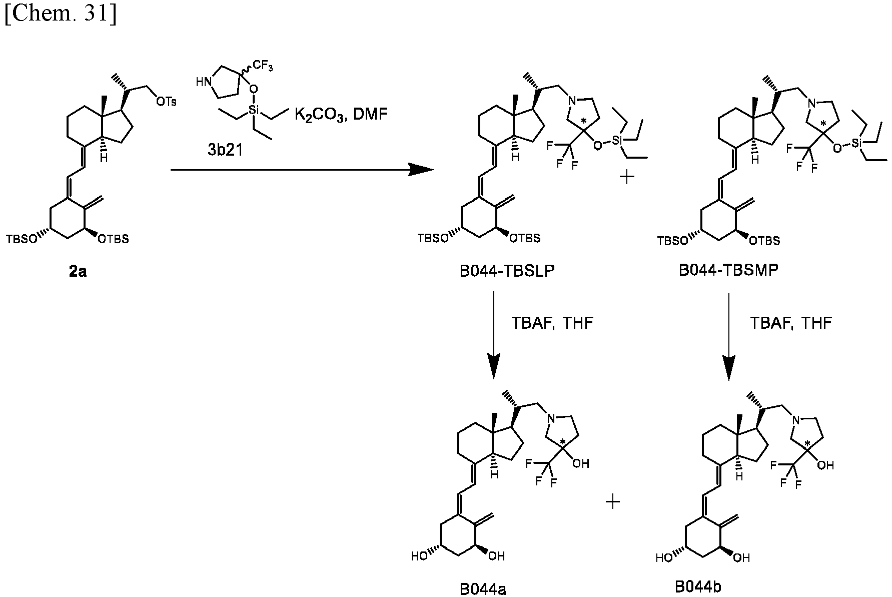

### Step 1

A solution of compound (2a) [100 mg, 0.137 mmol], triethyl[3-(trifluoromethyl)pyrrolidin-3-yl]oxysilane (Compound 3b21) [80 mg, 0.297 mmol], and potassium carbonate [60 mg, 0.434 mmol] in DMF [1 mL] was stirred at 60°C overnight. The reaction mixture was quenched with saturated brine and extracted with ethyl acetate. The organic layer was dried over anhydrous magnesium sulfate and concentrated under reduced pressure. The residue was purified by silica gel column chromatography to afford the target product with low polarity (Compound B044-TBSLP) [32.2 mg, 0.039 mmol] and the target product with high polarity (Compound B044-TBSMP) [35.5 mg, 0.043 mmol].

### Step 2a

TBAF [1 M in THF, 0.5 mL, 0.5 mmol] was added to a solution of compound B044-TBSLP [32.2 mg, 0.039 mmol] in THF [1 mL], and the mixture was stirred at 50°C overnight. The reaction mixture was quenched with saturated magnesium hydrogen carbonate and the organic layer was washed with saturated brine, dried over anhydrous magnesium sulfate, and concentrated under reduced pressure. The residue was purified by HPLC to obtain compound B044a [7.7 mg, 0.016 mmol].
¹H-NMR (CD₃OD) δ: 6.27 (1H, d, J = 11.2 Hz), 6.04 (1H, d, J = 11.2 Hz), 5.24 (1H, dd, J = 2.4, 1.5 Hz), 4.30 (1H, t, J = 6.0 Hz), 4.12-4.00 (1H, m), 2.92 (1H, d, J = 10.7 Hz), 2.84-2.73 (2H, m), 2.52-2.41 (3H, m), 2.28-2.08 (5H, m), 2.02-1.22 (18H, m), 1.00 (3H, d, J = 6.3 Hz), 0.55 (3H, s).
Exact Mass = 483.3 (C₂₇H₄₀F₃NO₃) Obs. mass = 484.3 (M+H)

### Step 2b

Compound B044-TBSMP [35.5 mg, 0.043 mmol] was processed in the same manner as in step 2a to obtain Compound B044b [10.0 mg, 0.021 mmol].
¹H-NMR (CD₃OD) δ: 6.27 (1H, d, J = 11.2 Hz), 6.04 (1H, d, J = 10.7 Hz), 5.24 (1H, dd, J = 2.4, 1.0 Hz), 4.30 (1H, t, J = 5.9 Hz), 4.08 (1H, dt, J = 10.9, 3.8 Hz), 2.84-2.41 (7H, m), 2.26-1.20 (24H, m), 1.00 (3H, d, J = 6.3 Hz), 0.55 (3H, s).
Exact Mass = 483.3 (C₂₇H₄₀F₃NO₃) Obs. mass = 484.3 (M+H)

### [Example 57]

### Synthesis of (1R,3R)-5-(2-((1R,3aS,7aR,E)-1-((2S)-1-(3-hydroxy-3-(trifluoromethyl)pyrrolidin-1-yl)propan-2-yl)-7a-methyloctahydro-4H-inden-4-ylidene)ethylidene)cyclohexane-1,3-diol (Compound B045a, Compound B045b)

Compound B045a [9.3 mg, 0.020 mmol] and compound B045b [7.9 mg, 0.017 mmol] were obtained by performing the reaction in the same manner as in Example 56 using compound 2b [100 mg, 0.139 mmol] and triethyl[3-(trifluoromethyl)pyrrolidin-3-yl]oxysilane (Compound 3b21) [100.4 mg, 0.373 mmol] as starting materials.

### Compound B045a

¹H-NMR (CD₃OD) δ: 6.21 (1H, d, J= 10.7 Hz), 5.88 (1H, d, J= 11.2 Hz), 4.06-3.94 (2H, m), 3.08 (1H, d, J = 11.2 Hz), 2.94 (1H, q, J = 8.0 Hz), 2.83 (1H, dd, J = 11.2, 4.4 Hz), 2.74-2.55 (3H, m), 2.48-2.37 (3H, m), 2.26-2.03 (6H, m), 2.02-1.95 (2H, m), 1.94-1.24 (15H, m), 1.05 (3H, d, J = 6.3 Hz), 0.60 (3H, s).
Exact Mass = 471.30 (C₂₆H₄₀F₃NO₃) Obs. mass = 472.25 (M+H)

### Compound B045b

¹H-NMR (CD₃OD) δ: 6.21 (1H, d, J= 10.7 Hz), 5.89 (1H, d, J= 11.2 Hz), 4.06-3.94 (2H, m), 2.85-2.75 (4H, m), 2.66 (1H, dd, J = 15.6, 7.3 Hz), 2.61-2.13 (8H, m), 2.10-1.98 (2H, m), 1.93-1.24 (13H, m), 1.06 (3H, d, J = 6.8 Hz), 0.60 (3H, s).
Exact Mass = 471.30 (C₂₆H₄₀F₃NO₃) Obs. mass = 472.25 (M+H)

### [Example 58]

### Synthesis of (1R,3R)-5-(2-((1R,3aS,7aR,E)-1-((2S)-1-(3-hydroxy-3-(trifluoromethyl)pyrrolidin-1-yl)propan-2-yl)-7a-methyloctahydro-4H-inden-4-ylidene)ethylidene)-2-methylenecyclohexane-1,3-diol (Compound B046a, Compound B046b)

Compound B046a [8.1 mg, 0.017 mmol] and compound B046b [5.6 mg, 0.012 mmol] were obtained by performing the reaction in the same manner as in Example 56 using compound 2c [100 mg, 0.137 mmol] and triethyl[3-(trifluoromethyl)pyrrolidin-3-yl]oxysilane (Compound 3b21) [144.3 mg, 0.536 mmol] as starting materials.

### Compound B046a

¹H-NMR (CD₃OD) δ: 6.26 (1H, d, J = 10.7 Hz), 5.91 (1H, d, J = 11.2 Hz), 5.05 (2H, d, J = 6.8 Hz), 4.43-4.35 (2H, m), 3.03 (1H, d, J = 11.2 Hz), 2.91-2.83 (2H, m), 2.69-2.17 (10H, m), 2.08-2.00 (2H, m), 1.95-1.85 (2H, m), 1.70-1.49 (6H, m), 1.40-1.25 (3H, m), 1.05 (3H, d, J = 6.3 Hz), 0.61 (3H, s).
Exact Mass = 483.3 (C₂₇H₄₀F₃NO₃) Obs. mass = 484.25 (M+H)

### Compound B046b

¹H-NMR (CD₃OD) δ: 6.26 (1H, d, J = 11.2 Hz), 5.91 (1H, d, J = 10.7 Hz), 5.05 (2H, d, J = 6.8 Hz), 4.39 (2H, ddd, J = 13.9, 7.1, 4.6 Hz), 2.87-2.77 (4H, m), 2.71-2.65 (2H, m), 2.50-2.18 (6H, m), 2.09-2.00 (2H, m), 1.98-1.90 (2H, m), 1.71-1.52 (6H, m), 1.39-1.25 (3H, m), 1.06 (3H, d, J = 6.3 Hz), 0.61 (3H, s).
Exact Mass = 483.3 (C₂₇H₄₀F₃NO₃) Obs. mass = 484.30 (M+H)

### [Example 59]

### Synthesis of (1R,3S,Z)-5-(2-((1R,3aS,7aR,E)-1-((S)-1-((S)-3-(difluoromethoxy)pyrrolidin-1-yl)propan-2-yl)-7a-methyloctahydro-4H-inden-4-ylidene)ethylidene)-4-methylenecyclohexane-1,3-diol (Compound B047)

Compound B047 [14.5 mg, 0.031 mmol] was obtained by performing the reaction in the same manner as in Example 1 using compound (2a) [50 mg, 0.069 mmol] and 3-(S)-(difluoromethoxy)pyrrolidine hydrochloride [30 mg, 0.173 mmol].
¹H-NMR (CD₃OD) δ: 6.40 (1H, t, J = 75.0 Hz), 6.33 (1H, d, J = 11.2 Hz), 6.09 (1H, d, J = 11.2 Hz), 5.29 (1H, dd, J = 2.2, 1.2 Hz), 4.80-4.75 (1H, m), 4.35 (1H, t, J = 5.9 Hz), 4.17-4.10 (1H, m), 3.03 (1H, dd, J = 11.2, 5.9 Hz), 2.95-2.85 (2H, m), 2.74 (1H, dd, J = 11.0, 3.2 Hz), 2.67-2.60 (1H, m), 2.55-2.42 (3H, m), 2.30-2.20 (2H, m), 2.08-1.95 (4H, m), 1.93-1.83 (3H, m), 1.77-1.22 (10H, m), 1.08 (3H, d, J = 6.3 Hz), 0.61 (3H, s).
Exact Mass = 465.31 (C₂₇H₄₁F₂NO₃) Obs. mass = 466.30 (M+H)

### [Example 60]

### Synthesis of (1R,3S,Z)-5-(2-((1R,3aS,7aR,E)-1-((S)-1-((R)-3-(difluoromethoxy)pyrrolidin-1-yl)propan-2-yl)-7a-methyloctahydro-4H-inden-4-ylidene)ethylidene)-4-methylenecyclohexane-1,3-diol (Compound B048)

Compound B048 [9.2 mg, 0.020 mmol] was obtained by performing the reaction in the same manner as in Example 1 using compound (2a) [50 mg, 0.069 mmol] and 3-(R)-(difluoromethoxy)pyrrolidine hydrochloride [30 mg, 0.173 mmol].
¹H-NMR (CD₃OD) δ: 6.39 (1H, t, J = 75.0 Hz), 6.33 (1H, d, J = 11.2 Hz), 6.09 (1H, d, J = 11.2 Hz), 5.29 (1H, dd, J = 2.4, 1.5 Hz), 4.80-4.73 (1H, m), 4.35 (1H, t, J = 6.1 Hz), 4.17-4.10 (1H, m), 2.90-1.94 (14H, m), 1.89 (3H, t, J= 5.4 Hz), 1.74-1.24 (10H, m), 1.07 (3H, d, J = 6.8 Hz), 0.61 (3H, s).
Exact Mass = 465.31 (C₂₇H₄₁F₂NO₃) Obs. mass = 466.40 (M+H)

### [Example 61]

### Synthesis of (1R,3R)-5-(2-((1R,3 aS,7aR,E)-1-((S)-1-((S)-3-(difluoromethoxy)pyrrolidin-1-yl)propan-2-yl)-7a-methyloctahydro-4H-inden-4-ylidene)ethylidene)-2-methylenecyclohexane-1,3-diol (Compound B049)

Compound B049 [13.4 mg, 0.029 mmol] was obtained by performing the reaction in the same manner as in Example 1 using compound (2c) [50 mg, 0.069 mmol] and 3-(S)-(difluoromethoxy)pyrrolidine hydrochloride [30 mg, 0.173 mmol].
¹H-NMR (CD₃OD) δ: 6.39 (1H, t, J = 75.0 Hz), 6.27 (1H, d, J = 11.2 Hz), 5.92 (1H, d, J = 11.2 Hz), 5.06 (2H, dd, J = 7.0, 2.0 Hz), 4.77-4.72 (1H, m), 4.40 (2H, ddd, J = 14.0, 7.0, 4.5 Hz), 2.96 (1H, dd, J = 11.0, 6.1 Hz), 2.90-2.76 (2H, m), 2.70-2.60 (2H, m), 2.54-2.18 (8H, m), 2.09-1.85 (4H, m), 1.71-1.50 (6H, m), 1.42-1.25 (3H, m), 1.07 (3H, d, J = 6.3 Hz), 0.62 (3H, s).
Exact Mass = 465.31 (C₂₇H₄₁F₂NO₃) Obs. mass = 466.40 (M+H)

### [Example 62]

### Synthesis of (1R,3R)-5-(2-((1R,3 aS,7aR,E)-1-((S)-1-((R)-3-(difluoromethoxy)pyrrolidin-1-yl)propan-2-yl)-7a-methyloctahydro-4H-inden-4-ylidene)ethylidene)-2-methylenecyclohexane-1,3-diol (Compound B050)

Compound B050 [13.0 mg, 0.028 mmol] was obtained by performing the reaction in the same manner as in Example 1 using compound (2c) [50 mg, 0.069 mmol] and 3-(R)-(difluoromethoxy)pyrrolidine hydrochloride [30 mg, 0.173 mmol].
¹H-NMR (CD₃OD) δ: 6.44 (1H, t, J = 75.0 Hz), 6.27 (1H, d, J = 11.2 Hz), 5.92 (1H, d, J = 11.2 Hz), 5.06 (2H, d, J = 5.9 Hz), 4.88-4.84 (1H, m), 4.40 (2H, ddd, J = 14.3, 7.2, 4.5 Hz), 3.08 (2H, d, J = 4.4 Hz), 3.06-2.85 (3H, m), 2.71-2.63 (3H, m), 2.49 (1H, dd, J = 13.4, 3.7 Hz), 2.35-1.98 (7H, m), 1.78-1.50 (6H, m), 1.41-1.28 (3H, m), 1.10 (3H, d, J = 6.3 Hz), 0.63 (3H, s).
Exact Mass = 465.31 (C₂₇H₄₁F₂NO₃) Obs. mass = 466.40 (M+H)

### [Example 63]

### Synthesis of (1R,3R)-5-(2-((1R,3 aS,7aR,E)-1-((S)-1-((S)-3-(difluoromethoxy)pyrrolidin-1-yl)propan-2-yl)-7a-methyloctahydro-4H-inden-4-ylidene)ethylidene)cyclohexane-1,3-diol (Compound B051)

Compound B051 [7.6 mg, 0.017 mmol] was obtained by performing the reaction in the same manner as in Example 1 using compound (2b) [50 mg, 0.070 mmol] and 3-(S)-(difluoromethoxy)pyrrolidine hydrochloride [30 mg, 0.173 mmol].
¹H-NMR (CD₃OD) δ: 6.42 (1H, t, J = 75.0 Hz), 6.22 (1H, d, J = 11.2 Hz), 5.90 (1H, d, J = 11.2 Hz), 4.84-4.79 (1H, m), 4.07-3.96 (2H, m), 3.12 (1H, dd, J = 11.7, 6.3 Hz), 3.03-2.96 (1H, m), 2.85 (2H, dd, J = 11.5, 3.2 Hz), 2.77-2.50 (4H, m), 2.41 (1H, dd, J = 13.7, 3.4 Hz), 2.33-1.96 (7H, m), 1.91-1.27 (12H, m), 1.09 (3H, d, J = 6.8 Hz), 0.62 (3H, s).
Exact Mass = 453.31 (C₂₆H₄₁F₂NO₃) Obs. mass = 454.35 (M+H)

### [Example 64]

### Synthesis of (1R,3R)-5-(2-((1R,3 aS,7aR,E)-1-((S)-1-((R)-3-(difluoromethoxy)pyrrolidin-1-yl)propan-2-yl)-7a-methyloctahydro-4H-inden-4-ylidene)ethylidene)cyclohexane-1,3-diol (Compound B052)

Compound B052 [6.6 mg, 0.015 mmol] was obtained by performing the reaction in the same manner as in Example 1 using compound (2b) [50 mg, 0.070 mmol] and 3-(R)-(difluoromethoxy)pyrrolidine hydrochloride [30 mg, 0.173 mmol].
¹H-NMR (CD₃OD) δ: 6.43 (1H, t, J = 75.0 Hz), 6.22 (1H, d, J = 11.2 Hz), 5.90 (1H, d, J = 11.2 Hz), 4.88-4.81 (1H, m), 4.07-3.95 (2H, m), 3.04-2.81 (5H, m), 2.65-2.55 (3H, m), 2.42 (1H, dd, J = 13.4, 3.2 Hz), 2.33-1.97 (7H, m), 1.91-1.27 (12H, m), 1.09 (3H, d, J = 6.8 Hz), 0.62 (3H, s).
Exact Mass = 453.31 (C₂₆H₄₁F₂NO₃) Obs. mass = 454.35 (M+H)

### [Reference example 9]

### Synthesis of 3-(S)-(2,2-difluoroethoxy)pyrrolidine hydrochloride (Compound 3b24)

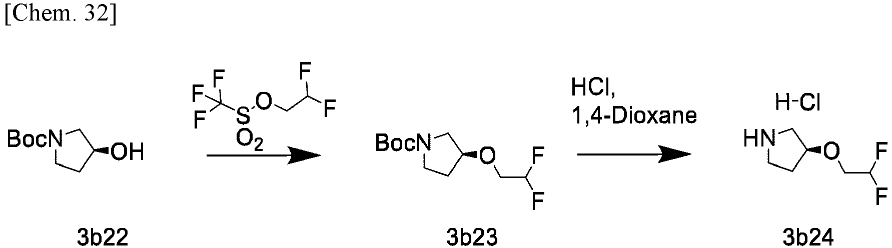

### Step 1

Sodium hydride [60% in oil, 0.32 g, 8 mmol] was added in a solution of t-butyl-3-(S)-hydroxypyrrolidine-1-carboxylate (Compound 3b22) [1.20 g, 6.41 mmol] in THF [17 mL] at 0°C, and the mixture was stirred at 0°C for 20 minutes. To the mixture, 2,2-difluoroethyl trifluoromethanesulfonate [1.65 g, 7.71 mmol] was added and the reaction mixture was stirred at 0°C for 2 hours. The reaction mixture was quenched with saturated ammonium chloride and saturated brine was added thereto. The mixture was extracted with ethyl acetate, and the organic layer was dried over anhydrous magnesium sulfate and concentrated under reduced pressure. The residue was purified by silica gel column chromatography to obtain t-butyl-3-(S)-(2,2-difluoroethoxy)pyrrolidine-1-carboxylate (Compound 3b23) [1.51 g, 6.01 mmol] (Yield = 94%).

### Step 2

A solution of t-butyl-3-(S)-(2,2-difluoroethoxy)pyrrolidine-1-carboxylate (Compound 3b23) [1.51 g, 6.01 mmol] 4 M hydrogen chloride in dioxane [15 mL] e was stirred at room temperature overnight. The reaction mixture was concentrated under reduced pressure and dried to obtain 3-(S)-(2,2-difluoroethoxy)pyrrolidine hydrochloride (Compound 3b24) [1.12 g, 5.97 mmol] (Yield = 99%).

¹H-NMR (DMSO-D₆) δ: 9.41 (2H, s), 6.14 (1H, tt, J = 54.9, 3.7 Hz), 4.34-4.27 (1H, m), 3.72 (2H, td, J = 15.1, 3.9 Hz), 3.25-3.06 (4H, m), 2.08-2.02 (1H, m), 1.97-1.87 (1H, m).

### [Reference example 10]

### Synthesis of 3-(R)-(2,2-difluoroethoxy)pyrrolidine hydrochloride (Compound 3b27)

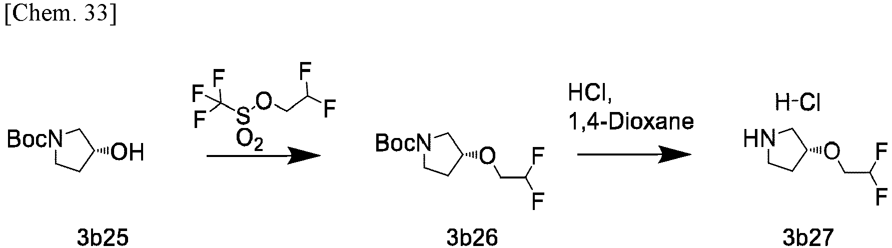

### Step 1

t-Butyl-3-(R)-(2,2-difluoroethoxy)pyrrolidine-1-carboxylate (Compound 3b26) [1.37 g, 5.45 mmol] was obtained by performing the reaction in the same manner as in step 1 of Reference example 9 using t-butyl-3-(R)-hydroxypyrrolidine-1-carboxylate (Compound 3b25) [1.13 g, 6.04 mmol] as a starting material. (Yield = 90%)

### Step 2

3-(R)-(2,2-Difluoroethoxy)pyrrolidine hydrochloride (Compound 3b27) [1.04 g, 5.54 mmol] was obtained by performing the reaction in the same manner as in step 2 of Reference example 9 using t-butyl-3-(R)-(2,2-difluoroethoxy)pyrrolidine-1-carboxylate (Compound 3b26) [1.37 g, 5.45 mmol] as a starting material. (Yield = 92%)

¹H-NMR (DMSO-D₆) δ: 9.54 (2H, s), 6.14 (1H, tt, J = 54.9, 3.7 Hz), 4.31 (1H, dd, J = 5.1, 3.2 Hz), 3.72 (2H, td, J = 15.1, 3.9 Hz), 3.24-3.06 (4H, m), 2.07-2.02 (1H, m), 1.98-1.86 (1H, m).

### [Example 65]

### Synthesis of (1R,3S,Z)-5-(2-((1R,3aS,7aR,E)-1-((S)-1-((S)-3-(2,2-difluoroethoxy)pyrrolidin-1-yl)propan-2-yl)-7a-methyloctahydro-4H-inden-4-ylidene)ethylidene)-4-methylenecyclohexane-1,3-diol (Compound B053)

Compound B053 [13.8 mg, 0.029 mmol] was obtained by performing the reaction in the same manner as in Example 1 using compound 2a [50 mg, 0.069 mmol] and 3-(S)-(2,2-difluoroethoxy)pyrrolidine hydrochloride (Compound 3b24) [40 mg, 213 mmol].
¹H-NMR (CD₃OD) δ: 6.33 (1H, d, J = 11.2 Hz), 6.10 (1H, d, J = 11.2 Hz), 5.94 (1H, tt, J = 55.0, 4.0 Hz), 5.29 (1H, dd, J = 2.4, 1.0 Hz), 4.37-4.30 (2H, m), 4.17-4.10 (1H, m), 3.71 (2H, tdd, J = 14.5, 3.7, 2.8 Hz), 3.28-3.08 (4H, m), 2.92-2.83 (2H, m), 2.76 (1H, t, J = 11.5 Hz), 2.52 (1H, dd, J = 13.2, 3.4 Hz), 2.30-2.17 (2H, m), 2.12-2.00 (4H, m), 1.91-1.29 (13H, m), 1.11 (3H, d, J = 6.3 Hz), 0.63 (3H, s).
Exact Mass = 479.32 (C₂₈H₄₃F₂NO₃) Obs. mass = 480.4 (M+H)

### [Example 66]

### Synthesis of (1R,3S,Z)-5-(2-((1R,3aS,7aR,E)-1-((S)-1-((R)-3-(2,2-difluoroethoxy)pyrrolidin-1-yl)propan-2-yl)-7a-methyloctahydro-4H-inden-4-ylidene)ethylidene)-4-methylenecyclohexane-1,3-diol (Compound B054)

Compound B054 [13.7 mg, 0.029 mmol] was obtained by performing the reaction in the same manner as in Example 1 using compound 2a [50 mg, 0.069 mmol] and 3-(R)-(2,2-difluoroethoxy)pyrrolidine hydrochloride (Compound 3b27) [35 mg, 187 mmol].

¹H-NMR (CD₃OD) δ: 6.32 (1H, d, J = 11.2 Hz), 6.08 (1H, d, J = 11.2 Hz), 5.89 (1H, tt, J = 55.4, 4.0 Hz), 5.28 (1H, dd, J = 1.2, 2.0 Hz), 4.89 (1H, d, J = 2.0 Hz), 4.34 (1H, t, J = 5.9 Hz), 4.16-4.10 (2H, m), 3.69-3.54 (2H, m), 2.86 (1H, dd, J = 12.2, 3.4 Hz), 2.68-2.23 (8H, m), 2.13-1.21 (17H, m), 1.04 (3H, d, J = 6.3 Hz), 0.59 (3H, s).

### [Example 67]

### Synthesis of (1R,3R)-5-(2-((1R,3aS,7aR,E)-1-((S)-1-((S)-3-(2,2-difluoroethoxy)pyrrolidin-1-yl)propan-2-yl)-7a-methyloctahydro-4H-inden-4-ylidene)ethylidene)-2-methylenecyclohexane-1,3-diol (Compound B055)

Compound B055 [15.0 mg, 0.031 mmol] was obtained by performing the reaction in the same manner as in Example 1 using compound 2c [50 mg, 0.069 mmol] and 3-(S)-(2,2-difluoroethoxy)pyrrolidine hydrochloride (Compound 3b24) [40 mg, 213 mmol].
¹H-NMR (CD₃OD) δ: 6.27 (1H, d, J = 11.2 Hz), 5.94 (1H, tt, J = 4.0, 55.0 Hz), 5.93 (1H, d, J = 11.2 Hz), 5.06 (2H, d, J = 5.9 Hz), 4.43-4.29 (3H, m), 3.78-3.64 (2H, m), 3.28-3.07 (4H, m), 2.87 (2H, dd, J = 12.4, 2.7 Hz), 2.76 (1H, t, J = 11.5 Hz), 2.67 (1H, dd, J = 13.2, 4.4 Hz), 2.49 (1H, dd, J = 13.2, 3.9 Hz), 2.33-2.04 (6H, m), 1.81-1.26 (9H, m), 1.12 (3H, d, J = 6.3 Hz), 0.64 (3H, s).
Exact Mass = 479.32 (C₂₈H₄₃F₂NO₃) Obs. mass = 480.35 (M+H)

### [Example 68]

### Synthesis of (1R,3R)-5-(2-((1R,3 aS,7aR,E)-1-((S)-1-((R)-3-(2,2-difluoroethoxy)pyrrolidin-1-yl)propan-2-yl)-7a-methyloctahydro-4H-inden-4-ylidene)ethylidene)-2-methylenecyclohexane-1,3-diol (Compound B056)

Compound B056 [13.2 mg, 0.028 mmol] was obtained by performing the reaction in the same manner as in Example 1 using compound 2c [50 mg, 0.069 mmol] and 3-(R)-(2,2-difluoroethoxy)pyrrolidine hydrochloride (Compound 3b27) [35 mg, 187 mmol].
¹H-NMR (CD₃OD) δ: 6.26 (1H, d, J = 11.2 Hz), 5.91 (1H, d, J = 11.2 Hz), 5.89 (1H, tt, J = 56.0, 4.0 Hz), 5.05 (2H, d, J = 8.0 Hz), 4.42-4.36 (2H, m), 4.17-4.10 (1H, m), 3.70-3.55 (2H, m), 2.85 (1H, dd, J = 12.0, 3.7 Hz), 2.69-2.24 (10H, m), 2.13-1.23 (15H, m), 1.05 (3H, d, J = 6.3 Hz), 0.61 (3H, s).
Exact Mass = 479.32 (C₂₈H₄₃F₂NO₃) Obs. mass = 480.35 (M+H)

### [Example 69]

### Synthesis of (1R,3R)-5-(2-((1R,3 aS,7aR,E)-1-((S)-1-((S)-3 -(2,2-difluoroethoxy)pyrrolidin-1-yl)propan-2-yl)-7a-methyloctahydro-4H-inden-4-ylidene)ethylidene)cyclohexane-1,3-diol (Compound B057)

Compound B057 [15.1 mg, 0.032 mmol] was obtained by performing the reaction in the same manner as in Example 1 using compound 2b [50 mg, 0.070 mmol] and 3-(S)-(2,2-difluoroethoxy)pyrrolidine hydrochloride (Compound 3b24) [40 mg, 214 mmol].

¹H-NMR (CD₃OD) δ: 6.22 (1H, d, J = 10.7 Hz), 5.94 (1H, tt, J = 55.0, 4.0 Hz), 5.91 (1H, d, J = 10.7 Hz), 4.33-4.29 (1H, m), 4.07-3.96 (2H, m), 3.71 (2H, dtd, J = 3.0, 14.0, 4.0 Hz), 3.29-3.05 (4H, m), 2.86 (2H, d, J = 12.2 Hz), 2.75 (1H, t, J = 11.5 Hz), 2.60 (1H, dd, J = 13.4, 3.7 Hz), 2.42 (1H, dd, J = 13.4, 3.2 Hz), 2.26-1.95 (7H, m), 1.88-1.30 (11H, m), 1.11 (3H, d, J = 6.3 Hz), 0.64 (3H, s).

### [Example 70]

### Synthesis of (1R,3R)-5-(2-((1R,3 aS,7aR,E)-1-((S)-1-((R)-3-(2,2-difluoroethoxy)pyrrolidin-1-yl)propan-2-yl)-7a-methyloctahydro-4H-inden-4-ylidene)ethylidene)cyclohexane-1,3-diol (Compound B058)

Compound B058 [10.3 mg, 0.022 mmol] was obtained by performing the reaction in the same manner as in Example 1 using compound 2b [50 mg, 0.070 mmol] and 3-(R)-(2,2-difluoroethoxy)pyrrolidine hydrochloride (Compound 3b27) [35 mg, 187 mmol].
¹H-NMR (CD₃OD) δ: 6.21 (1H, d, J = 11.2 Hz), 5.89 (1H, tt, J = 55.0, 4.0 Hz), 5.88 (1H, d, J = 11.2 Hz), 4.16-4.10 (1H, m), 4.06-3.95 (2H, m), 3.62 (2H, tt, J = 14.4, 4.0 Hz), 2.83 (1H, dd, J = 11.7, 3.9 Hz), 2.68-2.56 (4H, m), 2.49 (1H, q, J = 7.8 Hz), 2.40 (1H, dd, J = 13.2, 3.4 Hz), 2.35-1.21 (21H, m), 1.04 (3H, d, J = 6.3 Hz), 0.60 (3H, s).
Exact Mass = 467.32 (C₂₇H₄₃F₂NO₃) Obs. mass = 468.25 (M+H)

### [Reference example 11]

### Synthesis of 3-(S)-(3,3-difluoropropyl)pyrrolidine hydrochloride (Compound 3b32)

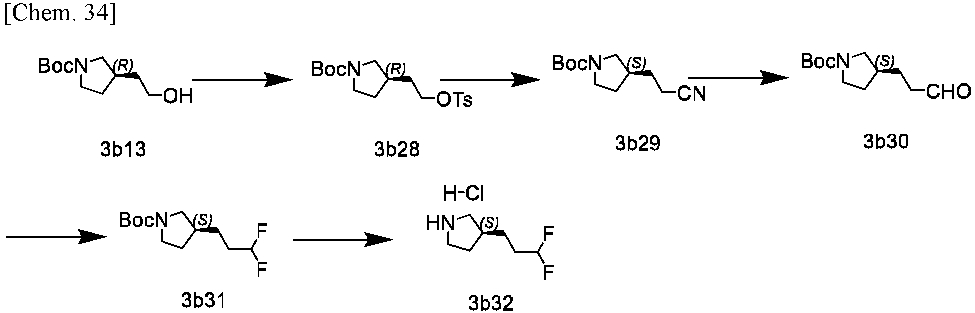

### Step 1

A solution of t-butyl-3-(R)-(2-hydroxyethyl)pyrrolidine-1-carboxylate (Compound 3b13) [1.05 g, 4.88 mmol] and p-toluenesulfonyl chloride [1.08 g, 5.66 mmol] in pyridine [10 mL] was stirred at room temperature overnight. The reaction mixture was transferred to brine and the mixture was extracted with ethyl acetate. The organic layer was dried over anhydrous magnesium sulfate and concentrated under reduced pressure. The residue was purified by silica gel column chromatography to obtain t-butyl-3-(R)-(2-(tosyloxy)ethyl)pyrrolidine-1-carboxylate (Compound 3b28) [1.46 g, 3.95 mmol] (Yield = 81%).

### Step 2

Potassium cyanide [1.009 g, 15.50 mmol] and 18-crown-6 [114 mg, 0.431 mmol] were added to a solution of t-butyl-3-(R)-(2-(tosyloxy)ethyl)pyrrolidine-1-carboxylate (Compound 3b28) [1.46 g, 3.95 mmol] in DMF [20 mL] and the reaction mixture was stirred at 60°C overnight. The reaction mixture was poured into saturated brine at room temperature and the mixture was extracted with ethyl acetate. The organic layer was dried over anhydrous magnesium sulfate and concentrated under reduced pressure. The residue was purified by silica gel column chromatography to obtain t-butyl-3-(S)-(2-cyanoethyl)pyrrolidine-1-carboxylate (Compound 3b29) [0.74 g, 3.3 mmol] (Yield = 68%).

### Step 3

A solution of t-butyl-3-(S)-(2-cyanoethyl)pyrrolidine-1-carboxylate (Compound 3b29) [0.74 g, 3.3 mmol] in toluene [30 mL] was cooled to -78°C under a nitrogen atmosphere. To the solution, a solution of diisobutylaluminum hydride (DIBAL-H) in toluene [1.5 M, 4 mL, 6 mmol] was added, and the mixture was stirred at the same temperature for 1.5 hours. The reaction mixture was warmed to room temperature and quenched with saturated ammonium chloride. A 2 M aqueous sodium hydroxide solution [10 mL] was added to the mixture, and the mixture was stirred at room temperature for 30 minutes. The mixture was extracted with ethyl acetate and the organic layer was washed with a saturated aqueous ammonium chloride solution, dried over anhydrous magnesium sulfate, and concentrated under reduced pressure. The residue was purified by silica gel column chromatography to obtain t-butyl-3-(S)-(2-oxopropyl)pyrrolidine-1-carboxylate (Compound 3b30) [280 mg, 1.23 mmol] (Yield = 37%).

### Step 4

A solution of t-butyl-3-(R)-(2-oxopropyl)pyrrolidine-1-carboxylate (Compound 3b30) [280 mg, 1.23 mmol] in dichloromethane [10 mL] was cooled to 0°C, DAST [0.60 mL, 4.55 mmol] was added to the solution, and the mixture was stirred overnight. The next morning, DAST [0.60 mL, 4.55 mmol] was further added to the mixture at 0°C, and the mixture was further stirred for 1 day. The reaction mixture was cooled to 0°C, quenched with a saturated aqueous sodium hydrogen carbonate solution, and extracted with ethyl acetate. The organic layer was dried over anhydrous magnesium sulfate and concentrated under reduced pressure. The residue was purified by silica gel column chromatography to obtain t-butyl-3-(S)-(3,3-difluoropropyl)pyrrolidine-1-carboxylate (Compound 3b31) [230.1 mg, 0.923 mmol] (Yield = 75%).

### Step 5

A solution of t-butyl-3-(S)-(3,3-difluoropropyl)pyrrolidine-1-carboxylate (Compound 3b31) [230.1 mg, 0.923 mmol] in 4 M hydrogen chloride in dioxane [5 mL, 20 mmol] was stirred at room temperature overnight. The reaction mixture was concentrated under reduced pressure and dried to obtain 3-(S)-(3,3-difluoropropyl)pyrrolidine hydrochloride (Compound 3b32) [173.7 mg, 0.923 mmol] (Yield = 100%).

¹H-NMR (DMSO-D₆) δ: 9.22 (2H, s), 6.07 (1H, tt, J = 56.7, 4.3 Hz), 3.35-3.15 (2H, m), 3.09-3.00 (1H, m), 2.68 (1H, dd, J = 11.4, 8.7 Hz), 2.23-2.11 (1H, m), 2.08-1.98 (1H, m), 1.91-1.76 (2H, m), 1.56-1.39 (3H, m).

### [Reference example 12]

### Synthesis of 3-(R)-(3,3-difluoropropyl)pyrrolidine hydrochloride (Compound 3b37)

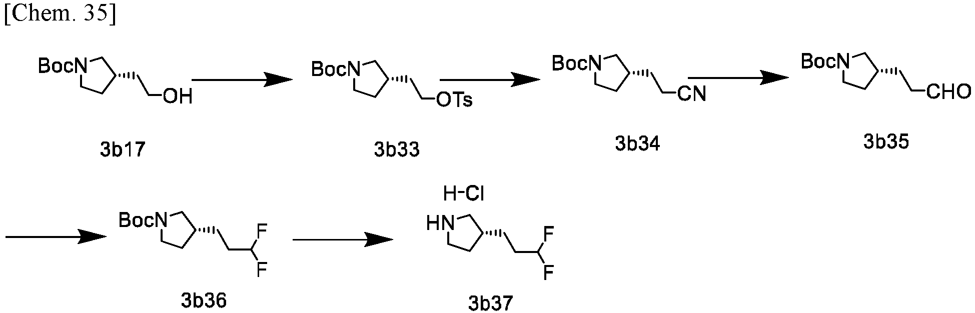

### Step 1

A solution of t-butyl-3-(S)-(2-hydroxyethyl)pyrrolidine-1-carboxylate (Compound 3b17) [1.06 g, 4.92 mmol], p-toluenesulfonyl chloride [1.08 g, 5.66 mmol], and 4-dimethylaminopyridine [20 mg, 0.163 mmol] in pyridine [10 mL] was stirred at room temperature for 1.5 hours. The reaction mixture was poured into brine and the mixture was extracted with ethyl acetate. The organic layer was dried over anhydrous magnesium sulfate and concentrated under reduced pressure. The residue was purified by silica gel column chromatography to obtain t-butyl-3-(S)-(2-(tosyloxy)ethyl)pyrrolidine-1-carboxylate (Compound 3b33) [1.28 g, 3.46 mmol] (Yield = 70%).

### Step 2

Potassium cyanide [618 mg, 9.49 mmol] and 18-crown-6 ether [97.2 mg, 0.368 mmol] were added to a solution of t-butyl-3-(S)-(2-(tosyloxy)ethyl)pyrrolidine-1-carboxylate (Compound 3b33) [1.28 g, 3.46 mmol] in DMF [20 mL] and the reaction mixture was stirred at 60°C overnight. The reaction mixture was poured into saturated brine and the mixture was extracted with ethyl acetate. The organic layer was dried over anhydrous magnesium sulfate and concentrated under reduced pressure. The residue was purified by silica gel column chromatography to obtain t-butyl-3-(R)-(2-cyanoethyl)pyrrolidine-1-carboxylate (Compound 3b34) [0.81 g, 3.46 mmol] (Yield = 100%).

### Step 3

A solution of t-butyl-3-(R)-(2-cyanoethyl)pyrrolidine-1-carboxylate (Compound 3b34) [0.81 g, 3.46 mmol] in toluene [30 mL] was cooled to -78°C under a nitrogen atmosphere. To the solution, a solution of diisobutylaluminum hydride (DIBAL-H) in toluene [1.5 M, 5.4 mL, 8.1 mmol] was added, and the mixture was stirred at the same temperature for 1.5 hours. The reaction mixture was warmed to room temperature and quenched with saturated ammonium chloride. A 2M aqueous sodium hydroxide solution [10 mL] was added to the mixture, and the mixture was stirred at room temperature for 30 minutes. The mixture was extracted with ethyl acetate, the organic layer was washed with a saturated aqueous ammonium chloride solution, dried over anhydrous magnesium sulfate, and concentrated under reduced pressure. The residue was purified by silica gel column chromatography to obtain t-butyl-3-(R)-(2-oxopropyl)pyrrolidine-1-carboxylate (Compound 3b35) [447.3 mg, 1.97 mmol] (Yield = 54%).

### Step 4

A solution of t-butyl-3-(R)-(2-oxopropyl)pyrrolidine-1-carboxylate (Compound 3b35) [447.3 mg, 1.97 mmol] in dichloromethane [30 mL] was cooled to 0°C and DAST [0.63 mL, 4.77 mmol] was added to the solution and the mixture was stirred overnight. The next morning, DAST [0.63 mL, 4.77 mmol] was further added to the mixture at 0°C, and the mixture was further stirred for 1 day. The reaction mixture was cooled to 0°C, quenched with a saturated aqueous sodium hydrogen carbonate solution, and extracted with ethyl acetate. The organic layer was dried over anhydrous magnesium sulfate and concentrated under reduced pressure. The residue was purified by silica gel column chromatography to obtain t-butyl-3-(R)-(3,3-difluoropropyl)pyrrolidine-1-carboxylate (Compound 3b36) [269.7 mg, 1.08 mmol].

### Step 5

A solution of t-butyl-3-(R)-(3,3-difluoropropyl)pyrrolidine-1-carboxylate (Compound 3b36) [269.7 mg, 1.08 mmol] in 4 M hydrogen chloride in dioxane [5 mL, 20 mmol] was stirred at room temperature overnight. The reaction mixture was concentrated under reduced pressure and dried to obtain 3-(R)-(3,3-difluoropropyl)pyrrolidine hydrochloride (Compound 3b37) [203.2 mg, 1.08 mmol] (Yield = 100%).

¹H-NMR (DMSO-D₆) δ: 9.03 (2H, s), 6.03 (1H, tt, J = 56.8, 4.3 Hz), 3.24 (1H, dd, J = 11.2, 7.8 Hz), 3.19-3.13 (1H, m), 3.06-2.98 (1H, m), 2.65 (1H, dd, J= 11.2, 8.8 Hz), 2.18-2.10 (1H, m), 2.08-1.97 (1H, m), 1.87-1.73 (2H, m), 1.53-1.36 (3H, m).

### [Example 71]

### Synthesis of (1R,3S,Z)-5-(2-((1R,3aS,7aR,E)-1-((S)-1-((S)-3-(3,3-difluoropropyl)pyrrolidin-1-yl)propan-2-yl)-7a-methyloctahydro-4H-inden-4-ylidene)ethylidene)-4-methylenecyclohexane-1,3-diol (Compound B059)

Compound B059 [12.3 mg, 0.257 mmol] was obtained by performing the reaction in the same manner as in Example 1 using compound (2a) [50.0 mg, 0.069 mmol] and 3-(S)-(3,3-difluoropropyl)pyrrolidine hydrochloride (Compound 3b32) [30 mg, 0.162 mmol].
¹H-NMR (CD₃OD) δ: 6.30 (1H, d, J = 11.4 Hz), 6.07 (1H, d, J = 11.4 Hz), 5.83 (1H, tt, J = 56.9, 4.4 Hz), 5.27 (1H, dd, J = 2.3, 1.4 Hz), 4.33 (1H, t, J = 5.9 Hz), 4.13-4.07 (1H, m), 2.91-2.83 (2H, m), 2.65-2.58 (1H, m), 2.54-2.43 (2H, m), 2.28-1.20 (32H, m), 1.03 (3H, d, J = 6.4 Hz), 0.58 (3H, s).
Exact Mass = 477.34 (C₂₉H₄₅F₂NO₂) Obs. mass = 478.25 (M+H)

### [Example 72]

### Synthesis of (1R,3S,Z)-5-(2-((1R,3aS,7aR,E)-1-((S)-1-((R)-3-(3,3-difluoropropyl)pyrrolidin-1-yl)propan-2-yl)-7a-methyloctahydro-4H-inden-4-ylidene)ethylidene)-4-methylenecyclohexane-1,3-diol (Compound B060)

Compound B060 [13.4 mg, 0.28 mmol] was obtained by performing the reaction in the same manner as in Example 1 using compound (2a) [50.0 mg, 0.069 mmol] and 3-(R)-(3,3-difluoropropyl)pyrrolidine hydrochloride (Compound 3b37) [35 mg, 0.189 mmol].

¹H-NMR (CD₃OD) δ: 6.32 (1H, d, J = 11.2 Hz), 6.08 (1H, d, J = 11.2 Hz), 5.85 (1H, tt, J = 56.0, 4.0 Hz), 5.28 (1H, dd, J = 2.0, 1.2 Hz), 4.34 (1H, t, J = 5.9 Hz), 4.15-4.10 (1H, m), 2.86 (1H, dd, J = 12.0, 4.6 Hz), 2.79-2.70 (2H, m), 2.51 (1H, dd, J = 13.4, 3.2 Hz), 2.40-1.24 (28H, m), 1.05 (3H, d, J = 6.8 Hz), 0.59 (3H, s).

### [Example 73]

### Synthesis of (1R,3R)-5-(2-((1R,3aS,7aR,E)-1-((S)-1-((S)-3-(3,3-difluoropropyl)pyrrolidin-1-yl)propan-2-yl)-7a-methyloctahydro-4H-inden-4-ylidene)ethylidene)-2-methylenecyclohexane-1,3-diol (Compound B061)

Compound B061 [14.7 mg, 0.31 mmol] was obtained by performing the reaction in the same manner as in Example 1 using compound (2c) [50.0 mg, 0.069 mmol] and 3-(S)-(3,3-difluoropropyl)pyrrolidine hydrochloride (Compound 3b32) [30 mg, 0.162 mmol].

¹H-NMR (CD₃OD) δ: 6.26 (1H, d, J = 11.0 Hz), 5.91 (1H, d, J = 11.0 Hz), 5.85 (1H, tt, J = 57.5, 4.0 Hz), 5.05 (2H, d, J = 6.4 Hz), 4.42-4.35 (2H, m), 2.92 (1H, t, J = 8.5 Hz), 2.85 (1H, dd, J= 12.1, 3.4 Hz), 2.70-2.60 (2H, m), 2.51-2.46 (2H, m), 2.31-2.25 (4H, m), 2.21-1.22 (22H, m), 1.05 (3H, d, J = 6.4 Hz), 0.61 (3H, s).

### [Example 74]

### Synthesis of (1R,3R)-5-(2-((1R,3aS,7aR,E)-1-((S)-1-((R)-3-(3,3-difluoropropyl)pyrrolidin-1-yl)propan-2-yl)-7a-methyloctahydro-4H-inden-4-ylidene)ethylidene)-2-methylenecyclohexane-1,3-diol (Compound B062)

Compound B062 [17.7 mg, 0.037 mmol] was obtained by performing the reaction in the same manner as in Example 1 using compound (2c) [50.0 mg, 0.069 mmol] and 3-(R)-(3,3-difluoropropyl)pyrrolidine hydrochloride (Compound 3b37) [30 mg, 0.205 mmol].
¹H-NMR (CD₃OD) δ: 6.28 (1H, d, J = 11.2 Hz), 5.93 (1H, d, J = 11.2 Hz), 5.91 (2H, tt, J = 57.0, 4.0 Hz), 5.06 (2H, d, J = 5.9 Hz), 4.40 (2H, ddd, J = 14.4, 7.1, 4.4 Hz), 3.51 (1H, dd, J = 11.0, 8.1 Hz), 3.42-3.26 (3H, m), 3.07-2.85 (4H, m), 2.67 (1H, dd, J = 13.4, 4.1 Hz), 2.49 (1H, dd, J = 13.7, 3.9 Hz), 2.45-2.20 (4H, m), 2.12-1.95 (3H, m), 1.90-1.33 (15H, m), 1.14 (3H, d, J = 6.8 Hz), 0.65 (3H, s).
Exact Mass = 477.34 (C₂₉H₄₅F₂NO₂) Obs. mass = 478.40 (M+H)

### [Example 75]

### Synthesis of (1R,3R)-5-(2-((1R,3aS,7aR,E)-1-((S)-1-((S)-3-(3,3-difluoropropyl)pyrrolidin-1-yl)propan-2-yl)-7a-methyloctahydro-4H-inden-4-ylidene)ethylidene)cyclohexane-1,3-diol (Compound B063)

Compound B063 [9.8 mg, 0.021 mmol] was obtained by performing the reaction in the same manner as in Example 1 using compound (2b) [50.0 mg, 0.070 mmol] and 3-(S)-(3,3-difluoropropyl)pyrrolidine hydrochloride (Compound 3b32) [30 mg, 0.162 mmol].
¹H-NMR (CD₃OD) δ: 6.21 (1H, d, J = 11.0 Hz), 5.89 (2H, d, J = 11.0 Hz), 5.85 (1H, tt, J = 4.5, 57.0 Hz), 4.06-3.95 (2H, m), 2.92 (1H, t, J = 8.5 Hz), 2.83 (1H, dd, J = 11.7, 3.9 Hz), 2.68-2.55 (2H, m), 2.52-2.45 (1H, m), 2.41 (1H, dd, J = 13.3, 3.2 Hz), 2.32-1.22 (31H, m), 1.05 (3H, d, J = 6.4 Hz), 0.60 (3H, s).
Exact Mass = 465.34 (C₂₈H₄₅F₂NO₂) Obs. mass = 466.30 (M+H)

### [Example 76]

### Synthesis of (1R,3R)-5-(2-((1R,3aS,7aR,E)-1-((S)-1-((R)-3-(3,3-difluoropropyl)pyrrolidin-1-yl)propan-2-yl)-7a-methyloctahydro-4H-inden-4-ylidene)ethylidene)cyclohexane-1,3-diol (Compound B064)

Compound B064 [10.3 mg, 0.022 mmol] was obtained by performing the reaction in the same manner as in Example 1 using compound (2b) [50.0 mg, 0.070 mmol] and 3-(R)-(3,3-difluoropropyl)pyrrolidine hydrochloride (Compound 3b37) [40 mg, 0.215 mmol].
¹H-NMR (CD₃OD) δ: 6.21 (1H, d, J = 11.2 Hz), 5.88 (1H, d, J = 11.2 Hz), 5.85 (1H, tt, J = 57.0, 4.0 Hz), 4.06-3.95 (2H, m), 2.83 (1H, dd, J = 12.0, 3.7 Hz), 2.77-2.70 (2H, m), 2.59 (1H, dd, J = 13.4, 3.7 Hz), 2.42-1.21 (31H, m), 1.05 (3H, d, J = 6.8 Hz), 0.60 (3H, s).
Exact Mass = 465.34 (C₂₈H₄₅F₂NO₂) Obs. mass = 466.35 (M+H)

### [Example 77]

### Synthesis of (1R,3S,Z)-5-(2-((1R,3aS,7aR,E)-7a-methyl-1-((S)-1-((S)-3-(trifluoromethoxy)pyrrolidin-1-yl)propan-2-yl)octahydro-4H-inden-4-ylidene)ethylidene)-4-methylenecyclohexane-1,3-diol (Compound B065)

Compound B065 [ 5.7 mg, 0.012 mmol] was obtained by performing the reaction in the same manner as in Example 1 using compound (2a) [50.0 mg, 0.069 mmol] and 3-(S)-(trifluoromethoxy)pyrrolidine hydrochloride [35 mg, 0.183 mmol].
¹H-NMR (CD₃OD) δ: 6.32 (1H, d, J = 11.2 Hz), 6.08 (1H, d, J = 11.2 Hz), 5.28 (1H, dd, J = 1.8, 1.0 Hz), 4.89 (1H, dd, J = 1.8, 1.0 Hz), 4.86-4.80 (2H, m), 4.34 (1H, t, J = 5.9 Hz), 4.14-4.05 (1H, m), 2.86 (2H, dd, J = 10.7, 5.9 Hz), 2.81-2.75 (1H, m), 2.62 (1H, dd, J = 10.7, 2.4 Hz), 2.51 (1H, dd, J = 13.2, 3.4 Hz), 2.38-2.16 (6H, m), 2.07-1.87 (7H, m), 1.73-1.21 (11H, m), 1.06 (3H, t, J = 8.1 Hz), 0.59 (3H, s).
Exact Mass = 483.30 (C₂₇H₄₀F₃NO₃) Obs. mass = 484.35 (M+H)

### [Example 78]

### Synthesis of (1R,3S,Z)-5-(2-((1R,3aS,7aR,E)-7a-methyl-1-((S)-1-((R)-3-(trifluoromethoxy)pyrrolidin-1-yl)propan-2-yl)octahydro-4H-inden-4-ylidene)ethylidene)-4-methylenecyclohexane-1,3-diol (Compound B066)

Compound B066 [6.1 mg, 0.013 mmol] was obtained by performing the reaction in the same manner as in Example 1 using compound [2a] [50.0 mg, 0.069 mmol] and 3-(R)-(trifluoromethoxy)pyrrolidine hydrochloride [35 mg, 0.183 mmol].

¹H-NMR (CD₃OD) δ: 6.32 (1H, d, J = 11.2 Hz), 6.08 (1H, d, J = 11.2 Hz), 5.28 (1H, dd, J = 2.4, 1.5 Hz), 4.89 (1H, dd, J = 2.2, 1.5 Hz), 4.86-4.82 (1H, m), 4.34 (1H, t, J = 5.9 Hz), 4.15-4.09 (1H, m), 2.86 (1H, dd, J = 12.2, 3.4 Hz), 2.78 (1H, dd, J = 11.2, 2.4 Hz), 2.67-2.62 (2H, m), 2.55-2.43 (2H, m), 2.35-2.20 (4H, m), 2.07-1.91 (4H, m), 1.88 (2H, t, J = 5.9 Hz), 1.75-1.63 (2H, m), 1.60-1.21 (8H, m), 1.04 (3H, d, J = 6.8 Hz), 0.59 (3H, s). Exact Mass = 483.30 (C₂₇H₄₀F₃NO₃) Obs. mass = 484.20 (M+H)

### [Example 79]

### Synthesis of (1R,3S)-5-(2-((1R,3aS,7aR,E)-7a-methyl-1-((S)-1-((S)-3-(trifluoromethoxy)pyrrolidin-1-yl)propan-2-yl)octahydro-4H-inden-4-ylidene)ethylidene)-2-methylenecyclohexane-1,3-diol (Compound B067)

Compound B067 [5.7 mg, 0.012 mmol] was obtained by performing the reaction in the same manner as in Example 1 using compound (2c) [50.0 mg, 0.069 mmol] and 3-(S)-(trifluoromethoxy)pyrrolidine hydrochloride [35 mg, 0.183 mmol].
¹H-NMR (CD₃OD) δ: 6.27 (1H, d, J = 10.7 Hz), 5.91 (1H, d, J = 11.2 Hz), 5.05 (2H, d, J = 7.3 Hz), 4.88-4.80 (2H, m), 4.42-4.36 (2H, m), 2.89-2.77 (3H, m), 2.65 (2H, td, J = 13.0, 4.0 Hz), 2.48 (1H, dd, J = 13.4, 4.1 Hz), 2.40-2.22 (7H, m), 2.07-1.90 (5H, m), 1.60 (7H, tt, J = 24.6, 8.1 Hz), 1.38-1.21 (4H, m), 1.05 (3H, d, J = 6.3 Hz), 0.61 (3H, s).
Exact Mass = 483.30 (C₂₇H₄₀F₃NO₃) Obs. mass = 484.40 (M+H)

### [Example 80]

### Synthesis of (1R,3S)-5-(2-((1R,3aS,7aR,E)-7a-methyl-1-((S)-1-((R)-3-(trifluoromethoxy)pyrrolidin-1-yl)propan-2-yl)octahydro-4H-inden-4-ylidene)ethylidene)-2-methylenecyclohexane-1,3-diol (Compound B068)

Compound B068 [6.7 mg, 0.014 mmol] was obtained by performing the reaction in the same manner as in Example 1 using compound (2c) [50.0 mg, 0.069 mmol] and 3-(R)-(trifluoromethoxy)pyrrolidine hydrochloride [35 mg, 0.183 mmol].
¹H-NMR (CD₃OD) δ: 6.26 (1H, d, J = 11.2 Hz), 5.91 (1H, d, J = 11.2 Hz), 5.05 (2H, d, J = 6.8 Hz), 4.86-4.83 (1H, m), 4.43-4.35 (2H, m), 2.87-2.79 (2H, m), 2.68-2.63 (3H, m), 2.49 (2H, dd, J = 15.9, 6.6 Hz), 2.37-2.20 (5H, m), 2.07-1.90 (4H, m), 1.70-1.21 (10H, m), 1.05 (3H, d, J = 6.3 Hz), 0.61 (3H, s).
Exact Mass = 483.30 (C₂₇H₄₀F₃NO₃) Obs. mass = 484.30 (M+H)

### [Example 81]

### Synthesis of (1R,3S)-5-(2-((1R,3aS,7aR,E)-7a-methyl-1-((S)-1-((S)-3-(trifluoromethoxy)pyrrolidin-1-yl)propan-2-yl)octahydro-4H-inden-4-ylidene)ethylidene)cyclohexane-1,3-diol (Compound B069)

Compound B069 [6.8 mg, 0.014 mmol] was obtained by performing the reaction in the same manner as in Example 1 using compound (2b) [50.0 mg, 0.070 mmol] and 3-(S)-(trifluoromethoxy)pyrrolidine hydrochloride [35 mg, 0.183 mmol].
¹H-NMR (CD₃OD) δ: 6.20 (1H, d, J = 11.0 Hz), 5.87 (1H, d, J = 11.0 Hz), 4.85-4.80 (1H, m), 4.04-3.93 (2H, m), 2.87-2.73 (3H, m), 2.66-2.53 (2H, m), 2.41-2.11 (7H, m), 2.05-1.47 (12H, m), 1.37-1.23 (3H, m), 1.03 (3H, d, J = 6.4 Hz), 0.58 (3H, s).
Exact Mass = 471.30 (C₂₆H₄₀F₃NO₃) Obs. mass = 472.25 (M+H)

### [Example 82]

### Synthesis of (1R,3S)-5-(2-((1R,3aS,7aR,E)-7a-methyl-1-((S)-1-((R)-3-(trifluoromethoxy)pyrrolidin-1-yl)propan-2-yl)octahydro-4H-inden-4-ylidene)ethylidene)cyclohexane-1,3-diol (Compound B070)

Compound B070 [ 6.2 mg, 0.013 mmol] was obtained by performing the reaction in the same manner as in Example 1 using compound (2b) [50.0 mg, 0.070 mmol] and 3-(R)-(trifluoromethoxy)pyrrolidine hydrochloride [35 mg, 0.183 mmol].
¹H-NMR (CD₃OD) δ: 6.20 (1H, d, J = 11.4 Hz), 5.87 (1H, d, J = 11.0 Hz), 4.85-4.81 (1H, m), 4.04-3.93 (2H, m), 2.82 (1H, dd, J = 11.7, 3.9 Hz), 2.78 (1H, dd, J = 3.0, 12.0 Hz), 2.66-2.61 (2H, m), 2.57 (1H, dd, J = 13.3, 3.7 Hz), 2.46 (1H, q, J = 7.9 Hz), 2.39 (1H, dd, J = 13.3, 3.2 Hz), 2.34-2.11 (5H, m), 2.05-1.71 (6H, m), 1.65-1.47 (6H, m), 1.38-1.21 (3H, m), 1.03 (3H, d, J = 6.4 Hz), 0.58 (3H, s).
Exact Mass = 471.30 (C₂₆H₄₀F₃NO₃) Obs. mass = 472.25 (M+H)

### [Example 83]

### Synthesis of (1R,3S,Z)-5-(2-((1R,3aS,7aR,E)-1-((S)-1-((S)-3-hydroxy-3-methylpyrrolidin-1-yl)propan-2-yl)-7a-methyloctahydro-4H-inden-4-ylidene)ethylidene)-4-methylenecyclohexane-1,3-diol (compound B071)

Compound B071 [10.7 mg, 0.025 mmol] was obtained by performing the reaction in the same manner as in Example 1 using compound (2a) [50.0 mg, 0.069 mmol] and (S)-3-methylpyrrolidin-3-ol hydrochloride [30 mg, 0.218 mmol].
¹H-NMR (CD₃OD) δ: 6.32 (1H, d, J = 11.2 Hz), 6.08 (1H, d, J = 10.7 Hz), 5.28 (1H, s), 4.34 (1H, t, J = 5.9 Hz), 4.18-4.08 (2H, m), 2.86 (1H, dd, J = 12.2, 3.4 Hz), 2.76 (1H, q, J = 8.0 Hz), 2.63-2.40 (4H, m), 2.35-2.20 (4H, m), 2.07-1.25 (21H, m), 1.05 (3H, d, J = 6.8 Hz), 0.59 (3H, s).
Exact Mass = 429.32 (C₂₇H₄₃NO₃) Obs. mass = 430.30 (M+H)

### [Example 84]

### Synthesis of (1R,3S)-5-(2-((1R,3aS,7aR,E)-1-((S)-1-((S)-3-hydroxy-3-methylpyrrolidin-1-yl)propan-2-yl)-7a-methyloctahydro-4H-inden-4-ylidene)ethylidene)cyclohexane-1,3-diol (Compound B072)

Compound B072 [9.7 mg, 0.023 mmol] was obtained by performing the reaction in the same manner as in Example 1 using compound (2b) [50.0 mg, 0.070 mmol] and (S)-3-methylpyrrolidin-3-ol hydrochloride [30 mg, 0.218 mmol].
¹H-NMR (CD₃OD) δ: 6.21 (1H, d, J = 11.2 Hz), 5.88 (1H, d, J = 11.2 Hz), 4.06-3.95 (2H, m), 2.83 (1H, dd, J = 11.7, 3.9 Hz), 2.77 (1H, dd, J = 16.6, 7.3 Hz), 2.61-2.13 (9H, m), 2.06-1.72 (7H, m), 1.69-1.48 (6H, m), 1.40-1.33 (2H, m), 1.33 (3H, s), 1.29-1.21 (1H, m), 1.05 (3H, d, J = 6.3 Hz), 0.59 (3H, s).
Exact Mass = 417.32 (C₂₆H₄₃NO₃) Obs. mass = 418.30 (M+H)

### [Example 85]

### Synthesis of (1R,3S)-5-(2-((1R,3aS,7aR,E)-1-((S)-1-((S)-3-hydroxy-3-methylpyrrolidin-1-yl)propan-2-yl)-7a-methyloctahydro-4H-inden-4-ylidene)ethylidene)-2-methylenecyclohexane-1,3-diol (Compound B073)

Compound B073 [9.6 mg, 0.022 mmol] was obtained by performing the reaction in the same manner as in Example 1 using compound (2c) [50.0 mg, 0.069 mmol] and (S)-3-methylpyrrolidin-3-ol hydrochloride [30 mg, 0.218 mmol].
¹H-NMR (CD₃OD) δ: 6.26 (1H, d, J = 11.2 Hz), 5.91 (1H, d, J = 11.2 Hz), 5.05 (2H, d, J = 6.8 Hz), 4.42-4.36 (2H, m), 2.85 (1H, dd, J = 12.0, 3.7 Hz), 2.77 (1H, dd, J = 16.6, 7.3 Hz), 2.67 (1H, dd, J= 13.4, 4.1 Hz), 2.63-2.40 (4H, m), 2.36-2.25 (4H, m), 2.10-1.90 (3H, m), 1.86-1.75 (2H, m), 1.72-1.50 (6H, m), 1.37 (2H, td, J = 11.8, 3.1 Hz), 1.33 (3H, s), 1.30-1.21 (2H, m), 1.05 (3H, d, J = 6.3 Hz), 0.60 (3H, s).
Exact Mass = 429.32 (C₂₇H₄₃NO₃) Obs. mass = 430.30 (M+H)

### [Example 86]

### Synthesis of (1R,3S,Z)-5-(2-((1R,3aS,7aR,E)-1-((S)-1-((R)-3-hydroxy-3-methylpyrrolidin-1-yl)propan-2-yl)-7a-methyloctahydro-4H-inden-4-ylidene)ethylidene)-4-methylenecyclohexane-1,3-diol (compound B074)

Compound B074[10.3 mg, 0.024 mmol] was obtained by performing the reaction in the same manner as in Example 1 using compound (2a) [50.0 mg, 0.069 mmol] and (R)-3-methylpyrrolidin-3-ol hydrochloride [30 mg, 0.218 mmol].
¹H-NMR (CD₃OD) δ: 6.32 (1H, d, J = 11.2 Hz), 6.08 (1H, d, J = 11.2 Hz), 5.28 (1H, dd, J = 2.4, 1.5 Hz), 4.34 (1H, t, J = 6.1 Hz), 4.16-4.05 (1H, m), 2.86 (1H, dd, J = 12.0, 3.7 Hz), 2.75-2.53 (3H, m), 2.51 (1H, dd, J = 13.4, 3.7 Hz), 2.38 (1H, d, J = 9.8 Hz), 2.34-2.22 (3H, m), 2.07-1.77 (9H, m), 1.73-1.36 (9H, m), 1.33 (3H, s), 1.31-1.23 (3H, m), 1.05 (3H, d, J = 6.3 Hz), 0.59 (3H, s).
Exact Mass = 429.32 (C₂₇H₄₃NO₃) Obs. mass =430.30 (M+H)

### [Example 87]

### Synthesis of (1R,3S)-5-(2-((1R,3aS,7aR,E)-1-((S)-1-((R)-3-hydroxy-3-methylpyrrolidin-1-yl)propan-2-yl)-7a-methyloctahydro-4H-inden-4-ylidene)ethylidene)cyclohexane-1,3-diol (Compound B075)

Compound B075 [9.5 mg, 0.023 mmol] was obtained by performing the reaction in the same manner as in Example 1 using compound (2b) [50.0 mg, 0.070 mmol] and (R)-3-methylpyrrolidin-3-ol hydrochloride [30 mg, 0.218 mmol].
¹H-NMR (CD₃OD) δ: 6.21 (1H, d, J = 11.2 Hz), 5.88 (1H, d, J = 10.7 Hz), 4.06-3.95 (2H, m), 2.83 (1H, dd, J= 12.2, 3.4 Hz), 2.73-2.52 (4H, m), 2.42-2.13 (6H, m), 2.07-1.48 (14H, m), 1.42-1.35 (2H, m), 1.33 (3H, s), 1.31-1.22 (2H, m), 1.05 (3H, d, J= 6.3 Hz), 0.59 (3H, s).
Exact Mass = 417.32 (C₂₆H₄₃NO₃) Obs. mass = 418.30 (M+H)

### [Example 88]

### Synthesis of (1R,3S)-5-(2-((1R,3aS,7aR,E)-1-((S)-1-((R)-3-hydroxy-3-methylpyrrolidin-1-yl)propan-2-yl)-7a-methyloctahydro-4H-inden-4-ylidene)ethylidene)-2-methylenecyclohexane-1,3-diol (Compound B076)

Compound B076[13.3 mg, 0.031 mmol] was obtained by performing the reaction in the same manner as in Example 1 using compound (2c) [50.0 mg, 0.069 mmol] and (R)-3-methylpyrrolidin-3-ol hydrochloride [30 mg, 0.218 mmol].
¹H-NMR (CD₃OD) δ: 6.26 (1H, d, J = 10.7 Hz), 5.91 (1H, d, J = 11.2 Hz), 5.05 (2H, d, J = 6.8 Hz), 4.42-4.36 (2H, m), 2.85 (1H, dd, J = 12.2, 3.9 Hz), 2.73-2.62 (3H, m), 2.58 (1H, dd, J = 14.6, 8.3 Hz), 2.48 (1H, dd, J = 13.4, 3.7 Hz), 2.40-2.16 (5H, m), 2.07-1.77 (5H, m), 1.70-1.49 (6H, m), 1.43-1.23 (6H, m), 1.06 (3H, d, J = 6.8 Hz), 0.60 (3H, s).
Exact Mass = 429.32 (C₂₇H₄₃NO₃) Obs. mass = 430.25 (M+H)

### [Example 89]

### Synthesis of (1R,3R)-5-(2-((1R,3aS,7aR,E)-1-((2S)-1-(3-cyclopropyl-3-hydroxypyrrolidin-1-yl)propan-2-yl)-7a-methyloctahydro-4H-inden-4-ylidene)ethylidene)-2-methylenecyclohexane-1,3-diol (B077)

Compound B077 [9.6 mg, 0.021 mmol] was obtained by performing the reaction in the same manner as in Example 1 using compound (2c) [50.0 mg, 0.069 mmol] and 3-cyclopropylpyrrolidin-3-ol hydrochloride [30 mg, 0.183 mmol].
¹H-NMR (CD₃OD) δ: 6.26 (1H, d, J = 11.2 Hz), 5.91 (1H, d, J = 11.2 Hz), 5.05 (2H, d, J = 6.8 Hz), 4.42-4.36 (2H, m), 2.99-2.24 (12H, m), 2.07-1.28 (16H, m), 1.08-1.00 (1H, m), 1.05 (3H, d, J = 6.3 Hz), 0.60 (3H, s), 0.45-0.30 (4H, m).
Exact Mass = 455.34 (C₂₉H₄₅NO₃) Obs. mass = 456.35 (M+H)

### [Example 90]

### Synthesis of (1R,3R)-5-(2-((1R,3aS,7aR,E)-7a-methyl-1-((2S)-1-(3-(trifluoromethyl)pyrrolidin-1-yl)propan-2-yl)octahydro-4H-inden-4-ylidene)ethylidene)cyclohexane-1,3-diol (Compound B078)

Compound B078 [7.2 mg, 0.016 mmol] was obtained by performing the reaction in the same manner as in Example 1 using compound (2b) [50.0 mg, 0.070 mmol] and 3-trifluoromethylpyrrolidine hydrochloride [48.3 mg, 0.275 mmol].

¹H-NMR (CD₃OD) δ: 6.21 (1H, d, J = 10.7 Hz), 5.89 (1H, d, J = 11.2 Hz), 4.06-3.95 (2H, m), 2.99-2.81 (3H, m), 2.69-2.49 (4H, m), 2.45-2.10 (6H, m), 2.07-1.48 (14H, m), 1.39-1.20 (4H, m), 1.04 (3H, d, J = 6.3 Hz), 0.60 (3H, s).

Exact Mass = 455.30 (C₂₆H₄₀F₃NO₂) Obs. mass = 456.30 (M+H)

### [Example 91]

### Synthesis of (1R,3R)-5-(2-((1R,3aS,7aR,E)-7a-methyl-1-((2S)-1-(3-(trifluoromethyl)pyrrolidin-1-yl)propan-2-yl)octahydro-4H-inden-4-ylidene)ethylidene)-2-methylenecyclohexane-1,3-diol (Compound B079)

Compound B079 [11.4 mg, 0.024 mmol] was obtained by performing the reaction in the same manner as in Example 1 using compound (2c) [100.0 mg, 0.137 mmol] and 3-trifluoromethylpyrrolidine hydrochloride [48.3 mg, 0.275 mmol].

Exact Mass = 467.30 (C₂₇H₄₀F₃NO₂) Obs. mass = 468.35 (M+H)

### [Example 92]

### Synthesis of (1R,3S,Z)-5-(2-((1R,3aS,7aR,E)-1-((S)-1-(4-hydroxy-4-(trifluoromethyl)piperidin-1-yl)propan-2-yl)-7a-methyloctahydro-4H-inden-4-ylidene)ethylidene)-4-methylenecyclohexane-1,3-diol (Compound C001)

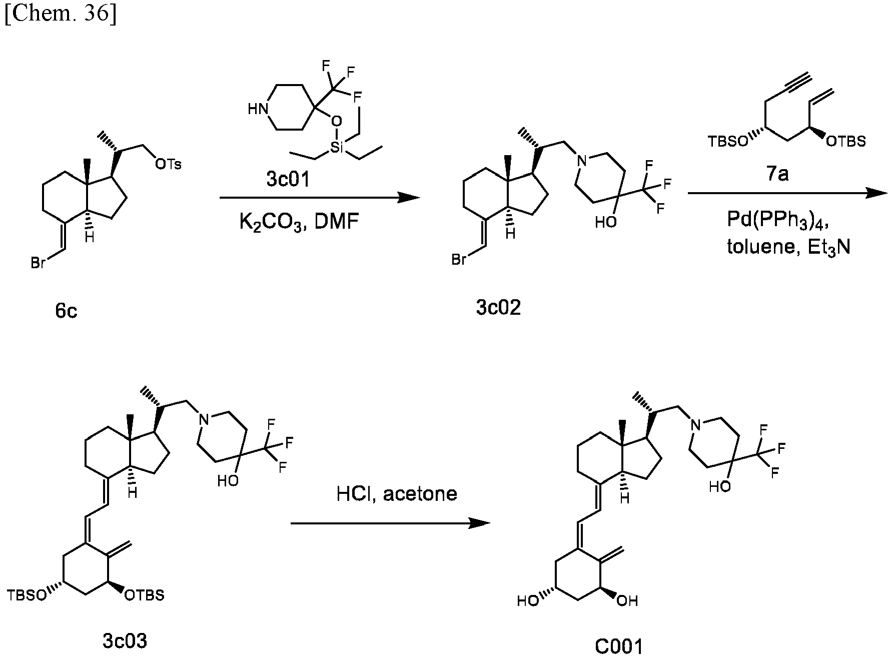

### Step 1

A solution of (2S)-2-((1R,3aS,7aR,E)-4-(bromomethylene)-7a-methyloctahydro-1H-inden-1-yl)propyl 4-methylbenzenesulfonate (Compound 6c, CAS Registry No. 173388-39-1) [388 mg, 0.879 mmol], triethyl-[[4-(trifluoromethyl)-4-piperidyl]oxy]silane (Compound 3c01) [653 mg, 2.30 mmol] and potassium carbonate [551 mg, 3.99 mmol] in DMF [2.5 mL] was heated and stirred at 100°C for 4 hours. The reaction mixture was poured into a saturated aqueous ammonium chloride solution and the mixture was extracted with ethyl acetate. The organic layer was dried over anhydrous magnesium sulfate and concentrated under reduced pressure. The residue was purified by silica gel column chromatography to obtain 1-((2S)-2-((1R,3aS,7aR,E)-4-(bromomethylene)-7a-methyloctahydro-1H-inden-1-yl)propyl)-4-(trifluoromethyl)piperidin-4-ol (Compound 3c02) [266.4 mg, 0.608 mmol].

¹H-NMR (CDCl₃) δ: 5.65 (1H, s), 2.91-2.85 (1H, m), 2.80 (1H, d, J = 11.2 Hz), 2.63 (1H, dd, J = 5.9 Hz), 4.15-4.06 (1H, m), 2.89-2.81 (2H, m), 2.63 (1H, d, J = 11.2 Hz), 2.51 (1H, dd, J = 11.2, 4.0 Hz), 2.37 (1H, td, J = 12.0, 2.3 Hz), 2.28 (1H, dd, J = 12.2, 3.4 Hz), 2.09-1.84 (8H, m), 1.68-1.23 (16H, m), 1.00 (3H, d, J = 6.9 Hz), 0.59 (3H, s).

### Step 2

Compound 3c02 [47.1 mg, 0.107 mmol] synthesized in step 1, (5R,7S)-2,2,3,3,9,9,10,10-octamethyl-5-(prop-2-yn-1-yl)-7-vinyl-4,8-dioxa-3,9-disilaundecane (Compound 7a, CAS Registry No. 161055-41-0) [49.4 mg, 0.134 mmol], and tetrakis(triphenylphosphine) palladium(0) [12.4 mg, 0.011 mmol] were dissolved in a mixture of toluene [0.5 mL] and triethylamine [0.5 mL], and the solution was heated and stirred at 100°C for 3 hours under a nitrogen atmosphere. The reaction mixture was concentrated under reduced pressure and roughly purified by thin-layer chromatography to obtain a crude product [73.9 mg] containing compound 3c03.

### Step 3

The crude product [73.9 mg] containing compound 3c03 obtained in step 2 was dissolved in acetone [1 mL], 6 N hydrochloric acid [0.2 mL] was added to the solution, and the mixture was stirred at room temperature for 8 hours. The reaction mixture was quenched, diluted with saturated brine, and extracted with ethyl acetate. The organic layer was dried over anhydrous magnesium sulfate and concentrated under reduced pressure. The residue was purified by preparative thin-layer chromatography to obtain compound C001 [6.3 mg, 0.013 mmol].

¹H-NMR (CD₃OD) δ: 6.32 (1H, d, J = 11.2 Hz), 6.08 (1H, d, J = 11.2 Hz), 5.28 (1H, t, J = 1.2 Hz), 4.34 (1H, t, J = 5.9 Hz), 4.15-4.06 (1H, m), 2.89-2.81 (2H, m), 2.63 (1H, d, J = 11.2 Hz), 2.51 (1H, dd, J = 13.2, 3.4 Hz), 2.41-2.23 (3H, m), 2.11-2.02 (3H, m), 1.98-1.21 (19H, m), 1.03 (3H, d, J = 6.3 Hz), 0.60 (3H, s).

Exact Mass = 497.31 (C₂₈H₄₂F₃NO₃) Obs. mass = 498.4 (M+H)

### [Example 93]

### Synthesis of (1R,3R)-5-(2-((1R,3aS,7aR,E)-1-((S)-1-(4-hydroxy-4-(trifluoromethyl)piperidin-1-yl)propan-2-yl)-7a-methyloctahydro-4H-inden-4-ylidene)ethylidene)cyclohexane-1,3-diol (Compound C002)

Compound C002 [7.2 mg, 0.015 mmol] was obtained by performing the reaction in the same manner as in Example 1 using compound (2b) [100 mg, 0.139 mmol] and triethyl-[[4-(trifluoromethyl)-4-piperidyl]oxy]silane [86.4 mg, 0.305 mmol].

¹H-NMR (CD₃OD) δ: 6.21 (1H, d, J = 11.0 Hz), 5.89 (1H, d, J = 11.4 Hz), 4.06-3.95 (2H, m), 2.87-2.80 (2H, m), 2.65-2.57 (2H, m), 2.44-2.28 (3H, m), 2.23-1.48 (20H, m), 1.38-1.24 (4H, m), 1.03 (3H, d, J = 6.4 Hz), 0.60 (3H, s).

Exact Mass = 485.31 (C₂₇H₄₂F₃NO₃) Obs. mass = 486.25 (M+H)

### [Example 94]

### Synthesis of (1R,2S,3S,Z)-5-(2-((1R,3aS,7aR,E)-1-((S)-1-(4-hydroxy-4-(trifluoromethyl)piperidin-1-yl)propan-2-yl)-7a-methyloctahydro-4H-inden-4-ylidene)ethylidene)-2-methyl-4-methylenecyclohexane-1,3-diol (Compound C003)

Compound C003 [10.0 mg, 0.019 mmol] was obtained by processing in the same manner as in steps 2 and 3 of Example 92 using compound 3c02 [46.8 mg, 0.107 mmol] obtained in step 1 of Example 92, (5R,6S,7R)-2,2,3,3,6,9,9,10,10-nonamethyl-5-(prop-2-yn-1-yl)-7-vinyl-4,8-dioxa-3,9-disilaundecane (Compound 7b) [50 mg, 0.131 mmol], and tetrakis(triphenylphosphine) palladium(0) [12.4 mg, 0.011 mmol].

¹H-NMR (CD₃OD) δ: 6.33 (1H, d, J = 11.2 Hz), 6.10 (1H, d, J = 11.2 Hz), 5.22 (1H, d, J = 2.0 Hz), 4.22 (1H, d, J = 3.4 Hz), 3.72 (1H, td, J = 8.1, 4.1 Hz), 3.17 (1H, d, J = 10.2 Hz), 3.00 (1H, d, J = 11.2 Hz), 2.89-2.75 (2H, m), 2.67 (1H, d, J = 12.7 Hz), 2.62-2.52 (2H, m), 2.42 (1H, t, J = 11.7 Hz), 2.19-1.97 (5H, m), 1.93-1.20 (13H, m), 1.08 (3H, d, J = 6.8 Hz), 1.03 (3H, d, J = 6.8 Hz), 0.61 (3H, s).

Exact Mass = 511.33 (C₂₉H₄₄F₃NO₃) Obs. mass = 512.4 (M+H)

### [Example 95]

### Synthesis of (1R,3R)-5-(2-((1R,3aS,7aR,E)-1-((S)-1-(4-hydroxy-4-(trifluoromethyl)piperidin-1-yl)propan-2-yl)-7a-methyloctahydro-4H-inden-4-ylidene)ethylidene)-2-methylenecyclohexane-1,3-diol (Compound C004)

Compound C004 [18.7 mg, 0.038 mmol] was obtained by performing the reaction in the same manner as in Example 1 using compound (2c) [60.0 mg, 0.082 mmol] and triethyl-[[4-(trifluoromethyl)-4-piperidyl]oxy]silane [70 mg, 0.247 mmol].

¹H-NMR (CD₃OD) δ: 6.27 (1H, d, J = 10.7 Hz), 5.92 (1H, d, J = 11.2 Hz), 5.05 (2H, d, J = 6.3 Hz), 4.43-4.34 (2H, m), 3.28-3.24 (1H, m), 3.10 (1H, d, J = 11.7 Hz), 2.95-2.64 (5H, m), 2.56-2.46 (2H, m), 2.32-2.25 (2H, m), 2.12-1.96 (6H, m), 1.87-1.29 (12H, m), 1.10 (3H, d, J = 6.3 Hz), 0.63 (3H, s).

Exact Mass = 497.31 (C₂₈H₄₂F₃NO₃) Obs. mass = 498.4 (M+H)

### [Example 96]

### Synthesis of (1R,3S,Z)-5-(2-((1R,3aS,7aR,E)-7a-methyl-1-((S)-1-(4-(trifluoromethyl)piperidin-1-yl)propan-2-yl)octahydro-4H-inden-4-ylidene)ethylidene)-4-methylenecyclohexane-1,3-diol (Compound C007)

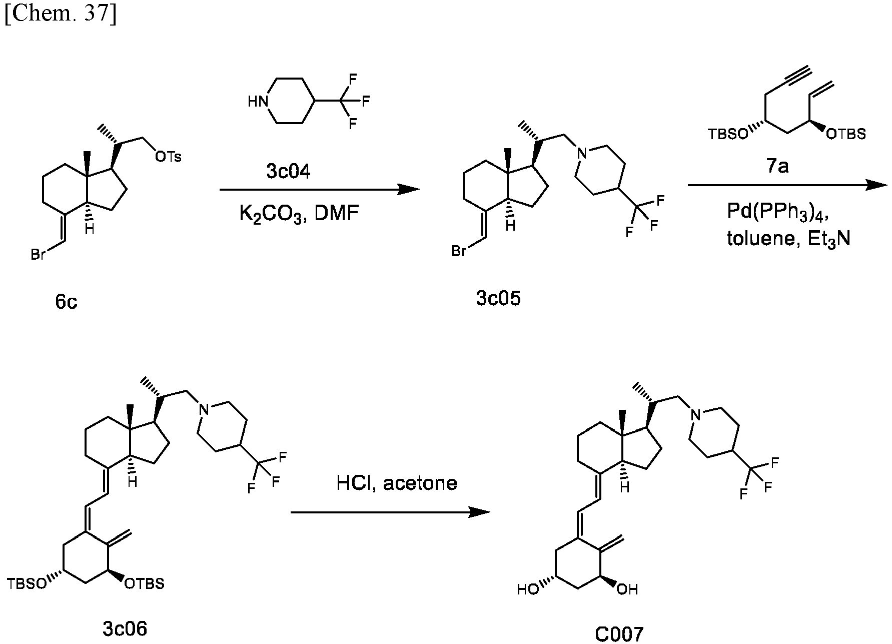

### Step 1

1-((2S)-2-((1R,3aS,7aR,E)-4-(Bromomethylene)-7a-methyloctahydro-1H-inden-1-yl)propyl)-4-(trifluoromethyl)piperidine (Compound 3c05) [36.0 mg, 0.085 mmol] was obtained by processing in the same manner as in step 1 of Example 92 using compound 6c [62.1 mg, 0.141 mmol] and 4-trifluoromethylpiperidine hydrochloride (Compound 3c04) [57.0 mg, 0.201 mmol] as starting materials.

### Step 2, Step 3

Compound C007 [5.5 mg, 0.011 mmol] was obtained by processing in the same manner as in steps 2 and 3 of Example 92 using 3c05 [36.0 mg, 0.085 mmol], compound 7a [41.0 mg, 0.111 mmol], and tetrakis(triphenylphosphine) palladium(0) [12.4 mg, 0.011 mmol] as raw materials.

¹H-NMR (CD₃OD) δ: 6.32 (1H, d, J = 11.2 Hz), 6.08 (1H, d, J = 11.7 Hz), 5.28 (1H, dd, J = 2.4, 1.5 Hz), 4.34 (1H, t, J = 5.9 Hz), 4.12 (1H, br s), 3.04 (1H, d, J = 11.2 Hz), 2.90-2.82 (2H, m), 2.53-2.49 (1H, m), 2.30-2.22 (3H, m), 2.09-1.22 (30H, m), 1.02 (3H, d, J = 6.8 Hz), 0.59 (3H, s).

Exact Mass = 481.32 (C₂₈H₄₂F₃NO₂) Obs. mass = 482.4 (M+H)

### [Example 97]

### Synthesis of (1R,3S)-5-(2-((1R,3aS,7aR,E)-7a-methyl-1-((S)-1-(4-(trifluoromethyl)piperidin-1-yl)propan-2-yl)octahydro-4H-inden-4-ylidene)ethylidene)cyclohexane-1,3-diol (Compound C008)

Compound C008 [9.8 mg, 0.021 mmol] was obtained by performing the reaction in the same manner as in Example 1 using compound (2b) [50.0 mg, 0.070 mmol] and 4-trifluoromethylpiperidine hydrochloride [30 mg, 0.196 mmol].

¹H-NMR (CD₃OD) δ: 6.21 (1H, d, J = 11.0 Hz), 5.88 (1H, d, J = 11.4 Hz), 4.06-3.95 (2H, m), 3.04 (1H, d, J = 11.4 Hz), 2.88-2.80 (2H, m), 2.59 (1H, dd, J = 13.3, 3.7 Hz), 2.40 (1H, dd, J = 13.3, 3.7 Hz), 2.26 (1H, dd, J = 12.1, 3.0 Hz), 2.23-1.73 (13H, m), 1.67-1.48 (8H, m), 1.38-1.22 (4H, m), 1.02 (3H, d, J = 6.4 Hz), 0.60 (3H, s).

Exact Mass = 469.32 (C₂₇H₄₂F₃NO₂) Obs. mass = 470.30 (M+H)

### [Example 98]

### Synthesis of (1R,3R)-5-(2-((1R,3aS,7aR,E)-7a-methyl-1-((S)-1-(4-(trifluoromethyl)piperidin-1-yl)propan-2-yl)octahydro-4H-inden-4-ylidene)ethylidene)-2-methylenecyclohexane-1,3-diol (Compound C009)

Compound C009 [7.6 mg, 0.016 mmol] was obtained by performing the reaction in the same manner as in Example 1 using compound (2c) [60.0 mg, 0.082 mmol] and 4-trifluoromethylpiperidine hydrochloride [50 mg, 0.326 mmol].

¹H-NMR (CD₃OD) δ: 6.26 (1H, d, J = 11.2 Hz), 5.91 (1H, d, J = 11.2 Hz), 5.05 (2H, d, J = 5.9 Hz), 4.43-4.35 (2H, m), 3.35-3.31 (1H, m), 3.14 (1H, d, J = 11.7 Hz), 2.86 (1H, dd, J = 12.4, 3.7 Hz), 2.69-2.04 (12H, m), 1.92-1.27 (13H, m), 1.07 (3H, d, J = 6.3 Hz), 0.62 (3H, s).

Exact Mass = 481.32 (C₂₈H₄₂F₃NO₂) Obs. mass = 482.4 (M+H)

### [Example 99]

### Synthesis of (1R,2S,3S,Z)-2-methyl-5-(2-((1R,3aS,7aR,E)-7a-methyl-1-((S)-1-(4-(trifluoromethyl)piperidin-1-yl)propan-2-yl)octahydro-4H-inden-4-ylidene)ethylidene)-4-methylenecyclohexane-1,3-diol (Compound C010)

Compound C010 [12.0 mg, 0.024 mmol] was obtained by performing the reaction in the same manner as in steps 2 and 3 of Example 92 using compound 3c05 [42.0 mg, 0.099 mmol] obtained in step 1 of Example 96, compound 7b [50 mg, 0.131 mmol], and tetrakis(triphenylphosphine) palladium(0) [20.0 mg, 0.017 mmol] as starting materials.

¹H-NMR (DMSO-D₆) δ: 6.19 (1H, d, J = 11.2 Hz), 5.99 (1H, d, J = 11.2 Hz), 5.17 (1H, d, J = 2.4 Hz), 4.75 (2H, dd, J = 11.7, 3.4 Hz), 4.57 (1H, d, J = 4.4 Hz), 4.15 (1H, t, J = 3.4 Hz), 3.63-3.59 (1H, m), 2.93 (1H, d, J = 11.2 Hz), 2.77 (2H, t, J = 12.7 Hz), 2.46 (1H, dd, J = 14.1, 3.4 Hz), 2.22-1.14 (27H, m), 0.95 (3H, d, J = 5.9 Hz), 0.85 (3H, d, J = 6.8 Hz), 0.52 (3H, s).

Exact Mass = 495.33 (C₂₉H₄₄F₃NO₂) Obs. mass = 496.4 (M+H)

### [Example 100]

### Synthesis of (1R,3S,Z)-5-(2-((1R,3aS,7aR,E)-1-((S)-1-(4-(difluoromethyl)piperidin-1-yl)propan-2-yl)-7a-methylcyclohexane-4H-inden-4-ylidene)ethylidene)-4-methylenecyclohexane-1,3-diol (Compound C013)

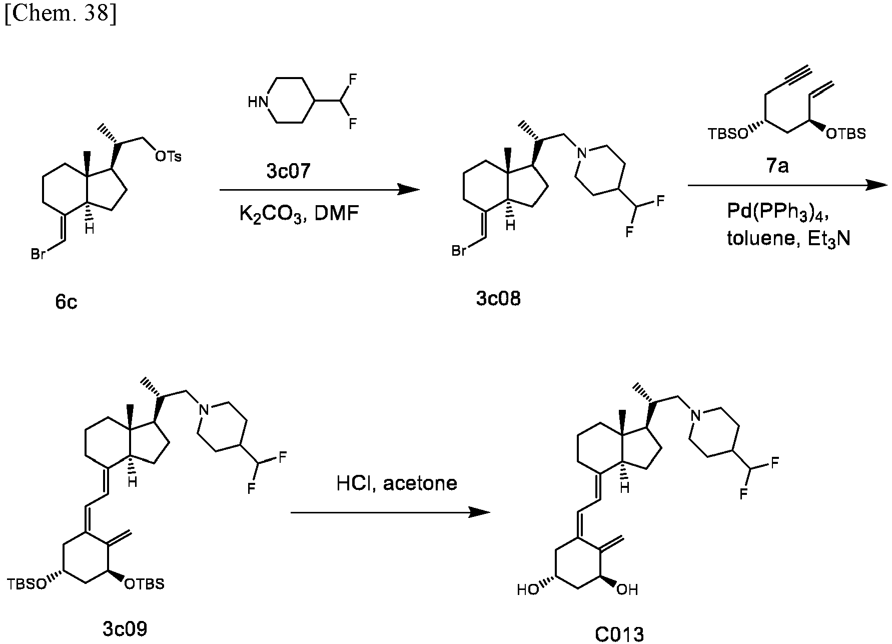

### Step 1

1-((2S)-2-((1R,3aS,7aR,E)-4-(Bromomethylene)-7a-methyloctahydro-1H-inden-1-yl)propyl)-4-(difluoromethyl)piperidine (Compound 3c08) [16.0 mg, 0.0394 mmol] was obtained by processing in the same manner as in step 1 of Example 92 using compound 6c [59.7 mg, 0.135 mmol] and 4-difluoromethylpiperidine hydrochloride (Compound 3c07) [79.4 mg, 0.294 mmol] as starting materials.

### Step 2, Step 3

Compound C013 [2.5 mg, 0.0054 mmol] was obtained by processing in the same manner as in steps 2 and 3 of Example 92 using 3c08 [16.0 mg, 0.0394 mmol], compound 7a [20.0 mg, 0.0542 mmol], and tetrakis(triphenylphosphine) palladium(0) [10.0 mg, 0.0086 mmol] as raw materials.

¹H-NMR (CD₃OD) δ: 6.32 (1H, d, J = 11.2 Hz), 6.08 (1H, d, J = 11.2 Hz), 5.68 (1H, td, J = 57.0, 5.0 Hz), 5.28 (1H, d, J = 1.5 Hz), 4.34 (1H, d, J = 5.9 Hz), 4.12 (1H, brs), 3.05-2.82 (3H, m), 2.51 (1H, dd, J = 10.2, 5.1 Hz), 2.28-2.22 (2H, m), 2.06-1.22 (22H, m), 1.02 (3H, d, J = 6.8 Hz), 0.59 (3H, s).

Exact Mass = 463.33 (C₂₈H₄₃F₂NO₂) Obs. mass = 464.4 (M+H)

### [Example 101]

### Synthesis of (1R,3R)-5-(2-((1R,3aS,7aR,E)-1-((S)-1-(4-(difluoromethyl)piperidin-1-yl)propan-2-yl)-7a-methyloctahydro-4H-inden-4-ylidene)ethylidene)cyclohexane-1,3-diol (Compound C014)

Compound C014 [8.3 mg, 0.018 mmol] was obtained by performing the reaction in the same manner as in Example 1 using compound (2b) [50.0 mg, 0.070 mmol] and 4-difluoromethylpiperidine hydrochloride [30 mg, 0.222 mmol].

¹H-NMR (CD₃OD) δ: 6.21 (1H, d, J = 11.0 Hz), 5.88 (1H, d, J = 11.0 Hz), 5.65 (1H, td, J = 56.8, 4.3 Hz), 4.05-3.95 (2H, m), 3.02 (1H, d, J = 11.0 Hz), 2.83 (2H, dd, J = 12.3, 3.0 Hz), 2.59 (1H, dd, J = 13.3, 3.7 Hz), 2.40 (1H, dd, J = 13.3, 3.2 Hz), 2.27-2.13 (3H, m), 2.05-1.21 (22H, m), 1.02 (3H, d, J = 6.4 Hz), 0.60 (3H, s).

Exact Mass = 451.33 (C₂₇H₄₃F₂NO₂) Obs. mass = 452.25 (M+H)

### [Example 102]

### Synthesis of (1R,3R)-5-(2-((1R,3aS,7aR,E)-1-((S)-1-(4-(difluoromethyl)piperidin-1-yl)propan-2-yl)-7a-methyloctahydro-4H-inden-4-ylidene)ethylidene)-2-methylenecyclohexane-1,3-diol (Compound C015)

Compound C015 [14.2 mg, 0.031 mmol] was obtained by performing the reaction in the same manner as in Example 1 using compound (2c) [50.0 mg, 0.069 mmol] and 4-difluoromethylpiperidine hydrochloride [35 mg, 0.204 mmol].

¹H-NMR (CD₃OD) δ: 6.27 (1H, d, J = 10.7 Hz), 5.91 (2H, d, J = 10.7 Hz), 5.76 (2H, td, J = 56.0, 5.0 Hz), 5.05 (2H, d, J = 5.9 Hz), 4.42-4.36 (2H, m), 3.48 (2H, t, J = 11.5 Hz), 2.88-2.00 (15H, m), 1.91-1.32 (13H, m), 1.10 (3H, d, J = 6.3 Hz), 0.64 (3H, s).

Exact Mass = 463.33 (C₂₈H₄₃F₂NO₂) Obs. mass = 464.40 (M+H)

### [Example 103]

### Synthesis of (1R,2S,3S,Z)-5-(2-((1R,3aS,7aR,E)-1-((S)-1-(4-(difluoromethyl)piperidin-1-yl)propan-2-yl)-7a-methyloctahydro-4H-inden-4-ylidene)ethylidene)-2-methyl-4-methylenecyclohexane-1,3-diol (Compound C016)

Compound C016 [8.0 mg, 0.017 mmol] was obtained by processing in the same manner as in steps 2 and 3 of Example 100 using compound 3c08 [35.7 mg, 0.093 mmol] obtained in step 1 of Example 100, compound 7b [50 mg, 0.131 mmol], and tetrakis(triphenylphosphine) palladium(0) [20.0 mg, 0.017 mmol] as starting materials.

¹H-NMR (CD₃OD) δ: 6.33 (1H, d, J = 11.2 Hz), 6.09 (1H, d, J = 11.2 Hz), 5.67 (1H, td, J = 56.8, 4.2 Hz), 5.22 (1H, d, J = 1.5 Hz), 4.22 (1H, d, J = 3.4 Hz), 3.72 (1H, td, J = 8.2, 4.2 Hz), 3.10 (1H, d, J = 11.2 Hz), 2.89 (2H, dd, J = 16.6, 11.7 Hz), 2.59 (1H, dd, J = 13.4, 4.1 Hz), 2.35 (1H, t, J = 6.1 Hz), 2.21-1.90 (7H, m), 1.86-1.26 (19H, m), 1.03 (6H, d, J = 6.8 Hz), 0.59 (3H, s).

Exact Mass = 477.34 (C₂₉H₄₅F₂NO₂) Obs. mass = 478.4 (M+H)

### [Example 104]

### Synthesis of (1R,3S,Z)-5-(2-((1R,3aS,7aR,E)-1-((S)-1-(4-(difluoromethyl)-4-hydroxypiperidin-1-yl)propan-2-yl)-7a-methyloctahydro-4H-inden-4-ylidene)ethylidene)-4-methylenecyclohexane-1,3-diol (Compound C021)

Compound C021 [18.3 mg, 0.038 mmol] was obtained by performing the reaction in the same manner as in Example 1 using compound (2a) [90.0 mg, 0.123 mmol] and 4-(difluoromethyl)-4-((trimethylsilyl)oxy)piperidine [83.2 mg, 0.373 mmol].

¹H-NMR (CD₃OD) δ: 6.32 (1H, d, J = 11.2 Hz), 6.08 (1H, d, J = 11.2 Hz), 5.51 (1H, t, J = 56.4 Hz), 5.28 (1H, t, J = 1.2 Hz), 4.89 (1H, d, J = 2.4 Hz), 4.34 (1H, t, J = 6.0 Hz), 4.16-4.09 (1H, m), 2.86 (1H, dd, J = 12.2, 3.4 Hz), 2.79 (1H, d, J = 10.7 Hz), 2.60 (1H, d, J = 11.2 Hz), 2.51 (1H, dd, J = 13.2, 3.4 Hz), 2.45-2.36 (1H, m), 2.34-2.03 (7H, m), 1.98-1.27 (21H, m), 1.03 (3H, d, J = 6.3 Hz), 0.60 (3H, s).

Exact Mass = 479.32 (C₂₈H₄₃F₂NO₃) Obs. mass = 480.4 (M+H)

### [Example 105]

### Synthesis of (1R,3R)-5-(2-((1R,3aS,7aR,E)-1-((S)-1-(4-(difluoromethyl)-4-hydroxypiperidin-1-yl)propan-2-yl)-7a-methyloctahydro-4H-inden-4-ylidene)ethylidene)-2-methylenecyclohexane-1,3-diol (Compound C022)

Compound C022 [13.4 mg, 0.028 mmol] was obtained by performing the reaction in the same manner as in Example 1 using compound (2c) [50.0 mg, 0.069 mmol] and 4-(difluoromethyl)-4-((trimethylsilyl)oxy)piperidine [38.5 mg, 0.172 mmol]. ¹H-NMR (CD₃OD) δ: 6.27 (1H, d, J = 11.2 Hz), 5.92 (1H, d, J = 11.2 Hz), 5.63 (1H, t, J = 56.1 Hz), 5.05 (2H, d, J = 6.3 Hz), 4.42-4.35 (2H, m), 3.19-2.25 (11H, m), 2.09-1.96 (5H, m), 1.90-1.28 (13H, m), 1.11 (3H, d, J = 6.3 Hz), 0.64 (3H, s).

Exact Mass = 479.32 (C₂₈H₄₃F₂NO₃) Obs. mass = 480.30 (M+H)

### [Example 106]

### Synthesis of (1R,2S,3S,Z)-5-(2-((1R,3aS,7aR,E)-1-((S)-1-(4-(difluoromethyl)-4-hydroxypiperidin-1-yl)propan-2-yl)-7a-methyloctahydro-4H-inden-4-ylidene)ethylidene)-2-methyl-4-methylenecyclohexane-1,3-diol (Compound C023)

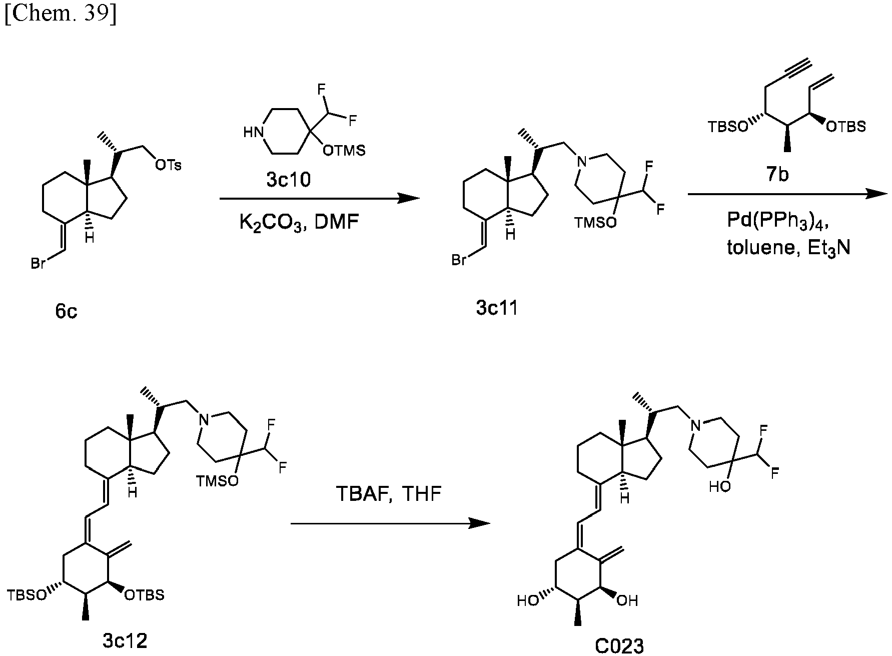

### Step 1

1-((2S)-2-((1R,3aS,7aR,E)-4-(Bromomethylene)-7a-methyloctahydro-1H-inden-1-yl)propyl)-4-(difluoromethyl)-4-((trimethylsilyl)oxy)piperidine (Compound 3c11) [54.2 mg, 0.110 mmol] was obtained by processing in the same manner as in step 1 of Example 92 using compound 6c [89.2 mg, 0.202 mmol] and 4-(difluoromethyl)-4-((trimethylsilyl)oxy)piperidine [94.3 mg, 0.422 mmol] as starting materials.

### Step 2, Step 3

Compound C023 [16.4 mg, 0.033 mmol] was obtained by processing in the same manner as in steps 2 and 3 of Example 92 using 3c11 [50.7 mg, 0.103 mmol], compound 7b [49.5 mg, 0.129 mmol], and tetrakis(triphenylphosphine) palladium(0) [23.0 mg, 0.020 mmol] as raw materials.

¹H-NMR (CD₃OD) δ: 6.33 (1H, d, J = 11.2 Hz), 6.09 (1H, d, J = 11.2 Hz), 5.51 (1H, t, J = 56.4 Hz), 5.22 (1H, d, J = 2.0 Hz), 4.89 (1H, d, J = 2.4 Hz), 4.22 (1H, d, J = 3.4 Hz), 3.72 (1H, td, J = 8.2, 4.2 Hz), 2.86 (1H, dd, J = 12.0, 3.7 Hz), 2.78 (1H, d, J = 11.2 Hz), 2.59 (2H, dd, J = 13.2, 3.9 Hz), 2.40 (1H, td, J = 11.6, 2.1 Hz), 2.30 (1H, dd, J = 12.2, 2.4 Hz), 2.19-1.20 (23H, m), 1.04 (6H, d, J = 8.0 Hz), 1.03 (6H, d, J = 6.3 Hz), 0.59 (3H, s). Exact Mass = 493.34 (C₂₉H₄₅F₂NO₃) Obs. mass = 494.40 (M+H)

### [Example 107]

### Synthesis of (1R,3S,Z)-5-(2-((1R,3aS,7aR,E)-1-((S)-1-(4-hydroxy-4-methylpiperidin-1-yl)propan-2-yl)-7a-methyloctahydro-4H-inden-4-ylidene)ethylidene)-4-methylenecyclohexane-1,3-diol (Compound C028)

Compound C028 [10.0 mg, 0.0225 mmol] was obtained by performing the reaction in the same manner as in Example 1 using compound (2a) [90.0 mg, 0.069 mmol] and 4-methylpiperidin-4-ol [42 mg, 0.365 mmol].

¹H-NMR (CD₃OD) δ: 6.32 (1H, d, J = 11.2 Hz), 6.09 (1H, d, J = 11.2 Hz), 5.28 (1H, dd, J = 2.4, 1.5 Hz), 4.35 (1H, t, J = 6.1 Hz), 4.15-4.10 (1H, m), 3.50-3.18 (4H, m), 3.10-2.92 (2H, m), 2.89-2.86 (1H, m), 2.77 (1H, t, J = 12.0 Hz), 2.51 (1H, dd, J = 13.2, 3.4 Hz), 2.26 (1H, dd, J= 13.2, 6.8 Hz), 2.08-2.01 (3H, m), 1.95-1.30 (17H, m), 1.28 (3H, s), 1.12 (3H, d, J = 6.3 Hz), 0.64 (3H, s).

Exact Mass = 443.34 (C₂₈H₄₅NO₃) Obs. mass = 444.40 (M+H)

### [Example 108]

### Synthesis of (1R,3S,Z)-5-(2-((1R,3aS,7aR,E)-1-((2S)-1-(3-hydroxy-3-(trifluoromethyl)piperidin-1-yl)propan-2-yl)-7a-methyloctahydro-4H-inden-4-ylidene)ethylidene)-4-methylenecyclohexane-1,3-diol (Compound C030a, Compound C030b)

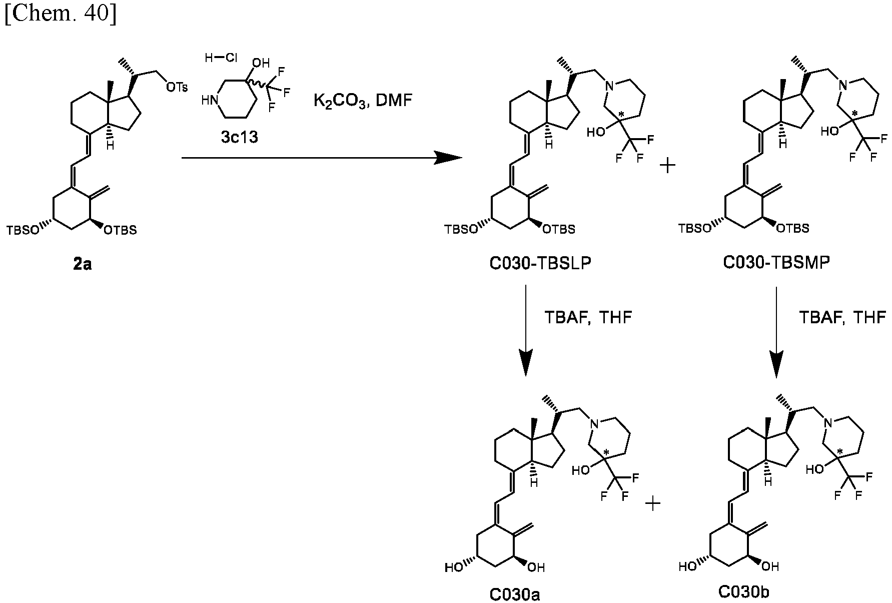

### Step 1

A solution of compound (2a) [100 mg, 0.137 mmol], 3-(trifluoromethyl)piperidin-3-ol hydrochloride (Compound 3c13) [60 mg 0.292 mmol], and potassium carbonate [60 mg, 0.434 mmol] in DMF [1 mL] was stirred at 60°C overnight. The reaction mixture was quenched with saturated brine and extracted with ethyl acetate. The organic layer was dried over anhydrous magnesium sulfate and concentrated under reduced pressure. The residue was purified by silica gel column chromatography to afford the target product with the low polarity (Compound C030-TBSLP) [21.2 mg, 0.0292 mmol] and the target product with the high polarity (Compound C030-TBSMP) [22.3 mg, 0.0307 mmol].

### Step 2a

TBAF [1 M in THF, 0.5 mL, 0.5 mmol] was added to a solution of compound C030-TBSLP [21.2 mg, 0.0292 mmol] in THF [1 mL], and the mixture was stirred at 50°C overnight. The reaction mixture was quenched with saturated magnesium hydrogen carbonate, and the organic layer was washed with saturated brine, dried over anhydrous magnesium sulfate, and concentrated under reduced pressure. The residue was purified by HPLC to obtain compound C030a [10.3 mg, 0.0207 mmol].

¹H-NMR (CD₃OD) δ: 6.32 (1H, d, J = 11.2 Hz), 6.08 (1H, d, J = 11.2 Hz), 5.28 (1H, t, J = 1.2 Hz), 4.34 (1H, t, J = 5.9 Hz), 4.15-4.09 (1H, m), 3.05-2.93 (1H, m), 2.87 (1H, dd, J = 12.2, 3.4 Hz), 2.80 (1H, d, J = 11.7 Hz), 2.53-2.44 (4H, m), 2.25 (1H, dd, J = 13.2, 6.8 Hz), 2.13 (2H, t, J = 11.5 Hz), 2.07-2.00 (4H, m), 1.94-1.24 (20H, m), 1.06 (3H, d, J = 6.3 Hz), 0.61 (3H, s).

Exact Mass = 497.31 (C₂₈H₄₂F₃NO₃) Obs. mass = 498.40 (M+H)

### Step 2b

Compound C030b [9.7 mg, 0.019 mmol] was obtained from Compound C030-TBSMP [22.3 mg, 0.0307 mmol] by performing the reaction in the same manner as in step 2a.

¹H-NMR (CD₃OD) δ: 6.32 (1H, d, J = 11.2 Hz), 6.09 (1H, d, J = 11.2 Hz), 5.28 (1H, dd, J = 2.4, 1.5 Hz), 4.34 (1H, t, J = 5.9 Hz), 4.15-4.09 (1H, m), 2.98 (1H, d, J = 11.2 Hz), 2.87 (2H, d, J = 11.2 Hz), 2.53 (2H, td, J = 12.3, 3.1 Hz), 2.45-1.98 (9H, m), 1.92-1.23 (18H, m), 1.05 (3H, d, J = 6.3 Hz), 0.61 (3H, s).

Exact Mass = 497.31 (C₂₈H₄₂F₃NO₃) Obs. mass = 498.40 (M+H)

### [Example 109]

### Synthesis of (1R,2S,3S,Z)-5-(2-((1R,3aS,7aR,E)-1-((2S)-1-(3-hydroxy-3-(trifluoromethyl)piperidin-1-yl)propan-2-yl)-7a-methyloctahydro-4H-inden-4-ylidene)ethylidene)-2-methyl-4-methylenecyclohexane-1,3-diol (Compound C031a, Compound C031b)

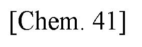

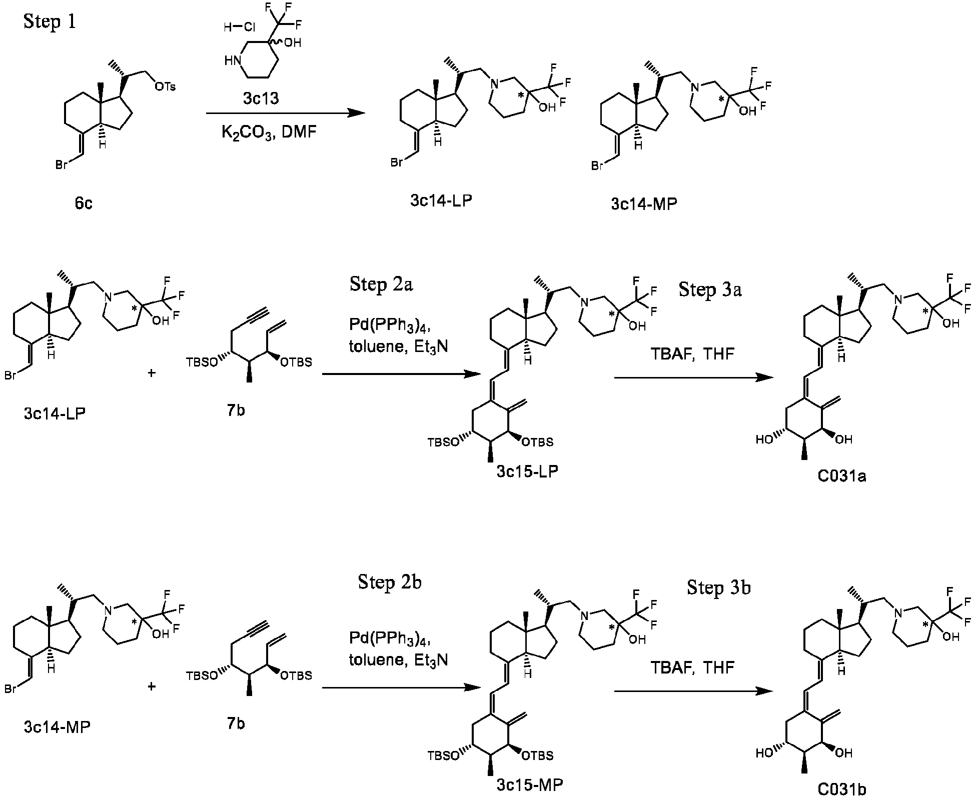

### Step 1

Potassium carbonate [390 mg, 2.82 mmol] was added to a solution [4 mL] of compound 6c [220.6 mg, 0.500 mmol] and 3-(trifluoromethyl)piperidin-3-ol hydrochloride (compound 3c13) [315 mg, 1.53 mmol] in DMF [4 mL] at room temperature, and the reaction mixture was heated and stirred at 60°C for 20 hours. The reaction mixture was cooled to room temperature and poured into saturated brine, and the mixture was extracted with ethyl acetate. The organic layer was dried over anhydrous magnesium sulfate and concentrated under reduced pressure. The residue was purified by silica gel column chromatography to afford the low-polarity compound 3c14-LP [65.6 mg, 0.150 mmol] and the high-polarity compound 3c14-MP [57.2 mg, 0.130 mmol].

### Steps 2a and 3a (Synthesis of Compound C031a)

### Step 2a

Tetrakis(triphenylphosphine) palladium(0) [15 mg, 0.013 mmol] was added to a mixed solution of compound 3c14-LP [29.9 mg, 0.0682 mmol] obtained in step 1 and compound 7b [38.7 mg, 0.101 mmol] in toluene [1 mL]/triethylamine [1 mL], and the mixture was heated and stirred at 100°C for 2 hours under a nitrogen atmosphere. After cooling to room temperature, the reaction mixture was concentrated under reduced pressure. The residue was roughly purified by silica gel column chromatography to obtain compound 3c15-LP [38.5 mg].

### Step 3a

TBAF [1 M in THF, 0.6 mL, 0.6 mmol] was added to a solution of compound 3c15-LP [38.5 mg, 0.052 mmol] in THF [1 mL], and the mixture was heated and stirred at 60°C overnight. After cooling to room temperature, a saturated aqueous sodium hydrogen carbonate solution was added to the reaction mixture, and the mixture was extracted with ethyl acetate. The organic layer was dried over anhydrous magnesium sulfate and concentrated under reduced pressure. The residue was purified to obtain compound C031a [10.5 mg, 0.0205 mmol].

¹H-NMR (CD₃OD) δ: 6.33 (1H, d, J = 10.7 Hz), 6.09 (1H, d, J = 11.2 Hz), 5.22 (1H, d, J = 2.0 Hz), 4.22 (1H, d, J = 3.4 Hz), 3.72 (1H, td, J = 8.1, 4.2 Hz), 2.97 (1H, d, J = 11.2 Hz), 2.86 (1H, dd, J = 11.7, 3.4 Hz), 2.77 (1H, d, J = 11.7 Hz), 2.59 (1H, dd, J = 13.4, 4.1 Hz), 2.47-2.41 (2H, m), 2.19-2.00 (5H, m), 1.94-1.23 (17H, m), 1.06 (3H, d, J = 6.3 Hz), 1.03 (3H, d, J = 6.8 Hz), 0.59 (3H, s).

Exact Mass = 511.33 (C₂₉H₄₄F₃NO₃) Obs. mass = 512.4 (M+H)

### Step 2b, Step 3b (Synthesis of Compound C031b)

Compound C031b [11.5 mg, 0.0225 mmol] was obtained by processing in the same manner as in steps 2a and 3a using compound 3c14-MP [26.6 mg, 0.0595 mmol] obtained in step 1 and compound 7b [33.2 mg, 0.0868 mmol] as starting materials.

¹H-NMR (DMSO-D₆) δ: 6.19 (1H, d, J = 11.2 Hz), 5.99 (1H, d, J = 11.2 Hz), 5.58 (1H, s), 5.17 (1H, d, J = 2.4 Hz), 4.77 (1H, d, J = 2.9 Hz), 4.75 (1H, d, J = 4.4 Hz), 4.57 (1H, d, J = 3.4 Hz), 4.15 (1H, t, J = 2.4 Hz), 3.61 (1H, br s), 2.79 (1H, dd, J = 12.7, 3.9 Hz), 2.66 (1H, d, J = 10.7 Hz), 2.46 (2H, dd, J = 14.9, 3.7 Hz), 2.18 (1H, dd, J = 12.2, 2.9 Hz), 2.11-1.13 (23H, m), 0.96 (3H, d, J = 6.3 Hz), 0.85 (3H, d, J = 6.8 Hz), 0.52 (3H, s).

Exact Mass = 511.33 (C₂₉H₄₄F₃NO₃) Obs. mass = 512.4 (M+H)

### [Example 110]

### Synthesis of (1R,3R)-5-(2-((1R,3aS,7aR,E)-1-((2S)-1-(3-hydroxy-3-(trifluoromethyl)piperidin-1-yl)propan-2-yl)-7a-methyloctahydro-4H-inden-4-ylidene)ethylidene)-2-methylenecyclohexane-1,3-diol (Compound C032a, Compound C032b)

Compound C032a [11.0 mg, 0.0221 mmol] and compound C032b [14.4 mg, 0.0289 mmol] were obtained by performing the reaction in the same manner as in Example 108 using compound (2c) [110 mg, 151 mmol] and 3-(trifluoromethyl)piperidin-3-ol hydrochloride (Compound 3c13) [125 mg 0.608 mmol] as starting materials.

### Compound C032a

¹H-NMR (CD₃OD) δ: 6.26 (1H, d, J = 11.2 Hz), 5.91 (1H, d, J = 11.2 Hz), 5.05 (2H, d, J = 6.3 Hz), 4.39 (2H, ddd, J = 13.9, 7.1, 4.4 Hz), 3.00 (1H, d, J = 10.7 Hz), 2.88-2.77 (2H, m), 2.67 (1H, dd, J = 13.2, 4.4 Hz), 2.50-2.46 (3H, m), 2.31-2.25 (2H, m), 2.16-2.00 (5H, m), 1.98-1.48 (12H, m), 1.39-1.21 (3H, m), 1.07 (3H, d, J = 6.3 Hz), 0.62 (3H, s).

Exact Mass = 497.31 (C₂₈H₄₂F₃NO₃) Obs. mass = 498.40 (M+H)

### Compound C032b

¹H-NMR (CD₃OD) δ: 6.26 (1H, d, J = 11.2 Hz), 5.91 (1H, d, J = 11.2 Hz), 5.05 (2H, d, J = 7.0 Hz), 4.42-4.33 (2H, m), 3.03 (1H, d, J = 11.7 Hz), 2.93 (1H, d, J = 11.7 Hz), 2.86 (1H, dd, J = 12.2, 3.4 Hz), 2.69-2.60 (2H, m), 2.50-2.25 (6H, m), 2.09-2.00 (4H, m), 1.93-1.25 (14H, m), 1.06 (3H, d, J = 6.3 Hz), 0.63 (3H, s).

Exact Mass = 497.31 (C₂₈H₄₂F₃NO₃) Obs. mass = 498.40 (M+H)

### [Example 111]

### Synthesis of (1R,3S,Z)-5-(2-((1R,3aS,7aR,E)-1-((2S)-1-(3-(difluoromethyl)-3-hydroxypiperidin-1-yl)propan-2-yl)-7a-methyloctahydro-4H-inden-4-ylidene)ethylidene)-4-methylenecyclohexane-1,3-diol (Compound C034a, Compound C034b)

Compound C034a [8.2 mg, 0.017 mmol] and compound C034b [3.7 mg, 0.0077 mmol] were obtained by performing the reaction in the same manner as in Example 108 using compound (2a) [100 mg, 0.137 mmol] and 3-(difluoromethyl)piperidin-3-ol hydrochloride [50 mg 0.267 mmol] as starting materials.

### Compound C034a

¹H-NMR (CD₃OD) δ: 6.33 (1H, d, J = 11.2 Hz), 6.09 (1H, d, J = 11.2 Hz), 5.85 (1H, t, J = 55.9 Hz), 5.29 (1H, dd, J = 2.4, 1.0 Hz), 4.35 (1H, t, J = 5.9 Hz), 4.16-4.10 (1H, m), 2.90-2.75 (3H, m), 2.54-2.42 (4H, m), 2.32-2.18 (2H, m), 2.07-1.99 (3H, m), 1.94-1.24 (18H, m), 1.06 (3H, d, J = 6.3 Hz), 0.61 (3H, s).

Exact Mass = 479.32 (C₂₈H₄₃F₂NO₃) Obs. mass = 480.45 (M+H)

### Compound C034b

¹H-NMR (CD₃OD) δ: 6.33 (1H, d, J = 10.7 Hz), 6.09 (1H, d, J = 11.2 Hz), 5.84 (1H, t, J = 55.9 Hz), 5.29 (1H, t, J = 1.2 Hz), 4.35 (1H, t, J = 5.9 Hz), 4.16-4.10 (1H, m), 2.88 (1H, dd, J = 12.4, 3.2 Hz), 2.67-2.40 (6H, m), 2.26 (1H, dd, J = 13.4, 7.1 Hz), 2.15 (1H, t, J = 11.2 Hz), 2.09-1.98 (2H, m), 1.91-1.22 (17H, m), 1.04 (3H, d, J = 6.3 Hz), 0.61 (3H, s). Exact Mass = 479.32 (C₂₈H₄₃F₂NO₃) Obs. mass = 480.35 (M+H)

### [Example 112]

### Synthesis of (1R,3R)-5-(2-((1R,3aS,7aR,E)-1-((2S)-1-(3-(difluoromethyl)-3-hydroxypiperidin-1-yl)propan-2-yl)-7a-methyloctahydro-4H-inden-4-ylidene)ethylidene)cyclohexane-1,3-diol (Compound C035a, Compound C035b)

Compound C035a [15.5 mg, 0.033 mmol] and compound C035b [15.0 mg, 0.032 mmol] were obtained by performing the reaction in the same manner as in Example 108 using compound (2b) [116.1 mg, 0.162 mmol] and 3-(difluoromethyl)piperidin-3-ol hydrochloride [70.0 mg 0.373 mmol] as starting materials.

### Compound C035a

¹H-NMR (CD₃OD) δ: 6.22 (1H, d, J = 11.2 Hz), 5.89 (1H, d, J = 11.2 Hz), 5.89 (1H, t, J = 56.0 Hz), 4.07-3.96 (2H, m), 2.84 (1H, dd, J = 11.5, 4.1 Hz), 2.67-2.55 (2H, m), 2.49-2.35 (2H, m), 2.29-2.04 (7H, m), 2.01-1.21 (19H, m), 1.02 (3H, d, J = 6.3 Hz), 0.60 (3H, s).

Exact Mass = 467.32 (C₂₇H₄₃F₂NO₃) Obs. mass = 468.35 (M+H)

### Compound C035b

¹H-NMR (CD₃OD) δ: 6.21 (1H, d, J = 11.2 Hz), 5.88 (1H, d, J = 11.2 Hz), 5.85 (1H, t, J = 56.0 Hz), 4.06-3.94 (2H, m), 2.83 (1H, dd, J = 11.5, 3.7 Hz), 2.59 (1H, dd, J = 13.2, 3.4 Hz), 2.46-1.50 (28H, m), 1.37-1.25 (3H, m), 1.02 (3H, d, J = 6.3 Hz), 0.59 (3H, s). Exact Mass = 467.32 (C₂₇H₄₃F₂NO₃) Obs. mass = 468.35 (M+H)

### [Example 113]

### Synthesis of (1R,3R)-5-(2-((1R,3aS,7aR,E)-1-((2S)-1-(3-(difluoromethyl)-3-hydroxypiperidin-1-yl)propan-2-yl)-7a-methyloctahydro-4H-inden-4-ylidene)ethylidene)-2-methylenecyclohexane-1,3-diol (Compound C036a, Compound C036b)

Compound C036a [17.0 mg, 0.0354 mmol] and compound C036b [14.5 mg, 0.030 mmol] were obtained by performing the reaction in the same manner as in Example 108 using compound (2c) [100 mg 0.137 mmol] and 3-(difluoromethyl)piperidin-3-ol hydrochloride [49.8 mg 329 mmol] as starting materials.

### Compound C036a

¹H-NMR (CD₃OD) δ: 6.27 (1H, d, J = 11.2 Hz), 5.92 (1H, d, J = 11.2 Hz), 5.84 (1H, t, J = 57.0 Hz), 5.06 (2H, d, J = 6.3 Hz), 4.40 (2H, ddd, J = 14.1, 7.1, 4.4 Hz), 2.86 (1H, dd, J = 12.2, 3.4 Hz), 2.80 (2H, d, J = 11.7 Hz), 2.68 (1H, dd, J = 13.4, 4.1 Hz), 2.56 (1H, dd, J = 11.7, 3.2 Hz), 2.51-2.45 (3H, m), 2.33-2.20 (3H, m), 2.10-2.00 (2H, m), 1.94-1.26 (14H, m), 1.07 (3H, d, J = 6.3 Hz), 0.63 (3H, s).

Exact Mass = 479.32 (C₂₈H₄₃F₂NO₃) Obs. mass = 480.40 (M+H)

### Compound C036b

¹H-NMR (CD₃OD) δ: 6.28 (1H, d, J = 10.7 Hz), 5.93 (1H, d, J = 10.0 Hz), 5.81 (1H, t, J = 55.0 Hz), 5.06 (2H, d, J = 7.0 Hz), 4.45-4.36 (2H, m), 3.01-1.99 (16H, m), 1.94-1.29 (14H, m), 1.07 (3H, d, J = 6.3 Hz), 0.65 (3H, s).

Exact Mass = 479.32 (C₂₈H₄₃F₂NO₃) Obs. mass = 480.40 (M+H)

### [Example 114]

### Synthesis of (1R,3S,Z)-5-(2-((1R,3aS,7aR,E)-1-((2S)-1-(3-hydroxy-3-methylpiperidin-1-yl)propan-2-yl)-7a-methyloctahydro-4H-inden-4-ylidene)ethylidene)-4-methylenecyclohexane-1,3-diol (Compound C037a, Compound C037b)

Compound C037a [8.1 mg, 0.018 mmol] and compound C037b [10.2 mg, 0.023 mmol] were obtained by performing the reaction in the same manner as in Example 108 using compound (2a) [90 mg, 0.123 mmol] and 3-methylpiperidin-3-ol [42 mg, 0.365 mmol] as starting materials.

### Compound C037a

¹H-NMR (CD₃OD) δ: 6.32 (1H, d, J = 11.0 Hz), 6.08 (1H, d, J = 11.0 Hz), 5.28 (1H, s), 4.34 (1H, t, J = 5.9 Hz), 4.12-4.06 (1H, m), 2.87 (1H, dd, J = 12.3, 3.0 Hz), 2.51 (1H, dd, J = 13.3, 3.2 Hz), 2.28-1.21 (24H, m), 1.18 (3H, s), 1.03 (3H, d, J = 6.4 Hz), 0.59 (3H, s).

### Compound C037b

¹H-NMR (DMSO-D₆) δ: 6.19 (1H, d, J = 11.4 Hz), 5.98 (1H, d, J = 11.4 Hz), 5.22 (1H, d, J = 1.4 Hz), 4.88 (1H, d, J = 5.0 Hz), 4.75 (1H, d, J = 1.8 Hz), 4.56 (1H, d, J = 3.7 Hz), 4.25-3.95 (3H, m), 2.79 (1H, dd, J = 11.0, 4.0 Hz), 2.36 (1H, d, J = 13.7 Hz), 2.18-1.15 (23H, m), 1.11 (3H, s), 0.96 (3H, d, J = 6.4 Hz), 0.52 (3H, s).

### [Example 115]

### Synthesis of (1R,3R)-5-(2-((1R,3aS,7aR,E)-1-((S)-1-((R)-3-hydroxy-3-methylpiperidin-1-yl)propan-2-yl)-7a-methyloctahydro-4H-inden-4-ylidene)ethylidene)cyclohexane-1,3-diol (Compound C038)

Compound C038 [13.9 mg, 0.032 mmol] was obtained by performing the reaction in the same manner as in Example 1 using compound (2b) [50 mg, 0.070 mmol] and (R)-3-methylpiperidin-3-ol hydrochloride [30 mg, 0.198 mmol] as starting materials. ¹H-NMR (CD₃OD) δ: 6.21 (1H, d, J = 11.2 Hz), 5.88 (1H, d, J = 11.2 Hz), 4.05-3.94 (2H, m), 2.83 (1H, dd, J = 12.2, 3.9 Hz), 2.59 (1H, dd, J = 13.2, 3.4 Hz), 2.42-2.13 (7H, m), 2.07-1.21 (21H, m), 1.18 (3H, s), 1.03 (3H, d, J = 6.3 Hz), 0.60 (3H, s).

Exact Mass = 431.34 (C₂₇H₄₅NO₃) Obs. mass = 432.30 (M+H)

### [Example 116]

### Synthesis of (1R,3R)-5-(2-((1R,3aS,7aR,E)-1-((S)-1-((S)-3-hydroxy-3-methylpiperidin-1-yl)propan-2-yl)-7a-methyloctahydro-4H-inden-4-ylidene)ethylidene)cyclohexane-1,3-diol (Compound C039)

Compound C039 [10.9 mg, 0.025 mmol] was obtained by performing the reaction in the same manner as in Example 1 using compound (2b) [50 mg, 0.070 mmol] and (S)-3-methylpiperidin-3-ol [30 mg, 0.198 mmol] as starting materials.

¹H-NMR (CD₃OD) δ: 6.21 (1H, d, J = 11.2 Hz), 5.88 (1H, d, J = 11.2 Hz), 4.06-3.95 (2H, m), 2.83 (1H, dd, J = 12.2, 3.4 Hz), 2.59 (1H, dd, J = 13.2, 3.4 Hz), 2.40 (1H, dd, J = 13.2, 3.4 Hz), 2.31-1.20 (26H, m), 1.18 (3H, s), 1.03 (3H, d, J = 6.3 Hz), 0.60 (3H, s).

Exact Mass = 431.34 (C₂₇H₄₅NO₃) Obs. mass = 432.30 (M+H)

### [Example 117]

### Synthesis of (1R,3R)-5-(2-((1R,3aS,7aR,E)-1-((S)-1-((R)-3-hydroxy-3-methylpiperidin-1-yl)propan-2-yl)-7a-methyloctahydro-4H-inden-4-ylidene)ethylidene)-2-methylenecyclohexane-1,3-diol (Compound C040)

Compound C040 [7.3 mg, 0.016 mmol] was obtained by performing the reaction in the same manner as in Example 1 using compound (2c) [50 mg, 0.069 mmol] and (R)-3-methylpiperidin-3-ol [30 mg, 0.198 mmol] as starting materials.

¹H-NMR (CD₃OD) δ: 6.26 (1H, d, J = 11.2 Hz), 5.90 (1H, d, J = 11.2 Hz), 5.05 (2H, d, J = 6.8 Hz), 4.43-4.35 (2H, m), 2.85 (1H, dd, J = 11.7, 3.4 Hz), 2.67 (1H, dd, J = 13.2, 4.4 Hz), 2.48 (1H, dd, J = 13.2, 3.9 Hz), 2.43-2.25 (6H, m), 2.10-1.90 (5H, m), 1.76-1.21 (15H, m), 1.18 (3H, s), 1.03 (3H, d, J = 6.3 Hz), 0.60 (3H, s).

Exact Mass = 443.34 (C₂₈H₄₅NO₃) Obs. mass = 444.35 (M+H)

### [Example 118]

### Synthesis of (1R,3R)-5-(2-((1R,3aS,7aR,E)-1-((S)-1-((S)-3-hydroxy-3-methylpiperidin-1-yl)propan-2-yl)-7a-methyloctahydro-4H-inden-4-ylidene)ethylidene)-2-methylenecyclohexane-1,3-diol (Compound C041)

Compound C041 [8.2 mg, 0.018 mmol] was obtained by performing the reaction in the same manner as in Example 1 using compound (2c) [50 mg, 0.069 mmol] and (S)-3-methylpiperidin-3-ol [30 mg, 0.198 mmol] as starting materials.

¹H-NMR (CD₃OD) δ: 6.26 (1H, d, J = 11.2 Hz), 5.91 (1H, d, J = 11.2 Hz), 5.05 (2H, d, J = 6.8 Hz), 4.42-4.36 (2H, m), 2.85 (1H, dd, J = 12.2, 3.4 Hz), 2.67 (1H, dd, J = 13.2, 4.4 Hz), 2.48 (2H, dd, J = 13.4, 3.7 Hz), 2.31-2.25 (4H, m), 2.15-1.88 (6H, m), 1.68-1.21 (14H, m), 1.18 (3H, s), 1.03 (3H, d, J = 6.3 Hz), 0.62 (3H, d, J = 14.6 Hz).

Exact Mass = 443.34 (C₂₈H₄₅NO₃) Obs. mass = 444.35 (M+H)

### [Example 119]

### Synthesis of (1R,3S,Z)-5-(2-((1R,3aS,7aR,E)-1-((2S)-1-(3-ethyl-3-hydroxypiperidin-1-yl)propan-2-yl)-7a-methyloctahydro-4H-inden-4-ylidene)ethylidene)-4-methylenecyclohexane-1,3-diol (Compound C042a, Compound C042b)

Compound C042a [18.0 mg, 0.041 mmol] and compound C042b [5.7 mg, 0.012 mmol] were obtained by performing the reaction in the same manner as in Example 108 using compound (2a) [100 mg, 0.137 mmol] and 3-ethylpiperidin-3-ol [40 mg, 0.310 mmol] as starting materials.

### Compound C042a

¹H-NMR (CD₃OD) δ: 6.32 (1H, d, J = 11.0 Hz), 6.08 (1H, d, J = 11.0 Hz), 5.28 (1H, s), 4.34 (1H, t, J = 5.9 Hz), 4.14-4.10 (1H, m), 2.86 (1H, dd, J = 12.6, 3.0 Hz), 2.51 (2H, dd, J = 13.0, 3.0 Hz), 2.25 (3H, dd, J = 13.3, 6.4 Hz), 2.10-1.85 (8H, m), 1.73-1.25 (15H, m), 1.03 (3H, d, J = 6.4 Hz), 0.89 (3H, t, J = 7.5 Hz), 0.59 (3H, s).

Exact Mass = 457.36 (C₂₉H₄₇NO₃) Obs. mass = 458.25 (M+H)

### Compound C042b

¹H-NMR (CD₃OD) δ: 6.32 (1H, d, J = 11.0 Hz), 6.08 (1H, d, J = 11.4 Hz), 5.28 (1H, s), 4.34 (1H, t, J = 5.9 Hz), 4.15-4.07 (1H, m), 2.86 (1H, dd, J = 12.3, 4.6 Hz), 2.51 (1H, dd, J = 13.3, 3.2 Hz), 2.42-2.20 (5H, m), 2.07-1.20 (22H, m), 1.03 (3H, d, J = 6.4 Hz), 0.90 (3H, t, J = 7.5 Hz), 0.59 (3H, s).

Exact Mass = 457.36 (C₂₉H₄₇NO₃) Obs. mass = 458.25 (M+H)

### [Example 120]

### Synthesis of (1R,3S,Z)-5-(2-((1R,3aS,7aR,E)-1-((2S)-1-(3-methoxy-3-methylpiperidin-1-yl)propan-2-yl)-7a-methyloctahydro-4H-inden-4-ylidene)ethylidene)-4-methylenecyclohexane-1,3-diol (Compound C043a, Compound C043b)

Compound C043a [5.2 mg, 0.011 mmol] and compound C043b [18.5 mg, 0.040 mmol] were obtained by performing the reaction in the same manner as in Example 108 using compound (2a) [100 mg, 0.310 mmol] and 3-methoxy-3-methylpiperidine [40 mg, 0.310 mmol] as starting materials.

### Compound C043a

¹H-NMR (CD₃OD) δ: 6.32 (1H, d, J = 11.4 Hz), 6.08 (1H, d, J = 11.0 Hz), 5.28 (1H, s), 4.34 (1H, t, J = 5.9 Hz), 4.15-4.10 (1H, m), 3.22 (3H, s), 2.86 (1H, dd, J = 11.9, 3.7 Hz), 2.51 (1H, dd, J = 13.5, 3.4 Hz), 2.42 (1H, br s), 2.28-2.20 (5H, m), 2.07-1.87 (6H, m), 1.68-1.21 (13H, m), 1.17 (3H, s), 1.02 (3H, d, J = 6.4 Hz), 0.59 (3H, s).

Exact Mass = 457.36 (C₂₉H₄₇NO₃) Obs. mass = 458.25 (M+H)

### Compound C043b

¹H-NMR (CD₃OD) δ: 6.32 (1H, d, J = 11.0 Hz), 6.08 (1H, d, J = 11.4 Hz), 5.28 (1H, d, J = 0.9 Hz), 4.34 (1H, t, J = 5.7 Hz), 4.13-4.08 (1H, m), 3.20 (3H, s), 2.86 (1H, dd, J = 12.1, 4.3 Hz), 2.58-2.48 (2H, m), 2.35-2.20 (4H, m), 2.07-1.21 (20H, m), 1.17 (3H, s), 1.03 (3H, d, J = 6.4 Hz), 0.59 (3H, s).

Exact Mass = 457.36 (C₂₉H₄₇NO₃) Obs. mass = 458.25 (M+H)

### [Example 121]

### Synthesis of (1R,3R)-5-(2-((1R,3aS,7aR,E)-1-((2S)-1-(3-ethyl-3-hydroxypiperidin-1-yl)propan-2-yl)-7a-methyloctahydro-4H-inden-4-ylidene)ethylidene)cyclohexane-1,3-diol compound (Compound C044a, Compound C044b)

Compound C044a [15.8 mg, 0.035 mmol] and compound C044b [7.5 mg, 0.017 mmol] were obtained by performing the reaction in the same manner as in Example 108 using compound (2b) [100 mg, 0.139 mmol] and 3-ethylpiperidin-3-ol [40 mg, 0.310 mmol] as starting materials.

### Compound C044a

¹H-NMR (CD₃OD) δ: 6.21 (1H, d, J = 11.0 Hz), 5.88 (1H, d, J = 11.0 Hz), 4.06-3.95 (2H, m), 2.84 (1H, dd, J = 12.3, 3.2 Hz), 2.59 (1H, dd, J = 13.3, 3.7 Hz), 2.40 (1H, dd, J = 13.5, 3.4 Hz), 2.29-1.20 (27H, m), 1.03 (3H, d, J = 6.4 Hz), 0.89 (3H, t, J = 7.3 Hz), 0.60 (3H, s).

Exact Mass = 445.36 (C₂₈H₄₇NO₃) Obs. mass = 446.30 (M+H)

### Compound C044b

¹H-NMR (CD₃OD) δ: 6.21 (1H, d, J = 11.4 Hz), 5.88 (1H, d, J = 11.0 Hz), 4.06-3.95 (2H, m), 2.83 (1H, dd, J = 12.0, 4.0 Hz), 2.59 (1H, dd, J = 14.0, 4.0 Hz), 2.42-1.21 (33H, m), 1.03 (3H, d, J = 6.4 Hz), 0.90 (3H, t, J = 7.3 Hz), 0.60 (3H, s).

Exact Mass = 445.36 (C₂₈H₄₇NO₃) Obs. mass = 446.30 (M+H)

### [Example 122]

### Synthesis of (1R,3R)-5-(2-((1R,3aS,7aR,E)-1-((2S)-1-(3-methoxy-3-methylpiperidine-1-yl)propan-2-yl)-7a-methyloctahydro-4H-inden-4-ylidene)ethylidene)cyclohexane-1,3-diol (Compound C045a, Compound C045b)

Compound C045a [6.5 mg, 0.015 mmol] and compound C045b [18.4 mg, 0.041 mmol] were obtained by performing the reaction in the same manner as in Example 108 using compound (2b) [100 mg, 0.139 mmol] and 3-methoxy-3-methylpiperidine [40 mg, 0.310 mmol] as starting materials.

### Compound C045a

¹H-NMR (CD₃OD) δ: 6.21 (1H, d, J = 11.4 Hz), 5.88 (1H, d, J = 11.0 Hz), 4.06-3.95 (2H, m), 3.22 (3H, s), 2.83 (1H, dd, J = 12.1, 3.9 Hz), 2.59 (1H, dd, J = 13.3, 3.7 Hz), 2.40 (2H, dd, J = 13.3, 3.7 Hz), 2.26-2.11 (6H, m), 2.07-1.51 (17H, m), 1.37-1.19 (4H, m), 1.17 (3H, s), 1.02 (3H, d, J = 6.4 Hz), 0.59 (3H, s).

Exact Mass = 445.36 (C₂₈H₄₇NO₃) Obs. mass = 446.30 (M+H)

### Compound C045b

¹H-NMR (CD₃OD) δ: 6.21 (1H, d, J = 11.0 Hz), 5.88 (1H, d, J = 11.0 Hz), 4.06-3.95 (2H, m), 3.21 (3H, s), 2.83 (1H, dd, J = 11.0, 4.6 Hz), 2.59 (2H, dd, J = 13.7, 3.7 Hz), 2.57-2.52 (2H, br m), 2.40 (1H, dd, J = 13.5, 3.4 Hz), 2.30-1.21 (26H, m), 1.17 (3H, s), 1.04 (3H, d, J = 6.4 Hz), 0.60 (3H, s).

Exact Mass = 445.36 (C₂₈H₄₇NO₃) Obs. mass = 446.30 (M+H)

### [Example 123]

### Synthesis of (1R,3R)-5-(2-((1R,3aS,7aR,E)-1-((2S)-1-(3-ethyl-3-hydroxypiperidin-1-yl)propan-2-yl)-7a-methyloctahydro-4H-inden-4-ylidene)ethylidene)-2-methylenecyclohexane-1,3-diol (Compound C046a, Compound C046b)

Compound C046a [16.8 mg, 0.037 mmol] and compound C046b [15.3 mg, 0.033 mmol] were obtained by performing the reaction in the same manner as in Example 108 using compound (2c) [100 mg, 0.137 mmol] and 3-ethylpiperidin-3-ol [60 mg, 0.464 mmol] as starting materials.

### Compound C046a

¹H-NMR (CD₃OD) δ: 6.26 (1H, d, J = 10.7 Hz), 5.91 (1H, d, J = 11.2 Hz), 5.05 (2H, d, J = 6.8 Hz), 4.43-4.33 (2H, m), 2.85 (1H, dd, J = 12.2, 3.4 Hz), 2.67 (1H, dd, J = 13.4, 4.1 Hz), 2.57-2.49 (1H, br m), 2.48 (1H, dd, J = 10.0, 5.0 Hz), 2.31-1.88 (11H, m), 1.68-1.25 (16H, m), 1.03 (3H, d, J = 6.3 Hz), 0.89 (3H, t, J = 7.6 Hz), 0.60 (3H, s).

Exact Mass = 457.36 (C₂₉H₄₇NO₃) Obs. mass = 458.35 (M+H)

### Compound C046b

¹H-NMR (CD₃OD) δ: 6.26 (1H, d, J = 11.2 Hz), 5.91 (1H, d, J = 11.2 Hz), 5.05 (2H, d, J = 6.8 Hz), 4.45-4.35 (2H, m), 2.85 (1H, dd, J = 11.0, 3.8 Hz), 2.67 (1H, dd, J = 13.2, 4.4 Hz), 2.48 (1H, dd, J = 13.2, 3.9 Hz), 2.42-2.25 (6H, m), 2.07-1.88 (6H, m), 1.76-1.21 (17H, m), 1.03 (3H, d, J = 6.3 Hz), 0.90 (3H, t, J = 7.6 Hz), 0.60 (3H, s).

Exact Mass = 457.36 (C₂₉H₄₇NO₃) Obs. mass = 458.35 (M+H)

### [Example 124]

### Synthesis of (1R,3R)-5-(2-((1R,3aS,7aR,E)-1-((2S)-1-(3-methoxy-3-methylpiperidine-1-yl)propan-2-yl)-7a-methyloctahydro-4H-inden-4-ylidene)ethylidene)-2-methylenecyclohexane-1,3-diol (Compound C047a, Compound C047b)

Compound C047a [18.1 mg, 0.040 mmol] and compound C047b [14.9 mg, 0.033 mmol] were obtained by performing the reaction in the same manner as in Example 108 using compound (2c) [100 mg, 0.137 mmol] and 3-methoxy-3-methylpiperidine [60 mg, 0.464 mmol] as starting materials.

### Compound C047a

¹H-NMR (CD₃OD) δ: 6.26 (1H, d, J = 11.2 Hz), 5.90 (1H, d, J = 11.2 Hz), 5.05 (2H, t, J = 3.9 Hz), 4.42-4.36 (2H, m), 3.22 (3H, s), 2.85 (1H, dd, J = 12.0, 3.7 Hz), 2.67 (1H, dd, J = 13.4, 4.1 Hz), 2.48 (1H, dd, J = 13.2, 3.9 Hz), 2.40-1.85 (12H, m), 1.70-1.20 (15H, m), 1.17 (3H, s), 1.03 (3H, d, J = 6.3 Hz), 0.60 (3H, s).

Exact Mass = 457.36 (C₂₉H₄₇NO₃) Obs. mass = 458.35 (M+H)

### Compound C047b

¹H-NMR (CD₃OD) δ: 6.26 (1H, d, J = 10.7 Hz), 5.91 (1H, d, J = 11.2 Hz), 5.05 (2H, d, J = 6.8 Hz), 4.42-4.36 (2H, m), 3.20 (3H, s), 2.85 (1H, dd, J = 12.2, 3.9 Hz), 2.67 (1H, dd, J = 13.2, 4.4 Hz), 2.56 (1H, s), 2.48 (1H, dd, J = 13.2, 3.9 Hz), 2.31-1.20 (28H, m), 1.17 (3H, s), 1.04 (3H, d, J = 6.3 Hz), 0.61 (3H, s).

Exact Mass = 457.36 (C₂₉H₄₇NO₃) Obs. mass = 458.35 (M+H)

### [Example 125]

### Synthesis of (1R,3S,Z)-5-(2-((1R,3aS,7aR,E)-1-((S)-1-((S)-3-(difluoromethyl)piperidin-1-yl)propan-2-yl)-7a-methyloctahydro-4H-inden-4-ylidene)ethylidene)-4-methylenecyclohexane-1,3-diol (Compound C048)

Compound C048 [6.0 mg, 0.013 mmol] was obtained by performing the reaction in the same manner as in Example 1 using compound (2a) [90 mg, 0.123 mmol] and (S)-3-(difluoromethyl)piperidine hydrochloride (CAS Registry No. 2227197-58-0) [90 mg, 0.524 mmol] as starting materials.

¹H-NMR (CD₃OD) δ: 6.32 (1H, d, J = 11.2 Hz), 6.08 (1H, d, J = 11.2 Hz), 5.77 (1H, td, J = 56.7, 4.7 Hz), 5.28 (1H, dd, J = 2.4, 1.5 Hz), 4.34 (1H, t, J = 5.9 Hz), 4.15-4.09 (1H, m), 3.05-2.70 (3H, m), 2.51 (1H, dd, J = 13.7, 3.4 Hz), 2.42 (1H, d, J = 9.8 Hz), 2.30-2.15 (3H, m), 2.10-1.96 (4H, m), 1.91-1.21 (18H, m), 1.04 (3H, d, J = 6.3 Hz), 0.61 (3H, s).

Exact Mass = 463.33 (C₂₈H₄₃F₂NO₂) Obs. mass = 464.50 (M+H)

### [Example 126]

### Synthesis of (1R,3R)-5-(2-((1R,3aS,7aR,E)-1-((S)-1-((S)-3-(difluoromethyl)piperidin-1-yl)propan-2-yl)-7a-methyloctahydro-4H-inden-4-ylidene)ethylidene)-2-methylenecyclohexane-1,3-diol (Compound C049)

Compound C049 [5.3 mg, 0.011 mmol] was obtained by performing the reaction in the same manner as in Example 1 using compound (2c) [50 mg, 0.069 mmol] and (S)-3-(difluoromethyl)piperidine hydrochloride [50 mg, 0.291 mmol] as starting materials.

¹H-NMR (CD₃OD) δ: 6.26 (1H, d, J = 11.2 Hz), 5.91 (2H, d, J = 11.2 Hz), 5.74 (2H, td, J = 56.5, 5.0 Hz), 5.05 (2H, d, J = 7.3 Hz), 4.45-4.35 (2H, m), 2.92-2.82 (2H, m), 2.69-2.61 (2H, m), 2.48 (1H, dd, J = 12.9, 4.1 Hz), 2.31-1.21 (28H, m), 1.02 (3H, d, J = 6.3 Hz), 0.61 (3H, s).

Exact Mass = 463.33 (C₂₈H₄₃F₂NO₂) Obs. mass = 464.50 (M+H)

### [Example 127]

### Synthesis of (1R,2S,3S,Z)-5-(2-((1R,3aS,7aR,E)-1-((S)-1-((S)-3-(difluoromethyl)piperidin -1-yl)propan-2-yl)-7a-methyloctahydro-4H-inden-4-ylidene)ethylidene)-2-methyl-4-methylenecyclohexane-1,3-diol (Compound C050)

Compound C050 [6.0 mg, 0.013 mmol] was obtained by performing the reaction in the same manner as in Example 1 using compound (2g) [50 mg, 0.067 mmol] and (S)-3-(difluoromethyl)piperidine hydrochloride [50 mg, 0.291 mmol] as starting materials.

¹H-NMR (CD₃OD) δ: 6.33 (1H, d, J = 11.2 Hz), 6.09 (1H, d, J = 11.2 Hz), 5.74 (1H, td, J = 56.8, 4.9 Hz), 5.22 (1H, d, J = 2.0 Hz), 4.22 (1H, d, J = 3.4 Hz), 3.72 (1H, td, J = 8.2, 4.2 Hz), 2.92-2.80 (2H, m), 2.64-2.57 (2H, m), 2.27 (1H, dd, J = 12.2, 2.9 Hz), 2.21-1.93 (7H, m), 1.81-1.20 (17H, m), 1.03 (3H, d, J = 7.1 Hz), 1.02 (3H, d, J = 6.5 Hz), 0.59 (3H, s).

Exact Mass = 477.34 (C₂₉H₄₅F₂NO₂) Obs. mass = 478.50 (M+H)

### [Example 128]

### Synthesis of (1R,3R)-5-(2-((1R,3aS,7aR,E)-1-((S)-1-((R)-3-(2,2-difluoroethyl)piperidin-1-yl)propan-2-yl)-7a-methyloctahydro-4H-inden-4-ylidene)ethylidene)cyclohexane-1,3-diol (Compound C051)

Compound C051 [12.2 mg, 0.026 mmol] was obtained by performing the reaction in the same manner as in Example 1 using compound (2b) [50 mg, 0.070 mmol] and (R)-3-(2,2-difluoroethyl)piperidine hydrochloride [35 mg, 0.189 mmol] as starting materials.

¹H-NMR (CD₃OD) δ: 6.21 (1H, d, J = 11.0 Hz), 6.10-5.79 (2H, m), 4.06-3.95 (2H, m), 2.90-2.81 (2H, m), 2.70-2.60 (1H,m), 2.59 (1H, dd, J = 13.7, 3.7 Hz), 2.41 (1H, dd, 13.3, 3.2 Hz), 2.27-2.13 (3H, m), 2.07-1.47 (20H, m), 1.37-1.20 (3H, m), 1.02 (3H, d, J = 6.4 Hz), 0.60 (3H, s).

Exact Mass = 465.34 (C₂₈H₄₅F₂NO₂) Obs. mass = 466.25 (M+H)

### [Example 129]

### Synthesis of (1R,3S,Z)-5-(2-((1R,3aS,7aR,E)-1-((S)-1-((S)-3-(1,1-difluoroethyl)piperidin-1-yl)propan-2-yl)-7a-methyloctahydro-4H-inden-4-ylidene)ethylidene)-4-methylenecyclohexane-1,3-diol (Compound C052)

Compound C052 [13.9 mg, 0.029 mmol] was obtained by performing the reaction in the same manner as in Example 1 using compound (2a) [90 mg, 0.123 mmol] and (S)-3-(1,1-difluoroethyl)piperidine hydrochloride [100 mg, 0.539 mmol] as starting materials.

¹H-NMR (CD₃OD) δ: 5.85 (1H, d, J = 10.7 Hz), 5.63 (1H, d, J = 11.2 Hz), 4.82 (1H, dd, J = 2.4, 1.5 Hz), 3.88 (1H, t, J= 6.1 Hz), 3.70-3.60 (1H, m), 2.78 (1H, d, J = 8.3 Hz), 2.57 (1H, d, J = 11.7 Hz), 2.41 (1H, dd, J = 12.0, 3.7 Hz), 2.15 (1H, dd, J = 12.7, 2.9 Hz), 2.05 (1H, dd, J = 13.2, 3.4 Hz), 1.94-1.53 (9H, m), 1.46-0.80 (22H, m), 0.60 (3H, d, J = 6.8 Hz), 0.16 (3H, s).

Exact Mass = 477.34 (C₂₉H₄₅F₂NO₂) Obs. mass = 478.50 (M+H)

### [Example 130]

### Synthesis of (1R,3R)-5-(2-((1R,3aS,7aR,E)-1-((S)-1-((S)-3-(1,1-difluoroethyl)piperidin-1-yl)propan-2-yl)-7a-methyloctahydro-4H-inden-4-ylidene)ethylidene)-2-methylenecyclohexane-1,3-diol (Compound C053)

Compound C053 [7.8 mg, 0.016 mmol] was obtained by performing the reaction in the same manner as in Example 1 using compound (2c) [50 mg, 0.069 mmol] and (S)-3-(1,1-difluoroethyl)piperidine hydrochloride [50 mg, 0.269 mmol] as starting materials. ¹H-NMR (CD₃OD) δ: 6.26 (1H, d, J = 11.2 Hz), 5.91 (1H, d, J = 11.2 Hz), 5.05 (2H, d, J = 6.5 Hz), 4.43-4.35 (2H, m), 3.20 (1H, d, J = 10.7 Hz), 2.99-2.93 (1H, m), 2.90-2.81 (1H, m), 2.69-1.94 (12H, m), 1.88-1.49 (13H, m), 1.40-1.20 (4H, m), 1.06 (3H, d, J = 6.8 Hz), 0.63 (3H, s).

Exact Mass = 477.34 (C₂₉H₄₅F₂NO₂) Obs. mass = 478.50 (M+H)

### [Example 131]

### Synthesis of (1R,2S,3S,Z)-5-(2-((1R,3aS,7aR,E)-1-((S)-1-((S)-3-(1,1-difluoroethyl)piperidin-1-yl)propan-2-yl)-7a-methyloctahydro-4H-inden-4-ylidene)ethylidene)-2-methyl-4-methylenecyclohexane-1,3-diol (Compound C054)

Compound C054 [8.0 mg, 0.016 mmol] was obtained by performing the reaction in the same manner as in Example 1 using compound (2g) [50 mg, 0.067 mmol] and (S)-3-(1,1-difluoroethyl)piperidine hydrochloride [50 mg, 0.269 mmol] as starting materials. ¹H-NMR (CD₃OD) δ: 6.33 (1H, d, J = 11.2 Hz), 6.09 (1H, d, J = 11.2 Hz), 5.22 (1H, d, J = 2.0 Hz), 4.22 (1H, d, J = 3.4 Hz), 3.72 (1H, td, J = 8.1, 4.2 Hz), 3.13 (1H, d, J = 9.3 Hz), 2.87 (2H, d, J = 12.2 Hz), 2.59 (1H, dd, J = 13.7, 3.9 Hz), 2.43 (1H, d, J = 12.2 Hz), 2.31-1.98 (9H, m), 1.89-1.17 (27H, m), 1.04 (3H, d, J = 6.3 Hz), 1.04 (3H, d, J = 6.8 Hz), 0.60 (3H, s).

Exact Mass = 491.36 (C₃₀H₄₇F₂NO₂) Obs. mass = 492.55 (M+H)

### [Example 132]

### Synthesis of (1R,3R)-5-(2-((1R,3aS,7aR,E)-1-((S)-1-(4-(1,1-difluoroethyl)piperidin-1-yl)propan-2-yl)-7a-methyloctahydro-4H-inden-4-ylidene)ethylidene)-2-methylenecyclohexane-1,3-diol (Compound C055)

Compound C055 [25.0 mg, 0.0523 mmol] was obtained by performing the reaction in the same manner as in Example 1 using compound (2c) [95.0 mg, 0.130 mmol] and 4-(1,1-difluoroethyl)piperidine hydrochloride [75.0 mg, 0.404 mmol] as starting materials.

1H-NMR (CD₃OD) δ: 6.26 (1H, d, J = 11.2 Hz), 5.91 (1H, d, J = 11.2 Hz), 5.05 (2H, d, J = 6.8 Hz), 4.43-4.35 (2H, m), 3.15 (1H, d, J = 11.7 Hz), 2.98-2.83 (2H, m), 2.67 (1H, dd, J = 13.4, 4.1 Hz), 2.48 (1H, dd, J = 13.2, 3.9 Hz), 2.39-1.92 (9H, m), 1.86-1.23 (19H, m), 1.04 (3H, d, J = 6.3 Hz), 0.61 (3H, s).

Exact Mass = 477.34 (C₂₉H₄₅F₂NO₂) Obs. mass =478.45 (M+H)

### [Example 133]

### Synthesis of (1R,3R)-5-(2-((1R,3aS,7aR,E)-1-((S)-1-(4-(2,2-difluoroethyl)piperidin-1-yl)propan-2-yl)-7a-methyloctahydro-4H-inden-4-ylidene)ethylidene)-2-methylenecyclohexane-1,3-diol (Compound C059)

Compound C059 [32.1 mg, 0.0672 mmol] was obtained by performing the reaction in the same manner as in Example 1 using compound (2c) [95.0 mg, 0.130 mmol] and 4-(2,2-difluoroethyl)piperidine hydrochloride [75.0 mg, 0.404 mmol] as starting materials.

¹H-NMR (CD₃OD) δ: 6.26 (1H, d, J = 11.2 Hz), 5.99 (2H, tt, J = 57.0, 5.0 Hz), 5.92 (2H, d, J = 11.0 Hz), 5.05 (2H, d, J = 8.0 Hz), 4.43-4.35 (2H, m), 3.43 (1H, d, J = 12.7 Hz), 2.87-2.57 (6H, m), 2.48 (1H, dd, J = 13.4, 4.1 Hz), 2.32-2.25 (2H, m), 2.09-1.96 (5H, m), 1.90-1.28 (16H, m), 1.10 (3H, d, J = 6.3 Hz), 0.64 (3H, s).

Exact Mass = 477.34 (C₂₉H₄₅F₂NO₂) Obs. mass =478.45 (M+H)

### [Example 134]

### Synthesis of (1R,3S,Z)-5-(2-((1R,3aS,7aR,E)-7a-methyl-1-((S)-1-morpholinopropan-2-yl)octahydro-4H-inden-4-ylidene)ethylidene)-4-methylenecyclohexane-1,3 -diol (Compound D001)

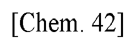

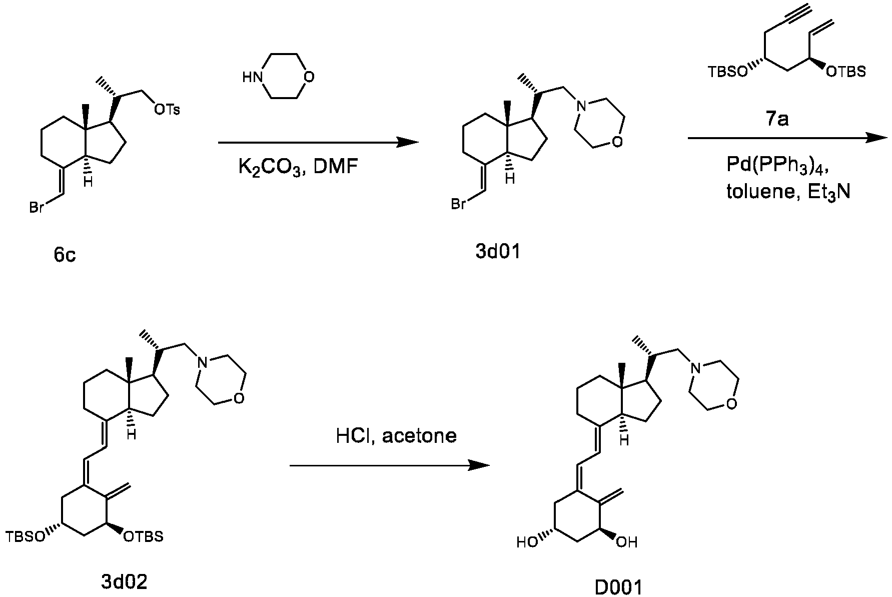

### Step 1

4-((2S)-2-((1R,3aS,7aR,E)-4-(Bromomethylene)-7a-methyloctahydro-1H-inden-1-yl)propyl)morpholine (Compound 3d01) [317.6 mg, 0.891 mmol] was obtained by performing the reaction in the same manner as in step 1 of Example 92 using compound 6c [437.6 mg, 0.991 mmol] and morpholine [0.26 mL, 3.0 mmol] as starting materials.

### Step 2

Compound D001 [7.7 mg, 0.019 mmol] was obtained by processing in the same manner as in step 2 and step 3 of Example 92 using the compound 3d01 [38 mg, 0.107 mmol] obtained in step 1, compound 7a [53.2 mg, 0.144 mmol], and tetrakistriphenylphosphine palladium(0) [15.0 mg, 0.013 mmol].

¹H-NMR (CD₃OD) δ: 6.31 (1H, d, J = 11.2 Hz), 6.08 (1H, d, J = 11.2 Hz), 5.28 (1H, dd, J = 2.4, 1.5 Hz), 4.89 (1H, s), 4.34 (1H, t, J = 5.9 Hz), 4.14-4.08 (1H, m), 3.76-3.66 (5H, m), 2.86 (1H, dd, J = 12.0, 3.7 Hz), 2.72-2.64 (3H, m), 2.53-2.40 (5H, m), 2.27-2.16 (3H, m), 2.11-1.98 (3H, m), 1.93-1.84 (4H, m), 1.79-1.25 (12H, m), 1.08-1.06 (1H, m), 1.05 (3H, d, J = 6.0 Hz), 0.60 (3H, s).

Exact Mass = 415.31 (C₂₆H₄₁NO₃) Obs. mass = 416.40 (M+H)

### [Example 135]

### Synthesis of (1R,2S,3S,Z)-2-methyl-5-(2-((1R,3aS,7aR,E)-7a-methyl-1-((S)-1-morpholinopropan-2-yl)octahydro-4H-inden-4-ylidene)ethylidene)-4-methylenecyclohexane-1,3-diol (Compound D002)

Compound D002 [2.6 mg, 0.006 mmol] was obtained by processing in the same manner as in step 2 and step 3 of Example 92 using compound 3d01 [38 mg, 0.107 mmol] obtained in step 1 of Example 134, compound 7b [55.7 mg, 0.146 mmol], and tetrakis(triphenylphosphine) palladium(0) [15.0 mg, 0.013 mmol].

¹H-NMR (CD₃OD) δ: 6.33 (1H, d, J = 11.2 Hz), 6.09 (1H, d, J = 11.2 Hz), 5.22 (1H, d, J = 2.4 Hz), 4.89 (1H, s), 4.22 (1H, d, J = 3.4 Hz), 3.75-3.61 (6H, m), 2.87 (1H, d, J = 12.7 Hz), 2.59 (1H, dd, J = 13.7, 4.4 Hz), 2.54-2.46 (2H, m), 2.25 (3H, dd, J = 12.2, 2.9 Hz), 2.17 (1H, dd, J = 13.4, 8.1 Hz), 2.06-1.21 (14H, m), 1.03 (3H, d, J = 6.6 Hz), 1.03 (3H, d, J = 7.0 Hz), 0.59 (3H, d, J = 7.3 Hz).

Exact Mass = 429.32 (C₂₇H₄₃NO₃) Obs. mass = 430.40 (M+H)

### [Example 136]

### Synthesis of (1R,3R)-5-(2-((1R,3aS,7aR,E)-7a-methyl-1-((S)-1-morpholinopropan-2-yl)-octahydro-4H-inden-4-ylidene)ethylidene)cyclohexane-1,3-diol (Compound D004)

Compound D004 [9.2 mg, 0.023 mmol] was obtained by performing the reaction in the same manner as in Example 1 using compound (2b) [50 mg, 0.07 mmol] and morpholine [0.05 mL] as starting materials.

¹H-NMR (CD₃OD) δ: 6.21 (1H, d, J = 11.2 Hz), 5.88 (1H, d, J = 11.2 Hz), 4.12-3.95 (2H, m), 3.72-3.61 (4H, m), 2.83 (1H, dd, J = 13.4, 3.7 Hz), 2.59 (1H, dd, J = 13.4, 3.7 Hz), 2.53-2.49 (2H, m), 2.40 (1H, dd, J = 13.7, 3.4 Hz), 2.28-2.13 (5H, m), 2.06-1.21 (17H, m), 1.03 (3H, d, J = 6.3 Hz), 0.59 (3H, s).

Exact Mass = 403.31 (C₂₅H₄₁NO₃) Obs. mass = 404.30 (M+H)

### [Example 137]

### Synthesis of (1R,3S,Z)-5-(2-((1R,3aS,7aR,E)-1-((S)-1-((S)-2-(difluoromethyl)morpholino)propan-2-yl)-7a-methyloctahydro-4H-inden-4-ylidene)ethylidene)-4-methylenecyclohexane-1,3-diol (Compound D005)

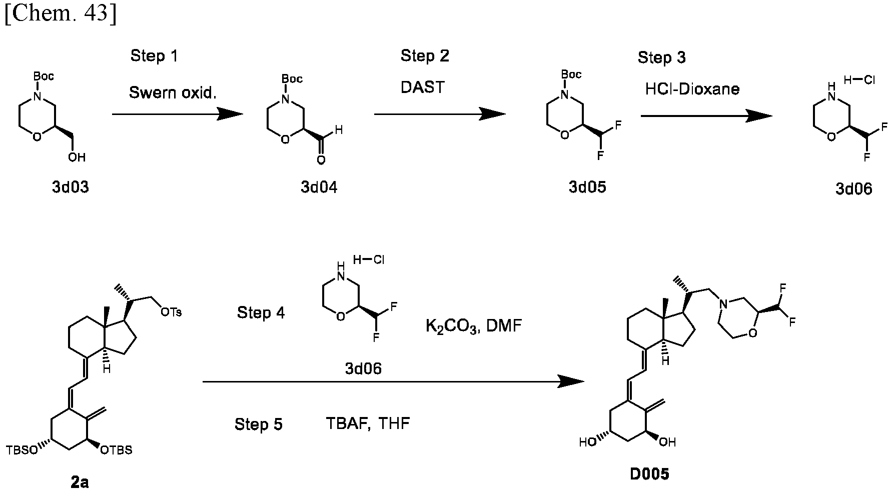

### Step 1

Under cooling to -78°C, a solution of dimethyl sulfoxide [1 mL, 14 mmol] in dichloromethane [4 mL] was added to a solution of oxalyl chloride [0.6 mL, 7 mmol] in dichloromethane [9 mL] and the mixture was stirred at the same temperature for 10 minutes. To the mixture, a solution of t-butyl-(S)-2-(hydroxymethyl)morpholine-4-carboxylate (Compound 3d03) [1.0 g, 4.6 mmol] in dichloromethane [10 mL] was added. The mixture was stirred at -78°C for 45 minutes. Triethylamine [3.3 mL, 23 mmol] was added to the mixture and the mixture was further stirred at the same temperature for 30 minutes. The reaction mixture was warmed to 0°C and stirred at the same temperature for 30 minutes. The reaction mixture was quenched with water and extracted with ethyl acetate. The organic layer was dried over anhydrous magnesium sulfate and concentrated under reduced pressure. The residue was purified by silica gel column chromatography to obtain t-butyl-(S)-2-formylmorpholine-4-carboxylate (Compound 3d04) [0.72 g, 3.3 mmol] (Yield = 73%).

### Step 2

A solution of t-butyl-(S)-2-formylmorpholine-4-carboxylate (Compound 3d04) [0.72 g, 3.3 mmol] in dichloromethane [20 mL] was cooled to 0°C and diethylaminosulfur trifluoride (DAST) [1 mL, 7.57 mmol] was added to the solution and the mixture was stirred overnight. The reaction mixture was cooled to 0°C, quenched with water and extracted with dichloromethane. The organic layer was dried over anhydrous magnesium sulfate and concentrated under reduced pressure. The residue was purified by silica gel column chromatography to obtain t-butyl-(S)-2-(difluoromethyl)morpholine-4-carboxylate (Compound 3d05) [0.58 g, 2.4 mmol] (Yield = 53%).

### Step 3

A 4 M hydrogen chloride dioxane solution [10 mL, 40 mmol] was added to t-butyl-(S)-2-(difluoromethyl)morpholine-4-carboxylate (Compound 3d05) [0.58 g, 2.4 mmol] and the mixture was stirred overnight. The reaction mixture was concentrated under reduced pressure and dried to obtain (S)-2-(difluoromethyl)morpholine hydrochloride (Compound 3d06) [382.5 mg, 2.20 mmol] (Yield = 90%).

¹H-NMR (DMSO-D₆) δ: 9.79 (2H, s), 6.13 (1H, td, J = 54.0, 3.3 Hz), 4.18-4.09 (1H, m), 4.03 (1H, dd, J = 12.7, 3.9 Hz), 3.85 (1H, td, J = 12.4, 2.4 Hz), 3.27 (1H, d, J = 12.2 Hz), 3.20 (1H, d, J = 13.2 Hz), 2.99 (1H, td, J = 12.4, 4.0 Hz), 2.92 (1H, t, J = 12.0 Hz).

### Step 4 and step 5

Compound D005 [6.0 mg, 0.013 mmol] was obtained by performing the reaction in the same manner as in Example 1 using compound (2a) [90 mg, 0.123 mmol] and compound (3d06) [65 mg, 0.374 mmol] as starting materials.

¹H-NMR (CD₃OD) δ: 6.32 (1H, d, J = 11.2 Hz), 6.08 (1H, d, J = 11.2 Hz), 5.77 (1H, td, J = 55.4, 4.2 Hz), 5.28 (1H, dd, J = 2.2, 1.2 Hz), 4.89 (1H, d, J = 1.5 Hz), 4.34 (1H, t, J = 5.9 Hz), 4.14-4.09 (1H, m), 3.86 (1H, d, J = 10.7 Hz), 3.70-3.61 (2H, m), 2.89-2.80 (2H, m), 2.57-2.49 (2H, m), 2.31-2.23 (3H, m), 2.07-1.21 (18H, m), 1.04 (3H, d, J = 6.3 Hz), 0.60 (3H, s).

Exact Mass = 465.31 (C₂₇H₄₁F₂NO₃) Obs. mass = 466.40 (M+H)

### [Example 138]

### Synthesis of (1R,3S,Z)-5-(2-((1R,3aS,7aR,E)-1-((S)-1-((R)-2-(difluoromethyl)morpholino)propan-2-yl)-7a-methyloctahydro-4H-inden-4-ylidene)ethylidene)-4-methylenecyclohexane-1,3-diol (Compound D006)

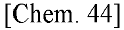

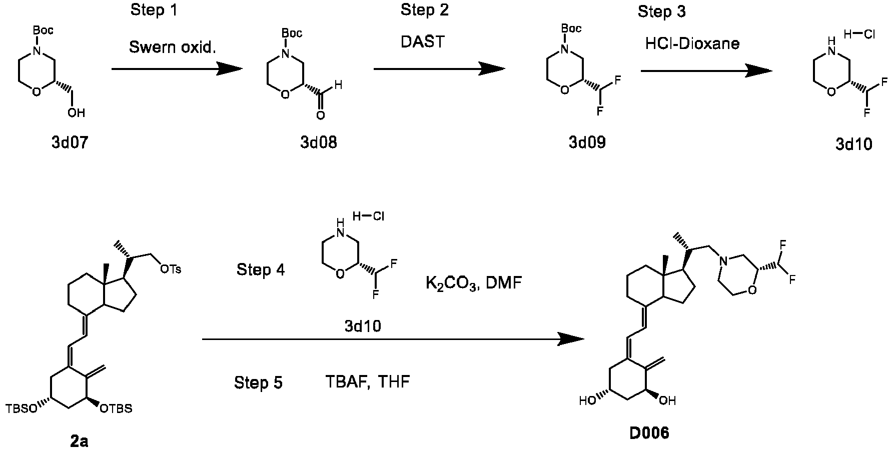

### Step 1

t-Butyl-(R)-2-formylmorpholine-4-carboxylate (Compound 3d08) [0.70 g, 3.3 mmol] was obtained by processing in the same manner as in step 1 of Example 137 using t-butyl-(R)-2-(hydroxymethyl)morpholine-4-carboxylate (Compound 3d07) [1.00 g, 3.3 mmol] as a starting material. (Yield = 71%)

### Step 2

t-Butyl-(R)-2-(difluoromethyl)morpholine-4-carboxylate (Compound 3d09) [0.55 g, 2.3 mmol] was obtained by processing in the same manner as in step 2 of Example 137 using t-butyl-(R)-2-formylmorpholine-4-carboxylate (Compound 3d08) [0.70 g, 3.3 mmol] as a starting material. (Yield = 50%)

### Step 3

(R)-2-(difluoromethyl)morpholine hydrochloride (Compound 3d10) [384.8 mg, 2.22 mmol] was obtained by processing in the same manner as in step 3 of Example 137 using t-butyl-(R)-2-(difluoromethyl)morpholine-4-carboxylate (Compound 3d09) [0.55 g, 2.3 mmol] as a starting material. (Yield = 96%)

¹H-NMR (DMSO-D₆) δ: 9.62 (2H, s), 6.13 (1H, td, J = 54.0, 3.3 Hz), 4.11 (1H, tdd, J = 14.8, 7.6, 3.8 Hz), 4.03 (1H, dd, J = 12.7, 3.9 Hz), 3.83 (1H, td, J = 12.4, 2.4 Hz), 3.28 (1H, d, J = 12.2 Hz), 3.20 (1H, dd, J = 12.0, 2.4 Hz), 3.00 (1H, td, J = 12.0, 3.9 Hz), 2.93 (1H, t, J = 12.0 Hz).

### Step 4 and step 5

Compound D006 [7.6 mg, 0.016 mmol] was obtained by performing the reaction in the same manner as in Example 1 using compound (2a) [90 mg, 0.123 mmol] and compound (3d10) [65 mg, 0.374 mmol] as starting materials.

¹H-NMR (CD₃OD) δ: 6.32 (1H, d, J = 10.7 Hz), 6.08 (1H, d, J = 11.2 Hz), 5.76 (1H, td, J = 55.4, 4.2 Hz), 5.28 (1H, t, J = 1.2 Hz), 4.34 (1H, t, J = 6.1 Hz), 4.15-4.09 (1H, m), 3.88 (1H, dt, J = 11.4, 2.6 Hz), 3.78-3.69 (1H, m), 3.61 (1H, td, J = 11.2, 2.4 Hz), 2.87 (1H, dd, J = 12.2, 3.4 Hz), 2.71 (2H, dd, J = 28.8, 11.2 Hz), 2.51 (1H, dd, J = 13.7, 3.4 Hz), 2.31 (1H, dd, J = 12.2, 2.9 Hz), 2.25 (1H, dd, J = 13.7, 6.8 Hz), 2.16 (1H, t, J = 10.7 Hz), 2.07-1.99 (4H, m), 1.96-1.26 (14H, m), 1.04 (3H, d, J = 6.3 Hz), 0.59 (3H, s).

Exact Mass = 465.31 (C₂₇H₄₁F₂NO₃) Obs. mass = 466.40 (M+H)

### [Example 139]

### Synthesis of (1R,3R)-5-(2-((1R,3aS,7aR,E)-1-((S)-1-((S)-2-(difluoromethyl)morpholino)propan-2-yl)-7a-methyloctahydro-4H-inden-4-ylidene)ethylidene)-2-methylenecyclohexane-1,3-diol (Compound D007)

Compound D007 [17.0 mg, 0.037 mmol] was obtained by performing the reaction in the same manner as in Example 1 using compound (2c) [90 mg, 0.123 mmol] and compound (3d06) [65 mg, 0.374 mmol] described in Example 137 as starting materials.

¹H-NMR (DMSO-D₆) δ: 6.14 (2H, d, J = 11.2 Hz), 6.02 (2H, td, J = 55.0, 4.0 Hz), 5.82 (1H, d, J = 11.2 Hz), 4.93-4.75 (2H, m), 4.91 (2H, d, J = 6.0 Hz), 4.24 (2H, td, J = 4.0, 16.0 Hz), 3.83 (1H, d, J = 10.7 Hz), 3.67-3.47 (2H, m), 2.78 (2H, d, J = 10.7 Hz), 2.55-2.50 (2H, m), 2.34 (1H, dd, J = 12.9, 3.7 Hz), 2.22-1.18 (21H, m), 0.98 (3H, d, J = 6.3 Hz), 0.54 (3H, s).

Exact Mass = 465.31 (C₂₇H₄₁F₂NO₃) Obs. mass = 466.35 (M+H)

### [Example 140]

### Synthesis of (1R,3R)-5-(2-((1R,3aS,7aR,E)-1-((S)-1-((R)-2-(difluoromethyl)morpholino)propan-2-yl)-7a-methyloctahydro-4H-inden-4-ylidene)ethylidene)-2-methylenecyclohexane-1,3-diol (Compound D008)

Compound D008 [17.3 mg, 0.037 mmol] was obtained by performing the reaction in the same manner as in Example 1 using compound (2c) [90 mg, 0.123 mmol] and compound (3d10) [65 mg, 0.374 mmol] described in Example 138 as starting materials.

¹H-NMR (DMSO-D₆) δ: 6.14 (2H, d, J = 11.2 Hz), 6.01 (2H, td, J = 55.0, 4.0 Hz), 5.82 (1H, d, J = 11.2 Hz), 4.91 (2H, d, J = 10.0 Hz), 4.89-4.80 (1H, m), 4.28-4.21 (2H, m), 3.84 (1H, d, J = 11.2 Hz), 3.74-3.65 (1H, m), 3.58-3.45 (2H, m), 2.79-2.74 (1H, m), 2.69 (1H, d, J = 11.7 Hz), 2.62 (1H, d, J = 11.2 Hz), 2.53 (1H, d, J = 6.0 Hz), 2.34 (1H, dd, J = 13.2, 3.4 Hz), 2.23-1.77 (9H, m), 1.65-1.18 (10H, m), 0.98 (3H, d, J = 6.3 Hz), 0.53 (3H, s).

Exact Mass = 465.31 (C₂₇H₄₁F₂NO₃) Obs. mass = 466.40 (M+H)

### [Example 141]

### Synthesis of (1R,2S,3S,Z)-2-methyl-5-(2-((1R,3aS,7aR,E)-7a-methyl-1-((2S)-1-(2-(trifluoromethyl)morpholino)propan-2-yl)octahydro-4H-inden-4-ylidene)ethylidene)-4-methylenecyclohexane-1,3-diol (Compound D009a, Compound D009b)

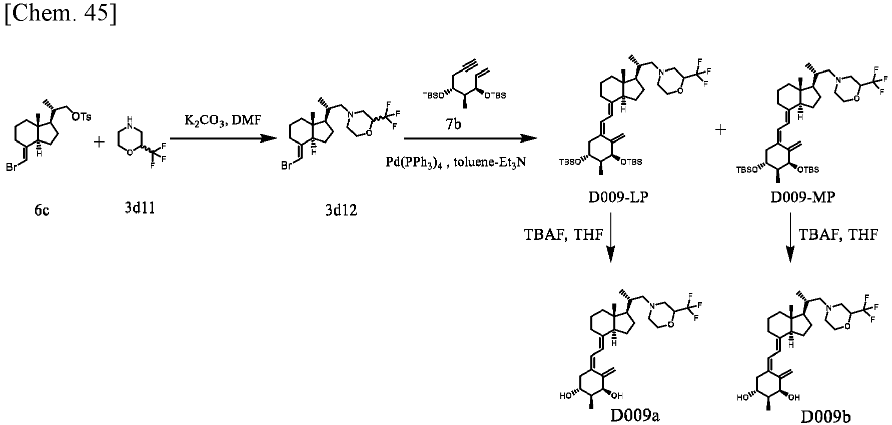

### Step 1

A suspension of compound 6c [203 mg, 0.46 mmol], 2-(trifluoromethyl)morpholine (compound 3d11) [153 mg, 0.986 mmol] and potassium carbonate [227 mg, 1.64 mmol] in DMF [5 mL] was stirred at 60°C overnight. The reaction mixture was quenched with saturated brine and extracted with ethyl acetate. The organic layer was dried over anhydrous magnesium sulfate and concentrated under reduced pressure. The residue was partially purified by silica gel column chromatography to obtain compound 3d12 [92.3 mg, 0.218 mmol] of a diastereomer mixture.

### Step 2

Tetrakis(triphenylphosphine) palladium(0) [25 mg, 0.021 mmol] was added to a mixed solution of compound 3d12 [92.3 mg, 0.218 mmol] obtained in step 1 and compound 7b [90 mg, 0.235 mmol] in toluene [1 mL]/triethylamine [1 mL] and the mixture was heated and stirred at 100°C for 2 hours under a nitrogen atmosphere. After cooling to room temperature, the reaction mixture was concentrated under reduced pressure. The residue was roughly purified by preparative thin-layer chromatography to afford the low-polarity compound D009-LP [56.3 mg] and the high-polarity compound D009-MP [42.0 mg].
D009-LP: Exact Mass = 725.48 (C₄₀H₇₀F₃NO₃Si₂) Obs. mass = 726.60 (M+H)
D009-MP: Exact Mass = 725.48 (C₄₀H₇₀F₃NO₃Si₂) Obs. mass = 726.60 (M+H)

### Step 3a

TBAF [1 M in THF, 1 mL] was added to a solution of compound D009-LP [56.3 mg] obtained in step 2 in THF [1 mL], and the mixture was stirred at 50°C overnight. The reaction mixture was quenched with a saturated aqueous sodium hydrogen carbonate solution and extracted with ethyl acetate. The organic layer was dried over anhydrous magnesium sulfate and concentrated under reduced pressure. The residue was purified by reverse phase HPLC to obtain compound D009a [16.7 mg, 0.0336 mmol].

¹H-NMR (CD₃OD) δ: 6.33 (1H, d, J = 11.2 Hz), 6.09 (1H, d, J = 11.2 Hz), 5.22 (1H, d, J = 2.0 Hz), 4.89 (1H, d, J = 2.0 Hz), 4.22 (1H, d, J = 3.4 Hz), 4.05-3.96 (1H, m), 3.92 (1H, dd, J = 11.0, 3.0 Hz), 3.72 (1H, td, J = 8.1, 4.4 Hz), 3.63 (1H, td, J = 11.3, 2.3 Hz), 2.87 (1H, dd, J= 11.0, 4.4 Hz), 2.77 (2H, d, J = 11.7 Hz), 2.59 (1H, dd, J= 13.4, 4.1 Hz), 2.32 (1H, dd, J = 12.2, 2.9 Hz), 2.23-2.14 (2H, m), 2.06-1.18 (18H, m), 1.03 (6H, d, J = 7.3 Hz), 0.58 (3H, s).
Exact Mass = 497.31 (C₂₈H₄₂F₃NO₃) Obs. mass = 498.40 (M+H)

### Step 3b

Compound D009b [9.1 mg, 0.018 mmol] was obtained by processing in the same manner as in step 3a using D009-MP [42.0 mg] as a starting material.

¹H-NMR (DMSO-D₆) δ: 6.19 (1H, d, J = 10.7 Hz), 5.99 (1H, d, J = 11.2 Hz), 5.17 (1H, d, J = 2.4 Hz), 4.77 (1H, d, J = 2.4 Hz), 4.75 (1H, d, J = 4.4 Hz), 4.57 (1H, d, J = 4.4 Hz), 4.15 (1H, t, J = 3.7 Hz), 4.10-4.00 (1H, m), 3.89 (1H, d, J = 10.2 Hz), 3.66-3.55 (2H, m), 2.88 (1H, d, J = 10.2 Hz), 2.79 (1H, d, J = 9.3 Hz), 2.56 (2H, d, J = 11.7 Hz), 2.25-1.16 (22H, m), 0.97 (3H, d, J = 5.9 Hz), 0.85 (3H, d, J = 6.8 Hz), 0.53 (3H, s).

Exact Mass = 497.31 (C₂₈H₄₂F₃NO₃) Obs. mass = 498.40 (M+H)

### [Example 142]

### Synthesis of (1R,3R)-5-(2-((1R,3aS,7aR,E)-7a-methyl-1-((S)-1-((S)-2-methylmorpholino)propan-2-yl)-octahydro-4H-inden-4-ylidene)ethylidene)-2-methylene-cyclohexane-1,3-diol (Compound D010)

Compound (D010) [11.6 mg, 0.027 mmol] was obtained by performing the reaction in the same manner as in Example 1 using compound (2c) [50 mg, 0.069 mmol] and (S)-2-methylmorpholine hydrochloride [30 mg, 0.297 mmol] as starting materials.

¹H-NMR (CD₃OD) δ: 6.26 (1H, d, J = 11.2 Hz), 5.91 (1H, d, J = 11.2 Hz), 5.05 (2H, d, J = 6.8 Hz), 4.43-4.35 (2H, m), 3.79 (1H, dd, J = 11.5, 1.7 Hz), 3.68-3.57 (2H, m), 2.85 (1H, dd, J = 12.0, 3.7 Hz), 2.75 (1H, d, J = 11.7 Hz), 2.69-2.61 (2H, m), 2.48 (1H, dd, J = 13.4, 4.1 Hz), 2.31-2.22 (3H, m), 2.07-1.82 (6H, m), 1.70-1.49 (6H, m), 1.38-1.25 (3H, m), 1.10 (3H, d, J = 6.3 Hz), 1.03 (3H, d, J = 6.3 Hz), 0.60 (3H, s).

Exact Mass = 429.32 (C₂₇H₄₃NO₃) Obs. mass = 430.35 (M+H)

### [Example 143]

### Synthesis of (1R,3R)-5-(2-((1R,3aS,7aR,E)-1-((S)-1-(4-oxa-7-azaspiro[2.5]octan-7-yl)propan-2-yl)-7a-methyloctahydro-4H-inden-4-ylidene)ethylidene)-2-methylenecyclohexane-1,3-diol (Compound D011)

Compound (D011) [11.3 mg, 0.0256 mmol] was obtained by performing the reaction in the same manner as in Example 1 using compound (2c) [50 mg, 0.069 mmol] and 4-oxa-7-azaspiro[2,5]octane hydrochloride [35 mg, 0.234 mmol] as starting materials. ¹H-NMR (CD₃OD) δ: 6.27 (1H, d, J = 10.7 Hz), 5.92 (1H, d, J = 10.7 Hz), 5.06 (2H, d, J = 7.3 Hz), 4.43-4.37 (2H, m), 3.80-3.68 (2H, m), 2.86 (1H, dd, J = 12.2, 3.9 Hz), 2.68 (1H, dd, J= 13.7, 4.4 Hz), 2.57-2.25 (8H, m), 2.08-1.90 (4H, m), 1.71-1.50 (6H, m), 1.40-1.26 (3H, m), 1.04 (3H, d, J = 6.3 Hz), 0.71 (2H, s), 0.61 (3H, s), 0.59-0.48 (2H, m).

Exact Mass = 441.32 (C₂₈H₄₃NO₃) Obs. mass = 442.35 (M+H)

### [Example 144]

### Synthesis of (1R,3R)-5-(2-((1R,3aS,7aR,E)-1-((S)-1-(6-oxa-9-azaspiro[4.5]decan-9-yl)propan-2-yl)-7a-methyloctahydro-4H-inden-4-ylidene)ethylidene)-2-methylenecyclohexane-1,3-diol (Compound D012)

Compound (D012) [9.9 mg, 21 mmol] was obtained by performing the reaction in the same manner as in Example 1 using compound (2c) [50 mg, 0.069 mmol] and 6-oxa-9-azaspiro[4,5]decane hydrochloride [35 mg, 0.248 mmol] as starting materials.

¹H-NMR (CD₃OD) δ: 6.26 (1H, d, J = 10.7 Hz), 5.91 (1H, d, J = 11.2 Hz), 5.05 (2H, d, J = 7.3 Hz), 4.45-4.33 (2H, m), 3.72-3.60 (2H, m), 2.85 (1H, dd, J = 12.7, 3.4 Hz), 2.67 (1H, dd, J = 13.2, 4.4 Hz), 2.48 (1H, dd, J = 13.2, 3.9 Hz), 2.39-2.19 (6H, m), 2.16-1.88 (5H, m), 1.84-1.25 (18H, m), 1.04 (3H, d, J = 6.8 Hz), 0.60 (3H, s).

Exact Mass = 469.36 (C₃₀H₄₇NO₃) Obs. mass = 470.35 (M+H)

### [Example 145]

### Synthesis of (1R,3R)-5-(2-((1R,3aS,7aR,E)-1-((S)-1-((2S,6R)-2,6-dimethylmorpholino)propan-2-yl)-7a-methyloctahydro-4H-inden-4-ylidene)ethylidene)-2-methylenecyclohexane-1,3-diol (Compound D013)

Compound (D013) [17.6 mg, 0.040 mmol] was obtained by performing the reaction in the same manner as in Example 1 using compound (2c) [50 mg, 0.069 mmol] and (2S,6R)-2,6-dimethylmorpholine [35 mg, 0.304 mmol] as starting materials.

¹H-NMR (CD₃OD) δ: 6.26 (1H, d, J = 11.2 Hz), 5.91 (1H, d, J = 11.2 Hz), 5.05 (2H, t, J = 3.9 Hz), 4.42-4.36 (2H, m), 3.73-3.59 (2H, m), 2.85 (1H, dd, J = 12.0, 3.7 Hz), 2.79 (1H, d, J= 11.2 Hz), 2.67 (1H, dd, J= 13.2, 4.4 Hz), 2.62 (1H, d, J= 11.2 Hz), 2.48 (1H, dd, J = 13.7, 3.9 Hz), 2.27 (2H, td, J = 12.7, 6.0 Hz), 2.22 (1H, dd, J = 12.0, 3.2 Hz), 2.16-1.89 (4H, m), 1.79 (1H, dd, J = 25.9, 15.6 Hz), 1.70-1.21 (12H, m), 1.12 (3H, d, J = 5.0 Hz), 1.10 (3H, d, J = 5.0 Hz), 1.03 (3H, d, J = 6.3 Hz), 0.60 (3H, s).

Exact Mass = 443.34 (C₂₈H₄₅NO₃) Obs. mass = 444.30 (M+H)

### [Example 146]

### Synthesis of (1R,3R)-5-(2-((1R,3aS,7aR,E)-1-((S)-1-(2,2-dimethylmorpholino)propan-2-yl)-7a-methyloctahydro-4H-inden-4-ylidene)ethylidene)-2-methylenecyclohexane-1,3-diol (Compound D014)

Compound (D014) [10.8 mg, 0.024 mmol] was obtained by performing the reaction in the same manner as in Example 1 using compound (2c) [50 mg, 0.069 mmol] and 2,2-dimethylmorpholine [35 mg, 0.304 mmol] as starting materials.

¹H-NMR (CD₃OD) δ: 6.26 (1H, d, J = 10.7 Hz), 5.91 (1H, d, J = 11.2 Hz), 5.05 (2H, d, J = 7.3 Hz), 4.43-4.35 (2H, m), 3.75-3.64 (2H, m), 2.85 (1H, dd, J = 11.0, 3.9 Hz), 2.67 (1H, dd, J = 13.4, 4.1 Hz), 2.48 (1H, dd, J = 13.2, 3.9 Hz), 2.40-1.88 (13H, m), 1.68-1.21 (18H, m), 1.04 (3H, d, J = 6.3 Hz), 0.60 (3H, s).

Exact Mass = 443.34 (C₂₈H₄₅NO₃) Obs. mass = 444.30 (M+H)

### [Example 147]

### Synthesis of (1R,3R)-5-(2-((1R,3aS,7aR,E)-7a-methyl-1-((S)-1-((S)-3-methylmorpholino)propan-2-yl)octahydro-4H-inden-4-ylidene)ethylidene)-2-methylenecyclohexane-1,3-diol (Compound D015)

Compound (D015) [2.0 mg, 0.0047 mmol] was obtained by performing the reaction in the same manner as in Example 1 using compound (2c) [50 mg, 0.069 mmol] and (S)-3-methylmorpholine hydrochloride [30 mg, 0.218 mmol] as starting materials.

¹H-NMR (CD₃OD) δ: 6.26 (1H, d, J = 11.2 Hz), 5.91 (1H, d, J = 10.7 Hz), 5.05 (2H, d, J = 6.8 Hz), 4.45-4.35 (2H, m), 3.75 (1H, dt, J = 11.4, 3.3 Hz), 3.60 (2H, ddd, J = 24.4, 11.2, 2.4 Hz), 3.21 (1H, dd, J = 11.0, 9.5 Hz), 2.84 (2H, td, J = 11.3, 3.3 Hz), 2.67 (1H, dd, J = 13.2, 4.4 Hz), 2.48 (1H, dd, J = 13.2, 3.9 Hz), 2.37 (1H, t, J = 11.5 Hz), 2.31-2.25 (3H, m), 2.08-2.02 (5H, m), 1.96 (1H, dd, J= 11.5, 8.1 Hz), 1.68-1.52 (6H, m), 1.32 (4H, dt, J = 32.9, 10.5 Hz), 1.02 (3H, d, J = 6.3 Hz), 0.93 (3H, d, J = 6.3 Hz), 0.61 (3H, s).

Exact Mass = 429.32 (C₂₇H₄₃NO₃) Obs. mass = 430.25 (M+H)

### [Example 148]

### Synthesis of (1R,3R)-5-(2-((1R,3aS,7aR,E)-1-((S)-1-((2R,5S)-2,5-dimethylmorpholino)propan-2-yl)-7a-methyloctahydro-4H-inden-4-ylidene)ethylidene)-2-methylenecyclohexane-1,3-diol (Compound D016)

Compound (D016) [5.0 mg, 0.011 mmol] was obtained by performing the reaction in the same manner as in Example 1 using compound (2c) [50 mg, 0.069 mmol] and (2R,5S)-2,5-dimethylmorpholine [30 mg, 0.260 mmol] as starting materials.

Exact Mass = 443.34 (C₂₈H₄₅NO₃) Obs. mass = 444.30 (M+H)

### [Example 149]

### Synthesis of (1R,3R)-5-(2-((1R,3aS,7aR,E)-1-((S)-1-((2R,5R)-2,5-dimethylmorpholino)propan-2-yl)-7a-methyloctahydro-4H-inden-4-ylidene)ethylidene)-2-methylenecyclohexane-1,3-diol (Compound D017)

Compound (D017) [8.2 mg, 0.018 mmol] was obtained by performing the reaction in the same manner as in Example 1 using compound (2c) [50 mg, 0.069 mmol] and (2R,5R)-2,5-dimethylmorpholine [30 mg, 0.260 mmol] as starting materials Exact Mass = 443.34 (C₂₈H₄₅NO₃) Obs. mass = 444.30 (M+H)

### [Example 150]

### Synthesis of (1R,3R)-5-(2-((1R,3aS,7aR,E)-7a-methyl-1-((S)-1-((R)-2-methylmorpholino)propan-2-yl)octahydro-4H-inden-4-ylidene)ethylidene)-2-methylenecyclohexane-1,3-diol (Compound D018)

Compound (D018) [5.3 mg, 0.012 mmol] was obtained by performing the reaction in the same manner as in Example 1 using compound (2c) [50 mg, 0.069 mmol] and (R)-2-methylmorpholine hydrochloride [40 mg, 0.291 mmol] as starting materials.

¹H-NMR (CD₃OD) δ: 6.26 (1H, d, J = 11.4 Hz), 5.91 (1H, d, J = 11.0 Hz), 5.05 (2H, d, J = 7.3 Hz), 4.42-4.36 (2H, m), 3.80-3.73 (1H, m), 3.67 (1H, td, J = 11.3, 2.4 Hz), 3.62-3.53 (1H, m), 2.85 (1H, dd, J = 12.1, 3.9 Hz), 2.80 (1H, d, J = 11.0 Hz), 2.67 (1H, dd, J = 13.3, 4.1 Hz), 2.56 (1H, dd, J = 11.9, 1.8 Hz), 2.48 (1H, dd, J = 13.3, 4.1 Hz), 2.31-2.22 (3H, m), 2.17 (1H, td, J = 11.5, 3.5 Hz), 2.07-1.89 (4H, m), 1.70-1.50 (7H, m), 1.38-1.21 (3H, m), 1.11 (3H, d, J = 5.9 Hz), 1.03 (3H, d, J = 6.4 Hz), 0.61 (3H, s).

Exact Mass = 429.32 (C₂₇H₄₃NO₃) Obs. mass = 430.40 (M+H)

### [Example 151]

### Synthesis of (1R,3R)-5-(2-((1R,3aS,7aR,E)-1-((S)-1-(4-oxa-7-azaspiro[2.5]octan-7-yl)propan-2-yl)-7a-methyloctahydro-4H-inden-4-ylidene)ethylidene)cyclohexane-1,3-diol (Compound D019)

Compound (D019) [11.2 mg, 0.0261 mmol] was obtained by performing the reaction in the same manner as in Example 1 using compound (2b) [50 mg, 0.070 mmol] and 4-oxa-7-azaspiro[2,5]octane hydrochloride [35 mg, 0.234 mmol] as starting materials. ¹H-NMR (CD₃OD) δ: 6.21 (1H, d, J = 11.2 Hz), 5.88 (1H, d, J = 11.2 Hz), 4.06-3.95 (2H, m), 3.79-3.67 (2H, m), 2.83 (1H, dd, J = 11.0, 3.9 Hz), 2.62-2.50 (2H, m), 2.44-2.13 (8H, m), 2.07-1.48 (14H, m), 1.38-1.20 (4H, m), 1.03 (3H, d, J = 6.3 Hz), 0.70 (2H, s), 0.59 (3H, s), 0.57-0.49 (2H, m).

Exact Mass = 429.32 (C₂₇H₄₃NO₃) Obs. mass = 430.30 (M+H)

### [Example 152]

### Synthesis of (1R,3R)-5-(2-((1R,3aS,7aR,E)-1-((S)-1-((2R,5S)-2,5-dimethylmorpholino)propan-2-yl)-7a-methyloctahydro-4H-inden-4-ylidene)ethylidene)cyclohexane-1,3-diol (Compound D020)

Compound (D020) [4.3 mg, 0.010 mmol] was obtained by performing the reaction in the same manner as in Example 1 using compound (2b) [50 mg, 0.070 mmol] and (2R,5S)-2,5-dimethylmorpholine [30 mg, 0.260 mmol] as starting materials.

Exact Mass = 431.34 (C₂₇H₄₅NO₃) Obs. mass = 432.25 (M+H)

### [Example 153]

### Synthesis of (1R,3R)-5-(2-((1R,3aS,7aR,E)-1-((S)-1-((2R,5R)-2,5-dimethylmorpholino)propan-2-yl)-7a-methyloctahydro-4H-inden-4-ylidene)ethylidene)cyclohexane-1,3-diol (Compound D021)

Compound (D021) [5.4 mg, 0.013 mmol] was obtained by performing the reaction in the same manner as in Example 1 using compound (2b) [50 mg, 0.070 mmol] and (2R,5R)-2,5-dimethylmorpholine [30 mg, 0.260 mmol] as starting materials.

Exact Mass = 431.34 (C₂₇H₄₅NO₃) Obs. mass = 432.30 (M+H)

### [Example 154]

### Synthesis of (1R,3S,Z)-5-(2-((1R,3aS,7aR,E)-7a-methyl-1-((S)-1-(4-(methylsulfonyl)piperazin-1-yl)propan-2-yl)octahydro-4H-inden-4-ylidene)ethylidene)-4-methylenecyclohexane-1,3-diol (Compound E001)

Compound (E001) [14.3 mg, 0.029 mmol] was obtained by performing the reaction in the same manner as in Example 92 using 1-methylsulfonylpiperazine instead of the starting material of triethyl-[[4-(trifluoromethyl)-4-piperidyl]oxy]silane in step 1 of Example 92.

¹H-NMR (CD₃OD) δ: 6.32 (1H, d, J = 10.7 Hz), 6.08 (1H, d, J = 11.2 Hz), 5.28 (1H, t, J = 1.2 Hz), 4.34 (1H, t, J = 6.1 Hz), 4.15-4.05 (1H, m), 3.25-3.15 (5H, m), 2.89-2.80 (4H, m), 2.66-2.03 (11H, m), 1.94-1.22 (13H, m), 1.04 (3H, d, J = 6.3 Hz), 0.60 (3H, s). Exact Mass = 492.30 (C₂₇H₄₄N₂O₄S) Obs. mass = 493.40 (M+H)

### [Example 155]

### Synthesis of (1R,3S)-5-(2-((1R,3aS,7aR,E)-7a-methyl-1-((S)-1-(4-(methylsulfonyl)piperazin-1-yl)propan-2-yl)octahydro-4H-inden-4-ylidene)ethylidene)-2-methylenecyclohexane-1,3-diol (Compound E003)

Compound (E003) [9.4 mg, 0.019 mmol] was obtained by performing the reaction in the same manner as in Example 1 using compound (2c) [50 mg, 0.069 mmol] and 1-(methylsulfonyl)piperazine [40 mg, 0.254 mmol] as starting materials.

¹H-NMR (CD₃OD) δ: 6.26 (1H, d, J = 11.0 Hz), 5.91 (1H, d, J = 11.0 Hz), 5.05 (2H, d, J = 7.3 Hz), 4.42-4.35 (2H, m), 3.26-3.13 (4H, m), 2.88-2.80 (1H, m), 2.82 (3H, s), 2.70-2.58 (3H, m), 2.48 (1H, dd, J = 13.3, 4.1 Hz), 2.40-2.25 (5H, m), 2.09-2.00 (3H, m), 1.98-1.90 (1H, m), 1.75-1.52 (6H, m), 1.39-1.28 (3H, m), 1.04 (3H, d, J = 6.4 Hz), 0.61 (3H, s).

Exact Mass = 492.30 (C₂₇H₄₄N₂O₄S) Obs. mass = 493.25 (M+H)

### [Example 156]

### Synthesis of (1R,3S,Z)-5-(2-((1R,3aS,7aR,E)-7a-methyl-1-((2S)-1-(3-(trifluoromethyl)piperazin-1-yl)propan-2-yl)octahydro-4H-inden-4-ylidene)ethylidene)-4-methylenecyclohexane-1,3-diol (Compounds E005a, E005b)

Compound (E005a) [11.3 mg, 0.0234 mmol] and compound (E005b) [9.7 mg, 0.020 mmol] were obtained by performing the reaction in the same manner as in Example 108 using compound (2a) [100 mg, 0.137 mmol] and 2-(trifluoromethyl)piperazine [50 mg, 0.324 mmol] as starting materials.

### Compound E005a

¹H-NMR (CD₃OD) δ: 6.32 (1H, d, J = 11.2 Hz), 6.08 (1H, d, J = 11.2 Hz), 5.28 (1H, dd, J = 2.4, 1.5 Hz), 4.34 (1H, t, J = 5.9 Hz), 4.15-4.09 (1H, m), 3.43-3.37 (1H, m), 3.00-2.95 (1H, m), 2.90-2.76 (4H, m), 2.51 (1H, dd, J = 13.4, 3.2 Hz), 2.34-1.99 (7H, m), 1.94-1.21 (15H, m), 1.02 (3H, d, J = 6.3 Hz), 0.59 (3H, s).

Exact Mass = 482.31 (C₂₇H₄₁F₃N₂O₂) Obs. mass = 483.4 (M+H)

### Compound E005b

1H-NMR (CD₃OD) δ: 6.32 (1H, d, J = 11.2 Hz), 6.08 (1H, d, J = 11.2 Hz), 5.28 (1H, dd, J = 2.4, 1.5 Hz), 4.34 (1H, t, J = 5.9 Hz), 4.15-4.09 (1H, m), 3.35-3.30 (1H, m), 2.95 (2H, d, J = 11.2 Hz), 2.85 (2H, td, J = 11.7, 2.9 Hz), 2.63 (1H, d, J = 11.7 Hz), 2.51 (1H, dd, J = 13.7, 3.4 Hz), 2.33-1.97 (7H, m), 1.94-1.21 (15H, m), 1.02 (3H, d, J = 6.8 Hz), 0.60 (3H, s).

Exact Mass = 482.31 (C₂₇H₄₁F₃N₂O₂) Obs. mass = 483.3 (M+H)

### [Example 157]

### Synthesis of (1R,3S,Z)-5-(2-((1R,3aS,7aR,E)-7a-methyl-1-((2S)-1-(3-(trifluoromethyl)piperazin-1-yl)propan-2-yl)octahydro-4H-inden-4-ylidene)ethylidene)cyclohexane-1,3-diol (Compounds E006a, E006b)

Compound (E006a) [7.0 mg, 0.015 mmol] and compound (E006b) [7.0 mg, 0.015 mmol] were obtained by performing the reaction in the same manner as in Example 108 using compound (2b) [100 mg, 0.139 mmol] and 2-(trifluoromethyl)piperazine [70 mg, 0.454 mmol] as starting materials.

### Compound E006a

¹H-NMR (CD₃OD) δ: 6.21 (1H, d, J = 11.4 Hz), 5.89 (1H, d, J = 11.0 Hz), 4.06-3.93 (2H, m), 3.41-3.37 (1H, m), 2.97 (1H, d, J = 12.3 Hz), 2.86-2.74 (4H, m), 2.59 (1H, dd, J = 13.5, 3.4 Hz), 2.40 (1H, dd, J = 13.3, 3.7 Hz), 2.31 (1H, dd, J = 11.9, 3.2 Hz), 2.23-1.48 (17H, m), 1.38-1.21 (4H, m), 1.02 (3H, d, J = 6.4 Hz), 0.60 (3H, s).

Exact Mass = 470.31 (C₂₆H₄₁F₃N₂O₂) Obs. mass = 471.25 (M+H)

### Compound E006b

¹H-NMR (CD₃OD) δ: 6.21 (1H, d, J = 11.4 Hz), 5.89 (1H, d, J = 11.0 Hz), 4.06-3.93 (2H, m), 3.36-3.30 (1H, m), 2.95 (2H, d, J = 11.9 Hz), 2.85 (2H, td, J = 11.5, 2.9 Hz), 2.65-2.54 (2H, m), 2.40 (1H, dd, J = 13.3, 3.7 Hz), 2.31 (1H, dd, J = 12.1, 3.0 Hz), 2.23-1.49 (16H, m), 1.38-1.21 (4H, m), 1.03 (3H, d, J = 6.4 Hz), 0.60 (3H, s).

Exact Mass = 470.31 (C₂₆H₄₁F₃N₂O₂) Obs. mass = 471.25 (M+H)

### [Example 158]

### Synthesis of (1R,2S,3S,Z)-2-methyl-5-(2-((1R,3aS,7aR,E)-7a-methyl-1-((2S)-1-(3-(trifluoromethyl)piperazin-1-yl)propan-2-yl)octahydro-4H-inden-4-ylidene)ethylidene)-4-methylenecyclohexane-1,3-diol (Compounds E007a, E007b)

Compound (E007a) [20.9 mg, 0.042 mmol] and compound (E007b) [27.4 mg, 0.055 mmol] were obtained by performing the reaction in the same manner as in Example 109 using compound (6c) [264.5 mg, 0.599 mmol] and 2-(trifluoromethyl)piperazine [220.7 mg, 1.43 mmol] as starting materials.

### Compound E007a:

¹H-NMR (CD₃OD) δ: 6.33 (1H, d, J = 11.0 Hz), 6.09 (1H, d, J = 11.0 Hz), 5.22 (1H, d, J = 2.0 Hz), 4.88 (1H, brs), 4.22 (1H, d, J = 3.4 Hz), 3.75-3.68 (1H, m), 3.47-3.30 (2H, m), 2.99-2.75 (6H, m), 2.59 (1H, dd, J = 13.7, 3.9 Hz), 2.33 (1H, dd, J = 12.0, 3.2 Hz), 2.19-2.01 (6H, m), 1.93-1.42 (12H, m), 1.38-1.21 (4H, m), 1.04 (3H, d, J = 6.3 Hz), 1.02 (3H, d, J = 6.4 Hz), 0.58 (3H, s).

### Compound E007b:

¹H-NMR (CD₃OD) δ: 6.33 (1H, d, J = 11.0 Hz), 6.09 (1H, d, J = 11.0 Hz), 5.22 (1H, d, J = 2.0 Hz), 4.89 (1H, brs), 4.22 (1H, d, J = 3.4 Hz), 3.75-3.69 (1H, m), 3.38-3.30 (1H, m), 2.98-2.80 (4H, m), 2.63-2.57 (2H, m), 2.30 (1H, dd, J = 12.2, 3.4 Hz), 2.17 (2H, dd, J = 11.2, 2.9 Hz), 2.06-1.97 (4H, m), 1.91-1.20 (13H, m), 1.04 (3H, d, J = 6.3 Hz), 1.02 (3H, d, J = 6.3 Hz), 0.59 (3H, s).

### [Example 159]

### Synthesis of (1R,3S,Z)-5-(2-((1R,3aS,7aR,E)-1-((S)-1-(4-(ethylsulfonyl)piperazin-1-yl)propan-2-yl)-7a-methyloctahydro-4H-inden-4-ylidene)ethylidene)-4-methylenecyclohexane-1,3-diol (compound E009)

Compound (E009) [20.6 mg, 0.041 mmol] was obtained by performing the reaction in the same manner as in Example 1 using compound (2a) [100 mg, 0.137 mmol] and 4-(ethylsulfonyl)piperazine [55 mg, 0.309 mmol] as starting materials.

¹H-NMR (CD₃OD) δ: 6.32 (1H, d, J = 11.2 Hz), 6.08 (1H, d, J = 11.2 Hz), 5.28 (1H, dd, J = 2.4, 1.0 Hz), 4.34 (1H, t, J = 5.9 Hz), 4.15-4.09 (1H, m), 3.28-3.23 (3H, m), 3.02 (2H, q, J = 7.3 Hz), 2.87 (1H, dd, J = 12.0, 3.7 Hz), 2.65-2.55 (2H, m), 2.51 (1H, dd, J = 13.4, 3.2 Hz), 2.39-2.30 (3H, m), 2.25 (1H, dd, J = 13.4, 6.6 Hz), 2.10-1.99 (3H, m), 1.94-1.21 (17H, m), 1.03 (3H, d, J = 6.3 Hz), 0.59 (3H, s).

### [Reference example 13]

### Synthesis of (3R)-3-((1R,3aS,7aR,E)-4-((Z)-2-((3S,5R)-3,5-bis((t-butyldimethylsilyl)oxy)-2-methylenecyclohexylidene)ethylidene)-7a-methyloctahydro-1H-inden-1-yl)butyl 4-methylbenzenesulfonate (Compound 16a)

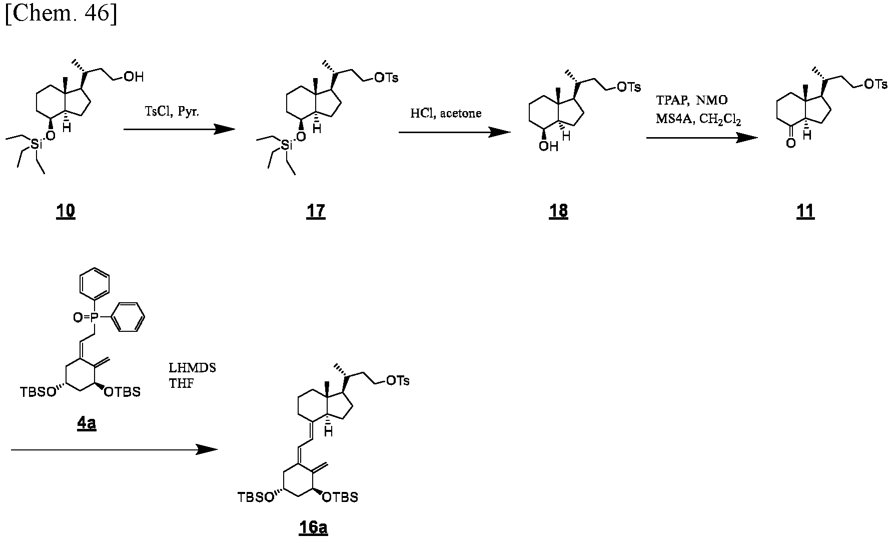

### Step 1

para-Toluenesulfonyl chloride [1.6 g, 8.4 mmol] was added to a solution of (3R)-3-((1R,4S,7aR)-7a-methyl-4-((triethylsilyl)oxy)octahydro-1H-inden-1-yl)butan-1-ol (Compound 10, CAS Registry No. 300344-39-2) [2.37 g, 6.68 mmol] in pyridine [12 mL], and the mixture was stirred at room temperature for 3 hours. The reaction mixture was transferred to saturated brine and the mixture was extracted with ethyl acetate. The organic layer was washed sequentially with 1 M hydrochloric acid, saturated sodium hydrogen carbonate and saturated brine. The organic layer was dried over anhydrous magnesium sulfate and concentrated under reduced pressure to obtain crude product containing compound 17.

### Step 2

The above crude product containing compound 17 was dissolved in acetone [30 mL], then to the solution, 2 M hydrochloric acid [10 mL, 20 mmol] was added at room temperature, and the mixture was stirred at room temperature for 1 hour. The reaction system was quenched with a saturated aqueous sodium hydrogen carbonate solution and extracted with ethyl acetate. The organic layer was dried over anhydrous magnesium sulfate and concentrated under reduced pressure. The residue was purified by silica gel column chromatography to obtain (3R)-3-((1R,4S,7aR)-4-hydroxy-7a-methyloctahydro-1H-inden-1-yl)butyl 4-methylbenzenesulfonate (Compound 18) [1.37 g, 3.47 mmol] (Two step yield = 52%).

### Step 3

A solution of compound 18 [1.37 g, 3.47 mmol] obtained in step 2, 4-methylmorpholine N-oxide (NMO) [0.67 g, 5.0 mmol] and molecular sieves 4A (MS4A) [1.5 g] in dichloromethane [30 mL] was stirred at 0°C for 1 hour. Tetrabutylammonium perruthenate (TPAP) [120 mg, 0.342 mmol] was added to the reaction mixture, and the mixture was stirred at 0°C for 1 hour. Heptane [30 mL] was added to the reaction mixture at room temperature and the mixture was filtered through Celite. The filtrate was washed sequentially with a saturated aqueous ammonium chloride solution and saturated brine. The organic layer was dried over anhydrous magnesium sulfate and concentrated under reduced pressure. The residue was purified by silica gel column chromatography to obtain (3R)-3-((1R,3aR,7aR)-7a-methyl-4-oxooctahydro-1H-inden-1-yl)butyl 4-methylbenzenesulfonate (Compound 11) [1.15 g, 2.93 mmol] (Yield = 84%). ¹H-NMR(CDCl₃) δ: 7.79 (2H, d, J = 7.8 Hz), 7.35 (2H, d, J = 7.8 Hz), 4.06-3.94 (2H, m), 2.45 (3H, s), 2.43 (1H, dd, J = 12.2 7.8 Hz), 2.33-2.17 (2H, m), 2.11-1.65 (6H, m), 1.60-0.99 (9H, m), 0.91 (3H, d, J = 6.3 Hz), 0.60 (3H, s).

### Step 4

Under a nitrogen atmosphere, LHMDS [1 M in THF, 5 mL, 5 mmol] was added to a solution of compound 4a [2.1 g, 3.6 mmol] in THF [21 mL] at -78°C and the reaction mixture was stirred at the same temperature for 30 minutes. A solution of compound 11 [0.85 g, 2.2 mmol] obtained in step 3 in THF [10 mL] was added to the mixture, and the reaction mixture was further stirred at the same temperature for 1.5 hours. The reaction mixture was warmed to room temperature, a saturated aqueous ammonium chloride solution was added to the mixture, and the mixture was extracted with ethyl acetate. The organic layer was dried over anhydrous magnesium sulfate and concentrated under reduced pressure. The residue was purified by silica gel column chromatography to obtain compound 16a [1.21 g, 1.63 mmol] (Yield = 72%).

¹H-NMR (CDCl₃) δ: 7.80 (2H, d, J = 8.2 Hz), 7.35 (2H, d, J = 8.2 Hz), 6.23 (1H, d, J = 11.0 Hz), 6.00 (1H, d, J = 11.4 Hz), 5.18 (1H, s), 4.86 (1H, d, J = 2.3 Hz), 4.37 (1H, dd, J = 7.0, 4.0 Hz), 4.23-4.16 (1H, m), 4.15-4.03 (2H, m), 2.82 (1H, d, J = 11.9 Hz), 2.48-2.41 (1H, m), 2.45 (3H, s), 2.21 (1H, dd, J = 13.0, 7.5 Hz), 1.96-1.58 (8H, m), 1.53-1.18 (10H, m), 0.88 (9H, s), 0.88 (9H, s), 0.84 (3H, d, J = 6.9 Hz), 0.49 (3H, s), 0.07 (3H, s), 0.06 (9H, s).

### [Reference example 14]

### Synthesis of (3R)-3-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)butyl 4-methylbenzenesulfonate (Compound 16b)

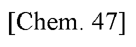

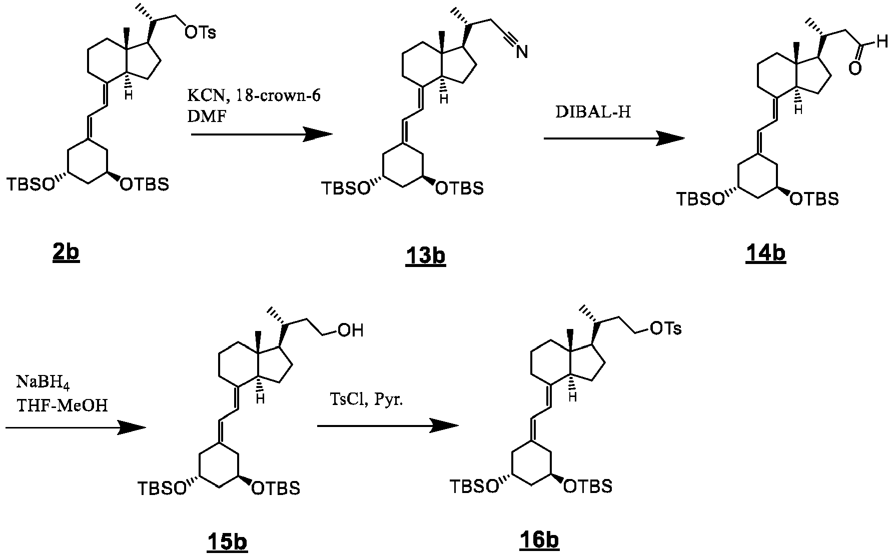

### Step 1

Potassium cyanide [2.16 g, 33.2 mmol] was added to a solution of compound 2b [11.94 g, 16.65 mmol] and 18-crown-6 [0.45 g, 1.7 mmol] in DMF [100 mL], and the mixture was heated and stirred at 60°C for 4 hours. After cooling to room temperature, the reaction mixture was poured into saturated brine and the mixture was extracted with ethyl acetate. The organic layer was washed with saturated brine. The organic layer was dried over anhydrous magnesium sulfate and concentrated under reduced pressure. The residue was purified by silica gel column chromatography to obtain compound 13b [8.02 g, 14.0 mmol] (Yield = 84.2%).

¹H-NMR (CDCl₃) δ: 6.16 (1H, d, J = 11.4 Hz), 5.82 (1H, d, J = 11.0 Hz), 4.11-4.02 (2H, m), 2.82 (1H, dd, J = 12.3, 2.7 Hz), 2.40-2.35 (3H, m), 2.30-2.22 (2H, m), 2.08 (2H, dt, J = 23.0, 7.9 Hz), 1.98-1.88 (2H, m), 1.82-1.59 (6H, m), 1.55-1.26 (8H, m), 1.18 (3H, d, J = 6.4 Hz), 0.87 (9H, s), 0.86 (9H, s), 0.56 (3H, s), 0.06 (3H, s), 0.05 (9H, s).

### Step 2

Under a nitrogen atmosphere, a solution of diisobutylaluminum hydride (DIBAL-H) [1 M in hexane, 42 mL, 42 mmol] was added to a solution of compound 13b [8.02 g, 14.0 mmol] in THF[120 mL] at -10°C, and the mixture was stirred at the same temperature for 1 hour. The reaction system was quenched with a saturated aqueous ammonium chloride solution. A saturated aqueous solution of potassium sodium tartrate (Rochelle salt) was added to the mixture, and the mixture was extracted with ethyl acetate. The organic layer was washed with saturated brine, dried over anhydrous magnesium sulfate, and concentrated under reduced pressure. The residue was purified by silica gel column chromatography to obtain compound 14b [4.93 g, 8.57 mmol] (Yield = 61%).

¹H-NMR (CDCl₃) δ: 9.76 (1H, dd, J = 3.2, 1.4 Hz), 6.16 (1H, d, J = 11.4 Hz), 5.82 (1H, d, J = 11.4 Hz), 4.10-4.04 (2H, m), 2.82 (1H, dd, J = 11.7, 3.9 Hz), 2.48 (1H, dd, J = 15.3, 3.0 Hz), 2.42-2.35 (2H, m), 2.27-1.77 (9H, m), 1.70-1.60 (4H, m), 1.58-1.50 (3H, m), 1.40-1.25 (5H, m), 1.03 (3H, d, J = 6.9 Hz), 0.87 (9H, s), 0.86 (9H, s), 0.59 (3H, s), 0.06 (3H, s), 0.05 (6H, s).

### Step 3

Compound 14b [4.92 g, 8.56 mmol] was dissolve in a mixed solution of THF [50 mL]/methanol [50 mL], sodium borohydride [0.65 g, 17.0 mmol] was added to the solution at 0°C, and the mixture was stirred at room temperature for 1 hour. The reaction mixture was quenched with saturated ammonium chloride at 0°C, poured into saturated brine, and the mixture was extracted with ethyl acetate. The organic layer was washed with saturated brine, dried over anhydrous magnesium sulfate, and concentrated under reduced pressure. The residue was purified by silica gel column chromatography to obtain compound 15b [4.32 g, 7.49 mmol] (Yield = 87%).

¹H-NMR (CDCl₃) δ: 6.17 (1H, d, J = 10.5 Hz), 5.82 (1H, d, J = 11.0 Hz), 4.12-4.03 (2H, m), 3.78-3.60 (2H, m), 2.81 (1H, d, J = 11.9 Hz), 2.42-2.35 (2H, m), 2.26 (1H, d, J = 13.3 Hz), 2.11 (1H, t, J = 10.1 Hz), 2.04-1.90 (3H, m), 1.82-1.60 (6H, m), 1.59-1.48 (4H, m), 1.35-1.22 (5H, m), 1.16 (1H, s), 0.99-0.92 (3H, m), 0.87 (9H, s), 0.86 (9H, s), 0.55 (3H, s), 0.08-0.03 (12H, m)

### Step 4

TsCl [3.45 g, 18.1 mmol] and 4-dimethylaminopyridine [94 mg, 0.77 mmol] were added to a solution of compound 15b [4.17 g, 7.23 mmol] in pyridine [40 mL], and the mixture was stirred at room temperature for 2 hours. The reaction mixture was poured into saturated brine and the mixture was extracted with ethyl acetate. The organic layer was washed with saturated brine, dried over anhydrous magnesium sulfate, and concentrated under reduced pressure. The residue was purified by silica gel column chromatography to obtain compound 16b [4.60 g, 6.29 mmol] (Yield = 87%).

¹H-NMR (CDCl₃) δ: 7.80 (2H, d, J = 8.3 Hz), 7.35 (2H, d, J = 8.3 Hz), 6.16 (1H, d, J = 11.2 Hz), 5.80 (1H, d, J = 11.2 Hz), 4.17-4.01 (4H, m), 2.85-2.70 (1H, m), 2.45 (3H, s), 2.41-2.23 (2H, m), 1.98-1.60 (8H, m), 1.51-1.18 (10H, m), 0.87-0.85 (27H, m), 0.50 (3H, s), 0.05 (6H, s), 0.05 (6H, s).

### [Reference example 15]

### Synthesis of (3R)-3-((1R,3aS,7aR,E)-4-(2-((3R,5R)-3,5-bis((t-butyldimethylsilyl)oxy)-4-methylenecyclohexylidene)ethylidene)-7a-methyloctahydro-1H-inden-1-yl)butyl 4-methylbenzenesulfonate (Compound 16c)

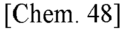

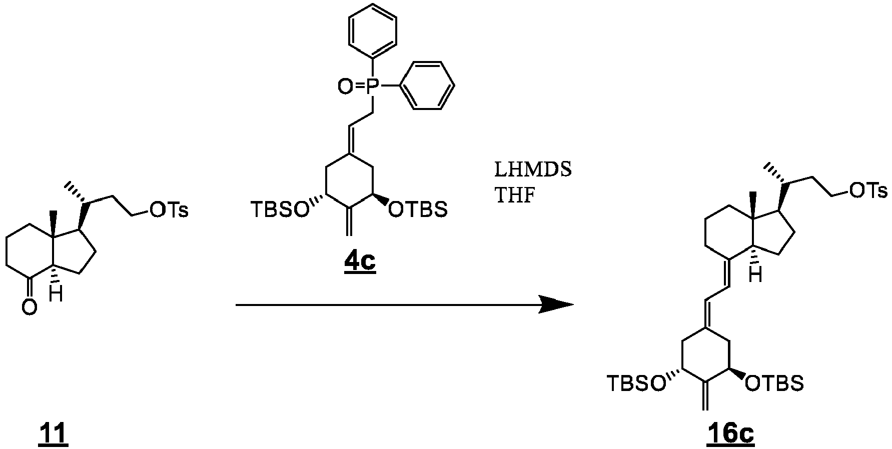

A solution of (3R)-3-((1R,3aR,7aR)-7a-methyl-4-oxooctahydro-1H-inden-1-yl)butyl 4-methylbenzenesulfonate (Compound 11) [1.24 g, 3.28 mmol] described in step 3 of Reference example 13, and compound 4c [1.3 g, 2.2 mmol] in THF [15 mL] was cooled to -78°C under a nitrogen atmosphere. LHMDS [1 M in THF, 4.5 mL, 4.5 mmol] was added dropwise to the solution, and the mixture was stirred at the same temperature for 3 hours. The reaction mixture was warmed to room temperature and quenched with a saturated aqueous ammonium chloride solution. The reaction mixture was extracted with ethyl acetate, the organic layer was dried over anhydrous magnesium sulfate, and concentrated under reduced pressure. The residue was purified by silica gel column chromatography to obtain compound 16c [0.83 g, 1.1 mmol] (Yield = 50%).

¹H-NMR (CDCl₃) δ: 7.80 (2H, d, J = 8.3 Hz), 7.35 (2H, d, J = 8.8 Hz), 6.21 (1H, d, J = 11.2 Hz), 5.83 (1H, d, J = 11.2 Hz), 4.97 (1H, s), 4.92 (1H, s), 4.43 (2H, dd, J = 7.6, 4.1 Hz), 4.11-4.02 (2H, m), 2.81 (1H, dd, J = 10.0, 3.8 Hz), 2.55-2.45 (2H, m), 2.45 (3H, s), 2.31 (1H, dd, J = 13.4, 3.2 Hz), 2.17 (1H, dd, J = 12.4, 8.5 Hz), 1.99-1.60 (6H, m), 1.52-1.18 (8H, m), 0.90 (9H, s), 0.87 (9H, s), 0.50 (3H, s), 0.07 (3H, s), 0.05 (3H, s), 0.03 (3H, s).

### [Example 160]

### Synthesis of (1R,3R)-5-(2-((1R,3aS,7aR,E)-1-((R)-4-(3-(difluoromethyl)azetidin-1-yl)butan-2-yl)-7a-methyloctahydro-4H-inden-4-ylidene)ethylidene)cyclohexane-1,3-diol (Compound A022)

Compound (A022) [14.7 mg, 0.0336 mmol] was obtained by performing the reaction in the same manner as in Example 1 using compound (16b) [100 mg, 0.137 mmol] and 3-difluoromethylazetidine hydrochloride [60 mg, 0.418 mmol] as starting materials.

¹H-NMR (CD₃OD) δ: 6.21 (1H, d, J = 11.2 Hz), 6.00 (1H, td, J = 57.0, 5.0 Hz), 5.88 (1H, d, J = 11.2 Hz), 4.05 (2H, s), 4.05-3.94 (2H, m), 3.38 (2H, t, J = 8.3 Hz), 3.17 (2H, t, J = 7.3 Hz), 2.91 (1H, td, J = 13.5, 7.3 Hz), 2.82 (1H, dd, J = 12.2, 3.9 Hz), 2.58 (1H, dd, J = 13.7, 3.4 Hz), 2.52 (1H, dd, J = 10.7, 4.9 Hz), 2.46-2.38 (2H, m), 2.22-2.12 (2H, m), 2.06-1.90 (3H, m), 1.86-1.27 (13H, m), 1.17-1.06 (1H, m), 0.96 (3H, d, J = 6.3 Hz), 0.57 (3H, s).

Exact Mass = 437.31 (C₂₆H₄₁F₂NO₂) Obs. mass = 438.25 (M+H)

### [Example 161]

### Synthesis of (1R,3R)-5-(2-((1R,3aS,7aR,E)-1-((R)-4-(3-hydroxy-3-(trifluoromethyl)azetidin-1-yl)butan-2-yl)-7a-methyloctahydro-4H-inden-4-ylidene)ethylidene)-2-methylenecyclohexane-1,3-diol (Compound A023)

Compound A023 [9.8 mg, 0.020 mmol] was obtained by performing the reaction in the same manner as in Example 1 using compound (16c) [50 mg, 0.067 mmol] and 3-hydroxy-3-(trifluoromethyl)azetidine [30 mg, 0.213 mmol] as starting materials.

¹H-NMR (CD₃OD) δ: 6.27 (1H, d, J = 11.2 Hz), 5.91 (1H, d, J = 11.2 Hz), 5.06 (2H, d, J = 6.8 Hz), 4.43-4.37 (2H, m), 3.70 (2H, d, J = 10.7 Hz), 2.86 (1H, dd, J = 12.0, 3.7 Hz), 2.75-2.53 (3H, m), 2.49 (1H, dd, J = 13.2, 3.9 Hz), 2.32-2.25 (2H, m), 2.08-1.98 (2H, m), 1.95-1.13 (12H, m), 0.98 (3H, d, J = 6.3 Hz), 0.59 (3H, s).

Exact Mass = 483.30 (C₂₇H₄₀F₃NO₃) Obs. mass = 484.25 (M+H)

### [Example 162]

### Synthesis of (1R,3R)-5-(2-((1R,3aS,7aR,E)-1-((R)-4-(3-(2,2-difluoroethyl)azetidin-1-yl)butan-2-yl)-7a-methyloctahydro-4H-inden-4-ylidene)ethylidene)cyclohexane-1,3-diol (Compound A024)

Compound A024 [17.0 mg, 0.376 mmol] was obtained by performing the reaction in the same manner as in Example 1 using compound (16b) [100 mg, 0.137 mmol] and 3-(2,2-difluoroethyl)azetidine hydrochloride [70 mg, 0.444 mmol] as starting materials.

¹H-NMR (CD₃OD) δ: 6.21 (1H, d, J = 11.2 Hz), 5.88 (1H, d, J = 11.2 Hz), 5.86 (1H, tt, J = 56.0, 4.5 Hz), 4.06-3.95 (2H, m), 3.49 (2H, t, J = 7.6 Hz), 2.87 (2H, dt, J = 3.0, 8.0 Hz), 2.83 (1H, dd, J = 13.0, 4.0 Hz), 2.75-2.63 (1H, m), 2.58 (1H, dd, J = 13.2, 3.9 Hz), 2.54-2.49 (1H, m), 2.44-2.36 (2H, m), 2.23-1.91 (7H, m), 1.87-1.44 (9H, m), 1.33 (3H, td, J = 9.6, 6.0 Hz), 1.15-1.05 (1H, m), 0.96 (3H, d, J = 6.8 Hz), 0.57 (3H, s).

Exact Mass = 451.33 (C₂₇H₄₃F₂NO₂) Obs. mass = 452.25 (M+H)

### [Example 163]

### Synthesis of (1R,3R)-5-(2-((1R,3aS,7aR,E)-1-((R)-4-(3-(difluoromethoxy)azetidin-1-yl)butan-2-yl)-7a-methyloctahydro-4H-inden-4-ylidene)ethylidene)cyclohexane-1,3-diol (Compound A025)

Compound A025 [21.6 mg, 0.476 mmol] was obtained by performing the reaction in the same manner as in Example 1 using compound (16b) [100 mg, 0.137 mmol] and 3-(difluoromethoxy)azetidine [70 mg, 0.444 mmol] as starting materials.

¹H-NMR (CD₃OD) δ: 6.38 (1H, t, J = 75.0 Hz), 6.21 (1H, d, J = 10.7 Hz), 5.88 (1H, d, J = 10.7 Hz), 4.76-4.70 (1H, m), 4.06-3.95 (2H, m), 3.64 (2H, td, J = 6.1, 2.4 Hz), 3.12-3.07 (2H, m), 2.83 (1H, dd, J = 11.5, 4.1 Hz), 2.61-2.44 (3H, m), 2.40 (1H, dd, J = 13.7, 3.4 Hz), 2.23-2.13 (2H, m), 2.05-1.92 (3H, m), 1.87-1.73 (2H, m), 1.68-1.45 (7H, m), 1.38-1.28 (3H, m), 1.17-1.06 (1H, m), 0.96 (3H, d, J = 6.3 Hz), 0.57 (3H, s).

Exact Mass = 453.31 (C₂₆H₄₁F₂NO₃) Obs. mass = 454.25 (M+H)

### [Example 164]

### Synthesis of (1R,3R)-5-(2-((1R,3aS,7aR,E)-1-((R)-4-(3-(2,2-difluoroethoxy)azetidin-1-yl)butan-2-yl)-7a-methyloctahydro-4H-inden-4-ylidene)ethylidene)cyclohexane-1,3-diol (Compound A026)

Compound A026 [12.8 mg, 0.0282 mmol] was obtained by performing the reaction in the same manner as in Example 1 using compound (16b) [50 mg, 0.068 mmol] and 3-(2,2-difluoroethoxy)azetidine hydrochloride [35 mg, 0.202 mmol] as starting materials.

¹H-NMR (CD₃OD) δ: 6.16 (1H, d, J = 10.7 Hz), 5.85 (1H, tt, J = 3.5, 55.0 Hz), 5.84 (1H, d, J = 10.7 Hz), 4.17-4.11 (1H, m), 4.02-3.90 (2H, m), 3.63-3.52 (4H, m), 2.92 (2H, dt, J = 22.9, 6.8 Hz), 2.78 (1H, dd, J = 12.0, 3.7 Hz), 2.54 (1H, dd, J = 13.2, 3.4 Hz), 2.44 (1H, dd, J = 12.2, 2.9 Hz), 2.36 (1H, dd, J = 13.2, 3.4 Hz), 2.26-2.09 (3H, m), 1.99-1.24 (15H, m), 0.94 (3H, d, J = 6.3 Hz), 0.53 (3H, s).

### [Example 165]

### Synthesis of (1R,3R)-5-(2-((1R,3aS,7aR,E)-1-((R)-4-(3-methoxy-3-(trifluoromethyl)azetidin-1-yl)butan-2-yl)-7a-methyloctahydro-4H-inden-4-ylidene)ethylidene)cyclohexane-1,3-diol (Compound A027)

Compound A027 [1.6 mg, 0.0033 mmol] was obtained by performing the reaction in the same manner as in Example 1 using compound (16b) [50 mg, 0.068 mmol] and 3-methoxy-3-(trifluoromethyl)azetidine hydrochloride [30 mg, 0.157 mmol] as starting materials.

Exact Mass = 485.31 (C₂₇H₄₂F₃NO₃) Obs. mass = 486.25 (M+H)

### [Example 166]

### Synthesis of (1R,3R)-5-(2-((1R,3aS,7aR,E)-7a-methyl-1-((R)-4-(3-(trifluoromethoxy)azetidin-1-yl)butan-2-yl)octahydro-4H-inden-4-ylidene)ethylidene)cyclohexane-1,3-diol (Compound A028)

Compound A028 [1.1 mg, 0.0023 mmol] was obtained by performing the reaction in the same manner as in Example 1 using compound (16b) [50 mg, 0.068 mmol] and 3-(trifluoromethoxy)azetidine hydrochloride [30 mg, 0.169 mmol] as starting materials.

Exact Mass = 471.30 (C₂₆H₄₀F₃NO₃) Obs. mass = 472.25 (M+H)

### [Example 167]

### Synthesis of (1R,3R)-5-(2-((1R,3aS,7aR,E)-1-((R)-4-(3-(2,2-difluoroethoxy)azetidin-1-yl)butan-2-yl)-7a-methyloctahydro-4H-inden-4-ylidene)ethylidene)-2-methylenecyclohexane-1,3-diol (Compound A029)

Compound A029 [15.9 mg, 0.0332 mmol] was obtained by performing the reaction in the same manner as in Example 1 using compound (16c) [50 mg, 0.067 mmol] and 3-(2,2-difluoroethoxy)azetidine hydrochloride [35 mg, 0.202 mmol] as starting materials.

¹H-NMR (CD₃OD) δ: 6.24 (1H, d, J = 11.4 Hz), 6.04-5.74 (2H, m), 5.03 (2H, d, J = 6.9 Hz), 4.42-4.32 (2H, m), 4.21-4.15 (1H, m), 3.62 (2H, dd, J = 14.4, 3.9 Hz), 3.59-3.56 (2H, m), 2.99-2.94 (2H, m), 2.83 (1H, dd, J = 12.3, 3.7 Hz), 2.65 (1H, dd, J = 13.3, 4.6 Hz), 2.57-2.40 (3H, m), 2.30-2.23 (2H, m), 2.05-1.85 (3H, m), 1.69-1.23 (10H, m), 1.16-1.06 (1H, m), 0.94 (3H, d, J = 6.4 Hz), 0.56 (3H, s).

Exact Mass = 479.32 (C₂₈H₄₃F₂NO₃) Obs. mass = 480.25 (M+H)

### [Example 168]

### Synthesis of (1R,3R)-5-(2-((1R,3aS,7aR,E)-1-((R)-4-(3-(difluoromethoxy)azetidin-1-yl)butan-2-yl)-7a-methyloctahydro-4H-inden-4-ylidene)ethylidene)-2-methylenecyclohexane-1,3-diol (Compound A030)

Compound A030 [10.9 mg, 0.234 mmol] was obtained by performing the reaction in the same manner as in Example 5 using compound (16c) [50 mg, 0.067 mmol] and 3-(difluoromethoxy)azetidine [30 mg, 0.244 mmol] as starting materials.

¹H-NMR (CD₃OD) δ: 6.38 (1H, t, J = 75.0 Hz), 6.26 (1H, d, J = 11.2 Hz), 5.90 (1H, d, J = 11.2 Hz), 5.05 (2H, d, J = 6.8 Hz), 4.76-4.70 (1H, m), 4.43-4.35 (2H, m), 3.68-3.60 (2H, m), 3.12-3.07 (2H, m), 2.84 (1H, dd, J = 4.0, 12.6 Hz), 2.66 (1H, dd, J = 12.6, 4.0 Hz), 2.60-2.42 (3H, m), 2.31-2.24 (2H, m), 2.07-2.00 (2H, m), 1.98-1.90 (2H, m), 1.69-1.40 (7H, m), 1.38-1.25 (3H, m), 1.17-1.09 (1H, m), 0.96 (3H, d, J = 6.3 Hz), 0.58 (3H, s).

Exact Mass = 465.31 (C₂₇H₄₁F₂NO₃) Obs. mass = 466.25 (M+H)

### [Example 169]

### Synthesis of (1R,3R)-5-(2-((1R,3aS,7aR,E)-1-((R)-4-(3-(difluoromethyl)azetidin-1-yl)butan-2-yl)-7a-methyloctahydro-4H-inden-4-ylidene)ethylidene)-2-methylenecyclohexane-1,3-diol (Compound A031)

Compound A031 [9.4 mg, 0.021 mmol] was obtained by performing the reaction in the same manner as in Example 1 using compound (16c) [50 mg, 0.067 mmol] and 3-(difluoromethyl)azetidine hydrochloride [30 mg, 0.209 mmol] as starting materials.

¹H-NMR (CD₃OD) δ: 6.27 (1H, d, J = 11.2 Hz), 6.10 (1H, td, J = 56.4, 4.2 Hz), 5.92 (1H, d, J = 11.2 Hz), 5.06 (2H, d, J = 6.8 Hz), 4.40 (2H, ddd, J = 14.3, 7.2, 4.5 Hz), 3.80 (2H, t, J = 9.3 Hz), 3.65 (2H, t, J = 8.1 Hz), 3.17-3.09 (1H, m), 2.89 (2H, ddd, J = 23.1, 11.6, 4.3 Hz), 2.78 (1H, td, J = 11.3, 5.2 Hz), 2.68 (1H, dd, J = 13.4, 4.1 Hz), 2.49 (1H, dd, J = 13.2, 3.9 Hz), 2.32-2.25 (2H, m), 2.11-1.97 (3H, m), 1.96-1.90 (1H, m), 1.72-1.18 (12H, m), 1.00 (3H, d, J = 6.3 Hz), 0.60 (3H, s).

Exact Mass = 449.31 (C₂₇H₄₁F₂NO₂) Obs. mass = 450.40 (M+H)

### [Example 170]

### Synthesis of (1R,3R)-5-(2-((1R,3aS,7aR,E)-1-((R)-4-(3-(1,1-difluoroethyl)azetidin-1-yl)butan-2-yl)-7a-methyloctahydro-4H-inden-4-ylidene)ethylidene)cyclohexane-1,3-diol (Compound A032)

Compound A032 [15.5 mg, 0.0343 mmol] was obtained by performing the reaction in the same manner as in Example 1 using compound (16b) [100 mg, 0.137 mmol] and 3-(1,1-difluoroethyl)azetidine hydrochloride [70 mg, 0.444 mmol] as starting materials.

¹H-NMR (CD₃OD) δ: 6.21 (1H, d, J = 11.2 Hz), 5.88 (1H, d, J = 11.2 Hz), 4.06-3.95 (2H, m), 3.43 (2H, t, J = 8.1 Hz), 3.12 (2H, td, J = 7.9, 2.1 Hz), 3.04-2.93 (1H, m), 2.83 (1H, dd, J = 12.0, 3.7 Hz), 2.58 (1H, dd, J = 13.7, 3.9 Hz), 2.54-2.49 (1H, m), 2.46-2.36 (2H, m), 2.23-2.13 (2H, m), 2.05-1.91 (3H, m), 1.87-1.41 (13H, m), 1.38-1.28 (3H, m), 1.16-1.03 (1H, m), 0.96 (3H, d, J = 6.8 Hz), 0.57 (3H, s).

Exact Mass = 451.33 (C₂₇H₄₃F₂NO₂) Obs. mass = 452.30 (M+H)

### [Example 171]

### Synthesis of (1R,3R)-5-(2-((1R,3aS,7aR,E)-1-((R)-4-(3-hydroxy-3-isopropylazetidin-1-yl)butan-2-yl)-7a-methyloctahydro-4H-inden-4-ylidene)ethylidene)cyclohexane-1,3-diol (Compound A034)

Compound A034 [8.1 mg, 0.018 mmol] was obtained using compound (16b) [50 mg, 0.068 mmol] and 3-isopropylazetidin-3-ol hydrochloride [30 mg, 0.218 mmol].

¹H-NMR (CD₃OD) δ: 6.21 (1H, d, J = 11.0 Hz), 5.88 (1H, d, J = 11.4 Hz), 4.06-3.95 (2H, m), 3.39 (1H, s), 3.37 (1H, s), 3.01 (1H, s), 2.99 (1H, s), 2.83 (1H, dd, J = 11.9, 3.7 Hz), 2.64-2.55 (2H, m), 2.49-2.36 (2H, m), 2.23-2.13 (2H, m), 2.05-1.29 (16H, m), 1.19-1.10 (1H, m), 0.97 (3H, d, J = 6.9 Hz), 0.90 (6H, d, J = 6.9 Hz), 0.57 (3H, s).

Exact Mass = 445.36 (C₂₈H₄₇NO₃) Obs. mass = 446.30 (M+H)

### [Example 172]

### Synthesis of (1R,3R)-5-(2-((1R,3aS,7aR,E)-1-((R)-4-((S)-3-fluoropyrrolidin-1-yl)butan-2-yl)-7a-methyloctahydro-4H-inden-4-ylidene)ethylidene)cyclohexane-1,3-diol (Compound B014)

Compound B014 [9.0 mg, 0.021 mmol] was obtained using compound (16b) [50 mg, 0.068 mmol] and (S)-3-fluoropyrrolidine hydrochloride [30 mg, 0.239 mmol]. Exact Mass = 419.32 (C₂₆H₄₂FNO₂) Obs. mass = 420.30 (M+H)

### [Example 173]

### Synthesis of (1R,3R)-5-(2-((1R,3aS,7aR,E)-1-((R)-4-((R)-3-fluoropyrrolidin-1-yl)butan-2-yl)-7a-methyloctahydro-4H-inden-4-ylidene)ethylidene)cyclohexane-1,3-diol (Compound B015)

Compound B015 [7.9 mg, 0.019 mmol] was obtained using compound (16b) [50 mg, 0.068 mmol] and (R)-3-fluoropyrrolidine hydrochloride [30 mg, 0.239 mmol]. Exact Mass = 419.32 (C₂₆H₄₂FNO₂) Obs. mass = 420.30 (M+H)

### [Example 174]

### Synthesis of (1R,3R)-5-(2-((1R,3aS,7aR,E)-1-((R)-4-((S)-3-(difluoromethyl)pyrrolidin-1-yl)butan-2-yl)-7a-methyloctahydro-4H-inden-4-ylidene)ethylidene)cyclohexane-1,3-diol (Compound B080)

Compound B080 [12.5 mg, 0.0277 mmol] was obtained using compound (16b) [50 mg, 0.068 mmol] and (S)-3-(difluoromethyl)pyrrolidine hydrochloride [30 mg, 0.190 mmol].

¹H-NMR (CD₃OD) δ: 6.22 (1H, d, J = 11.2 Hz), 5.89 (1H, d, J = 11.2 Hz), 5.81 (1H, td, J = 57.0, 5.0 Hz), 4.07-3.96 (2H, m), 2.84 (1H, dd, J = 12.0, 4.0 Hz), 2.80 (1H, t, J = 9.0 Hz), 2.71-2.39 (8H, m), 2.24-2.14 (2H, m), 2.07-1.92 (4H, m), 1.88-1.27 (15H, m), 0.99 (3H, d, J = 6.3 Hz), 0.59 (3H, s).

Exact Mass = 451.33 (C₂₇H₄₃F₂NO₂) Obs. mass = 452.30 (M+H)

### [Example 175]

### Synthesis of (1R,3R)-5-(2-((1R,3aS,7aR,E)-1-((R)-4-((R)-3-(difluoromethyl)pyrrolidin-1-yl)butan-2-yl)-7a-methyloctahydro-4H-inden-4-ylidene)ethylidene)cyclohexane-1,3-diol (Compound B081)

Compound B081 [12.4 mg, 0.0275 mmol] was obtained using compound (16b) [50 mg, 0.068 mmol] and (R)-3-(difluoromethyl)pyrrolidine hydrochloride [30 mg, 0.190 mmol].

¹H-NMR (CD₃OD) δ: 6.22 (1H, d, J = 11.2 Hz), 5.89 (1H, d, J = 11.2 Hz), 5.80 (1H, td, J = 56.5, 5.0 Hz), 4.07-3.96 (2H, m), 2.84 (1H, dd, J = 12.0, 3.7 Hz), 2.77-2.47 (8H, m), 2.41 (1H, dd, J = 13.4, 3.2 Hz), 2.24-2.14 (2H, m), 2.07-1.95 (4H, m), 1.92-1.27 (15H, m), 0.99 (3H, d, J = 6.3 Hz), 0.59 (3H, s).

Exact Mass = 451.33 (C₂₇H₄₃F₂NO₂) Obs. mass = 452.30 (M+H)

### [Example 176]

### Synthesis of (1R,3R)-5-(2-((1R,3aS,7aR,E)-1-((R)-4-((R)-3-(2,2-difluoroethyl)pyrrolidin-1-yl)butan-2-yl)-7a-methyloctahydro-4H-inden-4-ylidene)ethylidene)cyclohexane-1,3-diol (Compound B082)

Compound B082 [8.4 mg, 0.018 mmol] was obtained using compound (16b) [50 mg, 0.068 mmol] and (R)-3-(2,2-difluoroethyl)pyrrolidine hydrochloride [30 mg, 0.175 mmol].

¹H-NMR (CD₃OD) δ: 6.22 (1H, d, J = 11.2 Hz), 5.97 (1H, tt, J = 56.0, 4.0 Hz), 5.90 (1H, d, J = 11.2 Hz), 4.07-3.95 (2H, m), 3.35 (1H, dd, J = 10.5, 8.1 Hz), 3.18-2.83 (5H, m), 2.70 (1H, t, J = 9.8 Hz), 2.60 (1H, dd, J = 13.4, 3.7 Hz), 2.56-2.48 (1H, m), 2.41 (1H, dd, J = 13.2, 3.4 Hz), 2.27-1.95 (9H, m), 1.88-1.29 (16H, m), 1.01 (3H, d, J = 6.3 Hz), 0.60 (3H, s).

Exact Mass = 465.34 (C₂₈H₄₅F₂NO₂) Obs. mass = 466.35 (M+H)

### [Example 177]

### Synthesis of (1R,3R)-5-(2-((1R,3aS,7aR,E)-1-((R)-4-((S)-3-(1,1-difluoroethyl)pyrrolidin-1-yl)butan-2-yl)-7a-methyloctahydro-4H-inden-4-ylidene)ethylidene)cyclohexane-1,3-diol (Compound B083)

Compound B083 [9.3 mg, 0.020 mmol] was obtained using compound (16b) [50 mg, 0.068 mmol] and (S)-3-(1,1-difluoroethyl)pyrrolidine hydrochloride [30 mg, 0.175 mmol].

¹H-NMR (CD₃OD) δ: 6.22 (1H, d, J = 11.2 Hz), 5.89 (1H, d, J = 11.2 Hz), 4.07-3.96 (2H, m), 2.90 (1H, t, J = 9.3 Hz), 2.86-2.39 (9H, m), 2.24-2.14 (2H, m), 2.07-1.46 (15H, m), 1.59 (3H, t, J = 19.0 Hz), 1.39-1.28 (4H, m), 0.99 (3H, d, J = 6.8 Hz), 0.59 (3H, s). Exact Mass = 465.34 (C₂₈H₄₅F₂NO₂) Obs. mass = 466.35 (M+H)

### [Example 178]

### Synthesis of (1R,3R)-5-(2-((1R,3aS,7aR,E)-1-((R)-4-((S)-3-(difluoromethoxy)pyrrolidin-1-yl)butan-2-yl)-7a-methyloctahydro-4H-inden-4-ylidene)ethylidene)cyclohexane-1,3-diol (Compound B086)

Compound B086 [10.0 mg, 0.0214 mmol] was obtained using compound (16b) [50 mg, 0.068 mmol] and (S)-3-(difluoromethoxy)pyrrolidine hydrochloride [25 mg, 0.182 mmol].

¹H-NMR (CD₃OD) δ: 5.96 (1H, t, J = 75.0 Hz), 5.79 (1H, d, J = 11.4 Hz), 5.47 (1H, d, J = 11.4 Hz), 4.35-4.30 (1H, m), 3.65-3.53 (2H, m), 2.43-2.30 (4H, m), 2.19-1.97 (5H, m), 1.86-1.70 (3H, m), 1.64-0.85 (18H, m), 0.56 (3H, d, J = 6.4 Hz), 0.16 (3H, s).

Exact Mass = 467.32 (C₂₇H₄₃F₂NO₃) Obs. mass = 468.30 (M+H)

### [Example 179]

### Synthesis of (1R,3R)-5-(2-((1R,3aS,7aR,E)-1-((R)-4-((R)-3-(difluoromethoxy)pyrrolidin-1-yl)butan-2-yl)-7a-methyloctahydro-4H-inden-4-ylidene)ethylidene)cyclohexane-1,3-diol (Compound B087)

Compound B087 [6.5 mg, 0.014 mmol] was obtained using compound (16b) [50 mg, 0.068 mmol] and (R)-3-(difluoromethoxy)pyrrolidine hydrochloride [26.5 mg, 0.153 mmol].

¹H-NMR (CD₃OD) δ: 6.38 (1H, t, J = 75.0 Hz), 6.21 (1H, d, J = 11.4 Hz), 5.89 (1H, d, J = 11.4 Hz), 4.79-4.72 (1H, m), 4.06-3.95 (2H, m), 2.85-2.71 (4H, m), 2.59 (1H, dd, J = 13.3, 3.7 Hz), 2.48 (3H, dt, J = 14.2, 6.5 Hz), 2.41 (1H, dd, J = 13.5, 3.4 Hz), 2.27-2.13 (3H, m), 2.06-1.26 (18H, m), 0.98 (3H, d, J = 6.4 Hz), 0.58 (3H, s).

Exact Mass = 467.32 (C₂₇H₄₃F₂NO₃) Obs. mass = 468.30 (M+H)

### [Example 180]

### Synthesis of (1R,3R)-5-(2-((1R,3aS,7aR,E)-1-((R)-4-((S)-3-(difluoromethyl)pyrrolidin-1-yl)butan-2-yl)-7a-methyloctahydro-4H-inden-4-ylidene)ethylidene)-2-methylenecyclohexane-1,3-diol (Compound B088)

Compound B088 [12.1 mg, 0.0261 mmol] was obtained using compound (16c) [50 mg, 0.067 mmol] and (S)-3-(difluoromethoxy)pyrrolidine hydrochloride [30 mg, 0.190 mmol].

¹H-NMR (CD₃OD) δ: 6.27 (1H, d, J = 11.2 Hz), 5.93 (1H, dt, J = 6.0, 57.0 Hz), 5.92 (1H, d, J = 11.2 Hz), 5.06 (2H, d, J = 8.0 Hz), 4.40 (2H, ddd, J = 13.9, 7.1, 4.4 Hz), 3.23 (1H, dd, J = 11.2, 8.8 Hz), 3.10-2.80 (8H, m), 2.68 (1H, dd, J = 13.2, 4.4 Hz), 2.49 (1H, dd, J = 13.7, 3.9 Hz), 2.32-2.26 (2H, m), 2.20-1.91 (7H, m), 1.82-1.33 (12H, m), 1.01 (3H, d, J = 6.8 Hz), 0.60 (3H, s).

Exact Mass = 463.33 (C₂₈H₄₃F₂NO₂) Obs. mass = 464.40 (M+H)

### [Example 181]

### Synthesis of (1R,3R)-5-(2-((1R,3aS,7aR,E)-1-((R)-4-((R)-3-(difluoromethyl)pyrrolidin-1-yl)butan-2-yl)-7a-methyloctahydro-4H-inden-4-ylidene)ethylidene)-2-methylenecyclohexane-1,3-diol (Compound B089)

Compound B089 [10.0 mg, 0.0216 mmol] was obtained using compound (16c) [50 mg, 0.067 mmol] and (R)-3-(difluoromethoxy)pyrrolidine hydrochloride [33 mg, 0.209 mmol].

¹H-NMR (CD₃OD) δ: 6.27 (1H, d, J = 11.2 Hz), 5.91 (1H, d, J = 11.2 Hz), 5.90 (1H, td, J = 56.0, 5.0 Hz), 5.05 (2H, d, J = 7.0 Hz), 4.43-4.36 (2H, m), 3.12 (1H, dd, J = 11.0, 9.0 Hz), 3.04-2.71 (8H, m), 2.67 (1H, dd, J = 13.2, 4.4 Hz), 2.49 (1H, dd, J = 13.7, 3.9 Hz), 2.32-1.95 (7H, m), 1.92-1.32 (13H, m), 1.01 (3H, d, J = 6.3 Hz), 0.60 (3H, s).

Exact Mass = 463.33 (C₂₈H₄₃F₂NO₂) Obs. mass = 464.45 (M+H)

### [Example 182]

### Synthesis of (1R,3R)-5-(2-((1R,3aS,7aR,E)-1-((R)-4-((R)-3-(2,2-difluoroethyl)pyrrolidin-1-yl)butan-2-yl)-7a-methyloctahydro-4H-inden-4-ylidene)ethylidene)-2-methylenecyclohexane-1,3-diol (Compound B090)

Compound B090 [8.2 mg, 0.017 mmol] was obtained using compound (16c) [50 mg, 0.067 mmol] and (R)-3-(2,2-difluoroethyl)pyrrolidine hydrochloride [38 mg, 0.174 mmol].

¹H-NMR (CD₃OD) δ: 6.27 (1H, d, J = 10.7 Hz), 5.97 (1H, tt, J = 56.0, 4.0 Hz), 5.92 (1H, d, J = 10.7 Hz), 5.06 (2H, d, J = 6.3 Hz), 4.43-4.37 (2H, m), 3.41 (1H, dd, J = 10.7, 7.8 Hz), 3.24-3.12 (2H, m), 3.07 (1H, td, J = 12.0, 4.7 Hz), 2.96 (1H, td, J = 11.8, 4.7 Hz), 2.86 (1H, dd, J = 12.0, 3.7 Hz), 2.78 (1H, t, J = 10.2 Hz), 2.68 (1H, dd, J = 13.4, 4.1 Hz), 2.59-2.45 (2H, m), 2.33-2.20 (3H, m), 2.09-1.98 (5H, m), 1.81-1.33 (13H, m), 1.02 (3H, d, J = 6.3 Hz), 0.60 (3H, s).

Exact Mass = 477.34 (C₂₉H₄₅F₂NO₂) Obs. mass = 478.25 (M+H)

### [Example 183]

### Synthesis of (1R,3R)-5-(2-((1R,3aS,7aR,E)-1-((R)-4-((S)-3-(1,1-difluoroethyl)pyrrolidin-1-yl)butan-2-yl)-7a-methyloctahydro-4H-inden-4-ylidene)ethylidene)-2-methylenecyclohexane-1,3-diol (Compound B091)

Compound B091 [12,7 mg, 0.0166 mmol] was obtained using compound (16c) [50 mg, 0.067 mmol] and (S)-3-(1,1-difluoroethyl)pyrrolidine hydrochloride [35 mg, 0.161 mmol].

¹H-NMR (CD₃OD) δ: 6.27 (1H, d, J = 11.2 Hz), 5.92 (1H, d, J = 11.2 Hz), 5.06 (2H, d, J = 6.3 Hz), 4.40 (2H, ddd, J = 14.0, 7.0, 4.5 Hz), 3.37-3.28 (1H, m), 3.21-2.84 (7H, m), 2.68 (1H, dd, J = 13.2, 4.4 Hz), 2.49 (1H, dd, J = 13.7, 3.9 Hz), 2.32-2.08 (4H, m), 2.02-1.95 (2H, m), 1.87-1.33 (15H, m), 1.02 (3H, d, J = 6.3 Hz), 0.60 (3H, s).

Exact Mass = 477.34 (C₂₉H₄₅F₂NO₂) Obs. mass = 478.25 (M+H)

### [Example 184]

### Synthesis of (1R,3R)-5-(2-((1R,3aS,7aR,E)-1-((R)-4-((S)-3-(2,2-difluoroethyl)pyrrolidin-1-yl)butan-2-yl)-7a-methyloctahydro-4H-inden-4-ylidene)ethylidene)-2-methylenecyclohexane-1,3-diol (Compound B092)

Compound B092 [14.0 mg, 0.0293 mmol] was obtained using compound (16c) [50 mg, 0.067 mmol] and (S)-3-(2,2-difluoroethyl)pyrrolidine hydrochloride [40 mg, 0.233 mmol].

¹H-NMR (CD₃OD) δ: 6.28 (1H, d, J = 11.2 Hz), 5.99 (1H, tt, J = 56.0, 3.0 Hz), 5.93 (1H, d, J = 11.2 Hz), 5.06 (2H, d, J = 6.3 Hz), 4.40 (2H, ddd, J 14.0, 7.0, 4.5 Hz), 3.49 (1H, dd, J = 11.0, 7.6 Hz), 3.31-3.23 (2H, m), 3.17-3.03 (2H, m), 2.95-2.80 (2H, tm), 2.68 (1H, dd, J = 13.2, 4.4 Hz), 2.62-2.54 (1H, m), 2.50 (1H, dd, J = 13.7 3.9 Hz), 2.33-1.99 (8H, m), 1.85-1.34 (12H, m), 1.02 (3H, d, J = 6.3 Hz), 0.61 (3H, s).

Exact Mass = 477.34 (C₂₉H₄₅F₂NO₂) Obs. mass = 478.40 (M+H)

### [Example 185]

### Synthesis of (1R,3R)-5-(2-((1R,3aS,7aR,E)-1-((R)-4-((R)-3-(1,1-difluoroethyl)pyrrolidin-1-yl)butan-2-yl)-7a-methyloctahydro-4H-inden-4-ylidene)ethylidene)-2-methylenecyclohexane-1,3-diol (Compound B093)

Compound B093 [10.7 mg, 0.0224 mmol] was obtained using compound (16c) [50 mg, 0.067 mmol] and (R)-3-(1,1-difluoroethyl)pyrrolidine hydrochloride [38 mg, 0.221 mmol].

¹H-NMR (CD₃OD) δ: 6.27 (1H, d, J = 11.2 Hz), 5.92 (1H, d, J = 11.2 Hz), 5.06 (2H, d, J = 6.3 Hz), 4.46-4.35 (2H, m), 3.22-2.85 (8H, m), 2.68 (1H, dd, J = 13.4, 4.1 Hz), 2.49 (1H, dd, J = 13.4, 4.1 Hz), 2.33-1.97 (8H, m), 1.90-1.33 (16H, m), 1.02 (3H, d, J = 6.3 Hz), 0.60 (3H, s).

Exact Mass = 477.34 (C₂₉H₄₅F₂NO₂) Obs. mass = 478.40 (M+H)

### [Example 186]

### Synthesis of (1R,3R)-5-(2-((1R,3aS,7aR,E)-1-((R)-4-((R)-3-(3,3-difluoropropyl)pyrrolidin-1-yl)butan-2-yl)-7a-methyloctahydro-4H-inden-4-ylidene)ethylidene)-2-methylenecyclohexane-1,3-diol (Compound B094)

Compound B094 [13.1 mg, 0.0266 mmol] was obtained using compound (16c) [50 mg, 0.067 mmol] and (R)-3-(3,3-difluoropropyl)pyrrolidine hydrochloride [35 mg, 0.189 mmol].

¹H-NMR (CD₃OD) δ: 6.28 (1H, d, J = 11.2 Hz), 5.93 (1H, d, J = 11.2 Hz), 5.91 (1H, tt, J = 57.5, 4.0 Hz), 5.06 (2H, d, J = 5.9 Hz), 4.40 (2H, ddd, J = 14.0, 7.1, 4.4 Hz), 3.47 (1H, dd, J = 11.2, 7.8 Hz), 3.31-3.25 (2H, m), 3.19-2.84 (4H, tm), 2.68 (1H, dd, J = 13.4, 4.1 Hz), 2.50 (1H, dd, J = 13.7 3.9 Hz), 2.42-1.94 (8H, m), 1.90-1.30 (17H, m), 1.02 (3H, d, J = 6.3 Hz), 0.61 (3H, s).

Exact Mass = 491.36 (C₃₀H₄₇F₂NO₂) Obs. mass = 492.40 (M+H)

### [Example 187]

### Synthesis of (1R,3S,Z)-5-(2-((1R,3aS,7aR,E)-7a-methyl-1-((R)-4-morpholinobutan-2-yl)octahydro-4H-inden-4-ylidene)ethylidene)-4-methylenecyclohexane-1,3 -diol (Compound D022)

Compound 16a [100 mg, 0.134 mmol] was dissolved in THF [1 mL], morpholine [0.2 mL, 2 mmol] was added to the solution, and the mixture was stirred at 60°C overnight. The reaction mixture was once cooled to 50°C, TBAF [1 M in THF, 0.4 mL, 0.4 mmol] was added to the mixture, and the mixture was refluxed for 4 hours. The reaction mixture was cooled to room temperature, then saturated sodium hydrogen carbonate was added thereto, the mixture was quenched, and extracted with ethyl acetate. The organic layer was dried over anhydrous magnesium sulfate and concentrated under reduced pressure. The residue was purified by HPLC in the same manner as in Example 1 to obtain compound D022 [17.6 mg, 0.041 mmol].

¹H-NMR (CD₃OD) δ: 6.31 (1H, d, J = 11.2 Hz), 6.08 (1H, d, J = 11.2 Hz), 5.28 (1H, t, J = 1.2 Hz), 4.34 (1H, t, J = 5.9 Hz), 4.1-4.08 (1H, m), 3.75 (4H, t, J = 4.6 Hz), 2.86 (1H, dd, J = 12.0, 3.7 Hz), 2.76-2.66 (6H, m), 2.60 (1H, td, J = 11.7, 4.9 Hz), 2.51 (1H, dd, J = 13.7, 3.4 Hz), 2.25 (1H, dd, J = 13.2, 6.8 Hz), 2.11-2.00 (3H, m), 1.90-1.28 (17H, m), 1.04-0.99 (1H, m), 0.99 (3H, d, J = 6.8 Hz), 0.58 (3H, s).

Exact Mass = 429.32 (C₂₇H₄₃NO₃) Obs. mass = 430.30 (M+H)

### [Example 188]

### Synthesis of (1R,3R)-5-(2-((1R,3aS,7aR,E)-7a-methyl-1-((R)-4-morpholinobutan-2-yl)octahydro-4H-inden-4-ylidene)ethylidene)cyclohexane-1,3-diol (compound D023)

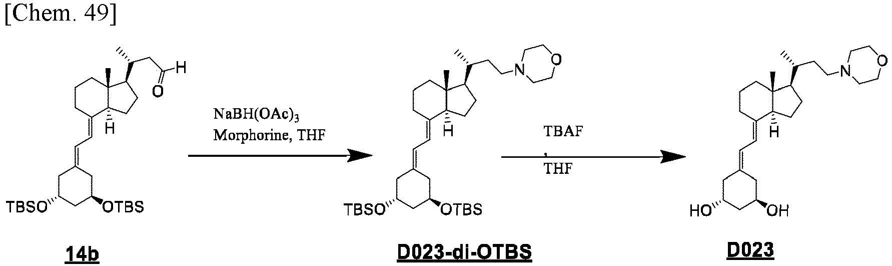

### Step 1

Morpholine [0.2 mL] and sodium triacetoxyborohydride [110 mg, 0.519 mmol] were added to a solution of compound 14b [100 mg, 0.174 mmol] described in step 2 of Reference example 14 in THF [2 mL] and the mixture was heated and stirred at 60°C overnight. The reaction system was cooled, then transferred to saturated brine, and the mixture was extracted with ethyl acetate. The organic layer was washed with saturated brine, dried over anhydrous magnesium sulfate, and concentrated under reduced pressure to obtain crude product containing D023-di-OTBS [81.1 mg]. The crude product was used in the next reaction without further purification.

### Step 2

TBAF [1 M in THF, 0.4 mL, 0.4 mmol] was added to a solution of the above crude product containing D023-di-OTBS [81.1 mg] in THF [1 mL], and the mixture was heated and stirred at 60°C for 5 hours. The reaction mixture was cooled, then poured into saturated sodium hydrogen carbonate, and the mixture was extracted with ethyl acetate. The organic layer was washed with saturated brine, dried over anhydrous magnesium sulfate, and concentrated under reduced pressure. The residue was purified by reverse phase HPLC to obtain compound D023 [22.1 mg, 0.053 mmol].

¹H-NMR (CD₃OD) δ: 6.21 (1H, d, J = 11.0 Hz), 5.88 (1H, d, J = 11.4 Hz), 4.06-3.95 (2H, m), 3.69 (4H, t, J = 4.6 Hz), 2.83 (1H, dd, J = 11.9, 4.1 Hz), 2.59 (1H, dd, J = 13.7, 3.7 Hz), 2.50-2.37 (6H, m), 2.33 (1H, td, J= 11.4, 5.3 Hz), 2.23-2.13 (2H, m), 2.06-1.93 (4H, m), 1.88-1.47 (9H, m), 1.39-1.27 (4H, m), 0.98 (3H, d, J = 6.4 Hz), 0.58 (3H, s).

Exact Mass = 417.32 (C₂₆H₄₃NO₃) Obs. mass = 418.35 (M+H)

### [Example 189]

### Synthesis of (1R,3R)-5-(2-((1R,3aS,7aR,E)-7a-methyl-1-((R)-4-morpholinobutan-2-yl)octahydro-4H-inden-4-ylidene)ethylidene)-2-methylenecyclohexane-1,3 -diol (compound D024)

Compound D024 [22.2 mg, 0.052 mmol] was obtained by performing the reaction in the same manner as in Example 1 using compound (16c) [120 mg, 0.097 mmol] and morpholine [0.1 mL, 1.1 mmol].

¹H-NMR (CD₃OD) δ: 6.26 (1H, d, J = 11.2 Hz), 5.91 (1H, d, J = 11.2 Hz), 5.05 (2H, dd, J = 8.0, 1.0 Hz), 4.42-4.36 (2H, m), 3.71 (4H, t, J = 4.6 Hz), 2.85 (1H, dd, J = 12.0, 3.7 Hz), 2.67 (1H, dd, J= 13.4, 4.1 Hz), 2.58-2.37 (7H, m), 2.31-2.24 (2H, m), 2.07-1.93 (3H, m), 1.49 (11H, ttt, J = 61.2, 17.2, 7.4 Hz), 0.99 (3H, d, J = 6.3 Hz), 0.58 (3H, s).

Exact Mass = 429.32 (C₂₇H₄₃NO₃) Obs. mass = 430.35 (M+H)

### [Example 190]

### Synthesis of (1R,3R)-5-(2-((1R,3aS,7aR,E)-7a-methyl-1-((R)-4-((S)-2-methylmorpholino)butan-2-yl)octahydro-4H-inden-4-ylidene)ethylidene)-2-methylenecyclohexane-1,3-diol (Compound D025)

Compound D025 [13.8 mg, 0.031 mmol] was obtained by processing in the same manner as in Example 1 using compound (16c) [50 mg, 0.067 mmol] and (S)-2-methylmorpholine [30 mg, 0.297 mmol].

¹H-NMR (CD₃OD) δ: 6.26 (1H, d, J = 11.2 Hz), 5.91 (1H, d, J = 11.2 Hz), 5.05 (2H, d, J = 8.0 Hz), 4.45-4.33 (2H, m), 3.82 (1H, dd, J = 10.2, 3.0 Hz), 3.66-3.55 (2H, m), 2.85 (1H, dd, J = 12.0, 3.7 Hz), 2.82-2.75 (2H, m), 2.67 (1H, dd, J = 13.7, 4.4 Hz), 2.48 (1H, dd, J = 13.7, 3.9 Hz), 2.44-2.24 (4H, m), 2.08-1.93 (4H, m), 1.79 (1H, dd, J = 11.7, 10.2 Hz), 1.71-1.26 (11H, m), 1.11 (3H, d, J = 6.3 Hz), 0.98 (3H, d, J = 6.8 Hz), 0.58 (3H, s). Exact Mass = 443.34 (C₂₈H₄₅NO₃) Obs. mass = 444.30 (M+H)

### [Example 191]

### Synthesis of (1R,3S)-5-(2-((1R,3aS,7aR,E)-1-((R)-4-(4-oxa-7-azaspiro[2.5]octan-7-yl)butan-2-yl)-7a-methyloctahydro-4H-inden-4-ylidene)ethylidene)-2-methylenecyclohexane-1,3-diol (Compound D026)

Compound D026 [12.0 mg, 0.026 mmol] was obtained by processing in the same manner as in Example 1 using compound (16c) [50 mg, 0.067 mmol] and 4-oxa-7-azaspiro[2.5]octane [30 mg, 0.265 mmol].

¹H-NMR (CD₃OD) δ: 6.26 (1H, d, J = 10.7 Hz), 5.90 (1H, d, J = 11.2 Hz), 5.05 (2H, d, J = 6.8 Hz), 4.42-4.36 (2H, m), 3.75 (2H, t, J = 4.9 Hz), 2.85 (1H, dd, J = 12.0, 3.7 Hz), 2.67 (1H, dd, J = 12.0, 4.0 Hz), 2.64-2.24 (9H, m), 2.07-1.96 (3H, m), 1.70-1.27 (11H, m), 0.98 (3H, d, J = 6.8 Hz), 0.77-0.70 (2H, m), 0.59-0.53 (2H, m), 0.58 (3H, s).

Exact Mass = 455.34 (C₂₉H₄₅NO₃) Obs. mass = 456.30 (M+H)

### [Example 192]

### Synthesis of (1R,3S)-5-(2-((1R,3aS,7aR,E)-1-((R)-4-(6-oxa-9-azaspiro[4.5]decan-9-yl)butan-2-yl)-7a-methyloctahydro-4H-inden-4-ylidene)ethylidene)-2-methylenecyclohexane-1,3-diol (Compound D027)

Compound D027 [18.0 mg, 0.037 mmol] was obtained by processing in the same manner as in Example 1 using compound (16c) [50 mg, 0.067 mmol] and 6-oxa-9-azaspiro[4.5]decane [30 mg, 0.212 mmol].

¹H-NMR (CD₃OD) δ: 6.27 (1H, d, J = 10.7 Hz), 5.92 (1H, d, J = 10.7 Hz), 5.06 (2H, t, J = 3.9 Hz), 4.43-4.37 (2H, m), 3.73-3.63 (2H, m), 2.86 (1H, dd, J = 12.0, 3.7 Hz), 2.67 (1H, dd, J = 13.4, 4.1 Hz), 2.49 (1H, dd, J = 13.4, 3.7 Hz), 2.40-2.25 (8H, m), 2.07-1.94 (3H, m), 1.81-1.18 (20H, m), 0.99 (3H, d, J = 6.3 Hz), 0.59 (3H, s).

Exact Mass = 483.37 (C₃₁H₄₉NO₃) Obs. mass = 484.35 (M+H)

### [Example 193]

### Synthesis of (1R,3R)-5-(2-((1R,3aS,7aR,E)-1-((R)-4-((2S,6R)-2,6-dimethylmorpholino)butan-2-yl)-7a-methyloctahydro-4H-inden-4-ylidene)ethylidene)-2-methylenecyclohexane-1,3-diol (Compound D028)

Compound D028 [17.3 mg, 0.0378 mmol] was obtained by processing in the same manner as in Example 1 using compound (16c) [50 mg, 0.067 mmol] and (2S,6R)-2,6-dimethylmorpholine [30 mg, 0.260 mmol].

¹H-NMR (CD₃OD) δ: 6.26 (1H, d, J = 11.2 Hz), 5.90 (1H, d, J = 11.2 Hz), 5.04 (2H, d, J = 6.8 Hz), 4.42-4.36 (2H, m), 3.69-3.63 (2H, m), 2.82 (3H, q, J = 12.0 Hz), 2.66 (1H, dd, J = 13.2, 3.9 Hz), 2.48 (1H, dd, J = 13.4, 3.7 Hz), 2.43-2.24 (4H, m), 2.06-1.96 (3H, m), 1.74-1.26 (15H, m), 1.12 (3H, d, J = 6.1 Hz), 1.12 (3H, d, J = 6.0 Hz), 0.98 (3H, d, J = 6.3 Hz), 0.58 (3H, s).

Exact Mass = 457.36 (C₂₉H₄₇NO₃₃) Obs. mass = 458.35 (M+H)

### [Example 194]

### Synthesis of (1R,3R)-5-(2-((1R,3aS,7aR,E)-1-((R)-4-(2,2-dimethylmorpholino)butan-2-yl)-7a-methyloctahydro-4H-inden-4-ylidene)ethylidene)-2-methylenecyclohexane-1,3-diol (Compound D029)

Compound D029 [13.6 mg, 0.030 mmol] was obtained by processing in the same manner as in Example 1 using compound (16c) [50 mg, 0.067 mmol] and 2,2-dimethylmorpholine [30 mg, 0.260 mmol].

¹H-NMR (CD₃OD) δ: 6.26 (1H, d, J = 11.2 Hz), 5.90 (1H, d, J = 11.2 Hz), 5.05 (2H, d, J = 6.8 Hz), 4.39 (2H, dt, J = 15.0, 4.8 Hz), 3.70 (2H, ddd, J = 21.5, 12.0, 4.4 Hz), 2.85 (1H, dd, J = 12.2, 3.4 Hz), 2.66 (1H, dd, J = 13.2, 4.4 Hz), 2.48 (1H, dd, J = 13.2, 3.9 Hz), 2.39-2.24 (7H, m), 2.16 (1H, d, J = 10.7 Hz), 2.08-1.92 (3H, m), 1.70-1.48 (8H, m), 1.38-1.31 (3H, m), 1.25-1.13 (8H, m), 0.98 (3H, d, J = 6.3 Hz), 0.58 (3H, s).

Exact Mass = 457.36 (C₂₉H₄₇NO₃) Obs. mass = 458.35 (M+H)

### [Example 195]

### Synthesis of (1R,3R)-5-(2-((1R,3aS,7aR,E)-1-((R)-4-(4-oxa-7-azaspiro[2.5]octan-7-yl)butan-2-yl)-7a-methyloctahydro-4H-inden-4-ylidene)ethylidene)cyclohexane-1,3-diol (Compound D030)

Compound D030 [11.2 mg, 0.0252 mmol] was obtained by processing in the same manner as in Example 1 using compound (16b) [50 mg, 0.068 mmol] and 4-oxa-7-azaspiro[2.5]octane [30 mg, 0.265 mmol].

¹H-NMR (CD₃OD) δ: 6.21 (1H, d, J = 11.2 Hz), 5.88 (1H, d, J = 11.2 Hz), 4.06-3.95 (2H, m), 3.75 (2H, t, J = 4.9 Hz), 2.83 (1H, dd, J = 12.2, 3.4 Hz), 2.61-2.31 (9H, m), 2.23-2.13 (2H, m), 2.05-1.28 (18H, m), 0.98 (3H, d, J = 6.8 Hz), 0.74 (2H, t, J = 5.6 Hz), 0.62-0.55 (2H, m), 0.57 (3H, s).

Exact Mass = 443.34 (C₂₈H₄₅NO₃) Obs. mass = 444.35 (M+H)

### [Example 196]

### Synthesis of (1R,3R)-5-(2-((1R,3aS,7aR,E)-7a-methyl-1-((R)-4-((R)-3-methylmorpholino)butan-2-yl)octahydro-4H-inden-4-ylidene)ethylidene)-2-methylenecyclohexane-1,3-diol (Compound D031)

Compound D031 [14.9 mg, 0.0336 mmol] was obtained by processing in the same manner as in Example 1 using compound (16c) [50 mg, 0.067 mmol] and (R)-3-methylmorpholine [30 mg, 0.297 mmol].

¹H-NMR (CD₃OD) δ: 6.26 (1H, d, J = 11.2 Hz), 5.91 (1H, d, J = 10.7 Hz), 5.05 (2H, d, J = 8.0 Hz), 4.42-4.36 (2H, m), 3.77 (1H, dt, J = 11.4, 2.8 Hz), 3.68-3.58 (2H, m), 3.23 (1H, dd, J = 11.2, 9.3 Hz), 2.88-2.81 (2H, m), 2.77 (1H, dt, J = 11.9, 2.7 Hz), 2.67 (1H, dd, J = 13.2, 4.4 Hz), 2.50-2.21 (6H, m), 2.08-1.93 (3H, m), 1.74-1.14 (13H, m), 1.00 (3H, d, J = 6.3 Hz), 0.99 (3H, d, J = 6.0 Hz), 0.58 (3H, s).

Exact Mass = 443.34 (C₂₈H₄₅NO₃) Obs. mass = 444.30 (M+H)

### [Example 197]

### Synthesis of (1R,3R)-5-(2-((1R,3aS,7aR,E)-7a-methyl-1-((R)-4-((S)-3-methylmorpholino)butan-2-yl)octahydro-4H-inden-4-ylidene)ethylidene)-2-methylenecyclohexane-1,3-diol (Compound D032)

Compound D032 [12.3 mg, 0.0277 mmol] was obtained by processing in the same manner as in Example 1 using compound (16c) [50 mg, 0.067 mmol] and (S)-3-methylmorpholine [30 mg, 0.297 mmol].

¹H-NMR (CD₃OD) δ: 6.26 (1H, d, J = 11.2 Hz), 5.91 (1H, d, J = 11.2 Hz), 5.05 (2H, d, J = 7.3 Hz), 4.43-4.33 (2H, m), 3.83-3.76 (1H, m), 3.62 (2H, ddd, J = 23.5, 13.5, 5.0 Hz), 3.23 (1H, dd, J = 11.2, 9.3 Hz), 2.87-2.64 (4H, m), 2.50-2.25 (6H, m), 2.07-1.91 (3H, m), 1.70-1.27 (11H, m), 1.00 (3H, d, J = 6.3 Hz), 0.98 (3H, d, J = 8.1 Hz), 0.59 (3H, s).

Exact Mass = 443.34 (C₂₈H₄₅NO₃) Obs. mass = 444.35 (M+H)

### [Example 198]

### Synthesis of (1R,3R)-5-(2-((1R,3aS,7aR,E)-1-((R)-4-((S)-2-(difluoromethyl)morpholino)butan-2-yl)-7a-methyloctahydro-4H-inden-4-ylidene)ethylidene)-2-methylenecyclohexane-1,3-diol (Compound D033)

Compound D033 [11.4 mg, 0.0238 mmol] was obtained by processing in the same manner as in Example 1 using compound (16c) [50 mg, 0.067 mmol] and (S)-2-(difluoromethyl)morpholine hydrochloride (Compound 3d06) [40 mg, 0.230 mmol] described in step 3 of Example 137.

¹H-NMR (CD₃OD) δ: 6.26 (1H, d, J = 10.7 Hz), 5.91 (1H, d, J = 10.7 Hz), 5.77 (1H, td, J = 55.0, 4.0 Hz), 5.05 (2H, d, J = 6.8 Hz), 4.43-4.33 (2H, m), 3.91 (1H, d, J = 11.7 Hz), 3.75-3.62 (2H, m), 2.92-2.80 (2H, m), 2.75 (1H, dd, J = 12.0, 3.2 Hz), 2.67 (1H, dd, J = 13.2, 4.4 Hz), 2.50-2.15 (7H, m), 2.07-1.91 (4H, m), 1.68-1.21 (12H, m), 0.99 (3H, d, J = 6.8 Hz), 0.58 (3H, s).

Exact Mass = 479.32 (C₂₈H₄₃F₂NO₃) Obs. mass = 480.30 (M+H)

### [Example 199]

### Synthesis of (1R,3R)-5-(2-((1R,3aS,7aR,E)-1-((R)-4-((S)-2-(difluoromethyl)morpholino)butan-2-yl)-7a-methyloctahydro-4H-inden-4-ylidene)ethylidene)cyclohexane-1,3-diol (Compound D034)

Compound D034 [7.5 mg, 0.016 mmol] was obtained by processing in the same manner as in Example 1 using compound (16b) [50 mg, 0.068 mmol] and (S)-2-(difluoromethyl)morpholine hydrochloride (Compound 3d06) [40 mg, 0.230 mmol] described in step 3 of Example 137.

¹H-NMR (CD₃OD) δ: 6.21 (1H, d, J = 11.2 Hz), 5.89 (1H, d, J = 11.2 Hz), 5.77 (1H, td, J = 55.0, 4.0 Hz), 4.06-3.95 (2H, m), 3.90 (1H, d, J = 10.0 Hz), 3.75-3.62 (2H, m), 2.88 (1H, d, J = 10.7 Hz), 2.83 (1H, dd, J = 10.7, 4.0 Hz), 2.75 (1H, dd, J = 11.7, 2.0 Hz), 2.58 (1H, dd, J = 13.2, 3.4 Hz), 2.48-2.35 (3H, m), 2.23-1.91 (7H, m), 1.87-1.26 (14H, m), 0.98 (3H, d, J = 6.8 Hz), 0.58 (3H, s).

Exact Mass = 467.32 (C₂₇H₄₃F₂NO₃) Obs. mass = 468.35 (M+H)

### [Example 200]

### Synthesis of (1R,3S,Z)-5-(2-((1R,3aS,7aR,E)-1-((R)-4-((S)-2-(difluoromethyl)morpholino)butan-2-yl)-7a-methyloctahydro-4H-inden-4-ylidene)ethylidene)-4-methyloctahydro-1,3-diol (Compound D035)

Compound D035 [4.3 mg, 0.0090 mmol] was obtained by processing in the same manner as in Example 1 using compound (16a) [50 mg, 0.067 mmol] and (S)-2-(difluoromethyl)morpholine hydrochloride (Compound 3d06) [40 mg, 0.230 mmol] described in step 3 of Example 137.

¹H-NMR (CD₃OD) δ: 6.32 (1H, d, J = 11.2 Hz), 6.08 (1H, d, J = 11.2 Hz), 5.77 (1H, td, J = 55.3, 4.2 Hz), 5.28 (1H, dd, J = 2.4, 1.5 Hz), 4.34 (1H, t, J = 5.9 Hz), 4.14-4.09 (1H, m), 3.90 (1H, d, J = 11.7 Hz), 3.73-3.62 (2H, m), 2.87 (2H, dd, J = 10.7, 5.9 Hz), 2.74 (1H, d, J = 11.7 Hz), 2.53-2.34 (3H, m), 2.28-2.14 (2H, m), 2.03-1.87 (7H, m), 1.73-1.20 (16H, m), 0.98 (3H, d, J = 6.3 Hz), 0.57 (3H, s).

Exact Mass = 479.32 (C₂₈H₄₃F₂NO₃) Obs. mass = 480.35 (M+H)

### [Example 201]

### Synthesis of (1R,3R)-5-(2-((1R,3aS,7aR,E)-1-((R)-4-((R)-2-(difluoromethyl)morpholino)butan-2-yl)-7a-methyloctahydro-4H-inden-4-ylidene)ethylidene)-2-methylenecyclohexane-1,3-diol (Compound D036)

Compound D036 [10.3 mg, 0.0215 mmol] was obtained by processing in the same manner as in Example 1 using compound (16c) [50 mg, 0.067 mmol] and (R)-2-(difluoromethyl)morpholine hydrochloride (Compound 3d10) [40 mg, 0.230 mmol] described in step 3 of Example 138.

¹H-NMR (CD₃OD) δ: 6.26 (1H, d, J = 11.2 Hz), 5.91 (1H, d, J = 11.2 Hz), 5.77 (1H, td, J = 55.0, 4.0 Hz), 5.05 (2H, d, J = 6.8 Hz), 4.44-4.34 (2H, m), 3.91 (1H, d, J = 11.2 Hz), 3.74-3.61 (2H, m), 2.84 (2H, d, J = 11.7 Hz), 2.78 (1H, d, J = 11.7 Hz), 2.67 (1H, dd, J = 13.2, 4.4 Hz), 2.50-2.24 (5H, m), 2.18-1.96 (5H, m), 1.72-1.22 (13H, m), 0.99 (3H, d, J = 6.3 Hz), 0.59 (3H, s).

Exact Mass = 479.32 (C₂₈H₄₃F₂NO₃) Obs. mass = 480.35 (M+H)

### [Example 202]

### Synthesis of (1R,3S,Z)-5-(2-((1R,3aS,7aR,E)-1-((R)-4-((R)-2-(difluoromethyl)morpholino)butan-2-yl)-7a-methyloctahydro-4H-inden-4-ylidene)ethylidene)-4-methylenecyclohexane-1,3-diol (Compound D037)

Compound D037 [2.0 mg, 0.0042 mmol] was obtained by processing in the same manner as in Example 1 using compound (16a) [50 mg, 0.067 mmol] and (R)-2-(difluoromethyl)morpholine hydrochloride (Compound 3d10) [40 mg, 0.230 mmol] described in step 3 of Example 138.

¹H-NMR (CD₃OD) δ: 6.32 (1H, d, J = 11.2 Hz), 6.08 (1H, d, J = 11.2 Hz), 5.77 (1H, td, J = 55.4, 4.1 Hz), 5.28 (1H, dd, J = 2.4, 1.5 Hz), 4.34 (1H, t, J = 5.9 Hz), 4.15-4.08 (1H, m), 3.91 (1H, d, J = 10.2 Hz), 3.76-3.68 (1H, m), 3.63 (1H, td, J = 11.5, 2.4 Hz), 2.88-2.73 (4H, m), 2.51 (1H, dd, J = 13.4, 3.2 Hz), 2.47-1.87 (14H, m), 1.61-1.36 (18H, m), 0.98 (3H, d, J = 6.3 Hz), 0.56 (3H, s).

Exact Mass = 479.32 (C₂₈H₄₃F₂NO₃) Obs. mass = 480.30 (M+H)

### [Example 203]

### Synthesis of (1R,3R)-5-(2-((1R,3aS,7aR,E)-1-((R)-4-((2R,5S)-2,5-dimethylmorpholino)butan-2-yl)-7a-methyloctahydro-4H-inden-4-ylidene)ethylidene)-2-methylenecyclohexane-1,3-diol (Compound D038)

Compound D038 [14.2 mg, 0.031 mmol] was obtained by processing in the same manner as in Example 1 using compound (16c) [50 mg, 0.068 mmol] and (2R,5S)-2,5-dimethylmorpholine [30 mg, 0.260 mmol].

Exact Mass = 457.36 (C₂₉H₄₇NO₃) Obs. mass = 458.30 (M+H)

### [Example 204]

### Synthesis of (1R,3R)-5-(2-((1R,3aS,7aR,E)-1-((R)-4-((2R,5R)-2,5-dimethylmorpholino)butan-2-yl)-7a-methyloctahydro-4H-inden-4-ylidene)ethylidene)-2-methylenecyclohexane-1,3-diol (Compound D039)

Compound D039 [13.8 mg, 0.030 mmol] was obtained by processing in the same manner as in Example 1 using compound (16c) [50 mg, 0.068 mmol] and (2R,5R)-2,5-dimethylmorpholine [30 mg, 0.260 mmol].

Exact Mass = 457.36 (C₂₉H₄₇NO₃) Obs. mass = 458.30 (M+H)

### [Example 205]

### Synthesis of (1R,3R)-5-(2-((1R,3aS,7aR,E)-7a-methyl-1-((R)-4-((R)-2-methylmorpholino)butan-2-yl)octahydro-4H-inden-4-ylidene)ethylidene)-2-methylenecyclohexane-1,3-diol (Compound D040)

Compound D040 [43.7 mg, 0.101 mmol] was obtained by processing in the same manner as in Example 1 using compound (16c) [100 mg, 0.136 mmol], (R)-2-methylmorpholine [0.2 mL], and THF [2 mL].

¹H-NMR (CD₃OD) δ: 6.26 (1H, d, J = 11.0 Hz), 5.91 (1H, d, J = 11.4 Hz), 5.05 (2H, d, J = 6.9 Hz), 4.43-4.35 (2H, m), 3.82 (1H, dd, J = 11.7, 2.1 Hz), 3.67-3.55 (2H, m), 2.88-2.81 (2H, m), 2.76 (1H, d, J = 11.9 Hz), 2.67 (1H, dd, J = 13.3, 4.1 Hz), 2.48 (1H, dd, J = 13.5, 3.9 Hz), 2.44-2.25 (4H, m), 2.10 (1H, td, J = 11.7, 3.4 Hz), 2.04-1.93 (3H, m), 1.77-1.27 (12H, m), 1.12 (3H, d, J = 6.4 Hz), 0.98 (3H, d, J = 6.4 Hz), 0.58 (3H, s).

Exact Mass = 443.34 (C₂₈H₄₅NO₃) Obs. mass = 432.35 (M+H)

### [Example 206]

### Synthesis of (1R,3R)-5-(2-((1R,3aS,7aR,E)-7a-methyl-1-((R)-4-((S)-2-methylmorpholino)butan-2-yl)octahydro-4H-inden-4-ylidene)ethylidene)cyclohexane-1,3-diol (Compound D041)

Compound D041 [30.0 mg, 0.070 mmol] was obtained by processing in the same manner as in Example 188 using compound (14b) [100 mg, 0.174 mmol], (S)-2-methylmorpholine [0.2 mL], and THF [2 mL].

¹H-NMR (CD₃OD) δ: 6.21 (1H, d, J = 11.0 Hz), 5.88 (1H, d, J = 11.4 Hz), 4.06-3.95 (2H, m), 3.82 (1H, dd, J = 11.7, 2.1 Hz), 3.65-3.58 (2H, m), 2.85-2.76 (3H, m), 2.59 (1H, dd, J = 13.3, 3.7 Hz), 2.42-2.28 (3H, m), 2.23-2.13 (2H, m), 2.08-1.92 (4H, m), 1.87-1.25 (14H, m), 1.11 (3H, d, J = 6.4 Hz), 0.98 (3H, d, J = 6.4 Hz), 0.58 (3H, s).

Exact Mass = 431.34 (C₂₇H₄₅NO₃) Obs. mass = 432.35 (M+H)

### [Example 207]

### Synthesis of (1R,3R)-5-(2-((1R,3aS,7aR,E)-7a-methyl-1-((R)-4-((R)-3-methylmorpholino)butan-2-yl)octahydro-4H-inden-4-ylidene)ethylidene)cyclohexane-1,3-diol (Compound D042)

Compound D042 [30.0 mg, 0.070 mmol] was obtained by processing in the same manner as in Example 188 using compound (14b) [100 mg, 0.174 mmol], (R)-3-methylmorpholine [0.2 mL], and THF [2 mL].

¹H-NMR (CD₃OD) δ: 6.21 (1H, d, J = 11.0 Hz), 5.89 (1H, d, J = 11.0 Hz), 4.04-3.96 (2H, m), 3.77 (1H, d, J = 11.4 Hz), 3.68-3.58 (2H, m), 3.23 (1H, dd, J = 11.2, 9.4 Hz), 2.88-2.80 (2H, m), 2.77 (1H, dt, J = 12.2, 3.0 Hz), 2.59 (1H, dd, J = 13.5, 3.0 Hz), 2.48-2.30 (3H, m), 2.28-2.13 (3H, m), 2.08-1.95 (4H, m), 1.88-1.17 (14H, m), 1.00 (6H, d, J = 6.8 Hz), 0.99 (6H, d, J = 5.7 Hz), 0.58 (3H, s).

Exact Mass = 431.34 (C₂₇H₄₅NO₃) Obs. mass = 432.35 (M+H)

### [Example 208]

### Synthesis of (1R,3R)-5-(2-((1R,3aS,7aR,E)-7a-methyl-1-((R)-4-((S)-3-methylmorpholino)butan-2-yl)octahydro-4H-inden-4-ylidene)ethylidene)cyclohexane-1,3-diol (Compound D043)

Compound D043 [40.9 mg, 0.095 mmol] was obtained by processing in the same manner as in Example 188 using compound (14b) [100 mg, 0.174 mmol], (S)-3-methylmorpholine [0.2 mL], and THF [2 mL].

¹H-NMR (CD₃OD) δ: 6.21 (1H, d, J = 11.4 Hz), 5.89 (1H, d, J = 11.0 Hz), 4.06-3.93 (2H, m), 3.78 (1H, dt, J = 11.4, 2.7 Hz), 3.68-3.55 (2H, m), 3.22 (1H, dd, J = 11.4, 9.6 Hz), 2.88-2.70 (3H, m), 2.59 (1H, dd, J = 13.3, 3.7 Hz), 2.42-2.13 (6H, m), 2.06-1.21 (18H, m), 0.99 (3H, d, J = 5.0 Hz), 0.98 (3H, t, J = 3.2 Hz), 0.58 (3H, s).

Exact Mass = 431.34 (C₂₇H₄₅NO₃) Obs. mass = 432.35 (M+H)

### [Example 209]

### Synthesis of (1R,3R,Z)-5-(2-((1R,3aS,7aR,E)-7a-methyl-1-((R)-4-((S)-3-methylmorpholino)butan-2-yl)octahydro-4H-inden-4-ylidene)ethylidene)-4-methylenecyclohexane-1,3-diol (Compound D045)

Compound D045 [5.6 mg, 0.013 mmol] was obtained by processing in the same manner as in Example 1 using compound (16a) [50 mg, 0.067 mmol] and (S)-3-methylmorpholine [0.023 mL, 0.202 mmol].

¹H-NMR (CD₃OD) δ: 6.32 (1H, d, J = 11.0 Hz), 6.08 (1H, d, J = 11.4 Hz), 5.28 (1H, s), 4.89 (1H, d, J = 1.8 Hz), 4.35 (1H, t, J = 5.7 Hz), 4.12 (1H, q, J = 6.1 Hz), 3.78 (1H, dt, J = 11.0, 3.5 Hz), 3.65 (1H, dd, J = 11.9, 3.2 Hz), 3.63-3.57 (1H, m), 3.23 (1H, dd, J = 11.2, 9.4 Hz), 2.88-2.70 (3H, m), 2.51 (1H, dd, J = 13.3, 3.2 Hz), 2.44-2.23 (4H, m), 2.10-1.92 (3H, m), 1.88 (2H, t, J = 5.5 Hz), 1.73-1.30 (12H, m), 0.99 (3H, d, J = 6.4 Hz), 0.98 (3H, d, J = 6.4 Hz), 0.58 (3H, s).

Exact Mass = 443.34 (C₂₈H₄₅NO₃) Obs. mass = 444.35 (M+H)

### [Example 210]

### Synthesis of (1R,3R,Z)-5-(2-((1R,3aS,7aR,E)-7a-methyl-1-((R)-4-((R)-3-methylmorpholino)butan-2-yl)octahydro-4H-inden-4-ylidene)ethylidene)-4-methylenecyclohexane-1,3-diol (Compound D046)

Compound D046 [5.5 mg, 0.012 mmol] was obtained by processing in the same manner as in Example 1 using compound (16a) [50 mg, 0.067 mmol] and (R)-3-methylmorpholine [0.023 mL, 0.202 mmol].

¹H-NMR (CD₃OD) δ: 6.32 (1H, d, J = 11.4 Hz), 6.08 (1H, d, J = 11.4 Hz), 5.28 (1H, s), 4.89 (1H, d, J = 2.3 Hz), 4.35 (1H, t, J = 5.9 Hz), 4.18-4.06 (1H, m), 3.77 (1H, d, J = 11.4 Hz), 3.68-3.55 (2H, m), 3.23 (1H, dd, J= 11.4, 9.1 Hz), 2.95-2.70 (3H, m), 2.55-2.15 (5H, m), 2.05-1.83 (5H, m), 1.73-1.16 (12H, m), 1.00 (3H, d, J = 6.4 Hz), 0.99 (3H, d, J = 6.4 Hz), 0.57 (3H, s).

Exact Mass = 443.34 (C₂₈H₄₅NO₃) Obs. mass = 444.35 (M+H)

### [Example 211]

### Synthesis of (1R,3R,Z)-5-(2-((1R,3aS,7aR,E)-7a-methyl-1-((R)-4-((R)-2-methylmorpholino)butan-2-yl)octahydro-4H-inden-4-ylidene)ethylidene)-4-methylenecyclohexane-1,3-diol (Compound D047)

Compound D047 [6.7 mg, 0.015 mmol] was obtained by processing in the same manner as in Example 1 using compound (16a) [50 mg, 0.067 mmol] and (R)-2-methylmorpholine hydrochloride [27.8 mg, 0.202 mmol].

¹H-NMR (CD₃OD) δ: 6.32 (1H, **d,** J = **11.4** Hz), 6.08 (1H, **d,** J = **11.0** Hz), 5.28 (1H, s), 4.89 (1H, d, J = 1.8 Hz), 4.34 (1H, t, J = 5.9 Hz), 4.14-4.10 (1H, m), 3.82 (1H, dd, J = 11.7, 2.1 Hz), 3.68-3.55 (2H, m), 2.89-2.73 (3H, m), 2.51 (1H, dd, J= 13.3, 3.7 Hz), 2.46-2.23 (3H, m), 2.11 (1H, td, J = 11.7, 3.4 Hz), 2.04-1.92 (3H, m), 1.88 (2H, t, J = 6.0 Hz), 1.78-1.25 (12H, m), 1.11 (3H, d, J = 6.4 Hz), 0.98 (3H, d, J = 6.4 Hz), 0.57 (3H, s).

Exact Mass = 443.34 (C₂₈H₄₅NO₃) Obs. mass = 444.35 (M+H)

### [Example 212]

### Synthesis of (1R,3R,Z)-5-(2-((1R,3aS,7aR,E)-7a-methyl-1-((R)-4-((S)-2-methylmorpholino)butan-2-yl)octahydro-4H-inden-4-ylidene)ethylidene)-4-methylenecyclohexane-1,3-diol (Compound D048)

Compound D048 [6.8 mg, 0.015 mmol] was obtained by processing in the same manner as in Example 1 using compound (16a) [50 mg, 0.067 mmol] and (S)-2-methylmorpholine hydrochloride [0.023 mL, 0.202 mmol].

¹H-NMR (CD₃OD) δ: 6.32 (1H, d, J = 11.0 Hz), 6.08 (1H, d, J = 11.0 Hz), 5.28 (1H, s), 4.89 (1H, d, J = 2.3 Hz), 4.34 (1H, t, J = 5.9 Hz), 4.15-4.10 (2H, m), 3.82 (1H, dd, J = 11.7, 2.1 Hz), 3.65-3.59 (2H, m), 2.87 (1H, d, J= 11.4 Hz), 2.79 (2H, t, J = 9.4 Hz), 2.51 (1H, dd, J = 13.3, 3.7 Hz), 2.44-2.23 (3H, m), 2.09-1.92 (4H, m), 1.88 (2H, t, J = 5.5 Hz), 1.81 (1H, dd, J = 20.4, 9.8 Hz), 1.71-1.28 (12H, m), 1.11 (3H, d, J = 5.9 Hz), 0.98 (3H, d, J = 6.4 Hz), 0.57 (3H, s).

Exact Mass = 443.34 (C₂₈H₄₅NO₃) Obs. mass = 444.35 (M+H)

### [Example 213]

### Synthesis of (1R,3R)-5-(2-((1R,3aS,7aR,E)-1-((R)-4-(3,3-dimethylmorpholino)butan-2-yl)-7a-methyloctahydro-4H-inden-4-ylidene)ethylidene)cyclohexane-1,3-diol

### (Compound D049)

Compound D049 [9.3 mg, 0.021 mmol] was obtained by processing in the same manner as in Example 188 using compound (14b) [50 mg, 0.087 mmol] and 3,3-dimethylmorpholine [0.054 mL, 0.435 mmol].

¹H-NMR (CD₃OD) δ: 6.21 (1H, d, J = 11.0 Hz), 5.88 (1H, d, J = 11.4 Hz), 4.04-3.95 (2H, m), 3.73-3.65 (2H, m), 2.83 (1H, dd, J = 12.1, 3.9 Hz), 2.61-2.52 (3H, m), 2.42-2.37 (3H, m), 2.23-2.13 (2H, m), 2.05-2.02 (1H, m), 1.96-1.93 (1H, m), 1.86-1.81 (1H, m), 1.78-1.72 (1H, m), 1.66-1.50 (6H, m), 1.38-1.32 (3H, m), 1.19-1.16 (1H, m), 1.01 (6H, d, J = 4.1 Hz), 0.97 (3H, d, J = 6.4 Hz), 0.57 (3H, s).

Exact Mass = 445.36 (C₂₈H₄₇NO₃) Obs. mass = 446.30 (M+H)

### [Example 214]

### Synthesis of (1R,3S,Z)-5-(2-((1R,3aS,7aR,E)-7a-methyl-1-((R)-4-(4-(methylsulfonyl)piperazin-1-yl)butan-2-yl)octahydro-4H-inden-4-ylidene)ethylidene)-4-methylenecyclohexane-1,3-diol (Compound E002)

Compound E002 [11.2 mg, 0.022 mmol] was obtained by processing in the same manner as in Example 1 using compound (16a) [100 mg, 0.134 mmol] and 4-(methylsulfonyl)piperazine [67 mg, 0.408 mmol].

¹H-NMR (CD₃OD) δ: 6.40 (1H, d, J = 11.2 Hz), 6.16 (1H, d, J = 11.2 Hz), 5.36 (1H, dd, J = 2.4, 1.5 Hz), 4.43 (1H, t, J = 5.9 Hz), 4.22-4.15 (1H, m), 2.95 (1H, dd, J = 9.0, 4.0 Hz), 2.91 (3H, s), 2.68-2.39 (7H, m), 2.33 (1H, dd, J = 13.4, 6.6 Hz), 2.27-1.34 (19H, m), 1.06 (3H, d, J = 6.3 Hz), 0.65 (3H, s).

### [Example 215]

### Synthesis of (1R,3S)-5-(2-((1R,3aS,7aR,E)-7a-methyl-1-((R)-4-(4-(methylsulfonyl)piperazin-1-yl)butan-2-yl)octahydro-4H-inden-4-ylidene)ethylidene)-2-methylenecyclohexane-1,3-diol (Compound E004)

Compound E004 [16.0 mg, 0.0316 mmol] was obtained by processing in the same manner as in Example 1 using compound (16c) [50 mg, 0.067 mmol] and 4-(methylsulfonyl)piperazine [40 mg, 0.244 mmol].

¹H-NMR (CD₃OD) δ: 6.26 (1H, d, J = 11.0 Hz), 5.91 (1H, d, J = 11.0 Hz), 5.05 (2H, d, J = 6.9 Hz), 4.43-4.35 (2H, m), 3.22 (4H, t, J = 5.0 Hz), 2.89-2.82 (4H, m), 2.83 (3H, s), 2.67 (1H, dd, J = 13.3, 4.1 Hz), 2.61-2.36 (7H, m), 2.31-2.25 (2H, m), 2.09-2.00 (2H, m), 1.98-1.90 (1H, m), 1.68-1.48 (7H, m), 1.40-1.25 (4H, m), 0.99 (3H, d, J = 6.4 Hz), 0.59 (3H, s).

Exact Mass = 506.32 (C₂₈H₄₆N₂O₄S) Obs. mass = 507.25 (M+H)

### [Reference example 16]

### Synthesis of (4R)-4-((1R,3 aS,7aR,E)-4-(2-((3R, SR)-3, 5-bis((t-butyldimethylsilyl)oxy)cyclohexylidene)ethylidene)-7a-methyloctahydro-1H-inden-1-yl)pentyl 4-methylbenzenesulfonate (Compound 19b)

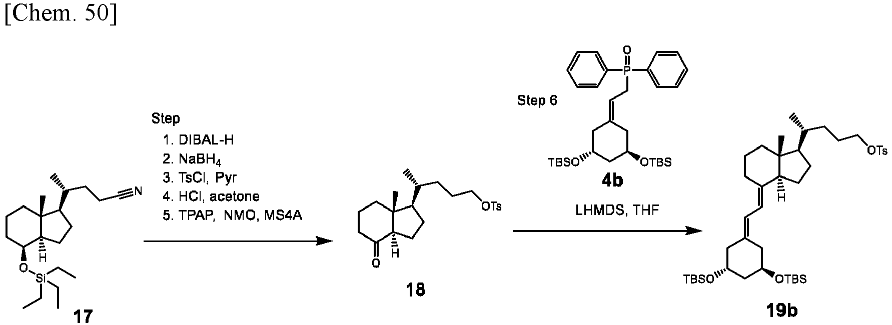

### Step 1

DIBAL-H [1.5 M in toluene, 10 mL] was added to a solution of (4R)-4-((1R,4S,7aR)-7a-methyl-4-((triethylsilyl)oxy)octahydro-1H-inden-1-yl)pentanenitrile [5.1 g, 15 mmol] in toluene [50 mL] at -78°C, and the mixture was stirred for 1.5 hours. The mixture was warmed to 0°C, and further stirred for 1 hour. The reaction system was quenched with methanol. A 5 M aqueous sodium hydroxide solution was added to the reaction system, and the mixture was stirred at room temperature for 10 minutes. The reaction system was poured into saturated brine and the mixture was extracted with ethyl acetate. The organic layer was washed with brine and a saturated aqueous ammonium chloride solution, dried over anhydrous magnesium sulfate, and concentrated under reduced pressure. The residue was partially purified by silica gel column chromatography to obtain (4R)-4-((1R,4S,7aR)-7a-methyl-4-((triethylsilyl)oxy)octahydro-1H-inden-1-yl)pentanal [3.65 g].

### Step 2

The above compound [3.65 g] obtained in step 1 was dissolved in a mixed solvent of toluene [20 mL] and methanol [20 mL], then to the solution, sodium tetrahydroborate [0.88 g, 23 mmol] was added at 0°C, and the mixture was stirred at the same temperature for 30 minutes. The reaction mixture was quenched with a saturated aqueous ammonium chloride solution, poured into saturated brine, and the mixture was extracted with ethyl acetate. The organic layer was dried over anhydrous magnesium sulfate and concentrated under reduced pressure. The residue was purified by silica gel column chromatography to obtain (4R)-4-((1R,4S,7aR)-7a-methyl-4-((triethylsilyl)oxy)octahydro-1H-inden-1-yl)pentan-1-ol [2.37 g, 6.68 mmol].

### Step 3

para-Toluenesulfonyl chloride [1.6 g, 8.4 mmol] was added to a solution of the above compound [2.37 g, 6.68 mmol] obtained in step 2 in pyridine [12 mL], and the mixture was stirred at room temperature for 3 hours. The reaction mixture was poured into saturated brine and the mixture was extracted with ethyl acetate. The organic layer was washed with saturated brine, dried over anhydrous magnesium sulfate, and concentrated under reduced pressure.

### Step 4

The residue obtained in step 3 was dissolved in acetone [30 mL], then to the solution, 2 M hydrochloric acid [10 mL] was added, and the mixture was stirred at room temperature for 1 hour. The reaction mixture was quenched with a saturated aqueous sodium hydrogen carbonate solution and extracted with ethyl acetate. The organic layer was dried over anhydrous magnesium sulfate and concentrated under reduced pressure. The residue was purified by silica gel column chromatography to obtain (4R)-4-((1R,4 S, 7aR)-4-hydroxy-7a-methyloctahydro-1H-inden-1-yl)pentyl 4-methylbenzenesulfonate [1.37 g, 3.47 mmol].

### Step 5

A solution of (4R)-4-((1R,4S,7aR)-4-hydroxy-7a-methyloctahydro-1H-inden-1-yl)pentyl 4-methylbenzenesulfonate [1.37 g, 3.47 mmol] obtained in step 4, N-methylmorpholine-N-oxide monohydrate [0.67 g, 5.0 mmol], and molecular sieves 4A [1.5 g] in dichloromethane [30 mL] was stirred at 0°C for 1 hour. Tetrapropylammonium perruthenate [120 mg, 0.342 mmol] was added to the reaction mixture, and the mixture was stirred at 0°C for 1 hour. The reaction mixture was diluted with heptane and filtered through Celite. The filtrate was washed with a saturated aqueous ammonium chloride solution, and then with saturated brine. The organic layer was dried over anhydrous magnesium sulfate and concentrated under reduced pressure. The residue was purified by silica gel column chromatography to obtain (4R)-4-((1R,7aR)-7a-methyl-4-oxooctahydro-1H-inden-1-yl)pentyl 4-methylbenzenesulfonate [1.15 g, 2.93 mmol].

¹H-NMR (CDCl₃) δ: 7.79 (2H, t, J = 4.1 Hz), 7.35 (2H, d, J = 7.8 Hz), 4.06-3.94 (2H, m), 2.45 (3H, s), 2.43 (1H, dd, J = 12.2, 7.8 Hz), 2.33-2.17 (2H, m), 2.11-1.65 (6H, m), 1.60-0.99 (9H, m), 0.91 (3H, d, J = 6.3 Hz), 0.60 (3H, s).

### Step 6

Under an argon atmosphere, at -78°C, LHMDS [1 M in THF, 2.6 mL, 2.6 mmol] was added to a solution of (2-((3R,5R)-3,5-bis((t-butyldimethylsilyl)oxy)cyclohexylidene)ethyl)diphenylphosphineoxide (Compound 4b, CAS Registry No. 139356-39-1) [0.99 g, 1.7 mmol] in THF [10 mL], and the mixture was stirred for 15 minutes. A solution of (4R)-4-((1R,7aR)-7a-methyl-4-oxooctahydro-1H-inden-1-yl)pentyl 4-methylbenzenesulfonate [1.15 g, 2.93 mmol] obtained in step 5 in THF [10 mL] was added to the reaction mixture, and the mixture was stirred at -78°C for 1.5 hours. The reaction mixture was returned to room temperature, then a saturated aqueous ammonium chloride solution was added to the mixture, and the mixture was extracted with ethyl acetate. The organic layer was dried over anhydrous magnesium sulfate and concentrated under reduced pressure. The residue was purified by silica gel column chromatography to obtain (4R)-4-((1R,3aS,7aR,E)-4-(2-((3R,5R)-3,5-bis((t-butyldimethysilyl)oxy)cyclohexylidene)ethylidene)-7a-methyloctahydro-1H-inden-1-yl)pentyl 4-methylbenzenesulfonate (Compound 19b) [0.67 g, 0.90 mmol].

¹H-NMR (CDCl₃) δ: 7.80 (2H, **d,** J = 8.3 Hz), 7.35 (2H, **d,** J = 8.8 Hz), 6.16 (1H, **d,** J = 10.7 Hz), 5.81 (1H, d, J = 11.2 Hz), 4.10-3.99 (4H, m), 2.80 (1H, d, J = 11.7 Hz), 2.45 (3H, s), 2.40-2.07 (4H, m), 1.96-1.61 (10H, m), 1.53-1.21 (12H, m), 1.07-1.00 (1H, m), 0.87 (9H, s), 0.86 (9H, s), 0.50 (3H, s), 0.05 (6H, s), 0.05 (6H, s).

### [Example 216]

### Synthesis of (1R,3R)-5-(2-((1R,3aS,7aR,E)-1-((R)-5-(3-(difluoromethoxy)azetidin-1-yl)pentan-2-yl)-7a-methyloctahydro-4H-inden-4-ylidene)ethylidene)cyclohexane-1,3-diol (Compound A033)

Compound A033 [10.6 mg, 0.0227 mmol] was obtained by processing in the same manner as in Example 1 using compound 19b [50 mg, 0.067 mmol] described in Reference example 16 and 3-difluoromethoxyazetidine [30 mg, 0.244 mmol] as starting materials.

¹H-NMR (CD₃OD) δ: 6.38 (1H, t, J = 75.0 Hz), 6.21 (1H, d, J = 10.7 Hz), 5.88 (1H, d, J = 10.7 Hz), 4.76-4.70 (1H, m), 4.06-3.94 (2H, m), 3.65 (2H, dd, J = 9.0, 6.6 Hz), 3.11 (2H, dd, J = 9.0, 6.0 Hz), 2.83 (1H, dd, J = 12.0, 3.7 Hz), 2.59 (1H, dd, J = 13.4, 3.7 Hz), 2.52-2.38 (3H, m), 2.23-2.13 (2H, m), 2.03-1.27 (17H, m), 1.12-1.03 (1H, m), 0.95 (3H, d, J = 6.3 Hz), 0.57 (3H, s).

Exact Mass = 467.32 (C₂₇H₄₃F₂NO₃) Obs. mass = 468.30 (M+H)

### [Example 217]

### Synthesis of (1R,3R)-5-(2-((1R,3aS,7aR,E)-1-((R)-5-((S)-3-fluoropyrrolidin-1-yl)pentan-2-yl)-7a-methyloctahydro-4H-inden-4-ylidene)ethylidene)cyclohexane-1,3-diol (Compound B016)

Compound B016 [12.0 mg, 0.0277 mmol] was obtained by processing in the same manner as in Example 1 using compound 19b [50 mg, 0.067 mmol] described in Reference Example 16 and (S)-3-fluoropyrrolidine hydrochloride [30 mg, 0.239 mmol] as starting materials.

Exact Mass = 433.34 (C₂₇H₄₄FNO₂) Obs. mass = 434.30 (M+H)

### [Example 218]

### Synthesis of (1R,3R)-5-(2-((1R,3aS,7aR,E)-1-((R)-5-((R)-3-fluoropyrrolidin-1-yl)pentan-2-yl)-7a-methyloctahydro-4H-inden-4-ylidene)ethylidene)cyclohexane-1,3-diol (Compound B017)

Compound B017 [14.0 mg, 0.0323 mmol] was obtained by performing the reaction in the same manner as in Example 1 using compound 19b [50 mg, 0.067 mmol] and (R)-3-fluoropyrrolidine hydrochloride [30 mg, 0.239 mmol] as starting materials. Exact Mass = 433.34 (C₂₇H₄₄FNO₂) Obs. mass = 434.30 (M+H)

### [Example 219]

### Synthesis of (1R,3R)-5-(2-((1R,3aS,7aR,E)-1-((R)-5-((S)-3-(difluoromethyl)pyrrolidin-1-yl)pentan-2-yl)-7a-methyloctahydro-4H-inden-4-ylidene)ethylidene)cyclohexane-1,3-diol (Compound B084)

Compound B084 [5.0 mg, 0.011 mmol] was obtained by processing in the same manner as in Example 1 using compound 19b [55 mg, 0.074 mmol] and (S)-3-(difluoromethyl)pyrrolidine hydrochloride [50 mg, 0.317 mmol] as starting materials. ¹H-NMR (CD₃OD) δ: 6.22 (1H, d, J = 10.7 Hz), 5.89 (2H, d, J = 10.7 Hz), 5.81 (2H, td, J = 56.0, 6.0 Hz), 4.13-3.96 (2H, m), 2.86-2.14 (13H, m), 2.05-1.29 (21H, m), 1.14-1.05 (1H, m), 0.98 (3H, d, J = 6.8 Hz), 0.58 (3H, s).

Exact Mass = 465.34 (C₂₈H₄₅F₂NO₂) Obs. mass = 466.40 (M+H)

### [Example 220]

### Synthesis of (1R,3R)-5-(2-((1R,3aS,7aR,E)-1-((R)-5-((R)-3-(difluoromethyl)pyrrolidin-1-yl)pentan-2-yl)-7a-methyloctahydro-4H-inden-4-ylidene)ethylidene)cyclohexane-1,3-diol (Compound B085)

Compound B085 [14.7 mg, 0.0316 mmol] was obtained by processing in the same manner as in Example 1 using compound 19b [55 mg, 0.074 mmol] and (R)-3-(difluoromethyl)pyrrolidine hydrochloride [50 mg, 0.317 mmol] as starting materials. ¹H-NMR (CD₃OD) δ: 6.22 (1H, d, J = 11.2 Hz), 5.89 (2H, d, J = 11.2 Hz), 5.80 (2H, td, J = 58.0, 5.0 Hz), 4.07-3.96 (2H, m), 2.84 (1H, dd, J = 12.2, 3.4 Hz), 2.77 (1H, t, J = 9.0 Hz), 2.69-2.39 (8H, m), 2.24-2.14 (2H, m), 2.06-1.31 (20H, m), 1.15-1.05 (1H, m), 0.98 (3H, d, J = 6.3 Hz), 0.58 (3H, s).

Exact Mass = 465.34 (C₂₈H₄₅F₂NO₂) Obs. mass = 466.40 (M+H)

### [Example 221]

### Synthesis of (1R,3R)-5-(2-((1R,3aS,7aR,E)-7a-methyl-1-((R)-5-morpholinopentan-2-yl)octahydro-4H-inden-4-ylidene)ethylidene)cyclohexane-1,3-diol (compound D044)

Compound D044 [15.1 mg, 0.0350 mmol] was obtained by processing in the same manner as in Example 1 using compound 19b [55 mg, 0.074 mmol] and morpholine [0.05 mL, 0.58 mmol] as starting materials.

¹H-NMR (CD₃OD) δ: 6.22 (1H, d, J = 10.7 Hz), 5.89 (1H, d, J = 11.2 Hz), 4.07-3.96 (2H, m), 3.70 (4H, t, J = 4.6 Hz), 2.84 (1H, dd, J = 12.0, 3.7 Hz), 2.60 (1H, dd, J = 13.2, 3.4 Hz), 2.48-2.14 (9H, m), 2.05-1.30 (18H, m), 1.13-1.04 (1H, m), 0.98 (3H, d, J = 6.3 Hz), 0.58 (3H, s).

Exact Mass = 431.34 (C₂₇H₄₅NO₃) Obs. mass = 432.30 (M+H)

### [Reference example 17]

### Synthesis of (2R)-2-((1R,3aS,7aR,E)-4-((Z)-2-((3S,5R)-3,5-dihydroxy-2-methylenecyclohexylidene)ethylidene)-7a-methyloctahydro-1H-inden-1-yl)propyl 4-methylbenzenesulfonate (Compound 23a)

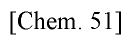

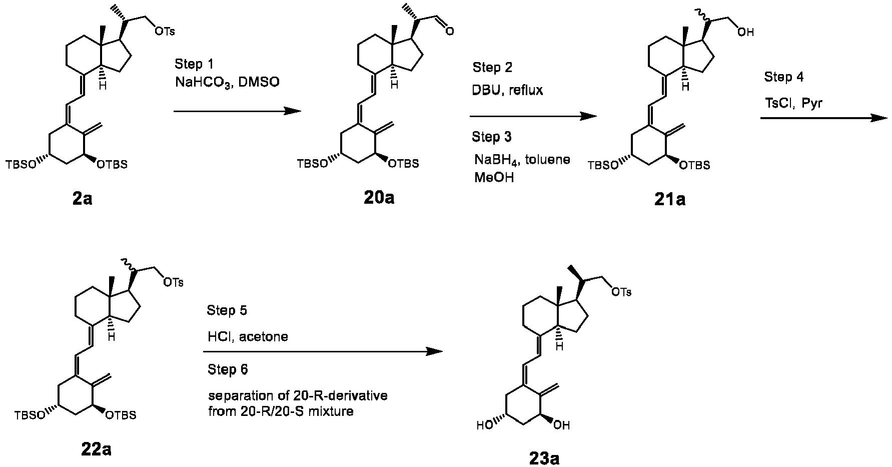

### Step 1

Sodium hydrogen carbonate [1.43 g, 17.0 mmol] was added to a mixed solution of compound 2a [4.15 g, 5.69 mmol] in DMSO [40 mL] and toluene [50 mL], and the reaction mixture was stirred at 100°C for 3 hours. After cooling to room temperature, the reaction mixture was added to a saturated aqueous ammonium chloride solution and the mixture was extracted with heptane. The organic layer was washed with saturated brine, dried over anhydrous magnesium sulfate, and concentrated under reduced pressure. The residue was purified by silica gel column chromatography to obtain 2-((1R,3aS,7aR,E)-4-((Z)-2-((3S,5R)-3,5-bis((t-butyldimethylsilyl)oxy)-2-methylenecyclohexylidene)ethylidene)-7a-methyloctahydro-1H-inden-1-yl)propanal (Compound 20a) [2.37 g, 4.14 mmol].

### Step 2

Diazabicycloundecene (DBU) [1.5 mL] was added to a solution of compound 20a [2.37 g, 4.14 mmol] in THF [50 mL], and the solution was heated and refluxed for 38 hours. After cooling to room temperature, the reaction mixture was transferred to saturated brine and the mixture was extracted with ethyl acetate. The organic layer was washed with saturated brine, dried over anhydrous magnesium sulfate, and concentrated under reduced pressure. The crude product was used in the next reaction without purification.

### Step 3

The crude product obtained in step 2 was dissolved in a mixed solution of THF [50 mL] and methanol [50 mL], then to the solution, sodium tetrahydroborate [0.38 g, 10 mmol] was added at 0°C, and the mixture was stirred at the same temperature for 1 hour. The reaction mixture was quenched with a saturated aqueous ammonium chloride solution, transferred to saturated brine, and the mixture was extracted with ethyl acetate. The organic layer was dried over anhydrous magnesium sulfate and concentrated under reduced pressure. The residue was purified by silica gel column chromatography to obtain 2-((1R,3aS,7aR,E)-4-((Z)-2-((3S,5R)-3,5-bis((t-butyldimethylsilyl)oxy)-2-methylenecyclohexylidene)ethylidene)-7a-methyloctahydro-1H-inden-1-yl)propan-1-ol (Compound 21a) [1.96 g, 3.41 mmol].

### Step 4

p-Toluenesulfonyl chloride [1.01 g, 5.30 mmol] and 4-dimethylaminopyridine (DMAP) [100 mg, 0.818 mmol] were added to a solution of compound 21a [1.96 g, 3.41 mmol] obtained in step 3 in pyridine [20 mL], and the mixture was stirred at room temperature overnight. The reaction mixture was concentrated under reduced pressure, diluted with toluene, and washed with saturated brine. The organic layer was dried over anhydrous magnesium sulfate and concentrated under reduced pressure. The crude product (Compound 22a) was used in the next reaction without purification.

### Step 5 and step 6

The crude product (Compound 22a) obtained in step 4 was dissolved in acetone [120 mL], then to the solution, 3 M hydrochloric acid [20 mL, 60 mmol] was added, and the mixture was stirred at room temperature for 6 hours. The reaction mixture was quenched with a 5 M aqueous sodium hydroxide solution and a saturated aqueous sodium hydrogen carbonate solution. Acetone in the reaction mixture was removed under reduced pressure. Ethyl acetate was added to the residue, and the mixture was washed with saturated brine. The organic layer was dried over anhydrous magnesium sulfate and concentrated under reduced pressure. The residue was purified by silica gel column chromatography to obtain a mixture of R and S diastereomers at C-20. A mixed solvent [10 mL] of heptane/ethyl acetate (1/10) was added to the mixture, and the mixture was irradiated with ultrasonic waves for 3 minutes. The solid [798 mg] generated in the solution by ultrasonic irradiation was collected by filtration. Anhydrous methanol was added to a portion [545 mg] of the solid and the suspension was heated and refluxed for 45 minutes. The suspension was slowly cooled to room temperature. The solid in the reaction liquid was filtered and recovered to obtain (2R)-2-((1R,3aS,7aR,E)-4-((Z)-2-((3S,5R)-3,5-dihydroxy-2-methylenecyclohexylidene)ethylidene)-7a-methyloctahydro-1H-inden-1-yl)propyl 4-methylbenzenesulfonate (Compound 23a) [342 mg, 683 mmol]. ¹H-NMR (CD₃OD) δ: 7.77 (2H, d, J = 8.2 Hz), 7.45 (2H, d, J = 8.2 Hz), 6.29 (1H, d, J = 11.0 Hz), 6.05 (1H, d, J = 11.0 Hz), 5.27 (1H, s), 4.33 (1H, t, J = 5.9 Hz), 4.14-4.07 (2H, m), 3.82 (1H, dd, J = 9.4, 6.6 Hz), 2.83 (1H, dd, J = 12.3, 3.2 Hz), 2.50 (1H, dd, J = 13.5, 3.4 Hz), 2.45 (3H, s), 2.24 (1H, dd, J = 13.3, 6.9 Hz), 1.96 (1H, dd, J = 11.7, 7.5 Hz), 1.89-1.27 (12H, m), 1.19 (1H, td, J = 12.5, 3.8 Hz), 0.89 (3H, d, J = 6.9 Hz), 0.45 (3H, s).

### [Example 222]

### Synthesis of (1R,3S,Z)-5-(2-((1R,3aS,7aR,E)-7a-methyl-1-((R)-1-(pyrrolidin-1-yl)propan-2-yl)octahydro-4H-inden-4-ylidene)ethylidene)-4-methylenecyclohexane-1,3-diol (Compound B009)

Compound 23a [40 mg, 0.080 mmol] was dissolved in pyrrolidine [0.6 mL] and the solution was stirred at 60°C overnight. The reaction mixture was concentrated under reduced pressure. The residue was purified by reverse phase HPLC to obtain compound B009 [16.0 mg, 0.040 mmol].

Exact Mass = 399.31 (C₂₆H₄₁NO₂) Obs. mass = 400.25 (M+H)

### [Example 223]

### Synthesis of (1R,3S,Z)-5-(2-((1R,3aS,7aR,E)-7a-methyl-1-((R)-1-((S)-3-methylpyrrolidin-1-yl)propan-2-yl)octahydro-4H-inden-4-ylidene)ethylidene)-4-methylenecyclohexane-1,3-diol (Compound B010)

A solution of compound 23a [40 mg, 0.080 mmol], (S)-3-methylpyrrolidine hydrochloride [30 mg, 0.247 mmol], and potassium carbonate [60 mg, 0.434 mmol] in DMF [1 mL] were stirred at 60°C overnight. The reaction mixture was quenched with saturated brine and extracted with ethyl acetate. The organic layer was dried over anhydrous magnesium sulfate and concentrated under reduced pressure. The residue was purified by reverse phase HPLC to obtain compound B010 [14.6 mg, 0.0353 mmol]. ¹H-NMR (CD₃OD) δ: 6.31 (1H, d, J = 11.4 Hz), 6.08 (1H, d, J = 11.4 Hz), 5.28 (1H, dd, J = 2.7, 1.4 Hz), 4.89 (1H, d, J = 2.7 Hz), 4.35 (1H, t, J = 5.9 Hz), 4.15-4.10 (1H, m), 2.86 (1H, dd, J= 11.9, 4.1 Hz), 2.78-2.67 (2H, m), 2.54-2.19 (7H, m), 2.07-1.85 (8H, m), 1.73-1.27 (12H, m), 1.02 (3H, d, J = 6.9 Hz), 0.94 (3H, d, J = 6.4 Hz), 0.59 (3H, s).

### [Example 224]

### Synthesis of (1R,3S,Z)-5-(2-((1R,3aS,7aR,E)-7a-methyl-1-((R)-1-((R)-3-methylpyrrolidin-1-yl)propan-2-yl)octahydro-4H-inden-4-ylidene)ethylidene)-4-methylenecyclohexane-1,3-diol (Compound B011)

Compound B011 [13.0 mg, 0.0314 mmol] was obtained by processing in the same manner as in Example 223 using compound 23a [40 mg, 0.080 mmol] and (R)-3-methylpyrrolidine hydrochloride [30 mg, 0.247 mmol] as starting materials.

¹H-NMR (CD₃OD) δ: 6.31 (1H, d, J = 11.0 Hz), 6.08 (1H, d, J = 11.4 Hz), 5.28 (1H, dd, J = 2.3, 1.4 Hz), 4.89 (1H, d, J = 2.3 Hz), 4.35 (1H, t, J = 5.9 Hz), 4.15-4.09 (1H, m), 2.93-2.84 (2H, m), 2.71-2.65 (1H, m), 2.53-2.42 (3H, m), 2.29-2.17 (3H, m), 2.06-1.80 (8H, m), 1.73-1.28 (11H, m), 1.02 (3H, d, J = 6.9 Hz), 0.94 (3H, d, J = 6.4 Hz), 0.59 (3H, s).

### [Example 225]

### Synthesis of (1R,3S,Z)-5-(2-((1R,3aS,7aR,E)-1-((R)-1-((S)-3-fluoropyrrolidin-1-yl)propan-2-yl)-7a-methyloctahydro-4H-inden-4-ylidene)ethylidene)-4-methylenecyclohexane-1,3-diol (Compound B018)

Compound B018 [10.6 mg, 0.0254 mmol] was obtained by processing in the same manner as in Example 223 using compound 23a [40 mg, 0.080 mmol] and (S)-3-fluoropyrrolidine hydrochloride [30 mg, 0.239 mmol] as starting materials.

¹H-NMR (CD₃OD) δ: 6.31 (1H, d, J= 11.4 Hz), 6.08 (1H, d, J = 11.0 Hz), 5.28 (1H, dd, J = 2.3, 1.1 Hz), 5.22-5.03 (1H, m), 4.89 (1H, d, J = 2.3 Hz), 4.35 (1H, t, J = 5.9 Hz), 4.15-4.08 (1H, m), 2.95-2.83 (2H, m), 2.76 (1H, dd, J = 14.6, 7.8 Hz), 2.59-1.82 (14H, m), 1.73-1.28 (10H, m), 0.95 (3H, d, J = 6.9 Hz), 0.59 (3H, s).

Exact Mass = 417.30 (C₂₆H₄₀FNO₂) Obs. mass = 418.25 (M+H)

### [Example 226]

### Synthesis of (1R,3S,Z)-5-(2-((1R,3aS,7aR,E)-1-((R)-1-((R)-3-fluoropyrrolidin-1-yl)propan-2-yl)-7a-methyloctahydro-4H-inden-4-ylidene)ethylidene)-4-methylenecyclohexane-1,3-diol (Compound B019)

Compound B019 [9.7 mg, 0.023 mmol] was obtained by processing in the same manner as in Example 223 using compound 23a [40 mg, 0.080 mmol] and (R)-3-fluoropyrrolidine hydrochloride [30 mg, 0.239 mmol] as starting materials.

¹H-NMR (CD₃OD) δ: 6.31 (1H, d, J = 11.4 Hz), 6.08 (1H, d, J = 11.0 Hz), 5.28 (1H, d, J = 2.3 Hz), 5.21-5.03 (1H, m), 4.89 (1H, d, J = 2.3 Hz), 4.35 (1H, t, J = 5.7 Hz), 4.16-4.10 (1H, m), 2.88-2.65 (4H, m), 2.54-2.49 (2H, m), 2.36-1.84 (11H, m), 1.73-1.28 (10H, m), 0.95 (3H, d, J = 6.4 Hz), 0.59 (3H, s).

Exact Mass = 417.30 (C₂₆H₄₀FNO₂) Obs. mass = 418.20 (M+H)

### [Example 227]

### Synthesis of (1R,3S,Z)-5-(2-((1R,3aS,7aR,E)-1-((R)-1-((S)-3-(difluoromethyl)pyrrolidin-1-yl)propan-2-yl)-7a-methyloctahydro-4H-inden-4-ylidene)ethylidene)-4-methylenecyclohexane-1,3-diol (Compound B095)

Compound B095 [14.5 mg, 0.0323 mmol] was obtained by processing in the same manner as in Example 223 using compound 23a [40 mg, 0.080 mmol] and (S)-3-(difluoromethyl)pyrrolidine hydrochloride [40 mg, 0.239 mmol] as starting materials. ¹H-NMR (CD₃OD) δ: 6.31 (1H, d, J = 11.4 Hz), 6.08 (1H, d, J = 11.4 Hz), 5.74 (1H, td, J = 57.3, 5.2 Hz), 5.28 (1H, dd, J = 2.3, 1.4 Hz), 4.89 (1H, dd, J = 2.3, 1.4 Hz), 4.35 (1H, t, J= 5.7 Hz), 4.15-4.10 (1H, m), 2.86 (1H, dd, J= 11.0, 4.0 Hz), 2.60-2.44 (7H, m), 2.35-2.20 (2H, m), 2.03-1.27 (16H, m), 0.94 (3H, d, J = 6.4 Hz), 0.59 (3H, s).

Exact Mass = 449.31 (C₂₇H₄₁F₂NO₂) Obs. mass = 450.25 (M+H)

### [Example 228]

### Synthesis of (1R,3S,Z)-5-(2-((1R,3aS,7aR,E)-1-((R)-1-((R)-3-(difluoromethyl)pyrrolidin-1-yl)propan-2-yl)-7a-methyloctahydro-4H-inden-4-ylidene)ethylidene)-4-methylenecyclohexane-1,3-diol (Compound B096)

Compound B096 [12.0 mg, 0.0267 mmol] was obtained by processing in the same manner as in Example 223 using compound 23a [40 mg, 0.080 mmol] and (R)-3-(difluoromethyl)pyrrolidine hydrochloride [40 mg, 0.239 mmol] as starting materials.

¹H-NMR (CD₃OD) δ: 6.31 (1H, d, J = 11.0 Hz), 6.08 (1H, d, J = 11.4 Hz), 5.76 (1H, td, J = 57.2, 5.9 Hz), 5.28 (1H, dd, J = 2.3, 1.4 Hz), 4.89 (1H, d, J = 1.4 Hz), 4.35 (1H, t, J = 5.9 Hz), 4.15-4.10 (1H, m), 2.86 (1H, dd, J = 12.1, 3.4 Hz), 2.71 (1H, t, J = 8.7 Hz), 2.60-2.23 (8H, m), 2.03-1.27 (16H, m), 0.94 (3H, d, J = 6.4 Hz), 0.59 (3H, s).

Exact Mass = 449.31 (C₂₇H₄₁F₂NO₂) Obs. mass = 450.20 (M+H)

### [Reference example 18]

### Synthesis of (2R)-2-((1R,3aS,7aR,E)-4-(2-((3S,5R)-3,5-dihydroxycyclohexylidene)ethylidene)-7a-methyloctahydro-1H-inden-1-yl)propyl 4-methylbenzenesulfonate (Compound 23b)

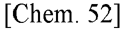

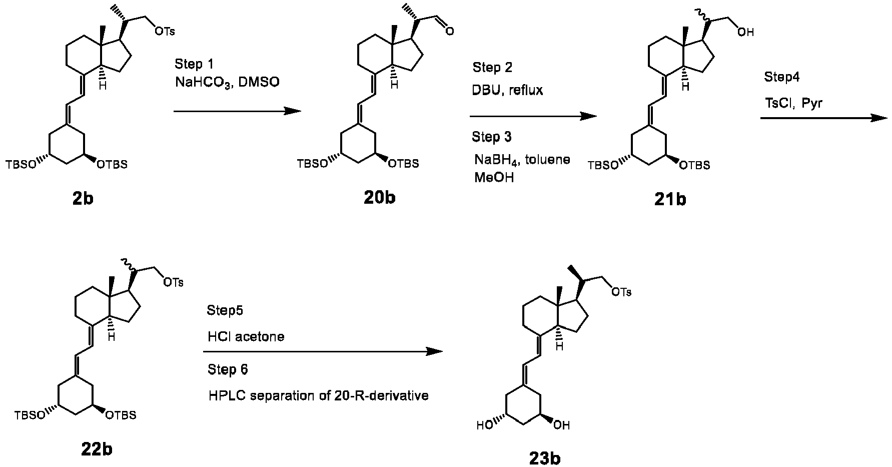

### Step 1

Compound 20b [3.31 g, 5.90 mmol] was obtained by processing in the same manner as in step 1 of Reference example 17 using the compound 2b [4.70 g, 6.55 mmol] as a starting material.

### Step 2 and step 3

Compound 21b [3.12 g, 5.54 mmol] was obtained by processing in the same manner as in steps 2 and 3 of Reference example 17 using the compound 20b [3.31 g, 5.90 mmol] as a starting material.

### Step 4

Compound 22b [3.12 g, 5.54 mmol] was obtained by processing in the same manner as in step 4 of Reference example 17 using compound 21b [3.12 g, 5.54 mmol] as a starting material.

### Step 5

A desilylated crude product was obtained by processing in the same manner as in step 5 of Reference example 17 using compound 22b [3.12 g, 5.54 mmol] as a starting material. This crude product was purified by preparative HPLC, and the compound with the high polarity was recovered to obtain compound 23b [0.66 g].

¹H-NMR (CD₃OD) δ: 7.77 (2H, d, J = 8.2 Hz), 7.44 (2H, d, J = 8.2 Hz), 6.19 (1H, d, J = 11.0 Hz), 5.86 (1H, d, J = 11.0 Hz), 4.09-3.95 (3H, m), 3.83 (1H, dd, J = 9.6, 6.9 Hz), 2.80 (1H, d, J = 13.3 Hz), 2.56 (1H, dd, J = 13.3, 3.7 Hz), 2.45 (3H, s), 2.40 (1H, dd, J = 14.4, 3.4 Hz), 2.22-2.12 (2H, m), 1.98 (1H, dd, J = 7.0, 12.0 Hz), 1.88-1.16 (14H, m), 0.90 (3H, d, J = 6.9 Hz), 0.45 (3H, s).

### [Example 229]

### Synthesis of (1R,3S)-5-(2-((1R,3aS,7aR,E)-1-((R)-1-((S)-3-fluoropyrrolidin-1-yl)propan-2-yl)-7a-methyloctahydro-4H-inden-4-ylidene)ethylidene)cyclohexane-1,3-diol (Compound B097)

Compound B097 [9.0 mg, 0.022 mmol] was obtained by processing in the same manner as in Example 223 using compound 23b [40 mg, 0.082 mmol] and (S)-3-fluoropyrrolidine hydrochloride [30 mg, 0.239 mmol] as starting materials.

¹H-NMR (CD₃OD) δ: 6.21 (1H, d, J = 11.0 Hz), 5.89 (1H, d, J = 11.4 Hz), 5.13 (1H, dt, J = 55.8, 5.7 Hz), 4.06-3.95 (2H, m), 2.94-2.74 (3H, m), 2.61-2.37 (6H, m), 2.32 (1H, t, J = 11.0 Hz), 2.23-1.27 (22H, m), 0.96 (3H, d, J = 6.9 Hz), 0.60 (3H, s).

Exact Mass = 405.30 (C₂₅H₄₀FNO₂) Obs. mass = 406.20 (M+H)

### [Example 230]

### Synthesis of (1R,3S)-5-(2-((1R,3aS,7aR,E)-1-((R)-1-((R)-3-fluoropyrrolidin-1-yl)propan-2-yl)-7a-methyloctahydro-4H-inden-4-ylidene)ethylidene)cyclohexane-1,3-diol (Compound B098)

Compound B098 [14.7 mg, 0.0362 mmol] was obtained by processing in the same manner as in Example 223 using compound 23b [40 mg, 0.082 mmol] and (R)-3-fluoropyrrolidine hydrochloride [30 mg, 0.239 mmol] as starting materials.

¹H-NMR (CD₃OD) δ: 6.21 (1H, d, J = 11.0 Hz), 5.89 (1H, d, J = 11.4 Hz), 5.12 (1H, dt, J = 56.0, 5.4 Hz), 4.06-3.95 (2H, m), 2.85-2.65 (4H, m), 2.59 (1H, dd, J = 13.3, 3.7 Hz), 2.52 (1H, dd, J = 11.7, 4.8 Hz), 2.41 (1H, dd, J = 13.3, 3.2 Hz), 2.36-1.28 (20H, m), 0.95 (3H, d, J = 6.4 Hz), 0.60 (3H, s).

Exact Mass = 405.30 (C₂₅H₄₀FNO₂) Obs. mass = 406.25 (M+H)

### [Example 231]

### Synthesis of (1R,3S)-5-(2-((1R,3aS,7aR,E)-1-((R)-1-((S)-3-(difluoromethyl)pyrrolidin-1-yl)propan-2-yl)-7a-methyloctahydro-4H-inden-4-ylidene)ethylidene)cyclohexane-1,3-diol (Compound B099)

Compound B099 [10.0 mg, 0.0229 mmol] was obtained by processing in the same manner as in Example 223 using compound 23b [40 mg, 0.082 mmol] and (S)-3-(difluoromethyl)pyrrolidine hydrochloride [35 mg, 0.222 mmol] as starting materials.

¹H-NMR (CD₃OD) δ: 6.21 (1H, d, J = 11.4 Hz), 5.89 (1H, d, J = 11.4 Hz), 5.74 (1H, td, J = 57.5, 5.0 Hz), 4.06-3.95 (2H, m), 2.83 (1H, dd, J = 11.7, 4.3 Hz), 2.61-2.44 (6H, m), 2.40 (1H, dd, J = 13.3, 3.7 Hz), 2.30 (1H, t, J = 11.0 Hz), 2.23-2.13 (2H, m), 2.05-1.27 (15H, m), 0.95 (3H, d, J = 6.4 Hz), 0.60 (3H, s).

Exact Mass = 437.31 (C₂₆H₄₁F₂NO₂) Obs. mass = 438.20 (M+H)

### [Example 232]

### Synthesis of (1R,3S)-5-(2-((1R,3aS,7aR,E)-1-((R)-1-((R)-3-(difluoromethyl)pyrrolidin-1-yl)propan-2-yl)-7a-methyloctahydro-4H-inden-4-ylidene)ethylidene)cyclohexane-1,3-diol (Compound B100)

Compound B100 [10.0 mg, 0.0229 mmol] was obtained by processing in the same manner as in Example 223 using compound 23b [40 mg, 0.082 mmol] and (R)-3-(difluoromethyl)pyrrolidine hydrochloride [35 mg, 0.222 mmol] as starting materials.

¹H-NMR (CD₃OD) δ: 6.21 (1H, d, J = 11.4 Hz), 5.89 (1H, d, J = 11.4 Hz), 5.76 (1H, td, J = 57.0, 5.0 Hz), 4.06-3.95 (2H, m), 2.83 (1H, dd, J = 12.3, 3.2 Hz), 2.71 (1H, t, J = 8.7 Hz), 2.61-2.37 (7H, m), 2.27 (1H, t, J = 10.7 Hz), 2.18 (2H, ddd, J = 25.6, 12.6, 5.3 Hz), 2.04-1.26 (16H, m), 0.95 (3H, d, J = 6.4 Hz), 0.59 (3H, s).

Exact Mass = 437.31 (C₂₆H₄₁F₂NO₂) Obs. mass = 438.25 (M+H)

### [Example 233]

### Synthesis of (1R,3S)-5-(2-((1R,3aS,7aR,E)-1-((R)-1-((S)-3-(1,1-difluoroethyl)pyrrolidin-1-yl)propan-2-yl)-7a-methyloctahydro-4H-inden-4-ylidene)ethylidene)cyclohexane-1,3-diol (Compound B101)

Compound B101 [8.1 mg, 0.018 mmol] was obtained by processing in the same manner as in Example 223 using compound 23b [40 mg, 0.082 mmol] and (S)-3-(1,1-difluoroethyl)pyrrolidine hydrochloride [35 mg, 0.204 mmol] as starting materials. ¹H-NMR (CD₃OD) δ: 6.21 (1H, d, J = 11.4 Hz), 5.89 (1H, d, J = 11.0 Hz), 4.06-3.95 (2H, m), 2.83 (1H, dd, J = 11.4, 3.2 Hz), 2.69-2.36 (7H, m), 2.31 (1H, t, J = 11.0 Hz), 2.23-2.13 (2H, m), 2.04-1.27 (20H, m), 0.95 (3H, d, J = 6.9 Hz), 0.60 (3H, s).

Exact Mass = 451.33 (C₂₇H₄₃F₂NO₂) Obs. mass = 452.25 (M+H)

### [Example 234]

### Synthesis of (1R,3S)-5-(2-((1R,3aS,7aR,E)-1-((R)-1-((R)-3-(1,1-difluoroethyl)pyrrolidin-1-yl)propan-2-yl)-7a-methyloctahydro-4H-inden-4-ylidene)ethylidene)cyclohexane-1,3-diol (Compound B102)

Compound B102 [11.3 mg, 0.0250 mmol] was obtained by processing in the same manner as in Example 223 using compound 23b [40 mg, 0.082 mmol] and (R)-3-(1,1-difluoroethyl)pyrrolidine hydrochloride [35 mg, 0.204 mmol] as starting materials. ¹H-NMR (CD₃OD) δ: 6.21 (1H, d, J = 11.0 Hz), 5.89 (1H, d, J = 11.0 Hz), 4.06-3.95 (2H, m), 2.86-2.78 (2H, m), 2.70-2.55 (4H, m), 2.52-2.38 (4H, m), 2.30-2.13 (5H, m), 2.04-1.27 (24H, m), 0.95 (3H, d, J = 6.9 Hz), 0.60 (3H, s).

Exact Mass = 451.33 (C₂₇H₄₃F₂NO₂) Obs. mass = 452.25 (M+H)

### [Example 235]

### Synthesis of (1R,3S)-5-(2-((1R,3aS,7aR,E)-1-((R)-1-((S)-3-(2,2-difluoroethyl)pyrrolidin-1-yl)propan-2-yl)-7a-methyloctahydro-4H-inden-4-ylidene)ethylidene)cyclohexane-1,3-diol (Compound B103)

Compound B103 [14.7 mg, 0.0326 mmol] was obtained by processing in the same manner as in Example 223 using compound 23b [40 mg, 0.082 mmol] and (S)-3-(2,2-difluoroethyl)pyrrolidine hydrochloride [40 mg, 0.233 mmol] as starting materials. ¹H-NMR (CD₃OD) δ: 6.21 (1H, d, J = 11.4 Hz), 5.89 (1H, d, J = 11.4 Hz), 5.88 (1H, tt, J = 56.0, 5.0 Hz), 4.06-3.95 (2H, m), 2.90 (1H, t, J = 8.5 Hz), 2.83 (1H, dd, J = 11.7, 3.9 Hz), 2.67-1.28 (33H, m), 0.95 (3H, d, J = 6.4 Hz), 0.60 (3H, s).

Exact Mass = 451.33 (C₂₇H₄₃F₂NO₂) Obs. mass = 452.25 (M+H)

### [Example 236]

### Synthesis of (1R,3S,Z)-5-(2-((1R,3aS,7aR,E)-1-((R)-1-(4-hydroxy-4-(trifluoromethyl)piperidin-1-yl)propan-2-yl)-7a-methyloctahydro-4H-inden-4-ylidene)ethylidene)-4-methylenecyclohexane-1,3-diol (Compound C005)

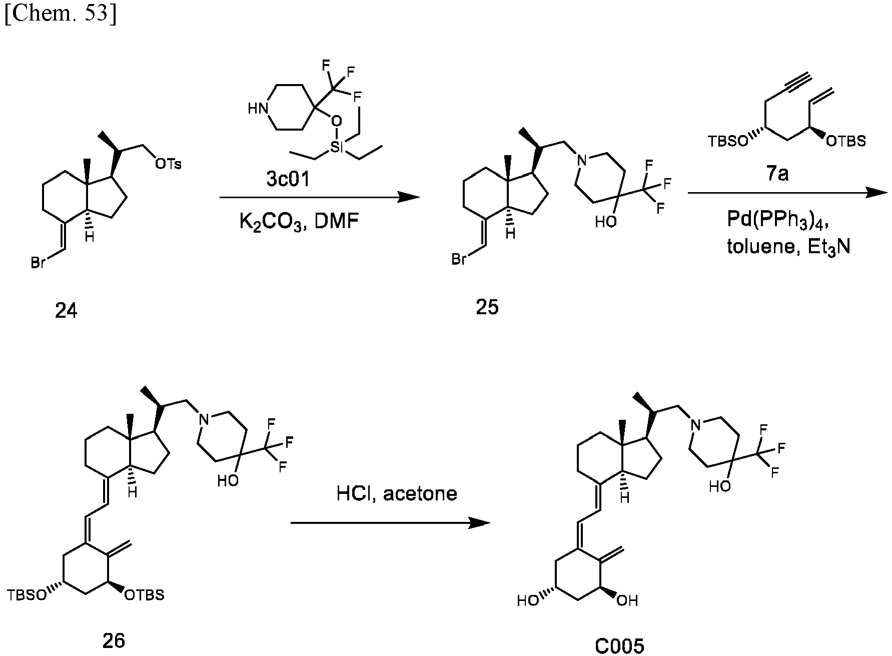

### Step 1

1-((2R)-2-((1R,3aS,7aR,E)-4-(Bromomethylene)-7a-methyloctahydro-1H-inden-1-yl)propyl)-4-(trifluoromethyl)piperidin-4-ol (Compound 25) [106.4 mg, 0.2427 mmol] was obtained by processing in the same manner as in Example 92 using (2R)-2-((1R,3aS,7aR,E)-4-(bromomethylene)-7a-methyloctahydro-1H-inden-1 -yl)propyl 4-methylbenzenesulfonate [200.6 mg, 0.4545 mmol] and triethyl-[[4-(trifluoromethyl)-4-piperidyl]oxy]silane [257.2 mg, 0.9075 mmol] as starting materials.

### Step 2 and step 3

Compound C005 [13.6 mg, 0.0273 mmol] was obtained by processing in the same manner as in step 2 and step 3 of Example 92 using compound 25 [49 mg, 0.111 mmol] obtained in step 1, compound 7a [57.2 mg, 0.155 mmol], and tetrakis(triphenylphosphine)palladium(0) [23.2 mg, 0.0201 mmol] as starting materials. ¹H-NMR (CD₃OD) δ: 6.32 (1H, d, J = 10.7 Hz), 6.09 (1H, d, J = 11.2 Hz), 5.29 (1H, t, J = 1.2 Hz), 4.35 (1H, t, J = 5.9 Hz), 4.15-4.10 (1H, m), 3.23 (1H, d, J = 11.2 Hz), 3.09 (1H, d, J = 11.7 Hz), 2.96-2.83 (3H, m), 2.63 (1H, td, J = 12.4, 2.8 Hz), 2.57-2.47 (2H, m), 2.26 (1H, dd, J = 13.7, 6.8 Hz), 2.10-1.97 (5H, m), 1.92-1.29 (17H, m), 0.99 (3H, d, J = 6.3 Hz), 0.61 (3H, s).

Exact Mass = 497.31 (C₂₈H₄₂F₃NO₃) Obs. mass = 498.3 (M+H)

### [Example 237]

### Synthesis of (1R,2S,3S,Z)-5-(2-((1R,3aS,7aR,E)-1-((R)-1-(4-hydroxy-4-(trifluoromethyl)piperidin-1-yl)propan-2-yl)-7a-methyloctahydro-4H-inden-4-ylidene)ethylidene)-4-methylenecyclohexane-1,3-diol (Compound C006)

Compound C006 [15.8 mg, 0.0309 mmol] was obtained by processing in the same manner as in step 2 and step 3 of Example 92 using compound 25 [49 mg, 0.111 mmol] obtained in step 1 of Example 236 and compound 7b [55 mg, 0.144 mmol] as starting materials.

¹H-NMR (CD₃OD) δ: 6.32 (1H, d, J = 11.2 Hz), 6.10 (1H, d, J = 11.2 Hz), 5.22 (1H, d, J = 2.0 Hz), 4.22 (1H, d, J = 3.4 Hz), 3.72 (1H, td, J = 8.1, 4.2 Hz), 3.23 (1H, d, J = 11.7 Hz), 3.09 (1H, d, J = 11.2 Hz), 2.95-2.83 (3H, m), 2.68-2.50 (3H, m), 2.17 (1H, dd, J = 13.2, 8.3 Hz), 2.09-1.98 (4H, m), 1.93-1.30 (15H, m), 1.04 (3H, d, J = 7.3 Hz), 0.98 (3H, d, J = 6.3 Hz), 0.60 (3H, s).

Exact Mass = 511.33 (C₂₉H₄₄F₃NO₃) Obs. mass = 512.4 (M+H)

### [Example 238]

### Synthesis of (1R,3S,Z)-5-(2-((1R,3aS,7aR,E)-7a-methyl-1-((R)-1-(4-(trifluoromethyl)piperidin-1-yl)propan-2-yl)octahydro-4H-inden-4-ylidene)ethylidene)-4-methylenecyclohexane-1,3-diol (Compound C011)

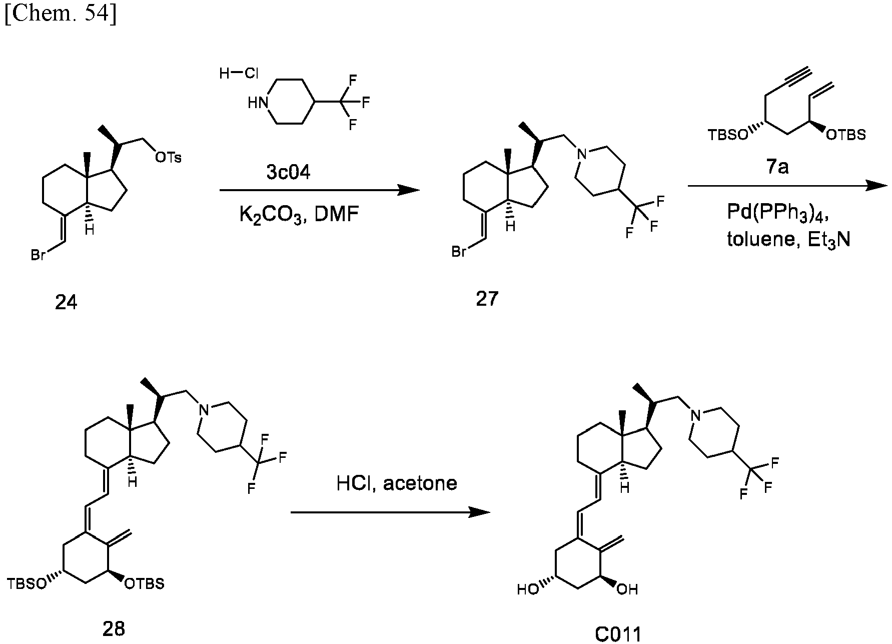

### Step 1

Compound 27 [105.7 mg, 0.250 mmol] was obtained by performing the reaction in the same manner as in step 1 of Example 236 using compound 24 [185 mg, 0.419 mmol] and compound 3c04 [215 mg, 1.40 mmol] as starting materials.

¹H-NMR (CDCl₃) δ: 5.65 (1H, t, J = 2.0 Hz), 2.98-2.86 (3H, m), 2.35 (1H, dd, J = 12.0, 5.1 Hz), 2.01-1.60 (15H, m), 1.56-1.21 (7H, m), 0.90 (3H, d, J = 6.8 Hz), 0.57 (3H, s). Exact Mass = 421.16 (C₂₀H₃₁BrF₃N) Obs. mass = 422.20 (M+H)

### Step 2 and step 3

Compound C011 [15.9 mg, 0.033 mmol] was obtained by processing in the same manner as in step 2 and step 3 of Example 92 using compound 27 [32 mg, 0.076 mmol] obtained in step 1, compound 7a [40.7 mg, 0.110 mmol], and tetrakis(triphenylphosphine)palladium(0) [15.0 mg, 0.013 mmol] as starting materials.

¹H-NMR (CD₃OD) δ: 6.31 (1H, d, J = 11.2 Hz), 6.09 (1H, d, J = 11.2 Hz), 5.28 (1H, dd, J = 2.4, 1.5 Hz), 4.35 (1H, t, J = 5.9 Hz), 4.15-4.07 (1H, m), 3.34 (1H, d, J = 10.2 Hz), 3.19 (1H, d, J = 11.7 Hz), 2.87 (1H, dd, J= 12.0, 3.7 Hz), 2.79 (1H, dd, J= 12.4, 4.1 Hz), 2.53-2.40 (3H, m), 2.35-2.23 (3H, m), 2.06-1.97 (2H, m), 1.93-1.28 (17H, m), 0.97 (3H, d, J = 6.3 Hz), 0.60 (3H, s).

Exact Mass = 481.32 (C₂₈H₄₂F₃NO₂) Obs. mass = 482.4 (M+H)

### [Example 239]

### Synthesis of (1R,2S,3S,Z)-2-methyl-5-(2-((1R,3aS,7aR,E)-7a-methyl-1-((R)-1-(4-(trifluoromethyl)piperidin-1-yl)propan-2-yl)octahydro-4H-inden-4-ylidene)ethylidene)-4-methylenecyclohexane-1,3-diol (Compound C012)

Compound C012 [15.8 mg, 0.0319 mmol] was obtained by processing in the same manner as in step 2 and step 3 of Example 92 using compound 27 [33 mg, 0.078 mmol] obtained in step 1 of Example 238, compound 7b [42.7 mg, 0.112 mmol], and tetrakis(triphenylphosphine)palladium(0) [15.0 mg, 0.013 mmol] as starting materials.

¹H-NMR (CD₃OD) δ: 6.33 (1H, d, J = 11.2 Hz), 6.11 (1H, d, J = 11.2 Hz), 5.23 (1H, d, J = 1.5 Hz), 4.23 (1H, d, J = 3.4 Hz), 3.73 (1H, td, J = 8.1, 4.4 Hz), 3.34 (1H, d, J = 10.0 Hz), 3.19 (1H, d, J = 11.7 Hz), 2.88 (1H, dd, J = 12.2, 3.9 Hz), 2.80 (1H, dd, J = 12.2, 3.9 Hz), 2.60 (1H, dd, J = 13.7, 4.4 Hz), 2.52-1.99 (8H, m), 1.89-1.29 (17H, m), 1.05 (3H, d, J = 6.8 Hz), 0.98 (3H, d, J = 6.3 Hz), 0.60 (3H, s).

Exact Mass = 495.33 (C₂₉H₄₄F₃NO₂) Obs. mass = 496.4 (M+H)

### [Example 240]

### Synthesis of (1R,3S,Z)-5-(2-((1R,3aS,7aR,E)-1-((R)-1-(4-(difluoromethyl)piperidin-1-yl)propan-2-yl)-7a-methyloctahydro-4H-inden-4-ylidene)ethylidene)-4-methylenecyclohexane-1,3-diol (Compound C017)

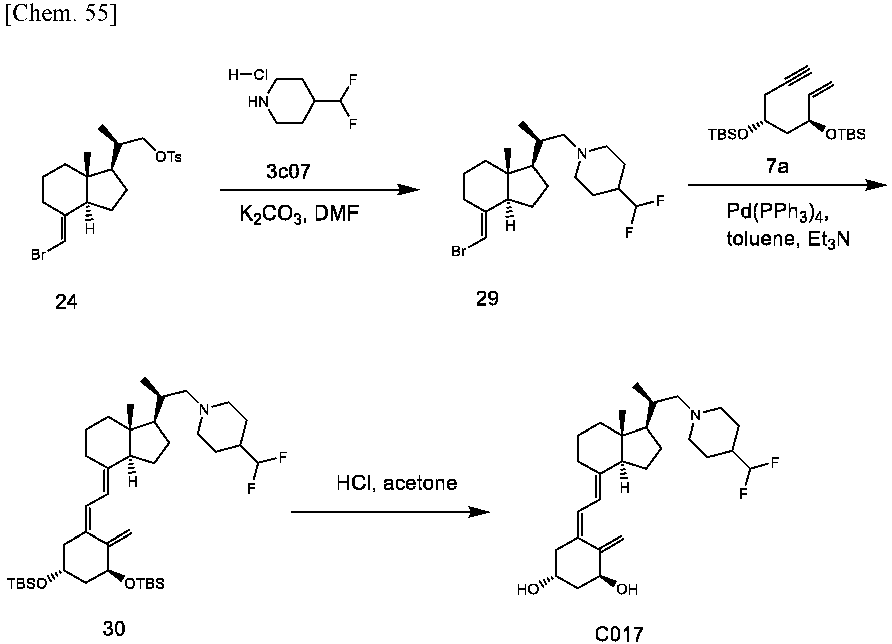

### Step 1

A solution of compound 24 [200 mg, 0.454 mmol], compound 3c07 [160 mg, 0.932 mmol], and potassium carbonate [250 mg, 1.81 mmol] in DMF [1 mL] was stirred at 80°C for 2 days. After cooling to room temperature, the reaction mixture was transferred to saturated brine and extracted with ethyl acetate. The organic layer was dried over anhydrous magnesium sulfate and concentrated under reduced pressure. The residue was purified by silica gel column chromatography to obtain compound 29 [58.6 mg, 0.145 mmol].

¹H-NMR (CDCl₃) δ: 5.65 (1H, t, J = 2.0 Hz), 5.56 (1H, td, J = 57.0, 4.0 Hz), 2.98-2.80 (3H, m), 2.35 (1H, dd, J= 12.2, 4.9 Hz), 2.00-1.61 (12H, m), 1.58-1.21 (9H, m), 0.90 (3H, d, J = 6.8 Hz), 0.51 (3H, s).

### Step 2 and step 3

Compound C017 [13.5 mg, 0.029 mmol] was obtained by processing in the same manner as in step 2 and step 3 of Example 92 using compound 29 [53 mg, 0.131 mmol] obtained in step 1 and (5R,7S)-2,2,3,3,9,9,10,10-octamethyl-5-(prop-2-yn-1-yl)-7-vinyl-4,8-dioxa-3,9-disilaundecane (Compound 3c04) [65 mg, 0.176 mmol], and tetrakis(triphenylphosphine)palladium(0) [23.0 mg, 0.020 mmol] as starting materials.

¹H-NMR (CD₃OD) δ: 6.31 (1H, d, J = 11.2 Hz), 6.09 (1H, d, J = 10.7 Hz), 5.76 (1H, td, J = 56.5, 4.1 Hz), 5.29 (1H, t, J = 1.2 Hz), 4.35 (1H, t, J = 5.9 Hz), 4.14-4.08 (1H, m), 3.47 (1H, d, J = 12.2 Hz), 3.00 (1H, dd, J = 12.4, 4.1 Hz), 2.87 (1H, dd, J = 12.3, 3.5 Hz), 2.75 (1H, td, J = 12.3, 2.8 Hz), 2.64 (1H, t, J = 11.7 Hz), 2.53 (2H, dd, J = 13.4, 11.0 Hz), 2.26 (1H, dd, J = 13.7, 6.8 Hz), 2.10-1.98 (3H, m), 1.91-1.31 (18H, m), 1.00 (3H, d, J = 6.8 Hz), 0.62 (3H, s).

Exact Mass = 463.33 (C₂₈H₄₃F₂NO₂) Obs. mass = 464.3 (M+H)

### [Example 241]

### Synthesis of (1R,3R)-5-(2-((1R,3aS,7aR,E)-1-((R)-1-(4-(difluoromethyl)piperidin-1-yl)propan-2-yl)-7a-methyloctahydro-4H-inden-4-ylidene)ethylidene)cyclohexane-1,3-diol (Compound C018)

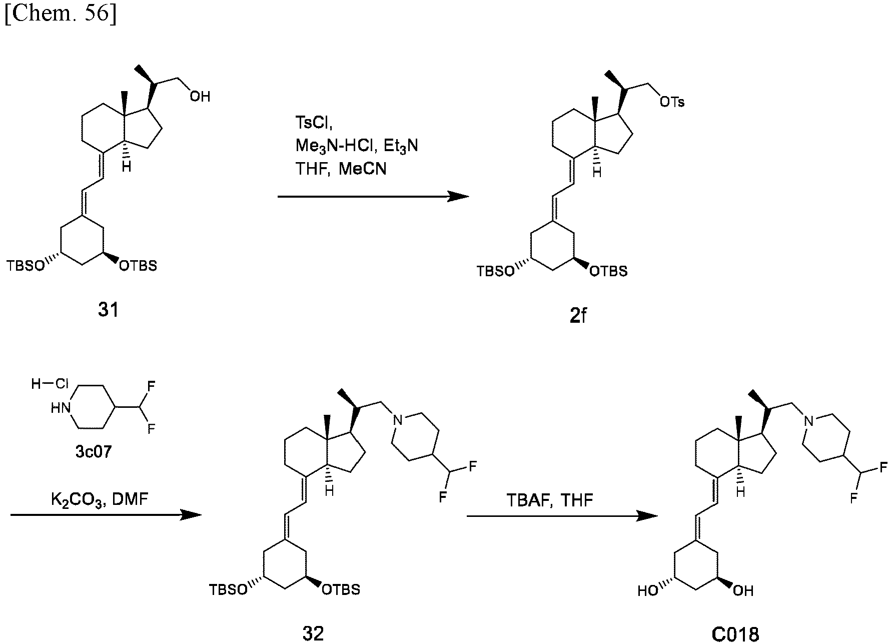

### Step 1

TsCl [40.7 mg, 0.213 mmol] was added to a mixed solution of (2R)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propan-1-ol (Compound 31, CAS Registry No. 161970-22-5) [80.2 mg, 0.142 mmol], trimethylamine hydrochloride [16.7 mg, 0.175 mmol], and triethylamine [0.08 mL, 0.57 mmol] in THF [1 mL]/acetonitrile [1 mL] at room temperature, and the reaction mixture was stirred at room temperature for 2 hours. The reaction mixture was quenched with methanol [0.2 mL] and concentrated under reduced pressure. The residue was diluted with ethyl acetate and washed with saturated brine. The organic layer was dried over anhydrous magnesium sulfate and concentrated under reduced pressure. The residue was purified by thin-layer chromatography [1 mm, heptane/EtOAc = 9/1] to obtain compound 2f [90.2 mg, 0.126 mmol].

¹H-NMR (CDCl₃) δ: 7.79 (2H, d, J = 8.3 Hz), 7.34 (2H, d, J = 8.3 Hz), 6.14 (1H, d, J = 11.2 Hz), 5.79 (1H, d, J = 11.2 Hz), 4.15-4.02 (4H, m), 3.82 (1H, dd, J = 9.3, 6.8 Hz), 2.85-2.75 (1H, m), 2.45 (3H, s), 2.36 (2H, dd, J = 13.4, 5.1 Hz), 2.25 (1H, dd, J = 13.7, 2.9 Hz), 2.09 (1H, dd, J = 13.2, 7.8 Hz), 1.95 (1H, t, J = 9.3 Hz), 1.88-1.71 (3H, m), 1.68-1.57 (4H, m), 1.55-1.18 (7H, m), 0.90 (3H, d, J = 6.3 Hz), 0.87 (9H, s), 0.85 (9H, s), 0.45 (3H, s), 0.05 (3H, s), 0.05 (3H, s), 0.04 (6H, s).

### Step 2

A solutions of compound 2f [40 mg, 0.056 mol] obtained in step 1, 4-difluoromethylpiperidine hydrochloride [3c09, 28.7 mg, 0.167 mmol], and potassium carbonate [38.5 mg, 0.279 mmol] in DMF [1 mL] was stirred at 60°C overnight. The reaction mixture was quenched with saturated brine and extracted with ethyl acetate. The organic layer was dried over anhydrous magnesium sulfate and concentrated under reduced pressure. The residue was partially purified by thin-layer chromatography [1 mm, heptane/EtOAc = 9/1] to obtain compound 32 [18.4 mg, 0.027 mmol].

Exact Mass = 679.50 (C₃₉H₇₁F₂NO₂Si₂) Obs. mass = 680.6 (M+H)

### Step 3

TBAF [1 M in THF, 0.5 mL, 0.5 mmol] was added to a solution of compound 32 [18.4 mg, 0.027 mmol] obtained in step 2 in THF [1 mL], and the mixture was stirred at 50°C overnight. The reaction mixture was quenched with a saturated aqueous sodium hydrogen carbonate solution and extracted with ethyl acetate. The organic layer was dried over anhydrous magnesium sulfate and concentrated under reduced pressure. The residue was purified by reverse phase HPLC to obtain compound C018 [6.3 mg, 0.014 mmol].

¹H-NMR (CD₃OD) δ: 6.21 (1H, d, J = 11.2 Hz), 5.90 (1H, d, J = 11.2 Hz), 5.75 (1H, td, J = 56.5, 4.0 Hz), 4.05-3.96 (2H, m), 3.41 (1H, d, J = 11.7 Hz), 3.26 (1H, d, J = 11.7 Hz), 2.93 (1H, dd, J = 12.4, 4.1 Hz), 2.85-1.97 (11H, m), 1.92-1.30 (16H, m), 0.99 (3H, d, J = 6.8 Hz), 0.62 (3H, d, J = 4.4 Hz).

Exact Mass = 451.33 (C₂₇H₄₃F₂NO₂) Obs. mass = 452.40 (M+H)

### [Example 242]

### Synthesis of (1R,3R)-5-(2-((1R,3aS,7aR,E)-1-((R)-1-(4-(difluoromethyl)piperidin-1-yl)propan-2-yl)-7a-methyloctahydro-4H-inden-4-ylidene)ethylidene)cyclohexane-1,3-diol (Compound C019)

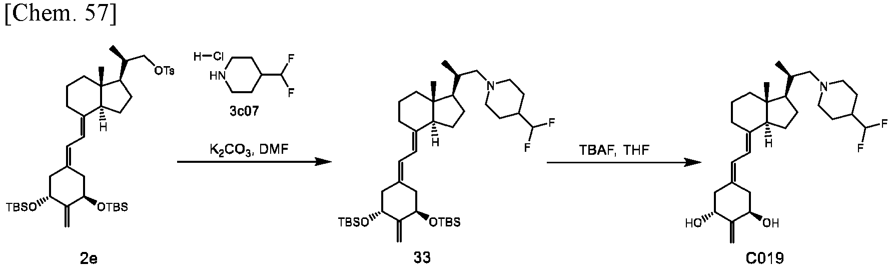

### Step 1

A solution of (2R)-2-((1R,3aS,7aR,E)-4-(2-((3R,5R)-3,5-bis((t-butyldimethylsilyl)oxy)-4-methylenecyclohexylidene)ethylidene)-7a-methyloctahydro-1H-inden-1-yl)propyl 4-methylbenzenesulfonate (Compound 2e, CAS Registry No. 1251827-23-2) [40 mg, 54.9 mmol], 4-difluoromethylpiperidine (Compound 3c07) [30.2 mg, 0.176 mmol], and potassium carbonate [40 mg, 0.289 mmol] in DMF [1 mL] was stirred at 60°C overnight. The reaction mixture was quenched with saturated brine and extracted with ethyl acetate. The organic layer was dried over anhydrous magnesium sulfate and concentrated under reduced pressure. The residue was partially purified by preparative thin-layer chromatography [1 mm, heptane/EtOAc = 9/1] to obtain compound 33 [19.0 mg, 0.0274 mmol].

Exact Mass = 691.50 (C₄oH₇₁F₂NO₂Si₂) Obs. mass = 692.6 (M+H)

### Step 2

TBAF [1 M in THF, 0.2 mL, 0.2 mmol] was added to a solution of compound 33 [19.0 mg, 0.0274 mmol] obtained in step 1 in THF [1 mL], and the mixture was stirred at 50°C overnight. The reaction mixture was quenched with a saturated aqueous sodium hydrogen carbonate solution and extracted with ethyl acetate. The organic layer was dried over anhydrous magnesium sulfate and concentrated under reduced pressure. The residue was purified by reverse phase HPLC to obtain compound C019 [6.0 mg, 0.013 mmol].

¹H-NMR (CD₃OD) δ: 6.26 (1H, d, J = 11.2 Hz), 5.92 (2H, d, J = 11.2 Hz), 5.75 (2H, td, J = 55.0, 4.0 Hz), 5.05 (2H, d, J = 6.3 Hz), 4.42-4.36 (2H, m), 3.43 (1H, d, J = 12.2 Hz), 2.94 (1H, dd, J = 12.7, 3.9 Hz), 2.86 (1H, dd, J = 12.4, 4.1 Hz), 2.69-2.24 (7H, m), 2.09-1.98 (3H, m), 1.89-1.28 (15H, m), 1.00 (3H, d, J = 6.3 Hz), 0.63 (3H, s).

Exact Mass = 463.33 (C₂₈H₄₃F₂NO₂) Obs. mass = 464.50 (M+H)

### [Example 243]

### Synthesis of (1R,2S,3S,Z)-5-(2-((1R,3aS,7aR,E)-1-((R)-1-(4-(difluoromethyl)piperidin-1-yl)propan-2-yl)-7a-methyloctahydro-4H-inden-4-ylidene)ethylidene)-2-methyl-4-methylenecyclohexane-1,3-diol (Compound C020)

Compound C020 [15.1 mg, 0.0316 mmol] was obtained by processing in the same manner as in step 2 and step 3 of Example 240 using compound 29 [34.7 mg, 0.086 mmol] obtained in step 1 of Example 240, compound 7b [44.5 mg, 0.116 mmol], and tetrakis(triphenylphosphine)palladium(0) [17.0 mg, 0.015 mmol] as starting materials.

¹H-NMR (CD₃OD) δ: 6.32 (1H, d, J = 11.2 Hz), 6.10 (1H, d, J = 11.2 Hz), 5.76 (1H, td, J = 56.4, 4.1 Hz), 5.22 (1H, d, J = 2.0 Hz), 4.88 (1H, d, J = 2.4 Hz), 4.22 (1H, d, J = 3.4 Hz), 3.72 (1H, td, J = 8.1, 4.2 Hz), 3.47 (1H, d, J = 12.2 Hz), 3.33 (1H, s), 2.99 (1H, dd, J = 12.4, 4.1 Hz), 2.87 (1H, dd, J = 12.0, 3.7 Hz), 2.78-2.71 (1H, m), 2.67-2.49 (3H, m), 2.20-1.97 (4H, m), 1.91-1.31 (17H, m), 1.04 (3H, d, J = 7.3 Hz), 0.99 (3H, d, J = 6.3 Hz), 0.61 (3H, s).

Exact Mass = 477.34 (C₂₉H₄₅F₂NO₂) Obs. mass = 478.40 (M+H)

### [Example 244]

### Synthesis of (1R,3S,Z)-5-(2-((1R,3aS,7aR,E)-1-((R)-1-(4-(difluoromethyl)-4-hydroxypiperidin-1-yl)propan-2-yl)-7a-methyloctahydro-4H-inden-4-ylidene)ethylidene)-4-methylenecyclohexane-1,3-diol (Compound C024)

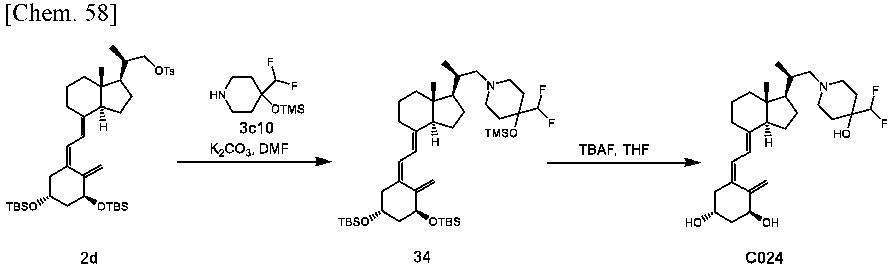

Compound C024 [17.9 mg, 0.0373 mmol] was obtained by performing the reaction in the same manner as in step 1 and step 2 of Example 242 using compound (2d) [80.0 mg, 0.110 mmol] and 4-(difluoromethyl)-4-((trimethylsilyl)oxy)piperidine [76.7 mg, 0.343 mmol].

¹H-NMR (CD₃OD) δ: 6.32 (1H, d, J = 10.7 Hz), 6.09 (1H, d, J = 11.2 Hz), 5.61 (1H, t, J = 56.1 Hz), 5.28 (1H, dd, J = 2.4, 1.5 Hz), 4.35 (1H, t, J = 5.9 Hz), 4.15-4.09 (1H, m), 3.24 (1H, d, J = 11.7 Hz), 3.09 (1H, d, J = 11.7 Hz), 2.99-2.85 (3H, m), 2.74-2.65 (1H, m), 2.59-2.49 (2H, m), 2.28-1.95 (5H, m), 1.91-1.28 (18H, m), 0.99 (3H, d, J = 6.3 Hz), 0.62 (3H, s).

Exact Mass = 479.32 (C₂₈H₄₃F₂NO₃) Obs. mass = 480.30 (M+H)

### [Example 245]

### Synthesis of (1R,3R)-5-(2-((1R,3aS,7aR,E)-1-((R)-1-(4-(difluoromethyl)-4-hydroxypiperidin-1-yl)propan-2-yl)-7a-methyloctahydro-4H-inden-4-ylidene)ethylidene)cyclohexane-1,3-diol (Compound C025)

Compound C025 [6.5 mg, 0.014 mmol] was obtained by performing the reaction in the same manner as in Example 242 using compound 2f [40 mg, 0.056 mmol] described in step 1 of Example 241 and 4-(difluoromethyl)-4-((trimethylsilyl)oxy)piperidine [37 mg, 0.166 mmol].

¹H-NMR (CD₃OD) δ: 6.21 (1H, d, J = 11.2 Hz), 5.90 (1H, d, J = 11.2 Hz), 5.61 (1H, t, J = 56.1 Hz), 4.06-3.95 (2H, m), 3.29-3.24 (1H, m), 3.11 (1H, d, J = 11.2 Hz), 3.00-2.56 (7H, m), 2.41 (1H, dd, J= 13.2, 3.4 Hz), 2.23-1.95 (7H, m), 1.88-1.28 (16H, m), 1.00 (3H, d, J = 6.3 Hz), 0.62 (3H, s).

Exact Mass = 467.32 (C₂₇H₄₃F₂NO₃) Obs. mass = 468.30 (M+H)

### [Example 246]

### Synthesis of (1R,3R)-5-(2-((1R,3aS,7aR,E)-1-((R)-1-(4-(difluoromethyl)-4-hydroxypiperidin-1-yl)propan-2-yl)-7a-methyloctahydro-4H-inden-4-ylidene)ethylidene)-2-methylenecyclohexane-1,3-diol (Compound C026)

Compound C026 [6.2 mg, 0.013 mmol] was obtained by performing the reaction in the same manner as in Example 242 using compound 2e [40 mg, 0.055 mmol] and 4-(difluoromethyl)-4-((trimethylsilyl)oxy)piperidine [40 mg, 0.171 mmol].

¹H-NMR (CD₃OD) δ: 6.26 (1H, d, J = 11.2 Hz), 5.92 (1H, d, J = 11.2 Hz), 5.62 (1H, t, J = 55.9 Hz), 5.05 (2H, d, J = 5.9 Hz), 4.42-4.36 (2H, m), 3.16 (1H, d, J = 12.2 Hz), 3.02 (2H, td, J = 12.3, 2.6 Hz), 2.88-2.61 (4H, m), 2.48 (1H, dd, J = 13.4, 4.1 Hz), 2.32-1.97 (6H, m), 1.90-1.31 (13H, m), 1.01 (3H, d, J = 6.3 Hz), 0.63 (3H, s).

Exact Mass = 479.32 (C₂₈H₄₃F₂NO₃) Obs. mass = 480.40 (M+H)

### [Example 247]

### Synthesis of (1R,2S,3S,Z)-5-(2-((1R,3aS,7aR,E)-1-((R)-1-(4-(difluoromethyl)-4-hydroxypiperidin-1-yl)propan-2-yl)-7a-methyloctahydro-4H-inden-4-ylidene)ethylidene)-2-methyl-4-methylenecyclohexane-1,3-diol (Compound C027)

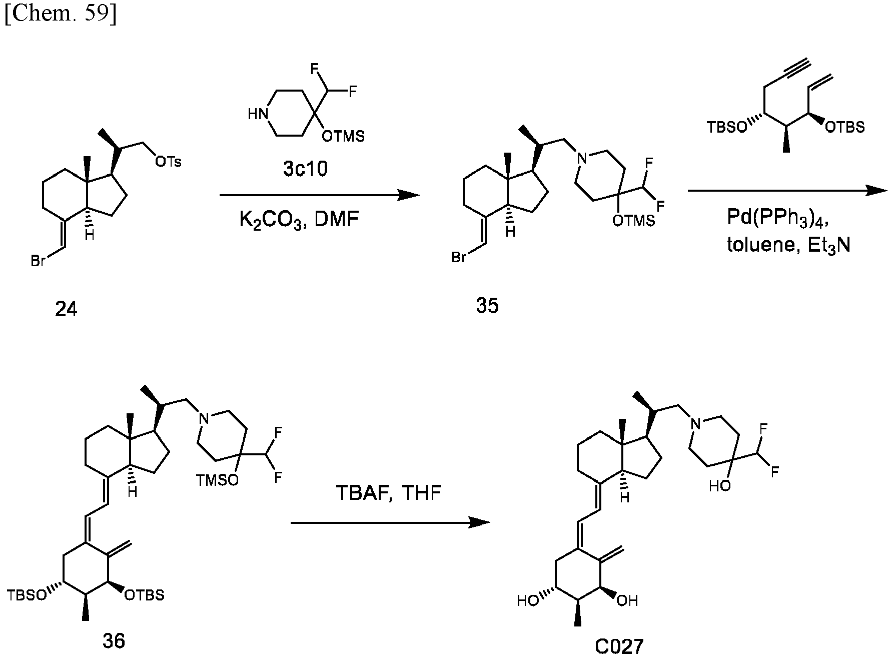

### Step 1

A solution of compound 24 [120 mg, 0.272 mmol], compound 3c10 [189 mg, 0.848 mmol], and potassium carbonate [183 mg, 1.32 mmol] in DMF [1 mL] was stirred at 60°C overnight. The reaction mixture was quenched with saturated brine and extracted with ethyl acetate. The organic layer was dried over anhydrous magnesium sulfate and concentrated under reduced pressure. The residue was purified by silica gel column chromatography to obtain compound 35 [76.9 mg, 0.156 mmol].

¹H-NMR (CDCl₃) δ: 5.65 (1H, s), 5.46 (1H, t, J = 56.1 Hz), 2.93-2.85 (1H, m), 2.63 (2H, dd, J = 35.4, 11.0 Hz), 2.45-2.22 (2H, m), 2.09-1.22 (25H, m), 0.90 (3H, d, J = 6.3 Hz), 0.58 (3H, s), 0.15 (9H, s).

### Step 2, step 3

A mixed solution of compound 35 [70.5 mg, 0.143 mmol] obtained in step 1, compound 7b [65.3 mg, 0.171 mmol], and tetrakis(triphenylphosphine)palladium(0) [25.0 mg, 0.022 mmol] in toluene [1 mL]/triethylamine [1 mL] was stirred at 100°C for 2 hours under a nitrogen atmosphere. After cooling to room temperature, the reaction mixture was concentrated under reduced pressure. The residue was partially purified by preparative thin-layer chromatography to obtain 94.5 mg of a crude product containing compound 36. TBAF [1 M in THF, 1 mL, 1 mmol] was added to a solution of the crude product in THF [1 mL], and the mixture was stirred at 50°C for 1 day. The reaction mixture was quenched with a saturated aqueous sodium hydrogen carbonate solution and extracted with ethyl acetate. The organic layer was dried over anhydrous magnesium sulfate and concentrated under reduced pressure. The residue was purified by reverse phase HPLC to obtain compound C027 [24.1 mg, 0.0488 mmol].

¹H-NMR (CD₃OD) δ: 6.32 (1H, d, J = 11.2 Hz), 6.09 (1H, d, J = 11.2 Hz), 5.51 (1H, t, J = 56.6 Hz), 5.22 (1H, d, J = 2.0 Hz), 4.22 (1H, d, J = 3.4 Hz), 3.72 (1H, td, J = 8.2, 4.2 Hz), 2.86 (1H, dd, J = 12.0, 3.2 Hz), 2.78 (1H, d, J = 11.2 Hz), 2.65-2.57 (2H, m), 2.47 (1H, dd, J = 12.2, 4.4 Hz), 2.36 (1H, t, J = 10.7 Hz), 2.19-1.28 (27H, m), 1.04 (3H, d, J = 6.8 Hz), 0.92 (3H, d, J = 6.3 Hz), 0.58 (3H, s).

Exact Mass = 493.34 (C₂₉H₄₅F₂NO₃) Obs. mass = 494.40 (M+H)

### [Example 248]

### Synthesis of (1R,3S,Z)-5-(2-((1R,3aS,7aR,E)-1-((R)-1-(4-hydroxy-4-methylpiperidin-1-yl)propan-2-yl)-7a-methyloctahydro-4H-inden-4-ylidene)ethylidene)-4-methylenecyclohexane-1,3-diol (Compound C029)

Compound C029 [17.4 mg, 0.039 mmol] was obtained by processing in the same manner as in Example 242 using compound 2d [80 mg, 0.110 mmol] and 4-hydroxy-4-methylpiperidine [45 mg, 0.391 mmol] as starting materials.

Exact Mass = 443.34 (C₂₈H₄₅NO₃) Obs. mass = 444.40 (M+H)

### [Example 249]

### Synthesis of (1R,3S,Z)-5-(2-((1R,3aS,7aR,E)-1-((2R)-1-(3-hydroxy-3-(trifluoromethyl)piperidin-1-yl)propan-2-yl)-7a-methyloctahydro-4H-inden-4-ylidene)ethylidene)-4-methylenecyclohexane-1,3-diol (Compound C033a, Compound C033b)

Compound C033a [10 mg, 0.020 mmol] and compound C033b [9.0 mg, 0.018 mmol] were obtained by processing in the same manner as in Example 108 using compound 2d [105.4 mg, 0.145 mmol] as a starting material instead of compound 2a in Example 108.

### Compound C033a

¹H-NMR (CD₃OD) δ: 6.31 (1H, d, J = 11.2 Hz), 6.08 (1H, d, J = 11.2 Hz), 5.28 (1H, dd, J = 2.4, 1.5 Hz), 4.34 (1H, t, J = 5.9 Hz), 4.14-4.09 (1H, m), 2.95-2.80 (3H, m), 2.63 (1H, dd, J = 12.2, 4.4 Hz), 2.51 (1H, dd, J = 13.4, 3.2 Hz), 2.28-2.19 (5H, m), 2.04-1.96 (3H, m), 1.90-1.28 (19H, m), 0.95 (3H, d, J = 6.3 Hz), 0.59 (3H, s).

Exact Mass = 497.31 (C₂₈H₄₂F₃NO₃) Obs. mass = 498.40 (M+H)

### Compound C033b

1H-NMR (CD₃OD) δ: 6.31 (1H, d, J = 10.7 Hz), 6.08 (1H, d, J = 11.2 Hz), 5.28 (1H, t, J = 1.2 Hz), 4.34 (1H, t, J = 5.9 Hz), 4.14-4.09 (1H, m), 2.87 (2H, d, J = 11.2 Hz), 2.71 (1H, d, J = 11.2 Hz), 2.56-2.48 (2H, m), 2.34 (1H, d, J = 11.7 Hz), 2.25 (1H, dd, J = 13.2, 6.8 Hz), 2.05-1.97 (4H, m), 1.90-1.25 (21H, m), 0.94 (3H, d, J = 6.8 Hz), 0.58 (3H, s).

Exact Mass = 497.31 (C₂₈H₄₂F₃NO₃) Obs. mass = 498.40 (M+H)

### [Example 250]

### Synthesis of (1R,3R)-5-(2-((1R,3aS,7aR,E)-1-((R)-1-(4-(2,2-difluoroethyl)piperidin-1-yl)propan-2-yl)-7a-methyloctahydro-4H-inden-4-ylidene)ethylidene)-2-methylenecyclohexane-1,3-diol (Compound C056)

Compound C056 [8.2 mg, 0.017 mmol] was obtained by processing in the same manner as in Example 242 using compound 2e [40 mg, 0.055 mmol] and 4-(2,2-difluoroethyl)piperidine hydrochloride [35 mg, 0.189 mmol] as starting materials.

¹H-NMR (CD₃OD) δ: 6.26 (1H, d, J = 11.2 Hz), 5.99 (2H, tt, J = 56.0, 4.0 Hz), 5.92 (2H, d, J = 11.2 Hz), 5.05 (2H, d, J = 5.9 Hz), 4.42-4.36 (2H, m), 3.49 (1H, d, J = 11.7 Hz), 3.35-3.30 (1H, m), 3.11 (1H, dd, J = 12.4, 4.1 Hz), 2.92-2.63 (5H, m), 2.48 (1H, dd, J = 13.2, 3.9 Hz), 2.32-1.94 (7H, m), 1.91-1.32 (14H, m), 1.02 (3H, d, J = 6.3 Hz), 0.63 (3H, s).

Exact Mass = 477.34 (C₂₉H₄₅F₂NO₂) Obs. mass = 478.40 (M+H)

### [Example 251]

### Synthesis of (1R,3R)-5-(2-((1R,3aS,7aR,E)-1-((R)-1-(4-(1,1-difluoroethyl)piperidin-1-yl)propan-2-yl)-7a-methyloctahydro-4H-inden-4-ylidene)ethylidene)-2-methylenecyclohexane-1,3-diol (Compound C057)

Compound C057 [6.7 mg, 0.014 mmol] was obtained by processing in the same manner as in Example 242 using compound 2e [40 mg, 0.055 mmol] and 4-(1,1-difluoroethyl)piperidine hydrochloride [37 mg, 0.200 mmol] as starting materials.

¹H-NMR (CD₃OD) δ: 6.26 (1H, d, J = 11.2 Hz), 5.92 (1H, d, J = 11.2 Hz), 5.05 (2H, t, J = 3.4 Hz), 4.42-4.36 (2H, m), 3.48 (1H, d, J = 12.2 Hz), 3.35-3.33 (1H, m), 2.99 (1H, dd, J = 12.7, 3.9 Hz), 2.86 (1H, dd, J = 12.0, 3.5 Hz), 2.79-1.94 (14H, m), 1.92-1.32 (17H, m), 1.01 (3H, d, J = 6.8 Hz), 0.63 (3H, s).

Exact Mass = 477.34 (C₂₉H₄₅F₂NO₂) Obs. mass = 478.40 (M+H)

### [Example 252]

### Synthesis of (1R,3S,Z)-5-(2-((1R,3aS,7aR,E)-1-((R)-1-(1-(4-fluoropiperidin-1-yl)propan-2-yl)-7a-methyloctahydro-4H-inden-4-ylidene)ethylidene)-4-methylenecyclohexane-1,3-diol (Compound C058)

Compound C058 [5.5 mg, 0.013 mmol] was obtained by processing in the same manner as in Example 242 using compound 2d [40 mg, 0.055 mmol] and 4-fluoropiperidine hydrochloride [23 mg, 0.165 mmol] as starting materials.

¹H-NMR (CD₃OD) δ: 6.31 (1H, d, J = 11.2 Hz), 6.08 (1H, d, J = 11.2 Hz), 5.28 (1H, dd, J = 2.4, 1.5 Hz), 4.89 (1H, s), 4.61 (1H, dt, J = 49.3, 3.4 Hz), 4.34 (1H, t, J = 5.9 Hz), 4.14-4.09 (1H, m), 2.86 (1H, dd, J = 12.0, 3.7 Hz), 2.64 (1H, s), 2.53-2.19 (7H, m), 2.07-1.96 (5H, m), 1.90-1.26 (17H, m), 0.92 (3H, d, J = 6.3 Hz), 0.58 (3H, s).

Exact Mass = 431.32 (C₂₇H₄₂FNO₂) Obs. mass = 432.30 (M+H)

### [Example 253]

### Synthesis of (1R,3R)-5-(2-((1R,3aS,7aR,E)-7a-methyl-1-((R)-1-(4-(trifluoromethyl)piperidin-1-yl)propan-2-yl)octahydro-4H-inden-4-ylidene)ethylidene)-2-methylenecyclohexane-1,3-diol (Compound C060)

Compound C060 [6.8 mg, 0.014 mmol] was obtained by processing in the same manner as in Example 242 using compound 2e [40 mg, 0.055 mmol] and 4-(trifluoromethyl)piperidine [30 mg, 0.196 mmol] as starting materials.

¹H-NMR (CD₃OD) δ: 6.26 (1H, d, J = 11.2 Hz), 5.92 (1H, d, J = 11.2 Hz), 5.05 (2H, d, J = 6.3 Hz), 4.42-4.36 (2H, m), 3.16 (1H, d, J = 11.7 Hz), 2.86 (1H, dd, J = 12.0, 3.7 Hz), 2.76 (1H, dd, J = 12.2, 4.4 Hz), 2.66 (1H, dd, J = 13.4, 4.1 Hz), 2.51-2.02 (8H, m), 1.93-1.28 (14H, m), 0.97 (3H, d, J = 6.3 Hz), 0.61 (3H, s).

Exact Mass = 481.32 (C₂₈H₄₂F₃NO₂) Obs. mass = 482.40 (M+H)

### [Example 254]

### Synthesis of (1R,2S,3S,Z)-2-methyl-5-(2-((1R,3aS,7aR,E)-7a-methyl-1-((R)-1-morpholinopropan-2-yl)octahydro-4H-inden-4-ylidene)ethylidene)-4-methylenecyclohexane-1,3-diol (Compound D003)

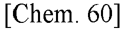

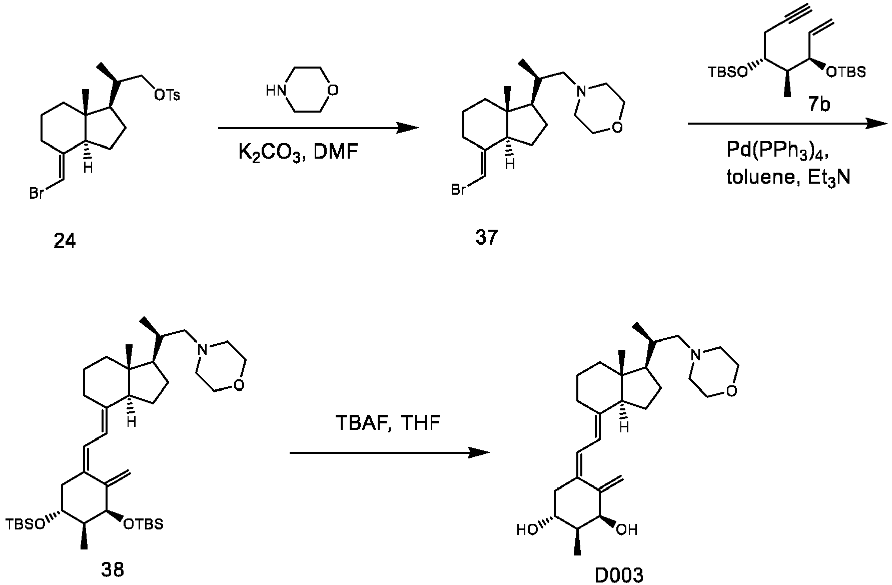

### Step 1

A solution of compound 24 [89.2 mg, 0.202 mmol], morpholine [0.1 mL, 1.16 mmol], and potassium carbonate [156 mg, 1.13 mmol] in DMF [1 mL] was stirred at 60°C for 17 hours. The reaction mixture was quenched with brine and extracted with ethyl acetate. The organic layer was dried over anhydrous magnesium sulfate and concentrated under reduced pressure. The residue was purified by silica gel column chromatography to obtain compound 37 [61.4 mg, 0.172 mmol].

¹H-NMR (CDCl₃) δ: 5.65 (1H, d, J = 2.0 Hz), 3.75-3.64 (4H, m), 2.91-2.83 (1H, m), 2.47-2.41 (2H, m), 2.38 (1H, dd, J = 11.7, 4.9 Hz), 2.33-2.24 (2H, m), 2.01-1.19 (12H, m), 0.92 (3H, d, J = 6.8 Hz), 0.57 (3H, s).

### Step 2 and step 3

Compound D003 [14.1 mg, 0.033 mmol] was obtained by processing in the same manner as in step 2 and step 3 of Example 236 using compound 37 [29.9 mg, 0.084 mmol], compound 7b [38.5 mg, 0.101 mmol], and tetrakis(triphenylphosphine)palladium(0) [11.0 mg, 0.009 mmol].

¹H-NMR (CD₃OD) δ: 6.33 (1H, d, J = 11.2 Hz), 6.10 (1H, d, J = 11.2 Hz), 5.23 (1H, d, J = 2.0 Hz), 4.23 (1H, d, J = 3.4 Hz), 3.78-3.68 (5H, m), 2.88 (1H, dd, J = 12.0, 3.7 Hz), 2.73-2.46 (7H, m), 2.27-2.10 (3H, m), 2.06-2.03 (1H, m), 2.01-1.97 (2H, m), 1.96-1.28 (14H, m), 1.04 (3H, d, J = 6.8 Hz), 0.96 (3H, d, J = 6.3 Hz), 0.60 (3H, s).

### [Example 255]

### Synthesis of (1R,3S,Z)-5-(2-((1R,3aS,7aR,E)-7a-methyl-1-((2R)-1-(3-(trifluoromethyl)piperazin-1-yl)propan-2-yl)octahydro-4H-inden-4-ylidene)ethylidene)-4-methylenecyclohexane-1,3-diol (Compound E008a, Compound E008b)

Compound E008a [3.3 mg, 0.007 mmol] and compound (E008b) [7.0 mg, 0.015 mmol] were obtained by performing the reaction in the same manner as in Example 108 using compound 24 [260 mg, 0.589 mmol] and 2-(trifluoromethyl)piperazine [190 mg, 1.23 mmol] as starting materials.

### Compound E008a

¹H-NMR (CD₃OD) δ: 6.31 (1H, d, J = 11.2 Hz), 6.08 (1H, d, J = 11.2 Hz), 5.28 (1H, t, J = 1.2 Hz), 4.34 (1H, t, J = 5.9 Hz), 4.15-4.08 (1H, m), 2.99-2.76 (7H, m), 2.53-1.21 (33H, m), 0.92 (3H, d, J = 6.8 Hz), 0.58 (3H, s).

Exact Mass = 482.31 (C₂₇H₄₁F₃N₂O₂) Obs. mass = 483.4 (M+H)

### Compound E008b

Exact Mass = 482.31 (C₂₇H₄₁F₃N₂O₂) Obs. mass = 483.4 (M+H)

### [Example 301]

### Synthesis of (1R,3S,Z)-5-(2-((1R,3aS,7aR,E)-7a-methyl-1-((S)-1-((S)-2-methylpiperidin-1-yl)propan-2-yl)octahydro-4H-inden-4-ylidene)ethylidene)-4-methylenecyclohexane-1,3-diol (Compound F001)

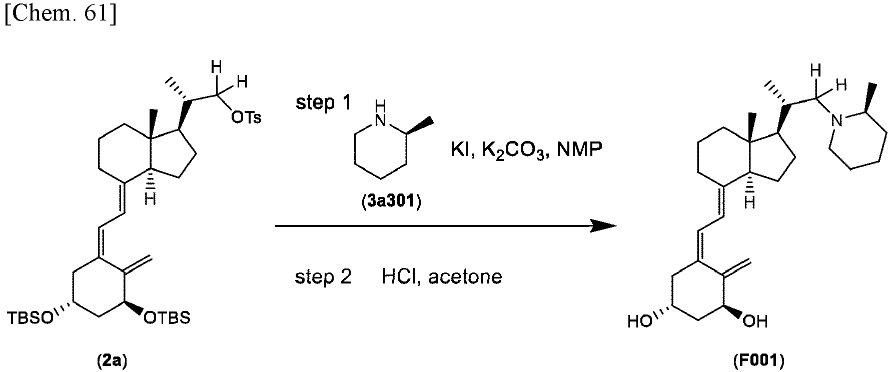

### Step 1

A solution of Compound (2a) [50 mg, 0.069 mmol] described in Reference example 1, (S)-2-methylpiperidine (3a301) [24.8 µL, 0.206 mmol], potassium iodide [22.8 mg, 0.137 mmol], and potassium carbonate [28.4 mg, 0.206 mmol] in NMP [1 mL] was heated and stirred at 60°C overnight. The reaction mixture was cooled to room temperature, saturated brine was added to the mixture, and the mixture was extracted with ethyl acetate. The organic layer was dried over anhydrous magnesium sulfate and concentrated under reduced pressure to obtain a crude containing coupling product.

### Step 2

2 M Hydrochloric acid [0.2 mL, 0.4 mmol] was added to a solution of the coupling product obtained in step 1 in acetone [0.6 mL], and the mixture was stirred at room temperature for 4 hours. The reaction mixture was neutralized with a saturated aqueous sodium hydrogen carbonate solution and extracted with ethyl acetate. The organic layer was washed with saturated brine, dried over anhydrous magnesium sulfate, and concentrated under reduced pressure. The residue was purified by preparative HPLC to obtain compound F001 [5.4 mg, 0.013 mmol].

¹H-NMR (CD₃OD) δ: 6.32 (1H, d, J = 11.0 Hz), 6.10 (1H, d, J = 11.4 Hz), 5.30-5.27 (1H, m), 4.35 (1H, t, J = 5.7 Hz), 4.15-4.10 (1H, m), 3.56-3.52 (1H, m), 3.27-3.25 (1H, m), 3.08-3.03 (1H, m), 2.92-2.87 (3H, m), 2.51 (1H, dd, J = 13.5, 3.4 Hz), 2.26 (1H, dd, J = 13.5, 6.6 Hz), 2.07-2.04 (2H, m), 1.90-1.86 (4H, m), 1.83-1.51 (11H, m), 1.43-1.28 (4H, m), 1.37 (3H, d, J = 9.0 Hz), 1.12 (3H, d, J = 6.4 Hz), 0.65 (3H, s).

LC-MS: Exact Mass = 427.35 (C₂₈H₄₅NO₂) Obs. mass = 428.25 (M+H)

In the following examples, each compound was synthesized in the same manner as in the synthesis method for the compound described in Example 301. In each example, only the raw material and the amine compound used are described. Potassium iodide and potassium carbonate are used as in Example 301, and the equivalent amount thereof is appropriately altered depending on the raw material to be used according to the conditions of Example 301.

### [Example 302]

### Synthesis of (1R,3S,Z)-5-(2-((1R,3aS,7aR,E)-7a-methyl-1-((S)-1-((R)-2-methylpiperidin-1-yl)propan-2-yl)octahydro-4H-inden-4-ylidene)ethylidene)-4-methylenecyclohexane-1,3-diol (Compound F002)

Compound F002 [19.2 mg, 0.0449 mmol] was obtained by performing the reaction in the same manner as in Example 301 using compound (2a) [50 mg, 0.069 mmol] and (R)-2-methylpiperidine [24.8 µL, 0.206 mmol].

¹H-NMR (CD₃OD): 6.32 (1H, d, J = 11.0 Hz), 6.10 (1H, d, J = 11.0 Hz), 5.29-5.28 (1H, m), 4.35 (1H, t, J = 5.9 Hz), 4.15-4.10 (1H, m), 3.42-3.39 (2H, m), 3.25-3.23 (1H, m), 3.12-3.09 (1H, m), 2.88 (1H, dd, J = 11.9, 3.7 Hz), 2.67 (1H, dd, J = 13.3, 10.5 Hz), 2.51 (1H, dd, J = 13.3, 3.7 Hz), 2.26 (1H, dd, J = 13.5, 6.7 Hz), 2.09-1.96 (4H, m), 1.91-1.51 (15H, m), 1.42-1.38 (3H, m), 1.36 (4H, d, J = 6.4 Hz), 1.15 (3H, d, J = 6.4 Hz), 0.65 (3H, s).

LC-MS: Exact Mass = 427.35 (C₂₈H₄₅NO₂) Obs. mass = 428.20 (M+H)

### [Example 303]

### Synthesis of (1R,3S,Z)-5-(2-((1R,3aS,7aR,E)-1-((S)-1-(2,2-dimethylpiperidin-1-yl)propan-2-yl)-7a-methyloctahydro-4H-inden-4-ylidene)ethylidene)-4-methylenecyclohexane-1,3-diol (Compound F003)

Compound F003 [8.2 mg, 0.019 mmol] was obtained by performing the reaction in the same manner as in Example 301 using compound (2a) [50 mg, 0.069 mmol] and 2,2-dimethylpiperidine hydrochloride [30.79 mg, 0.206 mmol].

¹H-NMR (CD₃OD) δ: 6.32 (1H, d, J = 11.0 Hz), 6.10 (1H, d, J = 11.0 Hz), 5.29-5.28 (1H, m), 4.35 (1H, t, J = 5.9 Hz), 4.14-4.10 (1H, m), 3.46-3.44 (1H, m), 3.14-3.11 (1H, m), 2.88 (1H, dd, J = 11.9, 4.1 Hz), 2.51 (1H, dd, J = 14.2, 4.6 Hz), 2.26 (1H, dd, J = 13.3, 6.9 Hz), 2.08-1.96 (4H, m), 1.91-1.70 (12H, m), 1.62-1.51 (3H, m), 1.45-1.36 (10H, m), 1.17 (3H, d, J = 6.4 Hz), 0.65 (3H, s).

LC-MS: Exact Mass = 441.36 (C₂₉H₄₇NO₂) Obs. mass = 442.25 (M+H)

### [Example 304]

### Synthesis of (1R,3S,Z)-5-(2-((1R,3aS,7aR,E)-7a-methyl-1-((S)-1-((S)-3-methylmorpholino)propan-2-yl)octahydro-4H-inden-4-ylidene)ethylidene)-4-methylenecyclohexane-1,3-diol (Compound F004)

Compound F004 [6.2 mg, 0.014 mmol] was obtained by performing the reaction in the same manner as in Example 301 using compound (2a) [50 mg, 0.069 mmol] and (S)-3-methylmorpholine [20.8 mg, 0.206 mmol].

¹H-NMR (CD₃OD) δ: 6.32 (1H, d, J = 11.0 Hz), 6.08 (1H, d, J = 11.0 Hz), 5.28 (1H, s), 4.89 (1H, d, J = 1.8 Hz), 4.34 (1H, t, J = 5.9 Hz), 4.15-4.10 (1H, m), 3.77 (1H, d, J = 11.4 Hz), 3.67-3.56 (2H, m), 3.26-3.20 (1H, m), 2.88-2.84 (2H, m), 2.51 (1H, dd, J = 13.5, 3.4 Hz), 2.41 (1H, t, J = 11.7 Hz), 2.37-2.31 (1H, m), 2.25 (1H, dd, J = 13.7, 6.9 Hz), 2.15-1.87 (8H, m), 1.73-1.21 (9H, m), 1.02 (3H, d, J = 6.4 Hz), 0.95 (3H, d, J = 6.4 Hz), 0.60 (3H, s).

LC-MS: Exact Mass = 429.32 (C₂₇H₄₃NO₃) Obs. mass = 430.25 (M+H)

### [Example 305]

### Synthesis of (1R,3S,Z)-5-(2-((1R,3aS,7aR,E)-7a-methyl-1-((S)-1-((R)-3-methylmorpholino)propan-2-yl)octahydro-4H-inden-4-ylidene)ethylidene)-4-methylenecyclohexane-1,3-diol (Compound F005)

Compound F005 [9.6 mg, 0.022 mmol] was obtained by performing the reaction in the same manner as in Example 301 using compound (2a) [50 mg, 0.069 mmol] and (R)-3-methylmorpholine [20.8 mg, 0.206 mmol].

¹H-NMR (CD₃OD) δ: 6.32 (1H, d, J = 11.0 Hz), 6.09 (1H, d, J = 11.0 Hz), 5.29-5.28 (1H, m), 4.34 (1H, t, J = 5.9 Hz), 4.13-4.10 (1H, m), 3.77-3.69 (3H, m), 3.39 (1H, dd, J = 11.7, 7.5 Hz), 2.94-2.76 (4H, m), 2.71-2.65 (1H, m), 2.51 (1H, dd, J = 13.5, 3.4 Hz), 2.26 (1H, dd, J = 13.5, 6.6 Hz), 2.12-1.99 (12H, m), 1.88 (2H, t, J = 5.5 Hz), 1.73-1.26 (10H, m), 1.10 (3H, d, J = 6.9 Hz), 1.08 (3H, d, J = 6.4 Hz), 0.60 (3H, s).

LC-MS: Exact Mass = 429.32 (C₂₇H₄₃NO₃) Obs. mass = 430.25 (M+H)

### [Example 306]

### Synthesis of (1R,3S,Z)-5-(2-((1R,3aS,7aR,E)-1-((S)-1-(3,3-dimethylmorpholino)propan-2-yl)-7a-methyloctahydro-4H-inden-4-ylidene)ethylidene)-4-methylenecyclohexane-1,3-diol (Compound F006)

Compound F006 [4.7 mg, 0.011 mmol] was obtained by performing the reaction in the same manner as in Example 301 using compound (2a) [50 mg, 0.069 mmol] and 3,3-dimethylmorpholine [15.8 mg, 0.137 mmol].

¹H-NMR (CD₃OD) δ: 6.32 (1H, d, J = 11.0 Hz), 6.08 (1H, d, J = 11.4 Hz), 5.29-5.27 (1H, m), 4.34 (1H, t, J = 5.9 Hz), 4.15-4.09 (1H, m), 3.84-3.80 (1H, m), 3.69-3.62 (1H, m), 3.45-3.34 (2H, m), 2.89-2.80 (2H, m), 2.74-2.67 (1H, m), 2.51 (1H, dd, J = 13.3, 3.7 Hz), 2.39-2.35 (2H, m), 2.26 (1H, dd, J = 13.3, 6.9 Hz), 2.07-1.99 (3H, m), 1.90-1.87 (2H, m), 1.72-1.65 (2H, m), 1.58-1.45 (4H, m), 1.38-1.25 (3H, m), 1.11 (3H, s), 1.07-1.03 (6H, m), 0.60 (3H, s).

LC-MS: Exact Mass = 443.34 (C₂₈H₄₅NO₃) Obs. mass = 444.20 (M+H)

### [Example 307]

### Synthesis of (1R,3S,Z)-5-(2-((1R,3aS,7aR,E)-1-((S)-1-(7-oxa-4-azaspiro[2.5]octan-4-yl)propan-2-yl)-7a-methyloctahydro-4H-inden-4-ylidene)ethylidene)-4-methylenecyclohexane-1,3-diol (Compound F007)

Compound F007 [6.1 mg, 0.014 mmol] was obtained by performing the reaction in the same manner as in Example 301 using compound (2a) [50 mg, 0.069 mmol] and 7-oxa-4-azaspiro[2.5]octane hydrochloride [20 mg, 0.134 mmol].

¹H-NMR (CD₃OD): 6.31 (1H, d, J = 11.0 Hz), 6.08 (1H, d, J = 11.0 Hz), 5.28 (1H, dd, J = 2.0, 1.1 Hz), 4.89 (1H, d, J = 2.0 Hz), 4.34 (1H, t, J = 5.9 Hz), 4.15-4.11 (1H, m), 3.83-3.77 (1H, m), 3.65 (1H, d, J = 11.4 Hz), 3.55 (1H, dt, J = 11.0, 4.0 Hz), 3.17 (1H, d, J = 11.4 Hz), 2.98 (2H, t, J = 11.2 Hz), 2.86 (1H, dd, J = 12.0, 4.0 Hz), 2.75 (1H, ddd, J = 13.5, 4.6, 3.0 Hz), 2.51 (1H, dd, J = 13.3, 3.7 Hz), 2.25 (1H, dd, J = 13.3, 6.9 Hz), 2.18-1.26 (22H, m), 1.21-0.90 (3H, m), 0.93 (3H, d, J = 6.4 Hz), 0.74-0.69 (1H, m), 0.60-0.41 (2H, m), 0.57 (3H, s).

LC-MS: Exact Mass = 441.32 (C₂₈H₄₃NO₃) Obs. mass = 442.20 (M+H)

### [Example 308]

### Synthesis of (1R,3S,Z)-5-(2-((1R,3aS,7aR,E)-1-((S)-1-((S)-3-cyclopropylmorpholino)propan-2-yl)-7a-methyloctahydro-4H-inden-4-ylidene)ethylidene)-4-methylenecyclohexane-1,3-diol (Compound F008)

Compound F008 [2.5 mg, 0.0055 mmol] was obtained by performing the reaction in the same manner as in Example 301 using compound (2a) [50 mg, 0.069 mmol] and (S)-3-cyclopropylmorpholine [26.2 mg, 0.206 mmol].

¹H-NMR (CD₃OD) δ: 6.32 (1H, d, J = 11.0 Hz), 6.09 (1H, d, J = 11.0 Hz), 5.28 (1H, s), 4.35 (1H, t, J = 5.7 Hz), 4.13-4.11 (1H, m), 3.77 (2H, dd, J = 11.0, 3.2 Hz), 3.58 (1H, dd, J = 11.4, 9.1 Hz), 3.41-3.35 (1H, m), 3.02-2.82 (3H, m), 2.51 (1H, dd, J = 13.5, 3.4 Hz), 2.27-2.24 (1H, m), 2.10-2.02 (5H, m), 1.89-1.86 (3H, m), 1.73-1.25 (11H, m), 1.06 (3H, d, J = 6.4 Hz), 0.70-0.66 (1H, m), 0.60 (3H, s), 0.51-0.44 (2H, m), 0.29-0.24 (1H, m), 0.11-0.07 (1H, m).

LC-MS: Exact Mass = 455.34 (C₂₉H₄₅NO₃) Obs. mass = 456.20 (M+H)

### [Example 309]

### Synthesis of (1R,3S,Z)-5-(2-((1R,3aS,7aR,E)-1-((S)-1-((S)-3-isopropylmorpholino)propan-2-yl)-7a-methyloctahydro-4H-inden-4-ylidene)ethylidene)-4-methylenecyclohexane-1,3-diol (Compound F009)

Compound F009 [8.9 mg, 0.019 mmol] was obtained by performing the reaction in the same manner as in Example 301 using compound (2a) [50 mg, 0.069 mmol] and (S)-3-isopropylmorpholine [27.0 mg, 0.210 mmol].

LC-MS: Exact Mass = 457.36 (C₂₉H₄₇NO₃) Obs. mass = 458.25 (M+H)

### [Example 310]

### Synthesis of (1R,3S,Z)-5-(2-((1R,3aS,7aR,E)-1-((S)-1-((R)-3-isopropylmorpholino)propan-2-yl)-7a-methyloctahydro-4H-inden-4-ylidene)ethylidene)-4-methylenecyclohexane-1,3-diol (Compound F010)

Compound F010 [8.2 mg, 0.018 mmol] was obtained by performing the reaction in the same manner as in Example 301 using compound (2a) [50 mg, 0.069 mmol] and (R)-3-isopropylmorpholine [26.6 mg, 0.206 mmol].

LC-MS: Exact Mass = 457.36 (C₂₉H₄₇NO₃) Obs. mass = 458.25 (M+H)

### [Example 311]

### Synthesis of (1R,3S,Z)-5-(2-((1R,3aS,7aR,E)-1-((S)-1-((S)-2,4-dimethylpiperazin-1-yl)propan-2-yl)-7a-methyloctahydro-4H-inden-4-ylidene)ethylidene)-4-methylenecyclohexane-1,3-diol (Compound F011)

Compound F011 [15.2 mg, 0.0343 mmol] was obtained by performing the reaction in the same manner as in Example 301 using compound (2a) [50 mg, 0.069 mmol] and (S)-2,4-dimethylpiperazine dihydrochloride [38.5 mg, 0.206 mmol].

¹H-NMR (CD₃OD) δ: 6.32 (1H, d, J = 11.0 Hz), 6.08 (1H, d, J = 11.4 Hz), 5.30-5.27 (1H, m), 4.34 (1H, t, J = 5.9 Hz), 4.13-4.11 (1H, m), 3.15-3.02 (3H, m), 2.87 (1H, d, J = 11.9 Hz), 2.66 (1H, t, J = 10.5 Hz), 2.56 (4H, s), 2.51 (2H, d, J = 11.4 Hz), 2.42 (1H, t, J = 10.7 Hz), 2.30-2.16 (3H, m), 2.08-1.99 (3H, m), 1.90-1.86 (2H, m), 1.73-1.26 (9H, m), 1.11 (3H, d, J = 5.9 Hz), 1.03 (3H, d, J = 6.4 Hz), 0.61 (3H, s).

LC-MS: Exact Mass = 442.36 (C₂₈H₄₆N₂O₂) Obs. mass = 443.20 (M+H)

### [Example 312]

### Synthesis of (1R,3S,Z)-5-(2-((1R,3aS,7aR,E)-1-((S)-1-((R)-2,4-dimethylpiperazin-1-yl)propan-2-yl)-7a-methyloctahydro-4H-inden-4-ylidene)ethylidene)-4-methylenecyclohexane-1,3-diol (Compound F012)

Compound F012 [18.7 mg, 0.0422 mmol] was obtained by performing the reaction in the same manner as in Example 301 using compound (2a) [50 mg, 0.069 mmol] and (R)-2,4-dimethylpiperazine dihydrochloride [38.5 mg, 0.206 mmol].

¹H-NMR (CD₃OD) δ: 6.31 (1H, d, J = 11.4 Hz), 6.08 (1H, d, J = 11.0 Hz), 5.28 (1H, s), 4.34 (1H, t, J = 5.9 Hz), 4.13-4.11 (1H, m), 2.97-2.62 (8H, m), 2.55 (3H, s), 2.53-2.46 (2H, m), 2.26 (1H, dd, J = 13.5, 6.6 Hz), 2.06-1.98 (4H, m), 1.89-1.87 (2H, m), 1.71-1.66 (2H, m), 1.57-1.21 (7H, m), 1.13 (3H, d, J = 6.4 Hz), 1.04 (3H, d, J = 6.4 Hz), 0.59 (3H, s).

LC-MS: Exact Mass = 442.36 (C₂₈H₄₆N₂O₂) Obs. mass = 443.20 (M+H)

### [Example 313]

### Synthesis of (1R,3S,Z)-5-(2-((1R,3aS,7aR,E)-1-((S)-1-((R)-2-(2-hydroxypropan-2-yl)pyrrolidin-1-yl)propan-2-yl)-7a-methyloctahydro-4H-inden-4-ylidene)ethylidene)-4-methylenecyclohexane-1,3-diol (Compound F013)

Compound F013 [2.0 mg, 0.0044 mmol] was obtained by performing the reaction in the same manner as in Example 301 using compound (2a) [50 mg, 0.069 mmol] and (R)-2-(pyrrolidin-2-yl)propan-2-ol hydrochloride [34.1 mg, 0.206 mmol].

¹H-NMR (CD₃OD) δ: 6.32 (1H, d, J = 11.0 Hz), 6.09 (1H, d, J = 11.4 Hz), 5.29-5.27 (1H, m), 4.34 (1H, t, J = 5.9 Hz), 4.14-4.11 (1H, m), 3.41 (1H, s), 3.20 (1H, s), 2.99-2.86 (4H, m), 2.51 (1H, dd, J = 13.5, 3.4 Hz), 2.26 (1H, dd, J = 13.3, 6.4 Hz), 2.06-1.94 (6H, m), 1.88-1.85 (3H, m), 1.74-1.68 (3H, m), 1.59-1.51 (3H, m), 1.40-1.31 (3H, m), 1.27 (3H, s), 1.24 (3H, s), 1.18 (3H, d, J = 6.4 Hz), 0.63 (3H, s).

LC-MS: Exact Mass = 457.36 (C₂₉H₄₇NO₃) Obs. mass = 458.20 (M+H)

### [Example 314]

### Synthesis of (1R,3S,Z)-5-(2-((1R,3aS,7aR,E)-1-((S)-1-((S)-2-(2-hydroxypropan-2-yl)pyrrolidin-1-yl)propan-2-yl)-7a-methyloctahydro-4H-inden-4-ylidene)ethylidene)-4-methylenecyclohexane-1,3-diol (Compound F014)

Compound F014 [20.5 mg, 0.0448 mmol] was obtained by performing the reaction in the same manner as in Example 301 using compound (2a) [50 mg, 0.069 mmol] and (S)-2-(pyrrolidin-2-yl)propan-2-ol hydrochloride [34.1 mg, 0.206 mmol].

¹H-NMR (CD₃OD) δ: 6.32 (1H, d, J = 11.4 Hz), 6.09 (1H, d, J = 11.0 Hz), 5.30-5.28 (1H, m), 4.34 (1H, t, J = 5.9 Hz), 4.15-4.10 (1H, m), 3.59-3.54 (1H, m), 3.49-3.39 (2H, m), 3.25-3.19 (1H, m), 2.89-2.79 (2H, m), 2.51 (1H, dd, J = 13.3, 3.2 Hz), 2.29-1.95 (9H, m), 1.90-1.87 (3H, m), 1.73-1.68 (2H, m), 1.60-1.50 (3H, m), 1.41-1.35 (3H, m), 1.29 (3H, s), 1.25 (3H, s), 1.19 (3H, d, J = 6.4 Hz), 0.64 (3H, s).

LC-MS: Exact Mass = 457.36 (C₂₉H₄₇NO₃) Obs. mass = 458.20 (M+H)

### [Example 315]

### Synthesis of (1R,3S,Z)-5-(2-((1R,3aS,7aR,E)-1-((S)-1-((R)-2-(2-methoxypropan-2-yl)pyrrolidin-1-yl)propan-2-yl)-7a-methyloctahydro-4H-inden-4-ylidene)ethylidene)-4-methylenecyclohexane-1,3-diol (Compound F015)

Compound F015 [18.4 mg, 0.039 mmol] was obtained by performing the reaction in the same manner as in Example 301 using compound (2a) [50 mg, 0.069 mmol] and (R)-2-(1-methoxy-1-methyl-ethyl)pyrrolidine [30 mg, 0.209 mmol].

¹H-NMR (CD₃OD): 6.23 (1H, d, J = 11.0 Hz), 6.02 (1H, d, J = 11.0 Hz), 5.26 (1H, s), 4.79 (1H, s), 4.25-4.20 (1H, m), 4.01 (1H, s), 3.12 (3H, s), 3.01-2.81 (2H, m), 2.54-2.49 (32H, m), 2.37 (2H, dd, J = 21.3, 11.2 Hz), 2.20 (1H, dd, J = 13.5, 5.7 Hz), 2.13-1.14 (20H, m), 1.10 (3H, s), 1.06 (3H, s), 1.03 (3H, s), 0.56 (3H, s).

LC-MS: Exact Mass = 471.37 (C₃₀H₄₉NO₃) Obs. mass = 472.20 (M+H)

### [Example 316]

### Synthesis of (1R,3S,Z)-5-(2-((1R,3aS,7aR,E)-1-((S)-1-((S)-2-(2-methoxypropan-2-yl)pyrrolidin-1-yl)propan-2-yl)-7a-methyloctahydro-4H-inden-4-ylidene)ethylidene)-4-methylenecyclohexane-1,3-diol (Compound F016)

Compound F016 [25 mg, 0.053 mmol] was obtained by performing the reaction in the same manner as in Example 301 using compound (2a) [50 mg, 0.069 mmol] and (S)-2-(1-methoxy-1-methyl-ethyl)pyrrolidine hydrochloride [37.0 mg, 0.209 mmol]. ¹H-NMR (CD₃OD) δ: 6.32 (1H, d, J = 11.0 Hz), 6.10 (1H, d, J = 11.0 Hz), 5.30-5.27 (1H, m), 4.89-4.88 (1H, m), 4.35 (1H, t, J = 5.9 Hz), 4.14-4.11 (1H, m), 3.60-3.52 (2H, m), 3.35 (1H, d, J = 12.8 Hz), 3.27-3.21 (1H, m), 2.90-2.78 (2H, m), 2.51 (1H, dd, J = 13.7, 3.7 Hz), 2.29-2.18 (2H, m), 2.10-1.97 (7H, m), 1.90-1.87 (2H, m), 1.73-1.68 (2H, m), 1.63-1.50 (3H, m), 1.42-1.35 (3H, m), 1.26 (3H, s), 1.25 (3H, s), 1.18 (3H, d, J = 6.4 Hz), 0.64 (3H, s).

LC-MS: Exact Mass = 471.37 (C₃₀H₄₉NO₃) Obs. mass = 472.20 (M+H)

### [Example 317]

### Synthesis of (1R,3R)-5-(2-((1R,3aS,7aR,E)-7a-methyl-1-((S)-1-((S)-2-methylpiperidin-1-yl)propan-2-yl)octahydro-4H-inden-4-ylidene)ethylidene)cyclohexane-1,3-diol

### (Compound G001)

Compound G001 [5.7 mg, 0.014 mmol] was obtained by performing the reaction in the same manner as in Example 301 using compound (2b) [50 mg, 0.070 mmol] and (S)-2-methylpiperidine [13.8 mg, 0.139 mmol].

¹H-NMR (CD₃OD) δ: 6.21 (1H, d, J = 11.0 Hz), 5.90 (1H, d, J = 11.0 Hz), 4.06-3.94 (2H, m), 3.55 (1H, d, J = 11.4 Hz), 3.27 (1H, s), 3.08-3.03 (1H, m), 2.93-2.83 (3H, m), 2.59 (1H, dd, J = 13.3, 3.7 Hz), 2.41 (1H, dd, J = 13.3, 3.2 Hz), 2.23-2.14 (2H, m), 2.10-2.03 (2H, m), 2.01-1.96 (1H, m), 1.89-1.88 (1H, m), 1.84-1.52 (11H, m), 1.43-1.32 (6H, m), 1.12 (3H, d, J = 6.4 Hz), 0.65 (3H, s).

LC-MS: Exact Mass = 415.35 (C₂₇H₄₅NO₂) Obs. mass = 416.20 (M+H)

### [Example 318]

### Synthesis of (1R,3R)-5-(2-((1R,3aS,7aR,E)-7a-methyl-1-((S)-1-((R)-2-methylpiperidin-1-yl)propan-2-yl)octahydro-4H-inden-4-ylidene)ethylidene)cyclohexane-1,3-diol (Compound G002)

Compound G002 [3.4 mg, 0.0082 mmol] was obtained by performing the reaction in the same manner as in Example 301 using compound (2b) [50 mg, 0.070 mmol] and (R)-2-methylpiperidine [13.8 mg, 0.139 mmol].

¹H-NMR (CD₃OD) δ: 6.21 (1H, d, J = 11.4 Hz), 5.91 (1H, d, J = 11.0 Hz), 4.05-3.94 (2H, m), 3.45-3.41 (2H, m), 3.25 (1H, s), 3.13-3.09 (1H, m), 2.87-2.83 (1H, m), 2.68 (1H, dd, J = 13.3, 10.5 Hz), 2.59 (1H, dd, J = 13.3, 3.7 Hz), 2.41 (1H, dd, J = 13.5, 3.4 Hz), 2.23-2.15 (2H, m), 2.09-2.02 (3H, m), 1.86-1.51 (14H, m), 1.43-1.39 (3H, m), 1.36 (3H, d, J = 6.9 Hz), 1.15 (3H, d, J = 6.4 Hz), 0.65 (3H, s).

LC-MS: Exact Mass = 415.35 (C₂₇H₄₅NO₂) Obs. mass = 416.20 (M+H)

### [Example 319]

### Synthesis of (1R,3R)-5-(2-((1R,3aS,7aR,E)-1-((S)-1-(2,2-dimethylpiperidin-1-yl)propan-2-yl)-7a-methyloctahydro-4H-inden-4-ylidene)ethylidene)cyclohexane-1,3-diol (Compound G003)

Compound G003 [3.5 mg, 0.0081 mmol] was obtained by performing the reaction in the same manner as in Example 301 using compound (2b) [50 mg, 0.070 mmol] and 2,2-dimethylpiperidine hydrochloride [20.9 mg, 0.139 mmol].

¹H-NMR (CD₃OD) δ: 6.21 (1H, d, J = 11.4 Hz), 5.90 (1H, d, J = 11.0 Hz), 4.05-3.95 (2H, m), 3.45 (1H, s), 3.12 (1H, s), 2.87-2.83 (1H, m), 2.60 (1H, dd, J = 13.3, 3.7 Hz), 2.41 (1H, dd, J = 13.5, 3.4 Hz), 2.22-2.15 (2H, m), 2.10-2.01 (3H, m), 1.83-1.52 (14H, m), 1.44-1.35 (9H, m), 1.18 (3H, d, J = 6.4 Hz), 0.66 (3H, s).

LC-MS: Exact Mass = 429.36 (C₂₈H₄₇NO₂) Obs. mass = 430.20 (M+H)

### [Example 320]

### Synthesis of (1R,3R)-5-(2-((1R,3aS,7aR,E)-7a-methyl-1-((S)-1-((S)-3-methylmorpholino)propan-2-yl)octahydro-4H-inden-4-ylidene)ethylidene)cyclohexane-1,3-diol (Compound G004)

Compound G004 [4.3 mg, 0.010 mmol] was obtained by performing the reaction in the same manner as in Example 301 using compound (2b) [50 mg, 0.070 mmol] and (S)-3-methylmorpholine [21.2 mg, 0.209 mmol].

¹H-NMR (CD₃OD) δ: 6.21 (1H, d, J = 11.4 Hz), 5.89 (1H, d, J = 11.0 Hz), 4.05-3.95 (2H, m), 3.77 (1H, d, J = 11.4 Hz), 3.67-3.56 (2H, m), 3.26-3.20 (1H, m), 2.88-2.81 (2H, m), 2.59 (1H, dd, J = 13.3, 3.7 Hz), 2.44-2.32 (3H, m), 2.23-1.51 (16H, m), 1.38-1.22 (3H, m), 1.03 (3H, d, J = 6.4 Hz), 0.95 (3H, d, J = 6.4 Hz), 0.60 (3H, s).

LC-MS: Exact Mass = 417.32 (C₂₆H₄₃NO₃) Obs. mass = 418.25 (M+H)

### [Example 321]

### Synthesis of (1R,3R)-5-(2-((1R,3aS,7aR,E)-7a-methyl-1-((S)-1-((R)-3-methylmorpholino)propan-2-yl)octahydro-4H-inden-4-ylidene)ethylidene)cyclohexane-1,3-diol (Compound G005)

Compound G005 [6.3 mg, 0.015 mmol] was obtained by performing the reaction in the same manner as in Example 301 using compound (2b) [50 mg, 0.070 mmol] and (R)-3-methylmorpholine [21.2 mg, 0.209 mmol].

¹H-NMR (CD₃OD) δ: 6.21 (1H, d, J = 11.0 Hz), 5.89 (1H, d, J = 11.0 Hz), 4.06-3.95 (2H, m), 3.71-3.63 (3H, m), 3.33-3.28 (1H, m), 2.85-2.72 (3H, m), 2.61-2.46 (3H, m), 2.40 (1H, dd, J = 13.5, 3.4 Hz), 2.23-2.13 (2H, m), 2.06-1.22 (16H, m), 1.05 (3H, d, J = 6.4 Hz), 1.02 (3H, d, J = 6.4 Hz), 0.59 (3H, s).

LC-MS: Exact Mass = 417.32 (C₂₆H₄₃NO₃) Obs. mass = 418.20 (M+H)

### [Example 322]

### Synthesis of (1R,3R)-5-(2-((1R,3aS,7aR,E)-1-((S)-1-(3,3-dimethylmorpholino)propan-2-yl)-7a-methyloctahydro-4H-inden-4-ylidene)ethylidene)cyclohexane-1,3-diol (Compound G006)

Compound G006 [4.9 mg, 0.011 mmol] was obtained by performing the reaction in the same manner as in Example 301 using compound (2b) [50 mg, 0.070 mmol] and 3,3-dimethylmorpholine [16.1 mg, 0.139 mmol].

¹H-NMR (CD₃OD) δ: 6.21 (1H, d, J = 11.0 Hz), 5.89 (1H, d, J = 11.4 Hz), 4.06-3.94 (2H, m), 3.82-3.78 (1H, m), 3.65-3.59 (1H, m), 3.39 (1H, d, J = 11.0 Hz), 3.32 (1H, s), 2.85-2.81 (1H, m), 2.73-2.69 (1H, m), 2.62-2.56 (2H, m), 2.40 (1H, dd, J = 13.5, 3.4 Hz), 2.33-2.13 (4H, m), 2.07-1.97 (3H, m), 1.87-1.81 (1H, m), 1.78-1.72 (1H, m), 1.67-1.50 (6H, m), 1.37-1.25 (3H, m), 1.06-1.04 (6H, m), 1.01 (3H, s), 0.60 (3H, s).

LC-MS: Exact Mass = 431.34 (C₂₇H₄₅NO₃) Obs. mass = 432.20 (M+H)

### [Example 323]

### Synthesis of (1R,3R)-5-(2-((1R,3aS,7aR,E)-1-((S)-1-(7-oxa-4-azaspiro[2.5]octan-4-yl)propan-2-yl)-7a-methyloctahydro-4H-inden-4-ylidene)ethylidene)cyclohexane-1,3-diol (Compound G007)

Compound G007 [2.2 mg, 0.0051 mmol] was obtained by performing the reaction in the same manner as in Example 301 using compound (2b) [50 mg, 0.070 mmol] and 7-oxa-4-azaspiro[2.5]octane hydrochloride [20.9 mg, 0.139 mmol].

¹H-NMR (CD₃OD) δ: 6.21 (1H, d, J = 11.0 Hz), 5.88 (1H, d, J = 11.0 Hz), 4.05-3.96 (2H, m), 3.83-3.78 (1H, m), 3.66 (1H, d, J = 11.4 Hz), 3.58-3.54 (1H, m), 3.18 (1H, d, J = 11.4 Hz), 3.03-2.97 (2H, m), 2.85-2.75 (2H, m), 2.59 (1H, dd, J = 13.3, 3.7 Hz), 2.40 (1H, dd, J = 13.5, 3.4 Hz), 2.22-2.13 (2H, m), 2.05-1.98 (4H, m), 1.86-1.74 (2H, m), 1.69-1.50 (5H, m), 1.40-1.21 (4H, m), 0.93 (3H, d, J = 6.4 Hz), 0.73-0.70 (1H, m), 0.58 (3H, s), 0.55-0.49 (2H, m), 0.46-0.42 (1H, m).

LC-MS: Exact Mass = 429.32 (C₂₇H₄₃NO₃) Obs. mass = 430.15 (M+H)

### [Example 324]

### Synthesis of (1R,3R)-5-(2-((1R,3aS,7aR,E)-1-((S)-1-((S)-3-cyclopropylmorpholino)propan-2-yl)-7a-methyloctahydro-4H-inden-4-ylidene)ethylidene)cyclohexane-1,3-diol (Compound G008)

Compound G008 [13.7 mg, 0.0309 mmol] was obtained by performing the reaction in the same manner as in Example 301 using compound (2b) [50 mg, 0.070 mmol] and (S)-3-cyclopropylmorpholine [26.6 mg, 0.209 mmol].

¹H-NMR (CD₃OD) δ: 6.22 (1H, d, J = 11.0 Hz), 5.89 (1H, d, J = 11.0 Hz), 4.05-3.96 (2H, m), 3.84-3.79 (2H, m), 3.61 (1H, td, J = 11.4, 2.3 Hz), 3.44 (1H, dd, J = 11.9, 10.1 Hz), 3.10-2.99 (2H, m), 2.84 (1H, dd, J = 11.9, 3.7 Hz), 2.59 (1H, dd, J = 13.3, 4.1 Hz), 2.41 (1H, dd, J= 13.5, 3.4 Hz), 2.25-2.15 (4H, m), 2.08-2.00 (2H, m), 1.94-1.92 (1H, m), 1.86-1.51 (9H, m), 1.39-1.29 (3H, m), 1.08 (3H, d, J = 6.4 Hz), 0.76-0.69 (1H, m), 0.61 (3H, s), 0.58-0.46 (2H, m), 0.35-0.29 (1H, m), 0.15-0.11 (1H, m).

LC-MS: Exact Mass = 443.34 (C₂₈H₄₅NO₃) Obs. mass = 444.20 (M+H)

### [Example 325]

### Synthesis of (1R,3R)-5-(2-((1R,3aS,7aR,E)-1-((S)-1-((S)-3-isopropylmorpholino)propan-2-yl)-7a-methyloctahydro-4H-inden-4-ylidene)ethylidene)cyclohexane-1,3-diol (Compound G009)

Compound G009 [6.1 mg, 0.014 mmol] was obtained by performing the reaction in the same manner as in Example 301 using compound (2b) [50 mg, 0.070 mmol] and (S)-3-isopropylmorpholine [27.0 mg, 0.209 mmol].

LC-MS: Exact Mass = 445.36 (C₂₈H₄₇NO₃) Obs. mass = 446.20 (M+H)

### [Example 326]

### Synthesis of (1R,3R)-5-(2-((1R,3aS,7aR,E)-1-((S)-1-((R)-3-isopropylmorpholino)propan-2-yl)-7a-methyloctahydro-4H-inden-4-ylidene)ethylidene)cyclohexane-1,3-diol (Compound G010)

Compound G010 [10.6 mg, 0.0238 mmol] was obtained by performing the reaction in the same manner as in Example 301 using compound (2b) [50 mg, 0.070 mmol] and (R)-3-isopropylmorpholine [27.0 mg, 0.209 mmol].

LC-MS: Exact Mass = 445.36 (C₂₈H₄₇NO₃) Obs. mass = 446.20 (M+H)

### [Example 327]

### Synthesis of (1R,3R)-5-(2-((1R,3aS,7aR,E)-1-((S)-1-((S)-2,4-dimethylpiperazin-1-yl)propan-2-yl)-7a-methyloctahydro-4H-inden-4-ylidene)ethylidene)cyclohexane-1,3-diol (Compound G011)

Compound G011 [7.9 mg, 0.018 mmol] was obtained by performing the reaction in the same manner as in Example 301 using compound (2b) [50 mg, 0.070 mmol] and (S)-1,3-dimethylpiperazine dihydrochloride [26.1 mg, 0.139 mmol].

¹H-NMR (CD₃OD) δ: 6.21 (1H, d, J = 11.0 Hz), 5.89 (1H, d, J = 11.0 Hz), 4.05-3.95 (2H, m), 3.14-2.99 (3H, m), 2.84 (1H, dd, J = 12.6, 3.0 Hz), 2.65-2.50 (7H, m), 2.42-2.38 (2H, m), 2.28-2.15 (4H, m), 2.06-2.02 (2H, m), 1.85-1.82 (1H, m), 1.78-1.52 (6H, m), 1.38-1.26 (3H, m), 1.11 (3H, d, J = 5.9 Hz), 1.03 (3H, d, J = 6.4 Hz), 0.61 (3H, s).

LC-MS: Exact Mass = 430.36 (C₂₇H₄₆N₂O₂) Obs. mass = 431.20 (M+H)

### [Example 328]

### Synthesis of (1R,3R)-5-(2-((1R,3aS,7aR,E)-1-((S)-1-((R)-2,4-dimethylpiperazin-1-yl)propan-2-yl)-7a-methyloctahydro-4H-inden-4-ylidene)ethylidene)cyclohexane-1,3-diol (Compound G012)

Compound G012 [10.3 mg, 0.0239 mmol] was obtained by performing the reaction in the same manner as in Example 301 using compound (2b) [50 mg, 0.070 mmol] and (R)-1,3-dimethylpiperazine dihydrochloride [26.1 mg, 0.139 mmol].

¹H-NMR (CD₃OD) δ: 6.21 (1H, d, J = 11.4 Hz), 5.89 (1H, d, J = 11.4 Hz), 4.05-3.95 (2H, m), 2.95 (3H, d, J = 11.0 Hz), 2.84 (2H, d, J = 11.4 Hz), 2.74-2.57 (4H, m), 2.54 (3H, s), 2.40 (2H, dd, J = 13.5, 3.9 Hz), 2.20-2.14 (2H, m), 2.05-2.00 (3H, m), 1.86-1.82 (1H, m), 1.79-1.73 (1H, m), 1.66-1.52 (6H, m), 1.43-1.25 (3H, m), 1.14 (3H, d, J = 6.4 Hz), 1.05 (3H, d, J = 6.4 Hz), 0.59 (3H, s).

LC-MS: Exact Mass = 430.36 (C₂₇H₄₆N₂O₂) Obs. mass = 431.20 (M+H)

### [Example 329]

### Synthesis of (1R,3R)-5-(2-((1R,3aS,7aR,E)-1-((S)-1-((R)-2-(2-hydroxypropan-2-yl)pyrrolidin-1-yl)propan-2-yl)-7a-methyloctahydro-4H-inden-4-ylidene)ethylidene)cyclohexane-1,3-diol (Compound G013)

Compound G013 [5.8 mg, 0.013 mmol] was obtained by performing the reaction in the same manner as in Example 301 using compound (2b) [50 mg, 0.070 mmol] and 2-[(2R)-pyrrolidin-2-yl]propan-2-ol hydrochloride [34.7 mg, 0.209 mmol].

¹H-NMR (CD₃OD) δ: 6.21 (1H, d, J = 11.0 Hz), 5.89 (1H, d, J = 11.0 Hz), 4.05-3.95 (2H, m), 3.01 (1H, s), 2.89-2.82 (3H, m), 2.73 (1H, s), 2.59 (1H, dd, J = 13.3, 3.7 Hz), 2.41 (1H, dd, J = 13.3, 3.2 Hz), 2.18 (2H, td, J = 14.1, 6.9 Hz), 2.06-1.48 (17H, m), 1.39-1.27 (3H, m), 1.22 (6H, d, J = 10.1 Hz), 1.16 (3H, d, J = 6.4 Hz), 0.62 (3H, s).

LC-MS: Exact Mass = 445.36 (C₂₈H₄₇NO₃) Obs. mass = 446.30 (M+H)

### [Example 330]

### Synthesis of (1R,3R)-5-(2-((1R,3aS,7aR,E)-1-((S)-1-((S)-2-(2-hydroxypropan-2-yl)pyrrolidin-1-yl)propan-2-yl)-7a-methyloctahydro-4H-inden-4-ylidene)ethylidene)cyclohexane-1,3-diol (Compound G014)

Compound G014 [14.2 mg, 0.0319 mmol] was obtained by performing the reaction in the same manner as in Example 301 using compound (2b) [50 mg, 0.070 mmol] and 2-[(2S)-pyrrolidin-2-yl]propan-2-ol [27.0 mg, 0.209 mmol].

¹H-NMR (CD₃OD) δ: 6.21 (1H, d, J = 11.4 Hz), 5.90 (1H, d, J = 11.4 Hz), 4.05-3.95 (2H, m), 3.61-3.55 (1H, m), 3.51-3.40 (2H, m), 3.26-3.20 (1H, m), 2.85-2.79 (2H, m), 2.59 (1H, dd, J = 13.5, 3.4 Hz), 2.40 (1H, dd, J = 13.5, 3.0 Hz), 2.22-1.34 (25H, m), 1.28 (6H, d, J = 16.9 Hz), 1.19 (3H, d, J = 6.4 Hz), 0.64 (3H, s).

LC-MS: Exact Mass = 445.36 (C₂₈H₄₇NO₃) Obs. mass = 446.30 (M+H)

### [Example 331]

### Synthesis of (1R,3R)-5-(2-((1R,3aS,7aR,E)-1-((S)-1-((R)-2-(2-methoxypropan-2-yl)pyrrolidin-1-yl)propan-2-yl)-7a-methyloctahydro-4H-inden-4-ylidene)ethylidene)cyclohexane-1,3-diol (Compound G015)

Compound G015 [15.6 mg, 0.0339 mmol] was obtained by performing the reaction in the same manner as in Example 301 using compound (2b) [50 mg, 0.070 mmol] and (R)-2-(1-methoxy-1-methyl-ethyl)-pyrrolidine [20.0 mg, 0.139 mmol].

¹H-NMR (CD₃OD) δ: 6.21 (1H, d, J = 11.0 Hz), 5.90 (1H, d, J = 11.0 Hz), 4.05-4.02 (1H, m), 3.99-3.95 (1H, m), 3.49-3.45 (2H, m), 3.10-3.04 (3H, m), 2.85 (1H, dd, J = 11.2, 3.4 Hz), 2.60 (1H, dd, J = 13.5, 3.9 Hz), 2.41 (1H, dd, J = 13.3, 3.2 Hz), 2.21-2.12 (3H, m), 2.11-1.97 (6H, m), 1.89-1.52 (8H, m), 1.41-1.33 (3H, m), 1.28 (3H, s), 1.26 (3H, s), 1.18 (3H, d, J = 6.4 Hz), 0.64 (3H, s).

LC-MS: Exact Mass = 459.37 (C₂₉H₄₉NO₃) Obs. mass = 460.25 (M+H)

### [Example 332]

### Synthesis of (1R,3R)-5-(2-((1R,3aS,7aR,E)-1-((S)-1-((S)-2-(2-methoxypropan-2-yl)pyrrolidin-1-yl)propan-2-yl)-7a-methyloctahydro-4H-inden-4-ylidene)ethylidene)cyclohexane-1,3-diol (Compound G016)

Compound G016 [5.6 mg, 0.012 mmol] was obtained by performing the reaction in the same manner as in Example 301 using compound (2b) [50 mg, 0.070 mmol] and (R)-2-(1-methoxy-1-methyl-ethyl)-pyrrolidine hydrochloride [25.1 mg, 0.139 mmol].

¹H-NMR (CD₃OD) δ: 6.22 (1H, d, J = 11.0 Hz), 5.91 (1H, d, J = 11.4 Hz), 4.06-4.03 (1H, m), 4.00-3.96 (1H, m), 3.60-3.52 (2H, m), 3.37 (1H, d, J = 11.9 Hz), 3.28-3.22 (1H, m), 2.87-2.79 (2H, m), 2.60 (1H, dd, J = 13.3, 3.7 Hz), 2.41 (1H, dd, J = 13.3, 3.2 Hz), 2.26-1.96 (11H, m), 1.88-1.83 (1H, m), 1.79-1.53 (6H, m), 1.42-1.35 (3H, m), 1.27 (3H, s), 1.26 (3H, s), 1.19 (3H, d, J = 6.4 Hz), 0.65 (3H, s).

LC-MS: Exact Mass = 459.37 (C₂₉H₄₉NO₃) Obs. mass = 460.25 (M+H)

### [Example 333]

### Synthesis of (1R,3R)-5-(2-((1R,3aS,7aR,E)-7a-methyl-1-((R)-4-((S)-2-methylpiperidin-1-yl)butan-2-yl)octahydro-4H-inden-4-ylidene)ethylidene)cyclohexane-1,3-diol (Compound G017)

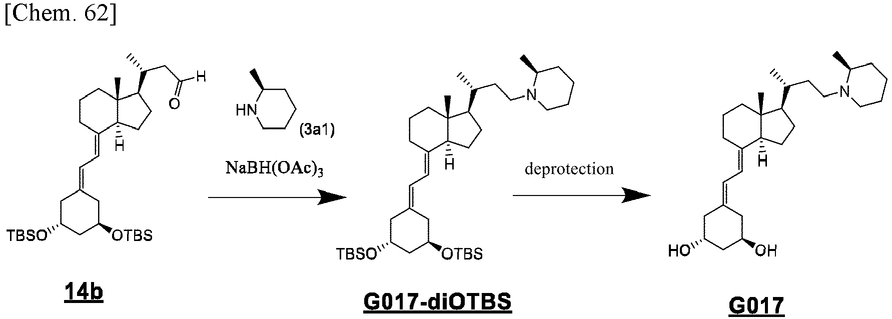

### Step 1

A solution of compound (14b) described in Reference example 14 [50 mg, 0.087 mmol], (S)-2-methylpiperidine [43.2 mg, 0.435 mmol], and sodium triacetoxyborohydride [55.3 mg, 0.261 mmol] in THF [1 mL] was stirred at room temperature overnight. A saturated aqueous sodium hydrogen carbonate solution was added to the reaction mixture to quench, and the mixture was extracted with ethyl acetate. The organic layer was dried over anhydrous magnesium sulfate and concentrated under reduced pressure to obtain a crude product containing compound (G017-diOTBS).

### Step 2

TBAF [1 M in THF, 0.2 mL, 0.2 mmol] was added to a solution of the crude product obtained in step 3 in THF [1 mL], and the mixture was stirred at 60°C overnight. A saturated aqueous sodium hydrogen carbonate solution was added to the reaction mixture to quench, and the mixture was extracted with ethyl acetate. The organic layer was dried over anhydrous magnesium sulfate and concentrated under reduced pressure.

The residue was purified by reverse phase HPLC to obtain compound (G017) [9.8 mg, 23 mmol].

¹H-NMR (CD₃OD) δ: 6.21 (1H, d, J = 11.0 Hz), 5.88 (1H, d, J = 11.0 Hz), 4.06-3.95 (2H, m), 2.89-2.81 (2H, m), 2.67-2.56 (2H, m), 2.50-2.38 (2H, m), 2.31 (1H, s), 2.24-2.13 (3H, m), 2.06-1.94 (3H, m), 1.87-1.18 (20H, m), 1.09 (3H, d, J = 6.4 Hz), 0.97 (3H, d, J = 6.4 Hz), 0.58 (3H, s).

LC-MS: Exact Mass = 429.36 (C₂₈H₄₇NO₂) Obs. mass = 430.30 (M+H)

### [Example 334]

### Synthesis of (1R,3R)-5-(2-((1R,3aS,7aR,E)-7a-methyl-1-((R)-4-((R)-2-methylpiperidin-1-yl)butan-2-yl)octahydro-4H-inden-4-ylidene)ethylidene)cyclohexane-1,3-diol

### (Compound G018)

Compound G018 [8.3 mg, 0.019 mmol] was obtained by performing the reaction in the same manner as in steps 1 and 2 of Example 333 using compound (14b) [50 mg, 0.087 mmol] described in Reference example 14, (R)-2-methylpiperidine [43.2 mg, 0.435 mmol], and sodium triacetoxyborohydride [55.3 mg, 0.261 mmol].

¹H-NMR (CD₃OD) δ: 6.21 (1H, d, J = 11.0 Hz), 5.88 (1H, d, J = 11.0 Hz), 4.05-3.95 (2H, m), 2.86-2.73 (3H, m), 2.59 (1H, dd, J = 13.5, 3.4 Hz), 2.42-2.34 (3H, m), 2.28-2.13 (3H, m), 2.06-1.95 (3H, m), 1.86-1.54 (12H, m), 1.39-1.29 (6H, m), 1.25-1.19 (1H, m), 1.09 (3H, d, J = 5.9 Hz), 0.98 (3H, d, J = 5.5 Hz), 0.57 (3H, s).

LC-MS: Exact Mass = 429.36 (C₂₈H₄₇NO₂) Obs. mass = 430.30 (M+H)

### [Example 335]

### Synthesis of (1R,3R)-5-(2-((1R,3aS,7aR,E)-1-((R)-4-((S)-2,4-dimethylpiperazin-1-yl)butan-2-yl)-7a-methyloctahydro-4H-inden-4-ylidene)ethylidene)cyclohexane-1,3-diol (Compound G019)

Compound G019 [3.7 mg, 0.0083 mmol] was obtained by performing the reaction in the same manner as in steps 1 and 2 of Example 333 using compound (14b) [50 mg, 0.087 mmol] described in Reference example 14, (S)-1,3-dimethylpiperazine dihydrochloride [81.4 mg, 0.435 mmol], and sodium triacetoxyborohydride [55.3 mg, 0.261 mmol].

¹H-NMR (CD₃OD) δ: 6.21 (1H, d, J = 11.0 Hz), 5.89 (1H, d, J = 11.0 Hz), 4.06-3.94 (2H, m), 3.20-3.16 (1H, m), 2.99-2.82 (5H, m), 2.66-2.50 (4H, m), 2.43-2.39 (4H, m), 2.26-2.13 (3H, m), 2.07-2.00 (3H, m), 1.87-1.81 (1H, m), 1.78-1.72 (1H, m), 1.67-1.52 (7H, m), 1.40-1.34 (4H, m), 1.20 (3H, d, J = 6.4 Hz), 0.99 (3H, d, J = 6.4 Hz), 0.59 (3H, s).

LC-MS: Exact Mass = 444.37 (C₂₈H₄₈N₂O₂) Obs. mass = 445.20 (M+H)

### [Example 336]

### Synthesis of (1R,3R)-5-(2-((1R,3aS,7aR,E)-1-((R)-4-((R)-2,4-dimethylpiperazin-1-yl)butan-2-yl)-7a-methyloctahydro-4H-inden-4-ylidene)ethylidene)cyclohexane-1,3-diol (Compound G020)

Compound G020 [4.3 mg, 0.0097 mmol] was obtained by performing the reaction in the same manner as in steps 1 and 2 of Example 333 using compound (14b) [50 mg, 0.087 mmol] described in Reference example 14, (R)-1,3-dimethylpiperazine dihydrochloride [81.4 mg, 0.435 mmol], and sodium triacetoxyborohydride [55.3 mg, 0.261 mmol].

¹H-NMR (CD₃OD) δ: 6.21 (1H, d, J = 11.0 Hz), 5.89 (1H, d, J = 11.0 Hz), 4.05-3.95 (2H, m), 3.16-3.13 (1H, m), 3.05 (1H, td, J = 12.6, 4.4 Hz), 2.91-2.82 (4H, m), 2.74 (1H, t, J = 10.7 Hz), 2.60-2.50 (3H, m), 2.42-2.39 (4H, m), 2.27-2.13 (3H, m), 2.08-1.98 (3H, m), 1.86-1.82 (1H, m), 1.78-1.27 (12H, m), 1.21 (3H, d, J = 6.4 Hz), 1.01 (3H, d, J = 6.4 Hz), 0.58 (3H, s).

LC-MS: Exact Mass = 444.37 (C₂₈H₄₈N₂O₂) Obs. mass = 445.20 (M+H)

### [Example 337]

### Synthesis of (1R,3R)-5-(2-((1R,3aS,7aR,E)-7a-methyl-1-((S)-1-((S)-2-methylpiperidin-1-yl)propan-2-yl)octahydro-4H-inden-4-ylidene)ethylidene)-2-methylenecyclohexane-1,3-diol (Compound H001)

Compound H001 [19.9 mg, 0.0465 mmol] was obtained by performing the reaction in the same manner as in Example 301 using compound (2c) [50 mg, 0.069 mmol] and (S)-2-methylpiperidine [20.4 mg, 0.206 mmol].

¹H-NMR (CD₃OD) δ: 6.27 (1H, d, J = 11.0 Hz), 5.93 (1H, d, J = 11.0 Hz), 5.05 (2H, d, J = 5.5 Hz), 4.42-4.36 (2H, m), 3.55-3.52 (1H, m), 3.27-3.25 (1H, m), 3.08-3.05 (1H, m), 2.93-2.85 (3H, m), 2.66 (1H, dd, J = 13.3, 4.1 Hz), 2.48 (1H, dd, J = 13.5, 3.9 Hz), 2.32-2.25 (2H, m), 2.11-1.89 (6H, m), 1.83-1.50 (9H, m), 1.44-1.34 (3H, m), 1.38 (3H, d, J = 7.0 Hz), 1.12 (3H, d, J = 6.4 Hz), 0.66 (3H, s).

LC-MS: Exact Mass = 427.35 (C₂₈H₄₅NO₂) Obs. mass = 428.20 (M+H)

### [Example 338]

### Synthesis of (1R,3R)-5-(2-((1R,3aS,7aR,E)-7a-methyl-1-((S)-1-((R)-2-methylpiperidin-1-yl)propan-2-yl)octahydro-4H-inden-4-ylidene)ethylidene)-2-methylenecyclohexane-1,3-diol (Compound H002)

Compound H002 [26.4 mg, 0.0617 mmol] was obtained by performing the reaction in the same manner as in Example 301 using compound (2c) [50 mg, 0.069 mmol] and (R)-2-methylpiperidine [20.4 mg, 0.206 mmol].

¹H-NMR (CD₃OD) δ: 6.27 (1H, d, J = 11.0 Hz), 5.93 (1H, d, J = 11.4 Hz), 5.05 (2H, d, J = 5.9 Hz), 4.42-4.36 (2H, m), 3.45-3.41 (2H, m), 3.25 (1H, s), 3.12-3.08 (1H, m), 2.87 (1H, dd, J = 12.3, 3.7 Hz), 2.71-2.65 (2H, m), 2.48 (1H, dd, J = 13.3, 4.1 Hz), 2.31-2.25 (2H, m), 2.11-2.00 (3H, m), 1.89-1.76 (5H, m), 1.72-1.53 (7H, m), 1.44-1.39 (3H, m), 1.36 (3H, d, J = 6.9 Hz), 1.15 (3H, d, J = 6.4 Hz), 0.66 (3H, s).

LC-MS: Exact Mass = 427.35 (C₂₈H₄₅NO₂) Obs. mass = 428.20 (M+H)

### [Example 339]

### Synthesis of (1R,3R)-5-(2-((1R,3aS,7aR,E)-1-((S)-1-(2,2-dimethylpiperidin-1-yl)propan-2-yl)-7a-methyloctahydro-4H-inden-4-ylidene)ethylidene)-2-methylenecyclohexane-1,3-diol (Compound H003)

Compound H003 [8.8 mg, 0.020 mmol] was obtained by performing the reaction in the same manner as in Example 301 using compound (2c) [50 mg, 0.069 mmol] and 2,2-dimethylpiperidine hydrochloride [30.8 mg, 0.206 mmol].

¹H-NMR (CD₃OD) δ: 6.27 (1H, d, J = 11.0 Hz), 5.93 (1H, d, J = 11.0 Hz), 5.05 (2H, d, J = 5.9 Hz), 4.42-4.36 (2H, m), 3.44 (1H, s), 3.12 (1H, s), 2.87 (1H, dd, J = 12.1, 3.4 Hz), 2.67 (1H, dd, J = 13.3, 4.1 Hz), 2.48 (1H, dd, J = 13.3, 4.1 Hz), 2.31-2.25 (2H, m), 2.12-2.03 (3H, m), 1.81-1.53 (11H, m), 1.45-1.36 (8H, m), 1.18 (3H, d, J = 6.4 Hz), 0.66 (3H, s).

LC-MS: Exact Mass = 441.36 (C₂₉H₄₇NO₂) Obs. mass = 442.20 (M+H)

### [Example 340]

### Synthesis of (1R,3R)-5-(2-((1R,3aS,7aR,E)-1-((S)-1-((S)-2,4-dimethylpiperazin-1-yl)propan-2-yl)-7a-methyloctahydro-4H-inden-4-ylidene)ethylidene)-2-methylenecyclohexane-1,3-diol (Compound H011)

Compound H011 [14.9 mg, 0.0337 mmol] was obtained by performing the reaction in the same manner as in Example 301 using compound (2c) [50 mg, 0.069 mmol] and (S)-1,3-dimethylpiperazine dihydrochloride [38.5 mg, 0.206 mmol].

¹H-NMR (CD₃OD) δ: 6.26 (1H, d, J = 11.0 Hz), 5.91 (1H, d, J = 11.4 Hz), 5.05 (2H, d, J = 6.4 Hz), 4.42-4.36 (2H, m), 3.16-3.02 (3H, m), 2.86 (1H, dd, J = 11.7, 3.9 Hz), 2.67 (2H, dd, J = 13.3, 5.0 Hz), 2.56 (4H, s), 2.54-2.40 (3H, m), 2.31-2.17 (4H, m), 2.10-1.98 (3H, m), 1.68-1.52 (6H, m), 1.40-1.25 (3H, m), 1.12 (3H, d, J = 5.9 Hz), 1.03 (3H, d, J = 6.4 Hz), 0.62 (3H, s).

LC-MS: Exact Mass = 442.36 (C₂₈H₄₆N₂O₂) Obs. mass = 443.20 (M+H)

### [Example 341]

### Synthesis of (1R,3R)-5-(2-((1R,3aS,7aR,E)-1-((S)-1-((R)-2,4-dimethylpiperazin-1-yl)propan-2-yl)-7a-methyloctahydro-4H-inden-4-ylidene)ethylidene)-2-methylenecyclohexane-1,3-diol (Compound H012)

Compound H012 [14.3 mg, 0.0323 mmol] was obtained by performing the reaction in the same manner as in Example 301 using compound (2c) [50 mg, 0.069 mmol] and (R)-1,3-dimethylpiperazine dihydrochloride [38.5 mg, 0.206 mmol].

¹H-NMR (CD₃OD) δ: 6.26 (1H, d, J = 11.4 Hz), 5.91 (1H, d, J = 11.4 Hz), 5.05 (2H, d, J = 6.4 Hz), 4.42-4.35 (2H, m), 2.93-2.82 (5H, m), 2.69-2.62 (4H, m), 2.50 (3H, s), 2.46 (1H, d, J = 3.7 Hz), 2.31-2.25 (2H, m), 2.05-1.98 (3H, m), 1.68-1.52 (6H, m), 1.40-1.25 (3H, m), 1.13 (3H, d, J = 6.4 Hz), 1.05 (3H, d, J = 6.4 Hz), 0.60 (3H, s).

LC-MS: Exact Mass = 442.36 (C₂₈H₄₆N₂O₂) Obs. mass = 443.20 (M+H)

### [Example 342]

### Synthesis of (1R,3R)-5-(2-((1R,3aS,7aR,E)-1-((S)-1-((R)-2-(2-hydroxypropan-2-yl)pyrrolidin-1-yl)propan-2-yl)-7a-methyloctahydro-4H-inden-4-ylidene)ethylidene)-2-methylenecyclohexane-1,3-diol (Compound H013)

Compound H013 [12.0 mg, 0.0260 mmol] was obtained by performing the reaction in the same manner as in Example 301 using compound (2c) [50 mg, 0.069 mmol] and (R)-2-(pyrrolidin-2-yl)propan-2-ol hydrochloride [34.1 mg, 0.206 mmol].

¹H-NMR (CD₃OD) δ: 6.27 (1H, d, J = 11.4 Hz), 5.92 (1H, d, J = 11.4 Hz), 5.05 (2H, d, J = 5.9 Hz), 4.42-4.36 (2H, m), 3.51-3.46 (1H, m), 3.33 (1H, s), 3.10-3.02 (3H, m), 2.86 (1H, dd, J = 12.1, 3.9 Hz), 2.67 (1H, dd, J = 13.3, 4.1 Hz), 2.48 (1H, dd, J = 13.5, 3.9 Hz), 2.31-2.25 (2H, m), 2.16-1.97 (6H, m), 1.90-1.80 (2H, m), 1.69-1.53 (5H, m), 1.41-1.34 (3H, m), 1.30 (3H, s), 1.26 (3H, s), 1.20 (3H, d, J = 6.4 Hz), 0.65 (3H, s).

LC-MS: Exact Mass = 457.36 (C₂₉H₄₇NO₃) Obs. mass = 458.20 (M+H)

### [Example 343]

### Synthesis of (1R,3R)-5-(2-((1R,3aS,7aR,E)-1-((S)-1-((S)-2-(2-hydroxypropan-2-yl)pyrrolidin-1-yl)propan-2-yl)-7a-methyloctahydro-4H-inden-4-ylidene)ethylidene)-2-methylenecyclohexane-1,3-diol (Compound H014)

Compound H014 [14.0 mg, 0.0306 mmol] was obtained by performing the reaction in the same manner as in Example 301 using compound (2c) [50 mg, 0.069 mmol] and (S)-2-(pyrrolidin-2-yl)propan-2-ol hydrochloride [34.1 mg, 0.206 mmol]. ¹H-NMR (CD₃OD) δ: 6.27 (1H, d, J = 11.0 Hz), 5.92 (1H, d, J = 11.0 Hz), 5.05 (2H, d, J = 5.9 Hz), 4.42-4.36 (2H, m), 3.60-3.54 (1H, m), 3.49 (1H, t, J = 7.5 Hz), 3.42 (1H, d, J = 12.8 Hz), 3.26-3.20 (1H, m), 2.88-2.80 (2H, m), 2.67 (1H, dd, J = 13.3, 4.6 Hz), 2.48 (1H, dd, J = 13.5, 3.9 Hz), 2.31-1.95 (10H, m), 1.92-1.88 (1H, m), 1.69-1.35 (8H, m), 1.30 (3H, s), 1.26 (3H, s), 1.20 (3H, d, J = 6.9 Hz), 0.65 (3H, s).

LC-MS: Exact Mass = 457.36 (C₂₉H₄₇NO₃) Obs. mass = 458.20 (M+H)

### [Example 344]

### Synthesis of (1R,3R)-5-(2-((1R,3aS,7aR,E)-1-((S)-1-((R)-2-(2-methoxypropan-2-yl)pyrrolidin-1-yl)propan-2-yl)-7a-methyloctahydro-4H-inden-4-ylidene)ethylidene)-2-methylenecyclohexane-1,3-diol (Compound H015)

Compound H015 [15.2 mg, 0.0322 mmol] was obtained by performing the reaction in the same manner as in Example 301 using compound (2c) [50 mg, 0.069 mmol] and (R)-2-(1-methoxy-1-methyl-ethyl)-pyrrolidine [30.0 mg, 0.209 mmol]. LC-MS: Exact Mass = 471.37 (C₃₀H₄₉NO₃) Obs. mass = 472.20 (M+H)

### [Example 345]

### Synthesis of (1R,3R)-5-(2-((1R,3aS,7aR,E)-1-((S)-1-((S)-2-(2-methoxypropan-2-yl)pyrrolidin-1-yl)propan-2-yl)-7a-methyloctahydro-4H-inden-4-ylidene)ethylidene)-2-methylenecyclohexane-1,3-diol (Compound H016)

Compound H016 [25.2 mg, 0.0534 mmol] was obtained by performing the reaction in the same manner as in Example 301 using compound (2c) [50 mg, 0.069 mmol] and (S)-2-(1-methoxy-1-methyl-ethyl)-pyrrolidine hydrochloride [37.0 mg, 0.206 mmol].

¹H-NMR (CD₃OD) δ: 6.27 (1H, d, J = 11.0 Hz), 5.93 (1H, d, J = 11.0 Hz), 5.06 (2H, d, J = 5.5 Hz), 4.43-4.36 (2H, m), 3.60-3.53 (2H, m), 3.38 (1H, d, J = 11.4 Hz), 3.28-3.22 (1H, m), 2.89-2.80 (2H, m), 2.67 (1H, dd, J = 13.3, 4.6 Hz), 2.49 (1H, dd, J = 13.5, 3.9 Hz), 2.31-2.21 (3H, m), 2.13-1.95 (7H, m), 1.72-1.54 (5H, m), 1.42-1.36 (3H, m), 1.27 (3H, s), 1.26 (3H, s), 1.19 (3H, d, J = 6.4 Hz), 0.66 (3H, s).

LC-MS: Exact Mass = 471.37 (C₃₀H₄₉NO₃) Obs. mass = 472.20 (M+H)

### [Example 346]

### Synthesis of (1R,3R,Z)-5-(2-((1R,3aS,7aR,E)-7a-methyl-1-((S)-1-((S)-2-methylpiperidin-1-yl)propan-2-yl)octahydro-4H-inden-4-ylidene)ethylidene)-4-methylenecyclohexane-1,3-diol (Compound I001)

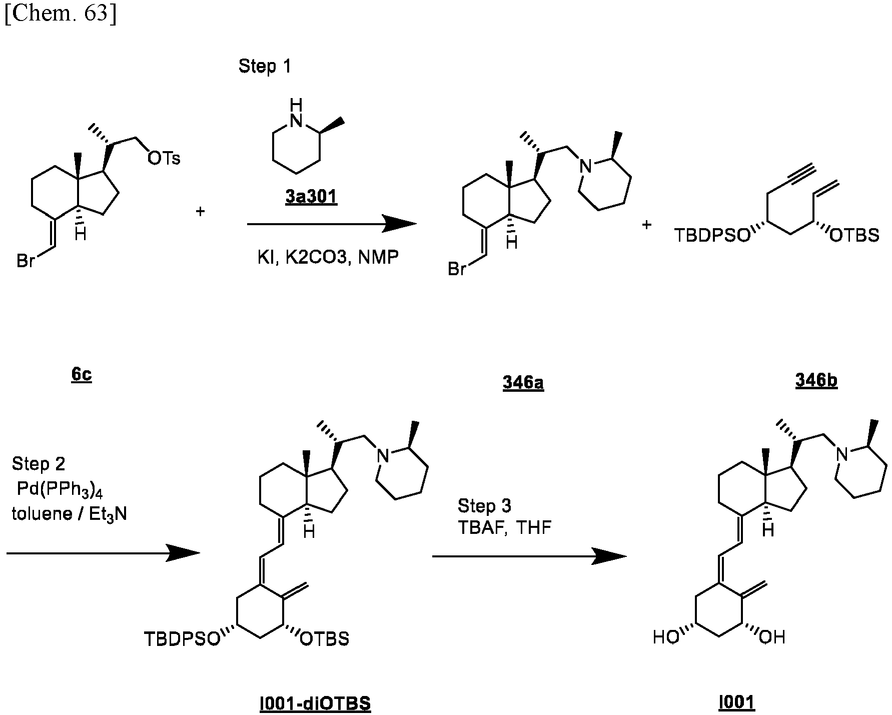

### Step 1

A suspension [1 mL] of compound (6c) [70 mg, 0.159 mmol], (S)-2-methylpiperidine [47.2 mg, 0.476 mmol], potassium iodide [52.7 mg, 0.317 mmol], and potassium carbonate [65.8 mg, 0.476 mmol] in NMP was stirred at 60°C overnight. The reaction mixture was transferred to saturated brine and the mixture was extracted with ethyl acetate. The organic layer was dried over anhydrous magnesium sulfate and concentrated under reduced pressure to obtain a crude product containing compound (346a).

### Step 2

A mixture of crude product containing compound (346a) obtained in step 1, compound (346b) [CAS Registry No. 143681-90-7, 93.8 mg, 0.190 mmol], tetrakis(triphenylphosphine) palladium(0) [36.7 mg, 0.0318 mmol], and toluene-triethylamine mixed liquid [1/1, 1 mL] was stirred at 100°C for 1 hour. The reaction mixture was concentrated, then diluted with ethyl acetate, and washed with saturated brine. The organic layer was dried over anhydrous magnesium sulfate and concentrated under reduced pressure to obtain a crude product containing compound (I001-diOTBS).

### Step 3

The compound (I001-diOTBS) obtained in Step 2 was dissolved in THF [1 mL], then to the solution, TBAF [1 M in THF, 0.5 mL, 0.5 mmol] was added, and the mixture was stirred at 50°C for 3 hours. The reaction mixture was transferred to a saturated aqueous sodium hydrogen carbonate solution and the mixture was extracted with ethyl acetate. The organic layer was dried over anhydrous magnesium sulfate and concentrated under reduced pressure. The residue was purified by HPLC to obtain compound I001 [5.6 mg, 0.013 mmol].

¹H-NMR (CD₃OD) δ: 6.34 (1H, d, J = 11.0 Hz), 6.03 (1H, d, J = 11.4 Hz), 5.36 (1H, t, J = 2.3 Hz), 3.98-3.95 (1H, m), 3.74-3.69 (1H, m), 3.56-3.52 (1H, m), 3.26 (1H, s), 3.08-3.02 (1H, m), 2.91-2.82 (4H, m), 2.57-2.54 (1H, m), 2.31-2.27 (1H, m), 2.16-2.04 (4H, m), 1.89-1.36 (24H, m), 1.15-1.10 (4H, m), 0.61 (3H, s).

LC-MS: Exact Mass = 427.35 (C₂₈H₄₅NO₂) Obs. mass = 428.20 (M+H)

### [Example 347]

### Synthesis of (1R,3R,Z)-5-(2-((1R,3aS,7aR,E)-7a-methyl-1-((S)-1-((R)-2-methylpiperidin-1-yl)propan-2-yl)octahydro-4H-inden-4-ylidene)ethylidene)-4-methylenecyclohexane-1,3-diol (Compound I002)

Compound I002 [7.0 mg, 0.016 mmol] was obtained by performing the reaction in the same manner as in steps 1 to 3 of Example 346 using compound (6c) [70 mg, 0.159 mmol] and (R)-2-methylpiperidine [47.2 mg, 0.476 mmol].

¹H-NMR (CD₃OD) δ: 6.34 (1H, d, J = 11.0 Hz), 6.03 (1H, d, J = 11.0 Hz), 5.36 (1H, t, J = 2.3 Hz), 3.96 (1H, dd, J = 11.2, 4.8 Hz), 3.74-3.68 (1H, m), 3.43-3.38 (3H, m), 3.25 (1H, d, J = 12.8 Hz), 3.12-3.07 (1H, m), 2.90-2.85 (1H, m), 2.67 (1H, t, J = 11.9 Hz), 2.57-2.54 (1H, m), 2.31-2.29 (1H, m), 2.15-2.00 (5H, m), 1.88-1.34 (26H, m), 1.17-1.13 (4H, m), 0.61 (3H, s).

LC-MS: Exact Mass = 427.35 (C₂₈H₄₅NO₂) Obs. mass = 428.20 (M+H)

### [Example 348]

### Synthesis of (1R,3R,Z)-5-(2-((1R,3aS,7aR,E)-1-((S)-1-(2,2-dimethylpiperidin-1-yl)propan-2-yl)-7a-methyloctahydro-4H-inden-4-ylidene)ethylidene)-4-methylenecyclohexane-1,3-diol (Compound I003)

Compound I003 [2.6 mg, 0.0059 mmol] was obtained by performing the reaction in the same manner as in steps 1 to 3 of Example 346 using compound (6c) [70 mg, 0.159 mmol] and 2,2-dimethylpiperidine hydrochloride [47.2 mg, 0.476 mmol].

¹H-NMR (CD₃OD) δ: 6.34 (1H, d, J = 11.4 Hz), 6.03 (1H, d, J = 11.0 Hz), 5.36 (1H, t, J = 2.1 Hz), 3.98-3.93 (1H, m), 3.73-3.68 (1H, m), 3.46-3.42 (2H, m), 3.12 (1H, s), 2.90-2.86 (1H, m), 2.57-2.54 (1H, m), 2.32-2.28 (1H, m), 2.16-2.03 (5H, m), 1.82-1.67 (14H, m), 1.56-1.35 (18H, m), 1.19-1.15 (4H, m), 0.62 (3H, s).

LC-MS: Exact Mass = 441.36 (C₂₉H₄₇NO₂) Obs. mass = 442.25 (M+H)

### [Example 349]

### Synthesis of (1R,3R,Z)-5-(2-((1R,3aS,7aR,E)-7a-methyl-1-((S)-1-((S)-3-methylmorpholino)propan-2-yl)octahydro-4H-inden-4-ylidene)ethylidene)-4-methylenecyclohexane-1,3-diol (Compound I004)

Compound I004 [10.5 mg, 0.024 mmol] was obtained by performing the reaction in the same manner as in steps 1 to 3 of Example 346 using compound (6c) [70 mg, 0.159 mmol] and (S)-3-methylmorpholine [32.1 mg, 0.317 mmol].

¹H-NMR (CD₃OD) δ: 6.34 (1H, d, J = 11.4 Hz), 6.01 (1H, d, J = 11.4 Hz), 5.37-5.35 (1H, m), 3.97 (1H, dd, J = 11.0, 4.6 Hz), 3.83-3.80 (1H, m), 3.74-3.60 (3H, m), 3.00 (1H, d, J = 12.3 Hz), 2.87 (1H, d, J = 12.3 Hz), 2.58-2.53 (3H, m), 2.32-2.25 (3H, m), 2.13-2.01 (4H, m), 1.94-1.90 (1H, m), 1.73-1.66 (4H, m), 1.55-1.30 (7H, m), 1.08-1.01 (7H, m), 0.58 (3H, s).

LC-MS: Exact Mass = 429.32 (C₂₇H₄₃NO₃) Obs. mass = 430.25 (M+H)

### [Example 350]

### Synthesis of (1R,3R,Z)-5-(2-((1R,3aS,7aR,E)-7a-methyl-1-((S)-1-((R)-3-methylmorpholino)propan-2-yl)octahydro-4H-inden-4-ylidene)ethylidene)-4-methylenecyclohexane-1,3-diol (Compound I005)

Compound I005 [9.3 mg, 0.022 mmol] was obtained by performing the reaction in the same manner as in steps 1 to 3 of Example 346 using compound (6c) [70 mg, 0.159 mmol] and (R)-3-methylmorpholine [32.1 mg, 0.317 mmol].

¹H-NMR (CD₃OD) δ: 6.33 (1H, d, J = 11.4 Hz), 6.01 (1H, d, J = 11.4 Hz), 5.37-5.35 (1H, m), 3.97 (1H, dd, J = 11.0, 4.6 Hz), 3.72-3.68 (5H, m), 2.88-2.80 (3H, m), 2.65-2.53 (3H, m), 2.32-2.28 (1H, m), 2.16-1.99 (5H, m), 1.94-1.93 (1H, m), 1.73-1.25 (12H, m), 1.05 (7H, dd, J = 6.4, 2.3 Hz), 0.56 (3H, s).

LC-MS: Exact Mass = 429.32 (C₂₇H₄₃NO₃) Obs. mass = 430.25 (M+H)

### [Example 351]

### Synthesis of (1R,3R,Z)-5-(2-((1R,3aS,7aR,E)-1-((S)-1-(3,3-dimethylmorpholino)propan-2-yl)-7a-methyloctahydro-4H-inden-4-ylidene)ethylidene)-4-methylenecyclohexane-1,3-diol (Compound I006)

Compound I006 [2.2 mg, 0.005 mmol] was obtained by performing the reaction in the same manner as in steps 1 to 3 of Example 346 using compound (6c) [70 mg, 0.159 mmol] and 3,3-dimethylmorpholine [36.5 mg, 0.317 mmol].

¹H-NMR (CD₃OD) δ: 6.33 (1H, d, J = 11.0 Hz), 6.01 (1H, d, J = 11.0 Hz), 5.35 (1H, t, J = 2.1 Hz), 3.97 (1H, dd, J = 11.0, 4.6 Hz), 3.82-3.79 (1H, m), 3.74-3.60 (3H, m), 3.43-3.38 (1H, m), 3.34 (1H, s), 2.88-2.74 (3H, m), 2.65-2.61 (1H, m), 2.54 (1H, dd, J = 12.6, 2.5 Hz), 2.35-2.28 (4H, m), 2.16-1.99 (5H, m), 1.70-1.67 (3H, m), 1.55-1.45 (6H, m), 1.39-1.24 (4H, m), 1.07-1.02 (11H, m), 0.56 (3H, s).WW

LC-MS: Exact Mass = 443.34 (C₂₈H₄₅NO₃) Obs. mass = 444.20 (M+H)

### [Example 352]

### Evaluation of VDR transcription activity in human osteoblasts (HOS cells)

(1) A reporter vector was constructed using pGL4.26 vector (Promega Corporation) and incorporating a sequence of DR3 (×4) gene promoter portion upstream of the luciferase gene. An expression vector was constructed by inserting a DNA sequence encoding human VDR and human RXRα into pcDNA3 vector (Thermo Fisher Scientific, Inc.). Human RenillaLuc-CMV vector (Promega Corporation) was used for the correction of gene transfer efficiency. HOS cells (ATCC) were cultured in DMEM medium containing 10% FBS under the condition of 37°C and 5% CO₂, and passaged every 2 or 3 days.
(2) The passaged cells were collected by centrifugation and dispersed in phenol red free DMEM medium containing deactivated and 5% charcoal-treated fetal bovine serum (CSS) at a density of 4×10⁵ cells/mL, and then the various vectors described in (1) were introduced using Lipofectamine LTX (Thermo Fisher Scientific, Inc.) reagent at a ratio of 0.25 mL per 1 mL of the cell solution. HOS cells into which the VDR gene was introduced were seeded on a white 384-well plate (Aurora Inc.) at 0.01 mL/well. After the incubation for 3 hours under the condition of 37°C and 5% CO₂, a solution (final concentration: 200 nM) of 1α,25-dihydroxyvitamin D₃ in DMSO and a solution (various concentrations) of the test compound in DMSO were added to be the final concentration of DMSO at 0.1%. The well containing DMSO alone was prepared as blank, and the well containing a solution (final concentration: 200 nM) of 1α,25-dihydroxyvitamin D₃ in DMSO was prepared as control.
(3) After the incubation for 21 hours under the condition of 37°C and 5% CO₂, the luciferase activity was measured by multi-label counter EnVision (PerkinElmer, Inc.) using Dual-Glo Luciferase Assay kit (Promega Corporation). At the same time, the activity of Renilla luciferase was measured and used to correct the gene transfer efficiency. The firefly luciferase activity was corrected using the Renilla luciferase activity, the transcriptional activity rate (blank: 0%, control: 100%) of the test compound was calculated, and then EC₅₀ values were calculated from Formula fit208 of XLfit (IDBS).
(4) Result

The activity of the derivatives of the present invention using the aforementioned evaluation is shown in the following table. The activity is shown by the following symbols.

| | |
|---|---|
| ∗ | 100 nM < EC₅₀ ≤ 1000 nM |
| ∗∗ | 10 nM < EC₅₀ ≤ 100 nM |
| ∗∗∗ | 1 nM < EC₅₀ ≤ 10 nM |
| ∗∗∗∗ | EC₅₀ ≤ 1 nM |

When the activities of 1α,25-dihydroxyvitamin D₃ were evaluated multiple times using this evaluation system, the activity values were shown as from 2.93 nM to 11.30 nM in EC₅₀.

### [Table 4-1]

### [Table 4-2]

**[Table 4-3]**

| Compound number | Activity | Compound number | Activity | Compound number | Activity |
|---|---|---|---|---|---|
| A001 | * | A002 | ** | A006 | ** |
| A007 | ** | A008 | ** | A009 | *** |
| A010 | *** | A011 | **** | A012 | ** |
| A013 | ^{∗} | A016 | ^{∗∗} | A018 | ^{∗∗∗} |
| A019 | ^{∗∗} | A021 | ^{∗} | A023 | ^{∗∗∗∗} |
| A025 | ^{∗} | A027 | ^{∗∗∗} | A028 | ^{∗} |
| A030 | ^{∗∗} | B002 | ^{∗∗} | B003 | ^{∗∗∗} |
| B004 | ^{∗∗∗} | B005 | ^{∗∗∗} | B008 | ^{∗∗} |
| B009 | ^{∗} | B010 | ^{∗∗} | B011 | ^{∗∗} |
| B012 | ^{∗} | B013 | ^{∗} | B014 | ^{∗∗} |
| B015 | ^{∗∗} | B016 | ^{∗∗} | B017 | ^{∗∗} |
| B018 | ^{∗∗} | B020 | ^{∗∗} | B021 | ^{∗∗} |
| B022 | ^{∗∗} | B023 | ^{∗∗∗} | B025 | ^{∗∗} |
| B026 | ^{∗∗} | B027 | ^{∗∗∗} | B028 | ^{∗∗∗∗} |
| B029 | ^{∗∗∗} | B030 | ^{∗∗} | B031 | ^{∗∗∗} |
| B032 | ^{∗∗∗} | BOSS | ^{∗∗∗} | B035 | ^{∗∗∗} |
| B036 | ^{∗∗} | B037 | ^{∗∗} | BOSS | ^{∗∗} |
| B039 | ^{∗∗∗} | B040 | ^{∗∗∗} | B041 | ^{∗∗∗} |
| B042 | ^{∗∗} | B043 | ^{∗∗} | B044a | ^{∗∗∗∗} |
| B044b | ^{∗∗∗∗} | B045a | ^{∗∗∗∗} | B045b | ^{∗∗∗∗} |
| B046a | ^{∗∗∗∗} | B046b | ^{∗∗∗∗} | B047 | ^{∗∗} |
| B048 | ^{∗∗} | B049 | ^{∗∗∗} | B050 | ^{∗∗∗} |
| B051 | ^{∗∗} | B052 | ^{∗∗} | B053 | ^{∗∗} |
| B054 | ^{∗∗} | B055 | ^{∗∗∗} | B056 | ^{∗∗} |
| B057 | ^{∗} | B059 | ^{∗} | B060 | ^{∗∗} |
| B061 | ^{∗∗∗} | B062 | ^{∗∗} | B063 | ^{∗∗} |
| B064 | ^{∗} | B065 | ^{∗∗} | B066 | ^{∗∗} |
| B067 | ^{∗∗∗} | B068 | ^{∗∗∗} | B069 | ^{∗∗} |
| B070 | ^{∗∗} | B071 | ^{∗∗} | B072 | ^{∗∗} |
| B073 | ^{∗∗∗} | B074 | ^{∗} | B075 | ^{∗} |
| B076 | ^{∗∗} | B077 | ^{∗∗} | B080 | ^{∗∗} |
| B081 | ^{∗∗} | B082 | ^{∗∗} | BOSS | ^{∗∗} |
| B084 | ^{∗∗} | B085 | ^{∗∗∗} | B087 | ^{∗} |
| BOSS | ^{∗∗∗} | B090 | ^{∗∗∗} | B091 | ^{∗∗∗} |
| B092 | ^{∗∗} | B093 | ^{∗∗∗} | B094 | ^{∗∗} |
| B095 | ^{∗} | B096 | ^{∗∗∗} | B098 | ^{∗∗} |
| B099 | ^{∗∗} | B100 | ^{∗∗∗} | B101 | ^{∗∗} |
| B102 | ^{∗∗∗} | B 103 | ^{∗∗} | C001 | ^{∗∗∗} |
| C002 | ^{∗∗} | C003 | ^{∗} | C004 | ^{∗∗} |
| C006 | ^{∗∗∗∗} | C009 | ^{∗∗} | C010 | ^{∗∗} |
| C011 | ^{∗∗∗} | C012 | ^{∗∗∗} | C015 | ^{∗∗} |
| C016 | ^{∗} | C017 | ^{∗∗} | C018 | ^{∗∗} |
| C019 | ^{∗∗∗} | C020 | ^{∗∗∗} | C021 | ^{∗} |
| C022 | ^{∗∗} | C023 | ^{∗∗∗} | C024 | ^{∗∗∗} |
| C025 | ^{∗∗} | C026 | ^{∗∗∗} | C029 | ^{∗} |
| C030b | ^{∗∗∗} | C031a | ^{∗∗∗∗} | C031b | ^{∗∗∗} |
| C033a | **** | C033b | *** | C034a | **** |
| C034b | ** | C035a | **** | C035b | ** |
| C036a | **** | C036b | ** | C037a | **** |
| C037b | ** | C039 | *** | C040 | ** |
| C041 | **** | C042a | **** | C043a | *** |
| C043b | * | C044a | ** | C044b | * |
| C045a | ** | C046a | **** | C046b | *** |
| C047a | *** | C047b | ** | C048 | ** |
| C049 | *** | C050 | *** | C051 | * |
| C052 | ** | C053 | *** | C054 | ** |
| C055 | ** | C056 | ** | C057 | *** |
| C058 | ** | C059 | ** | C060 | *** |
| D003 | ** | D005 | ** | D006 | ** |
| D007 | *** | D008 | *** | D009a | *** |
| D009b | ** | D010 | *** | D011 | *** |
| D012 | *** | D014 | *** | D015 | ** |
| D017 | ** | D019 | ** | D022 | ** |
| D023 | ** | D025 | ** | D026 | *** |
| D028 | *** | D029 | *** | D030 | *** |
| D031 | *** | D032 | ** | D033 | *** |
| D034 | ** | D035 | ** | D036 | *** |
| D039 | *** | D040 | *** | D041 | * |
| D042 | ** | D044 | *** | D045 | ** |
| D046 | ** | D049 | * | | |

From the results of the test, the vitamin D derivatives of the present invention shown in the above Table were confirmed to act on the vitamin D receptor (VDR).

On the other hand, the activity of derivatives of the present invention (compound F001 to I006) by this evaluation system showed EC₅₀ value of 100 nM or more in all the derivatives except compound G013. When the activity of 1α,25-dihydroxyvitamin D3 was evaluated by this evaluation system multiple times, the activity values showed EC₅₀ = 2.93 nM to 11.30 nM. From the above results, it was confirmed that the vitamin D derivative of the present invention (compound F001 to I006, except compound G013) has a weak activity (genomic action) on the vitamin D receptor.

### [Example 353]

### Evaluation of the promotive activity of rat oligodendrocyte progenitor cell differentiation-1

Regarding vitamin D derivatives, which was confirmed to act on the vitamin D receptor (VDR) in Example 352, the promotive activity of oligodendrocyte progenitor cell (OPC) differentiation was evaluated by MBP gene expression (mRNA).

### (1) Collection of rat oligodendrocyte progenitor cells

A forebrain obtained from SD rat (Charles River Japan, 1 day old) was crushed with 70 µm Cell Strainer (Falcon #352350), and a cell suspension was prepared by collecting the cells in oligodendrocyte progenitor cell separation medium (20% FBS/2 mM Glutamax (Gibco #35050-061)/1 mM sodium pyruvate (Gibco #11360-070)/1% penicillin-streptomycin (Invitrogen #15140-122)/DMEM (Gibco #11960-044)). The cell suspension was seeded in a poly-D-lysine coated T75 flask (Thermo Fisher Scientific #132704) and cultured for about 2 weeks. After culturing, the flask was shaken using a rotary shaker (WAKENYAKU #WB-101SRC) for about 1 hour (37°C, 100 rpm), and the supernatant was discarded to remove microglia. Subsequently, oligodendrocyte progenitor cell separation medium was added to the flask, and the mixture was similarly shaken for about 22 hours (37°C, 200 rpm), and the supernatant was collected to prepare oligodendrocyte progenitor cells.

### (2) Evaluation of oligodendrocyte progenitor cell differentiation

The oligodendrocyte progenitor cell prepared was suspended in a differentiation medium (2% B27 (Thermo Fisher Scientific, Inc. #17504-044)/1% penicillin-streptomycin (Invitrogen Corporation #15140-122)/10 ng/mL CNTF (PeproTech Inc. #AF-450-13)/DMEM (Gibco #11960-044)), and seeded on a 96-well plate coated with poly-L-ornithine (FUJIFILM Wako Pure Chemical Corporation #163-27421) under the condition of 0.65×10⁴ cells/well, and on a 384-well plate under the condition of 2×10³ cells/well using an automatic dispensing system (LABCYTE #Echo555). After cell seeding, the test compound (triiodothyronine (T₃) = 30 nM, vitamin D derivative of the present invention and GSK239512 = 1 µM) prepared in a differentiation medium were added from above and the cells were cultured for 4 days. After culturing, the cells seeded on the 96-well plate were subjected to immunostaining against MBP using following methods: fixation (4% paraformaldehyde solution, 30 minutes, room temperature), PBS washing, membrane permeation treatment (0.1% Trion X-100 (SIGMA #X100-100ML)/PBS, 3 minutes, room temperature), blocking (3% BSA (SIGMA#A9647-10G)/PBS, 1 hour, room temperature), primary antibody reaction (MBP antibody (abcam plc #ab40390), 2.5 µg/mL, 4°C, 24 hours), PBS washing, secondary antibody reaction (Alexa488 anti-rabbit IgG (Thermo Fisher Scientific, Inc. #A-11034), 5µg/mL, room temperature, 1 hour), PBS washing, and nuclear staining (Hoechst33342 (DOJINDO #NU043), 10 µg/mL, room temperature, 5 minutes). Subsequently, images of each well were acquired using a fluorescence microscope (KEYENCE CORPORATION #BZ-X800), the number of MBP-positive cells and Hoechst 33342-positive cells were measured using an image analysis application (KEYENCE CORPORATION #BZ-X800 Analyzer), and then the ratio (the number of MBP-positive cells/the number of Hoechst33342-positive cells) was calculated. The cells seeded on the 384-well plate were subjected to real-time PCR using Cells-to-CT 1-Step TaqMan Kit (Thermo Fisher Scientific, Inc. #A25602) and QuantStudio 12K Flex Real-Time PCR System (Thermo Fisher Scientific, Inc. #4472380). The experiment was performed in accordance with the protocol attached to the kit. MBP (Thermo Fisher Scientific, Inc. #Assay ID Rn00690616_m1) and β actin (Thermo Fisher Scientific, Inc. #Assay ID Rn00667869_m1) were used as primers, and the expression levels of each were measured and the ratio (MBP/β actin) was calculated. Analysis was performed using XLfit5 (IDBS), and the relative values of the average value of wells added with each compound were calculated by subtracting the average value of wells added with DMSO as background, and assuming the average value of wells added with triiodothyronine (T₃), a thyroid hormone, as 100%.

### (3) Result

In WO2013/107336 pamphlet, GSK239512 is reported to promote an oligodendrocyte progenitor cells (OPCs) differentiation. 1 µM of GSK239512 showed 1.8 times higher enhancement in the MBP gene expression compared with the wells added with DMSO in the evaluation of the present invention. This action intensity was shown in percentage (hereinafter referred to as T₃ base) assuming the average value of DMSO group as 0% and the average value of triiodothyronine (T₃) group as 100%, resulting a promotive activity of 18%. The 1α,25-dihydroxyvitamin D₃ showed no promotive activity in this evaluation (promoting activity of 0% at T₃ base). On the other hand, the derivative of the present invention at the amount of 1 µM showed the promotive activity of 20% or more at T₃ base in the following compounds: A019, B002, B004, B005, B006, B007, B008, B020, B021, B022, B023, B024, B026, B027, B028, B029, B030, B031, B032, B034, B035, B036, B037, B040, B041, B042, B043, B047, B048, B049, B051, B053, B054, B055, B056, B057, B058, B059, B061, B062, B063, B064, B067, B070, B073 B080, B081, B082, B086, B087, C001, C019, C022, C035a, C035b, C036a, C037a, C037b, C045a, C045b, C049, C051, C052, C053, C054, C055, C056, C057, D003, D004, D007, D008, D010, D012, D015, D017, D019, D023, D024, D025, D029, D030, D031, D032, D037, D038, D041, D043, D044, D047, D048. Furthermore, the promotive activity of 50% or more at T₃ base was shown in the following compounds: B005, B006, B007, B008, B020, B022, B023, B028, B029, B030, B031, B032, B034, B035, B036, B037, B041, B042, B043, B049, B053, B054, B056, B057, B058, B063, B064, B080, B081, B086, B087, C001, C019, C022, C036a, C037a, C049, C051, C053, C055, C056, C057, D019, D004, D010, D015.

From the above results, it was clarified that the vitamin D derivative of the present invention has a promotive activity equal to or higher than that of the well-known compound GSK239512.

### [Example 354]

### Evaluation of the promotive activity of rat oligodendrocyte progenitor cell differentiation-2

The promotive activity of oligodendrocyte progenitor cell (OPC) differentiation of the vitamin D derivatives was evaluated by immunostaining of MBP.

### (1) Collection of rat oligodendrocyte progenitor cells

Afore-brain obtained from SD rats (Charles River Japan, 1 day old) was crushed with a 70 µm cell strainer (Falcon #352350), and a cell suspension was prepared by collecting the cells in oligodendrocyte progenitor cell separation medium (20% FBS/2 mM Glutamax (Gibco #35050-061)/1 mM sodium pyruvate (Gibco #11360-070)/1% penicillin-streptomycin (Invitrogen #15140-122)/DMEM (Gibco #11960-044)). The cell suspension was seeded in a poly-D-lysine coated T75 flask (Thermo Fisher Scientific #132704) and cultured for 10 days. After culturing, the flask was shaken using a rotary shaker (WAKENYAKU #WB-101SRC) for about 1 hour (37°C, 100 rpm), and the supernatant was discarded to remove microglia. Subsequently, oligodendrocyte progenitor cell separation medium was added to the flask, and the mixture was similarly shaken for about 22 hours (37°C, 200 rpm), and the supernatant was collected to prepare oligodendrocyte progenitor cells.

### (2) Evaluation of oligodendrocyte progenitor cell differentiation

The oligodendrocyte progenitor cells prepared were suspended in a differentiation medium (2% B27 (Thermo Fisher Scientific, Inc. #17504-044)/1% penicillin-streptomycin (Invitrogen Corporation #15140-122)/1% sodium pyruvate (FUJIFILM Wako Pure Chemical Corporation #191-03061)/10 ng/mL CNTF (PeproTech Inc. #AF-450-13)/DMEM (Gibco #11960-044)), and seeded on a 96-well plate coated with poly-L-ornithine (FUJIFILM Wako Pure Chemical Corporation #163-27421) under the condition of 0.65×10⁴ cells/well. After cell seeding, the test compound (triiodothyronine (T₃) = 30 nM, vitamin D derivative of the present invention = 2 µM) prepared in a differentiation medium were added from above and the cells were cultured for 4 days. After culturing, the cells were subjected to immunostaining against MBP using following methods: fixation (4% paraformaldehyde solution, 30 minutes, room temperature), PBS washing, membrane permeation treatment (0.1% Trion X-100 (SIGMA #X100-100ML)/PBS, 3 minutes, room temperature), blocking (3% BSA (SIGMA#A9647-10G)/PBS, 1 hour, room temperature), primary antibody reaction (MBP antibody (abcam plc #ab40390), 2.5 µg/mL, 4°C, 24 hours), PBS washing, secondary antibody reaction (Alexa488 anti-rabbit IgG (Thermo Fisher Scientific, Inc. #A-11034), 5µg/mL, room temperature, 1 hour), PBS washing, and nuclear staining (Hoechst33342 (DOJINDO #NU043), 10µg/mL, room temperature, 5 minutes). Subsequently, images of each well were acquired using a fluorescence microscope (KEYENCE CORPORATION #BZ-X800), the number of MBP-positive cells and Hoechst 33342-positive cells were measured using an image analysis application (KEYENCE CORPORATION #BZ-X800 Analyzer), and then the ratio (the number of MBP-positive cells/the number of Hoechst33342-positive cells) was calculated.

### (3) Result

The results are shown by the following symbols in the table below. Note that the significant differences in the table below were obtained by the t-test performed in the DMSO group.

| | |
|---|---|
| * | <0.05 |
| ** | <0.01 |
| *** | <0.001 |
| **** | <0.0001 |
| ns | No significant difference |

**[Table 5-1]**

| Test 1 | | | |
|---|---|---|---|
| Compound name | Mean | Standard deviation (S.D.) | Significant difference |
| DMSO | 0.09 | 0.02 | |
| T3 | 0.49 | 0.03 | **** |
| Compound B020 | 0.38 | 0.02 | **** |
| Compound B022 | 0.47 | 0.05 | **** |
| Compound B023 | 0.50 | 0.04 | **** |
| Compound B024 | 0.28 | 0.01 | **** |
| Compound B026 | 0.38 | 0.03 | **** |
| Compound B027 | 0.29 | 0.03 | **** |
| Compound B029 | 0.31 | 0.05 | **** |
| Compound B034 | 0.57 | 0.03 | **** |
| Compound B036 | 0.59 | 0.05 | **** |
| Compound B037 | 0.49 | 0.07 | **** |
| Compound B040 | 0.59 | 0.03 | **** |
| Compound B041 | 0.56 | 0.05 | **** |
| Compound B042 | 0.62 | 0.02 | **** |

**[Table 5-2]**

| Test 2 | | | |
|---|---|---|---|
| Compound name | Mean | Standard deviation (S.D.) | Significant difference |
| DMSO | 0.07 | 0.03 | |
| T3 | 0.46 | 0.09 | **** |
| Compound B042 | 0.53 | 0.03 | **** |
| Compound B051 | 0.45 | 0.03 | **** |
| Compound B055 | 0.56 | 0.02 | **** |
| Compound B057 | 0.57 | 0.06 | **** |
| Compound B058 | 0.49 | 0.03 | **** |
| Compound B064 | 0.61 | 0.03 | **** |
| Compound C036a | 0.53 | 0.07 | **** |
| Compound C036b | 0.22 | 0.03 | *** |
| Compound C055 | 0.48 | 0.03 | **** |
| Compound D019 | 0.57 | 0.04 | **** |
| Compound D023 | 0.36 | 0.07 | **** |
| Compound D024 | 0.39 | 0.06 | **** |

**[Table 5-3]**

| Test 3 | | | |
|---|---|---|---|
| Compound name | Mean | Standard deviation (S.D.) | Significant difference |
| DMSO | 0.11 | 0.02 | |
| T3 | 0.40 | 0.04 | **** |
| Compound G001 | 0.34 | 0.03 | **** |
| Compound G002 | 0.33 | 0.06 | **** |
| Compound G003 | 0.47 | 0.03 | **** |
| Compound G004 | 0.27 | 0.05 | **** |
| Compound G005 | 0.34 | 0.05 | **** |
| Compound F004 | 0.37 | 0.04 | **** |
| Compound F005 | 0.40 | 0.04 | **** |

**[Table 5-4]**

| Test 4 | | | |
|---|---|---|---|
| Compound name | Mean | Standard deviation (S.D.) | Significant difference |
| DMSO | 0.16 | 0.02 | |
| T3 | 0.44 | 0.03 | **** |
| Compound F006 | 0.44 | 0.10 | **** |
| Compound G006 | 0.50 | 0.04 | **** |
| Compound G007 | 0.34 | 0.05 | **** |
| Compound I004 | 0.44 | 0.04 | **** |
| Compound I005 | 0.43 | 0.04 | **** |
| Compound I006 | 0.51 | 0.05 | **** |

**[Table 5-5]**

| Test 5 | | | |
|---|---|---|---|
| Compound name | Mean | Standard deviation (S.D.) | Significant difference |
| DMSO | 0.17 | 0.03 | |
| T3 | 0.45 | 0.05 | **** |
| Compound F008 | 0.45 | 0.04 | **** |
| Compound F011 | 0.26 | 0.04 | *** |
| Compound G008 | 0.36 | 0.06 | **** |
| Compound G011 | 0.22 | 0.02 | ** |
| Compound G012 | 0.22 | 0.04 | * |
| Compound H011 | 0.30 | 0.05 | *** |

**[Table 5-6]**

| Test 6 | | | |
|---|---|---|---|
| Compound name | Mean | Standard deviation (S.D.) | Significant difference |
| DMSO | 0.16 | 0.05 | |
| T3 | 0.56 | 0.09 | **** |
| Compound G013 | 0.55 | 0.08 | **** |
| Compound G014 | 0.44 | 0.05 | **** |
| Compound G015 | 0.52 | 0.06 | **** |
| Compound G017 | 0.52 | 0.05 | **** |
| Compound G018 | 0.48 | 0.04 | **** |

**[Table 5-7]**

| Test 7 | | | |
|---|---|---|---|
| Compound name | Mean | Standard deviation (S.D.) | Significant difference |
| DMSO | 0.18 | 0.06 | |
| T3 | 0.41 | 0.03 | **** |
| Compound F001 | 0.29 | 0.06 | ** |
| Compound F002 | 0.31 | 0.06 | ** |
| Compound F003 | 0.32 | 0.08 | ** |
| Compound F012 | 0.23 | 0.05 | ns |
| Compound G016 | 0.37 | 0.07 | *** |
| Compound H001 | 0.48 | 0.05 | **** |
| Compound H012 | 0.28 | 0.04 | ** |

**[Table 5-8]**

| Test 8 | | | |
|---|---|---|---|
| Compound name | Mean | Standard deviation (S.D.) | Significant difference |
| DMSO | 0.20 | 0.03 | |
| T3 | 0.41 | 0.05 | **** |
| Compound F014 | 0.29 | 0.06 | *** |
| Compound F015 | 0.35 | 0.07 | *** |
| Compound F016 | 0.32 | 0.08 | *** |
| Compound H013 | 0.35 | 0.04 | **** |
| Compound H014 | 0.37 | 0.04 | **** |
| Compound H015 | 0.34 | 0.04 | **** |
| Compound H016 | 0.30 | 0.06 | ** |

**[Table 5-9]**

| Test 9 | | | |
|---|---|---|---|
| Compound name | Mean | Standard deviation (S.D.) | Significant difference |
| DMSO | 0.19 | 0.02 | |
| T3 | 0.43 | 0.05 | **** |
| Compound F013 | 0.31 | 0.04 | **** |
| Compound H002 | 0.39 | 0.08 | **** |
| Compound H003 | 0.30 | 0.04 | *** |
| Compound I001 | 0.30 | 0.05 | *** |
| Compound I002 | 0.31 | 0.04 | **** |
| Compound I003 | 0.33 | 0.06 | **** |

As shown in table 5-1 and 5-2, vitamin D derivatives, which was confirmed to act on the vitamin D receptor (VDR) in Example 352, promoted OPC differentiation in the evaluation by immunostaining of MBP. In addition, as shown in Table 5-3 to table 5-9, vitamin D derivatives, which was confirmed to show weak activity on the vitamin D receptor (VDR) in Example 352, also promoted OPC differentiation. As a result of this study, it was clarified that the vitamin D derivatives in the present invention promote OPC differentiation independent of the activity on vitamin D receptor (VDR).

### [Example 355]

### The promoting activity of compounds in oligodendrocyte differentiation of mouse neural stem cell (mNSC)

The evaluation was carried out as follows in accordance with the method reported in NPL 2 (H. A. Shirazi et al., Experimental and Molecular Pathology, 2015, 98 (2), 240-245).

### (1) Cell preculture

OriCell Strain C57BL/6 mouse neural stem cells (NSCs) (Cyagen Biosciences Inc., Cat. No. MUBNF-01001) were used as mouse neural stem cells. OriCell NSC growth medium (Cyagen Biosciences Inc., Cat. No. GUXNX-90011) was used as the growth medium. To NSC basal medium (100 mL) included in this product, B27 (1 mL), Penicillin-Streptomycin (1 mL), Glutamine (1 mL), bFGF (20 µL), EGF (10 µL), and Heparin (100 µL) were added to prepare the growth medium. Mouse neural stem cells were reconstituted in a T-25 flask using the growth medium and proliferated as a neurosphere by suspension culture. The cells were expanded by passaging at a split ratio = 1:2 in a CO₂ incubator (5% CO₂, 37°C, wet) to obtain the required number of cells. ACCUTASE (Innovative Cell Technologies, Cat. No. AT104) was used as the passage reagent.

### (2) Sample preparation

OriCell NSC growth medium (Cyagen, Cat. No. GUXNX-90011) was used as the differentiation medium. To the NSC basal medium (100 mL) included in the product, B27 (1 mL), Penicillin-Streptomycin (1 mL), Glutamine (1 mL), and then FBS (2 mL) (Cell Culture Bioscience, Cat. No. 171012) were added to prepare the differentiation medium. The differentiation medium containing test substance was prepared to a target final concentration by diluting a solution of 20 mM compound in DMSO to 10-fold with DMSO and then to 2×10⁵-fold with the differentiation medium. Additive-free differentiation medium for control was prepared by diluting DMSO to 2×10⁵-fold with the differentiation medium to be a final concentration of 5×10⁻⁵%.

### (3) Differentiation induction and sample processing

The mouse neural stem cells were seeded on a poly-L-Lysine/Laminin-coated 96-well plate at a cell density of 2.0×10⁴ cells/100 µL/well using the differentiation medium and the culture was started in a CO₂ incubator (5% CO₂, 37°C, wet). The next day, the medium was replaced with the differentiation medium containing a test substance or an additive-free differentiation medium for control, and evaluation of the test substance (N = 5) was started (day 0). The medium was replaced every other day by 60% of the medium in the wells. On day 10, cells were fixed and immunostained.

### (4) Immunostaining

After the medium was removed, the cells were fixed by adding 100 µL of 4% paraformaldehyde-PBS and being incubated at room temperature for 30 minutes. Next, the cells were washed 3 times with PBS, 100 µL of 5% Goat Serum/PBS was added thereto, and the mixture was incubated at room temperature for 30 minutes to perform blocking and membrane permeation treatment. Next, the solution was replaced with 100 µL of a primary antibody solution (a solution of 3% BSA/PBS added with 1/200 times the amount of anti-GalC antibody (mouse IgG3, Millipore, Cat. No. MAB342)) and incubated at 4°C overnight. After the cells were washed 3 times with 3% BSA/PBS solution, the solution was replaced with 100 µL of a secondary antibody solution (a solution of 3% BSA/PBS added with 1/500 times the amount of secondary antibody Alexa Fluor 488 Goat Anti-mouse IgG (H+L) (Thermo Fisher Scientific, Inc., Cat. No. A-11001) and with 1/1000 times the amount of Hoechst 33342 (Thermo Fisher Scientific, Inc., Cat. No. H3570)) and incubated at room temperature for 2 hours. After the cells were washed 3 times with PBS, 100 µL of PBS was added to the wells, and the wells were photographed with fluorescence microscopy. Images were taken under the same condition in all wells, and blue fluorescence and green fluorescence were simultaneously photographed.

### (5) Quantification of cell differentiation ratio

Using the hybrid cell counting software provided in the fluorescence microscope (Keyence, BZ-X700), co-localized spots of green fluorescence and blue fluorescence were extracted, and the number of these spots was defined as the total number of GalC-positive cells, and the oligodendrocyte differentiation ratio was calculated by the following calculation: the total number of GalC-positive cells/the number of nuclei (number of blue fluorescence spots). A significant difference test was performed by t-test between the additive-free group and the sample-added group.

### (6) Result

The results are shown in the following table. In this experiment, treatment of neural stem cells (NSC) with 1000 nM of 1α,25-dihydroxyvitamin D₃ (1α,25(OH)₂D₃) showed 1.5 times of promoting activity in a Gal/C-positive oligodendrocyte differentiation. This was almost the same result as in NPL 2. On the other hand, the derivative of the present invention also showed a promoting activity in oligodendrocyte differentiation equal to or higher than that of 1α,25(OH)₂D₃

**[Table 6]**

| Test 1 | | | | |
|---|---|---|---|---|
| Compound name | Concentration (nM) | Gal/C positive ratio Average value (standard error) | P value | Significant difference |
| Vehicle | - | 8.38 (0.50) | | |
| 1α,25(OH)₂D₃ | 10 | 12.10 (0,80) | 0.00439 | ** |
| | 100 | 12.36 (0.80) | 0.00295 | ** |
| | 1000 | 13.17 (0.49) | 0.00013 | *** |
| D004 | 10 | 12.61 (0.44) | 0.00022 | *** |
| | 100 | 15.55 (0.39) | 0.000003 | *** |
| | 1000 | 16.76 (0.76) | 0.000015 | *** |
| D023 | 10 | 8.63 (0.47) | 0.71990 | |
| | 100 | 11.51 (1.38) | 0.66065 | |
| | 1000 | 12.19 (0.67) | 0.00192 | ** |
| D042 | 10 | 12.25 (0.62) | 0.00127 | ** |
| | 100 | 14.27 (1.76) | 0.01249 | * |
| | 1000 | 15.80 (0.44) | 0.000003 | *** |
| D043 | 10 | 12.81 (0.68) | 0.00079 | *** |
| | 100 | 12.27 (0.91) | 0.00567 | ** |
| | 1000 | 16.09 (1.88) | 0.00415 | ** |
| D044 | 10 | 9.10 (0.85) | 0.48634 | |
| | 100 | 8.22 (0.47) | 0.81953 | |
| | 1000 | 12.37 (0.99) | 0.00717 | ** |
| D045 | 10 | 11.46 (0.72) | 0.00820 | ** |
| | 100 | 12.01 (0.69) | 0.00288 | ** |
| | 1000 | 14.93 (1.29) | 0.00149 | ** |

| | | | | |
|---|---|---|---|---|
| (*: <0.05, **: <0.01, ***: <0.005) | | | | |

### [Example 356]

### Evaluation of promoting activity in oligodendrocyte progenitor cells differentiation using Vitamin D receptor (VDR) knockout mice

The promotive activity of oligodendrocyte progenitor cell (OPC) differentiation of the vitamin D derivatives was evaluated by using oligodendrocyte progenitor cells derived from VDR knockout mice.

### (1) Collection of neural stem cells from VDR KO mouse

The basal ganglia primordium obtained from VDRKO_Alop_fs1 (VDR V256fs) Mouse (Tokushima University, 16 days fetal age) was crushed with a 70 µm cell strainer (Falcon #352350) and a cell suspension was prepared by recovering in mouse neural stem cell growth medium (2 mM Glutamax (Gibco #35050-061)/1% B27 (Thermo Fisher Scientific #17504-044)/1% penicillin-streptomycin (Invitrogen #15140-122)/40 µg/mL basic fibroblast growth factor recombinant human (R&D #236-EG)/20 µg/mL Epidermal Growth Factor recombinant human (R&D #233-FB/CF)/50 IU/mL heparin sodium (FUJIFILM Wako Pure Chemical Corporation #081-00136)/DMEM/F-12, no glutamine (Gibco #21331-020)). The cell suspension was seeded in a T75 flask (Thermo Fisher Scientific #132704), and cell expansion was carried out. The cells obtained by the expansion culture were used as neural stem cells.

### (2) Collection of oligodendrocyte progenitor cells from the neural stem cells derived from VDR KO mouse

The neural stem cells were suspended in neural stem cell differentiation medium (2 mM Glutamax (Gibco #35050-061)/2% B27 (Thermo Fisher Scientific #17504-044)/1% penicillin-streptomycin (Invitrogen #15140-122)/1% Fetal bovine serum (SIGMA-ALDRICH #173012-500ML)/DMEM/F-12, no glutamine (Gibco #21331-020)), and were seeded under the condition of 10×10⁷ cells/dish in a 15 cm dish coated with poly-D-lysine (SIGMA-ALDRICH #P7886)/laminin (SIGMA-ALDRICH #L2020). After seeding, the medium was changed every 3 to 4 days, cells were recovered after the differentiation culture for 12 days, and PDGFRα-positive cells were recovered using CD140a (PDGFRα) MicroBeads kit, mouse (Miltenyi Biotec #130-101-502) to prepare VDR KO mouse oligodendrocyte progenitor cells.

### (3) Evaluation of oligodendrocyte progenitor cell differentiation

The oligodendrocyte progenitor cells derived from VDR KO mouse prepared were suspended in oligodendrocyte progenitor cell differentiation medium (2% B27 (Thermo Fisher Scientific #17504-044)/1% penicillin-streptomycin (Invitrogen #15140-122)/10ng/mL CNTF (PeproTech #AF-450-13)/DMEM/F-12, no glutamine (Gibco #21331-020)), and were seeded on a 96-well plate coated with poly-L-ornithine (FUJIFILM Wako Pure Chemical Corporation #163-27421) under the condition of 2.0×10⁴ cells/well. After cell seeding, the test compound (vitamin D derivative of the present invention = 1000 nM) prepared in a differentiation medium was added from above and the cells were cultured for 4 days. After culturing, the cells were subjected to immunostaining against MBP using the following methods: fixation (4% paraformaldehyde solution, 30 minutes, room temperature), PBS washing, membrane permeation treatment (0.1% Trion X-100 (SIGMA-ALDRICH #X100-100ML)/PBS, 3 minutes, room temperature), Blocking (5% Normal goat serum (Abcam #ab7481)/PBS, 1 hour, room temperature), primary antibody reaction (MBP antibody (BIO-RAD #MCA409S), 100-fold dilution, 4°C, 24 hours), PBS washing, secondary antibody reaction (Alexa488 anti-rat IgG (Thermo Fisher Scientific #A-11006), 4 µg/mL, room temperature, 1 hour), PBS washing, nuclear staining (Hoechst33342 (DOJINDO #NU043), 1 µg/mL, room temperature, 15 minutes). Subsequently, images of each well were acquired using a fluorescence microscope (KEYENCE CORPORATION #BZ-X800), the number of MBP-positive cells and Hoechst 33342-positive cells (total cells) were measured using an image analysis application (KEYENCE CORPORATION #BZ-X800 Analyzer), and then the ratio of number of MBP-positive cells/number of Hoechst33342-positive cells (total cells) was calculated, and then the ratio of those to the DMSO group was calculated as a result.

### (4) Result

The results are shown by the following symbols in the table below. Note that the significant differences in the table below were obtained by the t-test performed in the DMSO group "*" with a significant difference of <0.05.

**[Table 7]**

| Compound name | Mean | Standard deviation (S.D.) | Significant difference |
|---|---|---|---|
| DMSO | 100.0 | 22.2 | |
| Compound G005 | 215.1 | 34.9 | * |

The compound of the present invention also promoted of oligodendrocyte progenitor cells derived from VDR knockout mice. From the results of this test, it was clarified that VDR is not required for oligodendrocyte progenitor cells differentiation.

### [Example 357]

### Evaluation of calcium increasing action in normal mice

Calcium increasing action of the vitamin D derivatives of the present invention confirmed to have a weak activity (genomic action) on the vitamin D receptor (VDR) in Example 352 was evaluated.

After acclimatizing C57BL/6J mice (7 weeks old, male) for 1 week, the mice were divided into groups of 5 mice based on the body weight, and administration of the compound or vehicle was started. The administration solution used was a compound (in ethanol) or ethanol diluted 100-fold with 0.1% Trion X-100 (SIGMA #X100-100ML)/saline, and the repeated oral administration at once a day was performed. After administration of 2 weeks, euthanasia was performed by collecting all blood under general anesthesia. The calcium concentration of the obtained serum sample was measured with an automatic analyzer (Hitachi automatic analyzer 7180).

### Result

The calcium increasing action of the compound of the present invention is shown in the table below.

**[Table 8]**

| Test 1 | | | |
|---|---|---|---|
| Compound | Dose (µg/kg) | Serum calcium concentration (mg/dL) | Calcium concentration Fluctuation range (mg/dL) |
| Vehicle | - | 8.9 | |
| 1α,25-dihydroxyvitamin D₃ | 0.6 | 10.2 | 1.3 |
| | 2 | 12.9 | 4.0 |
| Compound G004 | 20 | 8.9 | 0 |
| | 60 | 8.8 | -0.1 |
| | 200 | 9.0 | 0.1 |
| Compound G005 | 20 | 9.1 | 0.2 |
| | 60 | 9.1 | 0.2 |
| | 200 | 9.0 | 0.1 |
| Compound G006 | 20 | 8.9 | 0 |
| | 60 | 8.9 | 0 |
| | 200 | 8.6 | -0.3 |

In this test, administration of 1α,25-dihydroxyvitamin D₃ increased serum calcium concentration from a low dose at 0.6 µg/kg, and the fluctuation range of calcium concentration was 4.0 mg/dL at a dose of 2 µg/kg, resulting in the state of hypercalcemia. On the other hand, the compound of the present invention, having very weak activity (genomic action) to the vitamin D receptor, did not increase serum calcium concentration even at a dose 10 times or more higher than that of 1α,25-of-dihydroxyvitamin D₃ (2.0 µg/kg). This result clearly showed that the derivative of the present invention was dissociated from the calcium action.

### [Example 358]

### Evaluation of brain penetration in mice

### Test 1 [1 mg/kg administration study]

The test compound (10 mg/mL in ethanol) was diluted 100-fold with 0.1% Trion X-100 (SIGMA #X100-100MI,)/saline to prepare an administration solution. The administration solution was administered orally to C57BL/6J mice (8 weeks old, male, n = 3) at a single dose (1 mg/kg). Mice were euthanized at 6 points of 0.25, 0.5, 1, 2, 4, and 8 hours after administration, and plasma and brain sampling were performed. After preparing a homogenate from the collected brain using Tissue Lyser II (QIAGEN #85300), acetonitrile was added to the homogenate and the homogenate was centrifuged (12000 rpm, 5 minutes) to extract the administered test compound contained in the brain. Similarly, the plasma was subjected to addition of acetonitrile and centrifugal separation to extract the administered test compound contained in the plasma. Each extracted sample was subjected to measurement of the concentration of administered test compound using LC/MS/MS (SHIMADZU UFLC/MS/MS (8050)).

The following table shows the results (mean ± standard deviation (S.D.))of measurement of the concentration in blood and the concentration in brain of each evaluated derivative at the maximum value of the concentration in brain (brain Cmax). In addition, the table shows the ratio of concentration in brain/concentration in blood which are obtained by dividing each brain concentration (average value) by the blood concentration (average value)).

### [Table 9-1]

**[Table 9-1]**

| Compound | At brain Cmax | | Concentration ratio in brain/in blood |
|---|---|---|---|
| | Concentration in blood (mean ± SD, nM) | Concentration in brain (mean ± SD, pmol/g) | |
| B018 | 841±197 | 533±197 | 0.63 |
| B020 | 676±175 | 410±23 | 0.61 |
| B021 | 320±53 | 237±33 | 0.74 |
| B022 | 316±84 | 529±53 | 1.67 |
| B023 | 248±28 | 852±48 | 3.44 |
| B024 | 431±46 | 652±85 | 1.51 |
| B026 | 218±31 | 384±40 | 1.76 |
| B027 | 126±14 | 414±40 | 3.29 |
| B029 | 161±40 | 156±15 | 0.97 |
| B034 | 251±9 | 562±50 | 2.24 |
| B036 | 61±11 | 68±36 | 1.12 |
| B037 | 88±19 | 74±17 | 0.84 |
| B040 | 70±10 | 153±35 | 2.18 |
| B041 | 207±59 | 317±19 | 1.53 |
| B042 | 79±7 | 60±5 | 0.75 |
| B043 | 74±1 | 80±9 | 1.08 |
| B045a | 530±97 | 185±116 | 0.35 |
| B045b | 444±113 | 109±22 | 0.25 |
| B051 | 304±35 | 644±92 | 2.12 |
| B055 | 309±17 | 327±28 | 1.06 |
| B057 | 271±16 | 141±8 | 0.52 |
| B058 | 78±15 | 67±9 | 0.86 |
| B064 | 72±11 | 38±12 | 0.53 |
| C002 | 402±38 | 156±14 | 0.39 |
| C006 | 510±55 | 239±23 | 0.47 |
| C019 | 335±85 | 614±153 | 1.83 |
| C036a | 849±40 | 566±89 | 0.67 |
| C036b | 853±89 | 495±16 | 0.58 |
| C039 | 132±15 | 101±8 | 0.77 |
| C055 | 190±23 | 338±36 | 1.78 |
| C059 | 167±29 | 241±21 | 1.44 |
| D019 | 280±53 | 811±163 | 2.89 |
| D023 | 580±107 | 595±23 | 1.03 |
| D024 | 368±26 | 726±88 | 1.97 |

### Test 2 [0.2 mg/kg administration study]

The test compound (2 mg/mL in ethanol) was diluted 100-fold with 0.1% Trion X-100 (SIGMA #X100-100MI,)/saline to prepare an administration solution. The administration solution was administered orally to C57BL/6J mice (8 weeks old, male, n = 3) at a single dose (0.2 mg/kg). Mice were euthanized at 6 points of 0.25, 0.5, 1, 2, 4, and 8 hours after administration, and plasma and brain sampling were performed. After preparing a homogenate from the collected brain using Tissue Lyser II (QIAGEN #85300), acetonitrile was added to the homogenate and the homogenate was centrifuged (12000 rpm, 5 minutes) to extract the administered compound contained in the brain. Similarly, the plasma was subjected to addition of acetonitrile and centrifugal separation to extract the administered test compound contained in the plasma. Each extracted sample was subjected to measurement of the concentration of administered test compound using LC/MS/MS (SHIMADZU UFLC/MS/MS (8050)).

The following table shows the results (mean ± standard deviation (S.D.)) of measurement of the concentration in blood and the concentration in brain of each evaluated derivative at the maximum value of the concentration in brain (brain Cmax). In addition, the table shows the ratio of concentration in brain/concentration in blood which are obtained by dividing each brain concentration (average value) by the blood concentration (average value)).

**[Table 10]**

| Compound | At brain Cmax | | Concentration ratio in brain/in blood |
|---|---|---|---|
| | Concentration in blood (nM) | Concentration in brain (pmol/g) | |
| G004 | 72.2±10.4 | 171.2±37.4 | 2.35 |
| G005 | 84.1±9.7 | 140.8±17.6 | 1.67 |
| G006 | 58.9±0.5 | 137.8±11.5 | 2.38 |

All of the compounds of the present invention showed a ratio of concentration in brain/concentration in blood of 0.25 to 3.44 even under the condition of oral administration. According to NPL 5, the ratio of concentration in brain/concentration in blood of 1α,25-dihydroxyvitamin D₃ is reported as 0.007 ± 0.003. Thus, the derivatives of the present invention can be demonstrated to have significantly improved central nervous system penetration compared with 1α,25-dihydroxyvitamin D₃.

### [Example 359]

### Evaluation of myelin regeneration promoting action in mouse cuprizone model

C57BL/6J mice (8 weeks old, male) were fed a diet containing 0.3% cuprizone (bis(cyclohexanone)oxaldihydrazone) (Research Diets, Inc. #D17042709) for 6 weeks to induce demyelination. Subsequently, the mice were divided into groups of 8 mice based on the body weight, the diet was switched to a cuprizone-free diet (Research Diets, Inc. #D10001), and administration of the test compound or vehicle was started. The administration solution was a compound (in ethanol) or ethanol diluted 100-fold with 0.1% Trion X-100 (SIGMA #X100-100MI,)/saline, and the repeated oral administration at once a day was performed. After administration of 4 weeks, the mice were euthanized, perfused with a 4% paraformaldehyde solution, and then brain sampling was performed. The brain sample obtained was embedded in paraffin to prepare a coronal section specimen at Bregma +1. The specimen was stained with Luxor Fast Blue (LFB) to visualize the myelin sheath, and images were acquired using a fluorescence microscope (KEYENCE #BZ-X710). The corpus callosum area and the LFB staining positive area in the corpus callosum of the obtained pathological specimen image were measured using an image analysis application (KEYENCE #BZ-H3C/BZ-H3XD), and the ratio (LFB staining positive area in the corpus callosum/ corpus callosum area) was calculated. The intact group is a non-pathogenic group, and the intact group and the control group were sampled after feeding the diet containing 0.3% cuprizone for 6 weeks.

### Result

The results are shown in the following table. In the table, the ratio of LFB staining positive area in the corpus callosum/ corpus callosum area of the untreated group, fed with the cuprizone-free diet for 6 weeks, was regarded as 100%. As a result, the control group, fed with the cuprizone-containing diet for 6 weeks, decreased the ratio to 23.6%, and was confirmed to progress demyelination. The group, in which the cuprizone-containing diet was fed for 6 weeks and switched to the cuprizone-free diet, and the vehicle was administered, improved the ratio of area of LFB staining positive/area of corpus callosum to 50.1%. On the other hand, among the groups in which the cuprizone-containing diet was fed for 6 weeks and switched to the cuprizone-free diet, the compound B034, B043, and D023 administration groups improved the ratios of area of LFB staining positive/area of corpus callosum to 88.1%, 84.9%, and 87.0%, respectively, and significantly promoted demyelination regeneration as compared with 50.1% in the vehicle-administered group. In addition, GSK239512 described in WO2013/107336 pamphlet was evaluated in the test to show the ratio of LFB staining positive area/corpus callosum area of 45.8%, in which no demyelination regeneration promoting action was shown as compared with the vehicle-administered group.

**[Table 11]**

| Compound name | Dose | Ratio (%): area of LFB staining positive/area of corpus callosum | |
|---|---|---|---|
| | | mean | S.D. |
| Untreated | | 100.0 | 14 |
| Control | | 23.6 | 5 |
| Vehicle | | 50.1 | 16 |
| Compound B034 | 6 µg/kg | 88.1 | 26 |
| Compound B043 | 2 µg/kg | 84.9 | 18 |
| Compound D023 | 20 µg/kg | 87.0 | 26 |
| GSK239512 | 10 mg/kg | 45.8 | 24 |

From the above results, it was revealed that the vitamin D derivative of the present invention has a stronger promoting remyelination activity than the well-known compound (GSK239512) which is reported to show a promoting activity in oligodendrocytes progenitor cells differentiation.

## Claims

1. A vitamin D derivative represented by the following formula (1) or a pharmaceutically acceptable salt or solvate thereof. 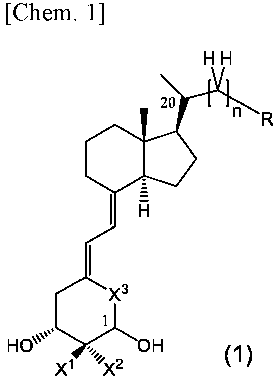 [In the formula, R represents any structure of Ra, Rb, Rc, Rd, or Re in the following formulas. 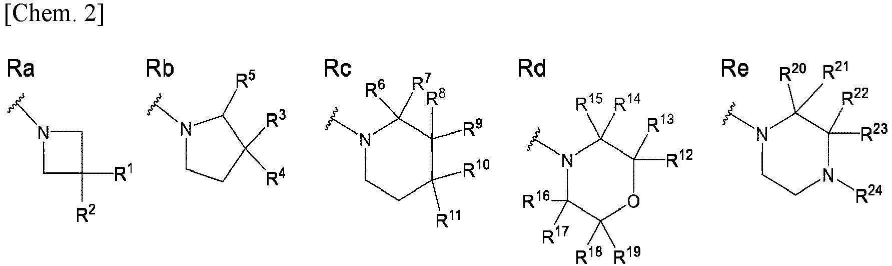
R¹, R³, R⁸ and R¹⁰ each independently represent a C₁ to C₆ alkyl group optionally substituted with 1 to 3 halogen atoms, a C₃ to C₆ cycloalkyl group, a C₁ to C₆ alkoxy group optionally substituted with 1 to 3 halogen atoms, a halogen atom, or a hydrogen atom.
R², R⁴, R⁹ and R¹¹ each independently represent a hydrogen atom, a hydroxy group, or a C₁ to C₆ alkyl group optionally substituted with 1 to 3 halogen atoms. (Wherein, when R¹, R³, R⁸ and R¹⁰ each independently represent a C₁ to C₆ alkoxy group optionally substituted with 1 to 3 halogen atoms, or a halogen atom, each of R², R⁴, R⁹ and R¹¹, substituting the same carbon atom together with R^{1,} R^{3,} R⁸, or R¹⁰, is not a hydroxy group.)
R⁶, R⁷, R¹², R¹³, R¹⁴, R¹⁵, R¹⁶, R¹⁷, R¹⁸ R¹⁹, R²⁰, R²¹, R²², and R²³, each independently represent a hydrogen atom, a C₁ to C₆ alkyl group optionally substituted with 1 to 3 halogen atoms, or a C₃ to C₆ cycloalkyl group.
Each pair of R¹ and R², R³ and R⁴, R⁶ and R⁷, R¹⁰ and R¹¹, R¹² and R¹³, R¹⁴ and R¹⁵, R¹⁶ and R¹⁷, R¹⁸ and R¹⁹, R²⁰ and R²¹, and R²² and R²³ can be bonded with each other to form a 3- to 5-membered ring structure.
R⁵ represents a hydrogen atom, a C₁ to C₆ alkyl group optionally substituted with a -OR⁵⁰¹ or a C₃ to C₆ cycloalkyl group optionally substituted with a -OR⁵⁰¹, and R⁵⁰¹ represents a hydrogen atom or a C₁ to C₆ alkyl group.
R²⁴ represents a hydrogen atom, a C₁ to C₃ alkyl group or a C₁ to C₃ alkylsulfonyl group.
The stereochemistry at C-2 in the pyrrolidine ring (Rb) represents either (R) configuration or (S) configuration.
X¹ and X² each independently represent a hydrogen atom or a C₁ to C₃ alkyl group, or X¹ and X² together form a methylidene group, or -(CH₂)ₘ- (wherein m is an integer of 2 to 5).
X³ represents a CH₂ group, or a C=CH₂ group (wherein, when X¹ and X² together form a methylidene group, X³ is not a C=CH₂ group).
n represents an integer of 1 to 3.
The stereochemistry of the hydroxyl group at C-1 represents either (R) configuration or (S) configuration.
The stereochemistry of the methyl group at C-20 represents either (R) configuration or (S) configuration.]

2. The vitamin D derivative according to claim 1, or a pharmaceutically acceptable salt or solvate thereof, wherein:
the vitamin D derivative represented by formula (1) is represented by the following formula (1A): 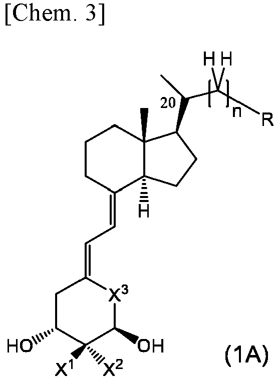 [In the formula, R represents any structure of Ra, Rb, Rc, Rd, or Re in the following formulas. 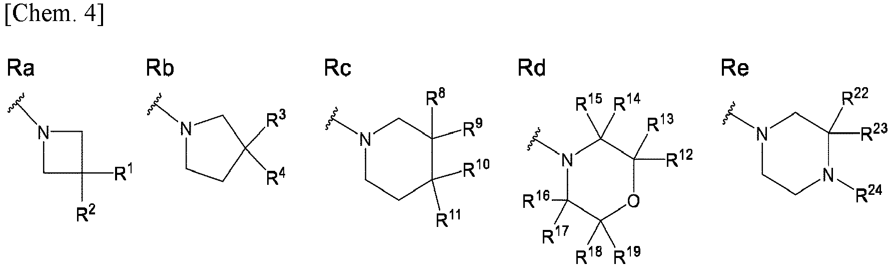
R¹, R³, R⁸ and R¹⁰ each independently represent a C₁ to C₆ alkyl group optionally substituted with 1 to 3 halogen atoms, a C₃ to C₆ cycloalkyl group, a C₁ to C₆ alkoxy group optionally substituted with 1 to 3 halogen atoms, a halogen atom, or a hydrogen atom.
R², R⁴, R⁹ and R¹¹ each independently represent a hydrogen atom, a hydroxy group, or a C₁ to C₆ alkyl group optionally substituted with 1 to 3 halogen atoms. (Wherein, when R¹, R³, R⁸ and R¹⁰ each independently represent a C₁ to C₆ alkoxy group optionally substituted with 1 to 3 halogen atoms, or a halogen atom, each of R², R⁴, R⁹ and R¹¹, substituting the same carbon atom together with R¹, R³, R⁸, or R¹⁰, is not a hydroxy group.)
R¹², R¹³, R¹⁴, R¹⁵, R¹⁶, R¹⁷, R¹⁸, R¹⁹, R²², and R²³ each independently represent a hydrogen atom, a C₁ to C₆ alkyl group optionally substituted with 1 to 3 halogen atoms, or a C₃ to C₆ cycloalkyl group.
Each pair of R¹ and R², R³ and R⁴, R⁸ and R⁹, R¹⁰ and R¹¹, R¹² and R¹³, R¹⁴ and R¹⁵, R¹⁶ and R¹⁷, R¹⁸ and R¹⁹, and R²² and R²³ can be bonded with each other to form a 3-to 5-membered ring structure.
R²⁴ represents a hydrogen atom, a C₁ to C₃ alkyl group or a C₁ to C₃ alkylsulfonyl group.
X¹ and X² each independently represent a hydrogen atom or a C₁ to C₃ alkyl group, or X¹ and X² together form a methylidene group, or -(CH₂)ₘ- (wherein m is an integer of 2 to 5).
X³ represents a CH₂ group, or a C=CH₂ group (wherein, when X¹ and X² together form a methylidene group, X³ is not a C=CH₂ group).
n represents an integer of 1 to 3.
The stereochemistry of the methyl group at C-20 represents either (R) configuration or (S) configuration.]

3. The vitamin D derivative according to claim 2 or a pharmaceutically acceptable salt or solvate thereof, wherein:
in the above formula (1)
R¹, R³, R⁸ and R¹⁰ each independently represent a C₁ to C₆ alkyl group optionally substituted with 1 to 3 fluorine atoms, a C₃ to C₆ cycloalkyl group, a C₁ to C₆ alkoxy group optionally substituted with 1 to 3 fluorine atoms, a fluorine atom, or a hydrogen atom.
R², R⁴, R⁹ and R¹¹ each independently represent a hydrogen atom, a hydroxy group, or a C₁ to C₃ alkyl group optionally substituted with 1 to 3 fluorine atoms. (Wherein, when R¹, R³, R⁸ and R¹⁰ each independently represent a C₁ to C₆ alkoxy group optionally substituted with 1 to 3 fluorine atoms, or a fluorine atom, each of R², R⁴, R⁹ and R¹¹, substituting the same carbon atom together with R^{1,} R^{3,} R⁸, or R¹⁰, is not a hydroxy group.)
R¹², R¹³, R¹⁴, R¹⁵, R¹⁶, R¹⁷, R¹⁸, R¹⁹, R²², and R²³, each independently represent a hydrogen atom, a C₁ to C₆ alkyl group optionally substituted with 1 to 3 fluorine atoms, or a C₃ to C₆ cycloalkyl group.
Each pair of R¹ and R², R³ and R⁴, R⁸ and R⁹, R¹⁰ and R¹¹, R¹² and R¹³, R¹⁴ and R¹⁵, R¹⁶ and R¹⁷, R¹⁸ and R¹⁹, and R²² and R²³ can be bonded with each other to form a 3-to 5-membered ring structure.
R²⁴ represents a hydrogen atom, a C₁ to C₃ alkyl group or a C₁ to C₃ alkylsulfonyl group.
X¹ and X² each independently represent a hydrogen atom or a methyl group, or X¹ and X² together form a methylidene group.
X³ represents a CH₂ group, or a C=CH₂ group (wherein, when X¹ and X² together form a methylidene group, X³ is not a C=CH₂ group).
n represents an integer of 1 to 3.
The stereochemistry of the methyl group at C-20 represents either (R) configuration or (S) configuration.

4. The vitamin D derivative according to any one of claims 1 to 3 or a pharmaceutically acceptable salt or solvate thereof, wherein R represents Ra.

5. The vitamin D derivative according to any one of claims 1 to 3 or a pharmaceutically acceptable salt or solvate thereof, wherein R represents Rb.

6. The vitamin D derivative according to any one of claims 1 to 3 or a pharmaceutically acceptable salt or solvate thereof, wherein R represents Rc.

7. The vitamin D derivative according to any one of claims 1 to 3 or a pharmaceutically acceptable salt or solvate thereof, wherein R represents Rd.

8. The vitamin D derivative according to any one of claims 1 to 3 or a pharmaceutically acceptable salt or solvate thereof, wherein R represents Re.

9. The vitamin D derivative according to any one of claims 1, or a pharmaceutically acceptable salt or solvate thereof, wherein:
the vitamin D derivative represented by formula (1) is represented by the following formula (1B): 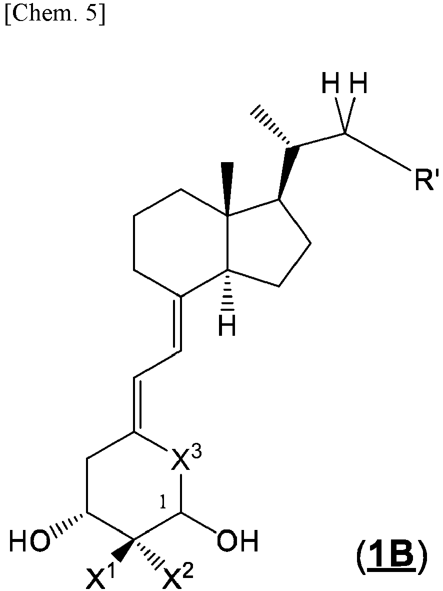 [In the formula, R' represents any structure of Rb', Rc', or Re' in the following formulas. 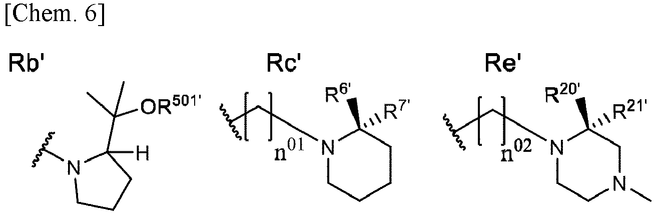
R^{6'}, R^{7'}, R^{20'}, and R^{21'} each independently represent a C₁ to C₆ alkyl group optionally substituted with 1 to 3 halogen atoms, a C₃ to C₆ cycloalkyl group or a hydrogen atom (for each pair of R^{6'}, R^{7'}, R^{20'}, and R^{21'}, except when both are a hydrogen atom at the same time).
Each pair of R^{6'} and R^{7'}, and R^{20'} and R^{21'} can be bonded with each other to form a 3- to 5- membered ring structure.
R^{501'} represents a hydrogen atom or a C₁ to C₆ alkyl group.
n⁰¹ and n⁰² each independently represent an integer of 0 or 1.
The stereochemistry at C-2 in the pyrrolidine ring (Rb') represents either (R) configuration or (S) configuration.
X¹ and X² each independently represent a hydrogen atom or a C₁ to C₃ alkyl group, or X¹ and X² together form a methylidene group, or -(CH₂)ₘ- (wherein m is an integer of 2 to 5).
X³ represents a CH₂ group, or a C=CH₂ group (wherein, when X¹ and X² together form a methylidene group, X³ is not a C=CH₂ group.).
The stereochemistry of the hydroxyl group at C-1 represents either (R) configuration or (S) configuration.]

10. The vitamin D derivative according to claim 10 or a pharmaceutically acceptable salt or solvate thereof, wherein R' represents Rb'.

11. The vitamin D derivative according to claim 10 or a pharmaceutically acceptable salt or solvate thereof, wherein R' represents Rc'.

12. The vitamin D derivative according to claim 10 or a pharmaceutically acceptable salt or solvate thereof, wherein R' represents Re'.

13. The vitamin D derivative according to any one of claims 1, or a pharmaceutically acceptable salt or solvate thereof, wherein:
the vitamin D derivative represented by formula (1) is represented by the following formula (1B-4): 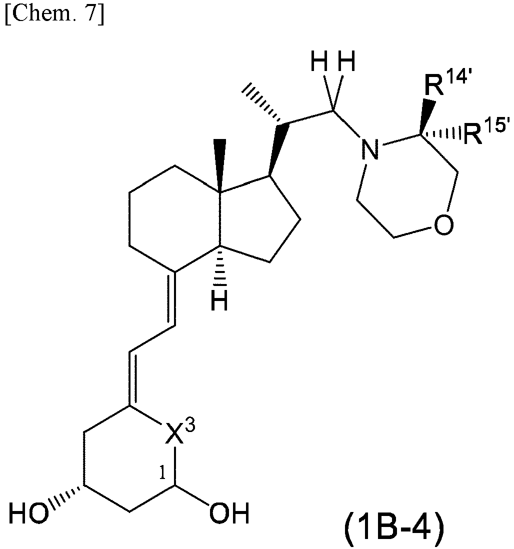 [In the formula (1B-4), R^{14'} and R^{15'} each independently represent a C₁ to C₆ alkyl group optionally substituted with 1 to 3 halogen atoms, a C₃ to C₆ cycloalkyl group, or a hydrogen atom. (Except when R^{14'} and R^{15'} are a hydrogen atom at the same time.)
R^{14'} and R^{15'} can be bonded with each other to form a 3- to 5-membered ring structure.
X³ represents a CH₂ group or C=CH₂ group.
The stereochemistry of the hydroxyl group at C-1 represents either (R) configuration or (S) configuration.]

14. The vitamin D derivative according to any one of claims 1 to 12 or a pharmaceutically acceptable salt or solvate thereof, wherein X¹ represents a hydrogen atom or a methyl group, X² represents a hydrogen atom, and X³ represents a C=CH₂ group.

15. The vitamin D derivative according to any one of claims 1 to 13 or a pharmaceutically acceptable salt or solvate thereof, wherein X¹ and X² represent a hydrogen atom, and X³ represents a C=CH₂ group.

16. The vitamin D derivative according to any one of claims 1 to 13 or a pharmaceutically acceptable salt or solvate thereof, wherein X¹ and X² represent a hydrogen atom and X³ represents a CH₂ group.

17. The vitamin D derivative according to any one of claims 1 to 12 or a pharmaceutically acceptable salt or solvate thereof, wherein X¹ and X² together form a methylidene group, and X³ represents a CH₂ group.

18. A vitamin D derivative of any of the followings or a pharmaceutically acceptable salt or solvate thereof:
(1) (1R,3S,Z)-5-(2-((1R,3aS,7aR,E)-7a-methyl-1-((S)-1-((S)-3-methylpyrrolidin-1-yl)propan-2-yl)octahydro-4H-inden-4-ylidene)ethylidene)-4-methylenecyclohexane-1,3-diol (Compound B001)
(2) (1R,3R)-5-(2-((1R,3aS,7aR,E)-7a-methyl-1-((S)-1-((S)-3-methylpyrrolidin-1-yl)propan-2-yl)octahydro-4H-inden-4-ylidene)ethylidene)-2-methylenecyclohexane-1,3-diol (Compound B002)
(3) (1R,3R)-5-(2-((1R,3aS,7aR,E)-7a-methyl-1-((S)-1-((R)-3-methylpyrrolidin-1-yl)propan-2-yl)octahydro-4H-inden-4-ylidene)ethylidene)-2-methylenecyclohexane-1,3-diol (Compound B003)
(4) (1R,3R)-5-(2-((1R,3aS,7aR,E)-7a-methyl-1-((S)-1-((R)-3-methylpyrrolidin-1-yl)propan-2-yl)octahydro-4H-inden-4-ylidene)ethylidene)cyclohexane-1,3 -diol (Compound B004)
(5) (1R,3R)-5-(2-((1R,3aS,7aR,E)-1-((S)-1-((S)-3-ethylpyrrolidin-1-yl)propan-2-yl)-7a-methyloctahydro-4H-inden-4-ylidene)ethylidene)-2-methylenecyclohexane-1,3-diol (Compound B005)
(6) (1R,3R)-5-(2-((1R,3aS,7aR,E)-1-((S)-1-((R)-3-ethylpyrrolidin-1-yl)propan-2-yl)-7a-methyloctahydro-4H-inden-4-ylidene)ethylidene)-2-methylenecyclohexane-1,3-diol (Compound B006)
(7) (1R,3R)-5-(2-((1R,3aS,7aR,E)-1-((S)-1-((S)-3-ethylpyrrolidin-1-yl)propan-2-yl)-7a-methyloctahydro-4H-inden-4-ylidene)ethylidene)cyclohexane-1,3-diol (Compound B007)
(8) (1R,3R)-5-(2-((1R,3aS,7aR,E)-1-((S)-1-((R)-3-ethylpyrrolidin-1-yl)propan-2-yl)-7a-methyloctahydro-4H-inden-4-ylidene)ethylidene)cyclohexane-1,3-diol (Compound B008)
(9) (1R,3S,Z)-5-(2-((1R,3aS,7aR,E)-1-((R)-1-((S)-3-fluoropyrrolidin-1-yl)propan-2-yl)-7a-methyloctahydro-4H-inden-4-ylidene)ethylidene)-4-methylenecyclohexane-1,3-diol (Compound B018)
(10) (1R,3S,Z)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)-4-methylenecyclohexane-1,3-diol (Compound B020)
(11) (1R,2S,3S,Z)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)-2-methyl-4-methylenecyclohexane-1,3-diol (Compound B021)
(12) (1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)cyclohexane-1,3-diol (Compound B022)
(13) (1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)-2-methylenecyclohexane-1,3-diol (Compound B023)
(14) (1R,3S,Z)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)-4-methylenecyclohexane-1,3-diol (Compound B024)
(15) (1R,2S,3S,Z)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)-2-methyl-4-methylenecyclohexane-1,3-diol (Compound B025)
(16) (1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)cyclohexane-1,3-diol (Compound B026)
(17) (1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)-2-methylenecyclohexane-1,3-diol (Compound B027)
(18) (1R,3S,Z)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)-4-methylenecyclohexane-1,3-diol (Compound B028)
(19) (1R,2S,3S,Z)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B029) )
(20) (1R,3R)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)cyclohexane-1,3-diol (Compound B030)
(21) (1R,3R)-5-(2-((1R,3aS,7aR,E)-1-((S)-1-((S)-3-(1,1-difluoroethyl)pyrrolidin)-1-yl)propan-2-yl)-7a-methyloctahydro-4H-inden-4-ylidene)ethylidene)-2-methylenecyclohexane-1,3-diol (Compound B031)
(22) (1R,3S,Z)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)-4-methylenecyclohexane-1,3-diol (Compound B032)
(23) (1R,2S,3S,Z)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B033) )
(24) (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (Compound B034)
(25) (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)-2-methylenecyclohexane-1,3-diol (Compound B035)
(26) (1R,3S,Z)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-4-methylenecyclohexane-1,3-diol (Compound B036)
(27) (1R,3S,Z)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)-4-methylenecyclohexane-1,3-diol (Compound B037)
(28) (1R,2S,3S,Z)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B038))
(29) (1R,2S,3S,Z)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-2-methyl-4-methylenecyclohexane-1,3-diol (Compound B039))
(30) (1R,3R)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)-2-methylenecyclohexane-1,3-diol (Compound B040)
(31) (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)-2-methylenecyclohexane-1,3-diol (Compound B041)
(32) (1R,3R)-5-(2-((1R,3aS,7aR,E)-1-((S)-1-((R)-3-(2,2-difluoroethyl)pyrrolidin-1-yl)propan-2-yl)-7a-methyloctahydro-4H-inden-4-ylidene)ethylidene)cyclohexane-1,3-diol (Compound B042)
(33) (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol (Compound B043)
(34) (1R,3S,Z)-5-(2-((1R,3 aS,7aR,E)-1-((2S)-1-(3-hydroxy-3-(trifluoromethyl)pyrrolidin-1-yl)propan-2-yl)-7a-methyloctahydro-4H-inden-4-ylidene)ethylidene)-4-methylenecyclohexane-1,3-diol (Compound B044)
(35) (1R,3R)-5-(2-((1R,3aS,7aR,E)-1-((2S)-1-(3-hydroxy-3-(trifluoromethyl)pyrrolidin-1-yl)propan-2-yl)-7a-methyloctahydro-4H-inden-4-ylidene)ethylidene)cyclohexane-1,3-diol (Compound B045)
(36) (1R,3R)-5-(2-((1R,3aS,7aR,E)-1-((2S)-1-(3-hydroxy-3-(trifluoromethyl)pyrrolidin-1-yl)propan-2-yl)-7a-methyloctahydro-4H-inden-4-ylidene)ethylidene)-2-methylenecyclohexane-1,3-diol (Compound B046)
(37) (1R,3S,Z)-5-(2-((1R,3aS,7aR,E)-1-((S)-1-((S)-3-(difluoromethoxy)pyrrolidin-1-yl)propan-2-yl)-7a-methyloctahydro-4H-inden-4-ylidene)ethylidene)-4-methylenecyclohexane-1,3-diol (Compound B047)
(38) (1R,3S,Z)-5-(2-((1R,3aS,7aR,E)-1-((S)-1-((R)-3-(difluoromethoxy)pyrrolidin-1-yl)propan-2-yl)-7a-methyloctahydro-4H-inden-4-ylidene)ethylidene)-4-methylenecyclohexane-1,3-diol (Compound B048)
(39) (1R,3R)-5-(2-((1R,3aS,7aR,E)-1-((S)-1-((S)-3-(difluoromethoxy)pyrrolidin-1-yl)propan-2-yl)-7a-methyloctahydro-4H-inden-4-ylidene)ethylidene)-2-methylenecyclohexane-1,3-diol (Compound B049)
(40) (1R,3R)-5-(2-((1R,3aS,7aR,E)-1-((S)-1-((R)-3-(difluoromethoxy)pyrrolidin-1-yl)propan-2-yl)-7a-methyloctahydro-4H-inden-4-ylidene)ethylidene)-2-methylenecyclohexane-1,3-diol (Compound B050)
(41) (1R,3R)-5-(2-((1R,3aS,7aR,E)-1-((S)-1-((S)-3-(difluoromethoxy)pyrrolidin-1-yl)propan-2-yl)-7a-methyloctahydro-4H-inden-4-ylidene)ethylidene)cyclohexane-1,3-diol (Compound B051)
(42) (1R,3R)-5-(2-((1R,3aS,7aR,E)-1-((S)-1-((R)-3-(difluoromethoxy)pyrrolidin-1-yl)propan-2-yl)-7a-methyloctahydro-4H-inden-4-ylidene)ethylidene)cyclohexane-1,3-diol (Compound B052)
(43) (1R,3S,Z)-5-(2-((1R,3aS,7aR,E)-1-((S)-1-((S)-3-(2,2-difluoroethoxy)pyrrolidin-1-yl)propan-2-yl)-7a-methyloctahydro-4H-inden-4-ylidene)ethylidene)-4-methylenecyclohexane-1,3-diol (Compound B053)
(44) (1R,3S,Z)-5-(2-((1R,3aS,7aR,E)-1-((S)-1-((R)-3-(2,2-difluoroethoxy)pyrrolidin-1-yl)propan-2-yl)-7a-methyloctahydro-4H-inden-4-ylidene)ethylidene)-4-methylenecyclohexane-1,3-diol (Compound B054)
(45) (1R,3R)-5-(2-((1R,3aS,7aR,E)-1-((S)-1-((S)-3-(2,2-difluoroethoxy)pyrrolidin-1-yl)propan-2-yl)-7a-methyloctahydro-4H-inden-4-ylidene)ethylidene)-2-methylenecyclohexane-1,3-diol (Compound B055)
(46) (1R,3R)-5-(2-((1R,3aS,7aR,E)-1-((S)-1-((R)-3-(2,2-difluoroethoxy)pyrrolidin-1-yl)propan-2-yl)-7a-methyloctahydro-4H-inden-4-ylidene)ethylidene)-2-methylenecyclohexane-1,3-diol (Compound B056)
(47) (1R,3R)-5-(2-((1R,3aS,7aR,E)-1-((S)-1-((S)-3-(2,2-difluoroethoxy)pyrrolidin-1-yl)propan-2-yl)-7a-methyloctahydro-4H-inden-4-ylidene)ethylidene)cyclohexane-1,3-diol (Compound B057)
(48) (1R,3R)-5-(2-((1R,3aS,7aR,E)-1-((S)-1-((R)-3-(2,2-difluoroethoxy)pyrrolidin-1-yl)propan-2-yl)-7a-methyloctahydro-4H-inden-4-ylidene)ethylidene)cyclohexane-1,3-diol (Compound B058)
(49) (1R,3S,Z)-5-(2-((1R,3aS,7aR,E)-1-((S)-1-((S)-3-(3,3-difluoropropyl)pyrrolidin-1-yl)propan-2-yl)-7a-methyloctahydro-4H-inden-4-ylidene)ethylidene)-4-methylenecyclohexane-1,3-diol (Compound B059)
(50) (1R,3S,Z)-5-(2-((1R,3aS,7aR,E)-1-((S)-1-((R)-3-(3,3-difluoropropyl)pyrrolidin-1-yl)propan-2-yl)-7a-methyloctahydro-4H-inden-4-ylidene)ethylidene)-4-methylenecyclohexane-1,3-diol (Compound B060)
(51) (1R,3R)-5-(2-((1R,3aS,7aR,E)-1-((S)-1-((S)-3-(3,3-difluoropropyl)pyrrolidin-1-yl)propan-2-yl)-7a-methyloctahydro-4H-inden-4-ylidene)ethylidene)-2-methylenecyclohexane-1,3-diol (Compound B061)
(52) (1R,3R)-5-(2-((1R,3aS,7aR,E)-1-((S)-1-((R)-3-(3,3-difluoropropyl)pyrrolidin-1-yl)propan-2-yl)-7a-methyloctahydro-4H-inden-4-ylidene)ethylidene)-2-methylenecyclohexane-1,3-diol (Compound B062)
(53) (1R,3R)-5-(2-((1R,3aS,7aR,E)-1-((S)-1-((S)-3-(3,3-difluoropropyl)pyrrolidin-1-yl)propan-2-yl)-7a-methyloctahydro-4H-inden-4-ylidene)ethylidene)cyclohexane-1,3-diol (Compound B063)
(54) (1R,3R)-5-(2-((1R,3aS,7aR,E)-1-((S)-1-((R)-3-(3,3-difluoropropyl)pyrrolidin-1-yl)propan-2-yl)-7a-methyloctahydro-4H-inden-4-ylidene)ethylidene)cyclohexane-1,3-diol (Compound B064)
(55) (1R,3R)-5-(2-((1R,3aS,7aR,E)-1-((R)-4-((S)-3-(difluoromethyl)pyrrolidin-1-yl)butan-2-yl)-7a-methyloctahydro-4H-inden-4-ylidene)ethylidene)cyclohexane-1,3-diol (Compound B080)
(56) (1R,3R)-5-(2-((1R,3aS,7aR,E)-1-((R)-4-((R)-3-(difluoromethyl)pyrrolidin-1-yl)butan-2-yl)-7a-methyloctahydro-4H-inden-4-ylidene)ethylidene)cyclohexane-1,3-diol (Compound B081)
(57) (1R,3R)-5-(2-((1R,3aS,7aR,E)-1-((R)-4-((S)-3-(difluoromethoxy)pyrrolidin-1-yl)butan-2-yl)-7a-methyloctahydro-4H-inden-4-ylidene)ethylidene)cyclohexane-1,3-diol (Compound B086)
(58) (1R,3R)-5-(2-((lR,3aS,7aR,E)-1-((R)-4-((R)-3-(difluoromethoxy)pyrrolidin-1-yl)butan-2-yl)-7a-methyloctahydro-4H-inden-4-ylidene)ethylidene)cyclohexane-1,3-diol (Compound B087)
(59) (1R,3S,Z)-5-(2-((1R,3aS,7aR,E)-1-((S)-1-(4-hydroxy-4-(trifluoromethyl)piperidin-1-yl)propan-2-yl)-7a-methyloctahydro-4H-inden-4-ylidene)ethylidene)-4-methylenecyclohexane-1,3-diol (Compound C001)
(60) (1R,3R)-5-(2-((1R,3aS,7aR,E)-1-((S)-1-(4-hydroxy-4-(trifluoromethyl)piperidin-1-yl)propan-2-yl)-7a-methyloctahydro-4H-inden-4-ylidene)ethylidene)cyclohexane-1,3-diol (Compound C002)
(61) (1R,3S,Z)-5-(2-((1R,3aS,7aR,E)-1-((R)-1-(4-hydroxy-4-(trifluoromethyl)piperidin-1-yl)propan-2-yl)-7a-methyloctahydro-4H-inden-4-ylidene)ethylidene)-4-methylenecyclohexane-1,3-diol (Compound C005)
(62) (1R,2S,3S,Z)-5-(2-((1R,3aS,7aR,E)-1-((R)-1-(4-hydroxy-4-(trifluoromethyl)piperidin-1-yl)propan-2-yl)-7a-methyloctahydro-4H-inden-4-ylidene)ethylidene)-4-methylenecyclohexane-1,3-diol (Compound C006)
(63) (1R,3R)-5-(2-((1R,3aS,7aR,E)-1-((R)-1-(4-(difluoromethyl)piperidin-1-yl)propan-2-yl)-7a-methyloctahydro-4H-inden-4-ylidene)ethylidene)cyclohexane-1,3-diol (Compound C019)
(64) (1R,3R)-5-(2-((1R,3aS,7aR,E)-1-((S)-1-(4-(difluoromethyl)-4-hydroxypiperidin-1-yl)propan-2-yl)-7a-methyloctahydro-4H-inden-4-ylidene)ethylidene)-2-methylenecyclohexane-1,3-diol (Compound C022)
(65) (1R,3R)-5-(2-((1R,3aS,7aR,E)-1-((2S)-1-(3-(difluoromethyl)-3-hydroxypiperidin-1-yl)propan-2-yl)-7a-methyloctahydro-4H-inden-4-ylidene)ethylidene)-2-methylenecyclohexane-1,3-diol (Compound C036)
(66) (1R,3S,Z)-5-(2-((1R,3aS,7aR,E)-1-((2S)-1-(3-hydroxy-3-methylpiperidin-1-yl)propan-2-yl)-7a-methyloctahydro-4H-inden-4-ylidene)ethylidene)-4-methylenecyclohexane-1,3-diol (Compound C037)
(67) (1R,3R)-5-(2-((1R,3aS,7aR,E)-1-((S)-1-((S)-3-hydroxy-3-methylpiperidin-1-yl)propan-2-yl)-7a-methyloctahydro-4H-inden-4-ylidene)ethylidene)cyclohexane-1,3-diol (Compound C039)
(68) (1R,3S,Z)-5-(2-((1R,3aS,7aR,E)-1-((S)-1-((S)-3-(difluoromethyl)piperidin-1-yl)propan-2-yl)-7a-methyloctahydro-4H-inden-4-ylidene)ethylidene)-4-methylenecyclohexane-1,3-diol (Compound C048)
(69) (1R,3R)-5-(2-((1R,3aS,7aR,E)-1-((S)-1-((S)-3-(difluoromethyl)piperidin-1-yl)propan-2-yl)-7a-methyloctahydro-4H-inden-4-ylidene)ethylidene)-2-methylenecyclohexane-1,3-diol (Compound C049)
(70) (1R,2S,3S,Z)-5-(2-((1R,3aS,7aR,E)-1-((S)-1-((S)-3-(difluoromethyl)piperidin-1-yl)propan-2-yl)-7a-methyloctahydro-4H-inden-4-ylidene)ethylidene)-2-methyl-4-methylenecyclohexane-1,3-diol (Compound C050)
(71) (1R,3R)-5-(2-((1R,3aS,7aR,E)-1-((S)-1-((R)-3-(2,2-difluoroethyl)piperidin-1-yl)propan-2-yl)-7a-methyloctahydro-4H-inden-4-ylidene)ethylidene)cyclohexane-1,3-diol (Compound C051)
(72) (1R,3S,Z)-5-(2-((1R,3aS,7aR,E)-1-((S)-1-((S)-3-(1,1-difluoroethyl)piperidin-1-yl)propan-2-yl)-7a-methyloctahydro-4H-inden-4-ylidene)ethylidene)-4-methylenecyclohexane-1,3-diol (Compound C052)
(73) (1R,3R)-5-(2-((1R,3aS,7aR,E)-1-((S)-1-((S)-3-(1,1-difluoroethyl)piperidin-1-yl)propan-2-yl)-7a-methyloctahydro-4H-inden-4-ylidene)ethylidene)-2-methylenecyclohexane-1,3-diol (Compound C053)
(74) (1R,2S,3S,Z)-5-(2-((1R,3aS,7aR,E)-1-((S)-1-((S)-3-(1,1-difluoroethyl)piperidin-1-yl)propan-2-yl)-7a-methyloctahydro-4H-inden-4-ylidene)ethylidene)-2-methyl-4-methylenecyclohexane-1,3-diol (Compound C054) )
(75) (1R,3R)-5-(2-((1R,3aS,7aR,E)-1-((S)-1-(4-(1,1-difluoroethyl)piperidin-1-yl)propan-2-yl)-7a-methyloctahydro-4H-inden-4-ylidene)ethylidene)-2-methylenecyclohexane-1,3-diol (Compound C055)
(76) (1R,3R)-5-(2-((1R,3aS,7aR,E)-1-((R)-1-(4-(2,2-difluoroethyl)piperidin-1-yl)propan-2-yl)-7a-methyloctahydro-4H-inden-4-ylidene)ethylidene)-2-methylenecyclohexane-1,3-diol (Compound C056)
(77) (1R,3R)-5-(2-((1R,3aS,7aR,E)-1-((R)-1-(4-(1,1-difluoroethyl)piperidin-1-yl)propan-2-yl)-7a-methyloctahydro-4H-inden-4-ylidene)ethylidene)-2-methylenecyclohexane-1,3-diol (Compound C057)
(78) (1R,3R)-5-(2-((1R,3aS,7aR,E)-1-((S)-1-(4-(2,2-difluoroethyl)piperidin-1-yl)propan-2-yl)-7a-methyloctahydro-4H-inden-4-ylidene)ethylidene)-2-methylenecyclohexane-1,3-diol (Compound C059)
(79) (1R,3R)-5-(2-((1R,3aS,7aR,E)-7a-methyl-1-((S)-1-morpholinopropan-2-yl)-octahydro-4H-inden-4-ylidene)ethylidene)cyclohexane-1,3-diol (Compound D004)
(80) (1R,3R)-5-(2-((1R,3aS,7aR,E)-1-((S)-1-((R)-2-(difluoromethyl)morpholino)propan-2-yl)-7a-methyloctahydro-4H-inden-4-ylidene)ethylidene)-2-methylenecyclohexane-1,3-diol (Compound D008)
(81) (1R,3R)-5-(2-((1R,3aS,7aR,E)-7a-methyl-1-((S)-1-((S)-2-methylmorpholino)propan-2-yl)-octahydro-4H-inden-4-ylidene)ethylidene)-2-methylenecyclohexane-1,3-diol (Compound DO10)
(82) (1R,3R)-5-(2-((1R,3aS,7aR,E)-1-((S)-1-(4-oxa-7-azaspiro[2.5]octan-7-yl)propan-2-yl)-7a-methyloctahydro-4H-inden-4-ylidene)ethylidene)cyclohexane-1,3-diol (Compound D019)
(83) (1R,3S,Z)-5-(2-((1R,3aS,7aR,E)-7a-methyl-1-((R)-4-morpholinobutan-2-yl)octahydro-4H-inden-4-ylidene)ethylidene)-4-methylenecyclohexane-1,3 -diol (Compound D022)
(84) (1R,3R)-5-(2-((1R,3aS,7aR,E)-7a-methyl-1-((R)-4-morpholinobutan-2-yl)octahydro-4H-inden-4-ylidene)ethylidene)cyclohexane-1,3-diol (Compound D023)
(85) (1R,3R)-5-(2-((1R,3aS,7aR,E)-7a-methyl-1-((R)-4-morpholinobutan-2-yl)octahydro-4H-inden-4-ylidene)ethylidene)-2-methylenecyclohexane-1,3-diol (Compound D024)
(86) (1R,3R)-5-(2-((1R,3aS,7aR,E)-7a-methyl-1-((R)-4-((S)-2-methylmorpholino)butan-2-yl)octahydro-4H-inden-4-ylidene)ethylidene)-2-methylenecyclohexane-1,3-diol (Compound D025)
(87) (1R,3R)-5-(2-((1R,3aS,7aR,E)-7a-methyl-1-((R)-4-((R)-3-methylmorpholino)butan-2-yl)octahydro-4H-inden-4-ylidene)ethylidene)-2-methylenecyclohexane-1,3-diol (Compound D031)
(88) (1R,3R)-5-(2-((1R,3aS,7aR,E)-7a-methyl-1-((R)-4-((S)-3-methylmorpholino)butan-2-yl)octahydro-4H-inden-4-ylidene)ethylidene)-2-methylenecyclohexane-1,3-diol (Compound D032)
(89) (1R,3R)-5-(2-((1R,3aS,7aR,E)-1-((R)-4-((S)-2-(difluoromethyl)morpholino)butan-2-yl)-7a-methyloctahydro-4H-inden-4-ylidene)ethylidene)-2-methylenecyclohexane-1,3-diol (Compound D033)
(90) (1R,3R)-5-(2-((1R,3aS,7aR,E)-1-((R)-4-((S)-2-(difluoromethyl)morpholino)butan-2-yl)-7a-methyloctahydro-4H-inden-4-ylidene)ethylidene)cyclohexane-1,3-diol (Compound D034)
(91) (1R,3S,Z)-5-(2-((1R,3aS,7aR,E)-1-((R)-4-((S)-2-(difluoromethyl)morpholino)butan-2-yl)-7a-methyloctahydro-4H-inden-4-ylidene)ethylidene)-4-methyloctahydro-1,3-diol (Compound D035)
(92) (1R,3R)-5-(2-((1R,3aS,7aR,E)-7a-methyl-1-((R)-4-((R)-2-methylmorpholino)butan-2-yl)octahydro-4H-inden-4-ylidene)ethylidene)-2-methylenecyclohexane-1,3-diol (Compound D040)
(93) (1R,3R)-5-(2-((1R,3aS,7aR,E)-7a-methyl-1-((R)-4-((S)-2-methylmorpholino)butan-2-yl)octahydro-4H-inden-4-ylidene)ethylidene)cyclohexane-1,3-diol (Compound D041)
(94) (1R,3R)-5-(2-((1R,3aS,7aR,E)-7a-methyl-1-((R)-4-((R)-3-methylmorpholino)butan-2-yl)octahydro-4H-inden-4-ylidene)ethylidene)cyclohexane-1,3-diol (Compound D042)
(95) (1R,3R)-5-(2-((1R,3aS,7aR,E)-7a-methyl-1-((R)-4-((S)-3-methylmorpholino)butan-2-yl)octahydro-4H-inden-4-ylidene)ethylidene)cyclohexane-1,3-diol (Compound D043)
(96) (1R,3R)-5-(2-((1R,3aS,7aR,E)-7a-methyl-1-((R)-5-morpholinopentan-2-yl)octahydro-4H-inden-4-ylidene)ethylidene)cyclohexane-1,3-diol (Compound D044)
(97) (1R,3R,Z)-5-(2-((1R,3aS,7aR,E)-7a-methyl-1-((R)-4-((S)-3-methylmorpholino)butan-2-yl)octahydro-4H-inden-4-ylidene)ethylidene)-4-methylenecyclohexane-1,3-diol (Compound D045)
(98) (1R,3R,Z)-5-(2-((1R,3aS,7aR,E)-7a-methyl-1-((R)-4-((R)-3-methylmorpholino)butan-2-yl)octahydro-4H-inden-4-ylidene)ethylidene)-4-methylenecyclohexane-1,3-diol (Compound D046)
(99) (1R,3R,Z)-5-(2-((1R,3aS,7aR,E)-7a-methyl-1-((R)-4-((R)-2-methylmorpholino)butan-2-yl)octahydro-4H-inden-4-ylidene)ethylidene)-4-methylenecyclohexane-1,3-diol (Compound D047)
(100) (1R,3R,Z)-5-(2-((1R,3aS,7aR,E)-7a-methyl-1-((R)-4-((S)-2-methylmorpholino)butan-2-yl)octahydro-4H-inden-4-ylidene)ethylidene)-4-methylenecyclohexane-1,3-diol (Compound D048)
(101) (1R,3R)-5-(2-((1R,3aS,7aR,E)-1-((R)-4-(3,3-dimethylmorpholino)butan-2-yl)-7a-methyloctahydro-4H-inden-4-ylidene)ethylidene)cyclohexane-1,3-diol (Compound D049)
(201) (1R,3S,Z)-5-(2-((1R,3aS,7aR,E)-7a-methyl-1-((S)-1-((S)-2-methylpiperidin-1-yl)propan-2-yl)octahydro-4H-inden-4-ylidene)ethylidene)-4-methylenecyclohexane-1,3-diol (Compound F001)
(202) (1R,3S,Z)-5-(2-((1R,3aS,7aR,E)-7a-methyl-1-((S)-1-((R)-2-methylpiperidin-1-yl)propan-2-yl)octahydro-4H-inden-4-ylidene)ethylidene)-4-methylenecyclohexane-1,3-diol (Compound F002)
(203) (1R,3S,Z)-5-(2-((1R,3aS,7aR,E)-1-((S)-1-(2,2-dimethylpiperidin-1-yl)propan-2-yl)-7a-methyloctahydro-4H-inden-4-ylidene)ethylidene)-4-methylenecyclohexane-1,3-diol (Compound F003)
(204) (1R,3S,Z)-5-(2-((1R,3aS,7aR,E)-7a-methyl-1-((S)-1-((S)-3-methylmorpholino)propan-2-yl)octahydro-4H-inden-4-ylidene)ethylidene)-4-methylenecyclohexane-1,3-diol (Compound F004)
(205) (1R,3S,Z)-5-(2-((1R,3aS,7aR,E)-7a-methyl-1-((S)-1-((R)-3-methylmorpholino)propan-2-yl)octahydro-4H-inden-4-ylidene)ethylidene)-4-methylenecyclohexane-1,3-diol (Compound F005)
(206) (1R,3S,Z)-5-(2-((1R,3aS,7aR,E)-1-((S)-1-(3,3-dimethylmorpholino)propan-2-yl)-7a-methyloctahydro-4H-inden-4-ylidene)ethylidene)-4-methylenecyclohexane-1,3-diol (Compound F006)
(207) (1R,3S,Z)-5-(2-((1R,3aS,7aR,E)-1-((S)-1-((R)-2-(2-hydroxypropan-2-yl)pyrrolidin-1-yl)propan-2-yl)-7a-methyloctahydro-4H-inden-4-ylidene)ethylidene)-4-methylenecyclohexane-1,3-diol (Compound F013)
(208) (1R,3 S,Z)-5-(2-((1R,3aS,7aR,E)-1-((S)-1-((S)-2-(2-hydroxypropan-2-yl)pyrrolidin-1-yl)propan-2-yl)-7a-methyloctahydro-4H-inden-4-ylidene)ethylidene)-4-methylenecyclohexane-1,3-diol (Compound F014)
(209) (1R,3S,Z)-5-(2-((1R,3aS,7aR,E)-1-((S)-1-((R)-2-(2-methoxypropan-2-yl)pyrrolidin-1-yl)propan-2-yl)-7a-methyloctahydro-4H-inden-4-ylidene)ethylidene)-4-methylenecyclohexane-1,3-diol (Compound F015)
(210) (1R,3S,Z)-5-(2-((1R,3aS,7aR,E)-1-((S)-1-((S)-2-(2-methoxypropan-2-yl)pyrrolidin-1-yl)propan-2-yl)-7a-methyloctahydro-4H-inden-4-ylidene)ethylidene)-4-methylenecyclohexane-1,3-diol (Compound F016)
(211) (1R,3R)-5-(2-((1R,3aS,7aR,E)-7a-methyl-1-((S)-1-((S)-2-methylpiperidin-1-yl)propan-2-yl)octahydro-4H-inden-4-ylidene)ethylidene)cyclohexane-1,3 -diol (Compound G001)
(212) (1R,3R)-5-(2-((1R,3aS,7aR,E)-7a-methyl-1-((S)-1-((R)-2-methylpiperidin-1-yl)propan-2-yl)octahydro-4H-inden-4-ylidene)ethylidene)cyclohexane-1,3 -diol (Compound G002)
(213) (1R,3R)-5-(2-((1R,3aS,7aR,E)-1-((S)-1-(2,2-dimethylpiperidin-1-yl)propan-2-yl)-7a-methyloctahydro-4H-inden-4-ylidene)ethylidene)cyclohexane-1,3-diol (Compound G003)
(214) (1R,3R)-5-(2-((1R,3aS,7aR,E)-7a-methyl-1-((S)-1-((S)-3-methylmorpholino)propan-2-yl)octahydro-4H-inden-4-ylidene)ethylidene)cyclohexane-1,3-diol (Compound G004)
(215) (1R,3R)-5-(2-((1R,3aS,7aR,E)-7a-methyl-1-((S)-1-((R)-3-methylmorpholino)propan-2-yl)octahydro-4H-inden-4-ylidene)ethylidene)cyclohexane-1,3-diol (Compound G005)
(216) (1R,3R)-5-(2-((1R,3aS,7aR,E)-1-((S)-1-(3,3-dimethylmorpholino)propan-2-yl)-7a-methyloctahydro-4H-inden-4-ylidene)ethylidene)cyclohexane-1,3-diol (Compound G006)
(217) (1R,3R)-5-(2-((1R,3aS,7aR,E)-1-((S)-1-((R)-2-(2-hydroxypropan-2-yl)pyrrolidin-1-yl)propan-2-yl)-7a-methyloctahydro-4H-inden-4-ylidene)ethylidene)cyclohexane-1,3-diol (Compound G013)
(218) (1R,3R)-5-(2-((1R,3aS,7aR,E)-1-((S)-1-((S)-2-(2-hydroxypropan-2-yl)pyrrolidin-1-yl)propan-2-yl)-7a-methyloctahydro-4H-inden-4-ylidene)ethylidene)cyclohexane-1,3-diol (Compound G014)
(219) (1R,3R)-5-(2-((1R,3aS,7aR,E)-1-((S)-1-((R)-2-(2-methoxypropan-2-yl)pyrrolidin-1-yl)propan-2-yl)-7a-methyloctahydro-4H-inden-4-ylidene)ethylidene)cyclohexane-1,3-diol (Compound G015)
(220) (1R,3R)-5-(2-((1R,3aS,7aR,E)-1-((S)-1-((S)-2-(2-methoxypropan-2-yl)pyrrolidin-1-yl)propan-2-yl)-7a-methyloctahydro-4H-inden-4-ylidene)ethylidene)cyclohexane-1,3-diol (Compound G016)
(221) (1R,3R)-5-(2-((1R,3aS,7aR,E)-7a-methyl-1-((R)-4-((R)-2-methylpiperidin-1-yl)butan-2-yl)octahydro-4H-inden-4-ylidene)ethylidene)cyclohexane-1,3-diol (Compound G018)
(222) (1R,3R)-5-(2-((1R,3aS,7aR,E)-1-((R)-4-((R)-2,4-dimethylpiperazin-1-yl)butan-2-yl)-7a-methyloctahydro-4H-inden-4-ylidene)ethylidene)cyclohexane-1,3-diol (Compound G020)
(223) (1R,3R)-5-(2-((1R,3aS,7aR,E)-7a-methyl-1-((S)-1-((S)-2-methylpiperidin-1-yl)propan-2-yl)octahydro-4H-inden-4-ylidene)ethylidene)-2-methylenecyclohexane-1,3-diol (Compound H001)
(224) (1R,3R)-5-(2-((1R,3aS,7aR,E)-7a-methyl-1-((S)-1-((R)-2-methylpiperidin-1-yl)propan-2-yl)octahydro-4H-inden-4-ylidene)ethylidene)-2-methylenecyclohexane-1,3-diol (Compound H002)
(225) (1R,3R)-5-(2-((1R,3aS,7aR,E)-1-((S)-1-(2,2-dimethylpiperidin-1-yl)propan-2-yl)-7a-methyloctahydro-4H-inden-4-ylidene)ethylidene)-2-methylenecyclohexane-1,3-diol (Compound H003)
(226) (1R,3R)-5-(2-((1R,3aS,7aR,E)-1-((S)-1-((R)-2-(2-hydroxypropan-2-yl)pyrrolidin-1-yl)propan-2-yl)-7a-methyloctahydro-4H-inden-4-ylidene)ethylidene)-2-methylenecyclohexane-1,3-diol (Compound H013)
(227) (1R,3R)-5-(2-((1R,3aS,7aR,E)-1-((S)-1-((S)-2-(2-hydroxypropan-2-yl)pyrrolidin-1-yl)propan-2-yl)-7a-methyloctahydro-4H-inden-4-ylidene)ethylidene)-2-methylenecyclohexane-1,3-diol (Compound H014)
(228) (1R,3R)-5-(2-((1R,3aS,7aR,E)-1-((S)-1-((R)-2-(2-methoxypropan-2-yl)pyrrolidin-1-yl)propan-2-yl)-7a-methyloctahydro-4H-inden-4-ylidene)ethylidene)-2-methylenecyclohexane-1,3-diol (Compound H015)
(229) (1R,3R)-5-(2-((1R,3aS,7aR,E)-1-((S)-1-((S)-2-(2-methoxypropan-2-yl)pyrrolidin-1-yl)propan-2-yl)-7a-methyloctahydro-4H-inden-4-ylidene)ethylidene)-2-methylenecyclohexane-1,3-diol (Compound H016)
(230) (1R,3R,Z)-5-(2-((1R,3aS,7aR,E)-7a-methyl-1-((S)-1-((S)-2-methylpiperidin-1-yl)propan-2-yl)octahydro-4H-inden-4-ylidene)ethylidene)-4-methylenecyclohexane-1,3-diol (Compound 1001)
(231) (1R,3R,Z)-5-(2-((1R,3aS,7aR,E)-7a-methyl-1-((S)-1-((R)-2-methylpiperidin-1-yl)propan-2-yl)octahydro-4H-inden-4-ylidene)ethylidene)-4-methylenecyclohexane-1,3-diol (Compound 1002)
(232) (1R,3R,Z)-5-(2-((1R,3aS,7aR,E)-1-((S)-1-(2,2-dimethylpiperidin-1-yl)propan-2-yl)-7a-methyloctahydro-4H-inden-4-ylidene)ethylidene)-4-methylenecyclohexane-1,3-diol (Compound 1003)
(233) (1R,3R,Z)-5-(2-((1R,3aS,7aR,E)-7a-methyl-1-((S)-1-((S)-3-methylmorpholino)propan-2-yl)octahydro-4H-inden-4-ylidene)ethylidene)-4-methylenecyclohexane-1,3-diol (Compound 1004)
(234) (1R,3R,Z)-5-(2-((1R,3aS,7aR,E)-7a-methyl-1-((S)-1-((R)-3-methylmorpholino)propan-2-yl)octahydro-4H-inden-4-ylidene)ethylidene)-4-methylenecyclohexane-1,3-diol (Compound 1005)
(235) (1R,3R,Z)-5-(2-((1R,3aS,7aR,E)-1-((S)-1-(3,3-dimethylmorpholino)propan-2-yl)-7a-methyloctahydro-4H-inden-4-ylidene)ethylidene)-4-methylenecyclohexane-1,3-diol (Compound 1006)

19. A vitamin D derivative having a tertiary amine in the side chain, or a pharmaceutically acceptable salt or solvate thereof, **characterized by** promoting differentiation from an oligodendrocyte progenitor cell to an oligodendrocyte by binding to Protein Disulfide isomerase A3 (PDIA3).

20. The vitamin D derivative according to any one of claims 1 to 18 or a pharmaceutically acceptable salt or solvate thereof, **characterized by** promoting differentiation from an oligodendrocyte progenitor cell to an oligodendrocyte by binding to Protein Disulfide isomerase A3 (PDIA3).

21. A pharmaceutical composition comprising the vitamin D derivative according to any one of claims 1 to 18 or a pharmaceutically acceptable salt or solvate thereof, and a pharmaceutically acceptable carrier or base.

22. A remyelination promoter comprising the vitamin D derivative according to any one of claims 1 to 18 or a pharmaceutically acceptable salt or solvate thereof.

23. A therapeutic agent comprising the vitamin D derivative according to any one of claims 1 to 18 or a pharmaceutically acceptable salt or solvate thereof as an active ingredient, the therapeutic agent for one or more diseases selected from the group consisting of multiple sclerosis, neuromyelitis optica, progressive multifocal leukoencephalopathy, multiple system atrophy, acute disseminated encephalomyelitis, atopic myelitis, HTLV-1-associated myelopathy, HIV-associated leukoencephalopathy, Krabbe's disease, Guillain-Barre syndrome, Fisher's syndrome, chronic inflammatory demyelinating polyneuropathy, Charcot-Marie-Tooth disease, Parkinson's disease, schizophrenia, bipolar disorder, major depressive disorder, autistic spectrum disorder, attention deficit hyperactivity disorder, obsessive-compulsive disorder, post-traumatic stress disorders, drug-dependent depression, autism, Alzheimer-type dementia, and ischemic stroke.
